(19) Europäisches Patentamt
European Patent Office
Office européen des brevets

(11) EP 4 778 540 A1

(12) EUROPEAN PATENT APPLICATION
published in accordance with Art. 153(4) EPC

(43) Date of publication:
**22.07.2026 Bulletin 2026/30**

(21) Application number: **24865508.6**

(22) Date of filing: **12.09.2024**

(51) International Patent Classification (IPC):
***A61K 47/68*** *(2017.01)* ***A61K 31/517*** *(2006.01)*
***A61K 39/395*** *(2006.01)* ***A61P 35/00*** *(2006.01)*
***A61P 35/02*** *(2006.01)* ***C07D 403/14*** *(2006.01)*
***C07D 487/08*** *(2006.01)* ***C07K 16/28*** *(2006.01)*

(52) Cooperative Patent Classification (CPC):
**A61K 31/517; A61K 39/395; A61K 47/68; A61P 35/00; A61P 35/02; C07D 403/14; C07D 487/08; C07K 16/28**

(86) International application number:
**PCT/JP2024/032664**

(87) International publication number:
**WO 2025/058008 (20.03.2025 Gazette 2025/12)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **13.09.2023 JP 2023148645**
**26.04.2024 JP 2024072128**

(71) Applicant: **Astellas Pharma Inc.**
**Tokyo 103-8411 (JP)**

(72) Inventors:
- **YAMASHITA, Yu**
  **Tokyo 103-8411 (JP)**
- **YOSHINARI, Tomohiro**
  **Tokyo 103-8411 (JP)**
- **ISHIOKA, Hiroki**
  **Tokyo 103-8411 (JP)**
- **KAWAGUCHI, Kenichi**
  **Tokyo 103-8411 (JP)**
- **WATANABE, Hideyuki**
  **Tokyo 103-8411 (JP)**
- **KAWAMINAMI, Eiji**
  **Tokyo 103-8411 (JP)**
- **HAMAGUCHI, Hisao**
  **Tokyo 103-8411 (JP)**
- **TAKAHASHI, Fumie**
  **Tokyo 103-8411 (JP)**
- **MORIKAWA, Takahiro**
  **Tokyo 103-8411 (JP)**
- **IMAIZUMI, Tomoyoshi**
  **Tokyo 103-8411 (JP)**
- **KAMIKUBO, Takashi**
  **Tokyo 103-8411 (JP)**
- **INAMI, Hiroshi**
  **Tokyo 103-8411 (JP)**
- **SAITO, Asako**
  **Tokyo 103-8411 (JP)**

(74) Representative: **Potter Clarkson**
**Chapel Quarter**
**Mount Street**
**Nottingham NG1 6HQ (GB)**

Remarks:
The complete document including Reference Table(s) and the Sequence Listing(s) can be downloaded from the EPO website

# (54) ANTIBODY-DRUG CONJUGATE CONTAINING HETEROCYCLIC COMPOUND HAVING ACTIVITY OF INDUCING DECOMPOSITION OF G12D MUTANT KRAS PROTEIN

(57) Provided is an antibody-drug conjugate for use in the treatment of a cancer expressing G12D mutant KRAS. Also provided is a drug-linker conjugate for use in the antibody-drug conjugate. The present inventors have conjugated a heterocyclic compound represented by formula (I) to an antibody, for example, an anti-EGFR antibody, to prepare an antibody-drug conjugate capable of delivering the compound to a cancer, for example, an EGFR-expressing cancer. Moreover, a drug-linker conjugate for use in the antibody-drug conjugate or a salt thereof has been found. The antibody-drug conjugate inhibits G12D mutant KRAS by inducing the degradation of G12D mutant KRAS protein, in cancer, for example, an EGFR-expressing cancer, and exhibits an antitumor effect.



Processed by Luminess, 75001 PARIS (FR)

## Description

TECHNICAL FIELD

The present invention relates to a pharmaceutical composition, particularly, an antibody-drug conjugate or a salt thereof containing a heterocyclic compound having a G12D mutant KRAS protein degradation-inducing effect (hereinafter, the compound is also referred to as a drug). The present invention also relates to a drug-linker conjugate or a salt thereof for use in the antibody-drug conjugate or the salt thereof.

BACKGROUND ART

RAS protein is an approximately 21 kDa low-molecular guanosine triphosphate (GTP) binding protein composed of 188 or 189 amino acids, and includes four types of major proteins (KRAS (KRAS4A and KRAS4B), NRAS, and HRAS) resulting from three genes, KRAS gene, NRAS gene, and HRAS gene. The RAS protein has a GTP-bound form which is an active form, and a GDP-bound form which is an inactive form. The RAS protein is activated by the exchange between guanosine diphosphate (GDP) and GTP through the ligand stimulation of a cell membrane receptor such as EGFR, for example. The active RAS binds to as many as 20 types of effector proteins, such as RAF, PI3K, and RALGDS, and activates downstream signal cascades. On the other hand, the active RAS becomes inactive by the conversion of GTP to GDP ascribable to endogenous GTP hydrolysis (GTPase) activity. This GTPase activity is enhanced by GTPase activating protein (GAP). Thus, RAS is responsible for an important function of "molecular switch" for the intracellular signaling pathway of EGFR or the like, and plays an important role in processes such as cell growth, proliferation, and angiogenesis (Nature Rev. Cancer, 2011, 11, p. 761-774; Nature Rev. Drug Discov., 2014, 13, p. 828-851; and Nature Rev. Drug Discov., 2016, 15, p. 771-785).

When amino acid substitution occurs due to a mutation in RAS gene, RAS becomes constantly active due to a reduced function as GTPase or reduced response to GAP and thus continues to send signals to downstream. This excessive signal brings about oncogenesis or increased tumor growth. For example, a mutation in KRAS gene is found in 90% or more patients with pancreatic ductal adenocarcinoma, and the mutation exists from an initial stage of pancreatic intraepithelial neoplasia (PanIN). Moreover, a mutation in KRAS gene is highly frequently found in lung cancer and colorectal cancer. Point mutations in codon 12 positioned in KRAS exon 2 (KRAS G12C mutation, KRAS G12D mutation, etc.) are commonly known as examples of the mutation in KRAS gene (Nat Rev Cancer. 2018. 18. 767-777).

In recent years, bifunctional compounds collectively called PROTAC (PROteolysis-TArgeting Chimera), SNIPER (Specific and Nongenetic IAP-dependent Protein Eraser), or the like have been found as techniques of inducing the degradation of targeted proteins, and expected as one of the novel drug discovery modalities (Drug. Discov. Today Technol., 2019, 31, p. 15-27). These bifunctional compounds promote the intracellular complex formation between a target protein and E3 ligase and ubiquitinate the target protein, thereby inducing the degradation of the target protein by the ubiquitin-proteasome system. The ubiquitin-proteasome system is one of the intracellular protein degradation mechanisms. A protein called E3 ligase recognizes and ubiquitinates the protein to be degraded so that its degradation in proteasome proceeds. More than 600 types of E3 ligases reside *in vivo*. Nonetheless, a limited number of E3 ligases have been used so far as bifunctional degradation inducers called PROTAC, SNIPER, or the like. Representative examples thereof include Von Hippel-Lindau (VHL), celebron (CRBN), inhibitor of apoptosis protein (IAP), and mouse double minute 2 homolog (MDM2).

These bifunctional compounds are compounds in which a ligand of a target protein and a ligand of E3 ligase are linked via a linker. PTL 1 and PTL 2 have each reported a bifunctional compound that decreases a G12D mutant KRAS protein level. PTL 3 has reported a bifunctional compound that regulates G12D mutant KRAS protein. PTL 4 has reported a compound having G12D mutant KRAS-degrading effect. PTL 5 has reported a quinazoline compound for inducing the degradation of G12D mutant KRAS protein. PTL 6 has reported a compound having G12D mutant KRAS-degrading effect. PTL 7 has reported a heteroaromatic cyclic compound having G12D mutant KRAS-degrading effect. PTL 8, which has been published after the priority date of the present application, has reported a heterocyclic compound for inducing the degradation of G12D mutant KRAS protein. Likewise, PTL 9, which has been published after the priority date of the present application, has reported a heterocyclic compound that acts on G12D mutant KRAS protein.

Meanwhile, for example, antibody-drug conjugates (ADC) in which a drug molecule having cytotoxicity is connected to an antibody targeting an antigen expressed on cancer cell surface, or immune-stimulating antibody conjugates (ISAC) in which a drug molecule having an immunostimulatory effect is connected thereto are under study as techniques of selectively delivering drugs to cancer cells, and this technique have been used in approved medicaments (Cancer Science, 2016, Vol. 107, No. 7, p. 1039-1046; Clinical Cancer Research, 2005, Vol. 11, p. 843-852; and Cancer Research, 2016, Vol. 76, No. 10, p. 3003-3013). PTL 10 to PTL 13 each describe ADC having a bifunctional compound having an effect that induces the degradation of a target protein as a drug. However, ADC containing a drug having a G12D mutant KRAS protein degradation-inducing effect has not been known so far.

CITATION LIST

PATENT LITERATURE

PTL 1: International Publication No. WO 2022/148421
PTL 2: International Publication No. WO 2022/148422
PTL 3: Chinese Patent Application Publication No. 115785199
PTL 4: International Publication No. WO 2023/077441
PTL 5: International Publication No. WO 2022/173032
PTL 6: International Publication No. WO 2022/228576
PTL 7: International Publication No. WO 2023/280026
PTL 8: International Publication No. WO 2023/171781
PTL 9: International Publication No. WO 2024/019103
PTL 10: International Publication No. WO 2017/201449
PTL 11: International Publication No. WO 2019/140003
PTL 12: International Publication No. WO 2021/195598
PTL 13: International Publication No. WO 2023/056069

SUMMARY OF INVENTION

TECHNICAL PROBLEM

Provided is a pharmaceutical composition, particularly, an antibody-drug conjugate or a salt thereof containing a heterocyclic compound having a G12D mutant KRAS protein degradation-inducing effect. Also provided is a drug-linker conjugate or a salt thereof for use in the antibody-drug conjugate or the salt thereof.

SOLUTION TO PROBLEM

The present inventors have conducted diligent studies on compounds useful as active ingredients for pharmaceutical compositions and consequently completed the present invention by finding that an antibody-drug conjugate having a structure of formula (I) has an excellent G12D mutant KRAS protein degradation-inducing effect, and further finding that a drug-linker conjugate containing a compound having a G12D mutant KRAS protein degradation-inducing effect is useful in the synthesis of the antibody-drug conjugate.

Specifically, the present invention relates to the following [1] to [45].

[1] An antibody-drug conjugate of formula (I) or a salt thereof:

[Chemical Formula 1]

$$Ab-(L^A-D)_n$$

(I)

wherein

Ab is an antibody or an antigen binding fragment thereof,
D is a heterocyclic compound having a G12D mutant KRAS protein degradation-inducing effect,
$L^A$ is a linker for connecting Ab and D, and
n is a number of 1 to 20.

[2] The antibody-drug conjugate or the salt thereof according to [1], wherein D is a heterocyclic compound represented by formula (II),

[Chemical Formula 2]

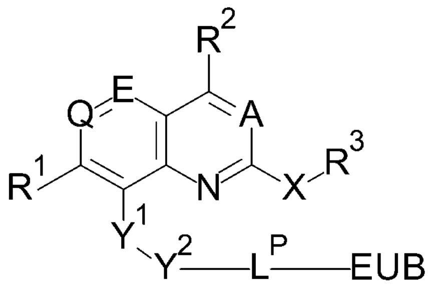

(II)

wherein

A is $CR^A$ or N,
$R^A$ is H, cyano, or optionally substituted $C_{1-3}$ alkyl,
Q is $CR^Q$ or N,
$R^Q$ is H, halogen, $C_{3-6}$ cycloalkyl, vinyl, or optionally substituted $C_{1-3}$ alkyl,
E is CH or N,
$R^1$ is naphthyl optionally substituted by one or two groups selected from the group consisting of cyano, OH, halogen, and optionally substituted $C_{1-3}$ alkyl, or is one group selected from the group consisting of the following formula (III), formula (IV), and formula (V):

[Chemical Formula 3]

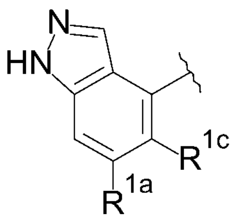

(III)

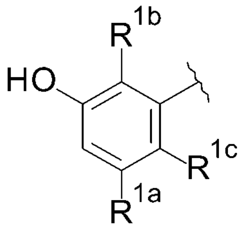

(IV)

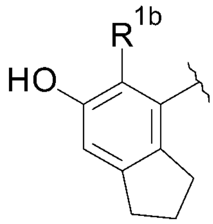

(V)

$R^{1a}$, $R^{1b}$, and $R^{1c}$ are each independently H, vinyl, halogen, or optionally substituted $C_{1-3}$ alkyl,
$R^2$ is $-V^1-V^2$ or W,
$V^1$ is a bond, $-CH_2-$, -O-, -S-, or $-N(R^{V1})-$,
$R^{V1}$ is H or optionally substituted $C_{1-3}$ alkyl,
$V^2$ is one group selected from the group consisting of the following formula (VI) and formula (VII):

[Chemical Formula 4]

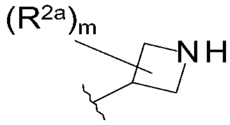

(VI)

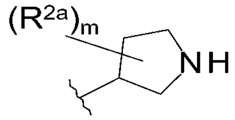

(VII)

W is one group selected from the group consisting of the following formula (VIII), formula (IX), formula (X), formula (XI), formula (XII), formula (XIII), formula (XIV), formula (XV), and formula (XVI):

[Chemical Formula 5]

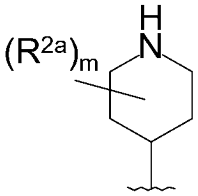
(VIII)

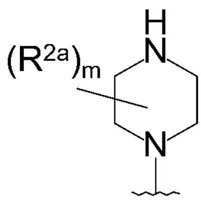
(IX)

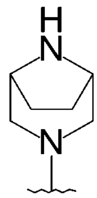
(X)

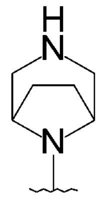
(XI)

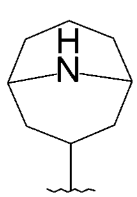
(XII)

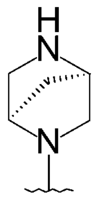
(XIII)

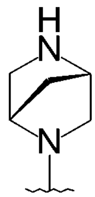
(XIV)

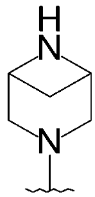
(XV)

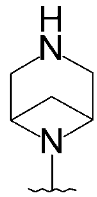
(XVI)

$R^{2a}$ are each independently OH, $OCH_3$, F, or optionally substituted $C_{1-3}$ alkyl, wherein $R^{2a}$ is bonded only to a carbon atom serving as a constituting atom of a ring selected from the group consisting of an azetidine ring represented by formula (VI), a pyrrolidine ring represented by formula (VII), a piperidine ring represented by formula (VIII), and a piperazine ring represented by formula (IX),
m is an integer of 0 to 2,
$R^3$ is optionally substituted $C_{1-6}$ alkyl, optionally substituted hetero cycloalkyl, or optionally substituted heteroaryl,
X is a bond, $-CH_2-$, -O-, -S-, or $-NR^{4X}-$,
$R^{4X}$ is H or optionally substituted $C_{1-3}$ alkyl,
$Y^1$ is -O-(optionally substituted $C_{1-3}$ alkylene)-$^{*Y2}$, -S-(optionally substituted $C_{1-3}$ alkylene)-$^{*Y2}$, $-SO_2$-(optionally substituted $C_{1-3}$ alkylene)-$^{*Y2}$, $-NR^Y$-(optionally substituted $C_{1-3}$ alkylene)-$^{*Y2}$, -(optionally substituted $C_{1-3}$ alkylene)-O-$^{*Y2}$, -(optionally substituted $C_{1-3}$ alkylene)-S-$^{*Y2}$, -(optionally substituted $C_{1-3}$ alkylene)-$SO_2$-$^{*Y2}$, or -(optionally substituted $C_{1-3}$ alkylene)-$NR^Y$-$^{*Y2}$ (wherein $^{*Y2}$ represents a site of connection to $Y^2$),
$R^Y$ is H or optionally substituted $C_{1-3}$ alkyl,
$Y^2$ is a bond, optionally substituted phenylene, or optionally substituted heteroarylene,
$L^P$ is a group that chemically bonds $Y^2$ to EUB,
EUB is a group having the ability to bind to one E3 ubiquitin ligase selected from the group consisting of VHL, celebron, IAP, MDM2, DCAF11, DCAF15, DCAF16, BIRC2, KEAP1, RNF4, RNF114, FEM1B, and AhR, and
$L^A$ is a linker for connecting Ab and D, wherein $L^A$ is bonded to an arbitrary nitrogen atom of -NH- or oxygen atom of -OH contained in D.

[3] The antibody-drug conjugate or the salt thereof according to [2], wherein

$V^2$ is one group selected from the group consisting of the following formula (VIa) and formula (VIIa) (wherein $^{*LA}$ represents a site of connection to $L^A$):

[Chemical Formula 6]

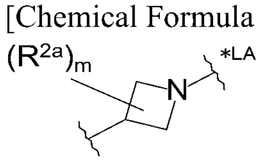
(VIa)

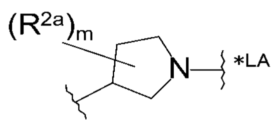
(VIIa)

W is one group selected from the group consisting of the following formula (VIIIa), formula (IXa), formula (Xa), formula (XIa), formula (XIIa), formula (XIIIa), formula (XIVa), formula (XVa), and formula (XVIa) (wherein $^{*LA}$ represents a site of connection to $L^A$):

[Chemical Formula 7]

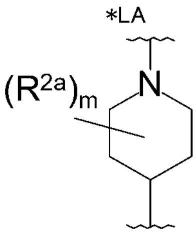 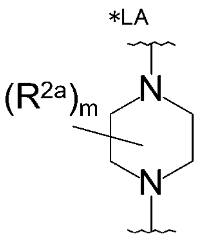 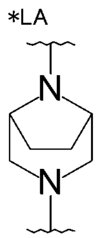 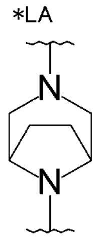

(VIIIa) (IXa) (Xa) (XIa)

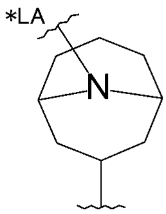 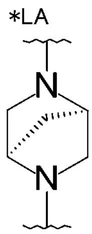 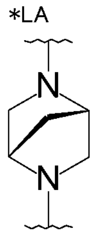 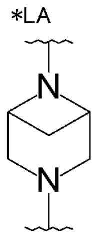 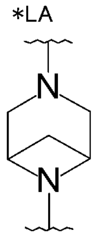

(XIIa) (XIIIa) (XIVa) (XVa) (XVIa)

and

$L^A$ is a linker for connecting Ab and D, wherein $L^A$ is bonded to D through a nitrogen atom contained in $V^2$ or W at the site of connection represented by $^{*LA}$.

[4] The antibody-drug conjugate or the salt thereof according to [2], wherein

A is N,
Q is $CR^Q$, $R^Q$ is cyclopropyl,
E is CH,
$R^1$ is the following formula (III-2):

[Chemical Formula 8]

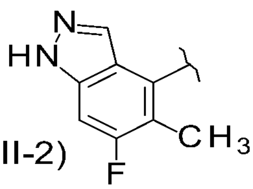

(III-2)

$R^2$ is $-V^1-V^2$ or W,
$V^1$ is $-N(CH_3)-$,
$V^2$ is the following formula (VII-2):

[Chemical Formula 9]

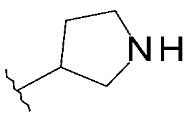

(VII-2)

W is the following formula (XIV):

[Chemical Formula 10]

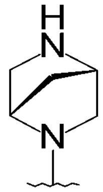

(XIV)

$R^3$ is n-propyl optionally substituted by $-OCH_3$, or tetrahydropyranyl,
X is -O-,
$Y^1$ is -O-(methylene)-$^{*Y2}$ (wherein $^{*Y2}$ represents a site of connection to $Y^2$),
$Y^2$ is phenylene,
$L^P$ is a group that chemically bonds $Y^2$ to EUB, and
EUB is a group having the ability to bind to one E3 ubiquitin ligase selected from the group consisting of VHL and celebron.

[5] The antibody-drug conjugate or the salt thereof according to [4], wherein

$V^2$ is the following formula (VII-2a) (wherein $^{*LA}$ represents a site of connection to $L^A$):

[Chemical Formula 11]

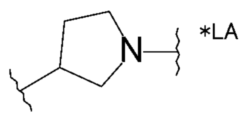

(VII-2a)

W is the following formula (XIVa) (wherein $^{*LA}$ represents a site of connection to $L^A$):

[Chemical Formula 12]

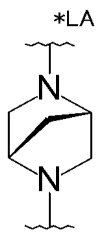

(XIVa)

and
$L^A$ is a linker for connecting Ab and D, wherein $L^A$ is bonded to D through a nitrogen atom contained in $V^2$ or W at the site of connection represented by $^{*LA}$.

[6] The antibody-drug conjugate or the salt thereof according to [1] or [2], wherein D is (4R)-1-[(2S)-2-(4-{4-[({6-cyclopropyl-4-[(1S,4S)-2,5-diazabicyclo[2.2.1]heptan-2-yl]-7-(6-fluoro-5-methyl-1H-indazol-4-yl)-2-[(oxan-4-yl)oxy]quinazolin-8-yl}oxy)methyl]phenyl}-1H-1,2,3-triazol-1-yl)-3-methylbutanoyl]-4-hydroxy-N-{(1R)-2-hydroxy-1-[4-(4-methyl-1,3-thiazol-5-yl)phenyl]ethyl}-L-prolinamide, (4R)-1-[(2S)-2-(4-{4-[({6-cyclopropyl-4-[(1S,4S)-2,5-diazabicyclo[2.2.1]heptan-2-yl]-7-(6-fluoro-5-methyl-1H-indazol-4-yl)-2-[(oxan-4-yl)oxy]quinazolin-8-yl}oxy)methyl]phenyl}-1H-1,2,3-triazol-1-yl)-3-methylbutanoyl]-4-hydroxy-N-{(1R)-2-hydroxy-1-[4-(2-oxo-1,3-oxazolidin-3-yl)phenyl]ethyl}-L-prolinamide, (4R)-1-[(2S)-2-(4-{4-[({6-cyclopropyl-4-[(1S,4S)-2,5-diazabicyclo[2.2.1]heptan-2-yl]-7-(6-fluoro-5-methyl-1H-indazol-4-yl)-2-[(oxan-4-yl)oxy]quinazolin-8-yl}oxy)methyl]phenyl}-1H-1,2,3-triazol-1-yl)-3-methylbutanoyl]-4-hydroxy-N-{(1R)-2-hydroxy-1-[4-(1-methyl-1H-pyrazol-5-yl)phenyl]ethyl}-L-prolinamide, (4R)-1-[(2S)-2-(4-{4-[({6-cyclopropyl-4-[(1S,4S)-2,5-diazabicyclo[2.2.1]heptan-2-yl]-7-(6-fluoro-5-methyl-1H-indazol-4-yl)-2-[(2S)-2-methoxypropoxy]quinazolin-8-yl}oxy)methyl]phenyl}-1H-1,2,3-triazol-1-yl)-3-methylbutanoyl]-4-hydroxy-N-{(1R)-2-hydroxy-1-[4-(4-methyl-1,3-thiazol-5-yl)phenyl]ethyl}-L-prolinamide, (4R)-1-[(2S)-2-(4-{4-[({6-cyclopropyl-4-[(1S,4S)-2,5-diazabicyclo[2.2.1]heptan-2-yl]-7-(6-fluoro-5-methyl-1H-indazol-4-yl)-2-[(2S)-2-methoxypropoxy]quinazolin-8-yl}oxy)methyl]phenyl}-1H-1,2,3-triazol-1-yl)-3-methylbutanoyl]-N-{(1R)-1-[4-(1-ethyl-1H-pyrazol-5-yl)phenyl]-2-hydroxyethyl}-4-hydroxy-L-prolinamide, (4R)-1-[(2S)-2-(4-{4-[({6-cyclopropyl-4-[(1S,4S)-2,5-diazabicyclo[2.2.1]heptan-2-yl]-7-(6-fluoro-5-methyl-1H-indazol-4-yl)-2-[(2S)-2-methoxypropoxy]quinazolin-8-yl}oxy)methyl]phenyl}-1H-1,2,3-triazol-1-yl)-3-methylbutanoyl]-4-hydroxy-N-{(1R)-2-hydroxy-1-[4-(4-methyl-1,3-oxazol-5-yl)phenyl]ethyl}-L-prolinamide, (4R)-1-[(2S)-2-{4-[4-({[(7M)-6-cyclopropyl-7-(6-fluoro-5-methyl-1H-indazol-4-yl)-2-[(2S)-2-methoxypropoxy]-4-{methyl[(3S)-pyrrolidin-3-yl]amino}quinazolin-8-yl]oxy}methyl)phenyl]-1H-1,2,3-triazol-1-yl}-3-methylbutanoyl]-4-hydroxy-N-{(1R)-2-hydroxy-1-[4-(4-methyl-1,3-thiazol-5-yl)phenyl]ethyl}-L-prolinamide, (4R)-1-[(2S)-2-{4-[4-({[(7M)-6-cyclopropyl-7-(6-fluoro-5-methyl-1H-indazol-4-yl)-2-[(2S)-2-methoxypropoxy]-4-{methyl[(3S)-pyrrolidin-3-yl]amino}quinazolin-8-yl]oxy}methyl)phenyl]-1H-1,2,3-triazol-1-yl}-3-methylbutanoyl]-N-{(1R)-1-[4-(1-ethyl-1H-pyrazol-5-yl)phenyl]-2-hydroxyethyl}-4-hydroxy-L-prolinamide, 3-(5-{[(3S)-4-{4-[({(7M)-6-cyclopropyl-4-[(1S,4S)-2,5-diazabicyclo[2.2.1]heptan-2-yl]-7-(6-fluoro-5-methyl-1H-indazol-4-yl)-2-[(oxan-4-yl)oxy]quinazolin-8-yl}oxy)methyl]benzoyl}-3-methylpiperazin-1-yl]methyl}-3-methyl-2-oxo-2,3-dihydro-1H-benzimidazol-1-yl)piperidine-2,6-dione, 1-(6-{[(3S)-4-{4-[({(7M)-6-cyclopropyl-4-[(1S,4S)-2,5-diazabicyclo[2.2.1]heptan-2-yl]-7-(6-fluoro-5-methyl-1H-indazol-4-yl)-2-[(oxan-4-yl)oxy]quinazolin-8-yl}oxy)methyl]benzoyl}-3-methylpiperazin-1-yl]methyl}-1-methyl-1H-indazol-3-yl)-1,3-diazinane-2,4-dione, or 3-(5-{[(3S)-4-{4-[({(7M)-6-cyclopropyl-4-[(1S,4S)-2,5-diazabicyclo[2.2.1]heptan-2-yl]-7-(6-fluoro-5-methyl-1H-indazol-4-yl)-2-[(2S)-2-methoxypropoxy]quinazolin-8-yl}oxy)methyl]benzoyl}-3-methylpiperazin-1-yl]methyl}-3-methyl-2-oxo-2,3-dihydro-1H-benzimidazol-1-yl)piperidine-2,6-dione, or (2R)-2-({(4R)-1-[(2S)-2-{4-[4-({[(7M)-6-cyclopropyl-7-(6-fluoro-5-methyl-1H-indazol-4-yl)-2-[(2S)-2-methoxypropoxy]-4-{methyl[(3S)-pyrrolidin-3-yl]amino}quinazolin-8-yl]oxy}methyl)phenyl]-1H-1,2,3-triazol-1-yl}-3-methylbutanoyl]-4-hydroxy-L-prolyl}amino)-2-[4-(1H-1,2,4-triazol-1-yl)phenyl]ethyl dihydrogen phosphate.

[7] The antibody-drug conjugate or the salt thereof according to any one of [1] to [6], wherein

$L^A$ is a linker represented by formula (XXVIII):

[Chemical Formula 13]

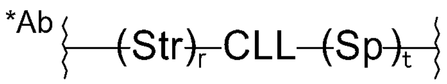

(XXVIII)

wherein
Str is a stretcher unit and is bonded to Ab and CLL, r is 0 or 1,
CLL is a partial structure cleavable *in vivo*,
Sp is a spacer unit and is bonded to CLL and D, t is 0 or 1, and
$*^{Ab}$ represents a site of connection to Ab.

[8] The antibody-drug conjugate or the salt thereof according to [7], wherein Str is a stretcher unit represented by formula (ST-1), and r is 1:

[Chemical Formula 14]

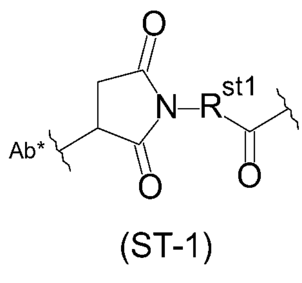

(ST-1)

wherein $R^{st1}$ is optionally substituted $C_{1-12}$ alkylene, $-(CH_2CH_2O)_{a1}-C_{1-6}$ alkylene-, $-C_{1-6}$ alkylene-$(OCH_2CH_2)_{a2}$-, -optionally substituted $C_{1-6}$ alkylene-NH-C(=O)-optionally substituted $C_{1-6}$ alkylene-, -optionally substituted $C_{1-6}$ alkylene-C(=O)-NH-optionally substituted $C_{1-6}$ alkylene-, optionally substituted $C_{1-6}$ alkylene-C(=O)-NH-$(CH_2CH_2O)_{a3}-C_{1-6}$ alkylene-, or optionally substituted $C_{1-6}$ alkylene-NH-C(=O)-$(CH_2CH_2O)_{a4}-C_{1-6}$ alkylene-, each of a1, a2, a3, and a4 is an integer of 1 to 10, and

$*^{Ab}$ represents a site of connection to Ab.

[9] The antibody-drug conjugate or the salt thereof according to [8], wherein $R^{st1}$ is $C_5$ alkylene.

[10] The antibody-drug conjugate or the salt thereof according to [7], wherein CLL is a partial structure cleavable *in vivo* represented by formula (CL-1):

[Chemical Formula 15]

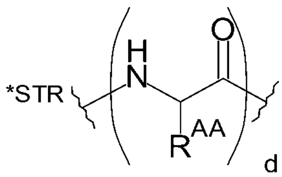

(CL-1)

wherein $R^{AA}$ are each independently H, methyl, isopropyl, isobutyl, sec-butyl, benzyl, p-hydroxybenzyl, hydroxymethyl, 1-hydroxyethyl, $-CH_2-C(=O)-OH$, $-(CH_2)_2-C(=O)-OH$, $-CH_2-C(=O)-NH_2$, $-(CH_2)_2-C(=O)-NH_2$, $-(CH_2)_4-NH_2$, $-(CH_2)_3-NH-C(=NH)-NH_2$, $-(CH_2)_3-NH-C(=O)-NH_2$, $-CH_2-SH$, or $-CH_2-S-CH_3$, or one group selected from the group consisting of the following formula (RAA-1) and (RAA-2):

[Chemical Formula 16]

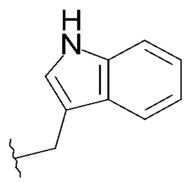

(RAA-1)

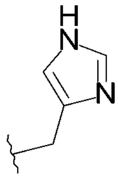

(RAA-2)

d is an integer of 1 to 6, and $*^{STR}$ represents a site of connection to Str.

[10a] The antibody-drug conjugate or the salt thereof according to [10], wherein $R^{AA}$ are each independently H, methyl, isopropyl, benzyl, $-(CH_2)_4-NH_2$, $-(CH_2)_3-NH-C(=NH)-NH_2$, or $-(CH_2)_3-NH-C(=O)-NH_2$, and d is an integer of 2 to 4.

[10b] The antibody-drug conjugate or the salt thereof according to [10], wherein $R^{AA}$ are each independently methyl, isopropyl, benzyl, $-(CH_2)_4-NH_2$, $-(CH_2)_3-NH-C(=NH)-NH_2$, or $-(CH_2)_3-NH-C(=O)-NH_2$, and d is 2.

[10c] The antibody-drug conjugate or the salt thereof according to [10], wherein $R^{AA}$ are each independently methyl, isopropyl, or $-(CH_2)_3-NH-C(=O)-NH_2$, and d is 2.

[11] The antibody-drug conjugate or the salt thereof according to [10], wherein $R^{AA}$ are each independently methyl or isopropyl, and d is 2.

[12] The antibody-drug conjugate or the salt thereof according to [10], wherein $R^{AA}$ are each independently isopropyl or $-(CH_2)_3-NH-C(=O)-NH_2$, and d is 2.

[13] The antibody-drug conjugate or the salt thereof according to [10], wherein $R^{AA}$ are each independently H or benzyl, and d is 4.

[14] The antibody-drug conjugate or the salt thereof according to [7], wherein Sp is a spacer unit represented by formula (SP-1), and t is 1:

[Chemical Formula 17]

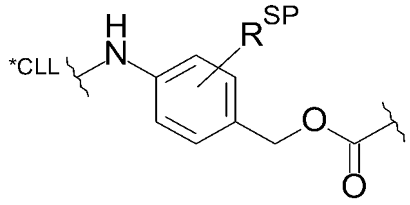

(SP-1)

wherein $R^{SP}$ is H, $C_{1-6}$ alkyl, -O-$C_{1-6}$ alkyl, halogen, or halogeno $C_{1-6}$ alkyl, and $^{*CLL}$ represents a site of connection to CLL.

[15] The antibody-drug conjugate or the salt thereof according to [14], wherein $R^{SP}$ is H.

[16] The antibody-drug conjugate or the salt thereof according to [7], wherein t is 0.

[17] The antibody-drug conjugate or the salt thereof according to [1], wherein formula (I) is represented by formula (AD-1a) or (AD-2a):

[Chemical Formula 18]

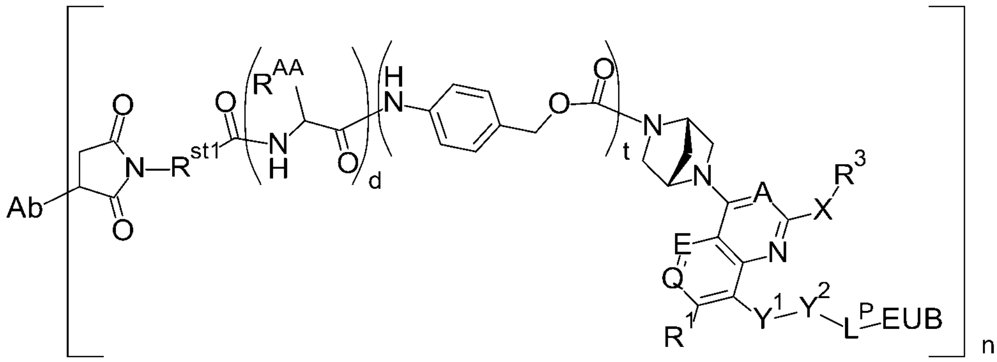

(AD-1a)

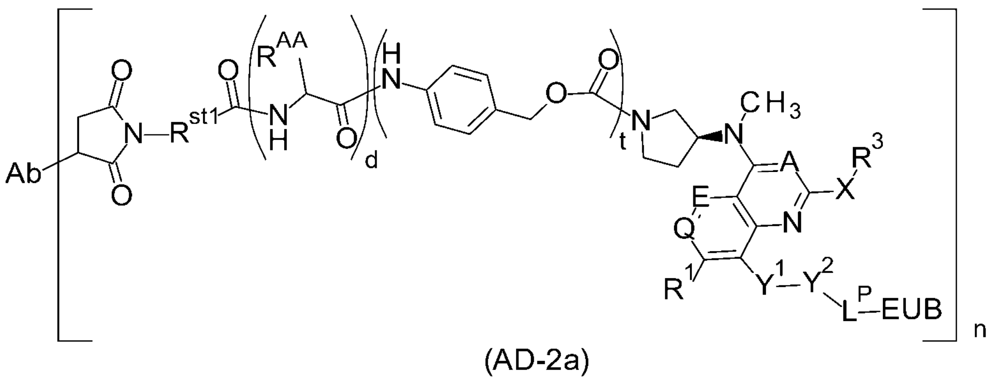

(AD-2a)

wherein

Ab is an antibody or an antigen binding fragment thereof,
A is $CR^A$ or N,
$R^A$ is H, cyano, or optionally substituted $C_{1-3}$ alkyl,
Q is $CR^Q$ or N,
$R^Q$ is H, halogen, $C_{3-6}$ cycloalkyl, vinyl, or optionally substituted $C_{1-3}$ alkyl,
E is CH or N,
$R^1$ is naphthyl optionally substituted by one or two groups selected from the group consisting of cyano, OH, halogen, and optionally substituted $C_{1-3}$ alkyl, or is one group selected from the group consisting of the following formula (III), formula (IV), and formula (V):

[Chemical Formula 19]

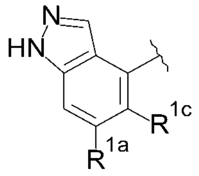
(III)

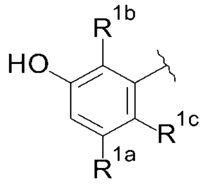
(IV)

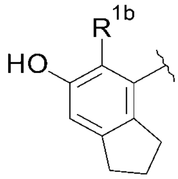
(V)

$R^{1a}$, $R^{1b}$, and $R^{1c}$ are each independently H, vinyl, halogen, or optionally substituted $C_{1-3}$ alkyl,
$R^3$ is optionally substituted $C_{1-6}$ alkyl, optionally substituted hetero cycloalkyl, or optionally substituted heteroaryl,
X is a bond, $-CH_2-$, -O-, -S-, or $-NR^{4X}-$,
$R^{4X}$ is H or optionally substituted $C_{1-3}$ alkyl,
$Y^1$ is -O-(optionally substituted $C_{1-3}$ alkylene)-$^{*Y2}$, -S-(optionally substituted $C_{1-3}$ alkylene)-$^{*Y2}$, $-SO_2$-(optionally substituted $C_{1-3}$ alkylene)-$^{*Y2}$, $-NR^Y$-(optionally substituted $C_{1-3}$ alkylene)-$^{*Y2}$, -(optionally substituted $C_{1-3}$ alkylene)-O-$^{*Y2}$, -(optionally substituted $C_{1-3}$ alkylene)-S-$^{*Y2}$, -(optionally substituted $C_{1-3}$ alkylene)-$SO_2$-$^{*Y2}$, or -(optionally substituted $C_{1-3}$ alkylene)-$NR^Y$-$^{*Y2}$ (wherein $^{*Y2}$ represents a site of connection to $Y^2$),
$R^Y$ is H or optionally substituted $C_{1-3}$ alkyl,
$Y^2$ is a bond, optionally substituted phenylene, or optionally substituted heteroarylene,
$L^P$ is a group that chemically bonds $Y^2$ to EUB,
EUB is a group having the ability to bind to one E3 ubiquitin ligase selected from the group consisting of VHL, celebron, IAP, MDM2, DCAF11, DCAF15, DCAF16, BIRC2, KEAP1, RNF4, RNF114, FEM1B, and AhR,
$R^{st1}$ is $C_{1-12}$ alkylene,
$R^{AA}$ are each independently H, methyl, isopropyl, benzyl, or $-(CH_2)_3-NH-C(=O)-NH_2$, d is an integer of 2 to 4,
t is 0 or 1, and
n is a number of 1 to 20.

[18] The antibody-drug conjugate or the salt thereof according to [1], wherein formula (I) is represented by formula (AD-3a), (AD-4a), (AD-5a), (AD-6a), (AD-7a), (AD-8a), (AD-9a), (AD-10a), (AD-11a), (AD-12a), (AD-13a), or (AD-25a):

[Chemical Formula 20-1]

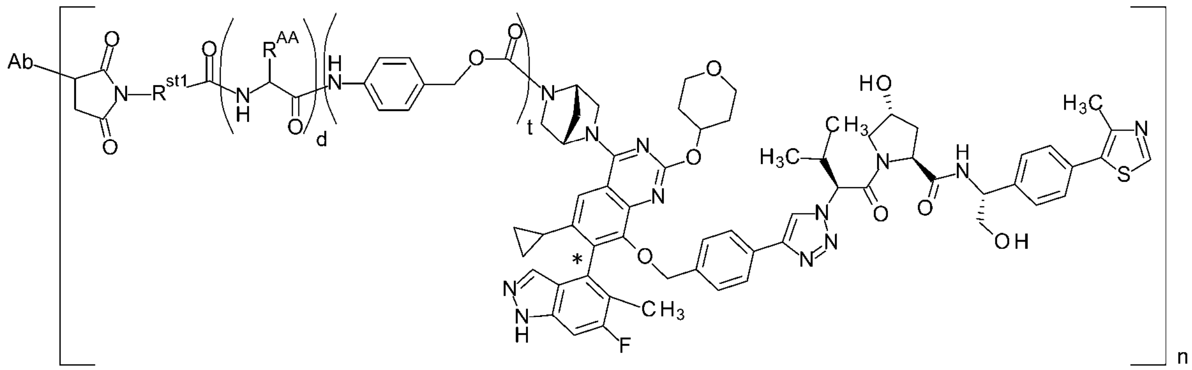

(AD-3a)

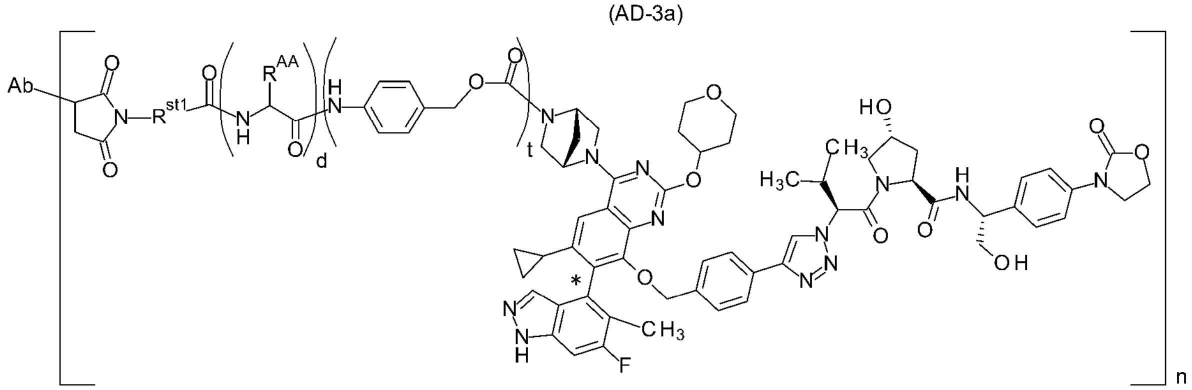

(AD-4a)

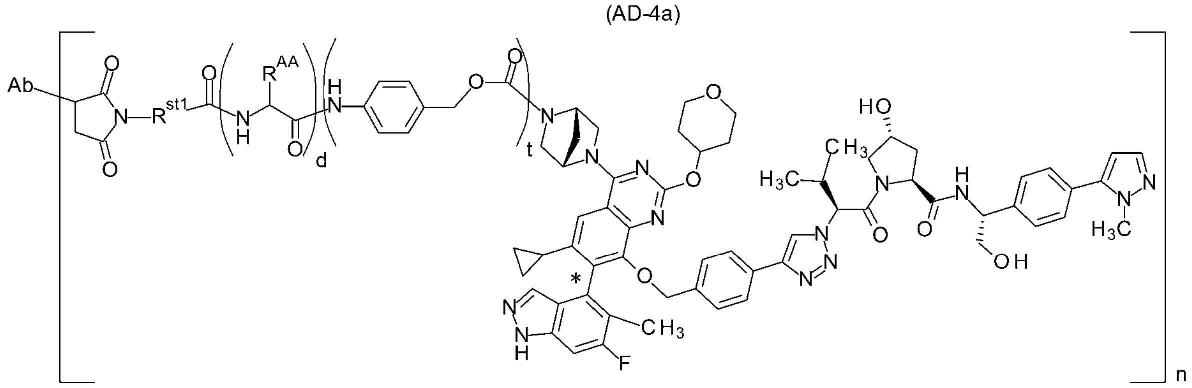

(AD-5a)

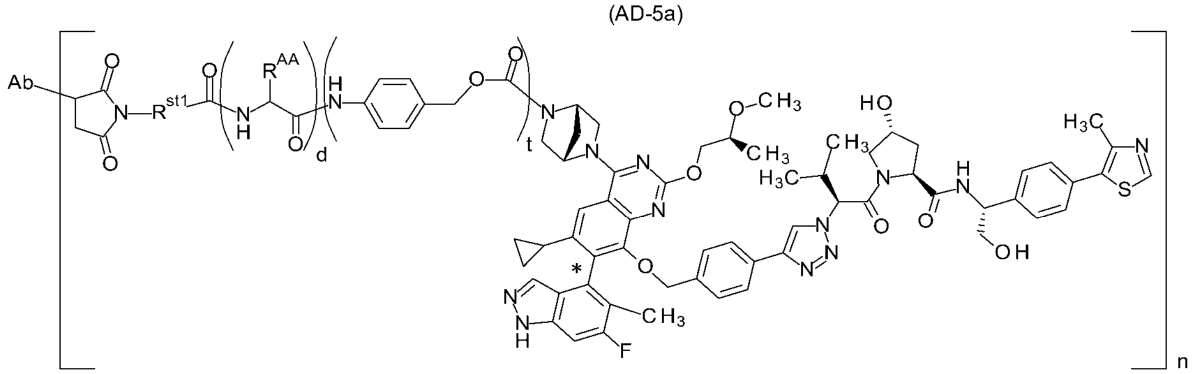

(AD-6a)

[Chemical Formula 20-2]

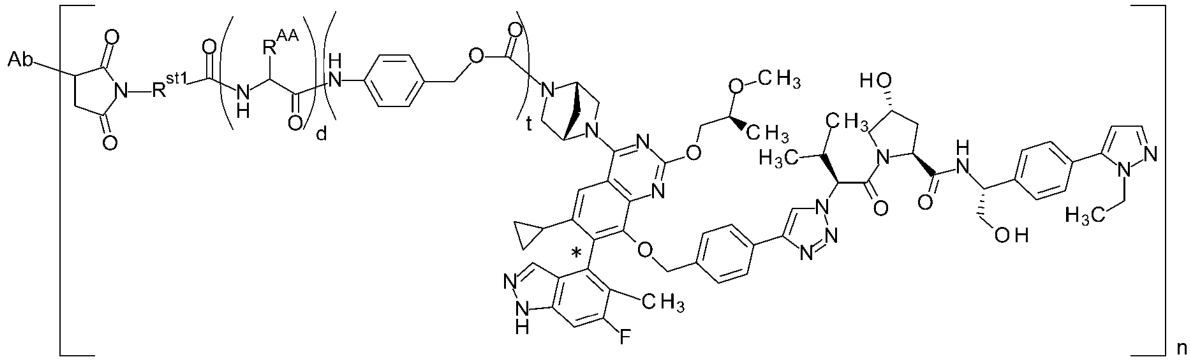

(AD-7a)

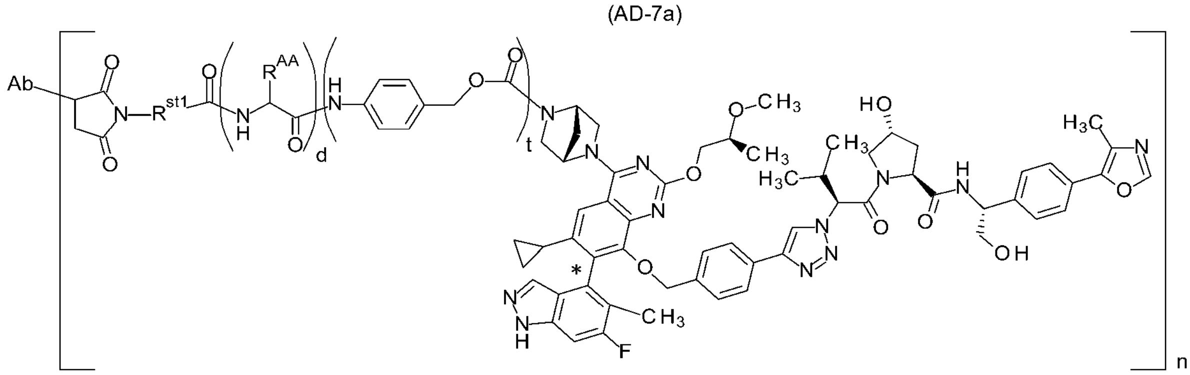

(AD-8a)

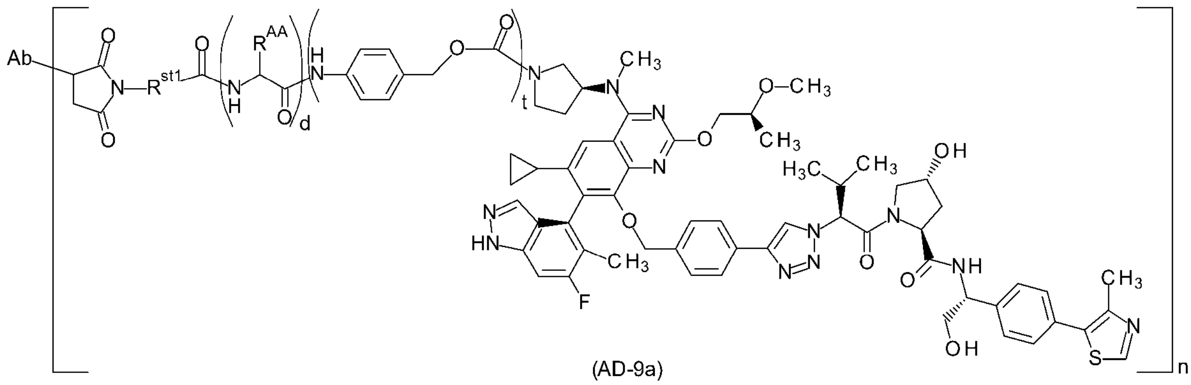

(AD-9a)

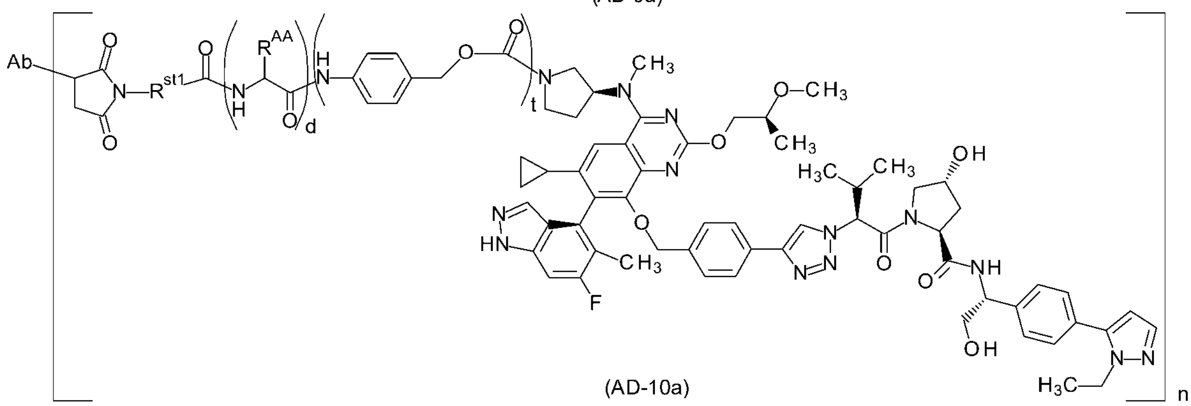

(AD-10a)

[Chemical Formula 20-3]

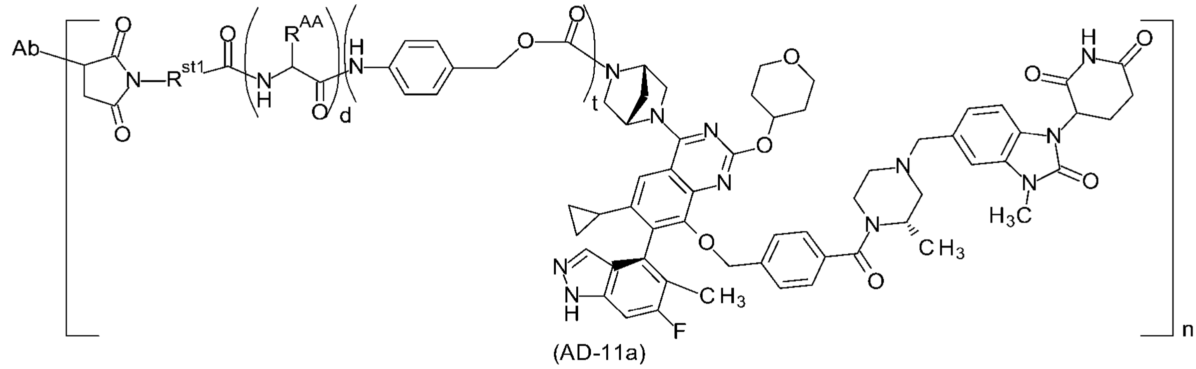

(AD-11a)

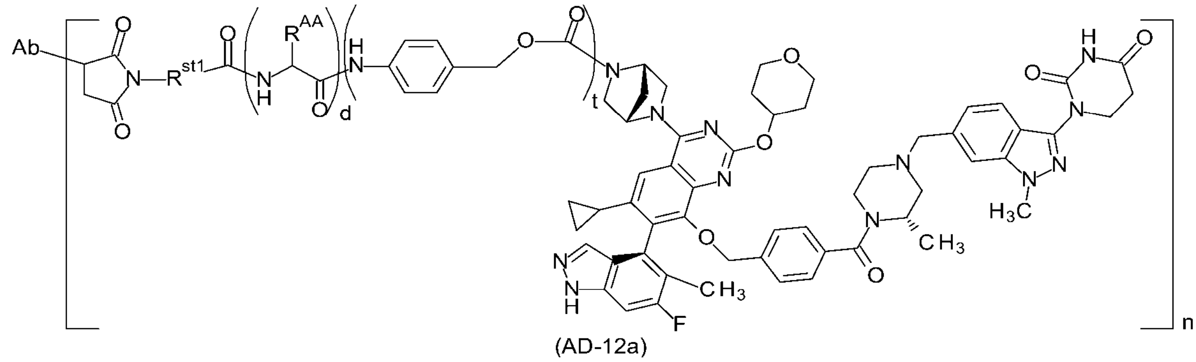

(AD-12a)

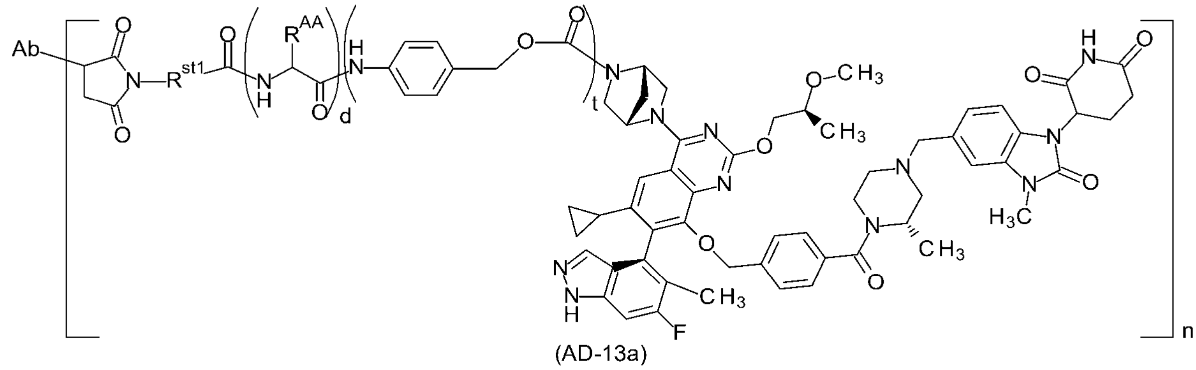

(AD-13a)

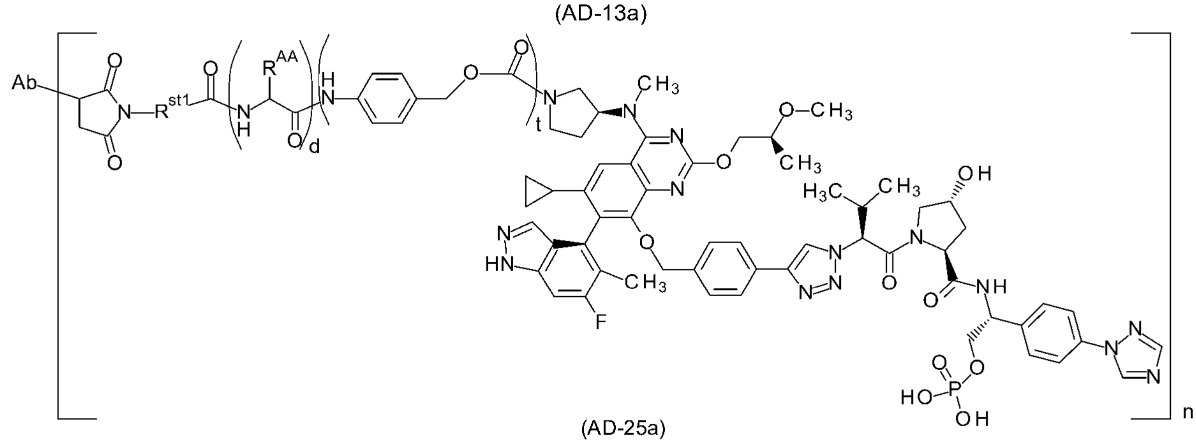

(AD-25a)

wherein $R^{st1}$ is $C_{1-12}$ alkylene, $R^{AA}$ are each independently H, methyl, isopropyl, benzyl, or -$(CH_2)_3$-NH-C(=O)-$NH_2$, d is an integer of 2 to 4, t is 0 or 1, and n is a number of 1 to 20 (a compound with "*" in the chemical structural formulas represents that the compound has single axial chirality or chiral center; the same holds true for the description below).

[19] The antibody-drug conjugate or the salt thereof according to [1], wherein formula (I) is represented by formula (AD-14), (AD-15), (AD-16), (AD-17), (AD-18), (AD-19), (AD-20), (AD-21), (AD-22), (AD-23), (AD-24), or (AD-25):

[Chemical Formula 21-1]

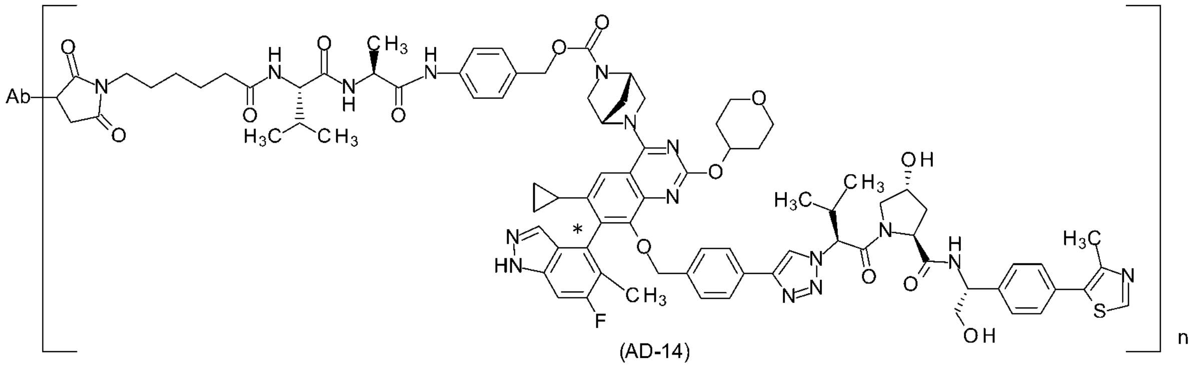

(AD-14)

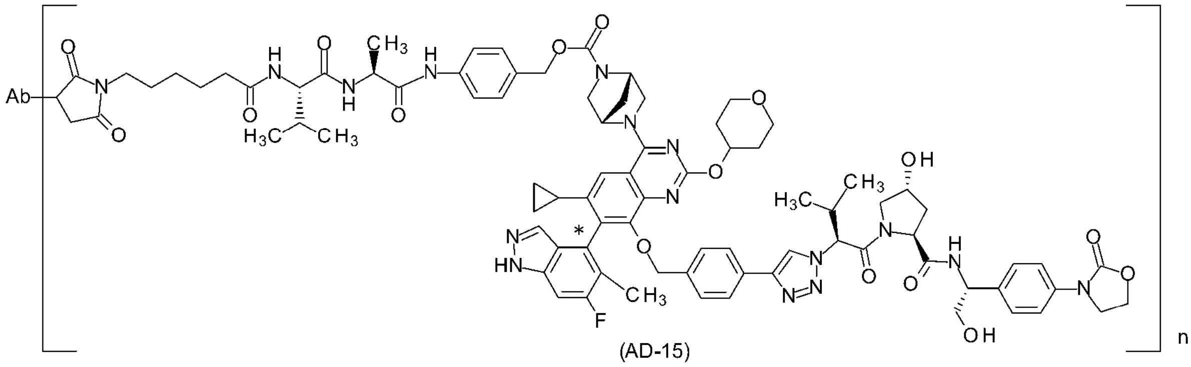

(AD-15)

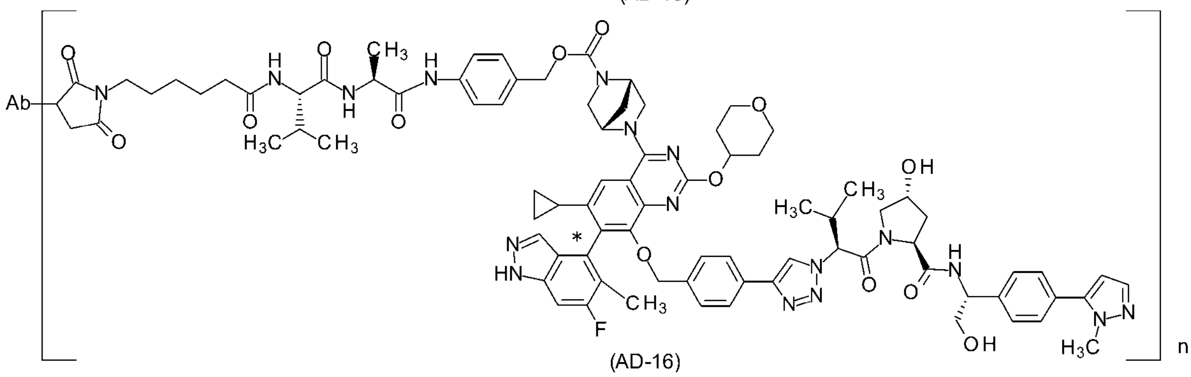

(AD-16)

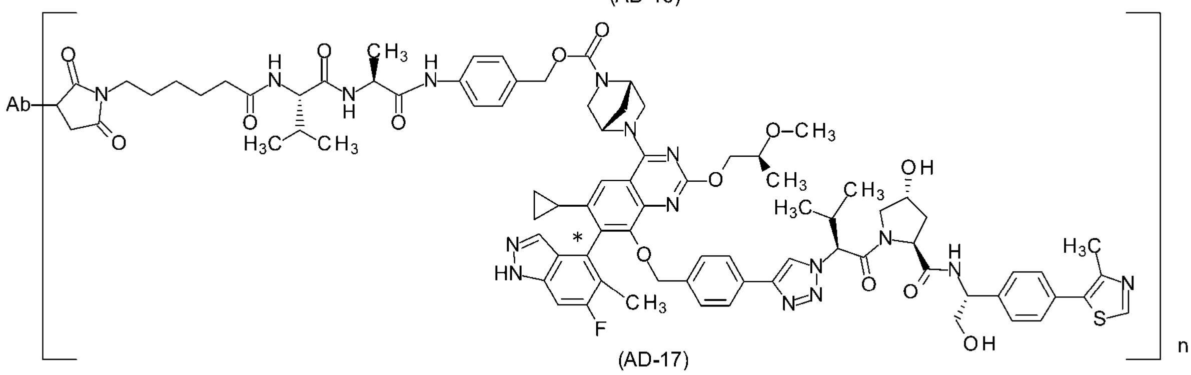

(AD-17)

[Chemical Formula 21-2]

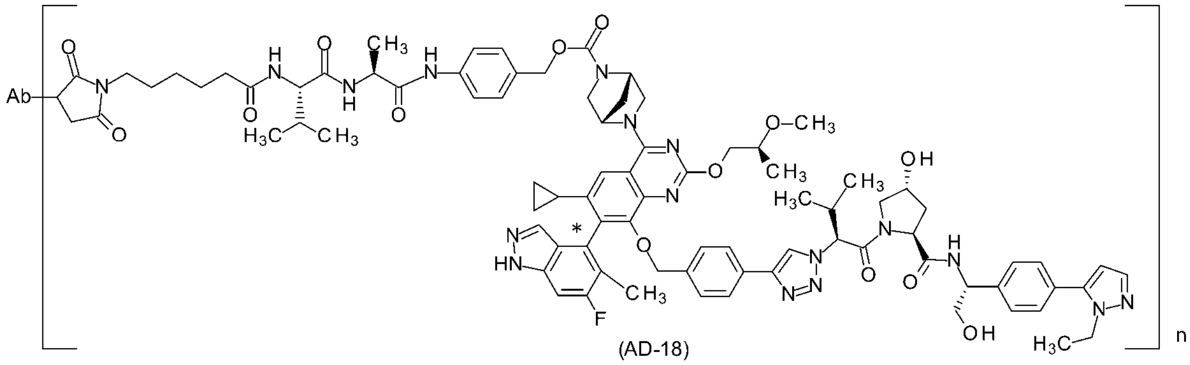

(AD-18)

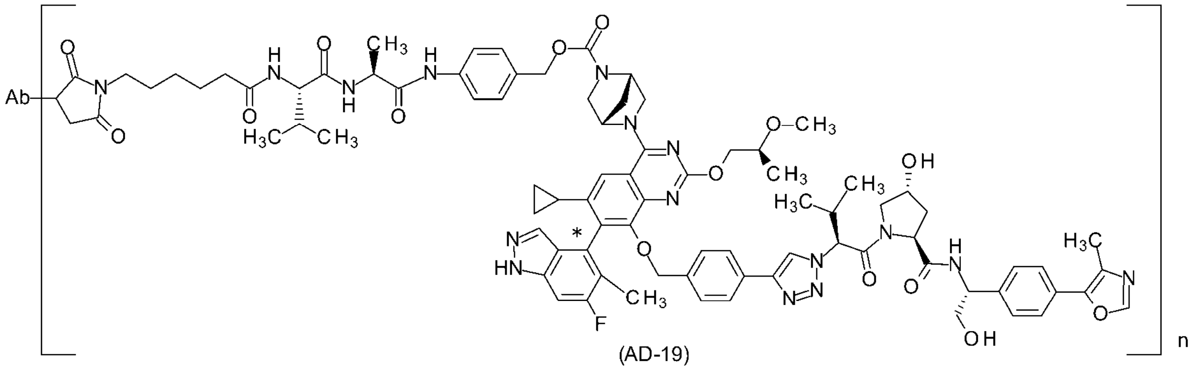

(AD-19)

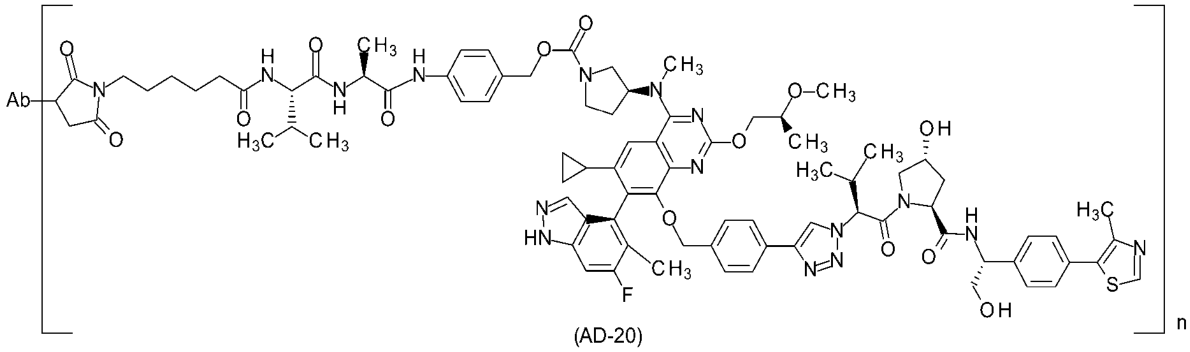

(AD-20)

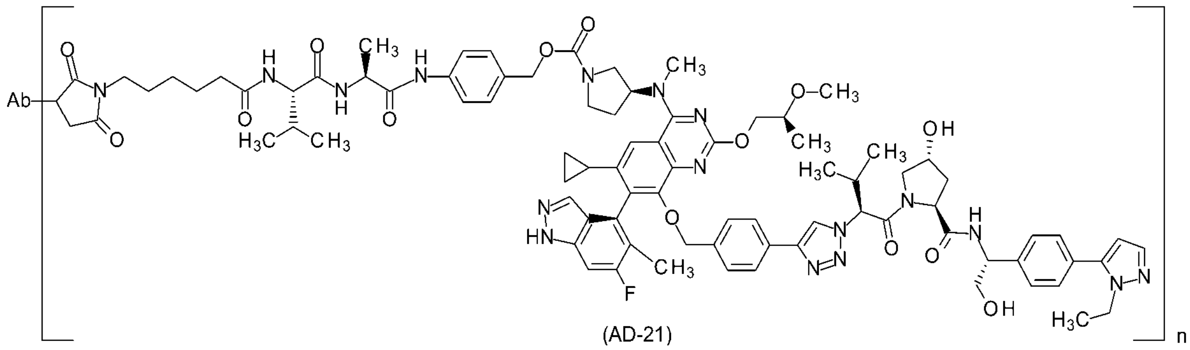

(AD-21)

[Chemical Formula 21-3]

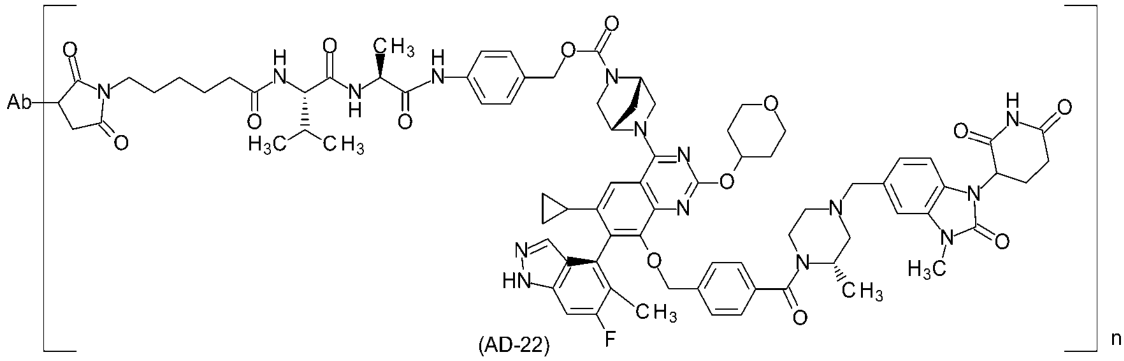

(AD-22)

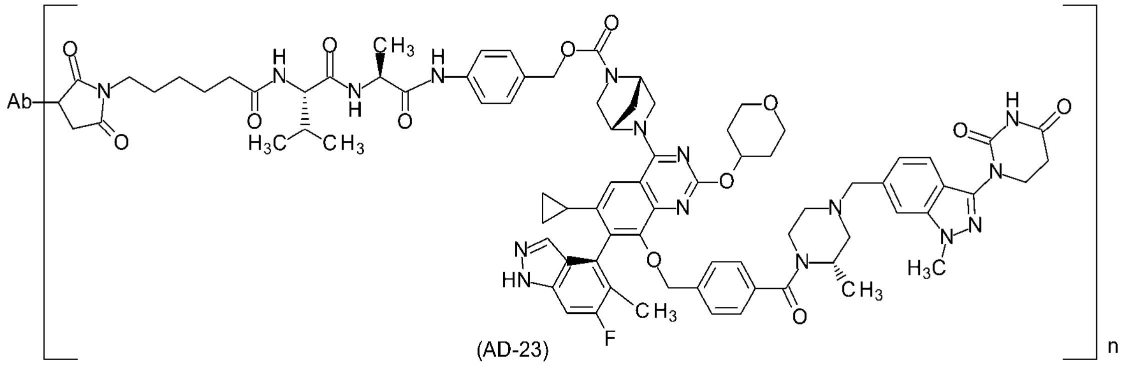

(AD-23)

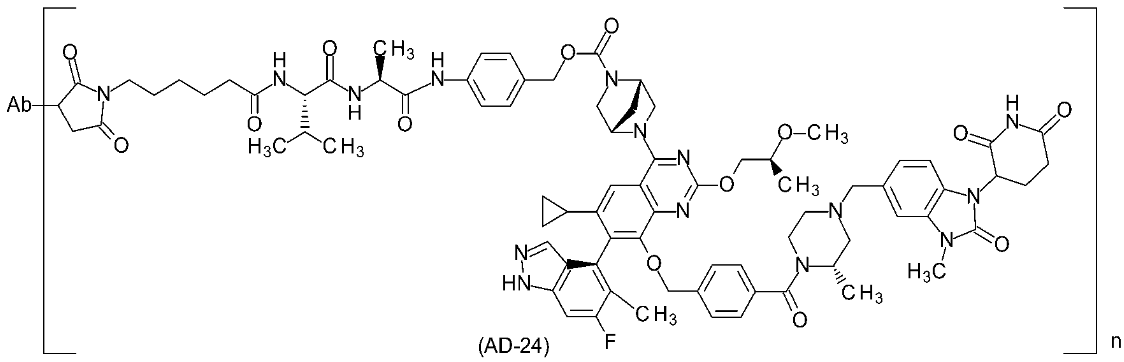

(AD-24)

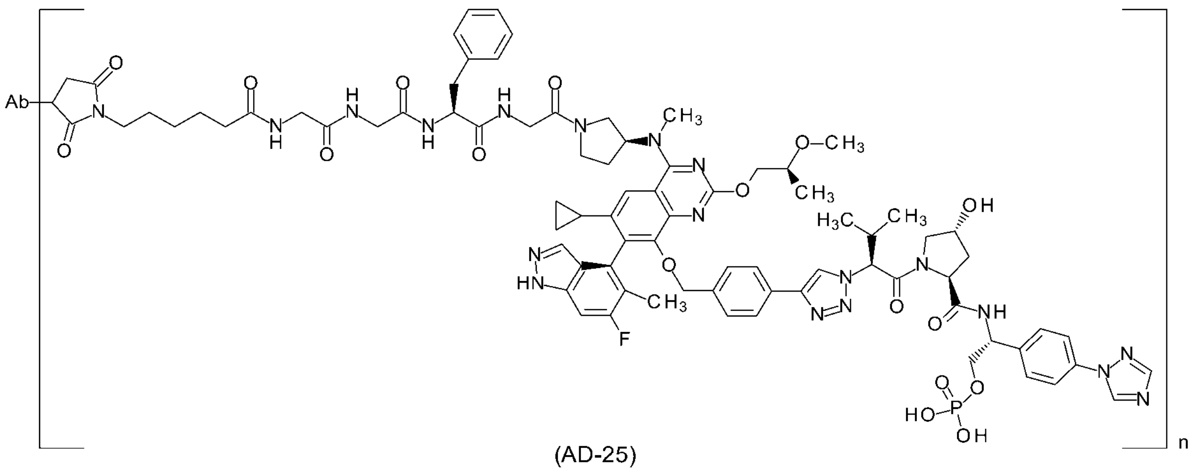

(AD-25)

wherein n is a number of 1 to 20.

[20] The antibody-drug conjugate or the salt thereof according to any one of [1] to [19], wherein Ab is cetuximab, and n is a number of 2 to 5.

[21] The antibody-drug conjugate or the salt thereof according to any one of [1] to [19], wherein Ab is an antibody or an antigen binding fragment that binds to one or two or more antigens selected from the group consisting of 5T4, ADAM9, ALPP, ALPPL2, AXL, B7H3, B7H4, BCMA, CA9, CCR2, CCR7, CD123, CD166, CD19, CD20, CD22, CD25, CD30,

CD33, CD37, CD38, CD45, CD46, CD70, CD74, CD79b, CDH3, CDH6, CLDN1, CLDN4, CLDN6, CLDN18.2, cMET, EGFR, EphA3, FAP, FGFR3, Fibronectin, FOLRa, Globo H, GPRC5D, HER2, HER3, IGF1R, Integrin αV, KAAG1, LIV1, MSLN, MT1-MMP, MUC1, MUC4, NaPi2b, Nectin4, PD-L1, PSMA, PTK7, ROR1, ROR2, SEZ6, SialylTn, TF, TROP2, TSPAN8, and VEGF.

[22] The antibody-drug conjugate or the salt thereof according to any one of [1] to [19], wherein Ab is an antibody or an antigen binding fragment that binds to one or two or more antigens selected from the group consisting of EGFR, HER2, cMET, and TROP2.

[23] The antibody-drug conjugate or the salt thereof according to any one of [1] to [19], wherein Ab is an anti-EGFR antibody or an antigen binding fragment thereof.

[24] The antibody-drug conjugate or the salt thereof according to [23], wherein Ab is an anti-EGFR antibody including a heavy chain variable region and a light chain variable region described in the following (1) or (2) or an antigen binding fragment thereof:

(1) a heavy chain variable region including CDR1 consisting of an amino acid sequence from amino acid positions 31 to 35 of SEQ ID NO: 1, CDR2 consisting of an amino acid sequence from amino acid positions 50 to 65 of SEQ ID NO: 1, and CDR3 consisting of an amino acid sequence from amino acid positions 98 to 108 of SEQ ID NO: 1, and
a light chain variable region including CDR1 consisting of an amino acid sequence from amino acid positions 24 to 34 of SEQ ID NO: 2, CDR2 consisting of an amino acid sequence from amino acid positions 50 to 56 of SEQ ID NO: 2, and CDR3 consisting of an amino acid sequence from amino acid positions 89 to 97 of SEQ ID NO: 2; or
(2) a heavy chain variable region including CDR1 consisting of an amino acid sequence from amino acid positions 31 to 35 of SEQ ID NO: 3, CDR2 consisting of an amino acid sequence from amino acid positions 50 to 65 of SEQ ID NO: 3, and CDR3 consisting of an amino acid sequence from amino acid positions 98 to 108 of SEQ ID NO: 3, and
a light chain variable region including CDR1 consisting of an amino acid sequence from amino acid positions 24 to 34 of SEQ ID NO: 4, CDR2 consisting of an amino acid sequence from amino acid positions 50 to 56 of SEQ ID NO: 4, and CDR3 consisting of an amino acid sequence from amino acid positions 89 to 97 of SEQ ID NO: 4.

[25] The antibody-drug conjugate or the salt thereof according to [23], wherein Ab is an anti-EGFR antibody including a heavy chain variable region and a light chain variable region selected from the group consisting of the following (1) to (3) or an antigen binding fragment thereof:

(1) a heavy chain variable region consisting of an amino acid sequence from amino acid positions 1 to 119 of SEQ ID NO: 1 and a light chain variable region consisting of an amino acid sequence from amino acid positions 1 to 107 of SEQ ID NO: 2;
(2) a heavy chain variable region consisting of an amino acid sequence from amino acid positions 1 to 119 of SEQ ID NO: 3 and a light chain variable region consisting of an amino acid sequence from amino acid positions 1 to 107 of SEQ ID NO: 4; and
(3) a heavy chain variable region and a light chain variable region having at least 90% or higher identity to the heavy chain variable region and the light chain variable region described in (1) or (2) above.

[26] The antibody-drug conjugate or the salt thereof according to [23], wherein Ab is an IgG1 or IgG4 type anti-EGFR antibody.

[27] The antibody-drug conjugate or the salt thereof according to [23], wherein Ab is an anti-EGFR antibody consisting of a heavy chain of SEQ ID NO: 1 and a light chain of SEQ ID NO: 2.

[28] The antibody-drug conjugate or the salt thereof according to any one of [21] to [23], wherein Ab is a post-translationally modified antibody, or an antibody that an arbitrary amino acid residue is substituted with cysteine or a non-natural amino acid.

[29] A pharmaceutical composition including an antibody-drug conjugate or a salt thereof according to any one of [1] to [28] and a pharmaceutically acceptable excipient.

[30] The pharmaceutical composition according to [29] for use in the treatment of a cancer.

[31] The pharmaceutical composition according to [30], wherein the cancer is blood cancer or solid cancer.

[32] The pharmaceutical composition according to [30], wherein the cancer is a cancer expressing G12D mutant KRAS.

[33] The antibody-drug conjugate or the salt thereof according to any one of [1] to [28] for use in the treatment of a cancer.

[34] A method for treating a cancer, including the step of administering a therapeutically effective amount of an antibody-drug conjugate or a salt thereof according to any one of [1] to [28] to a subject.

[35] Use of an antibody-drug conjugate or a salt thereof according to any one of [1] to [28] in the production of a pharmaceutical composition for the treatment of a cancer.
[36] A drug-linker conjugate represented by formula (LD-1) or a salt thereof:

[Chemical Formula 22]

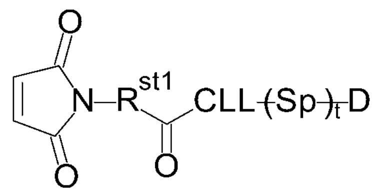

(LD-1)

wherein

D is a heterocyclic compound having a G12D mutant KRAS protein degradation-inducing effect,
$R^{st1}$ is $C_{1-12}$ alkylene,
CLL is a partial structure cleavable *in vivo*,
Sp is a spacer unit and is bonded to CLL and D, and t is 0 or 1.

[37] The drug-linker conjugate or the salt thereof according to [36], wherein

D is a heterocyclic compound represented by formula (II):

[Chemical Formula 23]

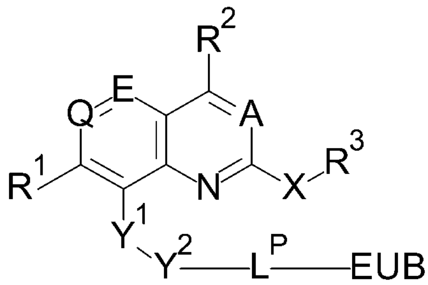

(II)

wherein
A is $CR^A$ or N,
$R^A$ is H, cyano, or optionally substituted $C_{1-3}$ alkyl,
Q is $CR^Q$ or N,
$R^Q$ is H, halogen, $C_{3-6}$ cycloalkyl, vinyl, or optionally substituted $C_{1-3}$ alkyl,
E is CH or N,
$R^1$ is naphthyl optionally substituted by one or two groups selected from the group consisting of cyano, OH, halogen, and optionally substituted $C_{1-3}$ alkyl, or is one group selected from the group consisting of the following formula (III), formula (IV), and formula (V):

[Chemical Formula 24]

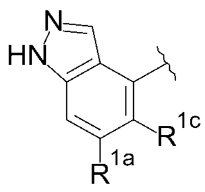

(III)

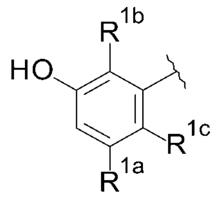

(IV)

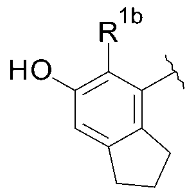

(V)

$R^{1a}$, $R^{1b}$, and $R^{1c}$ are each independently H, vinyl, halogen, or optionally substituted $C_{1-3}$ alkyl,
$R^2$ is $-V^1-V^2$ or W,
$V^1$ is a bond, $-CH_2-$, -O-, -S-, or $-N(R^{V1})-$,
$R^{V1}$ is H or optionally substituted $C_{1-3}$ alkyl,
$V^2$ is one group selected from the group consisting of the following formula (VI) and formula (VII):

[Chemical Formula 25]

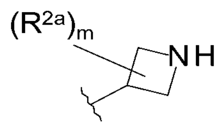

(VI)

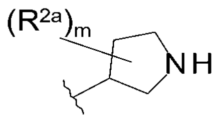

(VII)

W is one group selected from the group consisting of the following formula (VIII), formula (IX), formula (X), formula (XI), formula (XII), formula (XIII), formula (XIV), formula (XV) and formula (XVI):

[Chemical Formula 26]

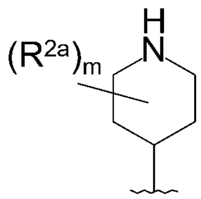

(VIII)

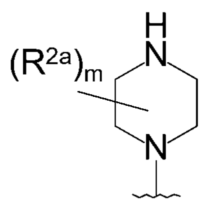

(IX)

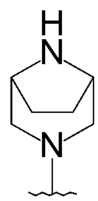

(X)

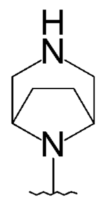

(XI)

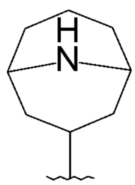

(XII)

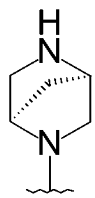

(XIII)

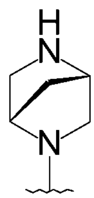

(XIV)

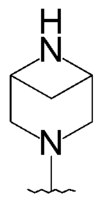

(XV)

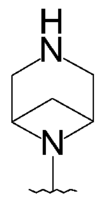

(XVI)

$R^{2a}$ are each independently OH, $OCH_3$, F, or optionally substituted $C_{1-3}$ alkyl, wherein $R^{2a}$ is bonded only to a carbon atom serving as a constituting atom of a ring selected from the group consisting of an azetidine ring represented by formula (VI), a pyrrolidine ring represented by formula (VII), a piperidine ring represented by formula (VIII) and a piperazine ring represented by formula (IX),
m is an integer of 0 to 2,
$R^3$ is optionally substituted $C_{1-6}$ alkyl, optionally substituted hetero cycloalkyl, or optionally substituted heteroaryl,
X is a bond, $-CH_2-$, -O-, -S-, or $-NR^{4X}-$,
$R^{4X}$ is H or optionally substituted $C_{1-3}$ alkyl,
$Y^1$ is -O-(optionally substituted $C_{1-3}$ alkylene)-$^{*Y2}$, -S-(optionally substituted $C_{1-3}$ alkylene)-$^{*Y2}$, $-SO_2$-(optionally

substituted $C_{1-3}$ alkylene)-$^{*Y2}$, -$NR^Y$-(optionally substituted $C_{1-3}$ alkylene)-$^{*Y2}$, -(optionally substituted $C_{1-3}$ alkylene)-O-$^{*Y2}$, -(optionally substituted $C_{1-3}$ alkylene)-S-$^{*Y2}$, -(optionally substituted $C_{1-3}$ alkylene)-$SO_2$-$^{*Y2}$, or -(optionally substituted $C_{1-3}$ alkylene)-$NR^Y$-$^{*Y2}$ (wherein $^{*Y2}$ represents a site of connection to $Y^2$),
$R^Y$ is H or optionally substituted $C_{1-3}$ alkyl,
$Y^2$ is a bond, optionally substituted phenylene, or optionally substituted heteroarylene,
$L^P$ is a group that chemically bonds $Y^2$ to EUB,
EUB is a group having the ability to bind to one E3 ubiquitin ligase selected from the group consisting of VHL, celebron, IAP, MDM2, DCAF11, DCAF15, DCAF16, BIRC2, KEAP1, RNF4, RNF114, FEM1B, and AhR, and
Sp is a spacer unit and is bonded to CLL and D, wherein Sp is bonded to an arbitrary nitrogen atom of -NH- or oxygen atom of -OH contained in D.

[38] The drug-linker conjugate or the salt thereof according to [37], wherein

$V^2$ is one group selected from the group consisting of the following formula (VIb) and formula (VIIb) (wherein $^{*SP}$ represents a site of connection to Sp):

[Chemical Formula 27]

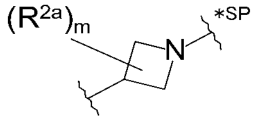

(VIb)

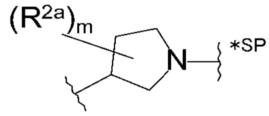

(VIIb)

W is one group selected from the group consisting of the following formula (VIIIb), formula (IXb), formula (Xb), formula (XIb), formula (XIIb), formula (XIIIb), formula (XIVb), formula (XVb), and formula (XVIb) (wherein $^{*SP}$ represents a site of connection to Sp):

[Chemical Formula 28]

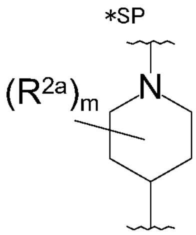

(VIIIb)

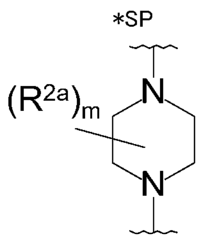

(IXb)

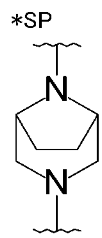

(Xb)

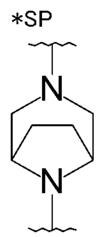

(XIb)

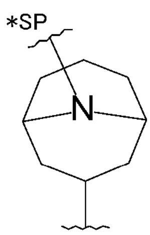

(XIIb)

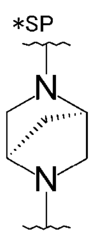

(XIIIb)

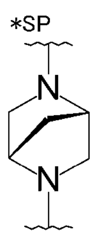

(XIVb)

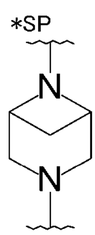

(XVb)

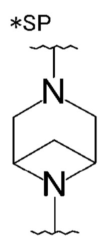

(XVIb)

and
Sp is a spacer unit and is bonded to CLL and D, wherein Sp is bonded to D through a nitrogen atom contained in $V^2$ or W at the site of connection represented by $^{*SP}$.

[39] The drug-linker conjugate or the salt thereof according to [37], wherein

A is N,

Q is $CR^Q$, $R^Q$ is cyclopropyl,
E is CH,
$R^1$ is the following formula (III-2):

[Chemical Formula 29]

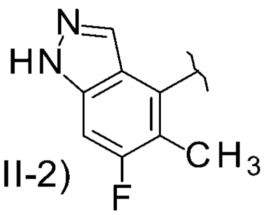

(III-2)

$R^2$ is $-V^1-V^2$ or W,
$V^1$ is $-N(CH_3)-$,
$V^2$ is the following formula (VII-2):

[Chemical Formula 30]

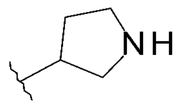

(VII-2)

W is the following formula (XIV):

[Chemical Formula 31]

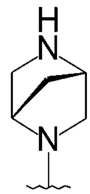

(XIV)

$R^3$ is n-propyl optionally substituted by $-OCH_3$, or tetrahydropyranyl,
X is -O-,
$Y^1$ is -O-(methylene)-$^{*Y2}$ (wherein $^{*Y2}$ represents a site of connection to $Y^2$),
$Y^2$ is phenylene,
$L^P$ is a group that chemically bonds $Y^2$ to EUB, and
EUB is a group having the ability to bind to one E3 ubiquitin ligase selected from the group consisting of VHL and celebron.

[40] The drug-linker conjugate or the salt thereof according to [39], wherein

$V^2$ is the following formula (VII-2b) (wherein $^{*SP}$ represents a site of connection to Sp):

[Chemical Formula 32]

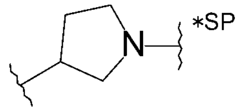

(VII-2b)

W is the following formula (XIVb) (wherein *SP represents a site of connection to Sp):

[Chemical Formula 33]

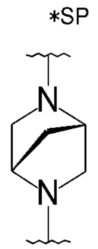

(XIVb)

and

Sp is a spacer unit and is bonded to CLL and D, wherein Sp is bonded to D through a nitrogen atom contained in $V^2$ or W at the site of connection represented by *SP.

[41] The drug-linker conjugate or the salt thereof according to [36] or [37], wherein D is (4R)-1-[(2S)-2-(4-{4-[({6-cyclopropyl-4-[(1S,4S)-2,5-diazabicyclo[2.2.1]heptan-2-yl]-7-(6-fluoro-5-methyl-1H-indazol-4-yl)-2-[(oxan-4-yl)oxy]quinazolin-8-yl}oxy)methyl]phenyl}-1H-1,2,3-triazol-1-yl)-3-methylbutanoyl]-4-hydroxy-N-{(1R)-2-hydroxy-1-[4-(4-methyl-1,3-thiazol-5-yl)phenyl]ethyl}-L-prolinamide, (4R)-1-[(2S)-2-(4-{4-[({6-cyclopropyl-4-[(1S,4S)-2,5-diazabicyclo[2.2.1]heptan-2-yl]-7-(6-fluoro-5-methyl-1H-indazol-4-yl)-2-[(oxan-4-yl)oxy]quinazolin-8-yl}oxy)methyl]phenyl}-1H-1,2,3-triazol-1-yl)-3-methylbutanoyl]-4-hydroxy-N-{(1R)-2-hydroxy-1-[4-(2-oxo-1,3-oxazolidin-3-yl)phenyl]ethyl}-L-prolinamide, (4R)-1-[(2S)-2-(4-{4-[({6-cyclopropyl-4-[(1S,4S)-2,5-diazabicyclo[2.2.1]heptan-2-yl]-7-(6-fluoro-5-methyl-1H-indazol-4-yl)-2-[(oxan-4-yl)oxy]quinazolin-8-yl}oxy)methyl]phenyl}-1H-1,2,3-triazol-1-yl)-3-methylbutanoyl]-4-hydroxy-N-{(1R)-2-hydroxy-1-[4-(1-methyl-1H-pyrazol-5-yl)phenyl]ethyl}-L-prolinamide, (4R)-1-[(2S)-2-(4-{4-[({6-cyclopropyl-4-[(1S,4S)-2,5-diazabicyclo[2.2.1]heptan-2-yl]-7-(6-fluoro-5-methyl-1H-indazol-4-yl)-2-[(2S)-2-methoxypropoxy]quinazolin-8-yl}oxy)methyl]phenyl}-1H-1,2,3-triazol-1-yl)-3-methylbutanoyl]-4-hydroxy-N-{(1R)-2-hydroxy-1-[4-(4-methyl-1,3-thiazol-5-yl)phenyl]ethyl}-L-prolinamide, (4R)-1-[(2S)-2-(4-{4-[({6-cyclopropyl-4-[(1S,4S)-2,5-diazabicyclo[2.2.1]heptan-2-yl]-7-(6-fluoro-5-methyl-1H-indazol-4-yl)-2-[(2S)-2-methoxypropoxy]quinazolin-8-yl}oxy)methyl]phenyl}-1H-1,2,3-triazol-1-yl)-3-methylbutanoyl]-N-{(1R)-1-[4-(1-ethyl-1H-pyrazol-5-yl)phenyl]-2-hydroxyethyl}-4-hydroxy-L-prolinamide, (4R)-1-[(2S)-2-(4-{4-[({6-cyclopropyl-4-[(1S,4S)-2,5-diazabicyclo[2.2.1]heptan-2-yl]-7-(6-fluoro-5-methyl-1H-indazol-4-yl)-2-[(2S)-2-methoxypropoxy]quinazolin-8-yl}oxy)methyl]phenyl}-1H-1,2,3-triazol-1-yl)-3-methylbutanoyl]-4-hydroxy-N-{(1R)-2-hydroxy-1-[4-(4-methyl-1,3-oxazol-5-yl)phenyl]ethyl}-L-prolinamide, (4R)-1-[(2S)-2-{4-[4-({[(7M)-6-cyclopropyl-7-(6-fluoro-5-methyl-1H-indazol-4-yl)-2-[(2S)-2-methoxypropoxy]-4-{methyl[(3S)-pyrrolidin-3-yl]amino}quinazolin-8-yl]oxy}methyl)phenyl]-1H-1,2,3-triazol-1-yl}-3-methylbutanoyl]-4-hydroxy-N-{(1R)-2-hydroxy-1-[4-(4-methyl-1,3-thiazol-5-yl)phenyl]ethyl}-L-prolinamide, (4R)-1-[(2S)-2-{4-[4-({[(7M)-6-cyclopropyl-7-(6-fluoro-5-methyl-1H-indazol-4-yl)-2-[(2S)-2-methoxypropoxy]-4-{methyl[(3S)-pyrrolidin-3-yl]amino}quinazolin-8-yl]oxy}methyl)phenyl]-1H-1,2,3-triazol-1-yl}-3-methylbutanoyl]-N-{(1R)-1-[4-(1-ethyl-1H-pyrazol-5-yl)phenyl]-2-hydroxyethyl}-4-hydroxy-L-prolinamide, 3-(5-{[(3S)-4-{4-[({(7M)-6-cyclopropyl-4-[(1S,4S)-2,5-diazabicyclo[2.2.1]heptan-2-yl]-7-(6-fluoro-5-methyl-1H-indazol-4-yl)-2-[(oxan-4-yl)oxy]quinazolin-8-yl}oxy)methyl]benzoyl}-3-methylpiperazin-1-yl]methyl}-3-methyl-2-oxo-2,3-dihydro-1H-benzimidazol-1-yl)piperidine-2,6-dione, 1-(6-{[(3S)-4-{4-[({(7M)-6-cyclopropyl-4-[(1S,4S)-2,5-diazabicyclo[2.2.1]heptan-2-yl]-7-(6-fluoro-5-methyl-1H-indazol-4-yl)-2-[(oxan-4-yl)oxy]quinazolin-8-yl}oxy)methyl]benzoyl}-3-methylpiperazin-1-yl]methyl}-1-methyl-1H-indazol-3-yl)-1,3-diazinane-2,4-dione, 3-(5-{[(3S)-4-{4-[({(7M)-6-cyclopropyl-4-[(1S,4S)-2,5-diazabicyclo[2.2.1]heptan-2-yl]-7-(6-fluoro-5-methyl-1H-indazol-4-yl)-2-[(2S)-2-methoxypropoxy]quinazolin-8-yl}oxy)methyl]benzoyl}-3-methylpiperazin-1-yl]methyl}-3-methyl-2-oxo-2,3-dihydro-1H-benzimidazol-1-yl)piperidine-2,6-dione, or (2R)-2-({(4R)-1-[(2S)-2-{4-[4-({[(7M)-6-cyclopropyl-7-(6-fluoro-5-methyl-1H-indazol-4-yl)-2-[(2S)-2-methoxypropoxy]-4-{methyl[(3S)-pyrrolidin-3-yl]amino}quinazolin-8-yl]oxy}methyl)phenyl]-1H-1,2,3-triazol-1-yl}-3-methylbutanoyl]-4-hydroxy-L-prolyl}amino)-2-[4-(1H-1,2,4-triazol-1-yl)phenyl]ethyl dihydrogen phosphate.

[42] The drug-linker conjugate or the salt thereof according to [36], wherein formula (LD-1) is represented by formula (LD-2a) or (LD-3a):

[Chemical Formula 34]

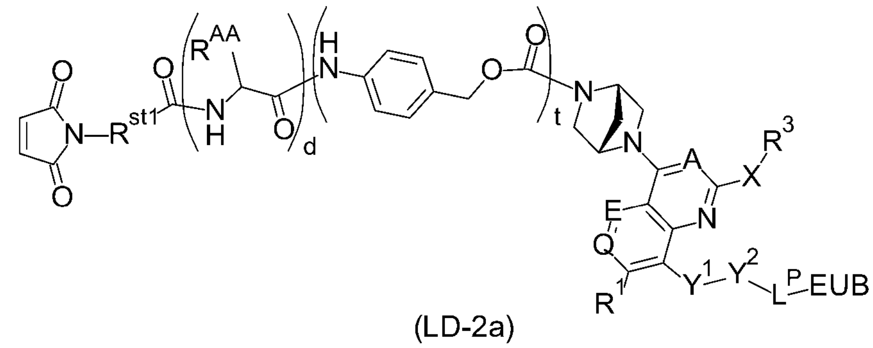

(LD-2a)

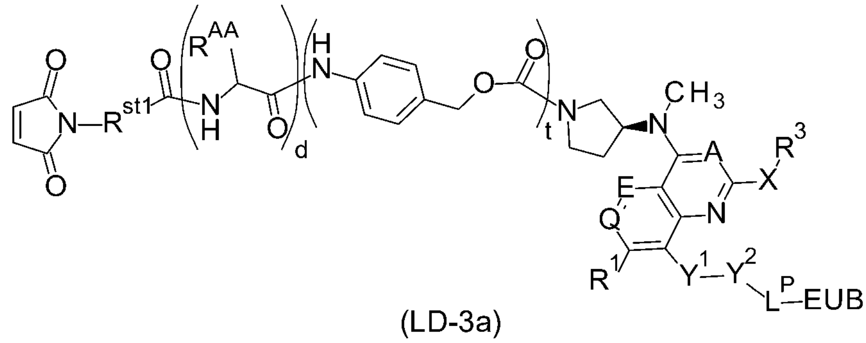

(LD-3a)

wherein

A is $CR^A$ or N,
$R^A$ is H, cyano, or optionally substituted $C_{1-3}$ alkyl,
Q is $CR^Q$ or N,
$R^Q$ is H, halogen, $C_{3-6}$ cycloalkyl, vinyl, or optionally substituted $C_{1-3}$ alkyl,
E is CH or N,
$R^1$ is naphthyl optionally substituted by one or two groups selected from the group consisting of cyano, OH, halogen, and optionally substituted $C_{1-3}$ alkyl, or is one group selected from the group consisting of the following formula (III), formula (IV), and formula (V):

[Chemical Formula 35]

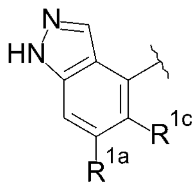

(III)

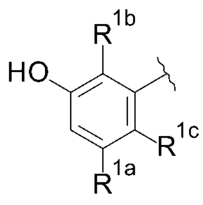

(IV)

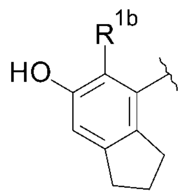

(V)

$R^{1a}$, $R^{1b}$, and $R^{1c}$ are each independently H, vinyl, halogen, or optionally substituted $C_{1-3}$ alkyl,
$R^3$ is optionally substituted $C_{1-6}$ alkyl, optionally substituted hetero cycloalkyl, or optionally substituted heteroaryl,
X is a bond, $-CH_2-$, -O-, -S-, or $-NR^{4X}-$,
$R^{4X}$ is H or optionally substituted $C_{1-3}$ alkyl,
$Y^1$ is -O-(optionally substituted $C_{1-3}$ alkylene)-$^{*Y2}$, -S-(optionally substituted $C_{1-3}$ alkylene)-$^{*Y2}$, $-SO_2-$(optionally substituted $C_{1-3}$ alkylene)-$^{*Y2}$, $-NR^Y-$(optionally substituted $C_{1-3}$ alkylene)-$^{*Y2}$, -(optionally substituted $C_{1-3}$ alkylene)-O-$^{*Y2}$, -(optionally substituted $C_{1-3}$ alkylene)-S-$^{*Y2}$, -(optionally substituted $C_{1-3}$ alkylene)-$SO_2$-$^{*Y2}$, or -(optionally substituted $C_{1-3}$ alkylene)-$NR^Y$-$^{*Y2}$ (wherein $^{*Y2}$ represents a site of connection to $Y^2$),
$R^Y$ is H or optionally substituted $C_{1-3}$ alkyl,
$Y^2$ is a bond, optionally substituted phenylene, or optionally substituted heteroarylene,

$L^P$ is a group that chemically bonds $Y^2$ to EUB,
EUB is a group having the ability to bind to one E3 ubiquitin ligase selected from the group consisting of VHL and celebron,
$R^{st1}$ is $C_{1-12}$ alkylene,
$R^{AA}$ are each independently H, methyl, isopropyl, benzyl, or $-(CH_2)_3-NH-C(=O)-NH_2$, d is an integer of 2 to 4, and t is 0 or 1.

[43] The drug-linker conjugate or the salt thereof according to [36], wherein formula (LD-1) is represented by formula (LD-4a), (LD-5a), (LD-6a), (LD-7a), (LD-8a), (LD-9a), (LD-10a), (LD-11a), (LD-12a), (LD-13a), (LD-14a), or (LD-26a):

[Chemical Formula 36-1]

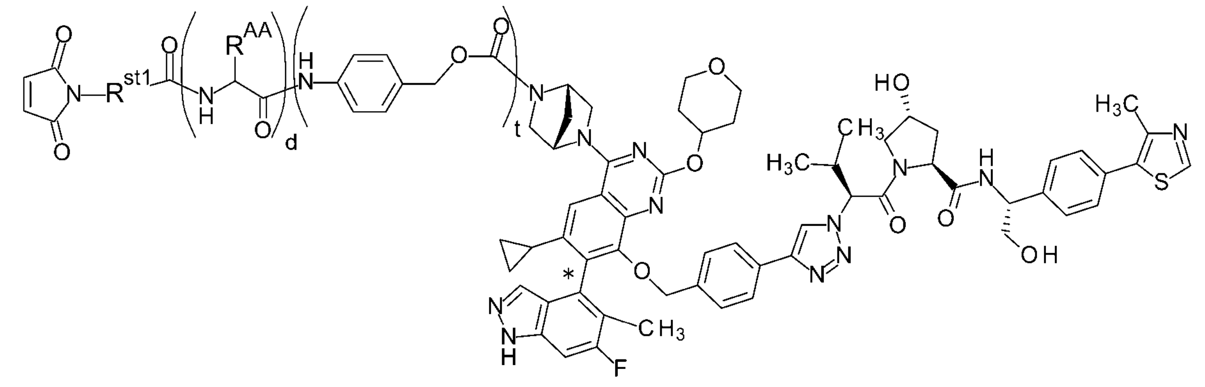

(LD-4a)

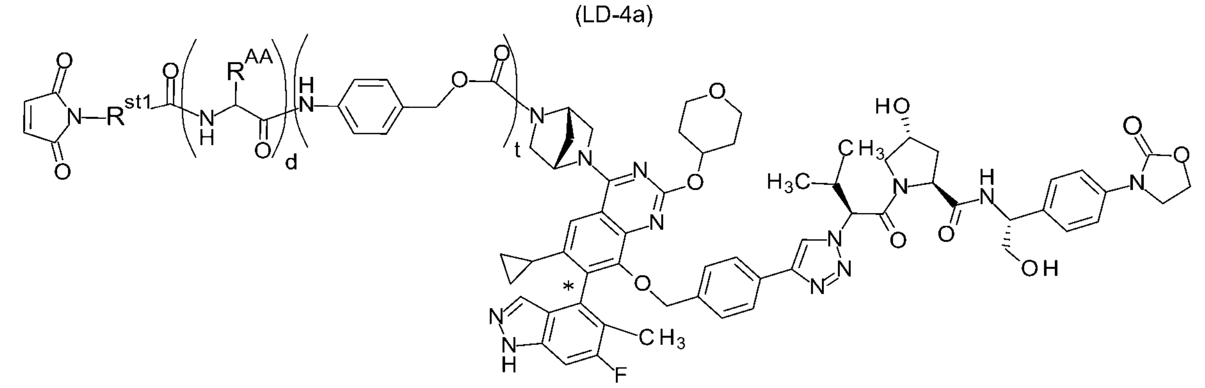

(LD-5a)

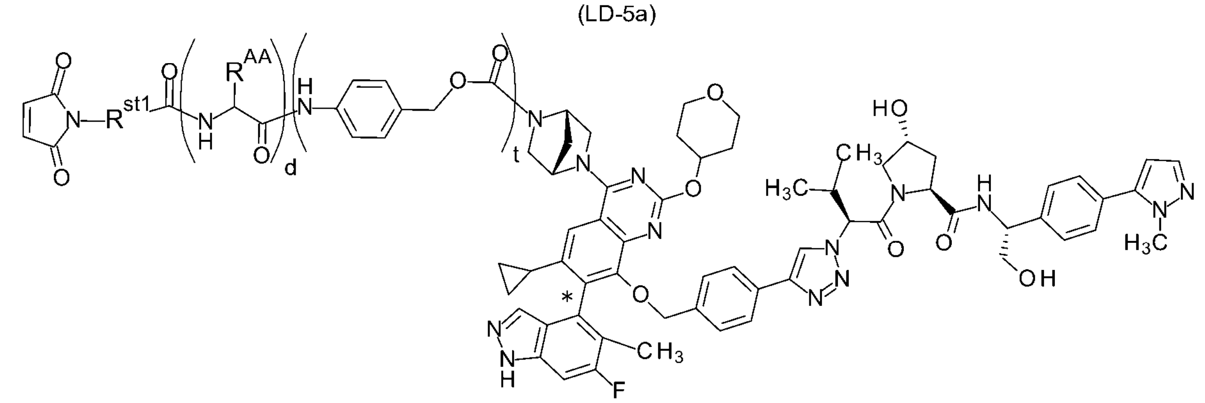

(LD-6a)

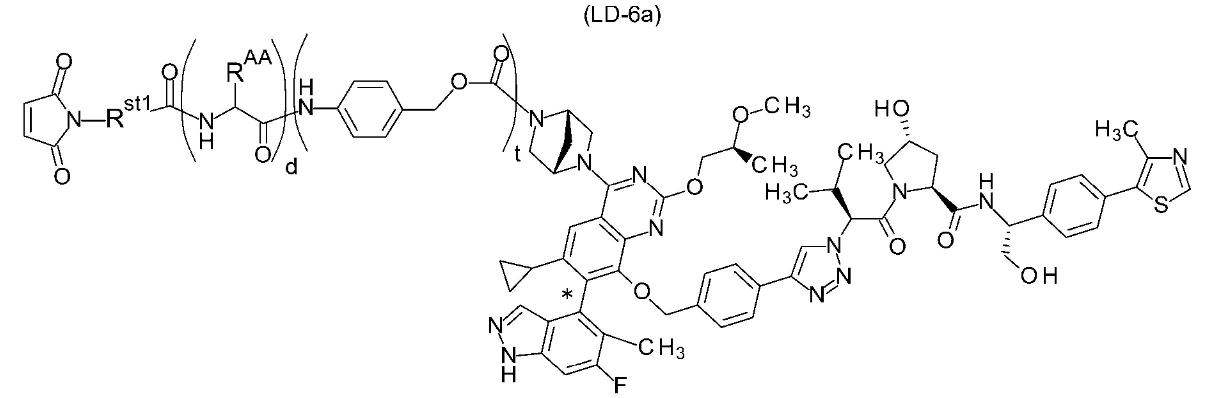

(LD-7a)

[Chemical Formula 36-2]

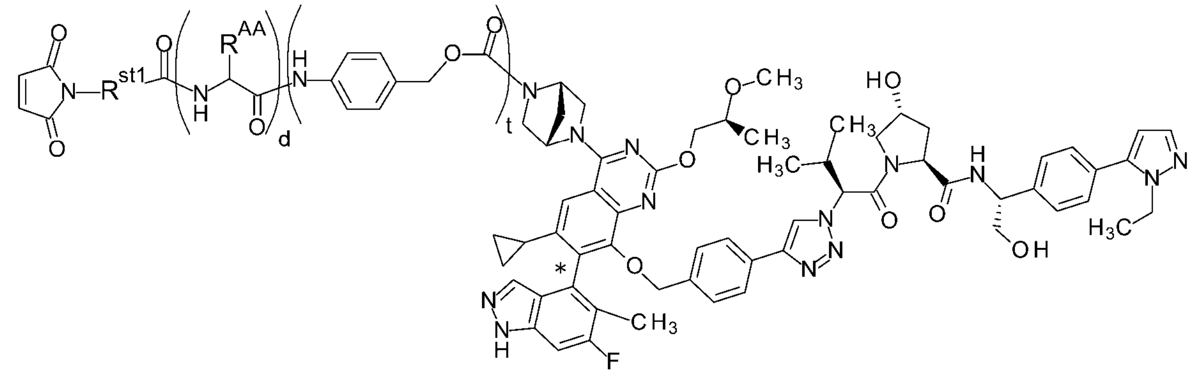

(LD-8a)

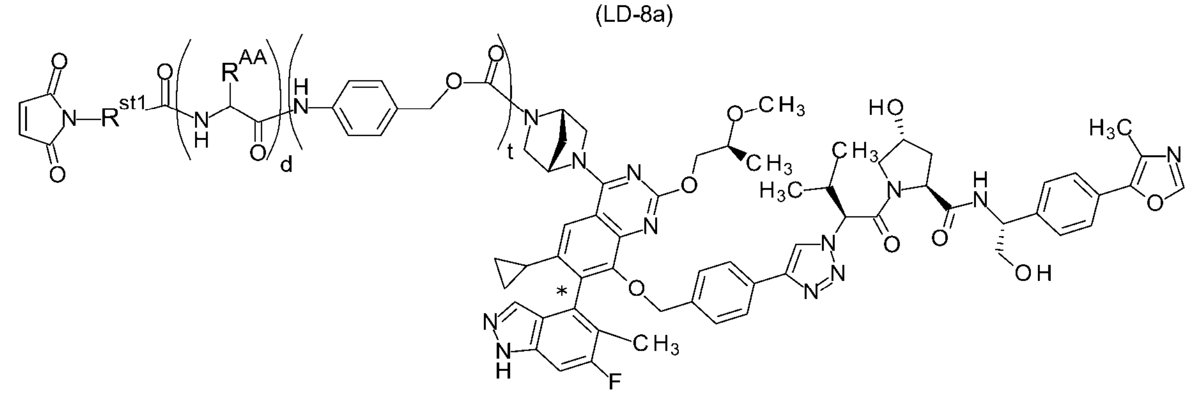

(LD-9a)

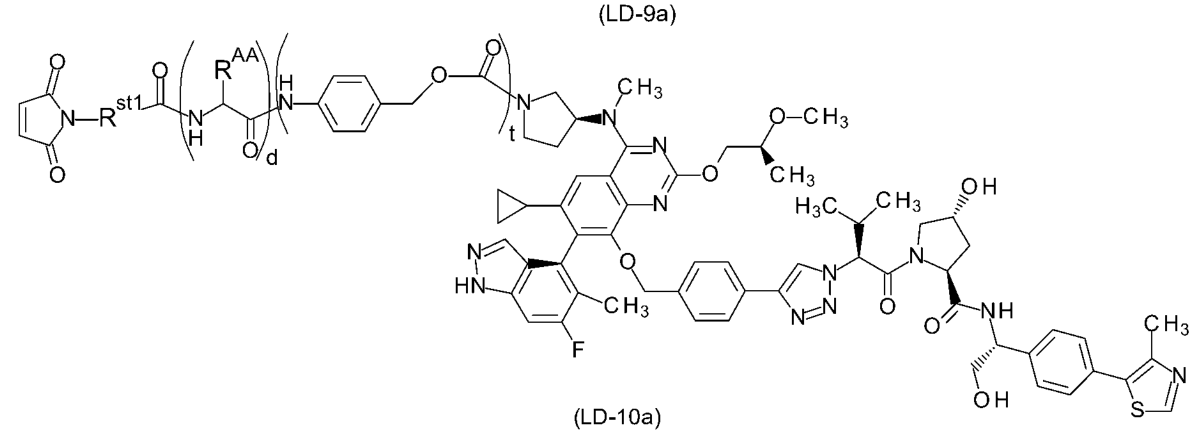

(LD-10a)

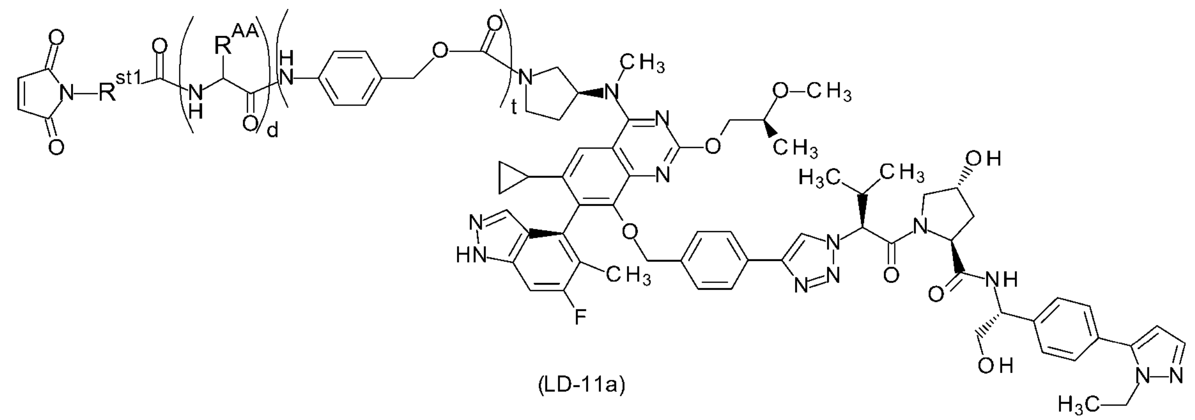

(LD-11a)

[Chemical Formula 36-3]

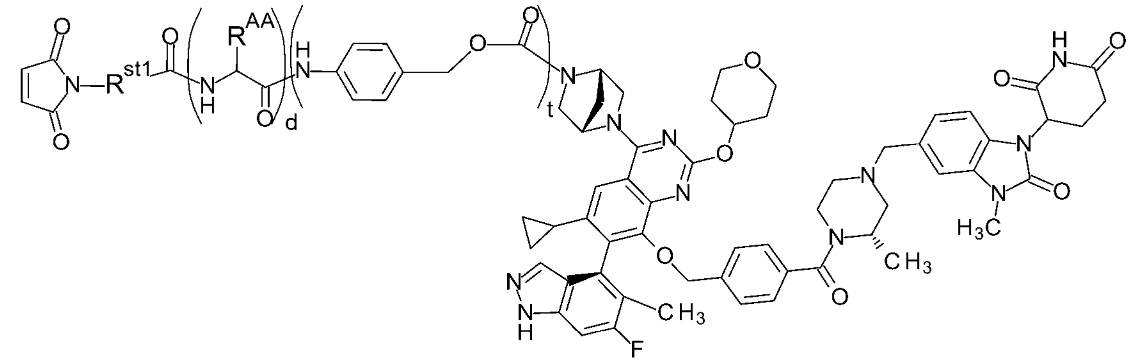

(LD-12a)

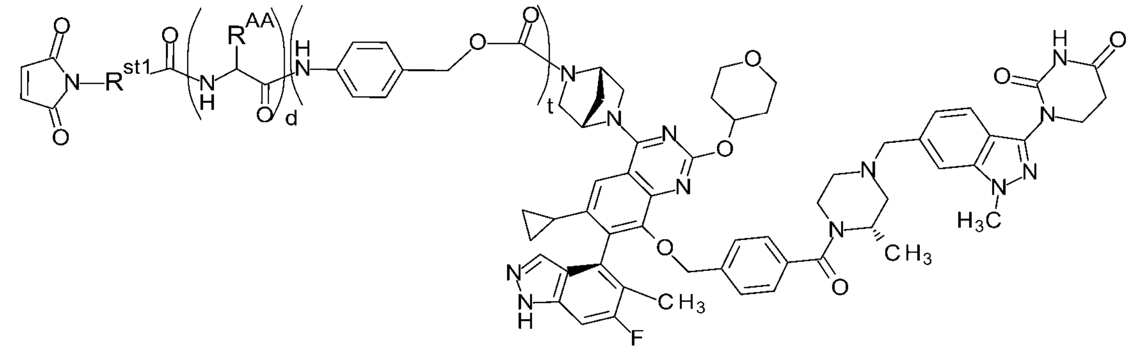

(LD-13a)

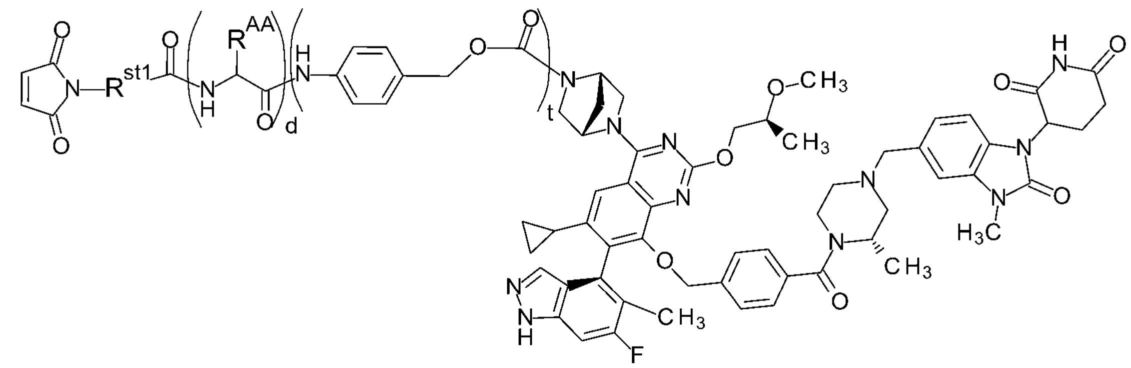

(LD-14a)

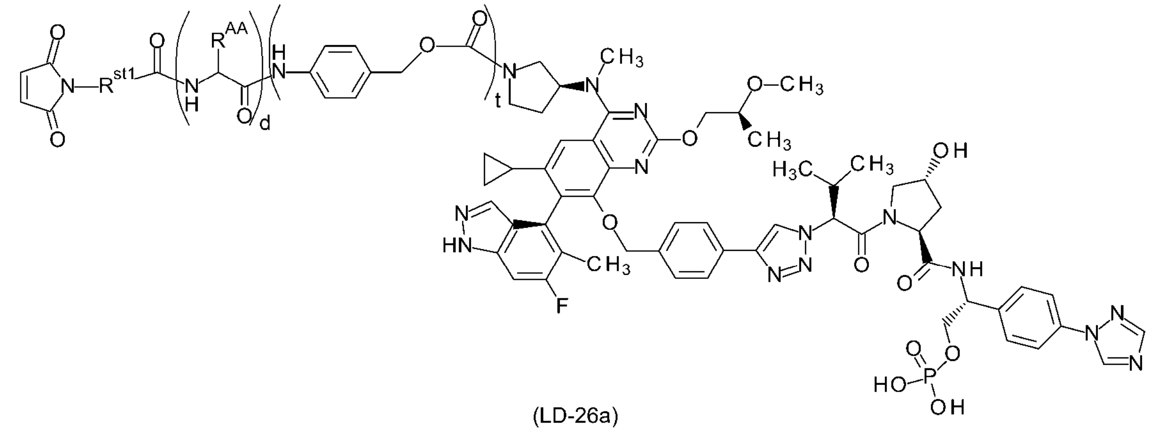

(LD-26a)

wherein $R^{st1}$ is $C_{1-12}$ alkylene, $R^{AA}$ are each independently H, methyl, isopropyl, benzyl, or - $(CH_2)_3$-NH-C(=O)-$NH_2$, d is an integer of 2 to 4, and t is 0 or 1.

[44] The drug-linker conjugate or the salt thereof according to [36], wherein formula (LD-1) is represented by formula (LD-15), (LD-16), (LD-17), (LD-18), (LD-19), (LD-20), (LD-21), (LD-22), (LD-23), (LD-24), (LD-25), or (LD-26):

[Chemical Formula 37-1]

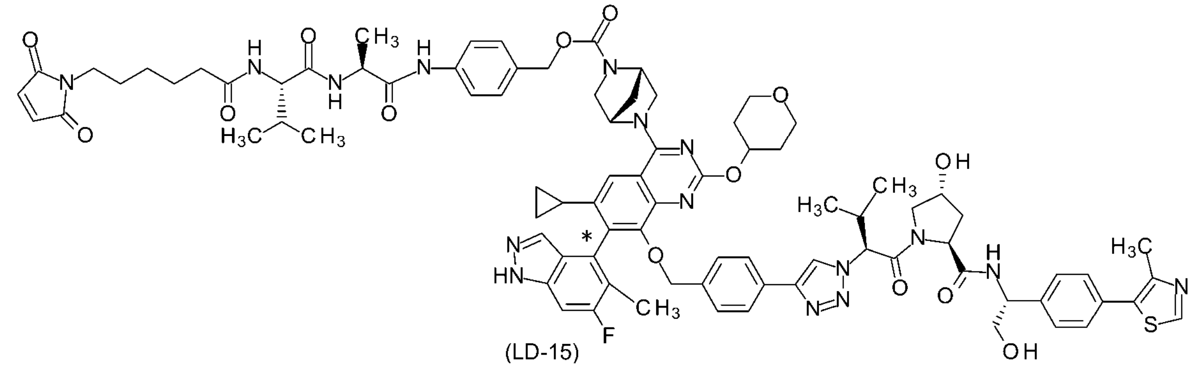

(LD-15)

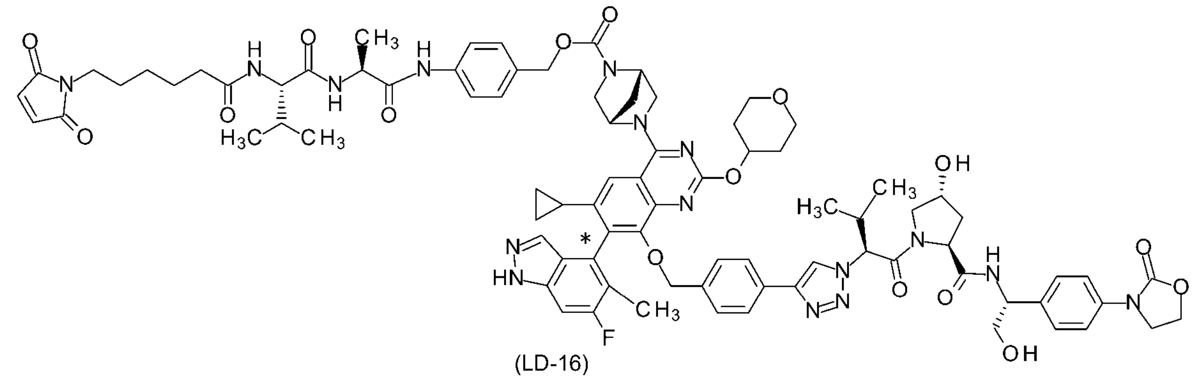

(LD-16)

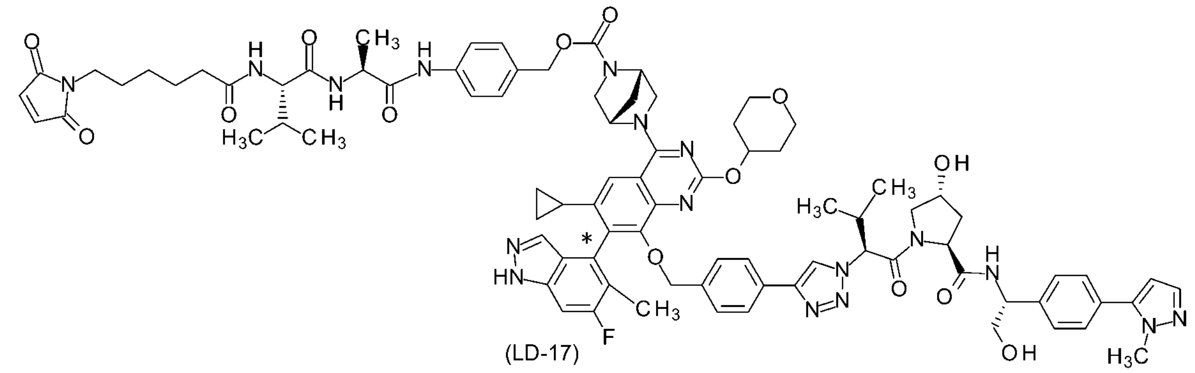

(LD-17)

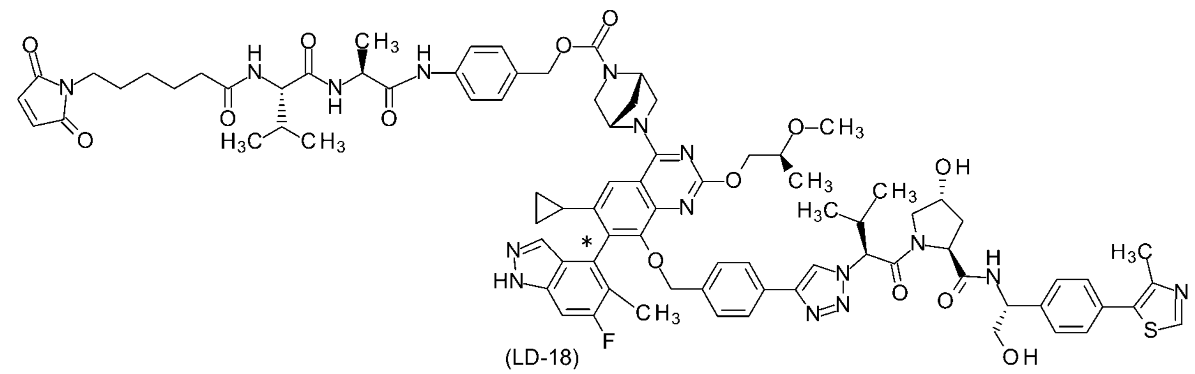

(LD-18)

[Chemical Formula 37-2]

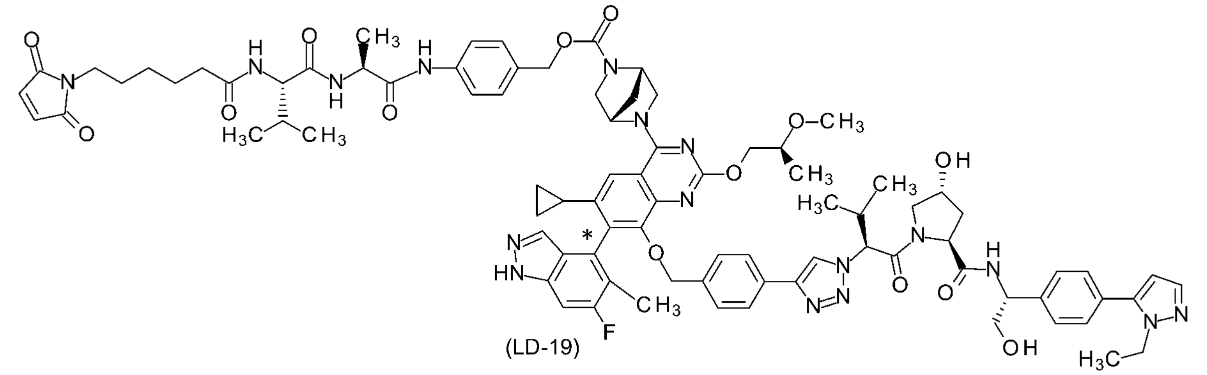

(LD-19)

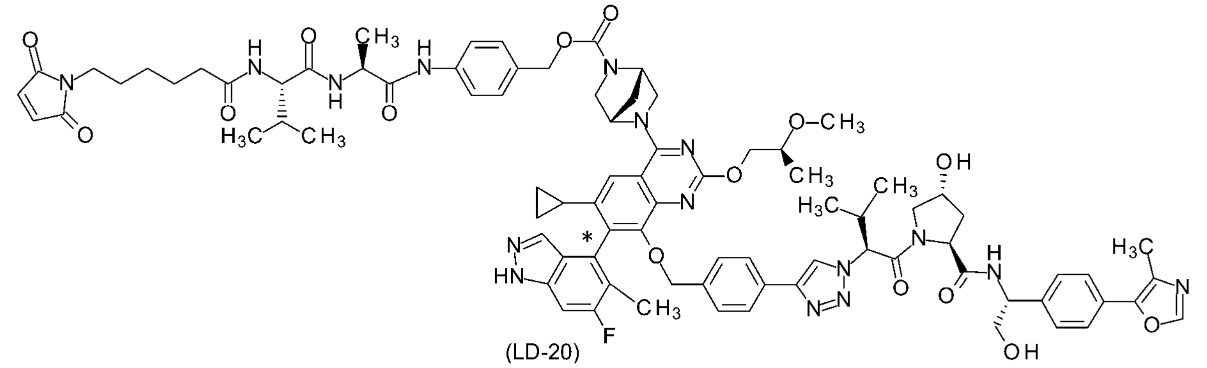

(LD-20)

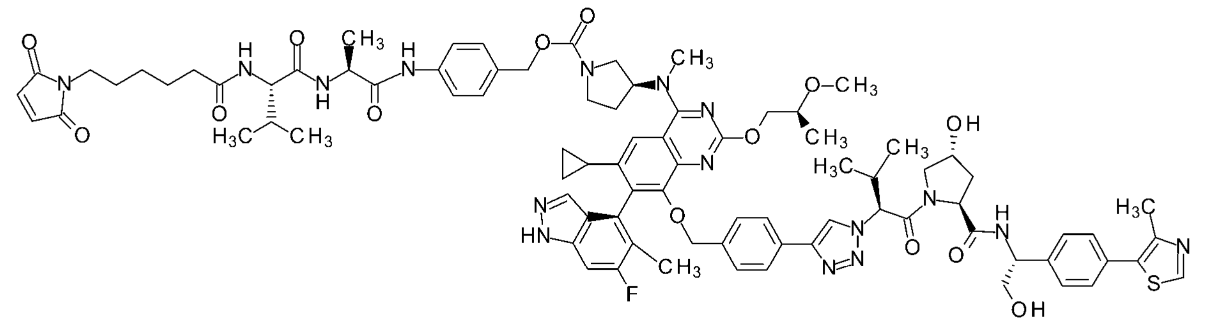

(LD-21)

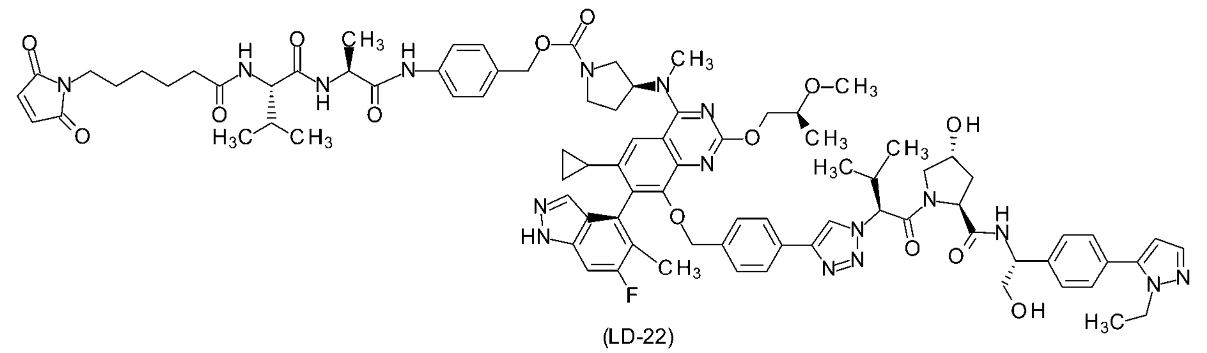

(LD-22)

[Chemical Formula 37-3]

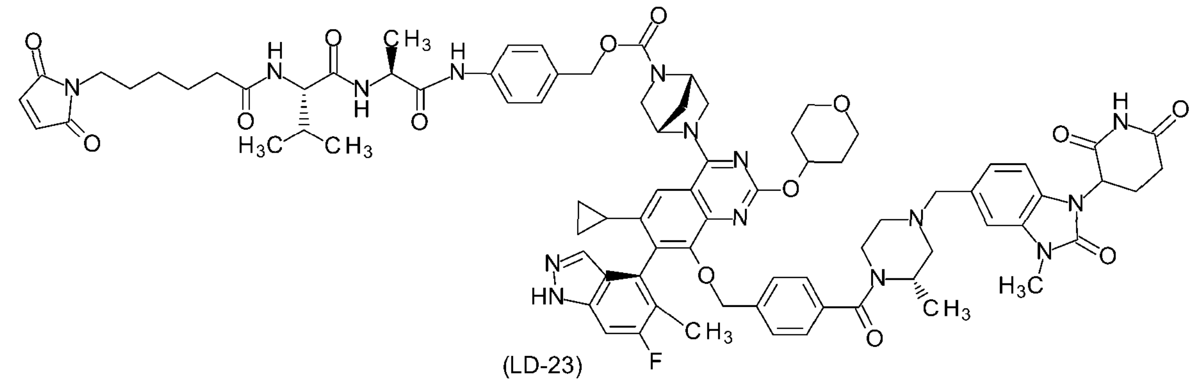

(LD-23)

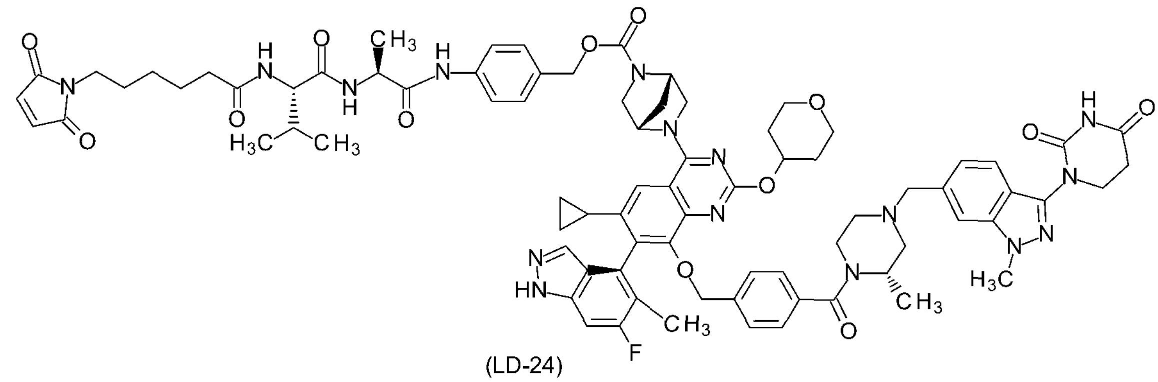

(LD-24)

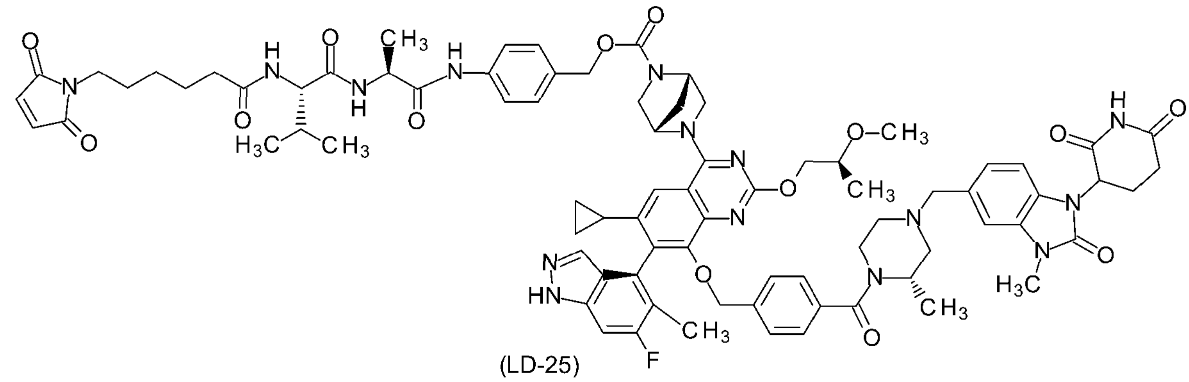

(LD-25)

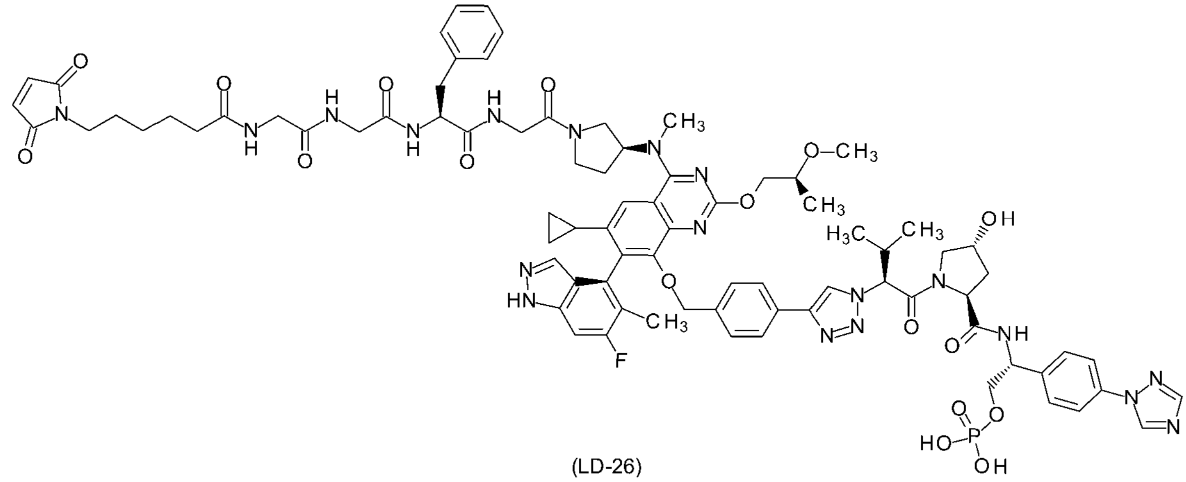

(LD-26)

[45] (2R)-2-({(4R)-1-[(2S)-2-{4-[4-({[(7M)-6-cyclopropyl-7-(6-fluoro-5-methyl-1H-indazol-4-yl)-2-[(2S)-2-methoxypropoxy]-4-{methyl[(3S)-pyrrolidin-3-yl]amino}quinazolin-8-yl]oxy}methyl)phenyl]-1H-1,2,3-triazol-1-yl}-3-methylbutanoyl]-4-hydroxy-L-prolyl}amino)-2-[4-(1H-1,2,4-triazol-1-yl)phenyl]ethyl dihydrogen phosphate or a salt thereof.

When a symbol in a chemical formula in the present specification is also used in another chemical formula, the same symbol represents the same meaning, unless otherwise specified.

ADVANTAGEOUS EFFECTS OF INVENTION

An antibody-drug conjugate of formula (I) or a salt thereof has a G12D mutant KRAS protein degradation-inducing effect and can be used as a therapeutic agent for cancers, particularly, cancers expressing G12D mutant KRAS. Moreover, the drug-linker conjugate of the present invention contains a compound having a G12D mutant KRAS protein degradation-inducing effect and can be used for synthesizing the antibody-drug conjugate of the present invention or the salt thereof.

DESCRIPTION OF EMBODIMENTS

Hereinafter, the present invention will be described in detail.

As used herein, "optionally substituted" means unsubstituted or having 1 to 5 substituents. In certain embodiments, it means unsubstituted or having 1 to 3 substituents. In the case of having a plurality of substituents, these substituents may be the same or may be different from each other.

"$C_{1-12}$ alkyl" is linear or branched alkyl having 1 to 12 carbon atoms, for example, methyl, ethyl, n-propyl, isopropyl, n-butyl, isobutyl, sec-butyl, tert-butyl, n-pentyl, n-hexyl, or dodecyl (hereinafter, the number of carbon atoms is similarly expressed). It is ethyl or dodecyl in certain embodiments.

Likewise, "$C_{1-6}$ alkyl" is linear or branched alkyl having 1 to 6 carbon atoms, for example, methyl, ethyl, n-propyl, isopropyl, n-butyl, isobutyl, sec-butyl, tert-butyl, n-pentyl, or n-hexyl, is methyl, ethyl, n-propyl, isopropyl, or sec-butyl in certain embodiments, is methyl, ethyl, n-propyl, isopropyl or tert-butyl in certain embodiments, is methyl, ethyl, n-propyl, isopropyl, or n-butyl in certain embodiments, is methyl, ethyl, or n-propyl in certain embodiments, is methyl or n-propyl in certain embodiments, is methyl in certain embodiments, is ethyl in certain embodiments, and is n-propyl in certain embodiments.

Likewise, "$C_{1-3}$ alkyl" is linear or branched alkyl having 1 to 3 carbon atoms, for example, methyl, ethyl, n-propyl, or isopropyl, is methyl or ethyl in certain embodiments, is n-propyl or isopropyl in certain embodiments, is methyl or isopropyl in certain embodiments, is methyl or n-propyl in certain embodiments, is ethyl or isopropyl in certain embodiments, is methyl in certain embodiments, is ethyl in certain embodiments, is isopropyl in certain embodiments, and is n-propyl in certain embodiments.

Likewise, "$C_5$ alkyl" is linear or branched alkyl having 5 carbon atoms, for example, n-pentyl, 1-methylbutyl, 2-methylbutyl, 3-methylbutyl, 1,1-dimethylpropyl, or 2,2-dimethylpropyl, and is n-pentyl in certain embodiments.

"$C_{3-6}$ cycloalkyl" is cycloalkyl having 3 to 6 carbon atoms, for example, cyclopropyl, cyclobutyl, cyclopentyl, cyclohexyl. It is cyclobutyl, cyclopentyl, or cyclohexyl in certain embodiments, is cyclobutyl or cyclopentyl in certain embodiments, is cyclopentyl or cyclohexyl in certain embodiments, is cyclopropyl or cyclobutyl in certain embodiments, is cyclopropyl in certain embodiments, is cyclobutyl in certain embodiments, is cyclopentyl in certain embodiments, and is cyclohexyl in certain embodiments.

"$C_{1-3}$ alkylene" is a divalent group in which a carbon atom of "$C_{1-3}$ alkyl" described above further has another bond. It is, for example, methylene, ethylene, trimethylene, methylmethylene, or 1,1-dimethylmethylene. It is linear or branched $C_{1-3}$ alkylene in certain embodiments, is methylene, ethylene, or trimethylene in certain embodiments, is methylene or ethylene in certain embodiments, is methylene in certain embodiments, and is ethylene in certain embodiments.

Likewise, "$C_{1-6}$ alkylene" is a divalent group in which a carbon atom of "$C_{1-6}$ alkyl" described above further has another bond. It is, for example, methylene, ethylene, trimethylene, methylethylene, tetramethylene, methyltrimethylene, ethylethylene, dimethylethylene, pentamethylene, or hexamethylene. It is methylene, ethylene, trimethylene, tetramethylene, pentamethylene, or hexamethylene in certain embodiments, and is $C_{1-3}$ alkylene in certain embodiments.

Likewise, "$C_{1-12}$ alkylene" is a divalent group in which a carbon atom of "$C_{1-12}$ alkyl" described above further has another bond. It is, for example, methylene, ethylene, trimethylene, tetramethylene, pentamethylene, hexamethylene, or dodecamethylene. It is linear or branched $C_{1-12}$ alkylene in certain embodiments, is $C_{3-8}$ alkylene in certain embodiments, is $C_5$ alkylene in certain embodiments, and is pentamethylene in certain embodiments.

Likewise, "$C_5$ alkylene" is a divalent group in which a carbon atom of "$C_5$ alkyl" described above further has another bond. It is, for example, 1-methyltetramethylene, 2-methyltetramethylene, 3-methyltetramethylene, 1,1-dimethyltrimethylene, or 2,2-dimethyltrimethylene, and is pentamethylene in certain embodiments.

"Hetero cycloalkyl" is a 4- to 7-membered saturated heterocyclic group containing 1 to 4 heteroatoms selected from the group consisting of oxygen, sulfur, and nitrogen as a ring-constituting atom, and may partially contain an unsaturated bond. The sulfur atom as a ring-constituting atom of the saturated heterocyclic group may be oxidized. "Hetero cycloalkyl" is "4- to 6-membered hetero cycloalkyl containing 1 or 2 heteroatoms selected from the group

consisting of oxygen, sulfur, and nitrogen as a ring-constituting atom" in certain embodiments, is "4- to 6-membered hetero cycloalkyl containing 1 or 2 oxygen atoms as a ring-constituting atom" in certain embodiments, is "4- to 6-membered hetero cycloalkyl containing 1 oxygen atom as a ring-constituting atom" in certain embodiments, is "4- to 6-membered hetero cycloalkyl containing 1 or 2 nitrogen atoms as a ring-constituting atom" in certain embodiments, is oxetanyl, tetrahydrofuranyl, tetrahydropyranyl, azetidinyl, pyrrolidinyl, piperidinyl, oxazolidinyl, imidazolidinyl, piperazinyl, morpholinyl, thiomorpholinyl, or dioxothiomorpholinyl in certain embodiments, is oxetanyl, tetrahydropyranyl, or tetrahydrofuranyl in certain embodiments, is tetrahydropyranyl or tetrahydrofuranyl in certain embodiments, is tetrahydropyranyl in certain embodiments, is tetrahydrofuranyl in certain embodiments, is azetidinyl, pyrrolidinyl, piperidinyl, or piperazinyl in certain embodiments, is piperidinyl or piperazinyl in certain embodiments, is piperidinyl in certain embodiments, and is piperazinyl in certain embodiments.

"Hetero cycloalkylene" is a divalent group in which a ring-constituting nitrogen atom or carbon atom of "hetero cycloalkyl" described above further has another bond. "Hetero cycloalkylene" is "4- to 6-membered hetero cycloalkylene containing 1 or 2 heteroatoms selected from the group consisting of oxygen, sulfur, and nitrogen as a ring-constituting atom" in certain embodiments, is "4- to 6-membered hetero cycloalkylene containing 1 or 2 oxygen atoms as a ring-constituting atom" in certain embodiments, is "4- to 6-membered hetero cycloalkylene containing 1 oxygen atom as a ring-constituting atom" in certain embodiments, is "4- to 6-membered hetero cycloalkylene containing 1 or 2 nitrogen atoms as a ring-constituting atom" in certain embodiments, is oxetanediyl, tetrahydrofurandiyl, tetrahydropyrandiyl, azetidinediyl, pyrrolidinediyl, piperidinediyl, oxazolidinediyl, imidazolidinediyl, piperazinediyl, morpholinediyl, thiomorpholinediyl, or dioxothiomorpholinediyl in certain embodiments, is oxetanediyl, tetrahydropyrandiyl, or tetrahydrofurandiyl in certain embodiments, is tetrahydropyrandiyl or tetrahydrofurandiyl in certain embodiments, is tetrahydropyrandiyl in certain embodiments, is tetrahydrofurandiyl in certain embodiments, is azetidinediyl, pyrrolidinediyl, piperidinediyl, or piperazinediyl in certain embodiments, is piperidinediyl or piperazinediyl in certain embodiments, is piperidinediyl in certain embodiments, and is piperazinediyl in certain embodiments.

"Bridged hetero cycloalkyl" is a 7- to 9-membered bridged heterocyclic group containing 1 or 2 nitrogen atoms as a ring-constituting atom. It is a saturated 7- to 9-membered bridged heterocyclic group containing 1 or 2 nitrogen atoms as a ring-constituting atom in certain embodiments, is saturated 7- to 9-membered bridged hetero cycloalkyl containing 2 nitrogen atoms as a ring-constituting atom in certain embodiments, and is saturated 7- to 9-membered bridged hetero cycloalkyl containing 2 nitrogen atoms as a ring-constituting atom in which one of the two nitrogen atoms is bonded to one hydrogen atom, in certain embodiments. It is, for example, diazabicyclo[2.2.2]octanyl, diazabicyclo[3.2.1]octanyl, diazabicyclo[3.1.1]heptanyl, diazabicyclo[2.2.1]heptanyl, or diazabicyclo[3.3.1]nonanyl. It is diazabicyclo[2.2.2]octanyl, diazabicyclo[3.2.1]octanyl, diazabicyclo[3.2.1]oct-6-enyl, diazabicyclo[3.2.1]oct-2-enyl, diazabicyclo[3.1.1]heptanyl, diazabicyclo[2.2.1]heptanyl, or diazabicyclo[2.2.1]hept-5-enyl in certain embodiments, is diazabicyclo[2.2.2]octanyl, diazabicyclo[3.2.1]octanyl, diazabicyclo[3.1.1]heptanyl, or diazabicyclo[2.2.1]heptanyl in certain embodiments, is diazabicyclo[2.2.1]heptanyl or diazabicyclo[3.2.1]octanyl in certain embodiments, is diazabicyclo[2.2.1]heptanyl in certain embodiments, is diazabicyclo[3.2.1]octanyl in certain embodiments, is 2,5-diazabicyclo[2.2.1]heptanyl or 3,8-diazabicyclo[3.2.1]octanyl in certain embodiments, is 2,5-diazabicyclo[2.2.1]heptanyl in certain embodiments, and is 3,8-diazabicyclo[3.2.1]octanyl in certain embodiments.

"Bridged hetero cycloalkylene" is a divalent group in which a ring-constituting nitrogen atom or carbon atom of "bridged hetero cycloalkyl" described above further has another bond. It is saturated 7- to 9-membered bridged hetero cycloalkylene containing 2 nitrogen atoms as a ring-constituting atom in certain embodiments, and is saturated 7- to 9-membered bridged hetero cycloalkylene containing 2 nitrogen atoms as a ring-constituting atom in which one of the two nitrogen atoms is bonded to one hydrogen atom, in certain embodiments. It is, for example, diazabicyclo[2.2.2]octanediyl, diazabicyclo[3.2.1]octanediyl, diazabicyclo[3.1.1]heptanediyl, diazabicyclo[2.2.1]heptanediyl, or diazabicyclo[3.3.1]nonanediyl. It is diazabicyclo[2.2.2]octanediyl, diazabicyclo[3.2.1]octanediyl, diazabicyclo[3.2.1]oct-6-enediyl, diazabicyclo[3.2.1]oct-2-enediyl, diazabicyclo[3.1.1]heptanediyl, diazabicyclo[2.2.1]heptanediyl, or diazabicyclo[2.2.1]hept-5-enediyl in certain embodiments, is diazabicyclo[2.2.2]octanediyl, diazabicyclo[3.2.1]octanediyl, diazabicyclo[3.1.1]heptanediyl, or diazabicyclo[2.2.1]heptanediyl in certain embodiments, is diazabicyclo[2.2.1]heptanediyl or diazabicyclo[3.2.1]octanediyl in certain embodiments, is diazabicyclo[2.2.1]heptanediyl in certain embodiments, is diazabicyclo[3.2.1]octanediyl in certain embodiments, is 2,5-diazabicyclo[2.2.1]heptanediyl or 3,8-diazabicyclo[3.2.1]octanediyl in certain embodiments, is 2,5-diazabicyclo[2.2.1]heptanediyl in certain embodiments, and is 3,8-diazabicyclo[3.2.1]octanediyl in certain embodiments.

"Bridged piperazinyl" is piperazinyl having a bridged structure in carbon atoms on a ring, and the bridged structure is constituted by the carbon atoms. It is, for example, diazabicyclo[2.2.1]heptanyl, diazabicyclo[3.2.1]octanyl, or diazabicyclo[3.1.1]heptanyl. It is diazabicyclo[2.2.1]heptanyl in certain embodiments. It is diazabicyclo[3.2.1]octanyl in certain embodiments. It is diazabicyclo[3.1.1]heptanyl in certain embodiments, is 2,5-diazabicyclo[2.2.1]heptanyl or 3,8-diazabicyclo[3.2.1]octanyl in certain embodiments, is 2,5-diazabicyclo[2.2.1]heptanyl in certain embodiments, and is 3,8-diazabicyclo[3.2.1]octanyl in certain embodiments.

"Bridged piperazinediyl" is a divalent group in which a ring-constituting nitrogen atom of "bridged piperazinyl"

described above further has another bond. It is, for example, diazabicyclo[2.2.1]heptanediyl, diazabicyclo[3.2.1]octanediyl, or diazabicyclo[3.1.1]heptanediyl. It is diazabicyclo[2.2.1]heptanediyl in certain embodiments. It is diazabicyclo [3.2.1]octanediyl in certain embodiments. It is diazabicyclo[3.1.1]heptanediyl in certain embodiments, is 2,5-diazabicyclo [2.2.1]heptanediyl or 3,8-diazabicyclo[3.2.1]octanediyl in certain embodiments, is 2,5-diazabicyclo[2.2.1]heptanediyl in certain embodiments, and is 3,8-diazabicyclo[3.2.1]octanediyl in certain embodiments.

"Spiro hetero cycloalkyl" is a saturated 7- to 9-membered heterocyclic group containing 1 or 2 nitrogen atoms as a ring-constituting atom and having a spiro atom. It is a saturated 7- to 9-membered heterocyclic group containing 2 nitrogen atoms as a ring-constituting atom and having a spiro atom in certain embodiments. It is, for example, diazaspiro[3.3] heptanyl, diazaspiro[3.4]octanyl, diazaspiro[3.5]nonanyl, or diazaspiro[4.4]nonanyl. It is 2,6-diazaspiro[3.4]octanyl in certain embodiments, and is 2,6-diazaspiro[3.3]heptanyl in certain embodiments.

"Spiro hetero cycloalkylene" is a divalent group having 2 nitrogen atoms as a ring-constituting atom of "spiro hetero cycloalkyl" described above in which the two nitrogen atoms each have a bond. It is, for example, 2,6-diazaspiro [3.3]heptanediyl, 2,6-diazaspiro[3.4]octanediyl, 2,7-diazaspiro[3.5]nonanediyl, 2,7-diazaspiro[4.4]nonanediyl, in certain embodiments, 2,6-diazaspiro[3.4]octanediyl, in certain embodiments, or 2,6-diazaspiro[3.3]heptanediyl. Alternatively, "spiro hetero cycloalkylene" may be a divalent group in which a ring-constituting nitrogen atom or carbon atom of "spiro hetero cycloalkyl" described above further has another bond. Specifically, "spiro hetero cycloalkylene" is saturated 7- to 9-membered spiro hetero cycloalkylene having 1 or 2 nitrogen atoms in certain embodiments and is, for example, 2,6-diazaspiro[3.3]heptanediyl, 2,6-diazaspiro[3.4]octanediyl, 2,7-diazaspiro[3.5]nonanediyl, 2,7-diazaspiro[4.4]nonanediyl, 2-azaspiro[3.3]heptanediyl, 2-azaspiro[3.4]octanediyl, 6-azaspiro[3.4]octanediyl, 2-azaspiro[3.5]nonanediyl, 7-azaspiro[3.5]nonanediyl, 2-azaspiro[4.4]nonanediyl, 7-azaspiro[4.4]nonanediyl, in certain embodiments, 2,6-diazaspiro[3.4]octanediyl, in certain embodiments, or 2,6-diazaspiro[3.3]heptanediyl.

"Heterocyclic ring" is an aromatic heterocyclic ring containing 1 to 4 heteroatoms selected from the group consisting of oxygen, sulfur, and nitrogen as a ring-constituting atom.

"5-Membered heterocyclic ring" is a 5-membered heterocyclic ring containing 1 to 4 heteroatoms selected from the group consisting of oxygen, sulfur, and nitrogen as a ring-constituting atom. "5-Membered heterocyclic ring" is a pyrazole ring, an imidazole ring, a triazole ring, a tetrazole ring, an oxazole ring, an isoxazole ring, a thiazole ring, an isothiazole ring, an oxadiazole ring, or a thiadiazole ring in certain embodiments, and is a pyrazole ring, an imidazole ring, a triazole ring, an oxazole ring, an isoxazole ring, a thiazole ring, an isothiazole ring, an oxadiazole ring, or a thiadiazole ring in certain embodiments.

"6-Membered heterocyclic ring" is a 6-membered heterocyclic ring having 1 to 3 nitrogen atoms as a ring-constituting atom. "6-Membered heterocyclic ring" is a 6-membered heterocyclic ring having 1 to 3 nitrogen atoms as a ring-constituting atom in certain embodiments, and is a pyridine ring, a pyrimidine ring, a pyrazine ring, a pyridazine ring, or a triazine ring in certain embodiments.

"5- or 6-Membered heterocyclic ring" is a 5-membered heterocyclic ring or a 6-membered heterocyclic ring. "5- or 6-Membered heterocyclic ring" is a 5-membered heterocyclic ring in certain embodiments, and is a 6-membered heterocyclic ring in certain embodiments.

"Heteroaryl" is a 5- or 6-membered aromatic heterocyclic group containing 1 to 4 heteroatoms selected from the group consisting of oxygen, sulfur, and nitrogen as a ring-constituting atom. It is heteroaryl of a 5-membered ring containing 1 to 4 heteroatoms selected from the group consisting of oxygen, sulfur, and nitrogen as a ring-constituting atom, or heteroaryl of a 6-membered ring containing 1 to 3 nitrogen atoms as a ring-constituting atom in certain embodiments, is heteroaryl of a 5-membered ring containing 1 to 4 heteroatoms selected from the group consisting of oxygen, sulfur, and nitrogen as a ring-constituting atom in certain embodiments, is heteroaryl of a 6-membered ring containing 1 to 3 nitrogen atoms as a ring-constituting atom in certain embodiments, is pyrazolyl, imidazolyl, triazolyl, tetrazolyl, oxazolyl, isoxazolyl, thiazolyl, isothiazolyl, oxadiazolyl, thiadiazolyl, pyridyl, pyrimidinyl, pyrazinyl, pyridazinyl, or triazinyl in certain embodiments, is pyrazolyl, imidazolyl, triazolyl, tetrazolyl, oxazolyl, isoxazolyl, thiazolyl, isothiazolyl, oxadiazolyl, or thiadiazolyl in certain embodiments, and is pyridyl, pyrimidinyl, pyrazinyl, pyridazinyl, or triazinyl in certain embodiments.

"Heteroarylene" is a divalent group in which two different ring-constituting carbon atoms and/or nitrogen atoms of "heteroaryl" described above have a bond. "Heteroarylene" is heteroarylene of a 5-membered ring containing 1 to 4 heteroatoms selected from the group consisting of oxygen, sulfur, and nitrogen as a ring-constituting atom or heteroarylene of a 6-membered ring containing 1 to 3 nitrogen atoms as a ring-constituting atom in certain embodiments, is heteroarylene of a 5-membered ring containing 1 to 4 heteroatoms selected from the group consisting of oxygen, sulfur, and nitrogen as a ring-constituting atom in certain embodiments, is heteroarylene of a 6-membered ring containing 1 to 3 nitrogen atoms as a ring-constituting atom in certain embodiments, is pyrazolediyl, imidazolediyl, triazolediyl, tetrazolediyl, oxazolediyl, isoxazolediyl, thiazolediyl, isothiazolediyl, oxadiazolediyl, thiadiazolediyl, pyridinediyl, pyrimidinediyl, pyrazinediyl, pyridazinediyl, or triazinediyl in certain embodiments, is pyrazolediyl, imidazolediyl, triazolediyl, tetrazolediyl, oxazolediyl, isoxazolediyl, thiazolediyl, isothiazolediyl, oxadiazolediyl, or thiadiazolediyl in certain embodiments, and is pyridinediyl, pyrimidinediyl, pyrazinediyl, pyridazinediyl, or triazinediyl in certain embodiments.

"4- to 8-Membered saturated heterocyclic ring" is a 4- to 8-membered saturated heterocyclic ring containing 1 or 2 heteroatoms selected from the group consisting of oxygen, sulfur, and nitrogen as a ring-constituting atom, may partially contain an unsaturated bond, may have a bridge, and may form a spiro ring, and the sulfur atom contained in the heterocyclic ring may be oxidized. It is oxetane, tetrahydrofuran, tetrahydropyran, azetidine, pyrrolidine, piperidine, azepane, oxazolidine, imidazolidine, piperazine, morpholine, thiomorpholine, dioxothiomorpholine, azabicyclo[2.2.1]heptane, diazabicyclo[2.2.1]heptane, azaspiro[3.3]heptane, azaspiro[3.4]octane, oxaazaspiro[3.3]heptane, or diazaspiro[3.3]heptane in certain embodiments, is oxetane, tetrahydrofuran, tetrahydropyran, azetidine, pyrrolidine, piperidine, piperazine, morpholine, thiomorpholine, dioxothiomorpholine, azaspiro[3.3]heptane, or oxaazaspiro[3.3]heptane in certain embodiments, is azetidine, tetrahydropyran, morpholine, or oxaazaspiro[3.3]heptane in certain embodiments, is azetidine or tetrahydropyran in certain embodiments, is morpholine or oxaazaspiro[3.3]heptane in certain embodiments, and is tetrahydropyran in certain embodiments.

The substituent acceptable for "optionally substituted hetero cycloalkyl", "optionally substituted hetero cycloalkylene", and "optionally substituted $C_{1-6}$ alkylene" included in $L^P$, $L^{1A}$, or $L^{4A}$ is $C_{1-3}$ alkyl, -O($C_{1-3}$ alkyl), C=O, halogen, or OH in certain embodiments, is $C_{1-3}$ alkyl, -O($C_{1-3}$ alkyl), or halogen in certain embodiments, is -O($C_{1-3}$ alkyl) or halogen in certain embodiments, is $C_{1-3}$ alkyl in certain embodiments, is OH in certain embodiments, is F in certain embodiments, is methyl in certain embodiments, and is ethyl in certain embodiments.

The substituent acceptable for "optionally substituted $C_{1-6}$ alkyl", "optionally substituted $C_{1-3}$ alkyl", and "optionally substituted $C_{1-3}$ alkylene" is $C_{1-3}$ alkyl, -O($C_{1-3}$ alkyl), C=O, halogen, or OH in certain embodiments, is $C_{1-3}$ alkyl, -O($C_{1-3}$ alkyl), or halogen in certain embodiments, is -O($C_{1-3}$ alkyl) or halogen in certain embodiments, is $C_{1-3}$ alkyl in certain embodiments, is OH in certain embodiments, is F in certain embodiments, is methyl in certain embodiments, and is ethyl in certain embodiments.

The substituent acceptable for "optionally substituted heteroarylene", "optionally substituted phenylene", and "optionally substituted heteroaryl" is $C_{1-3}$ alkyl, -O($C_{1-3}$ alkyl), halogen, or OH in certain embodiments, is $C_{1-3}$ alkyl, -O($C_{1-3}$ alkyl), or halogen in certain embodiments, is $C_{1-3}$ alkyl in certain embodiments, is methyl in certain embodiments, is ethyl in certain embodiments, is halogen in certain embodiments, and is F in certain embodiments.

The substituent acceptable for "optionally substituted oxazolyl" is $C_{1-3}$ alkyl in certain embodiments, is methyl or isopropyl in certain embodiments, is methyl in certain embodiments, and is isopropyl in certain embodiments.

The substituent acceptable for "optionally substituted $C_{1-12}$ alkylene", and "optionally substituted $C_{1-6}$ alkylene" included in $R^{st1}$ is -$NH_2$, -$C_{1-6}$ alkylene-$NH_2$, -NH-C(=O)-$C_{1-6}$ alkyl, or -NH-C(=O)-$C_{1-3}$ alkyl in certain embodiments, and is -$C_{1-6}$ alkylene-$NH_2$ in certain embodiments.

"Halogen" means F, Cl, Br, and I. It is F, Cl, or Br in certain embodiments, is F or Cl in certain embodiments, is F or Br in certain embodiments, is F in certain embodiments, is Cl in certain embodiments, and is Br in certain embodiments.

"Halogeno $C_{1-6}$ alkyl" is a linear or branched $C_{1-6}$ alkyl group substituted by one or more halogen. Examples include trifluoromethyl, trifluoroethyl, trifluoropropyl, 2-fluoro-2-methylpropyl, difluoromethyl, difluoroethyl, fluoromethyl, and chloromethyl. It is difluoroethyl, trifluoromethyl, or difluoromethyl in certain embodiments, is trifluoromethyl or difluoromethyl in certain embodiments, is trifluoromethyl in certain embodiments, and is difluoromethyl in alternative embodiments.

"EUB" is a group having the ability to bind to E3 ubiquitin ligase. It is a group having the ability to bind to one E3 ubiquitin ligase selected from the group consisting of VHL (von Hippel-Lindau), celebron, IAP (inhibitor of apoptosis protein), MDM2 (mouse double minute 2 homolog), DCAF11 (DDB1 and CUL4 associated factor 11), DCAF15 (DDB1 and CUL4 associated factor 15), DCAF16 (DDB1 and CUL4 associated factor 16), BIRC2 (Baculoviral IAP repeat containing 2), KEAP1 (Kelch-like ECH-associated protein 1), RNF4 (RING finger protein 4), RNF114 (RING finger protein 114), FEM1B (Protein fem-1 homolog B), and AhR (Aryl hydrocarbon receptor) in certain embodiments. It is a group having the ability to bind to one E3 ubiquitin ligase selected from the group consisting of VHL, celebron, IAP, and MDM2 in certain embodiments. It is a group having the ability to bind to one E3 ubiquitin ligase selected from the group consisting of VHL and celebron in certain embodiments. It is a group having the ability to bind to VHL in certain embodiments. It is a group having the ability to bind to celebron in certain embodiments. Those skilled in the art can understand it with reference to the following documents, though not limited thereto.

[Reference]

Current Research in Chemical Biology., 2022, 2, 100020
Front. Chem., 2021, 9, 707317
J. Am. Chem. Soc., 2021, 143, 5141
Signal Transduct. Target. Ther., 2020, 5, 129
Nat. Chem. Biol., 2019, 15 (7), 737

Communications Biology., 2020, 3, 140
Sci. Rep., 2020, 10 (1), 15543
ACS Chem. Biol., 2019, 14, 2430
Cell Chem. Biol., 2021, 28 (4), 559
J. Am. Chem. Soc., 2022, 144, 701
ACS Chem. Biol., 2019, 14, 2822

"G12D mutation" refers to a mutation that converts an amino acid residue corresponding to codon 12 of the wild-type protein from glycine to aspartic acid.

"G12D mutant KRAS" refers to KRAS having "G12D mutation" described above.

"$Z^{N1}$" is one group selected from the group consisting of the following formulas ($Z^{N1}$-1) to ($Z^{N1}$-15) (wherein $*^{LGZ}$ represents a site of connection to $LG^Z$ or $L^P$, $R^{Z1'}$ are each independently optionally substituted $C_{1-6}$ alkyl, halogen, cyano, -OH, -O-(optionally substituted $C_{1-6}$ alkyl), -S-(optionally substituted $C_{1-6}$ alkyl), -NH-(optionally substituted $C_{1-6}$ alkyl), or -N-(optionally substituted $C_{1-6}$ alkyl)$_2$, n' is an integer of 0 to 2, $R^{Z2'}$, $R^{Z3'}$, and $R^{Z4'}$ are each independently H or optionally substituted $C_{1-6}$ alkyl, and ring B1' is a benzene ring or a 6-membered heterocyclic ring, wherein $R^{Z1'}$ and -$*^{LGZ}$ form bonds with the carbon atoms constituting ring B1').

[Chemical Formula 38]

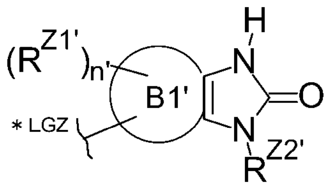

($Z^{N1}$−1)

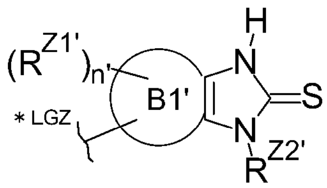

($Z^{N1}$−2)

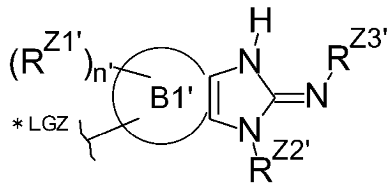

($Z^{N1}$−3)

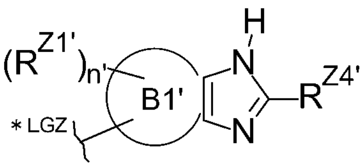

($Z^{N1}$−4)

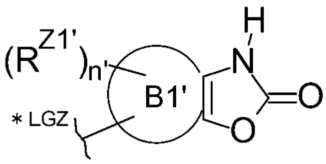

($Z^{N1}$−5)

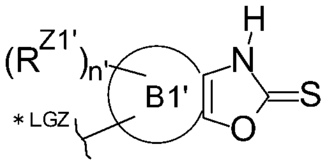

($Z^{N1}$−6)

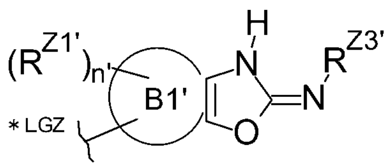

($Z^{N1}$−7)

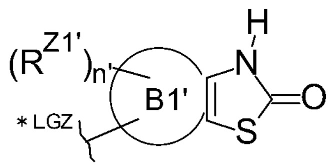

($Z^{N1}$−8)

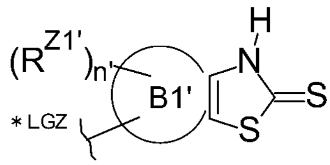

($Z^{N1}$−9)

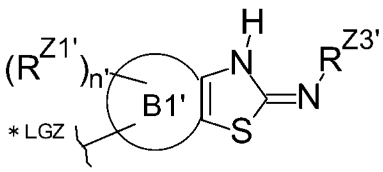

($Z^{N1}$−10)

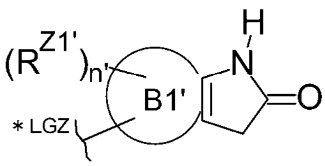

($Z^{N1}$−11)

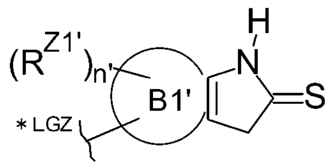

($Z^{N1}$−12)

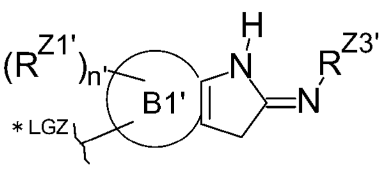

($Z^{N1}$−13)

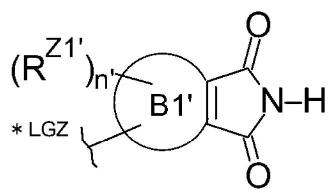

($Z^{N1}$−14)

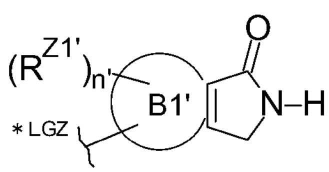

($Z^{N1}$−15)

"$Z^{N2}$" is one group selected from the group consisting of the following formulas ($Z^{N2}$-1) to ($Z^{N2}$-15) (wherein $^{*LGZ}$ represents a site of connection to $LG^Z$ or $L^P$, $R^{Z1'}$ are each independently optionally substituted $C_{1-6}$ alkyl, halogen, cyano, -OH, -O-(optionally substituted $C_{1-6}$ alkyl), -S-(optionally substituted $C_{1-6}$ alkyl), -NH-(optionally substituted $C_{1-6}$ alkyl), or -N-(optionally substituted $C_{1-6}$ alkyl)$_2$, n' is an integer of 0 to 2, $R^{Z2'}$ and $R^{Z3'}$ are each independently H or optionally substituted $C_{1-6}$ alkyl, and ring B1' is a benzene ring or a 6-membered heterocyclic ring, wherein $R^{Z1'}$ and -$^{*LGZ}$ form bonds with the carbon atoms constituting ring B1').

[Chemical Formula 39]

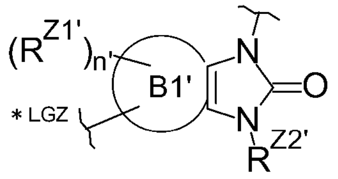

( $Z^{N2}$—1 )

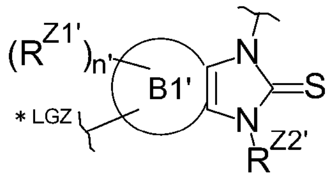

( $Z^{N2}$—2 )

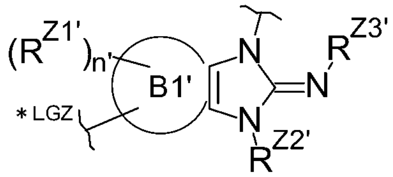

( $Z^{N2}$—3 )

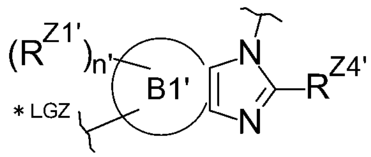

( $Z^{N2}$—4 )

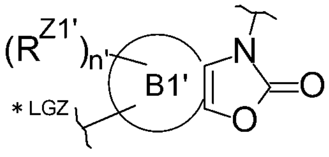

( $Z^{N2}$—5 )

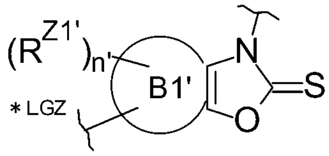

( $Z^{N2}$—6 )

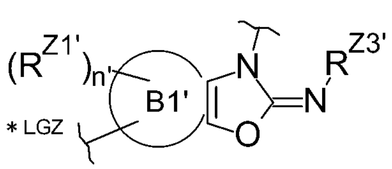

( $Z^{N2}$—7 )

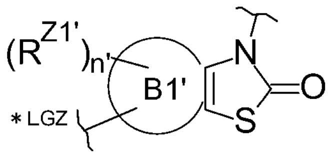

( $Z^{N2}$—8 )

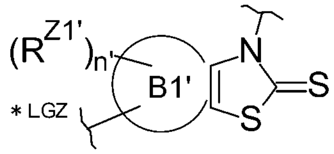

( $Z^{N2}$—9 )

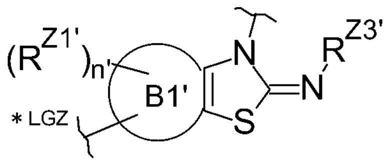

( $Z^{N2}$—10 )

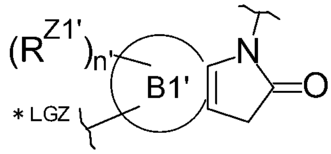

( $Z^{N2}$—11 )

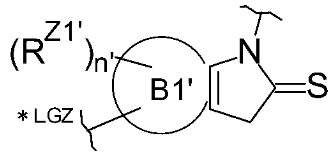

( $Z^{N2}$—12 )

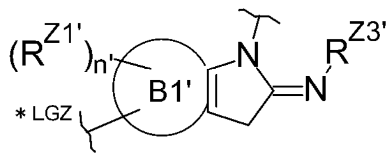

( $Z^{N2}$—13 )

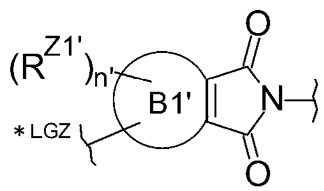

( $Z^{N2}$—14 )

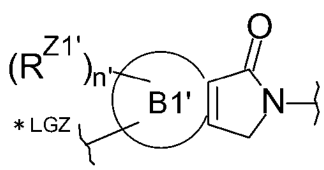

( $Z^{N2}$—15 )

"$Z^C$" is one group selected from the group consisting of the following formulas ($Z^C$-16) to ($Z^C$-27) (wherein $*^{LGZ}$ represents a site of connection to $LG^Z$ or $L^P$, $R^{Z1'}$ are each independently optionally substituted $C_{1-6}$ alkyl, halogen, cyano, -OH, -O-(optionally substituted $C_{1-6}$ alkyl), -S-(optionally substituted $C_{1-6}$ alkyl), -NH-(optionally substituted $C_{1-6}$ alkyl), or -N-(optionally substituted $C_{1-6}$ alkyl)$_2$, n' is an integer of 0 to 2, $R^{Z2'}$, $R^{Z4'}$, and $R^{Z5'}$ are each independently H or optionally substituted $C_{1-6}$ alkyl, M' is a bond, -O-, -S-, -N($R^{M'}$)-, or optionally substituted $C_{1-3}$ alkylene, $R^{M'}$ is H or optionally substituted $C_{1-3}$ alkyl, ring B1' is a benzene ring or a 6-membered heterocyclic ring, ring B2' is a benzene ring or a 5- or 6-membered heterocyclic ring, wherein $R^{Z1'}$ and -$*^{LGZ}$ form bonds with the carbon atoms constituting ring B1', and M', $R^{Z1'}$, and -$*^{LGZ}$ form bonds with the carbon atoms constituting ring B2').

[Chemical Formula 40]

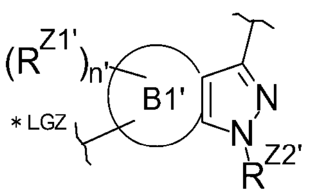

( $Z^C$—16 )

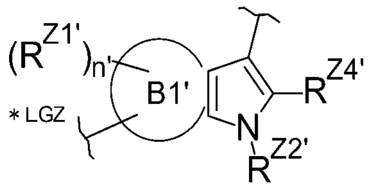

( $Z^C$—17 )

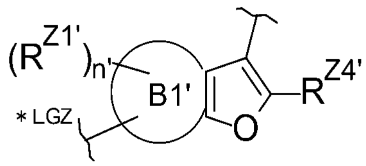

( $Z^C$—18 )

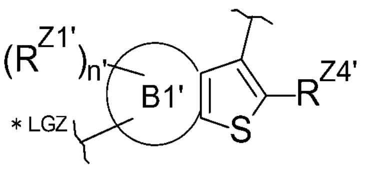

( $Z^C$—19 )

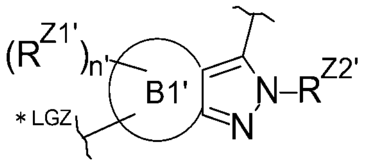

( $Z^C$—20 )

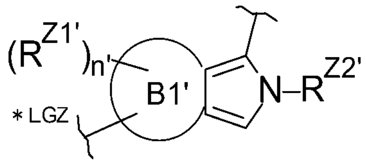

( $Z^C$—21 )

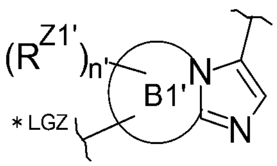

( $Z^C$—22 )

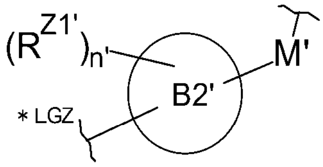

( $Z^C$—23 )

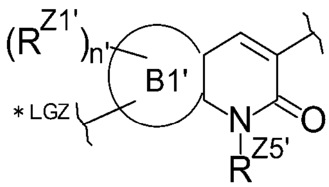

( $Z^C$—24 )

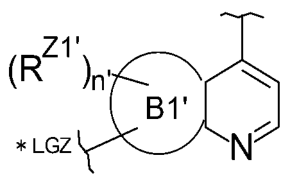

( $Z^C$—25 )

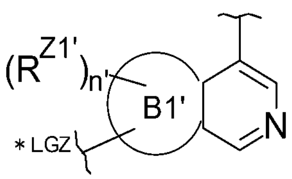

( $Z^C$—26 )

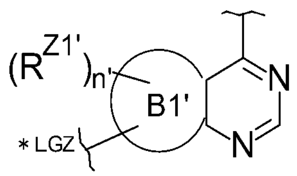

( $Z^C$—27 )

"Antigen" is used as a term that refers to a molecule or a portion of a molecule to which an antigen binding protein such as an antibody or an antigen binding fragment is capable of specifically binding. The antigen can be a molecule such as a protein or a nucleic acid. One antigen may have one or more epitopes that can interact with different antibodies or the like.

An antibody (or immunoglobulin) is a glycoprotein having, as a basic structure, a four-chain structure of a symmetric Y-shaped structure composed of two heavy chains having a single sequence and two light chains having a single sequence. The antibody has five classes, IgG, IgM, IgA, IgD, and IgE. The heavy chain usually consists of a polypeptide chain containing approximately 440 amino acids, has a structure characteristic to each class, and is designated as Igγ, Igμ, Igα, Igδ, and Igε corresponding to IgG, IgM, IgA, IgD, and IgE, respectively. Moreover, IgG has subclasses IgG1, IgG3, IgG3, and IgG4, and heavy chains corresponding to these subclasses are designated as Igγ1, Igy2, Igy3, and Igy4, respectively. The light chain usually consists of a polypeptide chain containing approximately 220 amino acids, is known to have two types, λ and κ light chains, which are designated as Igλ and Igκ, respectively. The two types of light chains can pair with any type of heavy chains.

The heavy chain has four (or five in Igμ and Igε) intrachain disulfide bonds of an antibody molecule, and the light chain has two intrachain disulfide bonds, with one loop formed every 100 to 110 amino acid residues. The three-dimensional structure is similar among loops and called structural unit or domain. In both the heavy chain and the light chain, a domain positioned at the N terminus is called variable region, which is known to have diverse amino acid sequences even among antibodies produced from the same class (or subclass) of animals of the same species, and to participate in the binding specificity of the antibody for an antigen. The amino acid sequence of a domain on the C-terminal

side downstream from the variable region is substantially constant in each class or subclass, and this domain is called constant region. The heavy chain has, from the N terminus toward the C terminus, a heavy chain variable region (VH) and a heavy chain constant region (CH). CH is further divided into three domains, CH1 domain, CH2 domain, and CH3 domain in the presented order from the N-terminal side. The light chain has, from the N terminus toward the C terminus, a light chain variable region (VL) and a light chain constant region (CL).

Three complementarity determining regions (CDRs) present in each of VH and VL vary very largely in amino acid sequence, and contribute to the variability of the variable regions. CDRs are regions of approximately 5 to 10 amino acid residues present at the N terminus of each of the heavy chain and the light chain in the order of CDR1, CDR2, and CDR3, and form an antigen binding site. On the other hand, moieties except for CDRs in the variable region are called framework regions (FRs), which consist of FR1 to FR4 and vary relatively small in amino acid sequence.

The treatment of the antibody with a proteolytic enzyme papain produces three antibody fragments. Two fragments on the N-terminal side are designated as Fab (fragment, antigen binding) regions, and a fragment on the C-terminal side is designated as an Fc (fragment, crystallizable) region.

As used herein, "antibody" is a polypeptide that specifically binds to an antigen and may have any structure as long as being capable of specifically binding to an antigen. The antibody includes, for example, a polypeptide having a four-chain structure as a basic structure, such as IgG, as well as polypeptides in various shapes such as an antigen binding fragment and a multispecific antibody (e.g., a bispecific antibody) mentioned later.

"Antigen binding fragment" is a molecule containing at least one polypeptide chain having antigen binding activity derived from an antibody. Representative examples of the antigen binding fragment include a single-chain variable region fragment (scFv), a Fab fragment, a Fab' fragment, and a F(ab')2 fragment. scFv is a monovalent antigen binding fragment constituted by VH and VL linked through a linker. The Fab fragment is a monovalent antigen binding fragment constituted by a fragment containing a light chain and the VH and CH1 domains of a heavy chain. The Fab' fragment is a monovalent antigen binding fragment constituted by a fragment containing a light chain, the VH and CH1 domains of a heavy chain, and a portion of a hinge region, and this moiety of the hinge region contains a cysteine residue constituting the S-S bond between the heavy chains. The F(ab')2 fragment is a bivalent molecule of Fab' fragments linked through a disulfide bond. Monovalence means that one antigen binding site is included, and bivalence means that two antigen binding sites are included.

"Polypeptide" means a plurality of amino acids linked through peptide bonds. The amino acids for use in the polypeptide may be natural amino acids or artificial amino acids. The number of amino acid residues contained in the polypeptide can be 2 or more and is preferably 10 or more.

"Multispecific antibody" is an antibody that can specifically bind to two or more different antigens, and is called bispecific antibody or trispecific antibody, for example, depending on the number of antigens to be bound. The multispecific antibody contains a complex of two or more antibodies and/or antigen binding fragments that can bind to different antigens. "Antibody" as used herein includes the multispecific antibody, unless otherwise specified in the context.

"Human antibody" refers to an antibody having a human immunoglobulin amino acid sequence. As used herein, "humanized antibody" refers to an antibody in which a portion of, most of, or all of amino acid residues excluding CDRs are replaced with amino acid residues derived from a human immunoglobulin molecule. A humanization method is not particularly limited, and the humanized antibody can be prepared with reference to, for example, U.S. Patent Nos. 5225539 and 6180370.

"Post-translational modification" means that an antibody expressed in a cell is modified after translation. Examples of the post-translational modification include N-linked or O-linked glycosylation, N-terminal or C-terminal processing, deamidation, isomerization of aspartic acid, and oxidation of methionine. Such post-translational modification is known to occur in various antibodies (J. Pharma. Sci., 2008; Vol. 97: p. 2426-2447).

An amino acid residue number of the antibody used in the present specification can be prescribed by specifying Kabat numbering or EU index (Kabat et al., Sequences of Proteins of Immunological Interest, 5th Ed., 1991, NIH Publication No. 91-3242) in accordance with these numbering systems.

"Identity" means a value of identity obtained using EMBOSS Needle (Nucleic Acids Res., 2015; Vol. 43, p. W580-W584) with parameters provided as default. The parameters described above are as follows.

Gap Open Penalty = 10

Gap Extend Penalty = 0.5

Matrix = EBLOSUM62

End Gap Penalty = false

In the present specification, the antibody and the antigen binding fragment are also collectively referred to as "Ab".

"Antibody-drug conjugate" is a conjugate of an antibody and a drug connected via a linker. It can transport the drug to cells or tissues targeted by the antibody. The antibody-drug conjugate is a conjugate of the antibody and the drug connected via a linker cleavable *in vivo* in certain embodiments. The antibody-drug conjugate is a conjugate of the antibody and the drug connected via a non-cleavable linker in certain embodiments.

"Linker" is a divalent chemical group that connects a functional compound to another functional compound. The linker is a linker cleavable *in vivo* in certain embodiments, and is a non-cleavable linker in certain embodiments.

"Linker cleavable *in vivo*" is a linker having a partial structure cleavable *in vivo*. "Partial structure cleavable *in vivo*" is a partial structure that can be cleaved by the action of an enzyme or the like *in vivo*. It is a partial structure that can be cleaved by the action of protease in certain embodiments, and is a partial structure that can be cleaved by the action of cathepsin B in certain embodiments.

"Non-cleavable linker" means a linker that is degraded neither under intralysosomal acidic conditions nor by any action of protease *in vivo*. It is, for example, C=O, $C_{1-6}$ alkylene, C(=O)NH-$C_{1-6}$ alkylene, or polyethylene glycol.

"Subject" means a human or other animals in need of prevention or treatment. It is a human in need of treatment or prevention in certain embodiments.

Embodiments of the antibody-drug conjugate of the present invention or the salt thereof, and drug (D), linker ($L^A$), and antibody or antigen binding fragment (Ab) constituting the antibody-drug conjugate or the salt thereof will be shown below. One or two or more embodiments can be combined with other embodiments unless a specific combination is described. In short, all embodiments can be freely combined.

1. Drug (D)

Drug (D) constituting the antibody-drug conjugate of the present invention or the salt thereof, and the drug-linker conjugate is a heterocyclic compound having a G12D mutant KRAS protein degradation-inducing effect. Some or all of linkers ($L^A$) constituting the antibody-drug conjugate of the present invention or the salt thereof are cleaved in tumor cells so that drugs (D) are liberated to exert an effect. When intramolecular -OH of drug (D) forms phosphoric acid ester, the phosphoric acid ester taken up into tumor cells may be cleaved by phosphatase to form a dephosphorylated form (Bioconjugate Chem., 2016; Vol. 27; p. 2081-2088). Certain embodiments of drug (D) according to the present invention will be shown below.

(1-1) A heterocyclic compound having a G12D mutant KRAS protein degradation-inducing effect or a salt thereof.

(1-2) A heterocyclic compound having a structure represented by formula (II) or a salt thereof:

[Chemical Formula 41]

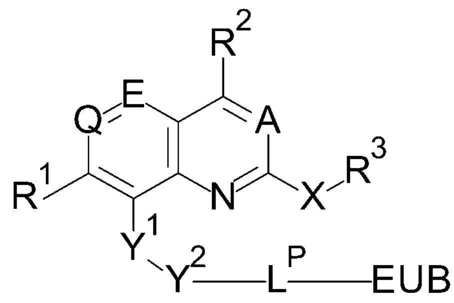

(II)

(2-1) The heterocyclic compound or the salt thereof according to (1-2), wherein A is $CR^A$ or N, and $R^A$ is H, cyano, or optionally substituted $C_{1-3}$ alkyl.

(2-2) The heterocyclic compound or the salt thereof according to (1-2), wherein A is CH or N.

(2-3) The heterocyclic compound or the salt thereof according to (1-2), wherein A is CH.

(2-4) The heterocyclic compound or the salt thereof according to (1-2), wherein A is N.

(3-1) The heterocyclic compound or the salt thereof according to (1-2), wherein Q is $CR^Q$ or N, and $R^Q$ is H, halogen, $C_{3-6}$ cycloalkyl, vinyl, or optionally substituted $C_{1-3}$ alkyl.

(3-2) The heterocyclic compound or the salt thereof according to (1-2), wherein Q is $CR^Q$, and $R^Q$ is $C_{3-6}$ cycloalkyl.
(3-3) The heterocyclic compound or the salt thereof according to (1-2), wherein Q is $CR^Q$, and $R^Q$ is cyclopropyl.
(4-1) The heterocyclic compound or the salt thereof according to (1-2), wherein E is CH or N.
(4-2) The heterocyclic compound or the salt thereof according to (1-2), wherein E is CH.
(5-1) The heterocyclic compound or the salt thereof according to (1-2), wherein $R^1$ is naphthyl optionally substituted by one or two groups selected from the group consisting of cyano, OH, halogen, and optionally substituted $C_{1-3}$ alkyl, or is one group selected from the group consisting of the following formula (III), formula (IV), and formula (V):

[Chemical Formula 42]

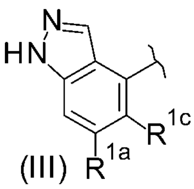
(III)
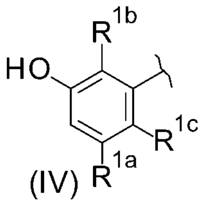
(IV)
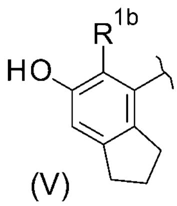
(V)

wherein $R^{1a}$, $R^{1b}$, and $R^{1c}$ are each independently H, vinyl, halogen, or optionally substituted $C_{1-3}$ alkyl.
(5-2) The heterocyclic compound or the salt thereof according to (1-2), wherein $R^1$ is the following formula (III):

[Chemical Formula 43]

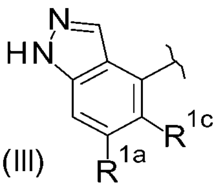
(III)

wherein $R^{1a}$ and $R^{1c}$ are each independently $C_{1-3}$ alkyl or halogen.
(5-3) The heterocyclic compound or the salt thereof according to (1-2), wherein $R^1$ is the following formula (III):

[Chemical Formula 44]

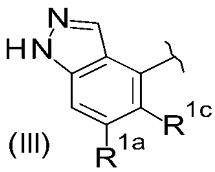
(III)

wherein $R^{1a}$ is halogen, and $R^{1c}$ is $C_{1-3}$ alkyl.
(5-4) The heterocyclic compound or the salt thereof according to (1-2), wherein $R^1$ is the following formula (III-2):

[Chemical Formula 45]

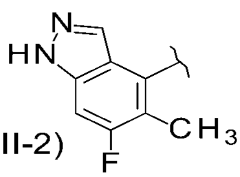
(III-2)

(6-1) The heterocyclic compound or the salt thereof according to (1-2), wherein

$R^2$ is $-V^1-V^2$ or W,
$V^1$ is a bond, $-CH_2-$, -O-, -S-, or $-N(R^{V1})-$,
$R^{V1}$ is H or optionally substituted $C_{1-3}$ alkyl,
$V^2$ is one group selected from the group consisting of the following formula (VI) and formula (VII):

[Chemical Formula 46]

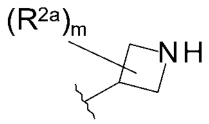

(VI)

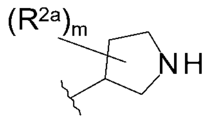

(VII)

W is one group selected from the group consisting of the following formula (VIII), formula (IX), formula (X), formula (XI), formula (XII), formula (XIII), formula (XIV), formula (XV) and formula (XVI):

[Chemical Formula 47]

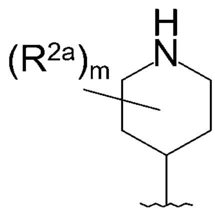

(VIII)

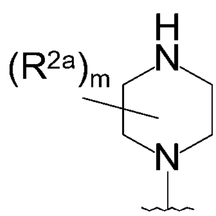

(IX)

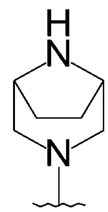

(X)

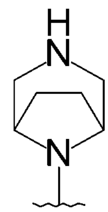

(XI)

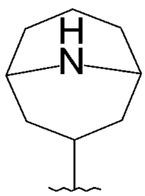

(XII)

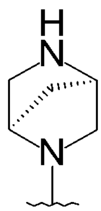

(XIII)

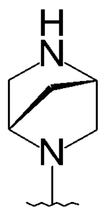

(XIV)

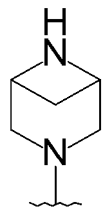

(XV)

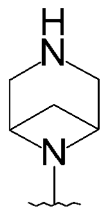

(XVI)

$R^{2a}$ are each independently OH, $OCH_3$, F, or optionally substituted $C_{1-3}$ alkyl, wherein $R^{2a}$ is bonded only to a carbon atom serving as a constituting atom of a ring selected from the group consisting of an azetidine ring represented by formula (VI), a pyrrolidine ring represented by formula (VII), a piperidine ring represented by formula (VIII), and a piperazine ring represented by formula (IX), and
m is an integer of 0 to 2.

(6-2) The heterocyclic compound or the salt thereof according to (1-2), wherein

$R^2$ is $-V^1-V^2$ or W,
$V^1$ is a bond, $-CH_2-$, -O-, -S-, or $-N(R^{V1})-$,
$R^{V1}$ is $C_{1-3}$ alkyl optionally substituted by one group selected from the group consisting of $C_{3-6}$ cycloalkyl, halogen, and -OH,
$V^2$ is one group selected from the group consisting of the following formula (VI-2) or (VII-2):

[Chemical Formula 48]

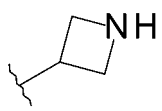

(VI-2)

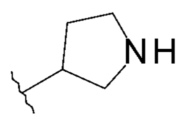

(VII-2)

and
W is one group selected from the group consisting of the following formula (VIII-2), formula (IX-2), formula (X), formula (XI), formula (XII), formula (XIII), formula (XIV), formula (XV), and formula (XVI):

[Chemical Formula 49]

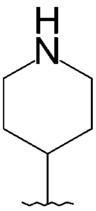
(VIII-2)

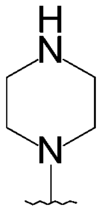
(IX-2)

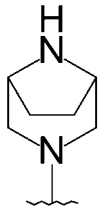
(X)

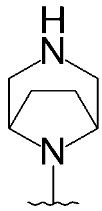
(XI)

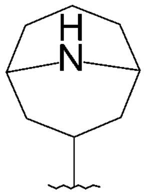
(XII)

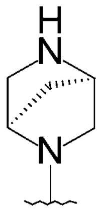
(XIII)

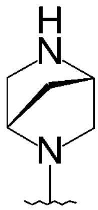
(XIV)

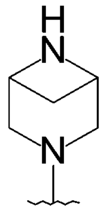
(XV)

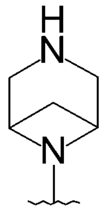
(XVI)

(6-3) The heterocyclic compound or the salt thereof according to (1-2), wherein

$R^2$ is $-V^1-V^2$ or W,
$V^1$ is a bond, -O- or $-N(R^{V1})-$,
$R^{V1}$ is $C_{1-3}$ alkyl,
$V^2$ is the following formula (VII-2):

[Chemical Formula 50]

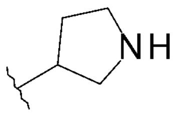
(VII-2)

and
W is the following formula (XIV):

[Chemical Formula 51]

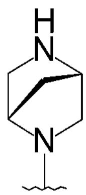
(XIV)

(6-4) The heterocyclic compound or the salt thereof according to (1-2), wherein

$R^2$ is $-V^1-V^2$ or W,
$V^1$ is -O- or $-N(CH_3)-$,
$V^2$ is the following formula (VII-2):

[Chemical Formula 52]

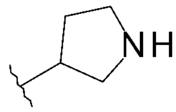

(VII-2)

and
W is the following formula (XIV):

[Chemical Formula 53]

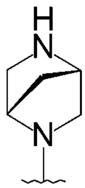

(XIV)

(6-5) The heterocyclic compound or the salt thereof according to (1-2), wherein

$R^2$ is $-V^1-V^2$ or W,
$V^1$ is -O-,
$V^2$ is the following formula (VII-2):

[Chemical Formula 54]

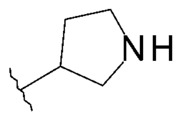

(VII-2)

and
W is the following formula (XIV):

[Chemical Formula 55]

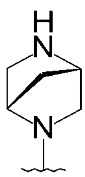

(XIV)

(6-6) The heterocyclic compound or the salt thereof according to (1-2), wherein

$R^2$ is $-V^1-V^2$ or W,
$V^1$ is $-N(CH_3)-$,
$V^2$ is the following formula (VII-2):

[Chemical Formula 56]

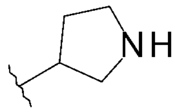

(VII-2)

and

W is the following formula (XIV):

[Chemical Formula 57]

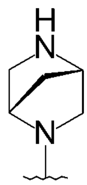

(XIV)

(6-7) The heterocyclic compound or the salt thereof according to (1-2), wherein

$R^2$ is $-V^1-V^2$ or W,
$V^1$ is a bond, $-CH_2-$, -O-, -S-, or $-N(R^{V1})-$,
$R^{V1}$ is $C_{1-3}$ alkyl optionally substituted by one group selected from the group consisting of $C_{3-6}$ cycloalkyl, halogen, and -OH,
$V^2$ is the following formula (VI-2) or (VII-2):

[Chemical Formula 58]

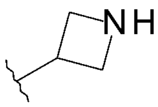

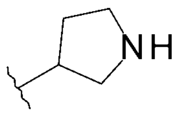

(VI-2) (VII-2)

and

W is one group selected from the group consisting of the following formulas (IX-2), (XIII), and (XIV):

[Chemical Formula 59]

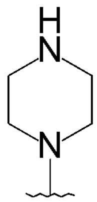

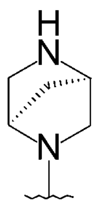

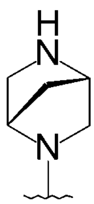

(IX-2) (XIII) (XIV)

(6-8) The heterocyclic compound or the salt thereof according to (1-2), wherein

$R^2$ is $-V^1-V^2$ or W,
$V^1$ is -O- or $-N(R^{V1})-$,
$R^{V1}$ is $C_{1-3}$ alkyl,
$V^2$ is the following formula (VI-2) or (VII-2):

[Chemical Formula 60]

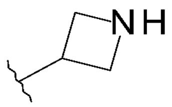 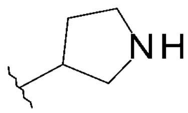

(VI-2) (VII-2)

and

W is one group selected from the group consisting of the following formulas (IX-2), (XIII), and (XIV):

[Chemical Formula 61]

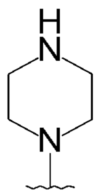 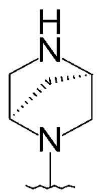 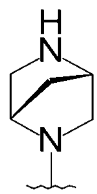

(IX-2) (XIII) (XIV)

(6-9) The heterocyclic compound or the salt thereof according to (1-2), wherein

$R^2$ is $-V^1-V^2$ or W,
$V^1$ is -O- or $-N(CH_3)-$,
$V^2$ is the following formula (VII-2):

[Chemical Formula 62]

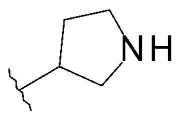

(VII-2)

and

W is the following formula (IX-2) or (XIV):

[Chemical Formula 63]

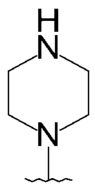 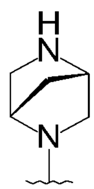

(IX-2) (XIV)

(6-10) The heterocyclic compound or the salt thereof according to (1-2), wherein

$R^2$ is $-V^1-V^2$ or W,
$V^1$ is a bond, $-CH_2-$, -O-, -S-, or $-N(R^{V1})-$,
$R^{V1}$ is H or optionally substituted $C_{1-3}$ alkyl,
$V^2$ is one group selected from the group consisting of the following formula (VI) and formula (VII):

[Chemical Formula 64]

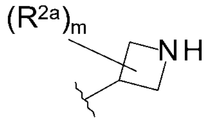 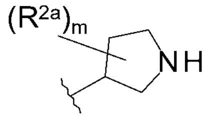

(VI) (VII)

W is the following formula (VIII), formula (IX), or one group selected from 7 to 9-membered bridged hetero cycloalkyl containing 1 or 2 nitrogen atoms:

[Chemical Formula 65]

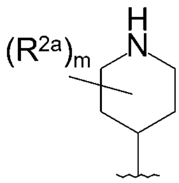 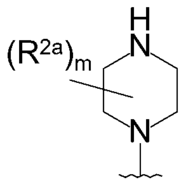

(VIII) (IX)

$R^{2a}$ are each independently OH, $OCH_3$, F, or optionally substituted $C_{1-3}$ alkyl, wherein $R^{2a}$ is bonded only to a carbon atom serving as a constituting atom of a ring selected from the group consisting of an azetidine ring represented by formula (VI), a pyrrolidine ring represented by formula (VII), a piperidine ring represented by formula (VIII), and a piperazine ring represented by formula (IX), and m is an integer of 0 to 2.

(6-11) The heterocyclic compound or the salt thereof according to (1-2), wherein

$R^2$ is $-V^1-V^2$ or W,
$V^1$ is a bond, $-CH_2-$, -O-, -S-, or $-N(R^{V1})-$,
$R^{V1}$ is H or optionally substituted $C_{1-3}$ alkyl,
$V^2$ is one group selected from the group consisting of the following formula (VI) and formula (VII):

[Chemical Formula 66]

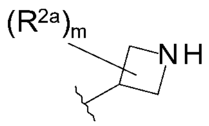 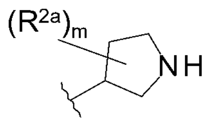

(VI) (VII)

W is one group selected from the group consisting of the following formula (VIII), formula (IX), formula (X), formula (XI), formula (XII-2), formula (XIII), formula (XIV), formula (XV), and formula (XVI):

[Chemical Formula 67]

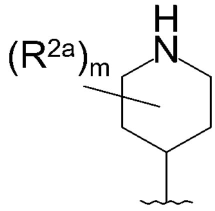
(VIII)

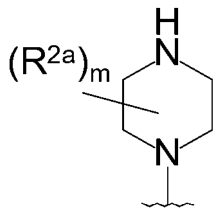
(IX)

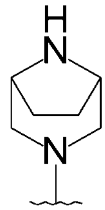
(X)

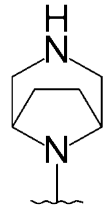
(XI)

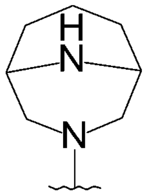
(XII-2)

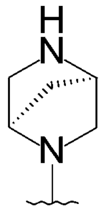
(XIII)

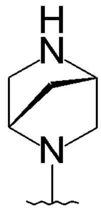
(XIV)

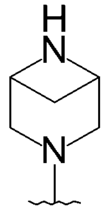
(XV)

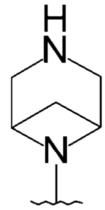
(XVI)

$R^{2a}$ are each independently OH, $OCH_3$, F, or optionally substituted $C_{1-3}$ alkyl, wherein $R^{2a}$ is bonded only to a carbon atom serving as a constituting atom of a ring selected from the group consisting of an azetidine ring represented by formula (VI), a pyrrolidine ring represented by formula (VII), a piperidine ring represented by formula (VIII), and a piperazine ring represented by formula (IX), and
m is an integer of 0 to 2.

(6-12) The heterocyclic compound or the salt thereof according to (1-2), wherein

$R^2$ is $-V^1-V^2$ or W,
$V^1$ is a bond, $-CH_2-$, -O-, -S-, or $-N(R^{V1})-$,
$R^{V1}$ is $C_{1-3}$ alkyl optionally substituted by one group selected from the group consisting of $C_{3-6}$ cycloalkyl, halogen, and -OH,
$V^2$ is one group selected from the group consisting of the following formulas (VI-2) and (VII-2):

[Chemical Formula 68]

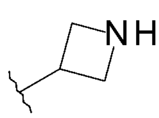
(VI-2)

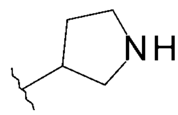
(VII-2)

and
W is one group selected from the group consisting of the following formula (VIII-2), formula (IX-2), formula (X), formula (XI), formula (XII-2), formula (XIII), formula (XIV), formula (XV), and formula (XVI):

[Chemical Formula 69]

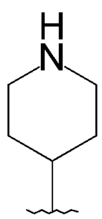
(VIII-2)
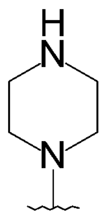
(IX-2)
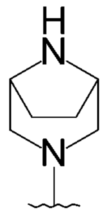
(X)
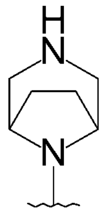
(XI)
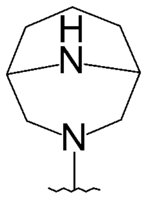
(XII-2)
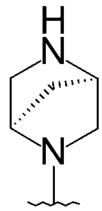
(XIII)
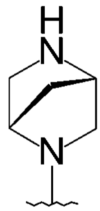
(XIV)
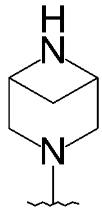
(XV)
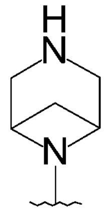
(XVI)

(6-13) The heterocyclic compound or the salt thereof according to (1-2), wherein $R^2$ is 7 to 9-membered bridged hetero cycloalkyl containing 1 or 2 nitrogen atoms.
(7-1) The heterocyclic compound or the salt thereof according to (1-2), wherein $R^3$ is optionally substituted $C_{1-6}$ alkyl, optionally substituted hetero cycloalkyl, or optionally substituted heteroaryl.
(7-2) The heterocyclic compound or the salt thereof according to (1-2), wherein $R^3$ is $C_{1-6}$ alkyl optionally substituted by one group selected from the group consisting of -O-($C_{1-6}$ alkyl), -S-($C_{1-6}$ alkyl), -N-($C_{1-6}$ alkyl)$_2$, and hetero cycloalkyl, or optionally substituted hetero cycloalkyl.
(7-3) The heterocyclic compound or the salt thereof according to (1-2), wherein $R^3$ is $C_{1-6}$ alkyl optionally substituted by one group selected from the group consisting of -O($C_{1-6}$ alkyl), oxetanyl, tetrahydrofuranyl, and tetrahydropyranyl, oxetanyl, tetrahydrofuranyl, or tetrahydropyranyl.
(7-4) The heterocyclic compound or the salt thereof according to (1-2), wherein $R^3$ is tetrahydrofuranyl, tetrahydropyranyl, or $C_{1-3}$ alkyl optionally substituted by $-OCH_3$ or tetrahydrofuranyl.
(7-5) The heterocyclic compound or the salt thereof according to (1-2), wherein $R^3$ is n-propyl optionally substituted by $-OCH_3$, or tetrahydropyranyl.
(8-1) The heterocyclic compound or the salt thereof according to (1-2), wherein X is a bond, $-CH_2-$, -O-, -S-, or $-NR^{4X}-$, and $R^{4X}$ is H or optionally substituted $C_{1-3}$ alkyl.
(8-2) The heterocyclic compound or the salt thereof according to (1-2), wherein X is -O- or $-NR^{4X}-$, and $R^{4X}$ is $C_{1-3}$ alkyl.
(8-3) The heterocyclic compound or the salt thereof according to (1-2), wherein X is -O-.
(9-1) The heterocyclic compound or the salt thereof according to (1-2), wherein $Y^1$ is -O-(optionally substituted $C_{1-3}$ alkylene)-$^{*Y2}$, -S-(optionally substituted $C_{1-3}$ alkylene)-$^{*Y2}$, $-SO_2$-(optionally substituted $C_{1-3}$ alkylene)-$^{*Y2}$, $-NR^Y$-(optionally substituted $C_{1-3}$ alkylene)-$^{*Y2}$, -(optionally substituted $C_{1-3}$ alkylene)-O-$^{*Y2}$, -(optionally substituted $C_{1-3}$ alkylene)-S-$^{*Y2}$, -(optionally substituted $C_{1-3}$ alkylene)-$SO_2$-$^{*Y2}$, or -(optionally substituted $C_{1-3}$ alkylene)-$NR^Y$-$^{*Y2}$ (wherein $^{*Y2}$ represents a site of connection to $Y^2$), and $R^Y$ is H or optionally substituted $C_{1-3}$ alkyl.
(9-2) The heterocyclic compound or the salt thereof according to (1-2), wherein $Y^1$ is -O-($C_{1-3}$ alkylene)-$^{*Y2}$ or -($C_{1-3}$ alkylene)-O-$^{*Y2}$ (wherein $^{*Y2}$ represents a site of connection to $Y^2$).
(9-3) The heterocyclic compound or the salt thereof according to (1-2), wherein $Y^1$ is -O-($C_{1-3}$ alkylene)-$^{*Y2}$ (wherein $^{*Y2}$ represents a site of connection to $Y^2$).
(9-4) The heterocyclic compound or the salt thereof according to (1-2), wherein $Y^1$ is -O-(methylene)-$^{*Y2}$ (wherein $^{*Y2}$ represents a site of connection to $Y^2$).
(10-1) The heterocyclic compound or the salt thereof according to (1-2), wherein $Y^2$ is a bond, optionally substituted phenylene, or optionally substituted heteroarylene.
(10-2) The heterocyclic compound or the salt thereof according to (1-2), wherein $Y^2$ is phenylene or pyridinediyl.
(10-3) The heterocyclic compound or the salt thereof according to (1-2), wherein $Y^2$ is phenylene optionally substituted by fluorine.
(10-4) The heterocyclic compound or the salt thereof according to (1-2), wherein $Y^2$ is phenylene.
(10-5) The heterocyclic compound or the salt thereof according to (1-2), wherein $Y^2$ is optionally substituted

phenylene or pyridinediyl.

(11-1) The heterocyclic compound or the salt thereof according to (1-2), wherein $L^P$ is a group that chemically bonds $Y^2$ to EUB.

(11-2) The heterocyclic compound or the salt thereof according to (1-2), wherein

$L^P$ is $-(L^1-L^2-L^3-L^4)-$,
$L^1$, $L^2$, $L^3$, and $L^4$ are each independently one group selected from the group consisting of a bond, C=O, -O-, -S-, $-SO_2-$, $-NR^L-$, acetylene-1,2-diyl, optionally substituted hetero cycloalkylene, optionally substituted heteroarylene, saturated 7- to 9-membered spiro hetero cycloalkylene containing 1 or 2 nitrogen atoms, saturated 7- to 9-membered bridged hetero cycloalkylene containing 2 nitrogen atoms, and optionally substituted $C_{1-6}$ alkylene, and $R^L$ is H or $C_{1-6}$ alkyl.

(11-3) The heterocyclic compound or the salt thereof according to (1-2), wherein

$L^P$ is $-(L^1-L^2-L^3-L^4)-$,
$L^1$ is C=O,
$L^2$ is piperidinediyl optionally substituted by $C_{1-3}$ alkyl, piperazinediyl optionally substituted by $C_{1-3}$ alkyl, pyrrolidinediyl optionally substituted by $C_{1-3}$ alkyl, bridged piperazinediyl, or 2,6-diazaspiro[3.4]octanediyl,
$L^3$ is a bond, $-N(R^{L3})-$, $C_{1-3}$ alkylene, or piperazinediyl,
$L^4$ is a bond, $-N(R^{L4})-$, -O-, piperazinediyl, or $C_{1-3}$ alkylene,
$R^{L3}$ is H or $C_{1-3}$ alkyl, and
$R^{L4}$ is H or $C_{1-3}$ alkyl.

(11-4) The heterocyclic compound or the salt thereof according to (1-2), wherein $L^P$ is one group selected from the group consisting of the following formulas (L-1), (L-2), (L-3), (L-4), (L-5), (L-6), and (L-7), wherein

C=O in formulas (L-1), (L-2), (L-3), (L-4), (L-5), (L-6), and (L-7) forms a bond with $Y^2$:

[Chemical Formula 70]

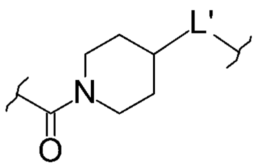
(L−1)

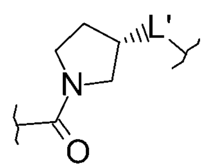
(L−2)

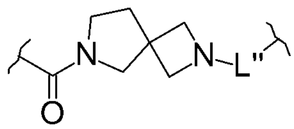
(L−3)

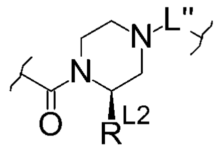
(L−4)

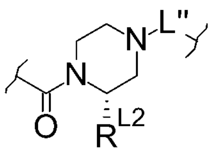
(L−5)

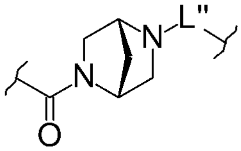
(L−6)

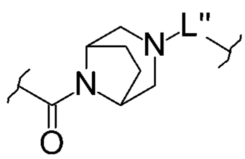
(L−7)

L' is -O-, $-(C_{1-3}$ alkylene)-NH-, $-N(CH_3)(C_{1-3}$ alkylene)-, piperazinediyl, or $-(C_{1-3}$ alkylene)-piperazinediyl,
L'' is a bond, $C_{1-3}$ alkylene, or $-(C_{1-3}$ alkylene)-O-, and
$R^{L2}$ is H or $C_{1-3}$ alkyl.

(11-5) The heterocyclic compound or the salt thereof according to (1-2), wherein $L^P$ is one group selected from the group consisting of the following formulas (L-1), (L-2), (L-3), (L-4), (L-5), and (L-7), wherein

C=O in formulas (L-1), (L-2), (L-3), (L-4), (L-5), and (L-7) forms a bond with $Y^2$:

[Chemical Formula 71]

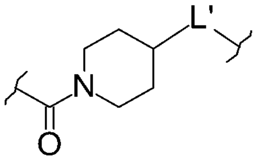

(L−1)

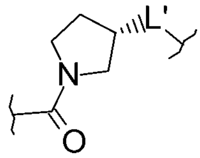

(L−2)

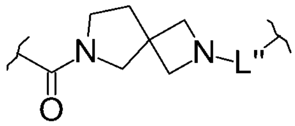

(L−3)

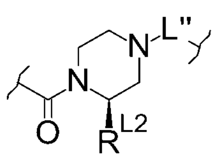

(L−4)

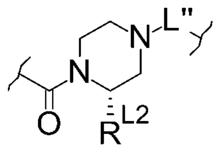

(L−5)

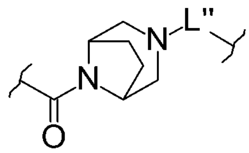

(L−7)

L' is -O-, -($C_{1-3}$ alkylene)-NH-, -N($CH_3$)($C_{1-3}$ alkylene)-, piperazinediyl, or -($C_{1-3}$ alkylene)-piperazinediyl,
L'' is a bond, $C_{1-3}$ alkylene, or -($C_{1-3}$ alkylene)-O-, and
$R^{L2}$ is H or $C_{1-3}$ alkyl.

(11-6) The heterocyclic compound or the salt thereof according to (1-2), wherein $L^P$ is the following formula (L-5A) or (L-7A), wherein C=O in formulas (L-5A) and (L-7A) forms a bond with $Y^2$:

[Chemical Formula 72]

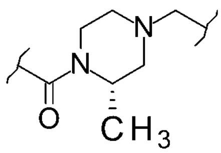

(L−5A)

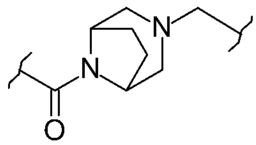

(L−7A)

(11-7) The heterocyclic compound or the salt thereof according to (1-2), wherein $L^P$ is the following formula (L-5A), wherein C=O in formula (L-5A) forms a bond with $Y^2$:

[Chemical Formula 73]

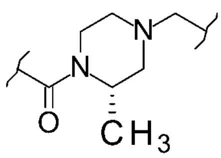

(L−5A)

(11-8) The heterocyclic compound or the salt thereof according to (1-2), wherein

$L^P$ is -$L^V$-J-,
$L^V$ is -($L^5$-$L^6$-$L^7$-$L^8$)-,
$L^5$, $L^6$, $L^7$, and $L^8$ are each independently a group selected from the group consisting of a bond, -O-, -$NR^{L5}$-, optionally substituted pyrrolidinediyl, optionally substituted piperidinediyl, optionally substituted piperazinediyl, optionally substituted $C_{1-3}$ alkylene, and C=O,
$R^{L5}$ is H or $C_{1-3}$ alkyl, and
J is NH or 5-membered heteroarylene containing 1 to 4 heteroatoms selected from the group consisting of oxygen, sulfur, and nitrogen.

(12-1) The heterocyclic compound or the salt thereof according to (11-8), wherein

$L^V$ is $-(L^5-L^6-L^7-L^8)-$,
$L^5$, $L^6$, $L^7$, and $L^8$ are each independently a group selected from the group consisting of a bond, -O-, $-NR^{L5}-$, optionally substituted pyrrolidinediyl, optionally substituted piperidinediyl, optionally substituted piperazinediyl, optionally substituted $C_{1-3}$ alkylene, and C=O, and
$R^{L5}$ is H or $C_{1-3}$ alkyl.

(12-2) The heterocyclic compound or the salt thereof according to (11-8), wherein $L^V$ is a bond, $C_{1-3}$ alkylene, C=O, or a group selected from the group consisting of the following formula (L-10), formula (L-11), formula (L-12), formula (L-13), formula (L-14), and formula (L-15) (wherein $^{*Y2}$ represents a site of connection to $Y^2$):

[Chemical Formula 74]

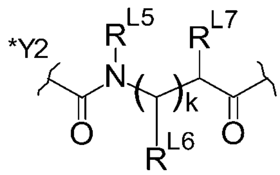
(L-10)
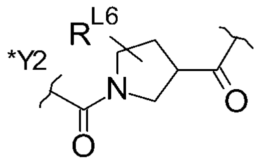
(L-11)
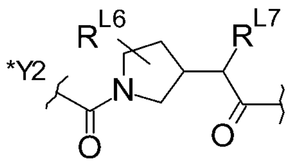
(L-12)
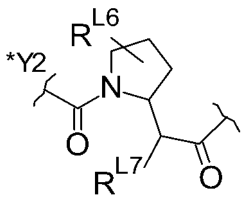
(L-13)
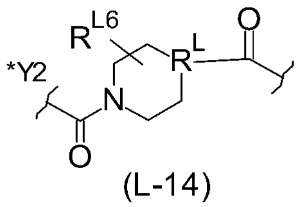
(L-14)
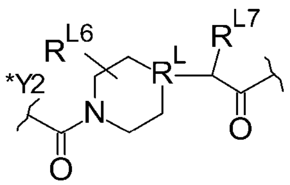
(L-15)

$R^{L5}$ is H or $C_{1-3}$ alkyl,
$R^{L6}$ and $R^{L7}$ are each independently H, F, OH, $OCH_3$, or optionally substituted $C_{1-3}$ alkyl,
$R^L$ is CH or N, and
k is an integer of 1 to 2.

(12-3) The heterocyclic compound or the salt thereof according to (11-8), wherein $L^V$ is a bond.
(13-1) The heterocyclic compound or the salt thereof according to (11-8), wherein J is NH or 5-membered heteroarylene containing 1 to 4 heteroatoms selected from the group consisting of oxygen, sulfur, and nitrogen.
(13-2) The heterocyclic compound or the salt thereof according to (11-8), wherein J is NH or a group selected from the group consisting of the following formula (L-16), formula (L-17), formula (L-18), and formula (L-19) (wherein $^{*LV}$ represents a site of connection to $L^V$):

[Chemical Formula 75]

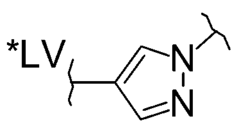
(L-16)
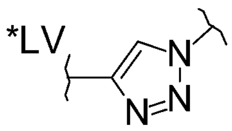
(L-17)
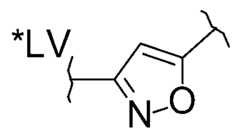
(L-18)
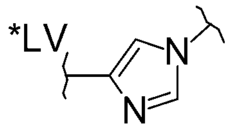
(L-19)

(13-3) The heterocyclic compound or the salt thereof according to (11-8), wherein J is the following formula (L-17) (wherein $^{*LV}$ represents a site of connection to $L^V$):

[Chemical Formula 76]

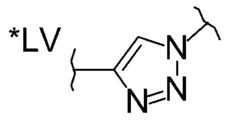

(L-17)

(14-1) The heterocyclic compound or the salt thereof according to (11-8), wherein - $Y^2$-$L^V$-J is the following formula (L-20) (wherein $^{*Y1}$ represents a site at which $Y^2$-$L^V$-J is bonded to the carbon atom of -O-$CH_2$- when $Y^1$ is -O-$CH_2$-):

[Chemical Formula 77]

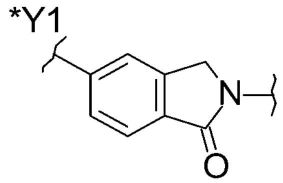

(L-20)

(15-1) The heterocyclic compound or the salt thereof according to (1-2), wherein EUB is a group having the ability to bind to one E3 ubiquitin ligase selected from the group consisting of VHL, celebron, IAP, MDM2, DCAF11, DCAF15, DCAF16, BIRC2, KEAP1, RNF4, RNF114, FEM1B, and AhR.
(15-2) The heterocyclic compound or the salt thereof according to (1-2), wherein EUB is a group having the ability to bind to one E3 ubiquitin ligase selected from the group consisting of VHL and celebron.
(15-2-1) A heterocyclic compound or a salt thereof, wherein EUB is a combination of embodiments described in (15-5) and (15-8) below.
(15-3) The heterocyclic compound or the salt thereof according to (1-2), wherein EUB is a group having the ability to bind to VHL.
(15-4) The heterocyclic compound or the salt thereof according to (1-2), wherein EUB is a group having the ability to bind to celebron.
(15-5) The heterocyclic compound or the salt thereof according to (1-2), wherein EUB is a group having the ability to bind to celebron, and the group having the ability to bind to celebron is the following formula (E-1):

[Chemical Formula 78]

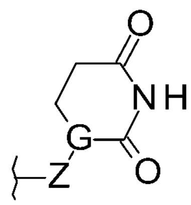

(E-1)

G is $CR^G$ or N,
$R^G$ is H or $C_{1-6}$ alkyl,
Z is one group selected from the group consisting of the following formulas (Z-1), (Z-2), (Z-3), (Z-4), (Z-5), (Z-6), (Z-7), (Z-8), (Z-9), (Z-10), (Z-11), (Z-12), (Z-13), (Z-14), (Z-15), (Z-16), (Z-17), (Z-18), (Z-19), (Z-20), (Z-21), (Z-22), (Z-23), (Z-24), (Z-25), (Z-26), and (Z-27), wherein
ring B1 and ring B2 in formulas (Z-1), (Z-2), (Z-3), (Z-4), (Z-5), (Z-6), (Z-7), (Z-8), (Z-9), (Z-10), (Z-11), (Z-12), (Z-13), (Z-14), (Z-15), (Z-16), (Z-17), (Z-18), (Z-19), (Z-20), (Z-21), (Z-22), (Z-23), (Z-24), (Z-25), (Z-26) and (Z-27) form a bond with $L^P$,
on the proviso that when G is N, Z is one group selected from the group consisting of formulas (Z-1), (Z-16), (Z-17), (Z-18), (Z-19), (Z-20), (Z-21), (Z-22), (Z-23), (Z-24), (Z-25), (Z-26), and (Z-27):

[Chemical Formula 79-1]

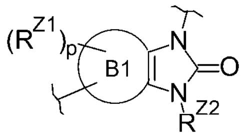
(Z-1)

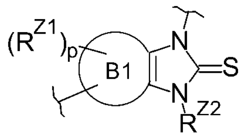
(Z-2)

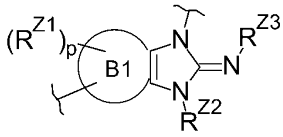
(Z-3)

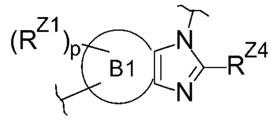
(Z-4)

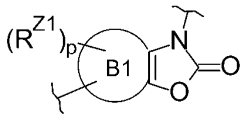
(Z-5)

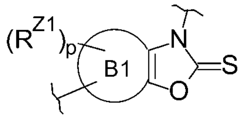
(Z-6)

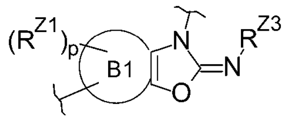
(Z-7)

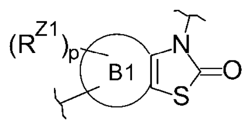
(Z-8)

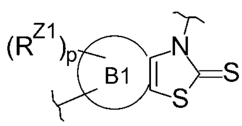
(Z-9)

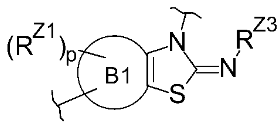
(Z-10)

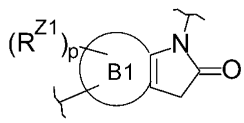
(Z-11)

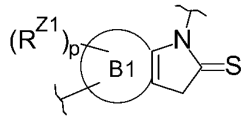
(Z-12)

[Chemical Formula 79-2]

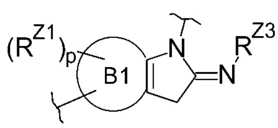
(Z-13)

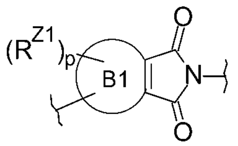
(Z-14)

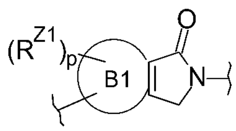
(Z-15)

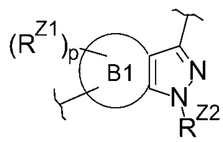
(Z-16)

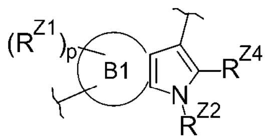
(Z-17)

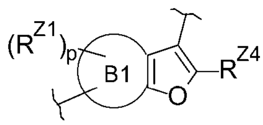
(Z-18)

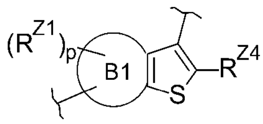
(Z-19)

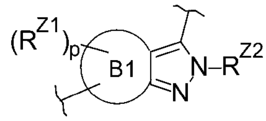
(Z-20)

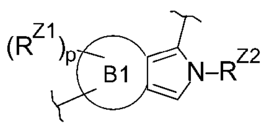
(Z-21)

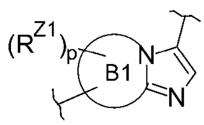
(Z-22)

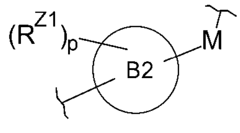
(Z-23)

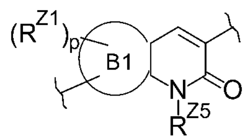
(Z-24)

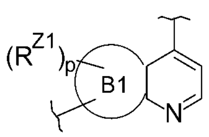
(Z-25)

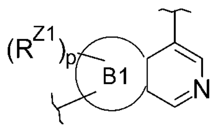
(Z-26)

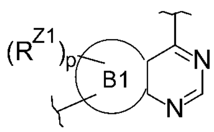
(Z-27)

$R^{Z1}$ are each independently optionally substituted $C_{1-6}$ alkyl, halogen, cyano, -OH, -O-(optionally substituted $C_{1-6}$ alkyl), -S-(optionally substituted $C_{1-6}$ alkyl), -NH-(optionally substituted $C_{1-6}$ alkyl), or -N-(optionally substituted $C_{1-6}$ alkyl)$_2$,
p is an integer of 0 to 2,
$R^{Z2}$, $R^{Z3}$, $R^{Z4}$, and $R^{Z5}$ are each independently H or optionally substituted $C_{1-6}$ alkyl,
M is a bond, -O-, -S-, -N($R^M$)-, or optionally substituted $C_{1-3}$ alkylene,
$R^M$ is H or optionally substituted $C_{1-3}$ alkyl,

ring B1 is a benzene ring or 6-membered heterocyclic ring, wherein
$R^{Z1}$ and $L^P$ form bonds with the carbon atoms constituting ring B1, and
ring B2 is a benzene ring or 5- or 6-membered heterocyclic ring,
wherein M, $R^{Z1}$, and $L^P$ form bonds with the carbon atoms constituting ring B2.

(15-6) The heterocyclic compound or the salt thereof according to (1-2), wherein EUB is a group having the ability to bind to celebron, and the group having the ability to bind to celebron is the following formula (E-1):

[Chemical Formula 80]

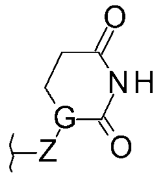

(E-1)

G is CH or N, and
Z is one group selected from the group consisting of the following formulas (Z-1A), (Z-1B), (Z-5A), (Z-5B), (Z-14A), (Z-14B), (Z-14C), (Z-15A), (Z-16A), (Z-16B), (Z-16C), (Z-16D), (Z-20A), (Z-22A), (Z-23A), (Z-23B), (Z-23C), (Z-23D), (Z-23E), (Z-23F), and (Z-24A):

[Chemical Formula 81]

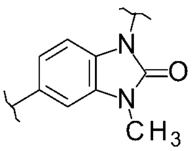
(Z−1A)

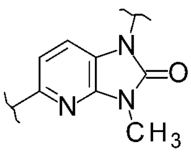
(Z−1B)

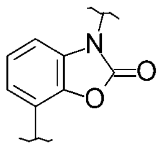
(Z−5A)

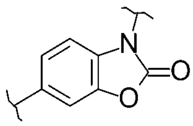
(Z−5B)

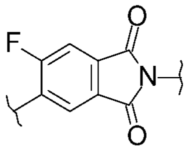
(Z−14A)

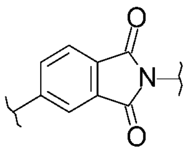
(Z−14B)

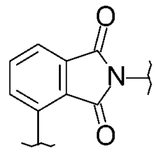
(Z−14C)

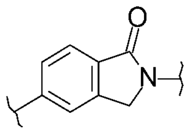
(Z−15A)

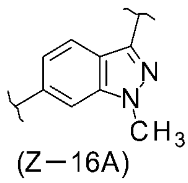
(Z−16A)

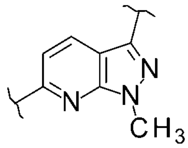
(Z−16B)

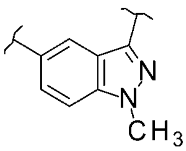
(Z−16C)

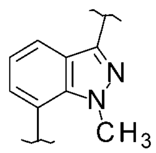
(Z−16D)

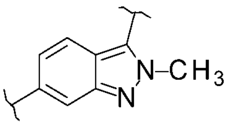
(Z−20A)

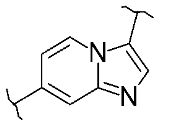
(Z−22A)

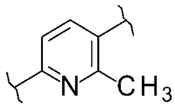
(Z−23A)

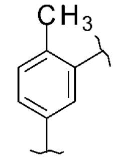
(Z−23B)

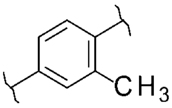
(Z−23C)

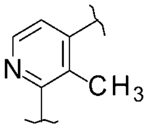
(Z−23D)

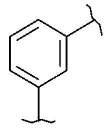
(Z−23E)

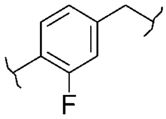
(Z−23F)

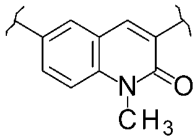
(Z−24A)

wherein the benzene ring or the 6-membered heterocyclic ring in formulas (Z-1A), (Z-1B), (Z-5A), (Z-5B), (Z-14A), (Z-14B), (Z-14C), (Z-15A), (Z-16A), (Z-16B), (Z-16C), (Z-16D), (Z-20A), (Z-22A), (Z-23A), (Z-23B), (Z-23C), (Z-23D), (Z-23E), and (Z-23F) forms a bond with $L^P$, and the benzene ring in (Z-24A) forms a bond with $L^P$, on the proviso that when G is N, Z is one group selected from the group consisting of (Z-1A), (Z-16A), (Z-16B), (Z-16C), (Z-16D), (Z-20A), (Z-22A), (Z-23A), (Z-23B), (Z-23C), (Z-23D), (Z-23E), (Z-23F), and (Z-24A).

(15-7) The heterocyclic compound or the salt thereof according to (1-2), wherein EUB is a group having the ability to bind to celebron, and the group having the ability to bind to celebron is the following formula (E-1):

[Chemical Formula 82]

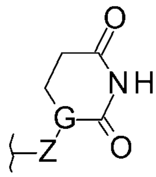

(E-1)

G is CH or N, and
Z is one group selected from the group consisting of the following formulas (Z-1A) and (Z-16A):

[Chemical Formula 83]

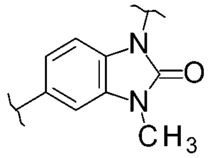

(Z−1A)

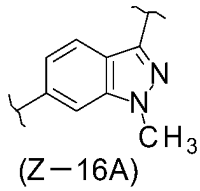

(Z−16A)

wherein the benzene ring in formulas (Z-1A) and (Z-16A) forms a bond with $L^P$.

(15-8) The heterocyclic compound or the salt thereof according to (1-2), wherein EUB is a group having the ability to bind to VHL, and the group having the ability to bind to VHL is the following formula (E-2):

[Chemical Formula 84]

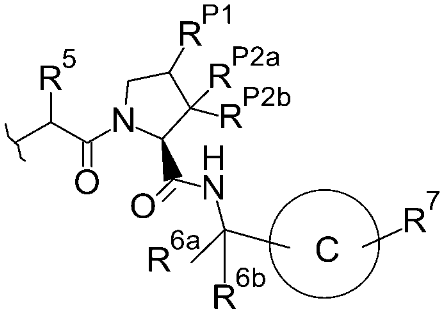

(E-2)

$R^5$ is optionally substituted $C_{1-6}$ alkyl, optionally substituted $C_{3-6}$ cycloalkyl, or optionally substituted 4- to 6-membered hetero cycloalkyl containing one heteroatom selected from the group consisting of oxygen, sulfur, and nitrogen,
$R^{6a}$ and $R^{6b}$ are each independently H or optionally substituted $C_{1-6}$ alkyl, or $R^{6a}$ and $R^{6b}$ optionally form optionally substituted $C_{3-6}$ cycloalkyl, or optionally substituted 4- to 6-membered hetero cycloalkyl containing one heteroatom selected from the group consisting of oxygen, sulfur, and nitrogen, together with carbon bonded thereto,
$R^7$ is H, halogen, $C_{1-3}$ alkyl, $-SO_2CH_3$, $C_{3-6}$ cycloalkyl, optionally substituted 4- to 6-membered hetero cycloalkyl containing 1 or 2 heteroatoms selected from the group consisting of oxygen, sulfur, and nitrogen, optionally substituted 5-membered heteroaryl containing 1 to 4 heteroatoms selected from the group consisting of oxygen, sulfur, and nitrogen, or 6-membered heteroaryl containing 1 to 3 nitrogen atoms,
$R^{P1}$ is OH or F,
$R^{P2a}$ is H or F,
$R^{P2b}$ is H, and

cyclic group C is optionally substituted phenylene or optionally substituted 6-membered heteroarylene containing 1 to 3 nitrogen atoms.

(15-9) The heterocyclic compound or the salt thereof according to (1-2), wherein EUB is a group having the ability to bind to VHL, and the group having the ability to bind to VHL is the following formula (E-2):

[Chemical Formula 85]

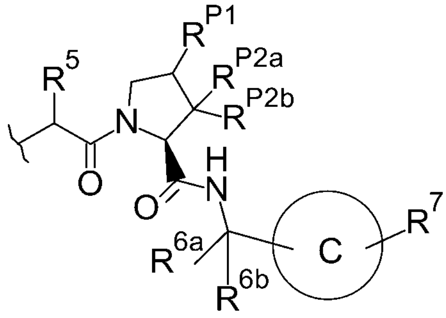

(E-2)

$R^5$ is isopropyl,
$R^{6a}$ is H, $R^{6b}$ is hydroxymethyl or $-CH_2-OP(=O)(OH)_2$,
$R^7$ is a group selected from the group consisting of the following formula (XVII-2), formula (XVIII-2), formula (XIX-2), formula (XIX-3), formula (XX), and formula (XXII-2):

[Chemical Formula 86]

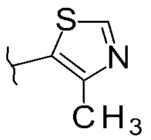

(XVII-2)

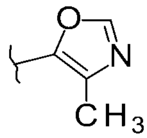

(XVIII-2)

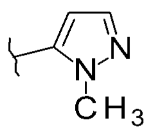

(XIX-2)

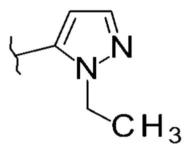

(XIX-3)

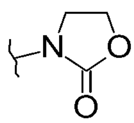

(XX)

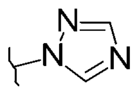

(XXII-2)

$R^{P1}$ is OH,
$R^{P2a}$ is H,
$R^{P2b}$ is H,
cyclic group C is the following formula (XXVII) (wherein $^{*R7}$ represents a site of connection to $R^7$):

[Chemical Formula 87]

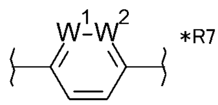

(XXVII)

and
both of $W^1$ and $W^2$ are CH.

(15-10) The heterocyclic compound or the salt thereof according to (15-8), wherein

$R^{P1}$ is OH,
$R^{P2a}$ is H, and
$R^{P2b}$ is H.

(16-1) The heterocyclic compound or the salt thereof according to (15-8), wherein $R^5$ is optionally substituted $C_{1-6}$

alkyl, optionally substituted $C_{3-6}$ cycloalkyl, or optionally substituted 4- to 6-membered hetero cycloalkyl containing one heteroatom selected from the group consisting of oxygen, sulfur, and nitrogen.

(16-2) The heterocyclic compound or the salt thereof according to (15-8), wherein $R^5$ is methyl, ethyl, isopropyl, tert-butyl, or $C_{3-6}$ cycloalkyl.

(16-3) The heterocyclic compound or the salt thereof according to (15-8), wherein $R^5$ is ethyl, isopropyl, tert-butyl, or $C_{3-6}$ cycloalkyl.

(16-4) The heterocyclic compound or the salt thereof according to (15-8), wherein $R^5$ is isopropyl or $C_{3-6}$ cycloalkyl.

(16-5) The heterocyclic compound or the salt thereof according to (15-8), wherein $R^5$ is isopropyl.

(17-1) The heterocyclic compound or the salt thereof according to (15-8), wherein $R^{6a}$ and $R^{6b}$ are each independently H or optionally substituted $C_{1-6}$ alkyl, or $R^{6a}$ and $R^{6b}$ optionally form optionally substituted $C_{3-6}$ cycloalkyl, or optionally substituted 4- to 6-membered hetero cycloalkyl containing one heteroatom selected from the group consisting of oxygen, sulfur, and nitrogen, together with carbon bonded thereto.

(17-2) The heterocyclic compound or the salt thereof according to (15-8), wherein $R^{6a}$ and $R^{6b}$ are each independently H or $C_{1-3}$ alkyl, wherein the $C_{1-3}$ alkyl is optionally substituted by a group selected from the group consisting of F, OH, $OCH_3$, and $N(CH_3)_2$, or $R^{6a}$ and $R^{6b}$ optionally form $C_{3-6}$ cycloalkyl together with carbon bonded thereto.

(17-3) The heterocyclic compound or the salt thereof according to (15-8), wherein $R^{6a}$ and $R^{6b}$ are each independently H or $C_{1-3}$ alkyl, wherein the $C_{1-3}$ alkyl is optionally substituted by a group selected from the group consisting of F, OH, and $N(CH_3)_2$, or $R^{6a}$ and $R^{6b}$ optionally form cyclopropyl together with carbon bonded thereto.

(17-4) The heterocyclic compound or the salt thereof according to (15-8), wherein $R^{6a}$ is H, and $R^{6b}$ is $C_{1-3}$ alkyl optionally substituted by OH.

(17-5) The heterocyclic compound or the salt thereof according to (15-8), wherein $R^{6a}$ is H, and $R^{6b}$ is hydroxymethyl.

(17-6) The heterocyclic compound or the salt thereof according to (15-8), wherein $R^{6a}$ is H, and $R^{6b}$ is $C_{1-3}$ alkyl optionally substituted by OH or $OP(=O)(OH)_2$.

(17-7) The heterocyclic compound or the salt thereof according to (15-8), wherein $R^{6a}$ is H, and $R^{6b}$ is hydroxymethyl or $-CH_2-OP(=O)(OH)_2$.

(17-8) The heterocyclic compound or the salt thereof according to (15-8), wherein $R^{6a}$ is H, and $R^{6b}$ is $-CH_2-OP(=O)(OH)_2$.

(18-1) The heterocyclic compound or the salt thereof according to (15-8), wherein $R^7$ is H, halogen, $C_{1-3}$ alkyl, $-SO_2CH_3$, $C_{3-6}$ cycloalkyl, optionally substituted 4- to 6-membered hetero cycloalkyl containing 1 or 2 heteroatoms selected from the group consisting of oxygen, sulfur, and nitrogen, optionally substituted 5-membered heteroaryl containing 1 to 4 heteroatoms selected from the group consisting of oxygen, sulfur, and nitrogen, or 6-membered heteroaryl containing 1 to 3 nitrogen atoms.

(18-2) The heterocyclic compound or the salt thereof according to (15-8), wherein $R^7$ is H, halogen, $C_{1-3}$ alkyl, $-SO_2CH_3$, $C_{3-6}$ cycloalkyl, or a group selected from the group consisting of the following formula (XVII), formula (XVIII), formula (XIX), formula (XX), formula (XXI), formula (XXII), formula (XXIII), formula (XXIV), formula (XXV), and formula (XXVI):

[Chemical Formula 88]

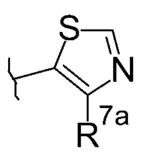
(XVII)

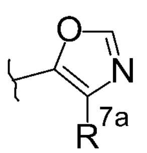
(XVIII)

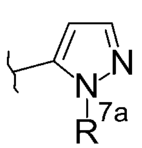
(XIX)

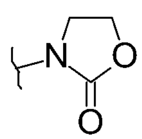
(XX)

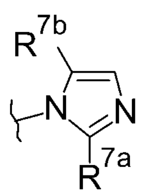
(XXI)

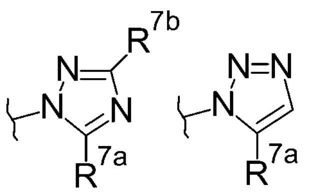
(XXII) (XXIII)

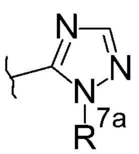
(XXIV)

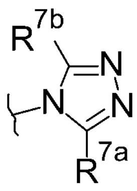
(XXV)

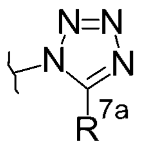
(XXVI)

and

$R^{7a}$ and $R^{7b}$ are each independently H, or $C_{1-3}$ alkyl optionally substituted by OH.

(18-3) The heterocyclic compound or the salt thereof according to (15-8), wherein $R^7$ is halogen, or a group selected from the group consisting of the following formula (XVII), formula (XVIII), formula (XIX), formula (XX), formula (XXI), and formula (XXII):

[Chemical Formula 89]

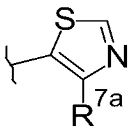
(XVII)
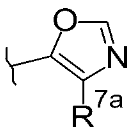
(XVIII)
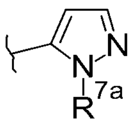
(XIX)
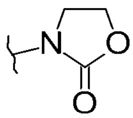
(XX)
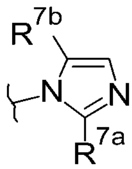
(XXI)
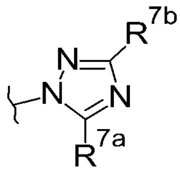
(XXII)

and
$R^{7a}$ and $R^{7b}$ are each independently H, or $C_{1-3}$ alkyl optionally substituted by OH.
(18-4) The heterocyclic compound or the salt thereof according to (15-8), wherein $R^7$ is the following formula (XVII), formula (XVIII), formula (XIX), or formula (XX):

[Chemical Formula 90]

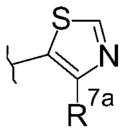
(XVII)
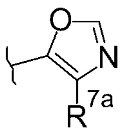
(XVIII)
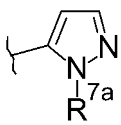
(XIX)
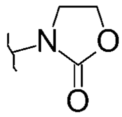
(XX)

and
$R^{7a}$ is $C_{1-3}$ alkyl optionally substituted by OH.
(18-5) The heterocyclic compound or the salt thereof according to (15-8), wherein $R^7$ is the following formula (XVII) or formula (XIX):

[Chemical Formula 91]

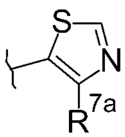
(XVII)
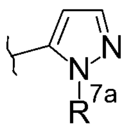
(XIX)

and
$R^{7a}$ is $C_{1-3}$ alkyl.
(18-6) The heterocyclic compound or the salt thereof according to (15-8), wherein $R^7$ is the following formula (XVII):

[Chemical Formula 92]

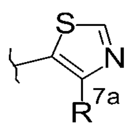
(XVII)

and
$R^{7a}$ is $C_{1-3}$ alkyl.
(18-7) The heterocyclic compound or the salt thereof according to (15-8), wherein $R^7$ is H.
(18-8) The heterocyclic compound or the salt thereof according to (15-8), wherein $R^7$ is a group selected from the group consisting of the following formula (XVII-2), formula (XVIII-2), formula (XIX-2), formula (XIX-3), formula (XX),

and formula (XXII-2):

[Chemical Formula 93]

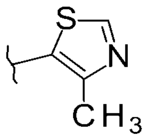
(XVII-2)

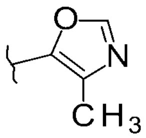
(XVIII-2)

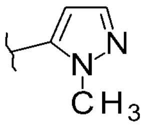
(XIX-2)

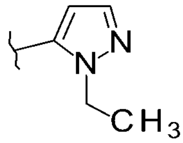
(XIX-3)

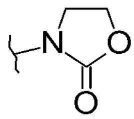
(XX)

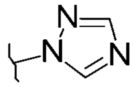
(XXII-2)

(19-1) The heterocyclic compound or the salt thereof according to (15-8), wherein cyclic group C is optionally substituted phenylene, or optionally substituted 6-membered heteroarylene containing 1 to 3 nitrogen atoms.

(19-2) The heterocyclic compound or the salt thereof according to (15-8), wherein cyclic group C is the following formula (XXVII) (wherein $^{*R7}$ represents a site of connection to $R^7$):

[Chemical Formula 94]

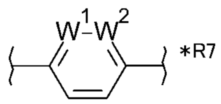
(XXVII)

and as for $W^1$ and $W^2$,

(i) $W^1$ is CH, and $W^2$ is C-$SO_2CH_3$, or
(ii) $W^1$ and $W^2$ are each independently CH, CF, CCl, $CCH_3$, or N, on the proviso that in the case of (i) described above, $R^7$ is H.

(19-3) The heterocyclic compound or the salt thereof according to (15-8), wherein cyclic group C is the following formula (XXVII) (wherein $^{*R7}$ represents a site of connection to $R^7$):

[Chemical Formula 95]

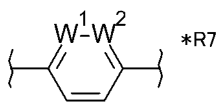
(XXVII)

and
$W^1$ and $W^2$ are each independently CH, or N.

(19-4) The heterocyclic compound or the salt thereof according to (15-8), wherein cyclic group C is the following formula (XXVII) (wherein $^{*R7}$ represents a site of connection to $R^7$):

[Chemical Formula 96]

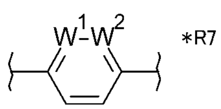
(XXVII)

and
$W^1$ and $W^2$ are each independently CH, CF, CCl, $CCH_3$, or N.

(19-5) The heterocyclic compound or the salt thereof according to (15-8), wherein cyclic group C is the following formula (XXVII) (wherein $^{*R7}$ represents a site of connection to $R^7$):

[Chemical Formula 97]

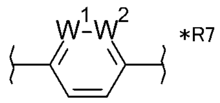

(XXVII)

and
both of $W^1$ and $W^2$ are CH.
(20) A heterocyclic compound or a salt thereof, wherein two or more of the embodiments described in (1-1) to (19-5) above are combined without contradicting each other.

Specific examples of certain embodiments of the heterocyclic compound for use as a drug constituting the antibody-drug conjugate of the present invention or the salt thereof include the following compounds or salts thereof: (4R)-1-[(2S)-2-(4-{4-[({6-cyclopropyl-4-[(1S,4S)-2,5-diazabicyclo[2.2.1]heptan-2-yl]-7-(6-fluoro-5-methyl-1H-indazol-4-yl)-2-[(oxan-4-yl)oxy]quinazolin-8-yl}oxy)methyl]phenyl}-1H-1,2,3-triazol-1-yl)-3-methylbutanoyl]-4-hydroxy-N-{(1R)-2-hydroxy-1-[4-(4-methyl-1,3-thiazol-5-yl)phenyl]ethyl}-L-prolinamide, (4R)-1-[(2S)-2-(4-{4-[({6-cyclopropyl-4-[(1S,4S)-2,5-diazabicyclo[2.2.1]heptan-2-yl]-7-(6-fluoro-5-methyl-1H-indazol-4-yl)-2-[(oxan-4-yl)oxy]quinazolin-8-yl}oxy)methyl]phenyl}-1H-1,2,3-triazol-1-yl)-3-methylbutanoyl]-4-hydroxy-N-{(1R)-2-hydroxy-1-[4-(2-oxo-1,3-oxazolidin-3-yl)phenyl]ethyl}-L-prolinamide, (4R)-1-[(2S)-2-(4-{4-[({6-cyclopropyl-4-[(1S,4S)-2,5-diazabicyclo[2.2.1]heptan-2-yl]-7-(6-fluoro-5-methyl-1H-indazol-4-yl)-2-[(oxan-4-yl)oxy]quinazolin-8-yl}oxy)methyl]phenyl}-1H-1,2,3-triazol-1-yl)-3-methylbutanoyl]-4-hydroxy-N-{(1R)-2-hydroxy-1-[4-(1-methyl-1H-pyrazol-5-yl)phenyl]ethyl}-L-prolinamide, (4R)-1-[(2S)-2-(4-{4-[({6-cyclopropyl-4-[(1S,4S)-2,5-diazabicyclo[2.2.1]heptan-2-yl]-7-(6-fluoro-5-methyl-1H-indazol-4-yl)-2-[(2S)-2-methoxypropoxy]quinazolin-8-yl}oxy)methyl]phenyl}-1H-1,2,3-triazol-1-yl)-3-methylbutanoyl]-4-hydroxy-N-{(1R)-2-hydroxy-1-[4-(4-methyl-1,3-thiazol-5-yl)phenyl]ethyl}-L-prolinamide, (4R)-1-[(2S)-2-(4-{4-[({6-cyclopropyl-4-[(1S,4S)-2,5-diazabicyclo[2.2.1]heptan-2-yl]-7-(6-fluoro-5-methyl-1H-indazol-4-yl)-2-[(2S)-2-methoxypropoxy]quinazolin-8-yl}oxy)methyl]phenyl}-1H-1,2,3-triazol-1-yl)-3-methylbutanoyl]-N-{(1R)-1-[4-(1-ethyl-1H-pyrazol-5-yl)phenyl]-2-hydroxyethyl}-4-hydroxy-L-prolinamide, (4R)-1-[(2S)-2-(4-{4-[({6-cyclopropyl-4-[(1S,4S)-2,5-diazabicyclo[2.2.1]heptan-2-yl]-7-(6-fluoro-5-methyl-1H-indazol-4-yl)-2-[(2S)-2-methoxypropoxy]quinazolin-8-yl}oxy)methyl]phenyl}-1H-1,2,3-triazol-1-yl)-3-methylbutanoyl]-4-hydroxy-N-{(1R)-2-hydroxy-1-[4-(4-methyl-1,3-oxazol-5-yl)phenyl]ethyl}-L-prolinamide, (4R)-1-[(2S)-2-{4-[4-({[(7M)-6-cyclopropyl-7-(6-fluoro-5-methyl-1H-indazol-4-yl)-2-[(2S)-2-methoxypropoxy]-4-{methyl[(3S)-pyrrolidin-3-yl]amino}quinazolin-8-yl]oxy}methyl)phenyl]-1H-1,2,3-triazol-1-yl}-3-methylbutanoyl]-4-hydroxy-N-{(1R)-2-hydroxy-1-[4-(4-methyl-1,3-thiazol-5-yl)phenyl]ethyl}-L-prolinamide, (4R)-1-[(2S)-2-{4-[4-({[(7M)-6-cyclopropyl-7-(6-fluoro-5-methyl-1H-indazol-4-yl)-2-[(2S)-2-methoxypropoxy]-4-{methyl[(3S)-pyrrolidin-3-yl]amino}quinazolin-8-yl]oxy}methyl)phenyl]-1H-1,2,3-triazol-1-yl}-3-methylbutanoyl]-N-{(1R)-1-[4-(1-ethyl-1H-pyrazol-5-yl)phenyl]-2-hydroxyethyl}-4-hydroxy-L-prolinamide, 3-(5-{[(3S)-4-{4-[({(7M)-6-cyclopropyl-4-[(1S,4S)-2,5-diazabicyclo[2.2.1]heptan-2-yl]-7-(6-fluoro-5-methyl-1H-indazol-4-yl)-2-[(oxan-4-yl)oxy]quinazolin-8-yl}oxy)methyl]benzoyl}-3-methylpiperazin-1-yl]methyl}-3-methyl-2-oxo-2,3-dihydro-1H-benzimidazol-1-yl)piperidine-2,6-dione, 1-(6-{[(3S)-4-{4-[({(7M)-6-cyclopropyl-4-[(1S,4S)-2,5-diazabicyclo[2.2.1]heptan-2-yl]-7-(6-fluoro-5-methyl-1H-indazol-4-yl)-2-[(oxan-4-yl)oxy]quinazolin-8-yl}oxy)methyl]benzoyl}-3-methylpiperazin-1-yl]methyl}-1-methyl-1H-indazol-3-yl)-1,3-diazinane-2,4-dione, 3-(5-{[(3S)-4-{4-[({(7M)-6-cyclopropyl-4-[(1S,4S)-2,5-diazabicyclo[2.2.1]heptan-2-yl]-7-(6-fluoro-5-methyl-1H-indazol-4-yl)-2-[(2S)-2-methoxypropoxy]quinazolin-8-yl}oxy)methyl]benzoyl}-3-methylpiperazin-1-yl]methyl}-3-methyl-2-oxo-2,3-dihydro-1H-benzimidazol-1-yl)piperidine-2,6-dione, and (2R)-2-({(4R)-1-[(2S)-2-{4-[4-({[(7M)-6-cyclopropyl-7-(6-fluoro-5-methyl-1H-indazol-4-yl)-2-[(2S)-2-methoxypropoxy]-4-{methyl[(3S)-pyrrolidin-3-yl]amino}quinazolin-8-yl]oxy}methyl)phenyl]-1H-1,2,3-triazol-1-yl}-3-methylbutanoyl]-4-hydroxy-L-prolyl}amino)-2-[4-(1H-1,2,4-triazol-1-yl)phenyl]ethyl dihydrogen phosphate.

Specific examples of certain embodiments of the heterocyclic compound for use as a drug constituting the antibody-drug conjugate of the present invention or the salt thereof include the following compounds or salts thereof: (4R)-1-[(2S)-2-(4-{4-[({6-cyclopropyl-4-[(1S,4S)-2,5-diazabicyclo[2.2.1]heptan-2-yl]-7-(6-fluoro-5-methyl-1H-indazol-4-yl)-2-[(oxan-4-yl)oxy]quinazolin-8-yl}oxy)methyl]phenyl}-1H-1,2,3-triazol-1-yl)-3-methylbutanoyl]-4-hydroxy-N-{(1R)-2-hydroxy-1-[4-(4-methyl-1,3-thiazol-5-yl)phenyl]ethyl}-L-prolinamide, (4R)-1-[(2S)-2-(4-{4-[({6-cyclopropyl-4-[(1S,4S)-2,5-diazabicyclo[2.2.1]heptan-2-yl]-7-(6-fluoro-5-methyl-1H-indazol-4-yl)-2-[(oxan-4-yl)oxy]quinazolin-8-yl}oxy)methyl]phenyl}-1H-1,2,3-triazol-1-yl)-3-methylbutanoyl]-4-hydroxy-N-{(1R)-2-hydroxy-1-[4-(2-oxo-1,3-oxazolidin-3-yl)phenyl]ethyl}-L-prolinamide, (4R)-1-[(2S)-2-(4-{4-[({6-cyclopropyl-4-[(1S,4S)-2,5-diazabicyclo[2.2.1]heptan-2-yl]-7-(6-fluoro-5-methyl-1H-indazol-4-yl)-2-[(oxan-4-yl)oxy]quinazolin-8-yl}oxy)methyl]phenyl}-1H-1,2,3-triazol-1-yl)-3-methylbutanoyl]-4-hydroxy-N-{(1R)-2-hydroxy-1-[4-(1-methyl-1H-pyrazol-5-yl)phenyl]ethyl}-L-prolinamide, (4R)-1-[(2S)-2-(4-{4-[({6-cyclopropyl-4-[(1S,4S)-2,5-diazabicyclo[2.2.1]heptan-2-yl]-7-(6-fluoro-5-methyl-1H-indazol-4-yl)-2-[(2S)-2-methoxypropoxy]quinazolin-8-yl}oxy)methyl]phenyl}-1H-1,2,3-triazol-1-yl)-3-methylbutanoyl]-4-hydroxy-N-{(1R)-2-hydroxy-1-[4-(4-methyl-1,3-thiazol-5-yl)phenyl]ethyl}-L-prolinamide, (4R)-1-[(2S)-2-(4-{4-[({6-cyclopropyl-4-[(1S,4S)-2,5-diazabicyclo[2.2.1]heptan-2-yl]-7-(6-fluoro-5-methyl-1H-indazol-4-yl)-2-[(2S)-2-methoxypropoxy] quinazolin-8-yl}oxy)methyl]phenyl}-1H-1,2,3-triazol-1-yl)-3-methylbutanoyl]-N-{(1R)-1-[4-(1-ethyl-1H-pyrazol-5-yl)phenyl]-2-hydroxyethyl}-4-hydroxy-L-prolinamide, (4R)-1-[(2S)-2-(4-{4-[({6-cyclopropyl-4-[(1S,4S)-2,5-diazabicyclo[2.2.1] heptan-2-yl]-7-(6-fluoro-5-methyl-1H-indazol-4-yl)-2-[(2S)-2-methoxypropoxy]quinazolin-8-yl}oxy)methyl]phenyl}-1H-1,2,3-triazol-1-yl)-3-methylbutanoyl]-4-hydroxy-N-{(1R)-2-hydroxy-1-[4-(4-methyl-1,3-oxazol-5-yl)phenyl] ethyl}-L-prolinamide, (4R)-1-[(2S)-2-{4-[4-({[(7M)-6-cyclopropyl-7-(6-fluoro-5-methyl-1H-indazol-4-yl)-2-[(2S)-2-methoxypropoxy]-4-{methyl[(3S)-pyrrolidin-3-yl]amino}quinazolin-8-yl]oxy}methyl)phenyl]-1H-1,2,3-triazol-1-yl}-3-methylbutanoyl]-4-hydroxy-N-{(1R)-2-hydroxy-1-[4-(4-methyl-1,3-thiazol-5-yl)phenyl]ethyl}-L-prolinamide, (4R)-1-[(2S)-2-{4-[4-({[(7M)-6-cyclopropyl-7-(6-fluoro-5-methyl-1H-indazol-4-yl)-2-[(2S)-2-methoxypropoxy]-4-{methyl [(3S)-pyrrolidin-3-yl]amino}quinazolin-8-yl]oxy}methyl)phenyl]-1H-1,2,3-triazol-1-yl}-3-methylbutanoyl]-N-{(1R)-1-[4-(1-ethyl-1H-pyrazol-5-yl)phenyl]-2-hydroxyethyl}-4-hydroxy-L-prolinamide, 3-(5-{[(3S)-4-{4-[({(7M)-6-cyclopropyl-4-[(1S,4S)-2,5-diazabicyclo[2.2.1]heptan-2-yl]-7-(6-fluoro-5-methyl-1H-indazol-4-yl)-2-[(oxan-4-yl)oxy]quinazolin-8-yl}oxy)methyl]benzoyl}-3-methylpiperazin-1-yl]methyl}-3-methyl-2-oxo-2,3-dihydro-1H-benzimidazol-1-yl)piperidine-2,6-dione, 1-(6-{[(3S)-4-{4-[({(7M)-6-cyclopropyl-4-[(1S,4S)-2,5-diazabicyclo[2.2.1]heptan-2-yl]-7-(6-fluoro-5-methyl-1H-indazol-4-yl)-2-[(oxan-4-yl)oxy]quinazolin-8-yl}oxy)methyl]benzoyl}-3-methylpiperazin-1-yl]methyl}-1-methyl-1H-indazol-3-yl)-1,3-diazinane-2,4-dione, and 3-(5-{[(3S)-4-{4-[({(7M)-6-cyclopropyl-4-[(1S,4S)-2,5-diazabicyclo[2.2.1]heptan-2-yl]-7-(6-fluoro-5-methyl-1H-indazol-4-yl)-2-[(2S)-2-methoxypropoxy]quinazolin-8-yl}oxy)methyl]benzoyl}-3-methylpiperazin-1-yl]methyl}-3-methyl-2-oxo-2,3-dihydro-1H-benzimidazol-1-yl)piperidine-2,6-dione.

Specific examples of certain embodiments of the heterocyclic compound for use as a drug constituting the antibody-drug conjugate of the present invention or the salt thereof include the following compounds or salts thereof: (4R)-1-[(2S)-2-(4-{4-[({(7M)-6-cyclopropyl-4-[(1S,4S)-2,5-diazabicyclo[2.2.1]heptan-2-yl]-7-(6-fluoro-5-methyl-1H-indazol-4-yl)-2-[(oxan-4-yl)oxy]quinazolin-8-yl}oxy)methyl]phenyl}-1H-1,2,3-triazol-1-yl)-3-methylbutanoyl]-4-hydroxy-N-{(1R)-2-hydroxy-1-[4-(4-methyl-1,3-thiazol-5-yl)phenyl]ethyl}-L-prolinamide, (4R)-1-[(2S)-2-(4-{4-[({(7M)-6-cyclopropyl-4-[(1S,4S)-2,5-diazabicyclo[2.2.1]heptan-2-yl]-7-(6-fluoro-5-methyl-1H-indazol-4-yl)-2-[(oxan-4-yl)oxy]quinazolin-8-yl}oxy)methyl]phenyl}-1H-1,2,3-triazol-1-yl)-3-methylbutanoyl]-4-hydroxy-N-{(1R)-2-hydroxy-1-[4-(2-oxo-1,3-oxazolidin-3-yl)phenyl]ethyl}-L-prolinamide, (4R)-1-[(2S)-2-(4-{4-[({(7M)-6-cyclopropyl-4-[(1S,4S)-2,5-diazabicyclo[2.2.1] heptan-2-yl]-7-(6-fluoro-5-methyl-1H-indazol-4-yl)-2-[(oxan-4-yl)oxy]quinazolin-8-yl}oxy)methyl]phenyl}-1H-1,2,3-triazol-1-yl)-3-methylbutanoyl]-4-hydroxy-N-{(1R)-2-hydroxy-1-[4-(1-methyl-1H-pyrazol-5-yl)phenyl]ethyl}-L-prolinamide, (4R)-1-[(2S)-2-(4-{4-[({(7M)-6-cyclopropyl-4-[(1S,4S)-2,5-diazabicyclo[2.2.1]heptan-2-yl]-7-(6-fluoro-5-methyl-1H-indazol-4-yl)-2-[(2S)-2-methoxypropoxy]quinazolin-8-yl}oxy)methyl]phenyl}-1H-1,2,3-triazol-1-yl)-3-methylbutanoyl]-4-hydroxy-N-{(1R)-2-hydroxy-1-[4-(4-methyl-1,3-thiazol-5-yl)phenyl]ethyl}-L-prolinamide, (4R)-1-[(2S)-2-(4-{4-[({(7M)-6-cyclopropyl-4-[(1S,4S)-2,5-diazabicyclo[2.2.1]heptan-2-yl]-7-(6-fluoro-5-methyl-1H-indazol-4-yl)-2-[(2S)-2-methoxypropoxy]quinazolin-8-yl}oxy)methyl]phenyl}-1H-1,2,3-triazol-1-yl)-3-methylbutanoyl]-N-{(1R)-1-[4-(1-ethyl-1H-pyrazol-5-yl)phenyl]-2-hydroxyethyl}-4-hydroxy-L-prolinamide, and (4R)-1-[(2S)-2-(4-{4-[({(7M)-6-cyclopropyl-4-[(1S,4S)-2,5-diazabicyclo[2.2.1]heptan-2-yl]-7-(6-fluoro-5-methyl-1H-indazol-4-yl)-2-[(2S)-2-methoxypropoxy]quinazolin-8-yl}oxy) methyl]phenyl}-1H-1,2,3-triazol-1-yl)-3-methylbutanoyl]-4-hydroxy-N-{(1R)-2-hydroxy-1-[4-(4-methyl-1,3-oxazol-5-yl) phenyl]ethyl}-L-prolinamide.

Specific examples of certain embodiments of the heterocyclic compound for use as a drug constituting the antibody-drug conjugate of the present invention or the salt thereof include the following compounds or salts thereof: (2R)-2-({(4R)-1-[(2S)-2-{4-[4-({[(7M)-6-cyclopropyl-7-(6-fluoro-5-methyl-1H-indazol-4-yl)-2-[(2S)-2-methoxypropoxy]-4-{methyl[(3S)-pyrrolidin-3-yl]amino}quinazolin-8-yl]oxy}methyl)phenyl]-1H-1,2,3-triazol-1-yl}-3-methylbutanoyl]-4-hydroxy-L-prolyl}amino)-2-[4-(1H-1,2,4-triazol-1-yl)phenyl]ethyl dihydrogen phosphate.

Other examples of the heterocyclic compound having a G12D mutant KRAS protein degradation-inducing effect for use as a drug constituting the antibody-drug conjugate of the present invention or the salt thereof will be shown below.

A heterocyclic compound of the following formula (TP-1) or (TP-2) (see International Publication No. WO 2022/148421):

[Chemical Formula 98-1]

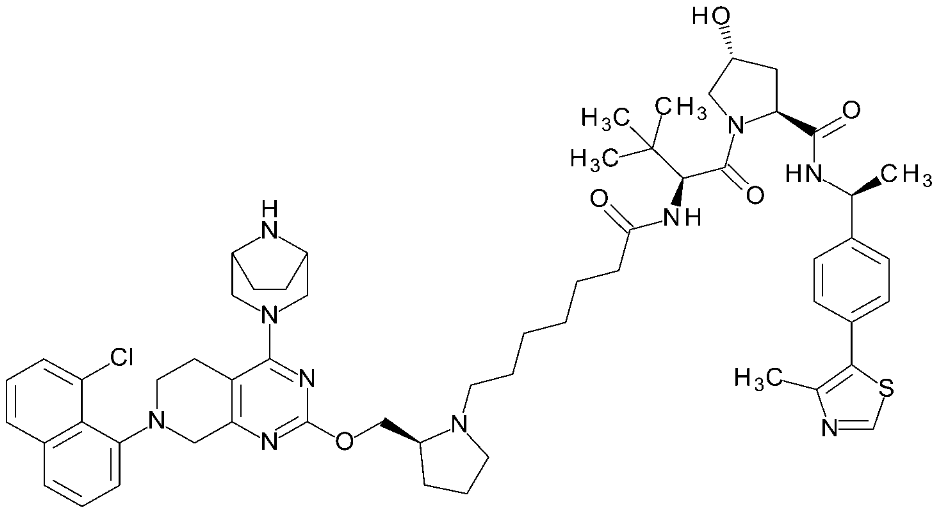

(TP-1)

[Chemical Formula 98-2]

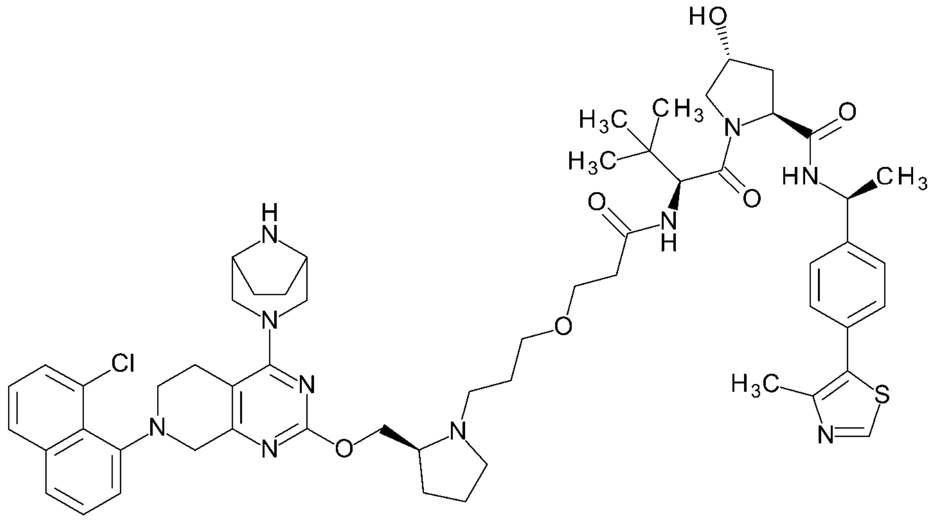

(TP-2)

A heterocyclic compound of the following formula (TP-3), (TP-4), (TP-5), (TP-6), or (TP-7) (see International Publication No. WO 2022/148422):

[Chemical Formula 99-1]

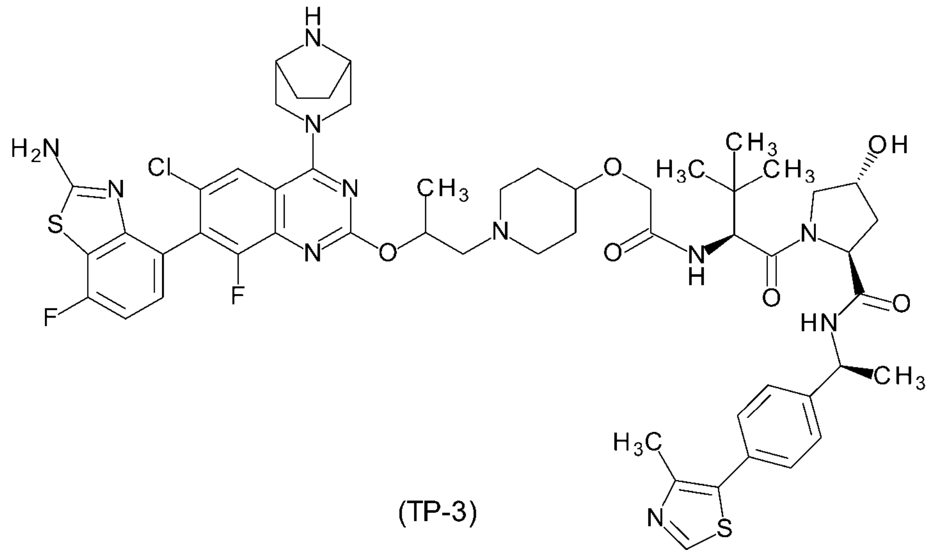

(TP-3)

[Chemical Formula 99-2]

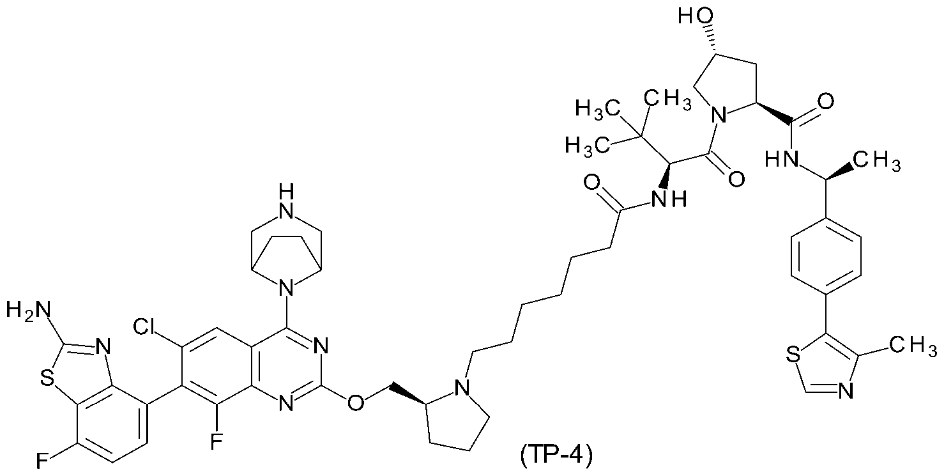

(TP-4)

[Chemical Formula 99-3]

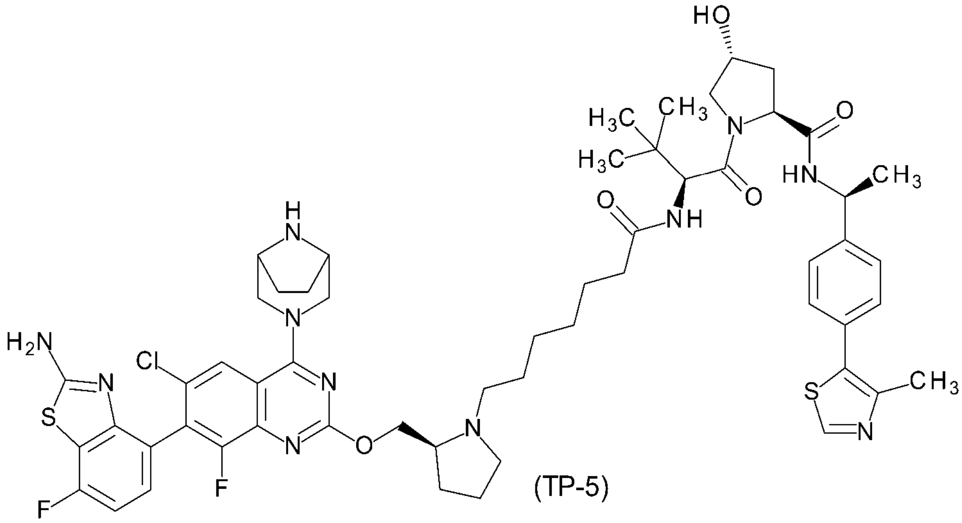

(TP-5)

[Chemical Formula 99-4]

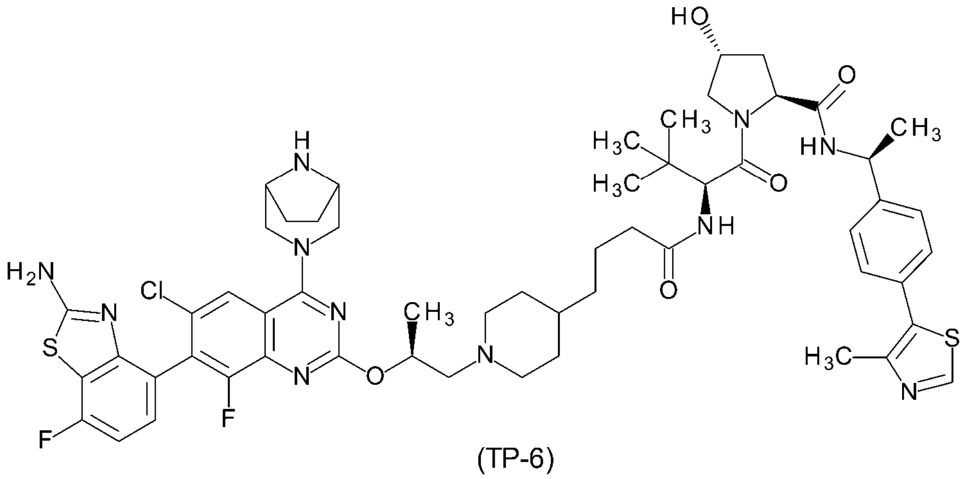

(TP-6)

[Chemical Formula 99-5]

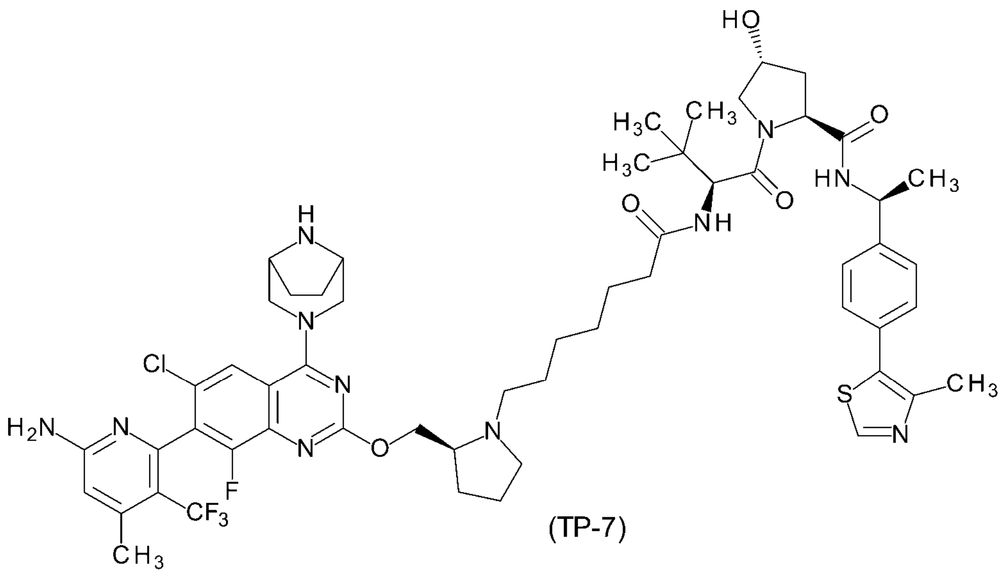

(TP-7)

A heterocyclic compound represented by general formula W-L-T,

wherein W is G12D mutant KRAS protein binding ligand of the following formula (TP-8) or (TP-9):

[Chemical Formula 100]

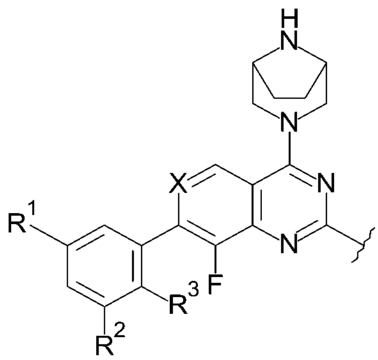

(TP-8)

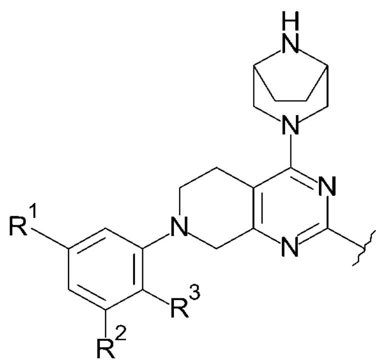

(TP-9)

X is N, C-H, C-F, C-Cl, C-$CH_3$, C-$C_2H_5$, or C-$C_3H_7$,
$R^1$ is a substituted or unsubstituted hydroxyl group, amino group, or mercapto group,
$R^2$ and $R^3$ are each independently H, halogen, or halomethyl (monohalomethyl, dihalomethyl, or trihalomethyl), or $R^2$ and $R^3$ form a substituted or unsubstituted condensed benzene ring (including, but not limited to, a naphthalene ring) together with the benzene ring bonded thereto, wherein the condensed benzene ring is optionally substituted by 1 or more, for example, 2 or 3 groups selected from the group consisting of halogen, a hydroxyl group, an amino group, a halomethyl group, $C_{1-2}$ alkyl, and $C_{2-4}$ alkynyl,
T is a group having the ability to bind to E3 ubiquitin ligase, and
L is a divalent linking moiety for connecting W and T (see Chinese Patent Application Publication No. 115785199).

A heterocyclic compound of the following formula (TP-10), (TP-11), (TP-12), (TP-13), (TP-14), (TP-15), (TP-16), or (TP-17) (see Chinese Patent Application Publication No. 115785199):

[Chemical Formula 101-1]

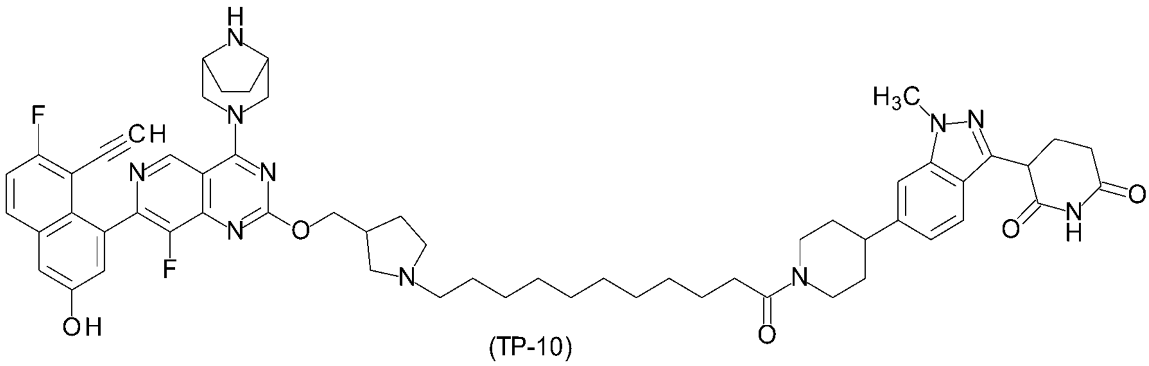

(TP-10)

[Chemical Formula 101-2]

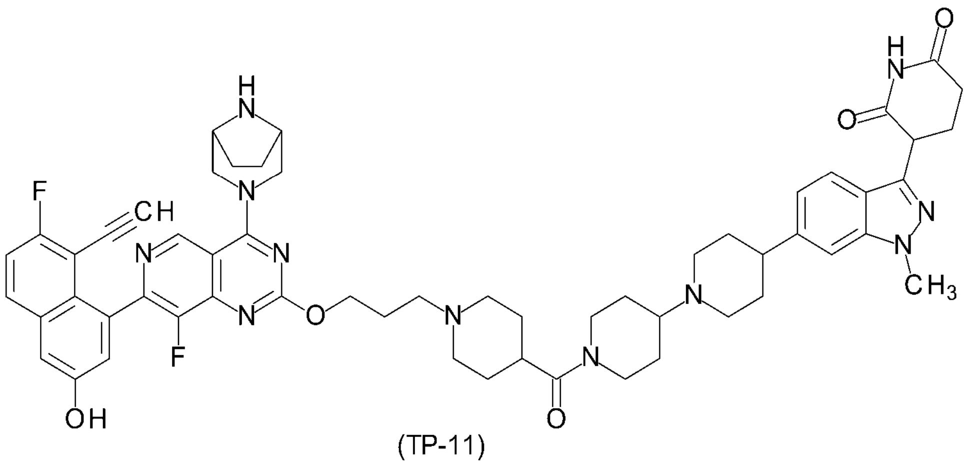

(TP-11)

[Chemical Formula 101-3]

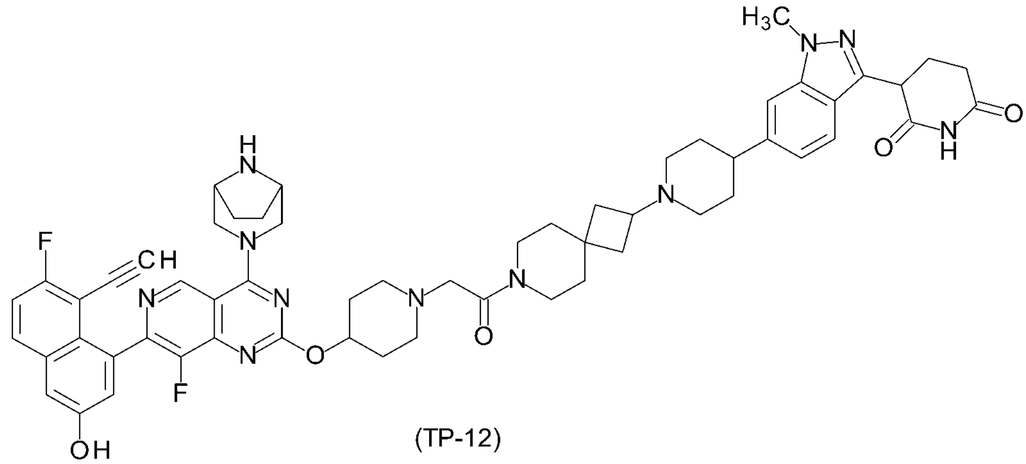

(TP-12)

[Chemical Formula 101-4]

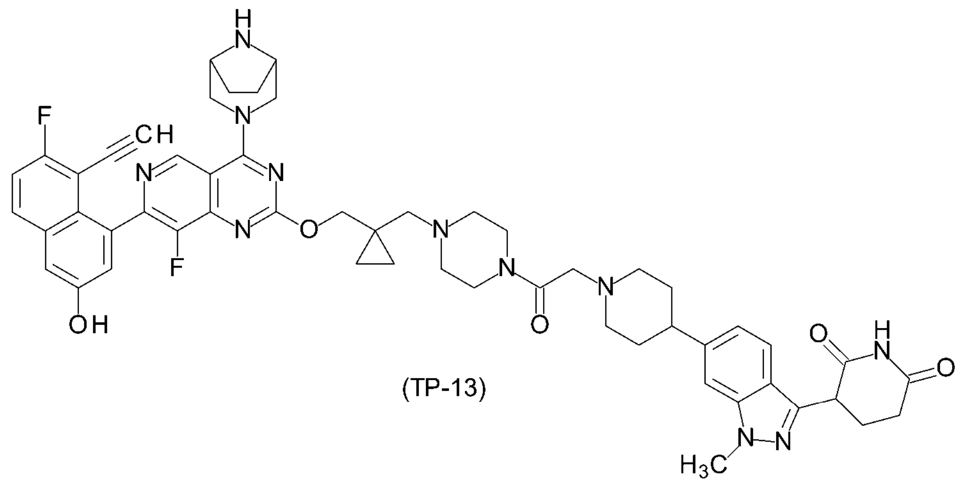

(TP-13)

[Chemical Formula 101-5]

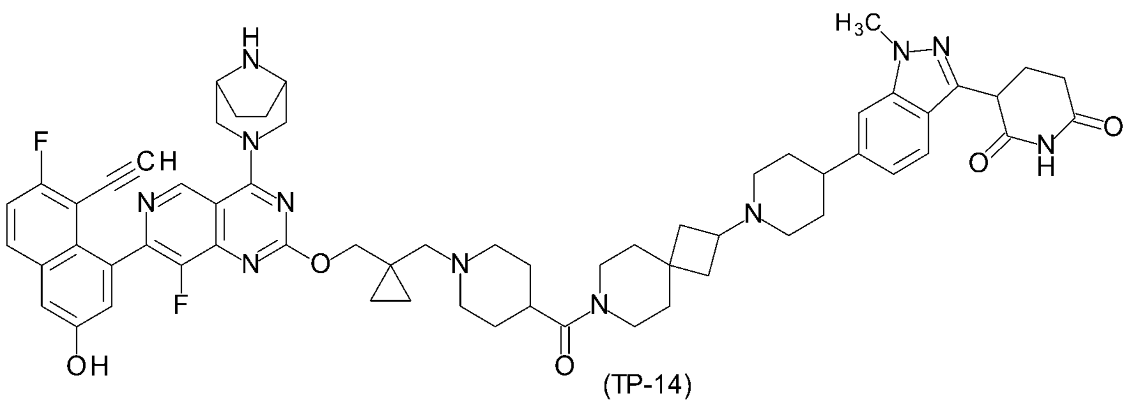

(TP-14)

[Chemical Formula 101-6]

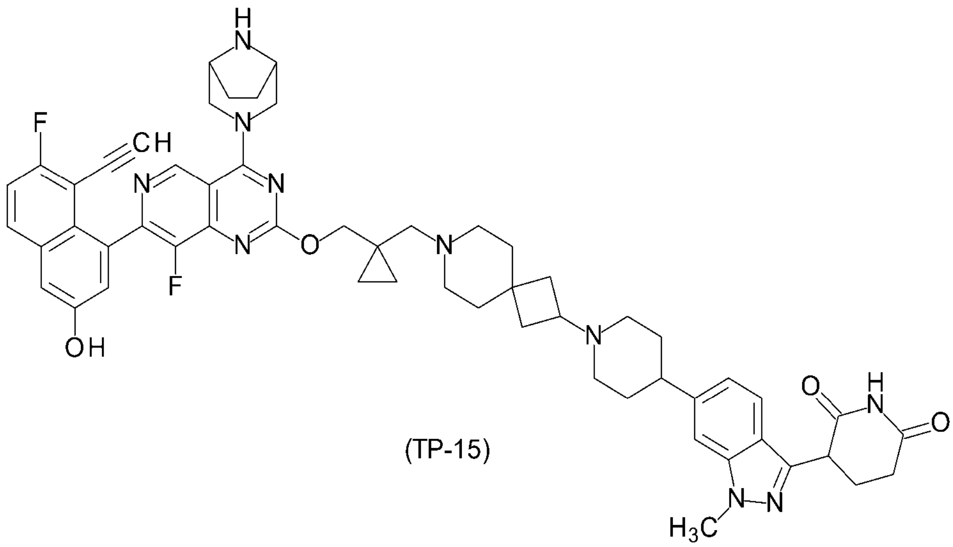

(TP-15)

[Chemical Formula 101-7]

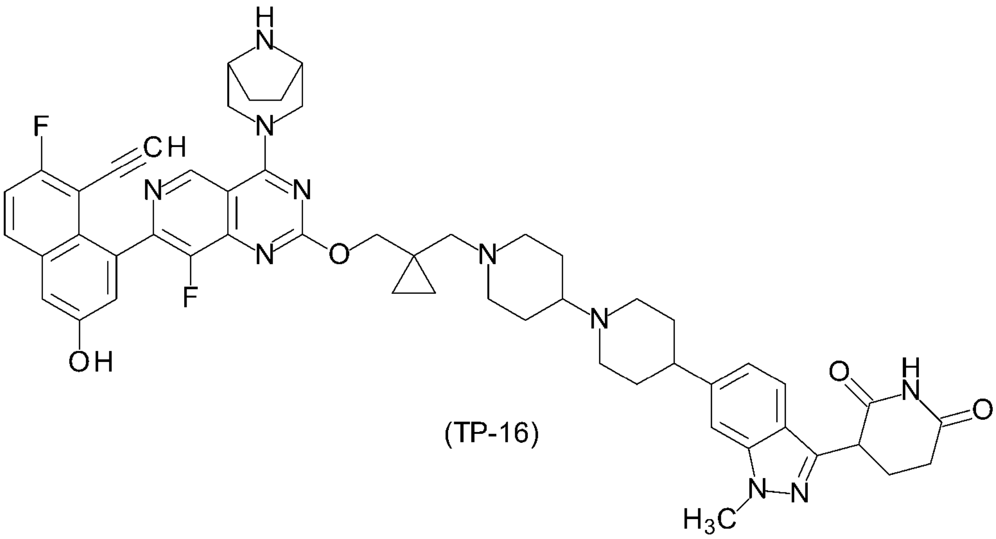

(TP-16)

[Chemical Formula 101-8]

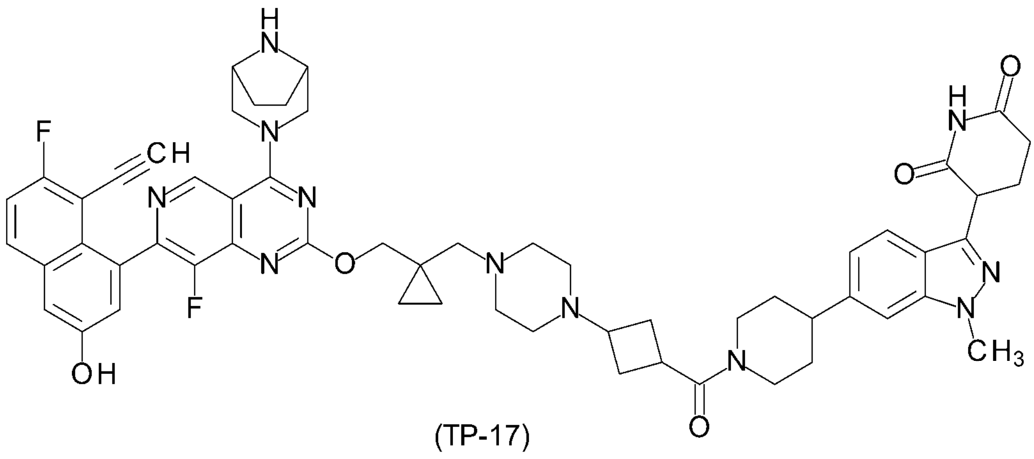

(TP-17)

A heterocyclic compound of the following formula (TP-18):

[Chemical Formula 102]

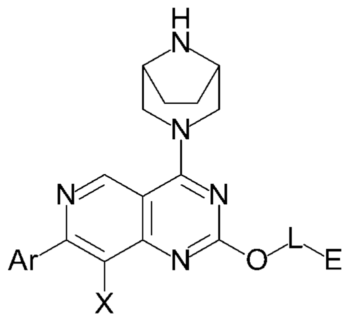

(TP-18)

wherein Ar is optionally substituted aryl or optionally substituted heteroaryl, X is H or halogen, L is a linking moiety, and E is a group having the ability to bind to E3 ubiquitin ligase (see International Publication No. WO 2023/077441).

A heterocyclic compound of the following formula (TP-19), (TP-20), (TP-21), or (TP-22) (see International Publication No. WO 2023/077441):

[Chemical Formula 103-1]

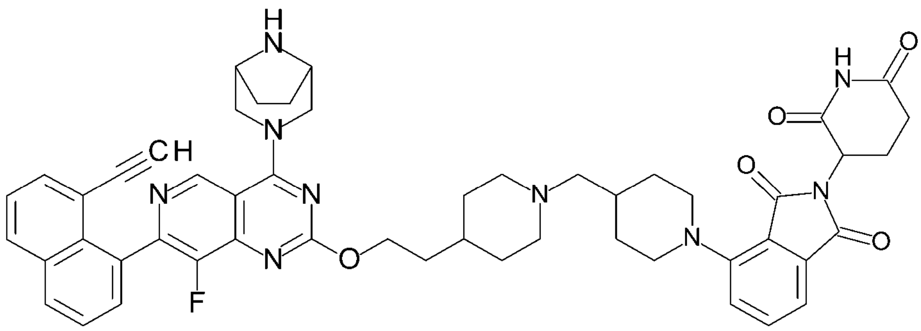

(TP-19)

[Chemical Formula 103-2]

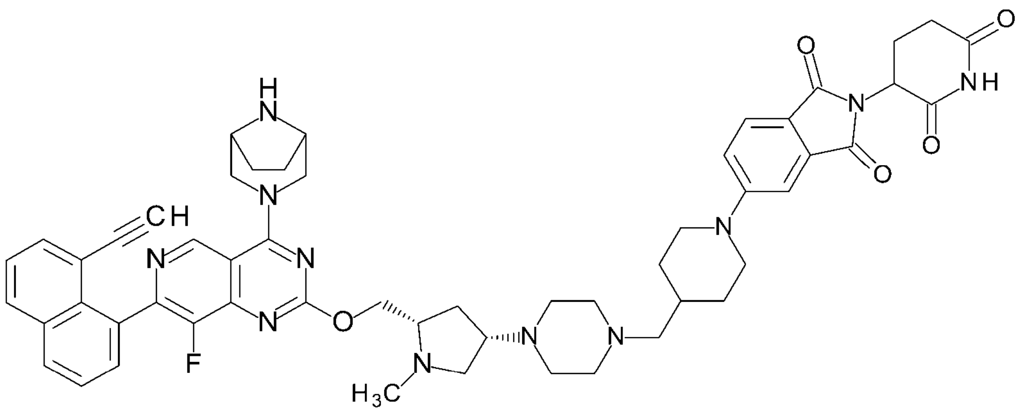

(TP-20)

[Chemical Formula 103-3]

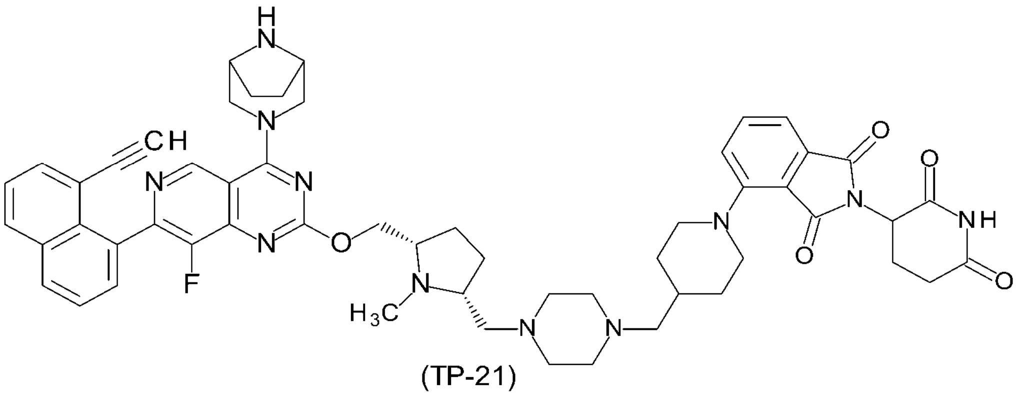

(TP-21)

[Chemical Formula 103-4]

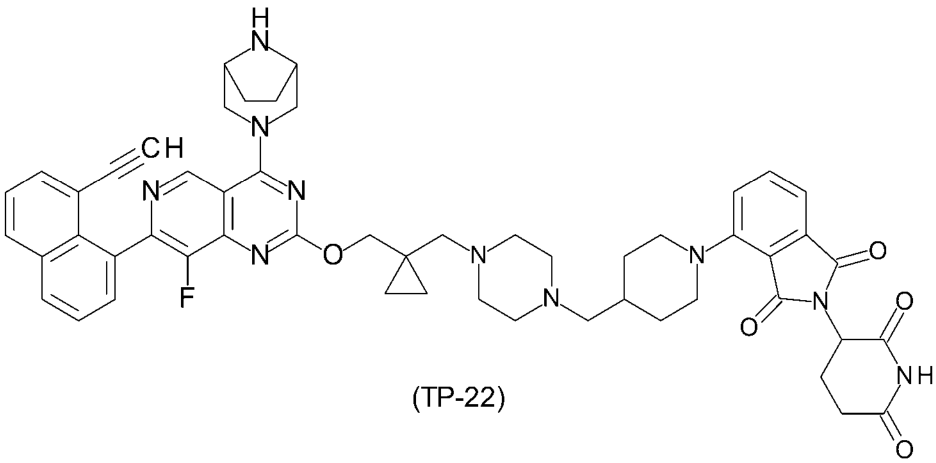

(TP-22)

A heterocyclic compound of the following formula (TP-23):

[Chemical Formula 104-1]

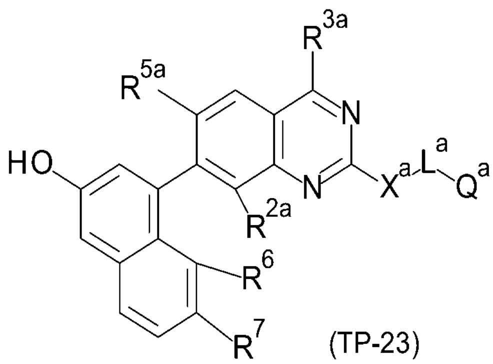

(TP-23)

wherein $R^{2a}$ is halogen, $R^{3a}$ is 7- or 8-membered bridged hetero cycloalkyl containing two N, $R^{5a}$ is H, $R^6$ and $R^7$ are each independently H, halogen, $C_{1-12}$ alkyl, or $C_{2-6}$ alkynyl, $X^a$ is

[Chemical Formula 104-2]

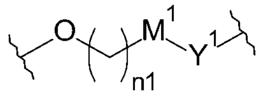

$M^1$ is 3- to 9-membered hetero cycloalkyl, $Y^1$ is absent, $L^a$ is

[Chemical Formula 104-3]

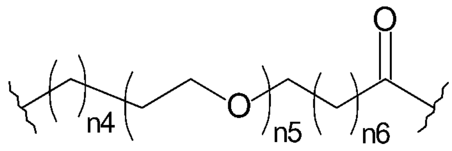

n1 is 1, 2, or 3, n4 is 0, 1, or 2, n5 is an integer of 1 to 5, n6 is 0, 1, or 2, and $Q^a$ is a group having the ability to bind to E3 ubiquitin ligase (see International Publication No. WO 2022/228576).

A heterocyclic compound of the following formula (TP-24), (TP-25), (TP-26), or (TP-27) (see International Publication No. WO 2022/228576):

[Chemical Formula 105-1]

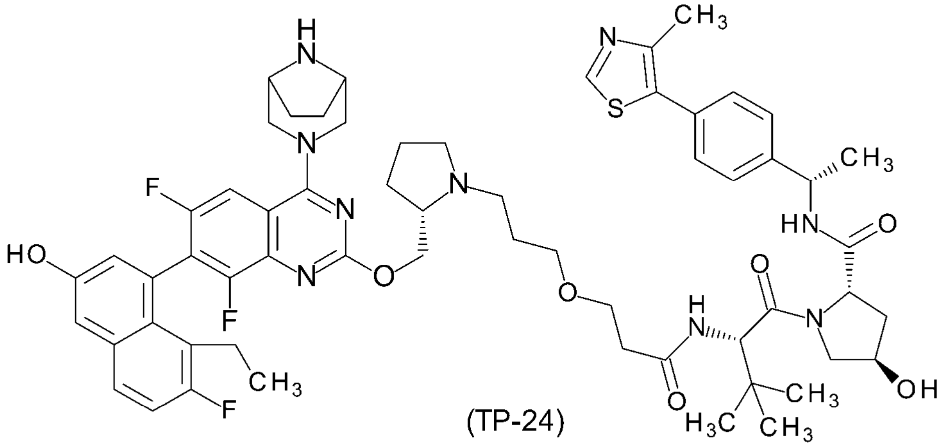

(TP-24)

[Chemical Formula 105-2]

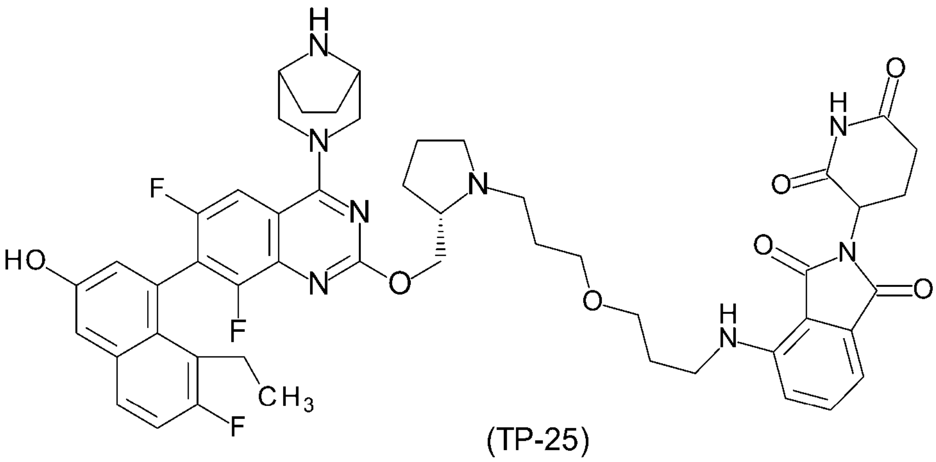

(TP-25)

[Chemical Formula 105-3]

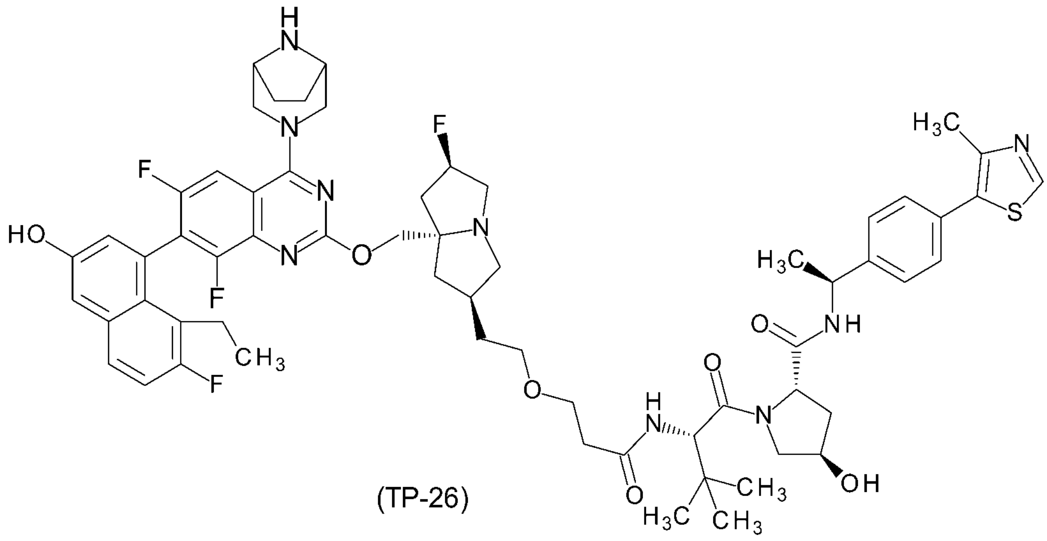

(TP-26)

[Chemical Formula 105-4]

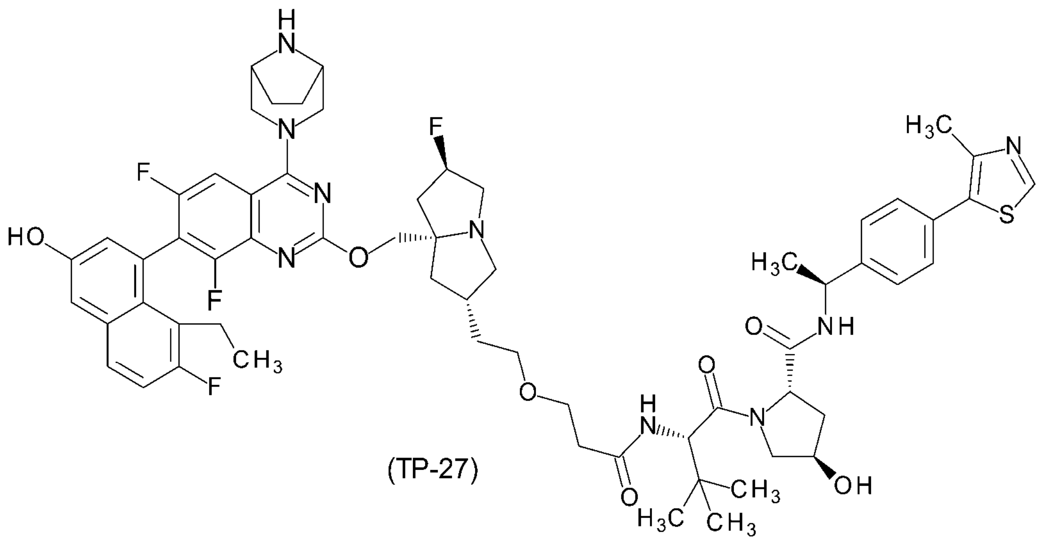

(TP-27)

A heterocyclic compound of the following formula (TP-28) or (TP-29):

[Chemical Formula 106-1]

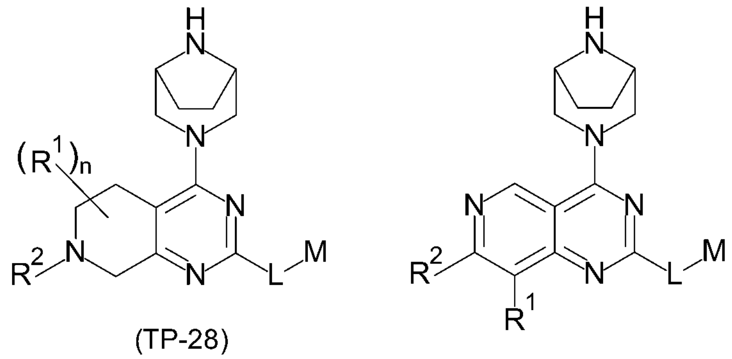

(TP-28)

(TP-29)

wherein $R^1$ is H or halogen, n is 0, 1, 2, 3, or 4, $R^2$ is 8-chloronaphthalen-1-yl, 3-hydroxynaphthalen-1-yl, 8-ethynyl-7-fluoro-3-hydroxynaphthalen-1-yl, or 8-ethynyl-7-fluoronaphthalen-1-yl, L is

[Chemical Formula 106-2]

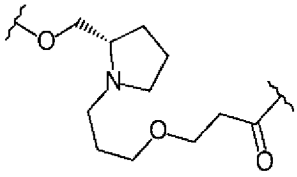
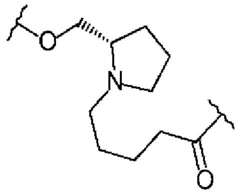
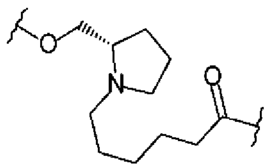
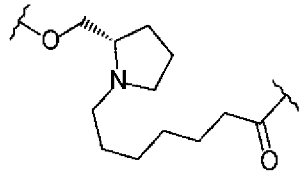

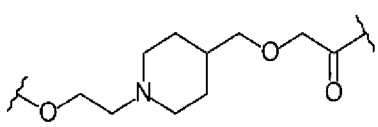
or
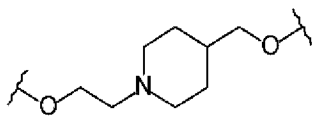

and
M is

[Chemical Formula 106-3]

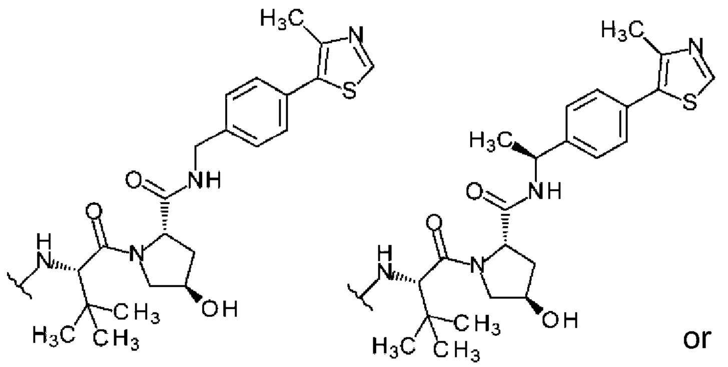
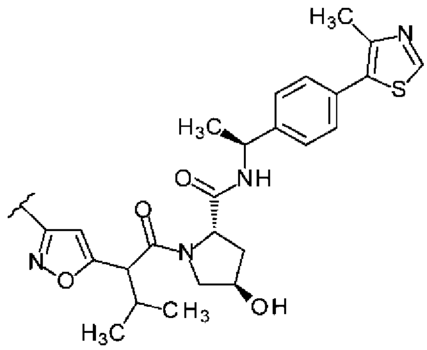

(see International Publication No. WO 2023/280026).

A heterocyclic compound of the following formula (TP-30) or (TP-31) (see International Publication No. WO 2023/280026):

[Chemical Formula 107-1]

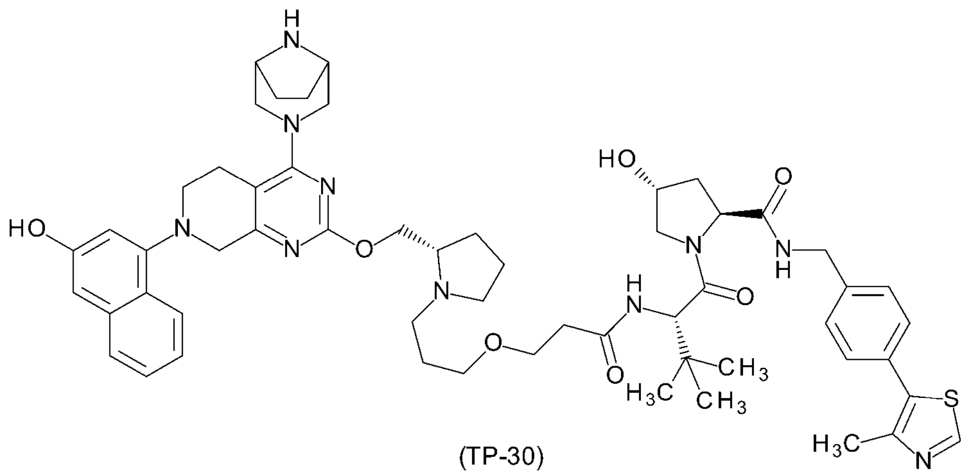

(TP-30)

[Chemical Formula 107-2]

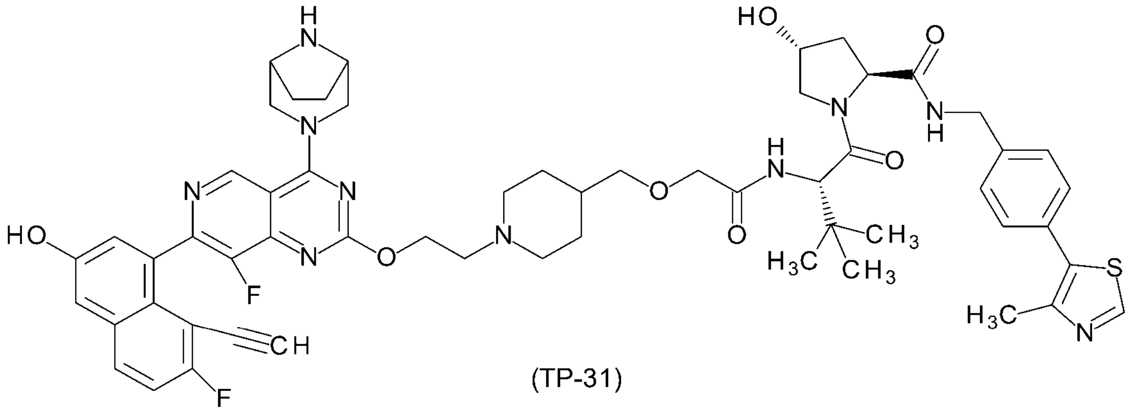

(TP-31)

2. Linker ($L^A$)

In the present specification, linker ($L^A$) constituting the antibody-drug conjugate of the present invention or the salt thereof is a bifunctional partial structure that connects one or more drugs (D) to antibody or antigen binding fragment (Ab) to form an antibody-drug conjugate. Linker ($L^A$) is a linker having a partial structure cleavable *in vivo* in certain embodiments and is, but not particularly limited to, a linker having a partial structure cleavable by an enzyme such as cathepsin B. The linker is cleaved so that drug (D) is released *in vivo*. Linker ($L^A$) is bonded to D through an arbitrary nitrogen atom of -NH- or oxygen atom of -OH contained in D in certain embodiments, and is bonded to D through a nitrogen atom contained in $V^2$ or W at the site of connection represented by $^{*LA}$ in certain embodiments.

Linker ($L^A$) according to the present invention is a linker represented by the following formula (XXVIII) in certain embodiments:

[Chemical Formula 108]

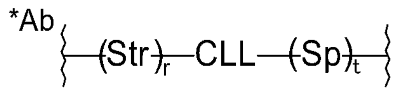

(XXVIII)

wherein

Str is a stretcher unit and is bonded to Ab and CLL, r is 0 or 1,
CLL is a partial structure cleavable *in vivo*,
Sp is a spacer unit and is bonded to CLL and D, t is 0 or 1, and
$^{*Ab}$ represents a site of connection to Ab.

As used herein, "stretcher unit (Str)", if present, is a structural unit that connects Ab and CLL. The stretcher unit forms a covalent bond with a functional group contained in Ab. Examples of the functional group contained in Ab, which forms a covalent bond with the stretcher unit, include, but are not particularly limited to, a mercapto group, an amino group, a hydroxyl group, and a carboxyl group. It is a mercapto group in certain embodiments. A mercapto group that can be used in covalent bond formation with Ab may be generated by reducing an intramolecular disulfide bond contained in Ab. When r is 0, the stretcher unit is absent and partial structure (CLL) cleavable *in vivo* is connected directly to Ab.

Certain embodiments of stretcher unit (Str) will be shown below.

(21-1) A stretcher unit of formula (ST-1):

[Chemical Formula 109]

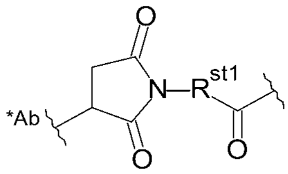

(ST-1)

wherein $R^{st1}$ is optionally substituted $C_{1-12}$ alkylene, $-(CH_2CH_2O)_{a1}-C_{1-6}$ alkylene-, $-C_{1-6}$ alkylene-$(OCH_2CH_2)_{a2}$-, -optionally substituted $C_{1-6}$ alkylene-NH-C(=O)-optionally substituted $C_{1-6}$ alkylene-, -optionally substituted $C_{1-6}$ alkylene-C(=O)-NH-optionally substituted $C_{1-6}$ alkylene-, optionally substituted $C_{1-6}$ alkylene-C(=O)-NH-$(CH_2CH_2O)_{a3}-C_{1-6}$ alkylene-, or optionally substituted $C_{1-6}$ alkylene-NH-C(=O)-$(CH_2CH_2O)_{a4}-C_{1-6}$ alkylene-, each of a1, a2, a3, and a4 is an integer of 1 to 10, and $^{*Ab}$ represents a site of connection to Ab.

(21-2) The stretcher unit according to (21-1), wherein $R^{st1}$ is $C_{1-12}$ alkylene.
(21-3) The stretcher unit according to (21-1), wherein $R^{st1}$ is $C_{3-8}$ alkylene.
(21-4) The stretcher unit according to (21-1), wherein $R^{st1}$ is $C_5$ alkylene.
(21-5) A stretcher unit of formula (ST-2) or (ST-3):

[Chemical Formula 110]

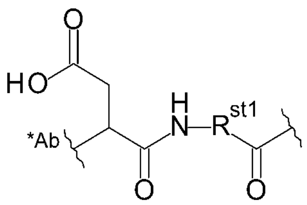

(ST-2)

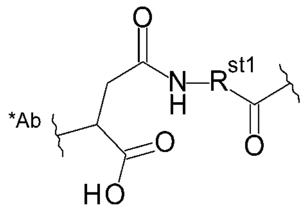

(ST-3)

wherein $R^{st1}$ is optionally substituted $C_{1-12}$ alkylene, $-(CH_2CH_2O)_{a1}-C_{1-6}$ alkylene-, $-C_{1-6}$ alkylene-$(OCH_2CH_2)_{a2}$-, -optionally substituted $C_{1-6}$ alkylene-NH-C(=O)-optionally substituted $C_{1-6}$ alkylene-, -optionally substituted $C_{1-6}$ alkylene-C(=O)-NH-optionally substituted $C_{1-6}$ alkylene-, optionally substituted $C_{1-6}$ alkylene-C(=O)-NH-$(CH_2CH_2O)_{a3}-C_{1-6}$ alkylene-, or optionally substituted $C_{1-6}$ alkylene-NH-C(=O)-$(CH_2CH_2O)_{a4}-C_{1-6}$ alkylene-, each of a1, a2, a3, and a4 is an integer of 1 to 10, and $^{*Ab}$ represents a site of connection to Ab.

(21-6) The stretcher unit according to (21-5), wherein $R^{st1}$ is $C_{1-12}$ alkylene.
(21-7) The stretcher unit according to (21-5), wherein $R^{st1}$ is $C_{3-8}$ alkylene.
(21-8) The stretcher unit according to (21-5), wherein $R^{st1}$ is $C_5$ alkylene.

Embodiments of "partial structure (CLL) cleavable *in vivo*" according to the present invention will be shown below.

(22-1) A partial structure cleavable *in vivo* of formula (CL-1):

[Chemical Formula 111]

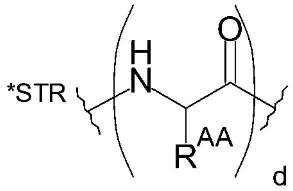

(CL-1)

wherein $R^{AA}$ are each independently H, methyl, isopropyl, isobutyl, sec-butyl, benzyl, p-hydroxybenzyl, hydroxymethyl, 1-hydroxyethyl, $-CH_2-C(=O)-OH$, $-(CH_2)_2-C(=O)-OH$, - $CH_2-C(=O)-NH_2$, $-(CH_2)_2-C(=O)-NH_2$, $-(CH_2)_4-NH_2$, $-(CH_2)_3-NH-C(=NH)-NH_2$, $-(CH_2)_3-NH-C(=O)-NH_2$, $-CH_2-SH$, or $-CH_2-S-CH_3$, or one group selected from the group consisting of the following formula (RAA-1) and (RAA-2):

[Chemical Formula 112]

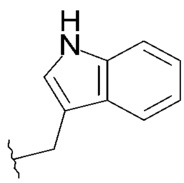

(RAA-1)

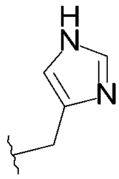

(RAA-2)

d is an integer of 1 to 6, and $^{*STR}$ represents a site of connection to Str.

(22-2) The partial structure cleavable *in vivo* according to (22-1), wherein $R^{AA}$ are each independently H, methyl, isopropyl, benzyl, $-(CH_2)_4-NH_2$, $-(CH_2)_3-NH-C(=NH)-NH_2$, or $-(CH_2)_3-NH-C(=O)-NH_2$, and d is an integer of 2 to 4.

(22-3) The partial structure cleavable *in vivo* according to (22-1), wherein $R^{AA}$ are each independently methyl, isopropyl, benzyl, $-(CH_2)_4-NH_2$, $-(CH_2)_3-NH-C(=NH)-NH_2$, or - $(CH_2)_3-NH-C(=O)-NH_2$, and d is 2.

(22-4) The partial structure cleavable *in vivo* according to (22-1), wherein $R^{AA}$ are each independently methyl, isopropyl, or $-(CH_2)_3-NH-C(=O)-NH_2$, and d is 2.

(22-5) The partial structure cleavable *in vivo* according to (22-1), wherein $R^{AA}$ are each independently methyl or isopropyl, and d is 2.

(22-6) The partial structure cleavable *in vivo* according to (22-1), wherein $R^{AA}$ are each independently isopropyl or $-(CH_2)_3-NH-C(=O)-NH_2$, and d is 2.

(22-7) The partial structure cleavable *in vivo* according to (22-1), wherein $R^{AA}$ are each independently H or benzyl, and d is 4.

As used herein, "spacer unit (Sp)" is a structural unit that connects CLL and drug (D), if present. The spacer unit, if present, forms a covalent bond with an arbitrary group of drug (D) in certain embodiments, forms a covalent bond with an arbitrary nitrogen atom of - NH- or oxygen atom of -OH of drug (D) in certain embodiments, forms a covalent bond with a nitrogen atom of one secondary amino group contained in formula (VI), formula (VII), formula (VIII), formula (IX), formula (X), formula (XI), formula (XII), formula (XIII), formula (XIV), formula (XV), or formula (XVI) of drug (D) in certain embodiments, and forms a covalent bond with a nitrogen atom of one secondary amino group contained as a ring-constituting atom in $V^2$ or W of drug (D) in certain embodiments. The spacer unit is a self-destruction spacer unit in certain embodiments. When CLL is cleaved, the self-destruction spacer unit is degraded in itself without undergoing further hydrolysis reaction so that free drug (D) is released. When t is 0, the spacer unit is absent and partial structure (CLL) cleavable *in vivo* is connected directly to drug (D).

Certain embodiments of the spacer unit will be shown below.

(23-1) A spacer unit of formula (SP-1):

[Chemical Formula 113]

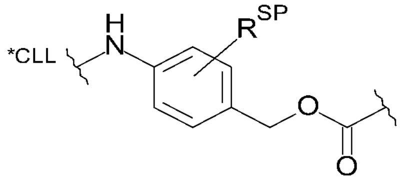

(SP-1)

wherein $R^{SP}$ is H, $C_{1-6}$ alkyl, -O-$C_{1-6}$ alkyl, halogen, or halogeno $C_{1-6}$ alkyl, and $^{*CLL}$ represents a site of connection to CLL.

(23-2) The spacer unit according to (23-1), wherein $R^{SP}$ is H.

Although not bound by the following theory, it is considered that the self-destruction spacer unit used in the present invention is degraded through the following mechanism after cleavage of the bond between CLL and the nitrogen atom so that drug (D) is released (Journal of Organic Chemistry, 2002, 67, p. 1866-1872).

[Chemical Formula 114]

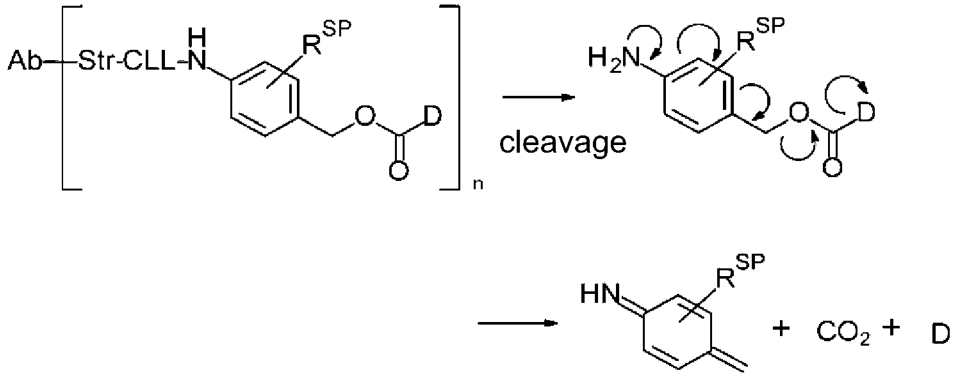


Certain embodiments of linker $L^A$ constituting the antibody-drug conjugate of the present invention or the salt thereof will be shown below.

(24-1) A linker of the following formula (LA-1) (wherein$^{*Ab}$ represents a site of connection to Ab):

[Chemical Formula 115]

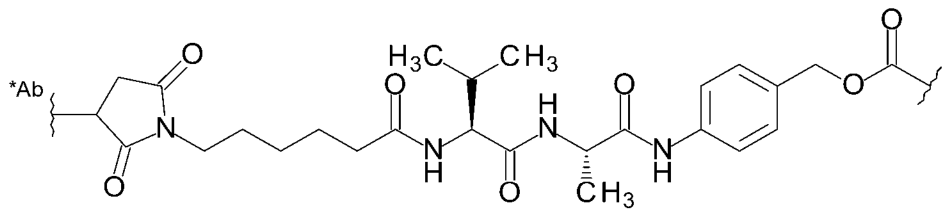

(LA-1)

(24-2) A linker of the following formula (LA-2) (wherein$^{*Ab}$ represents a site of connection to Ab):

[Chemical Formula 116]

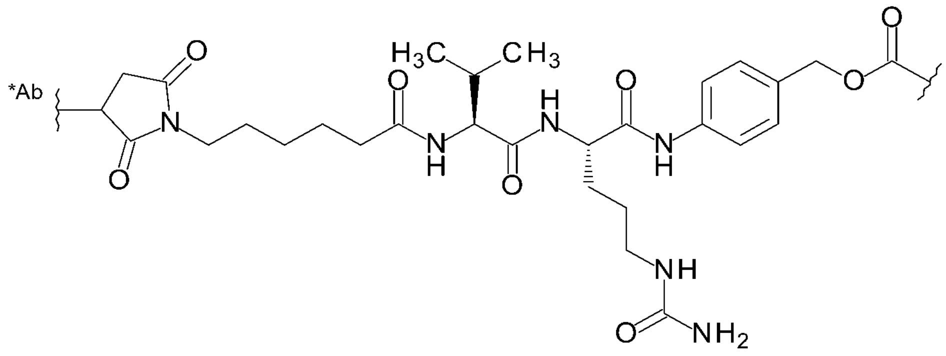

(LA-2)

Other examples of linker $L^A$ constituting the antibody-drug conjugate of the present invention or the salt thereof will be shown below.

A linker of the following formula (LA-3) (see International Publication No. WO 2015/095124; wherein $^{*Ab}$ represents a site of connection to Ab):

[Chemical Formula 117]

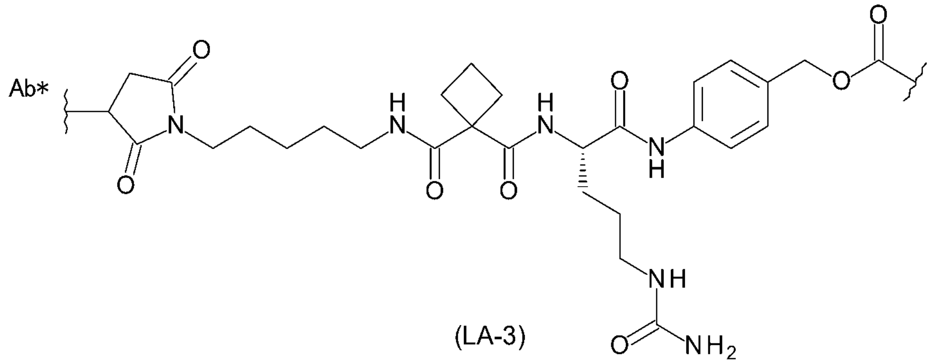

(LA-3)

A linker of the following formula (LA-4) (see International Publication No. WO 2015/095124; wherein $^{*Ab}$ represents a site of connection to Ab):

[Chemical Formula 118]

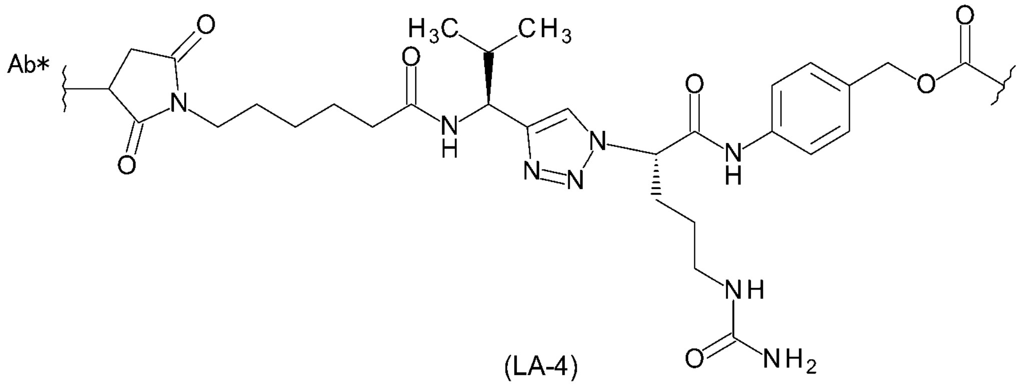

(LA-4)

A linker of the following formula (LA-5) (see International Publication No. WO 2015/095223; wherein $^{*Ab}$ represents a site of connection to Ab):

[Chemical Formula 119]

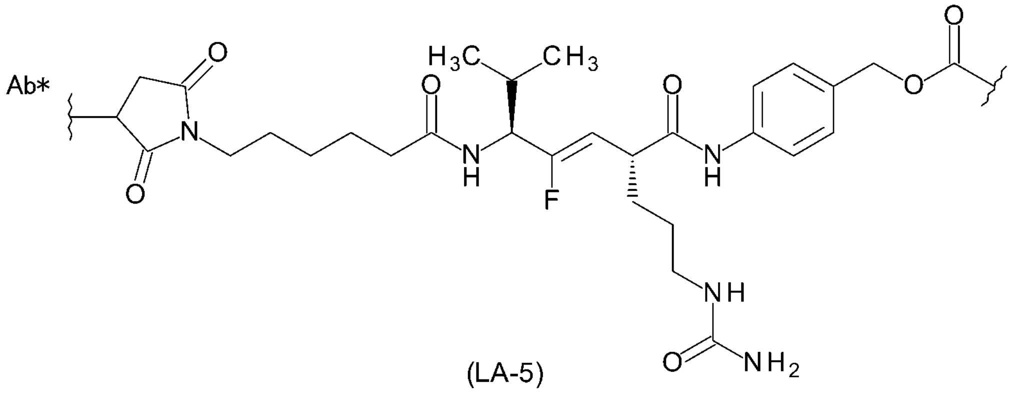

(LA-5)

A linker of the following formula (LA-6) (see International Publication No. WO 2015/095227; wherein *Ab represents a site of connection to Ab):

[Chemical Formula 120]

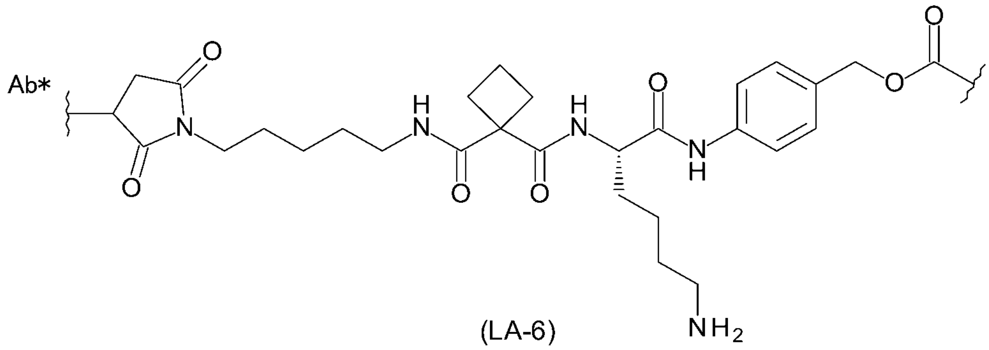

(LA-6)

A linker of the following formula (LA-7) (see International Publication No. WO 2014/057687; wherein *Ab represents a site of connection to Ab):

[Chemical Formula 121]

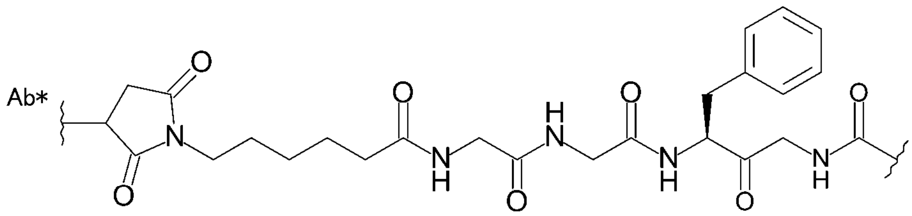

(LA-7)

A linker of the following formula (LA-8) (see International Publication No. WO 2013/173337; wherein *Ab represents a site of connection to Ab):

[Chemical Formula 122]

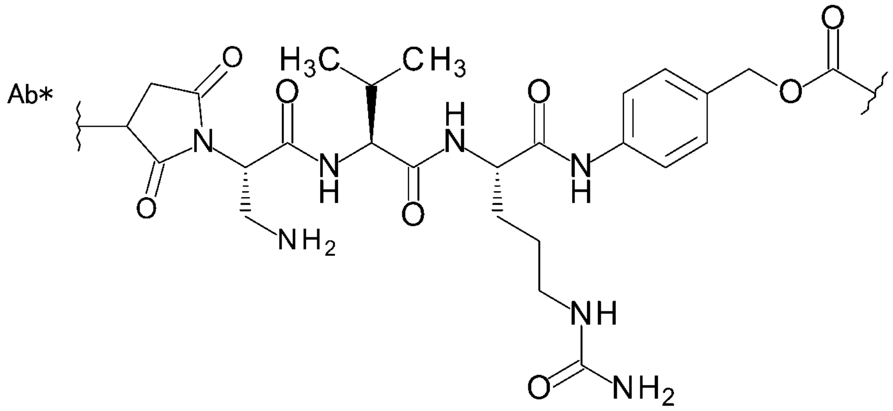

(LA-8)

3. Drug-linker conjugate ($L^A$-D)

As used herein, "drug-linker conjugate" is a compound of linker ($L^A$) and drug (D) connected to each other through a covalent bond. The drug-linker conjugate of the present invention or a salt thereof has a reactive site, is capable of forming a covalent bond through reaction with a functional group contained in antibody or antigen binding fragment (Ab), and is useful as an intermediate for synthesizing the antibody-drug conjugate of the present invention or the salt thereof. Examples of the functional group contained in Ab, which forms a covalent bond with the drug-linker conjugate of the present invention or the salt thereof, include, but are not particularly limited to, a mercapto group, an amino group, a hydroxyl group, and a carboxyl group. It is a mercapto group in certain embodiments. A mercapto group that can be used in covalent bond formation with Ab may be generated by reducing an intramolecular disulfide bond contained in Ab.

For example, when the drug-linker conjugate of the present invention or the salt thereof has a maleimide structure, the antibody-drug conjugate of the present invention or the salt thereof can be synthesized by forming the following partial structure through reaction with a mercapto group contained in Ab (wherein Ab'-SH represents Ab having a mercapto group).

[Chemical Formula 123]

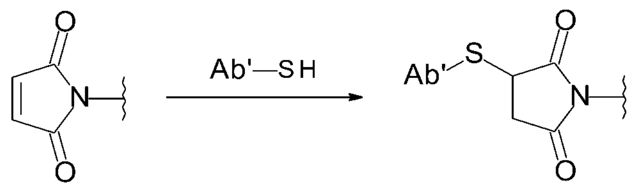


Embodiments of the drug-linker conjugate of the present invention or the salt thereof will be shown below.

(25-1) A drug-linker conjugate represented by formula (LD-1) or a salt thereof:

[Chemical Formula 124]

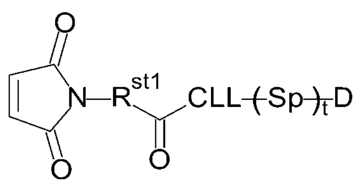

(LD-1)

wherein

D is a heterocyclic compound having a G12D mutant KRAS protein degradation-inducing effect,

$R^{st1}$ is $C_{1-12}$ alkylene,
CLL is a partial structure cleavable *in vivo,*
Sp is a spacer unit and is bonded to CLL and D, and t is 0 or 1.

(25-2) The drug-linker conjugate or the salt thereof according to (25-1), wherein D is a heterocyclic compound represented by formula (II):

[Chemical Formula 125]

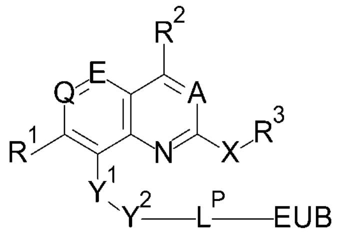

(II)

wherein

A is $CR^A$ or N,
$R^A$ is H, cyano, or optionally substituted $C_{1-3}$ alkyl,
Q is $CR^Q$ or N,
$R^Q$ is H, halogen, $C_{3-6}$ cycloalkyl, vinyl, or optionally substituted $C_{1-3}$ alkyl,
E is CH or N,
$R^1$ is naphthyl optionally substituted by one or two groups selected from the group consisting of cyano, OH, halogen, and optionally substituted $C_{1-3}$ alkyl, or is one group selected from the group consisting of the following formula (III), formula (IV), and formula (V):

[Chemical Formula 126]

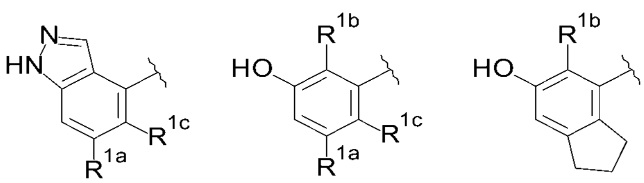

(III) (IV) (V)

$R^{1a}$, $R^{1b}$, and $R^{1c}$ are each independently H, vinyl, halogen, or optionally substituted $C_{1-3}$ alkyl,
$R^2$ is $-V^1-V^2$ or W,
$V^1$ is a bond, $-CH_2-$, -O-, -S-, or $-N(R^{V1})-$,
$R^{V1}$ is H or optionally substituted $C_{1-3}$ alkyl,
$V^2$ is one group selected from the group consisting of the following formula (VI) and formula (VII):

[Chemical Formula 127]

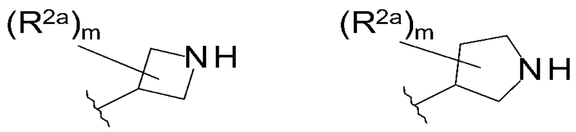

(VI) (VII)

W is one group selected from the group consisting of the following formula (VIII), formula (IX), formula (X), formula (XI), formula (XII), formula (XIII), formula (XIV), formula (XV), and formula (XVI):

[Chemical Formula 128]

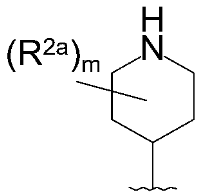
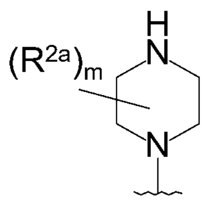
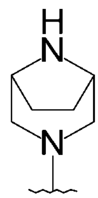
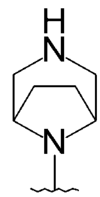

(VIII) (IX) (X) (XI)

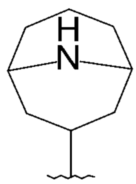
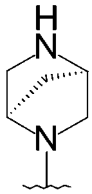
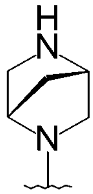
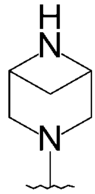
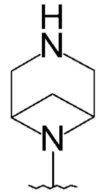

(XII) (XIII) (XIV) (XV) (XVI)

$R^{2a}$ are each independently OH, $OCH_3$, F, or optionally substituted $C_{1-3}$ alkyl, wherein $R^{2a}$ is bonded only to a carbon atom serving as a constituting atom of a ring selected from the group consisting of an azetidine ring represented by formula (VI), a pyrrolidine ring represented by formula (VII), a piperidine ring represented by formula (VIII), and a piperazine ring represented by formula (IX), m is an integer of 0 to 2,
$R^3$ is optionally substituted $C_{1-6}$ alkyl, optionally substituted hetero cycloalkyl, or optionally substituted heteroaryl,
X is a bond, $-CH_2-$, -O-, -S-, or $-NR^{4X}-$,
$R^{4X}$ is H or optionally substituted $C_{1-3}$ alkyl,
$Y^1$ is -O-(optionally substituted $C_{1-3}$ alkylene)-$^{*Y2}$, -S-(optionally substituted $C_{1-3}$ alkylene)-$^{*Y2}$, $-SO_2$-(optionally substituted $C_{1-3}$ alkylene)-$^{*Y2}$, $-NR^Y$-(optionally substituted $C_{1-3}$ alkylene)-$^{*Y2}$, -(optionally substituted $C_{1-3}$ alkylene)-O-$^{*Y2}$, -(optionally substituted $C_{1-3}$ alkylene)-S-$^{*Y2}$, -(optionally substituted $C_{1-3}$ alkylene)-$SO_2$-$^{*Y2}$, or -(optionally substituted $C_{1-3}$ alkylene)-$NR^Y$-$^{*Y2}$ (wherein $^{*Y2}$ represents a site of connection to $Y^2$),
$R^Y$ is H or optionally substituted $C_{1-3}$ alkyl,
$Y^2$ is a bond, optionally substituted phenylene, or optionally substituted heteroarylene,
$L^P$ is a group that chemically bonds $Y^2$ to EUB,
EUB is a group having the ability to bind to one E3 ubiquitin ligase selected from the group consisting of VHL, celebron, IAP, MDM2, DCAF11, DCAF15, DCAF16, BIRC2, KEAP1, RNF4, RNF114, FEM1B, and AhR,
CLL is a partial structure cleavable *in vivo*, and
Sp is a spacer unit and is bonded to CLL and D, wherein Sp is bonded to an arbitrary nitrogen atom of -NH- or oxygen atom of -OH contained in D.

(25-3) The drug-linker conjugate or the salt thereof according to (25-2), wherein

$V^2$ is one group selected from the group consisting of the following formula (VIb) and formula (VIIb) (wherein $^{*SP}$ represents a site of connection to Sp):

[Chemical Formula 129]

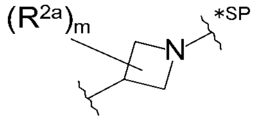
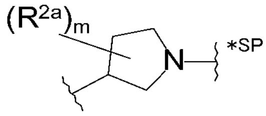

(VIb) (VIIb)

W is one group selected from the group consisting of the following formula (VIIIb), formula (IXb), formula (Xb), formula (XIb), formula (XIIb), formula (XIIIb), formula (XIVb), formula (XVb), and formula (XVIb) (wherein $^{*SP}$ represents a site of connection to Sp):

[Chemical Formula 130]

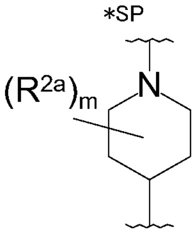
(VIIIb)
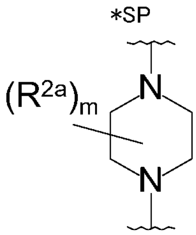
(IXb)
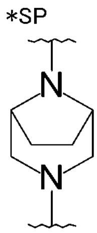
(Xb)
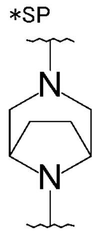
(XIb)

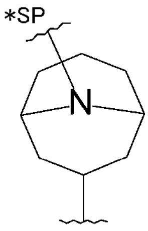
(XIIb)
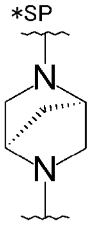
(XIIIb)
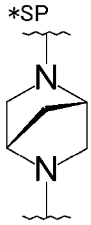
(XIVb)
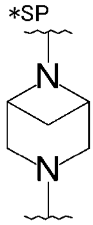
(XVb)
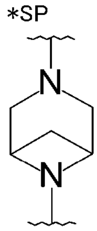
(XVIb)

and
Sp is a spacer unit and is bonded to CLL and D, wherein Sp is bonded to D through a nitrogen atom contained in $V^2$ or W at the site of connection represented by $^{*SP}$.

(25-4) The drug-linker conjugate or the salt thereof according to (25-2), wherein

A is N,
Q is $CR^Q$, $R^Q$ is cyclopropyl,
E is CH,
$R^1$ is the following formula (III-2):

[Chemical Formula 131]

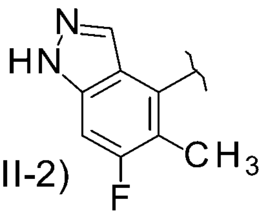
(III-2)

$R^2$ is $-V^1-V^2$ or W,
$V^1$ is $-N(CH_3)-$,
$V^2$ is the following formula (VII-2):

[Chemical Formula 132]

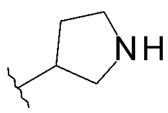
(VII-2)

W is the following formula (XIV):

[Chemical Formula 133]

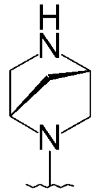

(XIV)

$R^3$ is n-propyl optionally substituted by $-OCH_3$, or tetrahydropyranyl,
X is -O-,
$Y^1$ is -O-(methylene)-$^{*Y2}$ (wherein $^{*Y2}$ represents a site of connection to $Y^2$),
$Y^2$ is phenylene,
$L^P$ is a group that chemically bonds $Y^2$ to EUB, and
EUB is a group having the ability to bind to one E3 ubiquitin ligase selected from the group consisting of VHL and celebron.

(25-5) The drug-linker conjugate or the salt thereof according to (25-4), wherein

$V^2$ is the following formula (VII-2b) (wherein $^{*SP}$ represents a site of connection to Sp):

[Chemical Formula 134]

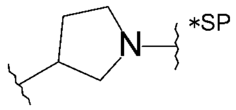

(VII-2b)

W is the following formula (XIVb) (wherein $^{*SP}$ represents a site of connection to Sp):

[Chemical Formula 135]

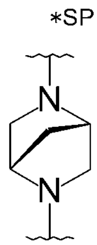

(XIVb)

and
Sp is a spacer unit and is bonded to CLL and D, wherein Sp is bonded to D through a nitrogen atom contained in $V^2$ or W at the site of connection represented by $^{*SP}$.

(25-6) The drug-linker conjugate or the salt thereof according to (25-1), wherein D is (4R)-1-[(2S)-2-(4-{4-[({6-cyclopropyl-4-[(1S,4S)-2,5-diazabicyclo[2.2.1]heptan-2-yl]-7-(6-fluoro-5-methyl-1H-indazol-4-yl)-2-[(oxan-4-yl)oxy]quinazolin-8-yl}oxy)methyl]phenyl}-1H-1,2,3-triazol-1-yl)-3-methylbutanoyl]-4-hydroxy-N-{(1R)-2-hydroxy-1-[4-(4-methyl-1,3-thiazol-5-yl)phenyl]ethyl}-L-prolinamide, (4R)-1-[(2S)-2-(4-{4-[({6-cyclopropyl-4-[(1S,4S)-2,5-diazabicyclo[2.2.1]heptan-2-yl]-7-(6-fluoro-5-methyl-1H-indazol-4-yl)-2-[(oxan-4-yl)oxy]quinazolin-8-yl}oxy)methyl]phenyl}-1H-1,2,3-triazol-1-yl)-3-methylbutanoyl]-4-hydroxy-N-{(1R)-2-hydroxy-1-[4-(2-oxo-1,3-oxazolidin-3-yl)phenyl]ethyl}-L-prolinamide, (4R)-1-[(2S)-2-(4-{4-[({6-cyclopropyl-4-[(1S,4S)-2,5-diazabicyclo[2.2.1]heptan-2-yl]-7-(6-fluoro-5-methyl-1H-indazol-4-yl)-2-[(oxan-4-yl)oxy]quinazolin-8-yl}oxy)methyl]phenyl}-1H-1,2,3-triazol-1-yl)-3-methylbutanoyl]-4-hydroxy-N-{(1R)-2-hydroxy-1-[4-(1-methyl-1H-pyrazol-5-yl)phenyl]ethyl}-L-prolinamide, (4R)-1-[(2S)-2-(4-{4-[({6-cyclopropyl-4-[(1S,4S)-2,5-diazabicyclo[2.2.1]heptan-2-yl]-7-(6-fluoro-5-methyl-1H-indazol-4-yl)-2-[(2S)-2-methoxypropoxy]quinazolin-8-yl}oxy)methyl]phenyl}-1H-1,2,3-triazol-1-yl)-3-methylbutanoyl]-4-hydroxy-N-{(1R)-2-hydroxy-1-[4-(4-methyl-1,3-thiazol-5-yl)phenyl]ethyl}-L-prolinamide, (4R)-1-[(2S)-2-(4-{4-[({6-cyclopropyl-4-[(1S,4S)-2,5-diazabicyclo[2.2.1]heptan-2-yl]-7-(6-fluoro-5-methyl-1H-indazol-4-yl)-2-[(2S)-2-methoxypropoxy]quinazolin-8-yl}oxy)methyl]phenyl}-1H-1,2,3-triazol-1-yl)-3-methylbutanoyl]-N-{(1R)-1-[4-(1-ethyl-1H-pyrazol-5-yl)phenyl]-2-hydroxyethyl}-4-hydroxy-L-prolinamide, (4R)-1-[(2S)-2-(4-{4-[({6-cyclopropyl-4-[(1S,4S)-2,5-diazabicyclo[2.2.1]heptan-2-yl]-7-(6-fluoro-5-methyl-1H-indazol-4-yl)-2-[(2S)-2-methoxypropoxy]quinazolin-8-yl}oxy)methyl]phenyl}-1H-1,2,3-triazol-1-yl)-3-methylbutanoyl]-4-hydroxy-N-{(1R)-2-hydroxy-1-[4-(4-methyl-1,3-oxazol-5-yl)phenyl]ethyl}-L-prolinamide, (4R)-1-[(2S)-2-{4-[4-({[(7M)-6-cyclopropyl-7-(6-fluoro-5-methyl-1H-indazol-4-yl)-2-[(2S)-2-methoxypropoxy]-4-{methyl[(3S)-pyrrolidin-3-yl]amino}quinazolin-8-yl]oxy}methyl)phenyl]-1H-1,2,3-triazol-1-yl}-3-methylbutanoyl]-4-hydroxy-N-{(1R)-2-hydroxy-1-[4-(4-methyl-1,3-thiazol-5-yl)phenyl]ethyl}-L-prolinamide, (4R)-1-[(2S)-2-{4-[4-({[(7M)-6-cyclopropyl-7-(6-fluoro-5-methyl-1H-indazol-4-yl)-2-[(2S)-2-methoxypropoxy]-4-{methyl[(3S)-pyrrolidin-3-yl]amino}quinazolin-8-yl]oxy}methyl)phenyl]-1H-1,2,3-triazol-1-yl}-3-methylbutanoyl]-N-{(1R)-1-[4-(1-ethyl-1H-pyrazol-5-yl)phenyl]-2-hydroxyethyl}-4-hydroxy-L-prolinamide, 3-(5-{[(3S)-4-{4-[({(7M)-6-cyclopropyl-4-[(1S,4S)-2,5-diazabicyclo[2.2.1]heptan-2-yl]-7-(6-fluoro-5-methyl-1H-indazol-4-yl)-2-[(oxan-4-yl)oxy]quinazolin-8-yl}oxy)methyl]benzoyl}-3-methylpiperazin-1-yl]methyl}-3-methyl-2-oxo-2,3-dihydro-1H-benzimidazol-1-yl)piperidine-2,6-dione, 1-(6-{[(3S)-4-{4-[({(7M)-6-cyclopropyl-4-[(1S,4S)-2,5-diazabicyclo[2.2.1]heptan-2-yl]-7-(6-fluoro-5-methyl-1H-indazol-4-yl)-2-[(oxan-4-yl)oxy]quinazolin-8-yl}oxy)methyl]benzoyl}-3-methylpiperazin-1-yl]methyl}-1-methyl-1H-indazol-3-yl)-1,3-diazinane-2,4-dione, 3-(5-{[(3S)-4-{4-[({(7M)-6-cyclopropyl-4-[(1S,4S)-2,5-diazabicyclo[2.2.1]heptan-2-yl]-7-(6-fluoro-5-methyl-1H-indazol-4-yl)-2-[(2S)-2-methoxypropoxy]quinazolin-8-yl}oxy)methyl]benzoyl}-3-methylpiperazin-1-yl]methyl}-3-methyl-2-oxo-2,3-dihydro-1H-benzimidazol-1-yl)piperidine-2,6-dione, or (2R)-2-({(4R)-1-[(2S)-2-{4-[4-({[(7M)-6-cyclopropyl-7-(6-fluoro-5-methyl-1H-indazol-4-yl)-2-[(2S)-2-methoxypropoxy]-4-{methyl[(3S)-pyrrolidin-3-yl]amino}quinazolin-8-yl]oxy}methyl)phenyl]-1H-1,2,3-triazol-1-yl}-3-methylbutanoyl]-4-hydroxy-L-prolyl}amino)-2-[4-(1H-1,2,4-triazol-1-yl)phenyl]ethyl dihydrogen phosphate.

(25-7) The drug-linker conjugate or the salt thereof according to (25-1), wherein formula (LD-1) is represented by formula (LD-2a) or (LD-3a):

[Chemical Formula 136]

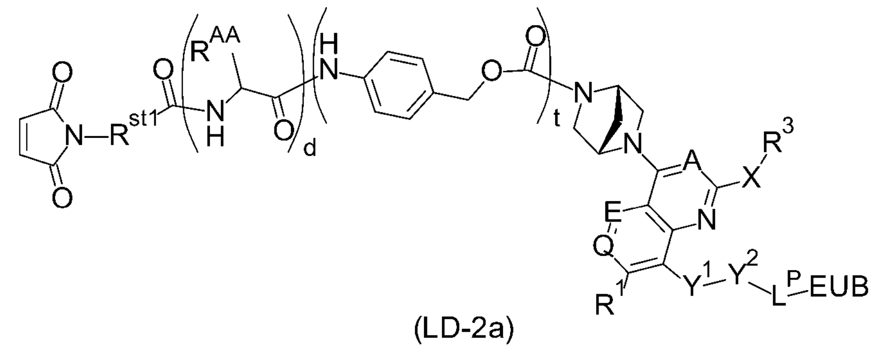

(LD-2a)

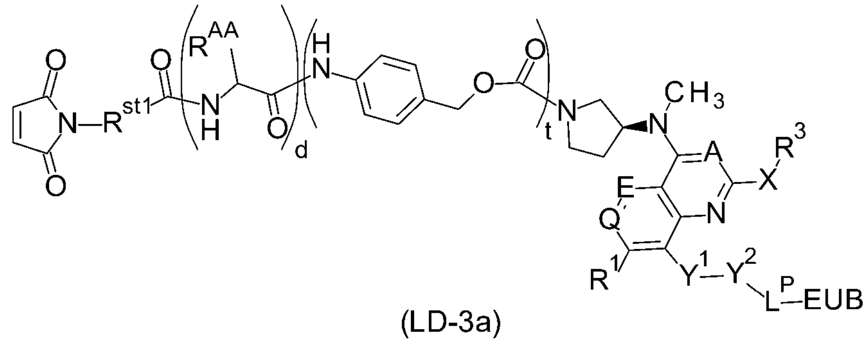

(LD-3a)

wherein

A is $CR^A$ or N,

$R^A$ is H, cyano, or optionally substituted $C_{1-3}$ alkyl,
Q is $CR^Q$ or N,
$R^Q$ is H, halogen, $C_{3-6}$ cycloalkyl, vinyl, or optionally substituted $C_{1-3}$ alkyl,
E is CH or N,
$R^1$ is naphthyl optionally substituted by one or two groups selected from the group consisting of cyano, OH, halogen, and optionally substituted $C_{1-3}$ alkyl, or is one group selected from the group consisting of the following formula (III), formula (IV), and formula (V):

[Chemical Formula 137]

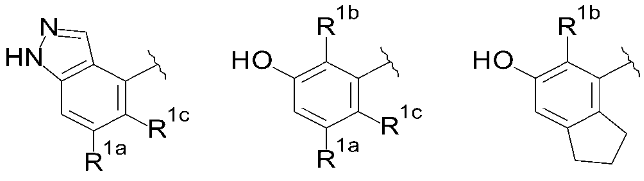

(III) (IV) (V)

$R^{1a}$, $R^{1b}$, and $R^{1c}$ are each independently H, vinyl, halogen, or optionally substituted $C_{1-3}$ alkyl,
$R^3$ is optionally substituted $C_{1-6}$ alkyl, optionally substituted hetero cycloalkyl, or optionally substituted heteroaryl,
X is a bond, $-CH_2-$, -O-, -S-, or $-NR^{4X}-$,
$R^{4X}$ is H or optionally substituted $C_{1-3}$ alkyl,
$Y^1$ is -O-(optionally substituted $C_{1-3}$ alkylene)-$^{*Y2}$, -S-(optionally substituted $C_{1-3}$ alkylene)-$^{*Y2}$, $-SO_2$-(optionally substituted $C_{1-3}$ alkylene)-$^{*Y2}$, $-NR^Y$-(optionally substituted $C_{1-3}$ alkylene)-$^{*Y2}$, -(optionally substituted $C_{1-3}$ alkylene)-O-$^{*Y2}$, -(optionally substituted $C_{1-3}$ alkylene)-S-$^{*Y2}$, -(optionally substituted $C_{1-3}$ alkylene)-$SO_2$-$^{*Y2}$, or -(optionally substituted $C_{1-3}$ alkylene)-$NR^Y$-$^{*Y2}$ (wherein $^{*Y2}$ represents a site of connection to $Y^2$),
$R^Y$ is H or optionally substituted $C_{1-3}$ alkyl,
$Y^2$ is a bond, optionally substituted phenylene, or optionally substituted heteroarylene,
$L^P$ is a group that chemically bonds $Y^2$ to EUB,
EUB is a group having the ability to bind to one E3 ubiquitin ligase selected from the group consisting of VHL and celebron,
$R^{st1}$ is $C_{1-12}$ alkylene,
$R^{AA}$ are each independently H, methyl, isopropyl, benzyl, or $-(CH_2)_3-NH-C(=O)-NH_2$, d is an integer of 2 to 4, and t is 0 or 1.

(25-8) The drug-linker conjugate or the salt thereof according to (25-1), wherein formula (LD-1) is represented by formula (LD-4a), (LD-5a), (LD-6a), (LD-7a), (LD-8a), (LD-9a), (LD-10a), (LD-11a), (LD-12a), (LD-13a), (LD-14a), or (LD-26a):

[Chemical Formula 138-1]

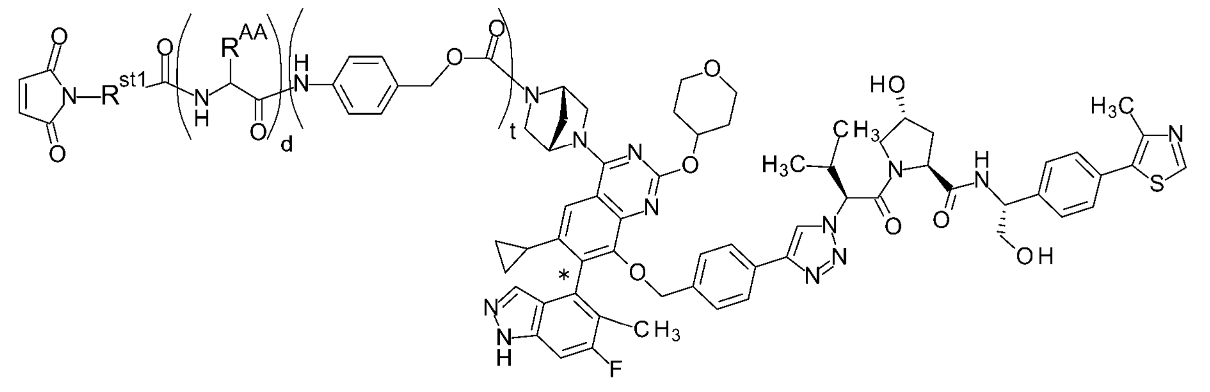

(LD-4a)

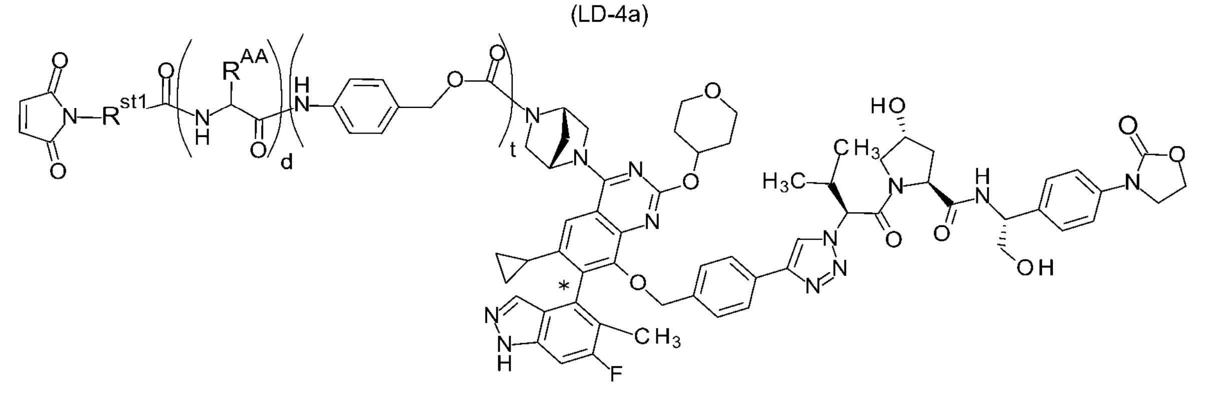

(LD-5a)

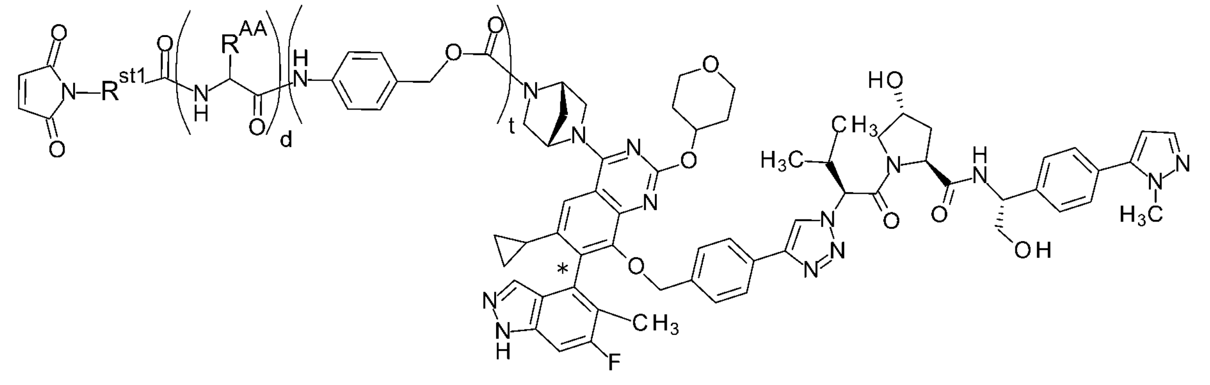

(LD-6a)

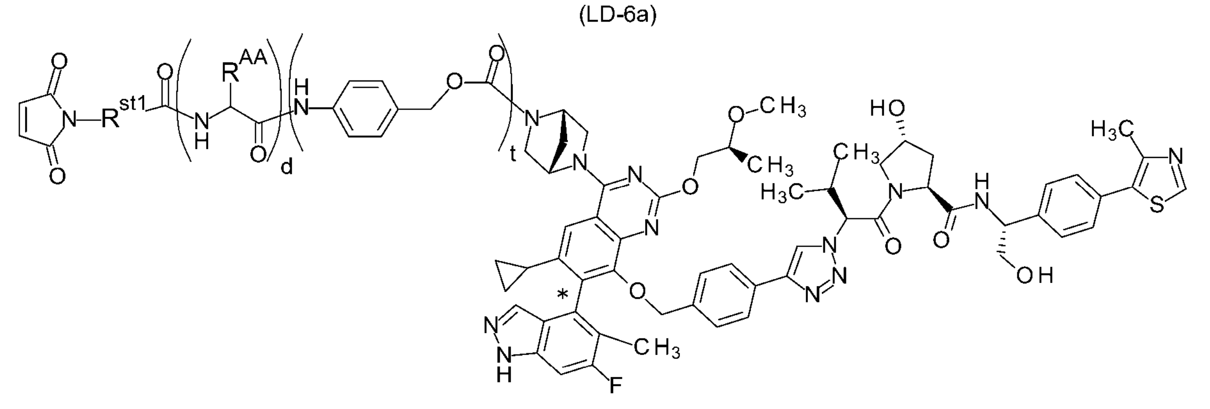

(LD-7a)

[Chemical Formula 138-2]

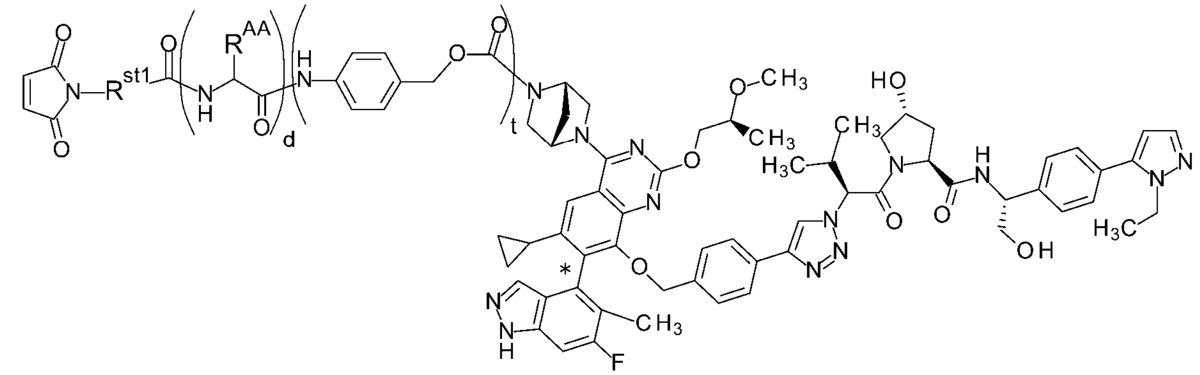

(LD-8a)

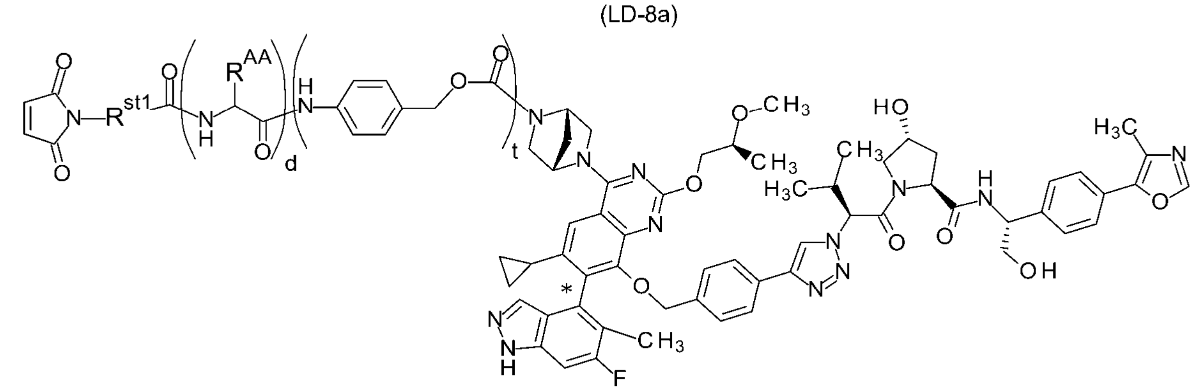

(LD-9a)

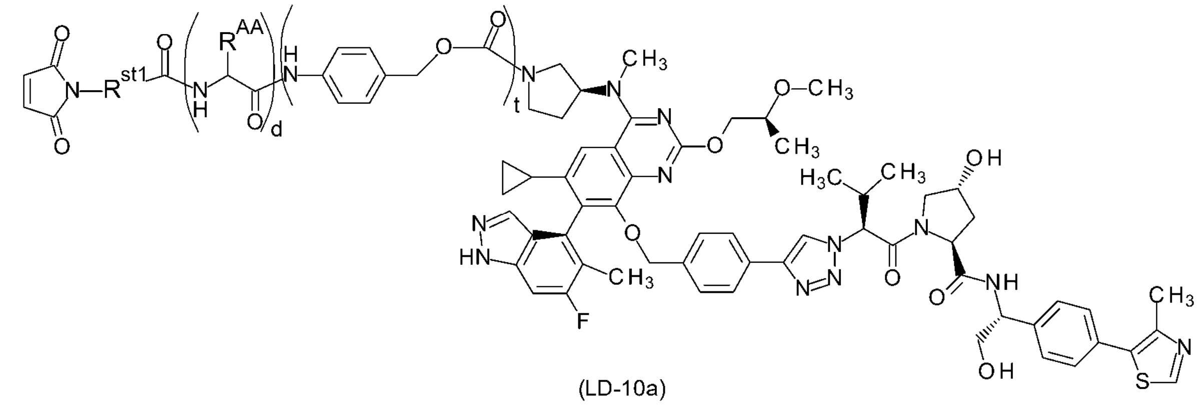

(LD-10a)

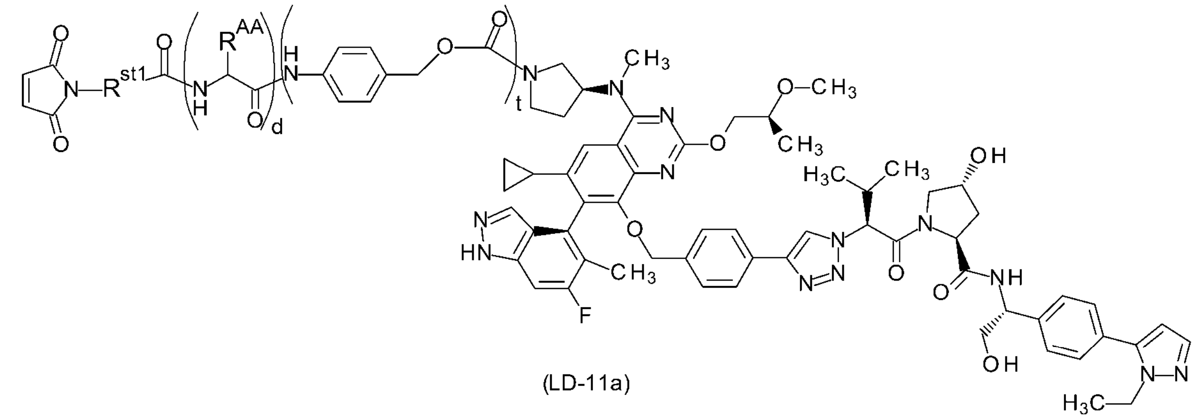

(LD-11a)

[Chemical Formula 138-3]

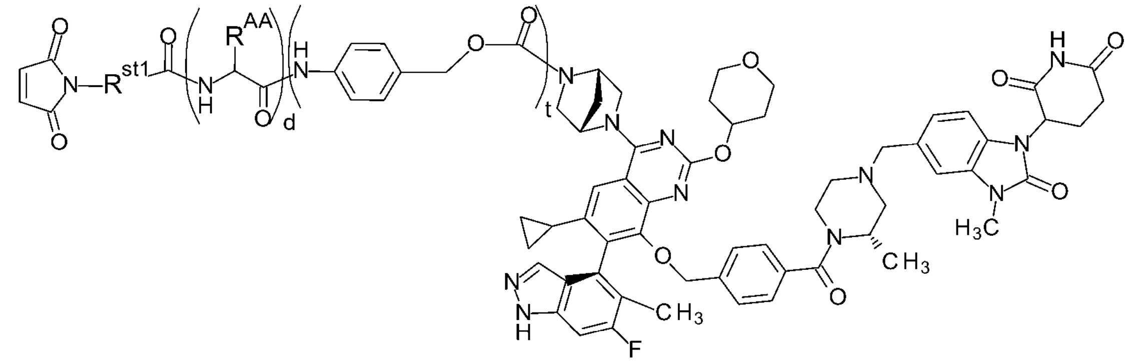

(LD-12a)

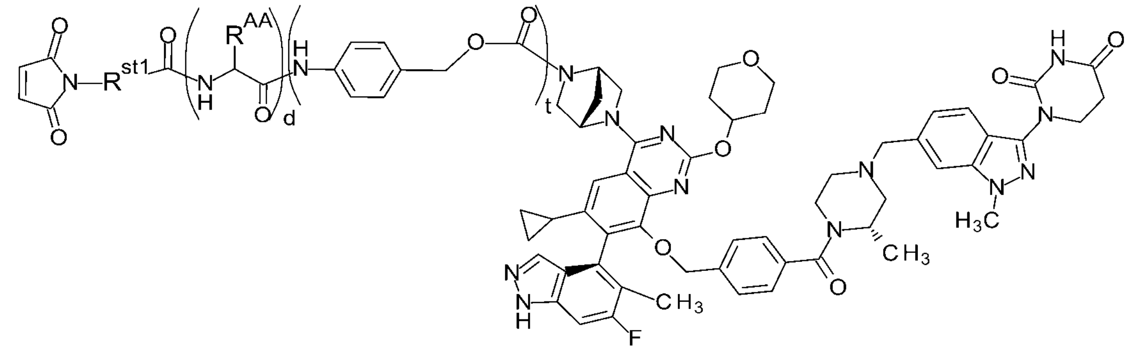

(LD-13a)

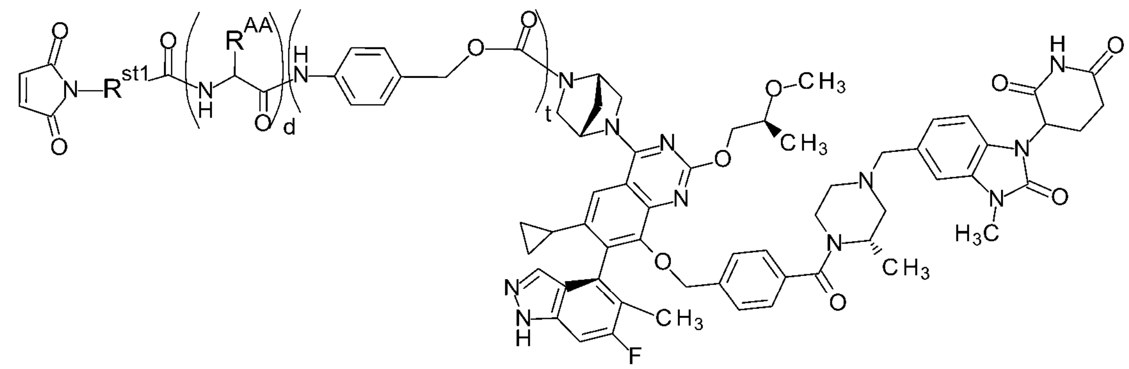

(LD-14a)

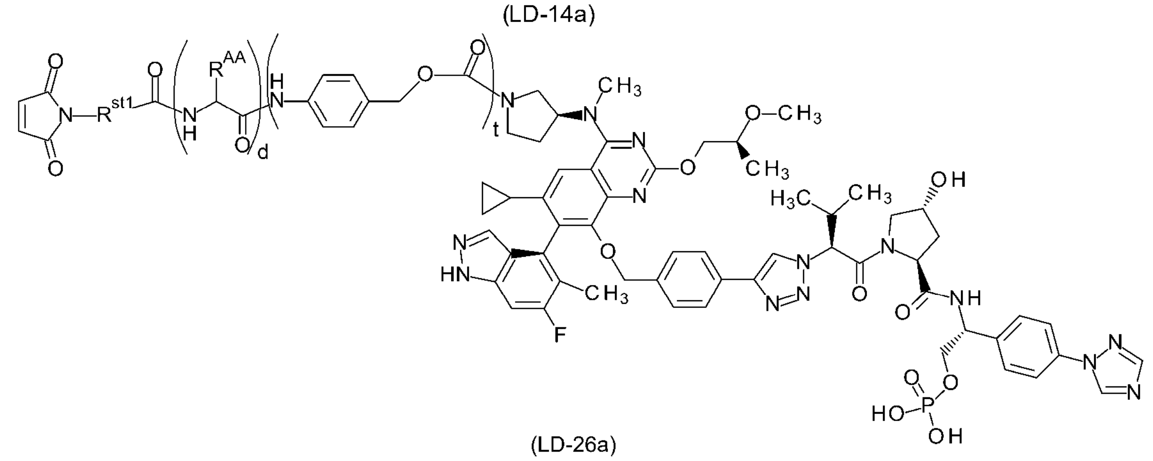

(LD-26a)

wherein $R^{st1}$ is $C_{1-12}$ alkylene, $R^{AA}$ are each independently H, methyl, isopropyl, benzyl, or - $(CH_2)_3$-NH-C(=O)-$NH_2$, d is an integer of 2 to 4, and t is 0 or 1.

(25-9) The drug-linker conjugate or the salt thereof according to (25-1), wherein formula (LD-1) is represented by formula (LD-15), (LD-16), (LD-17), (LD-18), (LD-19), (LD-20), (LD-21), (LD-22), (LD-23), (LD-24), (LD-25), or (LD-26):

[Chemical Formula 139-1]

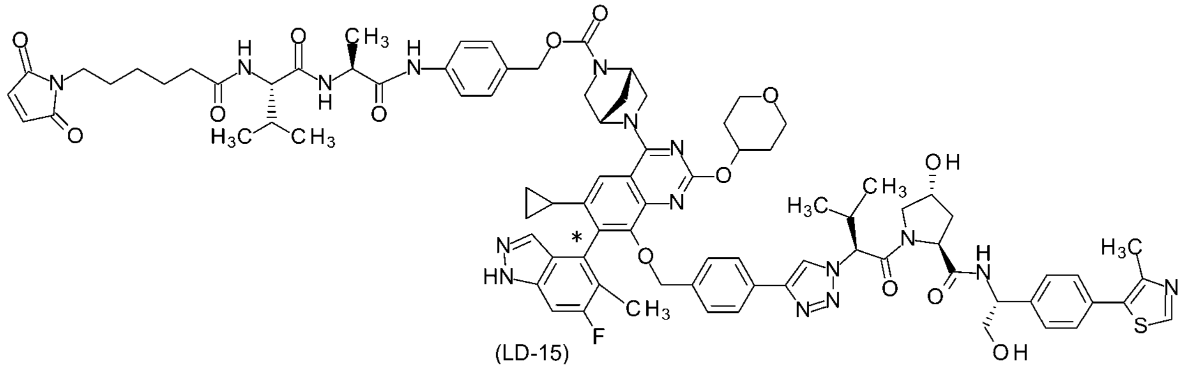

(LD-15)

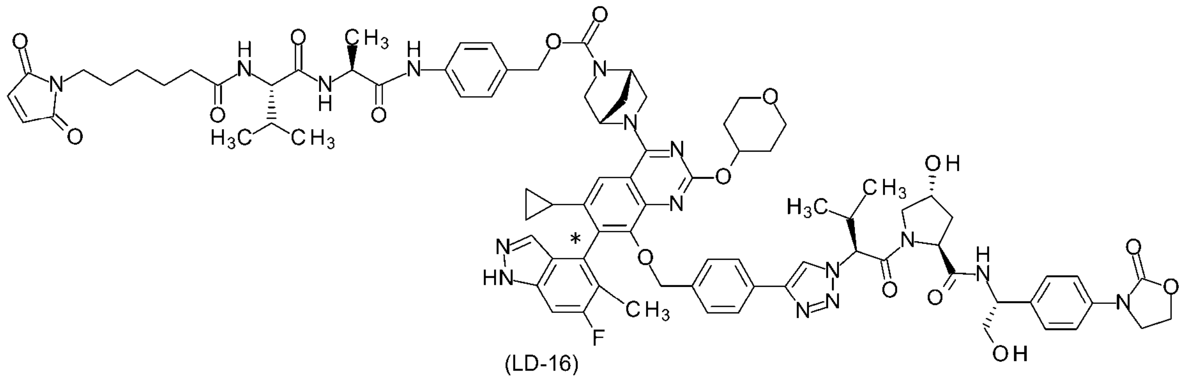

(LD-16)

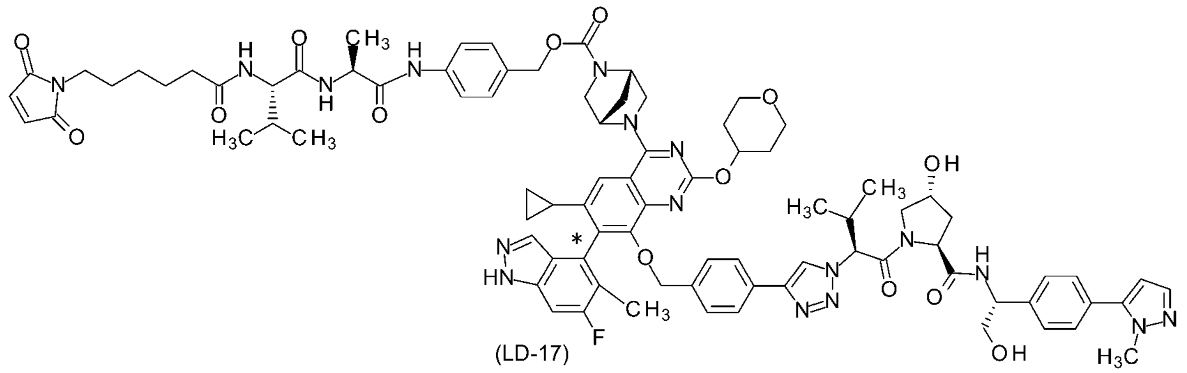

(LD-17)

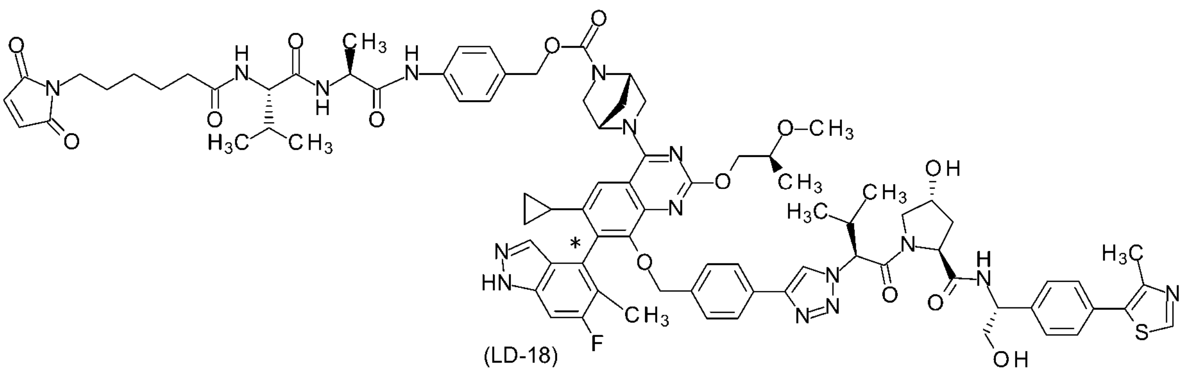

(LD-18)

[Chemical Formula 139-2]

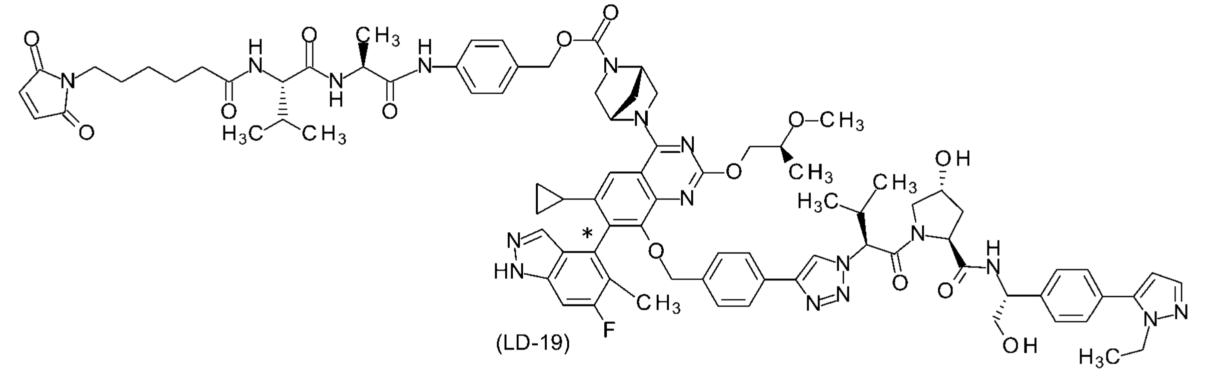

(LD-19)

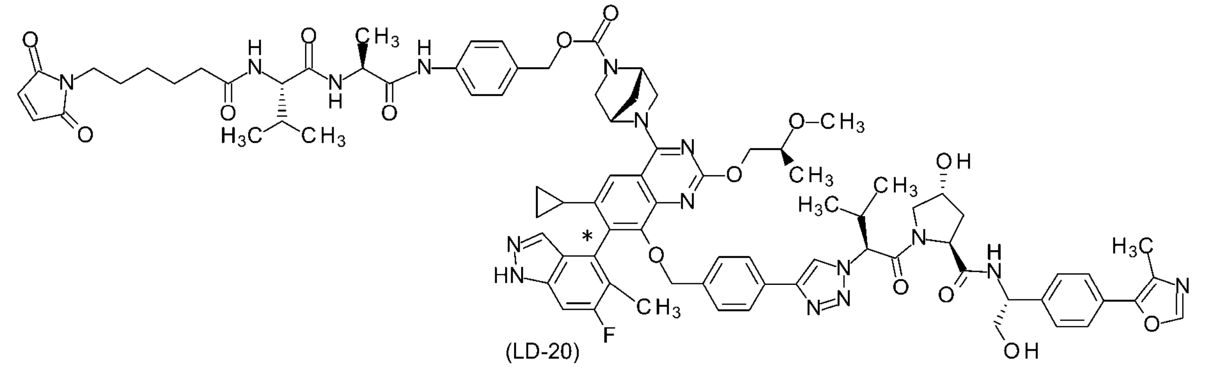

(LD-20)

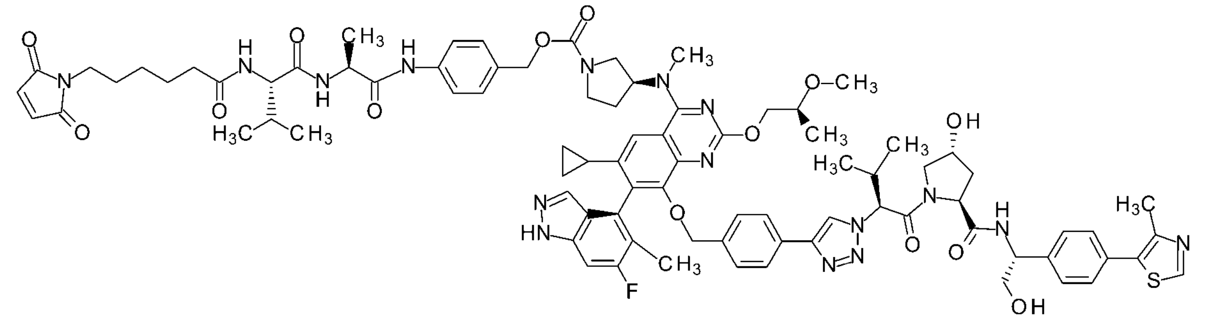

(LD-21)

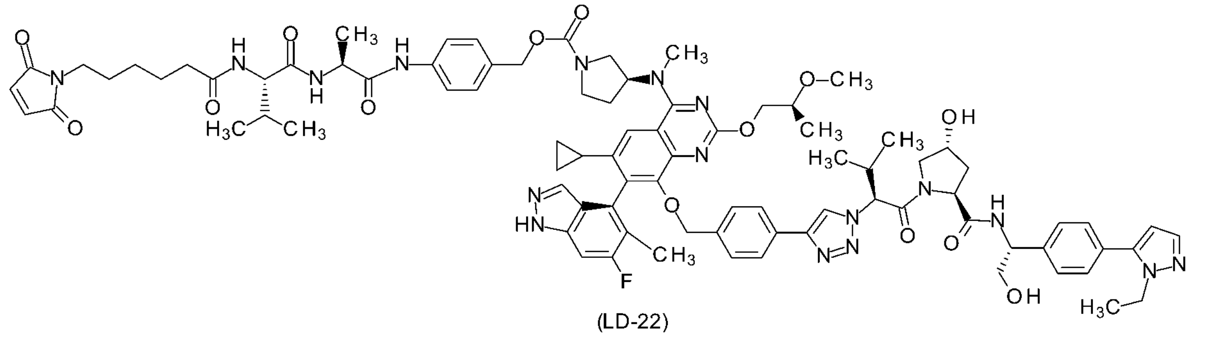

(LD-22)

[Chemical Formula 139-3]

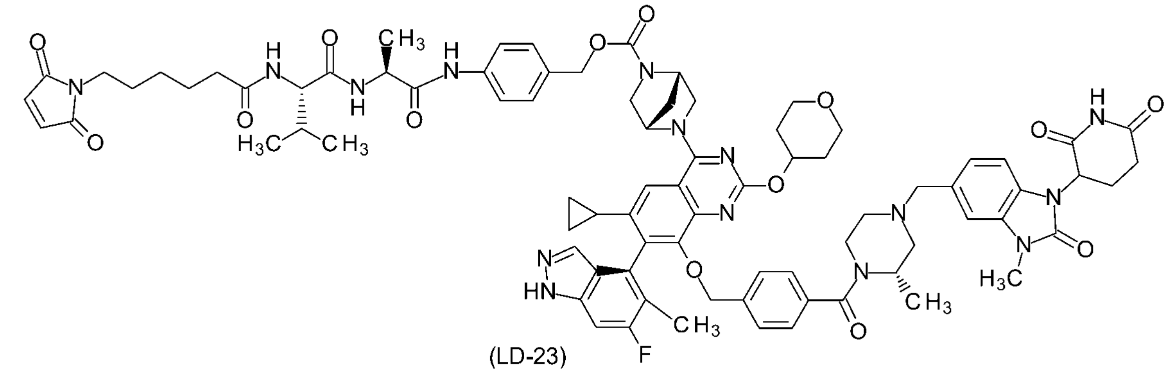

(LD-23)

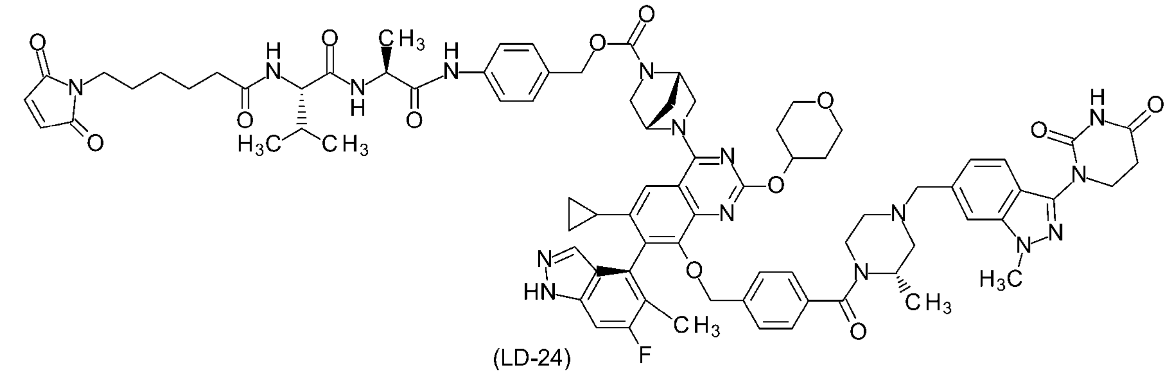

(LD-24)

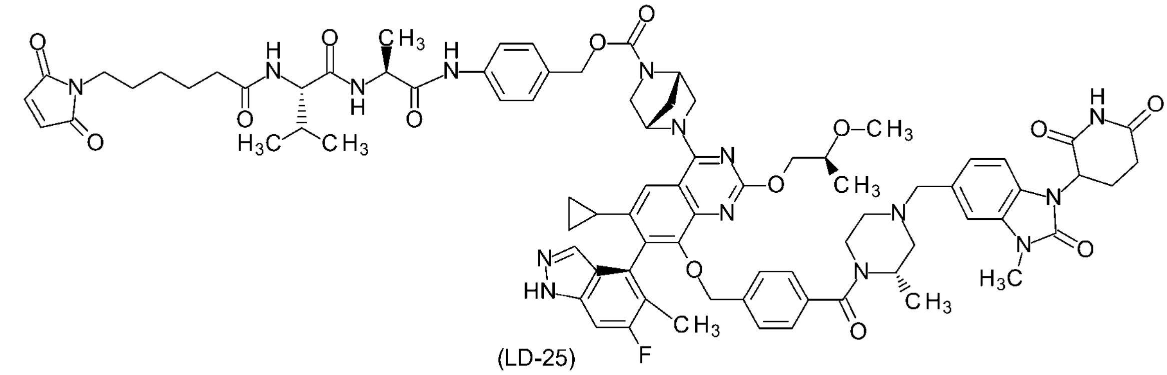

(LD-25)

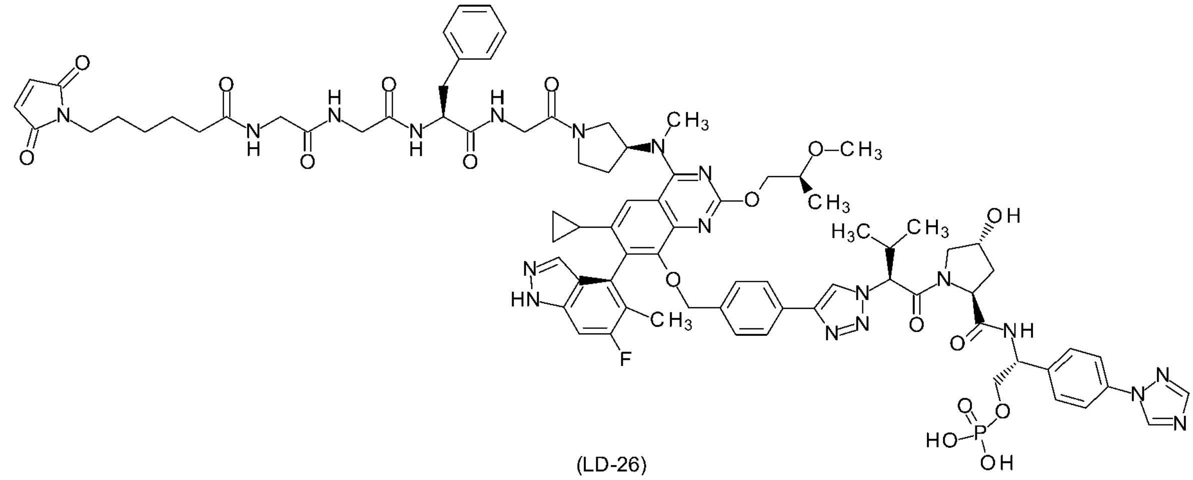

(LD-26)

4. Antibody or antigen binding fragment (Ab)

The antibody or the antigen binding fragment (also referred to as "Ab" in the present specification) used in the present invention binds to an antigen protein expressed on cell surface or a modified form (e.g., glycan modification) thereof. In certain embodiments, the antibody or the antigen binding fragment used in the present invention binds to a tumor-associated antigen or a cell surface receptor expressed on cancer cell surface.

In certain embodiments, Ab used in the present invention can be an antibody or an antigen binding fragment that binds to one or two or more antigens selected from the group consisting of 5T4, ADAM9, ALPP, ALPPL2, AXL, B7H3, B7H4, BCMA, CA9, CCR2, CCR7, CD123, CD166, CD19, CD20, CD22, CD25, CD30, CD33, CD37, CD38, CD45, CD46, CD70, CD74, CD79b, CDH3, CDH6, CLDN1, CLDN4, CLDN6, CLDN18.2, cMET, EGFR, EphA3, FAP, FGFR3, Fibronectin, FOLRa, Globo H, GPRC5D, HER2, HER3, IGF1R, Integrin $\alpha$V, KAAG1, LIV1, MSLN, MT1-MMP, MUC1, MUC4, NaPi2b, Nectin4, PD-L1, PSMA, PTK7, ROR1, ROR2, SEZ6, SialylTn, TF, TROP2, TSPAN8, and VEGF. Ab is an antibody or an antigen binding fragment that binds to one or two or more antigens selected from the group consisting of 5T4, ADAM9, ALPP, ALPPL2, AXL, B7H3, B7H4, BCMA, CA9, CCR2, CCR7, CD123, CD166, CD19, CD20, CD22, CD25, CD30, CD33, CD37, CD38, CD45, CD46, CD70, CD74, CD79b, CDH3, CDH6, CLDN1, CLDN6, EGFR, EphA3, FAP, FGFR3, Fibronectin, FOLRa, Globo H, GPRC5D, IGF1R, Integrin $\alpha$V, KAAG1, LIV1, MSLN, MT1-MMP, MUC4, NaPi2b, Nectin4, PD-L1, PSMA, PTK7, ROR1, ROR2, SEZ6, SialylTn, TF, and VEGF in certain embodiments, and is an antibody or an antigen binding fragment that binds to one or two or more antigens selected from the group consisting of EGFR, HER2, cMET and TROP2 in certain embodiments, and is an antibody or an antigen binding fragment that binds to EGFR in certain embodiments.

In certain embodiments, Ab used in the present invention can be one or two or more antibodies selected from the group consisting of an anti-5T4 antibody, an anti-ADAM9 antibody, an anti-ALPP antibody, an anti-ALPPL2 antibody, an anti-AXL antibody, an anti-B7H3 antibody, an anti-B7H4 antibody, an anti-BCMA antibody, an anti-CA9 antibody, an anti-CCR2 antibody, an anti-CCR7 antibody, an anti-CD123 antibody, an anti-CD166 antibody, an anti-CD19 antibody, an anti-CD20 antibody, an anti-CD22 antibody, an anti-CD25 antibody, an anti-CD30 antibody, an anti-CD33 antibody, an anti-CD37 antibody, an anti-CD38 antibody, an anti-CD45 antibody, an anti-CD46 antibody, an anti-CD70 antibody, an anti-CD74 antibody, an anti-CD79b antibody, an anti-CDH3 antibody, an anti-CDH6 antibody, an anti-CLDN1 antibody, an anti-CLDN4 antibody, an anti-CLDN6 antibody, an anti-CLDN18.2 antibody, an anti-cMET antibody, anti-EGFR antibody, an anti-EphA3 antibody, an anti-FAP antibody, an anti-FGFR3 antibody, an anti-Fibronectin antibody, an anti-FOLRa antibody, an anti-Globo H antibody, an anti-GPRC5D antibody, an anti-HER2 antibody, an anti-HER3 antibody, an anti-IGF1R antibody, an anti-Integrin$\alpha$V antibody, an anti-KAAG1 antibody, an anti-LIV1 antibody, an anti-MSLN antibody, an anti-MT1-MMP antibody, an anti-MUC1 antibody, an anti-MUC4 antibody, an anti-NaPi2b antibody, an anti-Nectin4 antibody, an anti-PD-L1 antibody, an anti-PSMA antibody, an anti-PTK7 antibody, an anti-ROR1 antibody, an anti-ROR2 antibody, an anti-SEZ6 antibody, an anti-SialylTn antibody, an anti-TF antibody, an anti-TROP2 antibody, an anti-TSPAN8 antibody, and an anti-VEGF antibody, or antigen binding fragments thereof. Ab used in the present invention is one or two or more antibodies selected from the group consisting of an anti-5T4 antibody, an anti-ADAM9 antibody, an anti-ALPP antibody, an anti-ALPPL2 antibody, an anti-AXL antibody, an anti-B7H3 antibody, an anti-B7H4 antibody, an anti-BCMA antibody, an anti-CA9 antibody, an anti-CCR2 antibody, an anti-CCR7 antibody, an anti-CD123 antibody, an anti-CD166 antibody, an anti-CD19 antibody, an anti-CD20 antibody, an anti-CD22 antibody, an anti-CD25 antibody, an anti-CD30 antibody, an anti-CD33 antibody, an anti-CD37 antibody, an anti-CD38 antibody, an anti-CD45 antibody, an anti-CD46 antibody, an anti-CD70 antibody, an anti-CD74 antibody, an anti-CD79b antibody, an anti-CDH3 antibody, an anti-CDH6 antibody, an anti-CLDN1 antibody, an anti-CLDN6 antibody, anti-EGFR antibody, an anti-EphA3 antibody, an anti-FAP antibody, an anti-FGFR3 antibody, an anti-Fibronectin antibody, an anti-FOLRa antibody, an anti-Globo H antibody, an anti-GPRC5D antibody, an anti-IGF1R antibody, an anti-Integrin$\alpha$V antibody, an anti-KAAG1 antibody, an anti-LIV1 antibody, an anti-MSLN antibody, an anti-MT1-MMP antibody, an anti-MUC4 antibody, an anti-NaPi2b antibody, an anti-Nectin4 antibody, an anti-PD-L1 antibody, an anti-PSMA antibody, an anti-PTK7 antibody, an anti-ROR1 antibody, an anti-ROR2 antibody, an anti-SEZ6 antibody, an anti-SialylTn antibody, an anti-TF antibody, and an anti-VEGF antibody, or antigen binding fragments thereof in certain embodiments, is one or two or more antibodies selected from the group consisting of anti-EGFR antibody, an anti-HER2 antibody, an anti-cMET antibody and an anti-TROP2 antibody, or antigen binding fragments thereof in certain embodiments, and is an anti-EGFR antibody or an antigen binding fragment thereof in certain embodiments.

Those skilled in the art can obtain, by a method known in the art, the antibody or the antigen binding fragment thereof used in the present invention. It can be obtained by use of a method that is usually carried out in the art, for example, by immunizing an animal with a polypeptide serving as an antigen, and collecting and purifying an antibody produced *in vivo.* The origin of the antigen is not limited to a human, and an animal may be immunized with an antigen derived from a non-human animal such as a mouse or a rat. In this case, an antibody applicable to a human disease can be selected by testing the obtained antibody that binds to the heterologous antigen for its cross-reactivity with the human antigen. In accordance with a method known in the art (e.g., Kohler and Milstein, Nature (1975) 256, p. 495-497; and Kennet, R. ed.,

Monoclonal Antibodies, p. 365-367, Plenum Press, N.Y. (1980)), antibody-producing cells that produce an antibody against the antigen are fused with myeloma cells so that hybridomas may be established to obtain a monoclonal antibody. The immunizing antigen can be obtained, for example, by transferring a gene encoding an arbitrary protein or polypeptide to host cells using a vector or the like, allowing the gene to be expressed, and purifying the expressed protein. Alternatively, the antibody can be obtained by a method of immunizing an animal with cells such as cells allowed to express an arbitrary protein by the gene manipulation described above, or a cell line endogenously expressing the antigen.

Whether or not the antibody or the antigen binding fragment binds to the antigen can be confirmed by use of a binding activity measurement method known in the art. Examples of the method for measuring binding activity include methods such as enzyme-linked immunosorbent assay (ELISA) and flow cytometry.

The antibody or the antibody binding fragment used in the present invention may be derived from any species such as a human, a rat, a mouse, or a rabbit. The antibody, when derived from a non-human species, is preferably chimerized or humanized by use of a well-known technique. The antibody or the antibody binding fragment for use in the antibody-drug conjugate of the present invention may be a polyclonal antibody or a monoclonal antibody.

In certain embodiments, Ab used in the present invention is an IgG type antibody or antibody binding fragment. IgG has subtypes of IgG1, IgG2, IgG3, and IgG4 depending on the structures of hinge and Fc regions, and may be appropriately selected by those skilled in the art in consideration of an effector function of the antibody, DAR, or the like. In certain embodiments, Ab used in the present invention is an IgG1 or IgG4 type.

In certain embodiments, Ab used in the present invention is one or two or more antibodies selected from the group consisting of an anti-EGFR antibody, an anti-HER2 antibody, an anti-cMET antibody, and an anti-TROP2 antibody, or antigen binding fragments thereof.

In certain embodiments, Ab used in the present invention is an anti-EGFR antibody, an anti-HER2 antibody, an anti-cMET antibody, or an anti-TROP2 antibody, or an antigen binding fragment thereof.

In certain embodiments, Ab used in the present invention is an anti-EGFR antibody or an antigen binding fragment thereof.

In certain embodiments, Ab used in the present invention is an anti-HER2 antibody or an antigen binding fragment thereof.

In certain embodiments, Ab used in the present invention is an anti-TROP2 antibody or an antigen binding fragment thereof.

In certain embodiments, Ab used in the present invention is an IgG1 or IgG4 type anti-EGFR antibody, anti-HER2 antibody, or anti-TROP2 antibody.

In certain embodiments, Ab used in the present invention is an IgG1 or IgG4 type anti-EGFR antibody.

In certain embodiments, Ab used in the present invention is an anti-EGFR antibody including a heavy chain variable region and a light chain variable region described in the following (1) or (2) or an antigen binding fragment thereof:

(1) a heavy chain variable region including CDR1 consisting of an amino acid sequence from amino acid positions 31 to 35 of SEQ ID NO: 1, CDR2 consisting of an amino acid sequence from amino acid positions 50 to 65 of SEQ ID NO: 1, and CDR3 consisting of an amino acid sequence from amino acid positions 98 to 108 of SEQ ID NO: 1, and
a light chain variable region including CDR1 consisting of an amino acid sequence from amino acid positions 24 to 34 of SEQ ID NO: 2, CDR2 consisting of an amino acid sequence from amino acid positions 50 to 56 of SEQ ID NO: 2, and CDR3 consisting of an amino acid sequence from amino acid positions 89 to 97 of SEQ ID NO: 2; or
(2) a heavy chain variable region including CDR1 consisting of an amino acid sequence from amino acid positions 31 to 35 of SEQ ID NO: 3, CDR2 consisting of an amino acid sequence from amino acid positions 50 to 65 of SEQ ID NO: 3, and CDR3 consisting of an amino acid sequence from amino acid positions 98 to 108 of SEQ ID NO: 3, and
a light chain variable region including CDR1 consisting of an amino acid sequence from amino acid positions 24 to 34 of SEQ ID NO: 4, CDR2 consisting of an amino acid sequence from amino acid positions 50 to 56 of SEQ ID NO: 4, and CDR3 consisting of an amino acid sequence from amino acid positions 89 to 97 of SEQ ID NO: 4.

In certain embodiments, Ab used in the present invention is an anti-EGFR antibody including a heavy chain variable region and a light chain variable region selected from the group consisting of the following (1) to (3) or an antigen binding fragment thereof:

(1) a heavy chain variable region consisting of an amino acid sequence from amino acid positions 1 to 119 of SEQ ID NO: 1 and a light chain variable region consisting of an amino acid sequence from amino acid positions 1 to 107 of SEQ ID NO: 2;
(2) a heavy chain variable region consisting of an amino acid sequence from amino acid positions 1 to 119 of SEQ ID NO: 3 and a light chain variable region consisting of an amino acid sequence from amino acid positions 1 to 107 of SEQ ID NO: 4; and
(3) a heavy chain variable region and a light chain variable region having at least 90% or higher identity to the heavy

chain variable region and the light chain variable region described in (1) or (2) above.

In certain embodiments, Ab used in the present invention is an anti-EGFR antibody consisting of a heavy chain of SEQ ID NO: 1 and a light chain of SEQ ID NO: 2.

The antibody or the antigen binding fragment used in the present invention may undergo post-translational modification such as N-linked or O-linked glycosylation, N-terminal or C-terminal processing, deamidation, isomerization of aspartic acid, or oxidation of methionine.

An arbitrary amino acid residue in the antibody or the antibody binding fragment used in the present invention may be substituted by cysteine or a non-natural amino acid. The substitution by cysteine can employ, for example, a method described in WO2008141044 or WO2016040856. The substitution by a non-natural amino acid can employ, for example, a method described in WO2010081111 or WO2013185115.

An Fc region of the antibody used in the present invention may have a mutation that reduces antibody-dependent cellular cytotoxicity (ADCC) or complement-dependent cytotoxicity (CDC). L234A is the substitution of leucine at amino acid position 234 based on the EU index in a human Igyl constant region by alanine. L235A is the substitution of leucine at amino acid position 235 based on the EU index in a human Igyl constant region by alanine. The amino acid mutation of L234A and L235A in the human Igyl constant region is called "LALA mutation". This mutation is known to reduce antibody-dependent cellular cytotoxicity or complement-dependent cytotoxicity of antibodies (Mol. Immunol., 1992; Vol. 29: p. 633-639). P331G or P331S is the substitution of proline at amino acid position 331 based on the EU index in a human Igyl constant region by glycine or serine. This mutation is known to reduce CDC of antibodies (J. Immunol., 2000, Vol/164 (8), p. 4178-4184).

5. Antibody-drug conjugate

The antibody-drug conjugate according to the present invention or the salt thereof is an antibody-drug conjugate represented by the following formula (I) or a salt thereof:

[Chemical Formula 140]

$$Ab\!-\!\left(L^{A}\!-\!D\right)_{n}$$

(I)

wherein

Ab is an antibody or an antigen binding fragment thereof,
D is a heterocyclic compound having a G12D mutant KRAS protein degradation-inducing effect,
$L^A$ is a linker for connecting Ab and D, and
n is a number of 1 to 20.

n represents a drug-to-antibody ratio (drug to antibody ratio; DAR), i.e., an average number of compounds conjugated per antibody in the antibody-drug conjugate. The antibody-drug conjugate of formula (I) or the salt thereof is an antibody-drug conjugate or a salt thereof, wherein n is a number of 1 to 20 in certain embodiments, is an antibody-drug conjugate or a salt thereof, wherein n is a number of 1 to 10 in certain embodiments, is an antibody-drug conjugate or a salt thereof, wherein n is a number of 2 to 8 in certain embodiments, and is an antibody-drug conjugate or a salt thereof, wherein n is a number of 2 to 5 in certain embodiments. The antibody-drug conjugate of formula (I) or the salt thereof is an antibody-drug conjugate or a salt thereof, wherein n is a number of 1 to 6 in certain embodiments, is an antibody-drug conjugate or a salt thereof, wherein n is a number of 1 to 5 in certain embodiments, is an antibody-drug conjugate or a salt thereof, wherein n is a number of 2 to 3 in certain embodiments, is an antibody-drug conjugate or a salt thereof, wherein n is a number of 3 to 4 in certain embodiments, is an antibody-drug conjugate or a salt thereof, wherein n is a number of 4 to 5 in certain embodiments, is an antibody-drug conjugate or a salt thereof, wherein n is a number of 2 to 4 in certain embodiments, and is an antibody-drug conjugate or a salt thereof, wherein n is a number of 3 to 5 in certain embodiments.

Embodiments of the antibody-drug conjugate according to the present invention or the salt thereof will be shown below.

(26-1) An antibody-drug conjugate of formula (I) or a salt thereof:

[Chemical Formula 141]

$$Ab\text{—}(L^A\text{—}D)_n$$

(I)

wherein

Ab is an antibody or an antigen binding fragment thereof,
D is a heterocyclic compound having a G12D mutant KRAS protein degradation-inducing effect,
$L^A$ is a linker for connecting Ab and D, and
n is a number of 1 to 20.

(26-2) The antibody-drug conjugate or the salt thereof according to (26-1), wherein D is heterocyclic compound represented by formula (II),

[Chemical Formula 142]

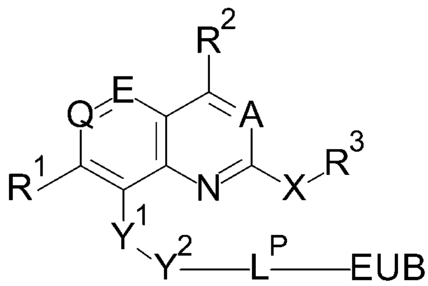

(II)

wherein

A is $CR^A$ or N,
$R^A$ is H, cyano, or optionally substituted $C_{1-3}$ alkyl,
Q is $CR^Q$ or N,
$R^Q$ is H, halogen, $C_{3-6}$ cycloalkyl, vinyl, or optionally substituted $C_{1-3}$ alkyl,
E is CH or N,
$R^1$ is naphthyl optionally substituted by one or two groups selected from the group consisting of cyano, OH, halogen, and optionally substituted $C_{1-3}$ alkyl, or is one group selected from the group consisting of the following formula (III), formula (IV), and formula (V):

[Chemical Formula 143]

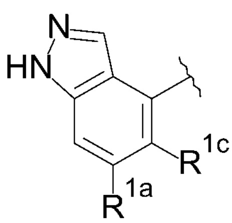

(III)

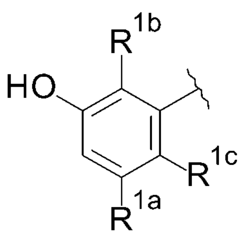

(IV)

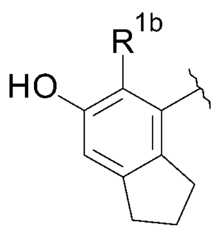

(V)

$R^{1a}$, $R^{1b}$, and $R^{1c}$ are each independently H, vinyl, halogen, or optionally substituted $C_{1-3}$ alkyl,
$R^2$ is $-V^1-V^2$ or W,
$V^1$ is a bond, $-CH_2-$, -O-, -S-, or $-N(R^{V1})-$,

$R^{V1}$ is H or optionally substituted $C_{1-3}$ alkyl,
$V^2$ is one group selected from the group consisting of the following formula (VI) and formula (VII):

[Chemical Formula 144]

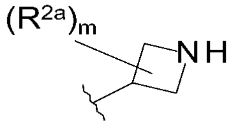
(VI)

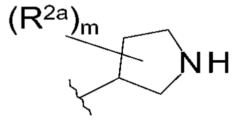
(VII)

W is one group selected from the group consisting of the following formula (VIII), formula (IX), formula (X), formula (XI), formula (XII), formula (XIII), formula (XIV), formula (XV), and formula (XVI):

[Chemical Formula 145]

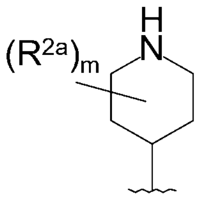
(VIII)

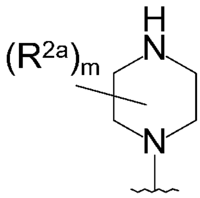
(IX)

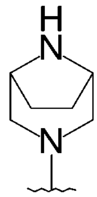
(X)

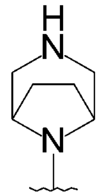
(XI)

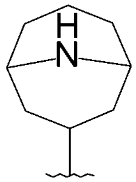
(XII)

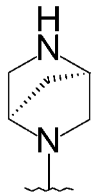
(XIII)

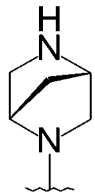
(XIV)

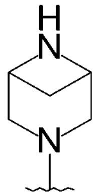
(XV)

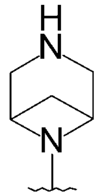
(XVI)

$R^{2a}$ are each independently OH, $OCH_3$, F, or optionally substituted $C_{1-3}$ alkyl, wherein $R^{2a}$ is bonded only to a carbon atom serving as a constituting atom of a ring selected from the group consisting of an azetidine ring represented by formula (VI), a pyrrolidine ring represented by formula (VII), a piperidine ring represented by formula (VIII), and a piperazine ring represented by formula (IX),
m is an integer of 0 to 2,
$R^3$ is optionally substituted $C_{1-6}$ alkyl, optionally substituted hetero cycloalkyl, or optionally substituted heteroaryl,
X is a bond, $-CH_2-$, -O-, -S-, or $-NR^{4X}-$,
$R^{4X}$ is H or optionally substituted $C_{1-3}$ alkyl,
$Y^1$ is -O-(optionally substituted $C_{1-3}$ alkylene)-$^{*Y2}$, -S-(optionally substituted $C_{1-3}$ alkylene)-$^{*Y2}$, $-SO_2$-(optionally substituted $C_{1-3}$ alkylene)-$^{*Y2}$, $-NR^Y$-(optionally substituted $C_{1-3}$ alkylene)-$^{*Y2}$, -(optionally substituted $C_{1-3}$ alkylene)-O-$^{*Y2}$, -(optionally substituted $C_{1-3}$ alkylene)-S-$^{*Y2}$, -(optionally substituted $C_{1-3}$ alkylene)-$SO_2$-$^{*Y2}$, or -(optionally substituted $C_{1-3}$ alkylene)-$NR^Y$-$^{*Y2}$ (wherein $^{*Y2}$ represents a site of connection to $Y^2$),
$R^Y$ is H or optionally substituted $C_{1-3}$ alkyl,
$Y^2$ is a bond, optionally substituted phenylene, or optionally substituted heteroarylene,
$L^P$ is a group that chemically bonds $Y^2$ to EUB,
EUB is a group having the ability to bind to one E3 ubiquitin ligase selected from the group consisting of VHL, celebron, IAP, MDM2, DCAF11, DCAF15, DCAF16, BIRC2, KEAP1, RNF4, RNF114, FEM1B, and AhR, and
$L^A$ is a linker for connecting Ab and D, wherein $L^A$ is bonded to an arbitrary nitrogen atom of -NH- or oxygen atom of -OH contained in D.

(26-3) The antibody-drug conjugate or the salt thereof according to (26-2), wherein

$V^2$ is one group selected from the group consisting of the following formula (VIa) and formula (VIIa) (wherein $^{*LA}$ represents a site of connection to $L^A$):

[Chemical Formula 146]

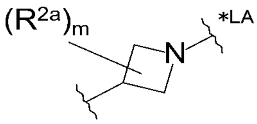

(VIa)

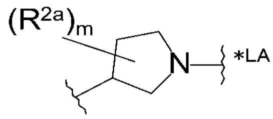

(VIIa)

W is one group selected from the group consisting of the following formula (VIIIa), formula (IXa), formula (Xa), formula (XIa), formula (XIIa), formula (XIIIa), formula (XIVa), formula (XVa), and formula (XVIa) (wherein $^{*LA}$ represents a site of connection to $L^A$):

[Chemical Formula 147]

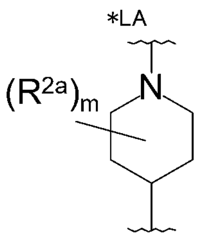

(VIIIa)

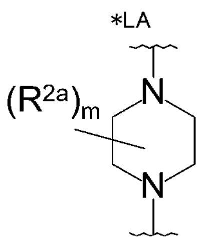

(IXa)

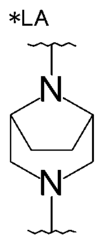

(Xa)

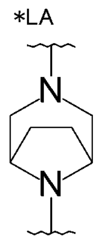

(XIa)

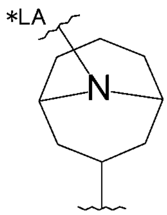

(XIIa)

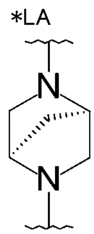

(XIIIa)

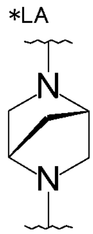

(XIVa)

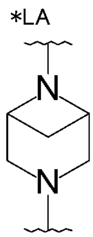

(XVa)

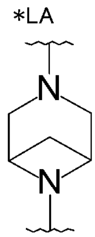

(XVIa)

and
$L^A$ is linker for connecting Ab and D, wherein $L^A$ is bonded to D through a nitrogen atom contained in $V^2$ or W at the site of connection represented by $^{*LA}$.

(26-4) The antibody-drug conjugate or the salt thereof according to (26-2), wherein

A is N,
Q is $CR^Q$, $R^Q$ is cyclopropyl,
E is CH,
$R^1$ is the following formula (III-2):

[Chemical Formula 148]

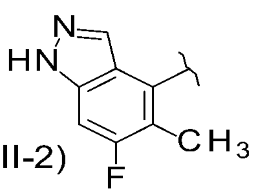

(III-2)

$R^2$ is $-V^1-V^2$ or W,
$V^1$ is $-N(CH_3)-$,
$V^2$ is the following formula (VII-2):

[Chemical Formula 149]

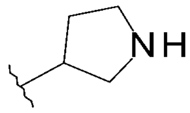

(VII-2)

W is the following formula (XIV):

[Chemical Formula 150]

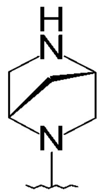

(XIV)

$R^3$ is n-propyl optionally substituted by $-OCH_3$, or tetrahydropyranyl,
X is -O-,
$Y^1$ is -O-(methylene)-$^{*Y2}$ (wherein $^{*Y2}$ represents a site of connection to $Y^2$),
$Y^2$ is phenylene,
$L^P$ is a group that chemically bonds $Y^2$ to EUB, and
EUB is a group having the ability to bind to one E3 ubiquitin ligase selected from the group consisting of VHL and celebron.

(26-5) The antibody-drug conjugate or the salt thereof according to (26-4), wherein

$V^2$ is the following formula (VII-2a) (wherein $^{*LA}$ represents a site of connection to $L^A$):

[Chemical Formula 151]

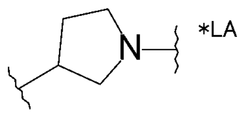

(VII-2a)

W is the following formula (XIVa) (wherein $^{*LA}$ represents a site of connection to $L^A$):

[Chemical Formula 152]

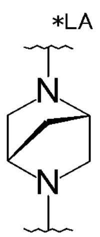

(XIVa)

and
$L^A$ is a linker for connecting Ab and D, wherein $L^A$ is bonded to D through a nitrogen atom contained in $V^2$ or W at the site of connection represented by $^{*LA}$.

(26-6) The antibody-drug conjugate or the salt thereof according to (26-1) or (26-2), wherein
D is (4R)-1-[(2S)-2-(4-{4-[({6-cyclopropyl-4-[(1S,4S)-2,5-diazabicyclo[2.2.1]heptan-2-yl]-7-(6-fluoro-5-methyl-1H-indazol-4-yl)-2-[(oxan-4-yl)oxy]quinazolin-8-yl}oxy)methyl]phenyl}-1H-1,2,3-triazol-1-yl)-3-methylbutanoyl]-4-hydroxy-N-{(1R)-2-hydroxy-1-[4-(4-methyl-1,3-thiazol-5-yl)phenyl]ethyl}-L-prolinamide, (4R)-1-[(2S)-2-(4-{4-[({6-cyclopropyl-4-[(1S,4S)-2,5-diazabicyclo[2.2.1]heptan-2-yl]-7-(6-fluoro-5-methyl-1H-indazol-4-yl)-2-[(oxan-4-yl)oxy]quinazolin-8-yl}oxy)methyl]phenyl}-1H-1,2,3-triazol-1-yl)-3-methylbutanoyl]-4-hydroxy-N-{(1R)-2-hydroxy-1-[4-(2-oxo-1,3-oxazolidin-3-yl)phenyl]ethyl}-L-prolinamide, (4R)-1-[(2S)-2-(4-{4-[({6-cyclopropyl-4-[(1S,4S)-2,5-diazabicyclo[2.2.1]heptan-2-yl]-7-(6-fluoro-5-methyl-1H-indazol-4-yl)-2-[(oxan-4-yl)oxy]quinazolin-8-yl}oxy)methyl]phenyl}-1H-1,2,3-triazol-1-yl)-3-methylbutanoyl]-4-hydroxy-N-{(1R)-2-hydroxy-1-[4-(1-methyl-1H-pyrazol-5-yl)phenyl]ethyl}-L-prolinamide, (4R)-1-[(2S)-2-(4-{4-[({6-cyclopropyl-4-[(1S,4S)-2,5-diazabicyclo[2.2.1]heptan-2-yl]-7-(6-fluoro-5-methyl-1H-indazol-4-yl)-2-[(2S)-2-methoxypropoxy]quinazolin-8-yl}oxy)methyl]phenyl}-1H-1,2,3-triazol-1-yl)-3-methylbutanoyl]-4-hydroxy-N-{(1R)-2-hydroxy-1-[4-(4-methyl-1,3-thiazol-5-yl)phenyl]ethyl}-L-prolinamide, (4R)-1-[(2S)-2-(4-{4-[({6-cyclopropyl-4-[(1S,4S)-2,5-diazabicyclo[2.2.1]heptan-2-yl]-7-(6-fluoro-5-methyl-1H-indazol-4-yl)-2-[(2S)-2-methoxypropoxy]quinazolin-8-yl}oxy)methyl]phenyl}-1H-1,2,3-triazol-1-yl)-3-methylbutanoyl]-N-{(1R)-1-[4-(1-ethyl-1H-pyrazol-5-yl)phenyl]-2-hydroxyethyl}-4-hydroxy-L-prolinamide, (4R)-1-[(2S)-2-(4-{4-[({6-cyclopropyl-4-[(1S,4S)-2,5-diazabicyclo[2.2.1]heptan-2-yl]-7-(6-fluoro-5-methyl-1H-indazol-4-yl)-2-[(2S)-2-methoxypropoxy]quinazolin-8-yl}oxy)methyl]phenyl}-1H-1,2,3-triazol-1-yl)-3-methylbutanoyl]-4-hydroxy-N-{(1R)-2-hydroxy-1-[4-(4-methyl-1,3-oxazol-5-yl)phenyl]ethyl}-L-prolinamide, (4R)-1-[(2S)-2-{4-[4-({[(7M)-6-cyclopropyl-7-(6-fluoro-5-methyl-1H-indazol-4-yl)-2-[(2S)-2-methoxypropoxy]-4-{methyl[(3S)-pyrrolidin-3-yl]amino}quinazolin-8-yl]oxy}methyl)phenyl]-1H-1,2,3-triazol-1-yl}-3-methylbutanoyl]-4-hydroxy-N-{(1R)-2-hydroxy-1-[4-(4-methyl-1,3-thiazol-5-yl)phenyl]ethyl}-L-prolinamide, (4R)-1-[(2S)-2-{4-[4-({[(7M)-6-cyclopropyl-7-(6-fluoro-5-methyl-1H-indazol-4-yl)-2-[(2S)-2-methoxypropoxy]-4-{methyl[(3S)-pyrrolidin-3-yl]amino}quinazolin-8-yl]oxy}methyl)phenyl]-1H-1,2,3-triazol-1-yl}-3-methylbutanoyl]-N-{(1R)-1-[4-(1-ethyl-1H-pyrazol-5-yl)phenyl]-2-hydroxyethyl}-4-hydroxy-L-prolinamide, 3-(5-{[(3S)-4-{4-[({(7M)-6-cyclopropyl-4-[(1S,4S)-2,5-diazabicyclo[2.2.1]heptan-2-yl]-7-(6-fluoro-5-methyl-1H-indazol-4-yl)-2-[(oxan-4-yl)oxy]quinazolin-8-yl}oxy)methyl]benzoyl}-3-methylpiperazin-1-yl]methyl}-3-methyl-2-oxo-2,3-dihydro-1H-benzimidazol-1-yl)piperidine-2,6-dione, 1-(6-{[(3S)-4-{4-[({(7M)-6-cyclopropyl-4-[(1S,4S)-2,5-diazabicyclo[2.2.1]heptan-2-yl]-7-(6-fluoro-5-methyl-1H-indazol-4-yl)-2-[(oxan-4-yl)oxy]quinazolin-8-yl}oxy)methyl]benzoyl}-3-methylpiperazin-1-yl]methyl}-1-methyl-1H-indazol-3-yl)-1,3-diazinane-2,4-dione, 3-(5-{[(3S)-4-{4-[({(7M)-6-cyclopropyl-4-[(1S,4S)-2,5-diazabicyclo[2.2.1]heptan-2-yl]-7-(6-fluoro-5-methyl-1H-indazol-4-yl)-2-[(2S)-2-methoxypropoxy]quinazolin-8-yl}oxy)methyl]benzoyl}-3-methylpiperazin-1-yl]methyl}-3-methyl-2-oxo-2,3-dihydro-1H-benzimidazol-1-yl)piperidine-2,6-dione, or (2R)-2-({(4R)-1-[(2S)-2-{4-[4-({[(7M)-6-cyclopropyl-7-(6-fluoro-5-methyl-1H-indazol-4-yl)-2-[(2S)-2-methoxypropoxy]-4-{methyl[(3S)-pyrrolidin-3-yl]amino}quinazolin-8-yl]oxy}methyl)phenyl]-1H-1,2,3-triazol-1-yl}-3-methylbutanoyl]-4-hydroxy-L-prolyl}amino)-2-[4-(1H-1,2,4-triazol-1-yl)phenyl]ethyl dihydrogen phosphate.
(26-7) The antibody-drug conjugate or the salt thereof according to any one of (26-1) to (26-6), wherein $L^A$ is a linker represented by formula (XXVIII):

[Chemical Formula 153]

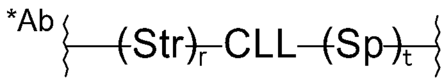

(XXVIII)

wherein

Str is a stretcher unit and is bonded to Ab and CLL, r is 0 or 1,
CLL is a partial structure cleavable *in vivo*,
Sp is a spacer unit and is bonded to CLL and D, t is 0 or 1, and
$^{*Ab}$ represents a site of connection to Ab.

(26-8) The antibody-drug conjugate or the salt thereof according to (26-7), wherein

Str is a stretcher unit represented by formula (ST-1), and r is 1:

[Chemical Formula 154]

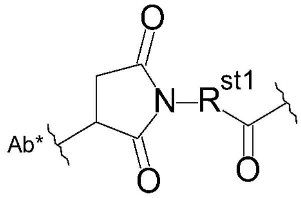

(ST-1)

wherein $R^{st1}$ is optionally substituted $C_{1-12}$ alkylene, $-(CH_2CH_2O)_{a1}-C_{1-6}$ alkylene-, $-C_{1-6}$ alkylene-$(OCH_2CH_2)_{a2}$-, -optionally substituted $C_{1-6}$ alkylene-NH-C(=O)-optionally substituted $C_{1-6}$ alkylene-, -optionally substituted $C_{1-6}$ alkylene-C(=O)-NH-optionally substituted $C_{1-6}$ alkylene-, optionally substituted $C_{1-6}$ alkylene-C(=O)-NH-$(CH_2CH_2O)_{a3}-C_{1-6}$ alkylene-, or optionally substituted $C_{1-6}$ alkylene-NH-C(=O)-$(CH_2CH_2O)_{a4}-C_{1-6}$ alkylene-, each of a1, a2, a3, and a4 is an integer of 1 to 10, and
$^{*Ab}$ represents a site of connection to Ab.

(26-9) The antibody-drug conjugate or the salt thereof according to (26-8), wherein $R^{st1}$ is $C_5$ alkylene.
(26-10) The antibody-drug conjugate or the salt thereof according to (26-7), wherein

CLL is a partial structure cleavable *in vivo* represented by formula (CL-1):

[Chemical Formula 155]

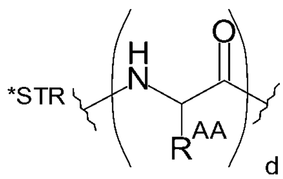

(CL-1)

wherein $R^{AA}$ are each independently H, methyl, isopropyl, isobutyl, sec-butyl, benzyl, p-hydroxybenzyl, hydroxymethyl, 1-hydroxyethyl, $-CH_2-C(=O)-OH$, $-(CH_2)_2-C(=O)-OH$, - $CH_2-C(=O)-NH_2$, $-(CH_2)_2-C(=O)-NH_2$, $-(CH_2)_4-NH_2$, $-(CH_2)_3-NH-C(=NH)-NH_2$, $-(CH_2)_3-NH-C(=O)-NH_2$, $-CH_2-SH$, or $-CH_2-S-CH_3$, or one group selected from the group consisting of the following formula (RAA-1) and (RAA-2):

[Chemical Formula 156]

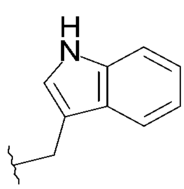

(RAA-1)

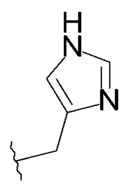

(RAA-2)

d is an integer of 1 to 6, and $^{*STR}$ represents a site of connection to Str.

(26-11) The antibody-drug conjugate or the salt thereof according to (26-10), wherein $R^{AA}$ are each independently methyl or isopropyl, and d is 2.
(26-12) The antibody-drug conjugate or the salt thereof according to (26-10), wherein $R^{AA}$ are each independently isopropyl or $-(CH_2)_3-NH-C(=O)-NH_2$, and d is 2.
(26-13) The antibody-drug conjugate or the salt thereof according to (26-10), wherein $R^{AA}$ are each independently H or benzyl, and d is 4.
(26-14) The antibody-drug conjugate or the salt thereof according to (26-7), wherein

Sp is a spacer unit represented by formula (SP-1), and t is 1:

[Chemical Formula 157]

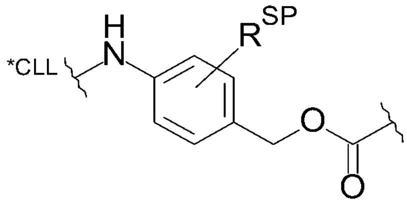

(SP-1)

wherein $R^{SP}$ is H, $C_{1-6}$ alkyl, -O-$C_{1-6}$ alkyl, halogen, or halogeno $C_{1-6}$ alkyl, and $^{*CLL}$ represents a site of connection to CLL.

(26-15) The antibody-drug conjugate or the salt thereof according to (26-14), wherein $R^{SP}$ is H.
(26-16) The antibody-drug conjugate or the salt thereof according to (26-7), wherein t is 0.
(26-17) The antibody-drug conjugate or the salt thereof according to (26-1), wherein formula (I) is represented by formula (AD-1a) or (AD-2a):

[Chemical Formula 158]

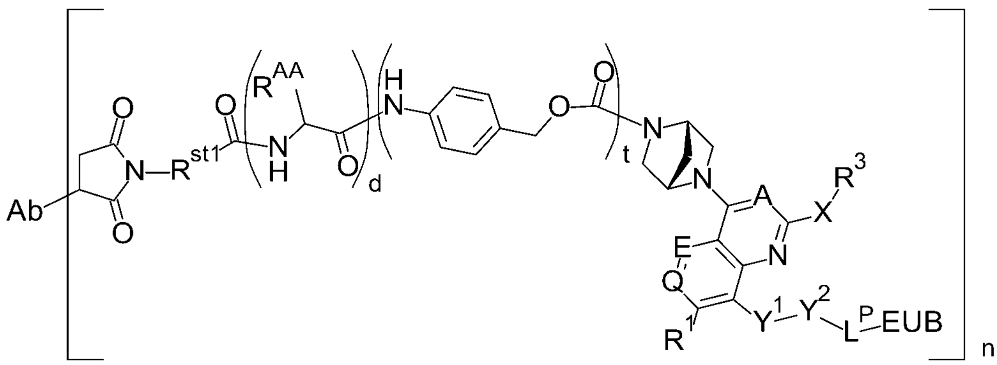

(AD-1a)

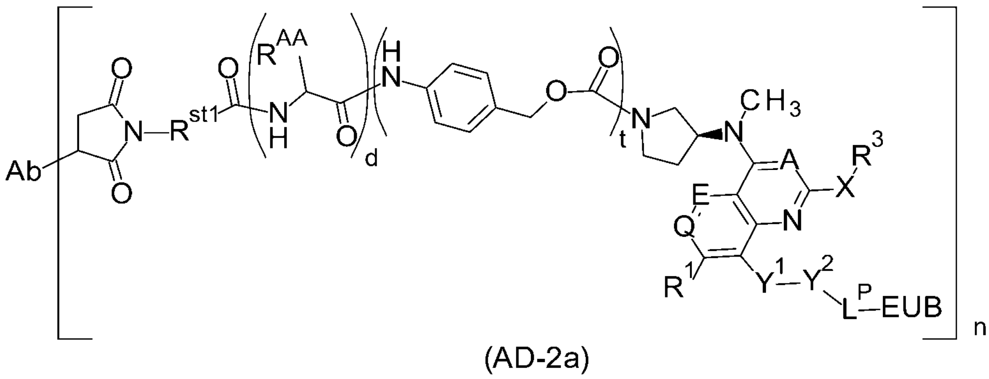

(AD-2a)

wherein

Ab is an antibody or an antigen binding fragment thereof,
A is $CR^A$ or N,
$R^A$ is H, cyano, or optionally substituted $C_{1-3}$ alkyl,
Q is $CR^Q$ or N,
$R^Q$ is H, halogen, $C_{3-6}$ cycloalkyl, vinyl, or optionally substituted $C_{1-3}$ alkyl,
E is CH or N,
$R^1$ is naphthyl optionally substituted by one or two groups selected from the group consisting of cyano, OH,

halogen, and optionally substituted $C_{1-3}$ alkyl, or is one group selected from the group consisting of the following formula (III), formula (IV), and formula (V):

[Chemical Formula 159]

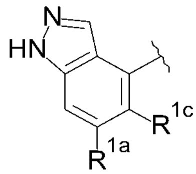

(III)

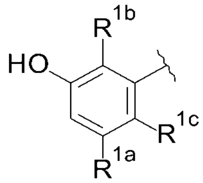

(IV)

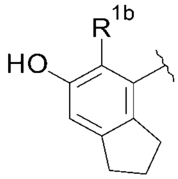

(V)

$R^{1a}$, $R^{1b}$, and $R^{1c}$ are each independently H, vinyl, halogen, or optionally substituted $C_{1-3}$ alkyl,
$R^3$ is optionally substituted $C_{1-6}$ alkyl, optionally substituted hetero cycloalkyl, or optionally substituted heteroaryl,
X is a bond, $-CH_2-$, -O-, -S-, or $-NR^{4X}-$,
$R^{4X}$ is H or optionally substituted $C_{1-3}$ alkyl,
$Y^1$ is -O-(optionally substituted $C_{1-3}$ alkylene)-$^{*Y2}$, -S-(optionally substituted $C_{1-3}$ alkylene)-$^{*Y2}$, $-SO_2$-(optionally substituted $C_{1-3}$ alkylene)-$^{*Y2}$, $-NR^Y$-(optionally substituted $C_{1-3}$ alkylene)-$^{*Y2}$, -(optionally substituted $C_{1-3}$ alkylene)-O-$^{*Y2}$, -(optionally substituted $C_{1-3}$ alkylene)-S-$^{*Y2}$, -(optionally substituted $C_{1-3}$ alkylene)-$SO_2$-$^{*Y2}$, or -(optionally substituted $C_{1-3}$ alkylene)-$NR^Y$-$^{*Y2}$ (wherein $^{*Y2}$ represents a site of connection to $Y^2$),
$R^Y$ is H or optionally substituted $C_{1-3}$ alkyl,
$Y^2$ is a bond, optionally substituted phenylene, or optionally substituted heteroarylene,
$L^P$ is a group that chemically bonds $Y^2$ to EUB,
EUB is a group having the ability to bind to one E3 ubiquitin ligase selected from the group consisting of VHL, celebron, IAP, MDM2, DCAF11, DCAF15, DCAF16, BIRC2, KEAP1, RNF4, RNF114, FEM1B, and AhR,
$R^{st1}$ is $C_{1-12}$ alkylene,
$R^{AA}$ are each independently H, methyl, isopropyl, benzyl, or $-(CH_2)_3-NH-C(=O)-NH_2$, d is an integer of 2 to 4,
t is 0 or 1, and
n is a number of 1 to 20.

(26-18) The antibody-drug conjugate or the salt thereof according to (26-1), wherein formula (I) is represented by formula (AD-3a), (AD-4a), (AD-5a), (AD-6a), (AD-7a), (AD-8a), (AD-9a), (AD-10a), (AD-11a), (AD-12a), (AD-13a), or (AD-25a):

[Chemical Formula 160-1]

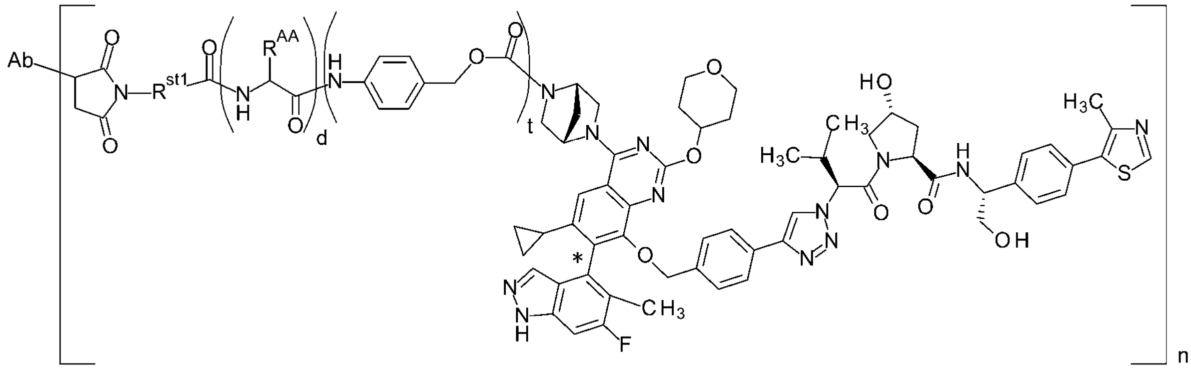

(AD-3a)

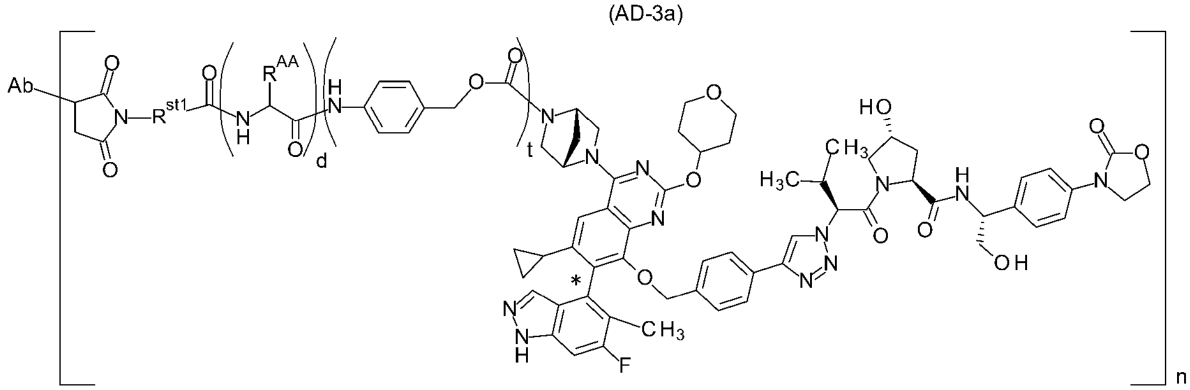

(AD-4a)

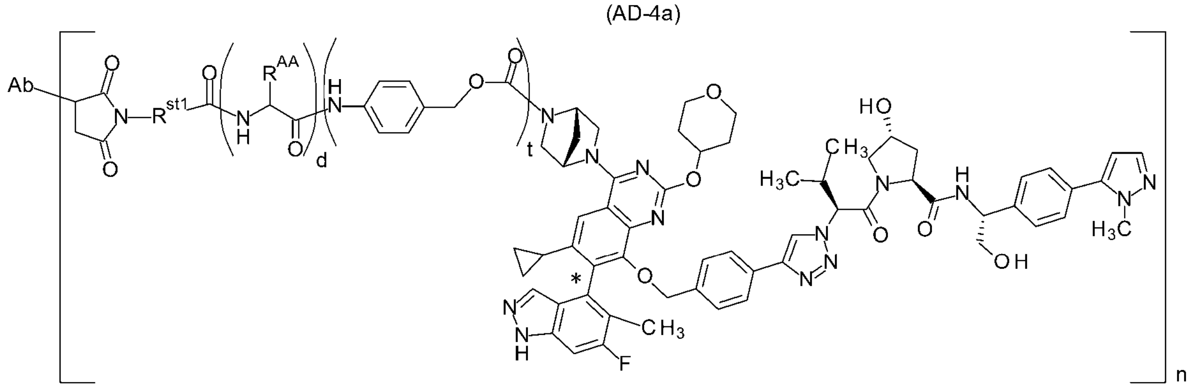

(AD-5a)

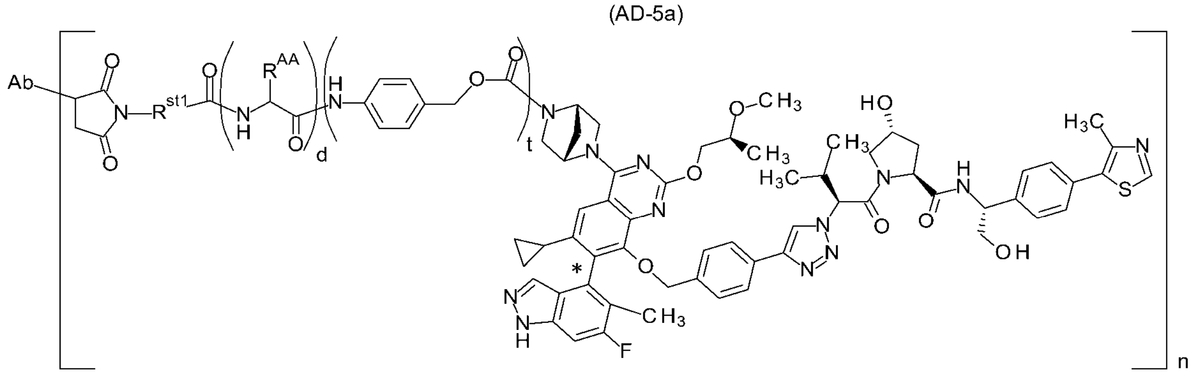

(AD-6a)

[Chemical Formula 160-2]

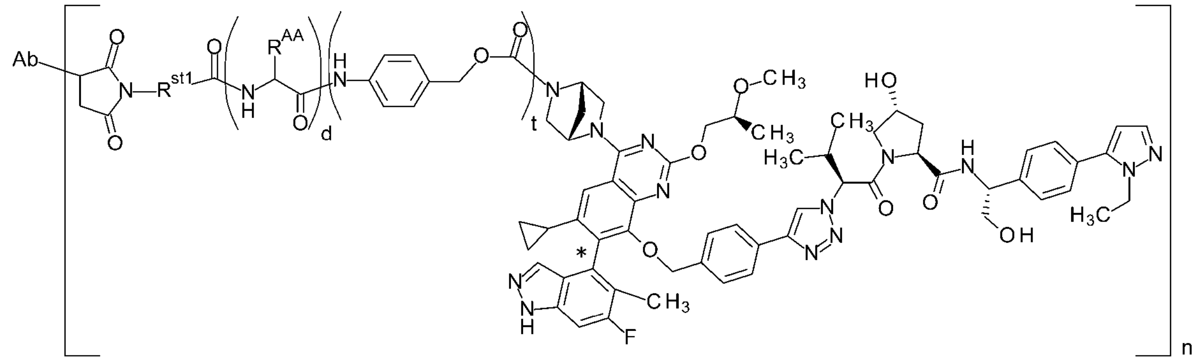

(AD-7a)

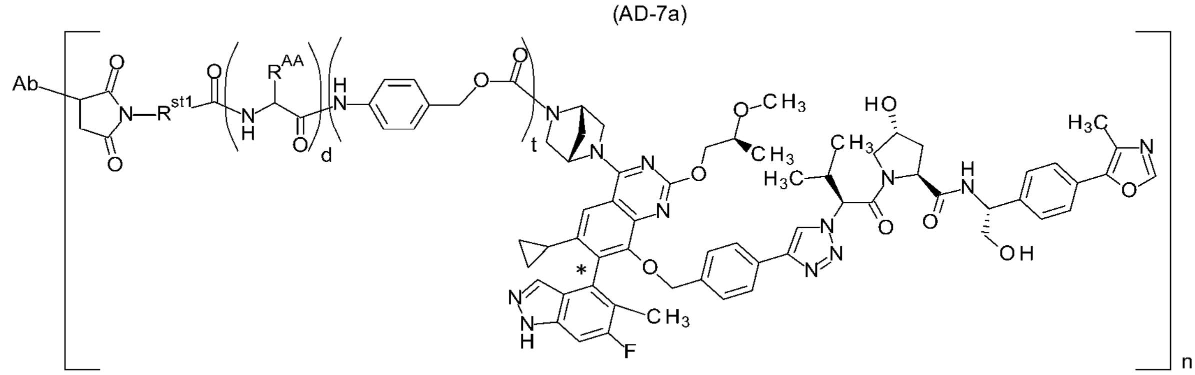

(AD-8a)

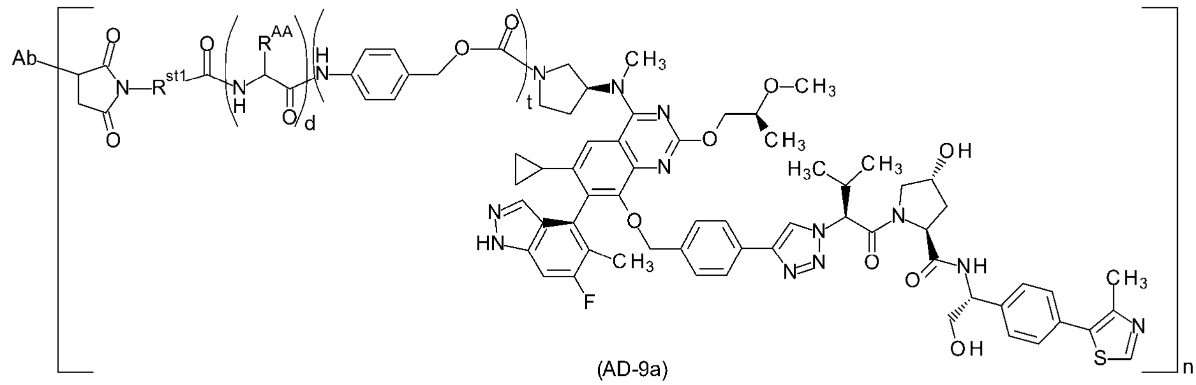

(AD-9a)

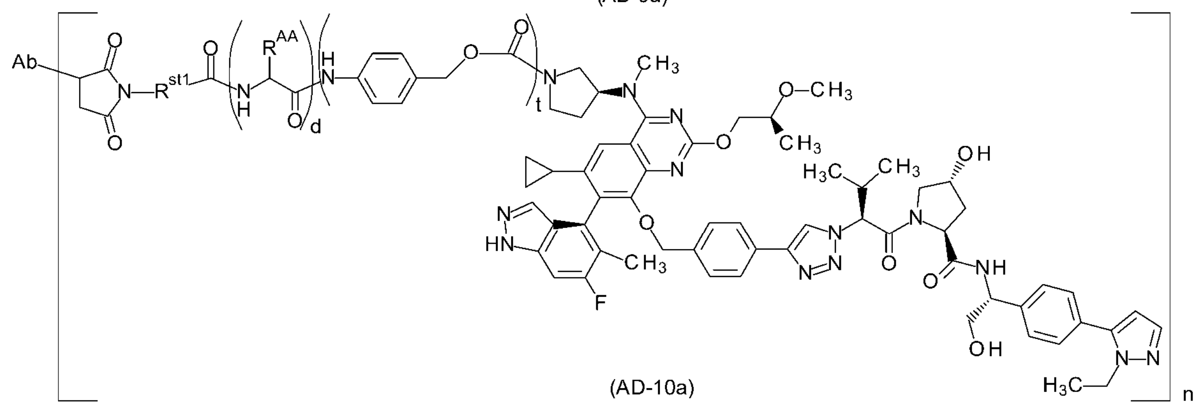

(AD-10a)

[Chemical Formula 160-3]

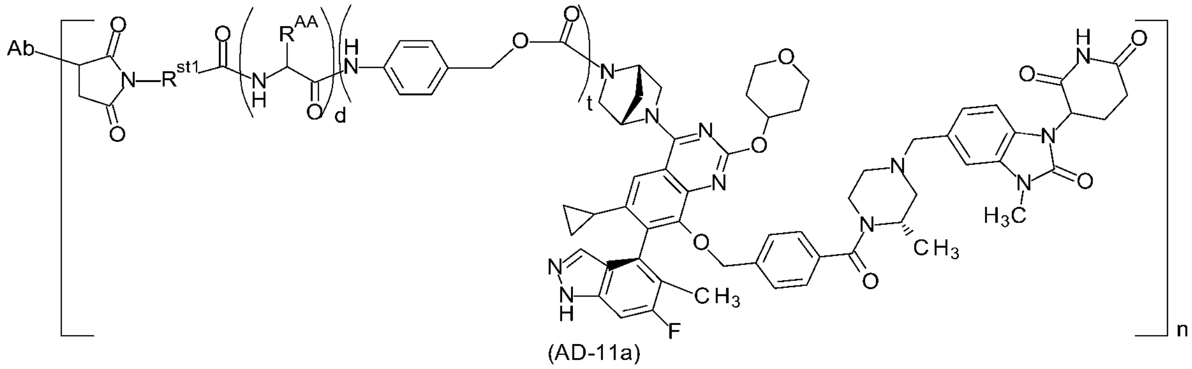

(AD-11a)

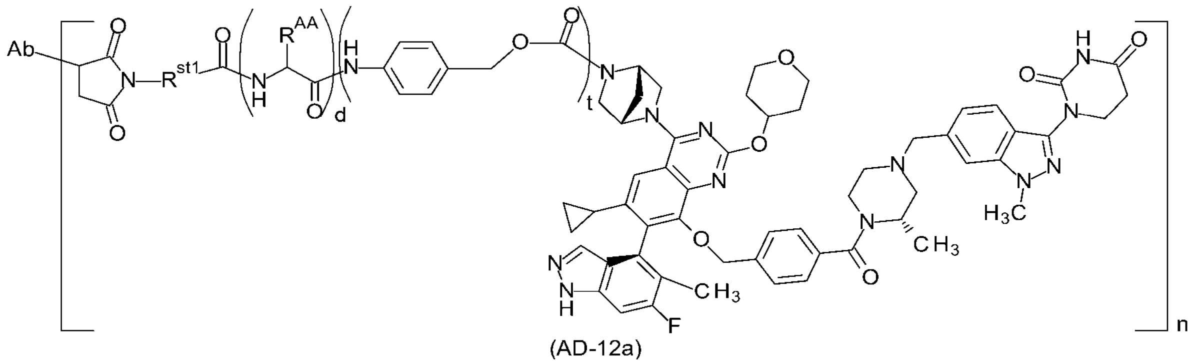

(AD-12a)

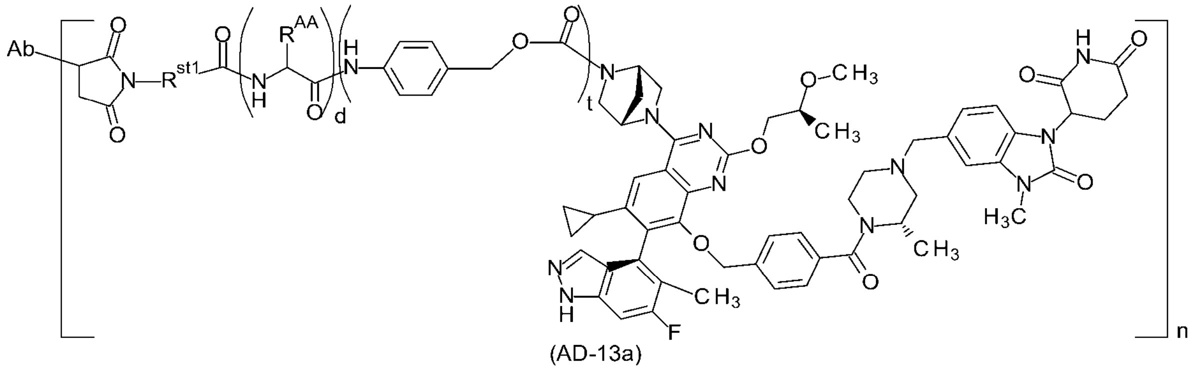

(AD-13a)

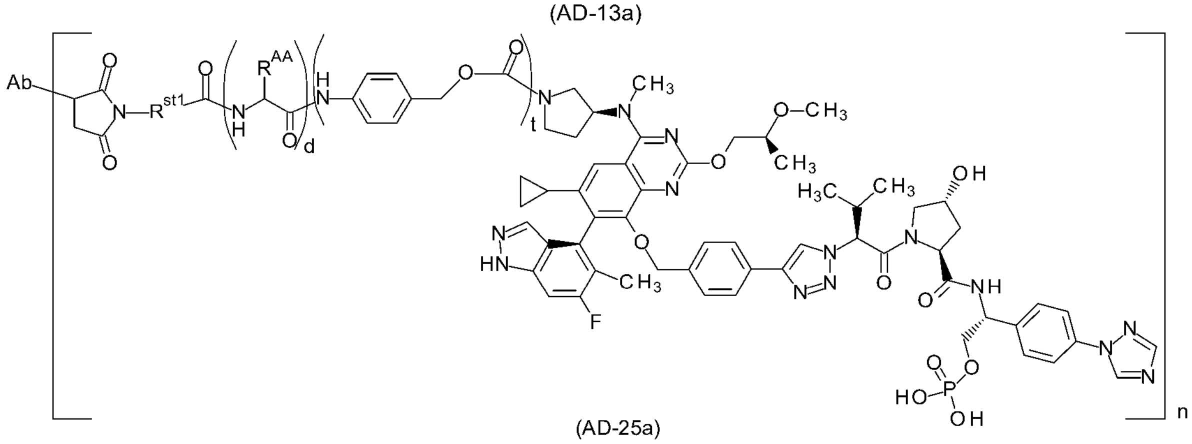

(AD-25a)

wherein $R^{st1}$ is $C_{1-12}$ alkylene, $R^{AA}$ are each independently H, methyl, isopropyl, benzyl, or - $(CH_2)_3$-NH-C(=O)-$NH_2$, d is an integer of 2 to 4, t is 0 or 1, and n is a number of 1 to 20.

(26-19) The antibody-drug conjugate or the salt thereof according to (26-1), wherein formula (I) is represented by formula (AD-14), (AD-15), (AD-16), (AD-17), (AD-18), (AD-19), (AD-20), (AD-21), (AD-22), (AD-23), (AD-24), or (AD-25):

[Chemical Formula 161-1]

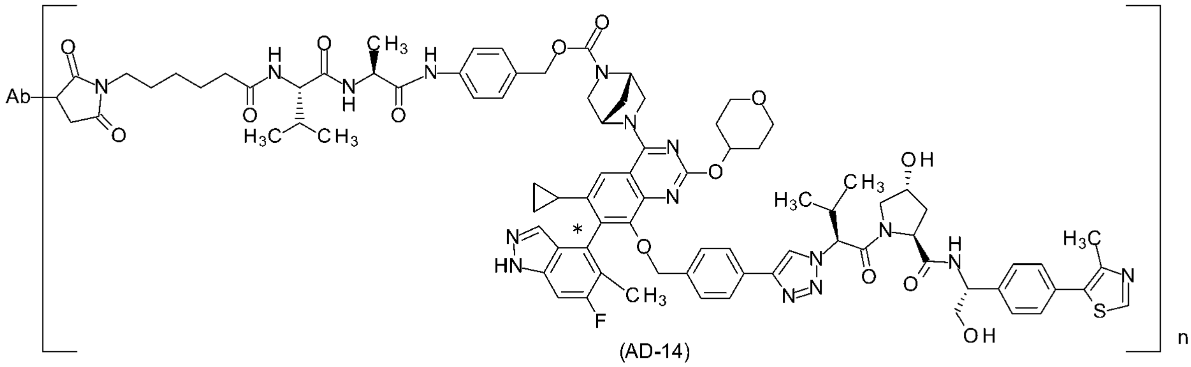

(AD-14)

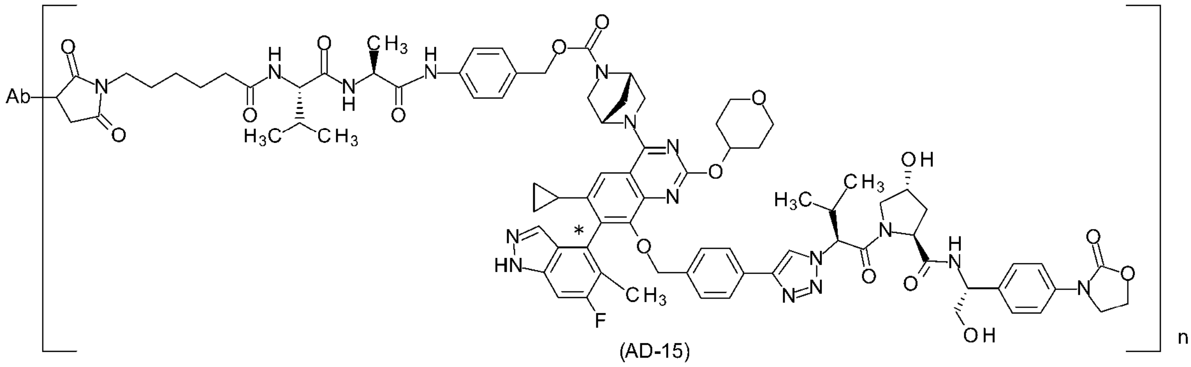

(AD-15)

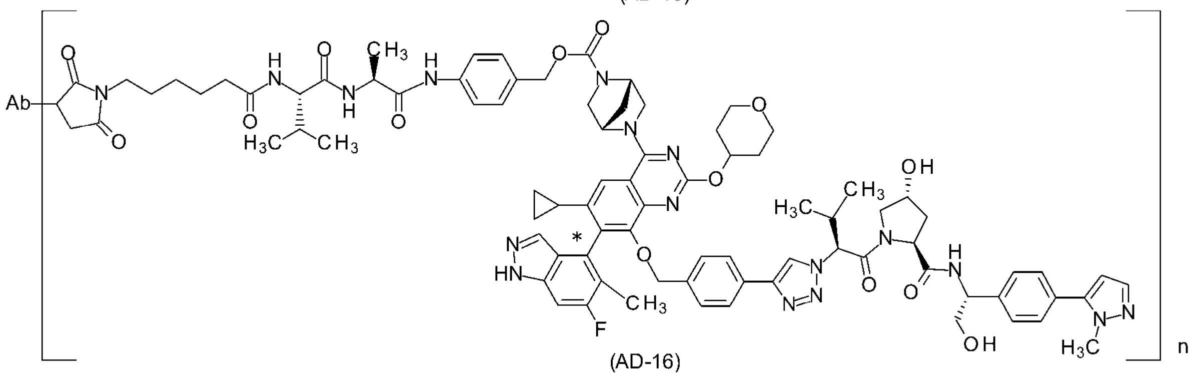

(AD-16)

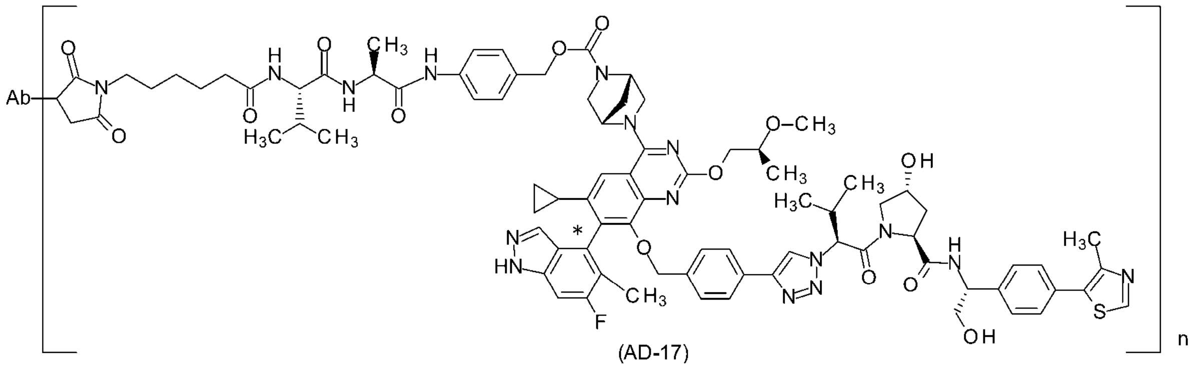

(AD-17)

[Chemical Formula 161-2]

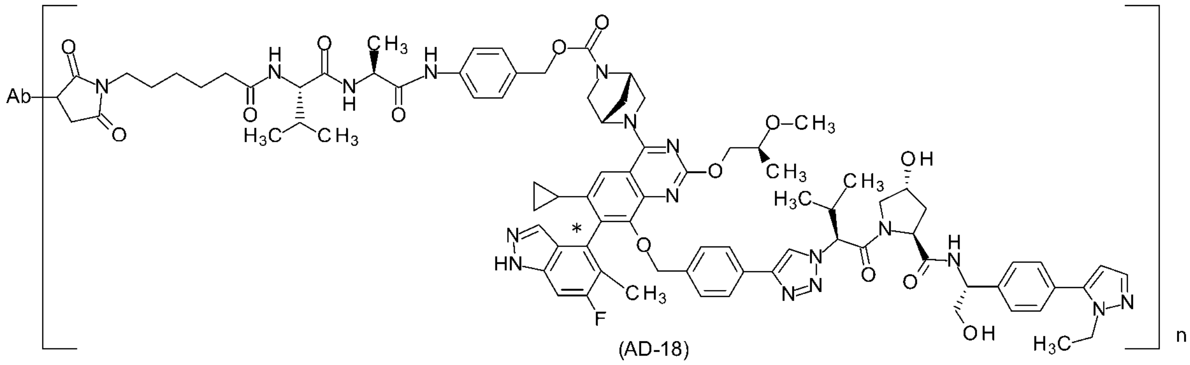

(AD-18)

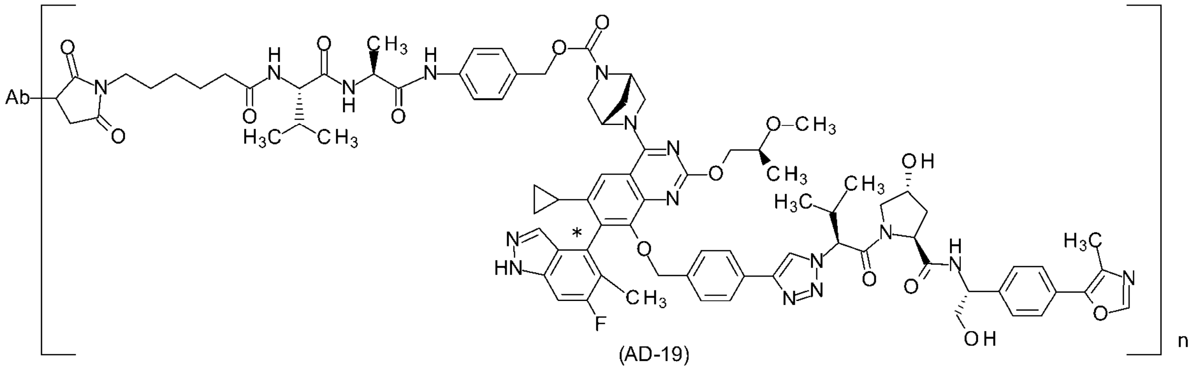

(AD-19)

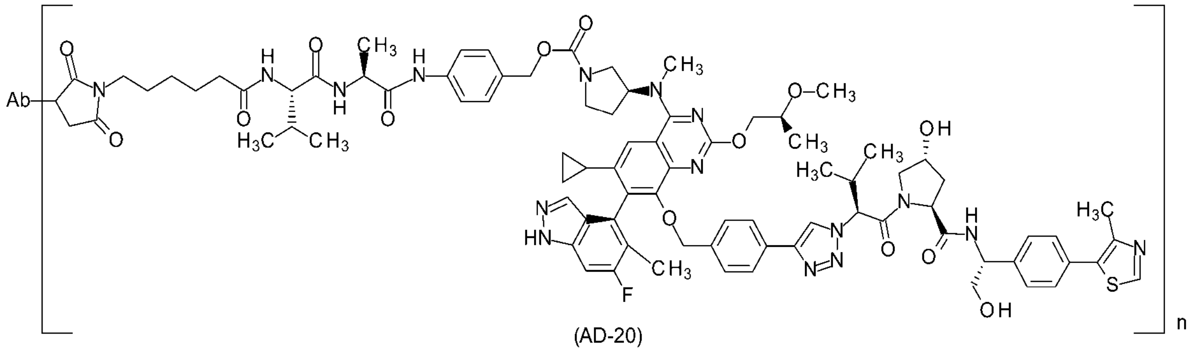

(AD-20)

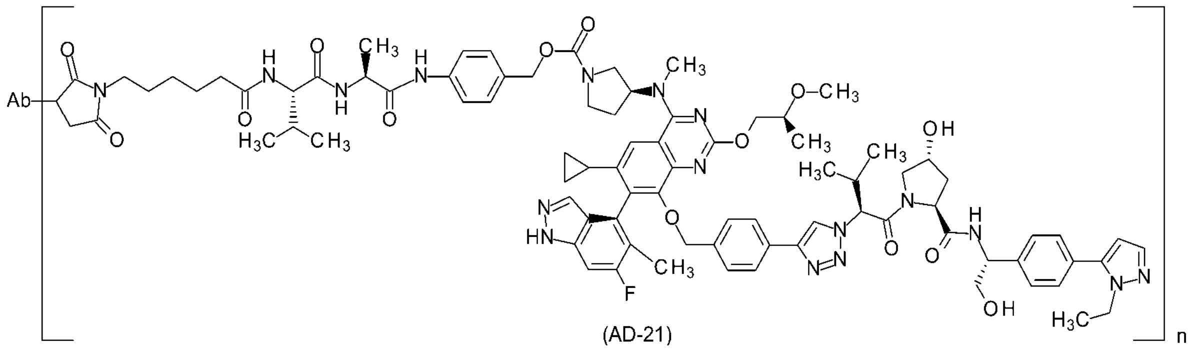

(AD-21)

[Chemical Formula 161-3]

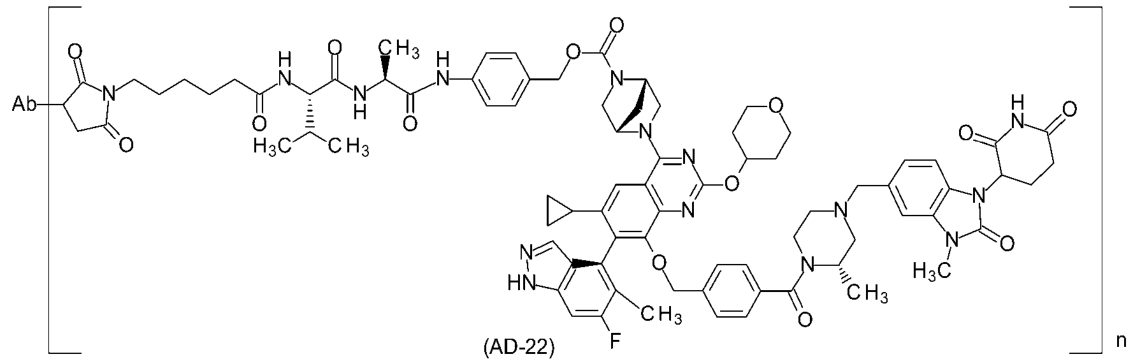

(AD-22)

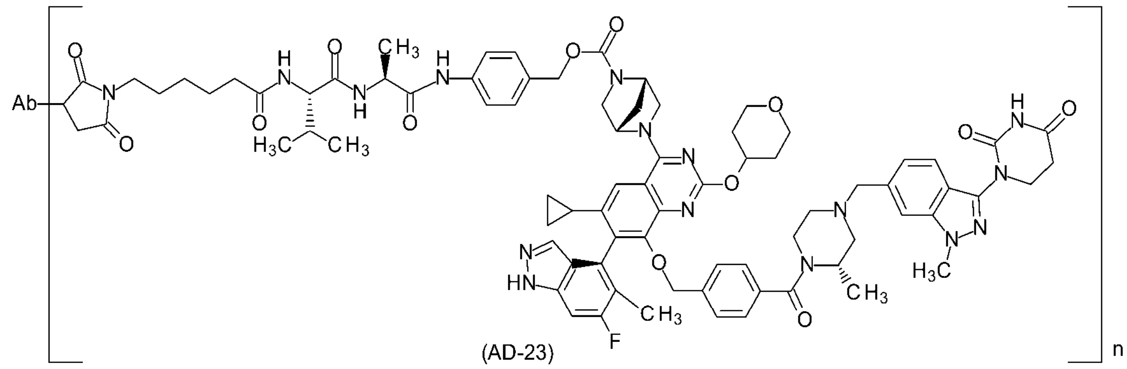

(AD-23)

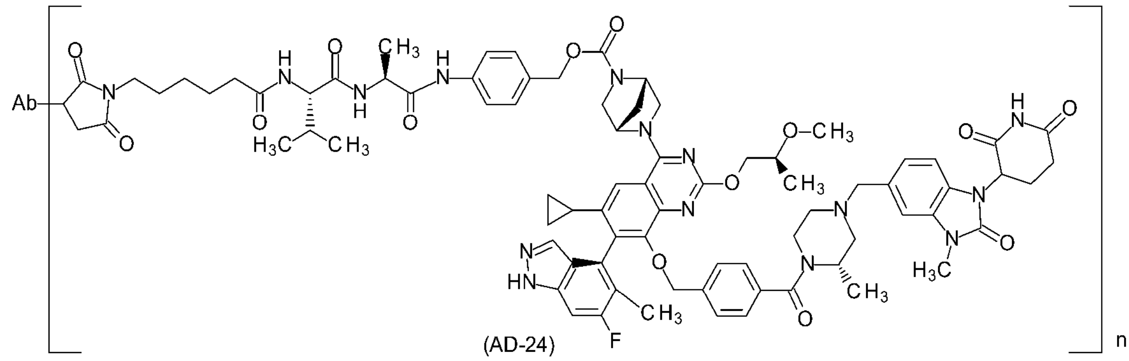

(AD-24)

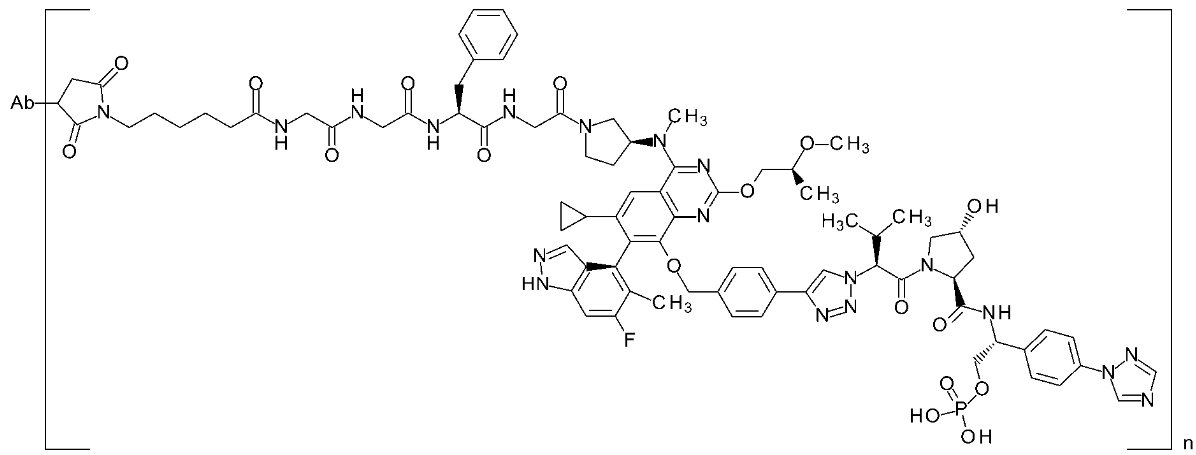

(AD-25)

wherein n is a number of 1 to 20.

(26-20) The antibody-drug conjugate or the salt thereof according to any one of (26-1) to (26-19), wherein Ab is cetuximab, and n is a number of 2 to 5.

The antibody-drug conjugate of formula (I) or the salt thereof may have tautomers or geometric isomers

depending on the types of substituents. In the present specification, the compound of formula (I) may be described in only one form of isomers. The present invention encompasses the other isomers and also encompasses separated isomers or mixtures thereof.

The antibody-drug conjugate of formula (I) or the salt thereof may have a chiral carbon atom or a chiral axis and may have diastereomers based thereon. The present invention also encompasses separated diastereomers of the antibody-drug conjugate of formula (I) or the salt thereof or mixtures thereof.

The antibody-drug conjugate of formula (I) or the salt thereof is an antibody-drug conjugate of formula (I) or a pharmaceutically acceptable salt of a salt thereof and may form an acid-addition salt or a salt with a base depending on the types of substituents. Examples thereof include salts described in P. Heinrich Stahl, Handbook of Pharmaceutical Salts Properties, Selection, and Use, Wiley-VCH, 2008. Specific examples thereof include acid-addition salts with inorganic acids such as hydrochloric acid, hydrobromic acid, hydroiodic acid, sulfuric acid, nitric acid, and phosphoric acid or organic acids such as formic acid, acetic acid, propionic acid, oxalic acid, malonic acid, succinic acid, fumaric acid, maleic acid, lactic acid, malic acid, mandelic acid, tartaric acid, dibenzoyltartaric acid, ditoluoyltartaric acid, citric acid, methanesulfonic acid, ethanesulfonic acid, benzenesulfonic acid, p-toluenesulfonic acid, aspartic acid, and glutamic acid, salts with inorganic metals such as sodium, potassium, magnesium, calcium, and aluminum, salts with organic bases such as methylamine, ethylamine, and ethanolamine, salts with various amino acids and amino acid derivatives such as acetylleucine, lysine, and ornithine, and ammonium salts.

The present invention further encompasses various hydrates or solvates and crystalline polymorphic substances of the antibody-drug conjugate of formula (I) or the salt thereof.

The present invention also encompasses every antibody-drug conjugate of formula (I) or salt thereof labeled with one or more pharmaceutically acceptable radioactive or nonradioactive isotopes. Preferred examples of the isotope for use in the isotope label for the compound of the present invention include isotopes of hydrogen ($^{2}H$ and $^{3}H$, etc.), carbon ($^{11}C$, $^{13}C$, and $^{14}C$, etc.), nitrogen ($^{13}N$ and $^{15}N$, etc.), oxygen ($^{15}O$, $^{17}O$, and $^{18}O$, etc.), fluorine ($^{18}F$, etc.), chlorine ($^{36}Cl$, etc.), iodine ($^{123}I$ and $^{125}I$, etc.), and sulfur ($^{35}S$, etc.).

The isotope-labeled compound of the present invention may be used in, for example, a study such as tissue distribution study of a drug and/or a substrate. For example, a radioactive isotope such as tritium ($^{3}H$) or carbon-14 ($^{14}C$) may be used for this purpose because of easy labeling and convenient detection.

Substitution by a heavier isotope, for example, substitution of hydrogen by deuterium ($^{2}H$), may be therapeutically advantageous (e.g., *in vivo* increase in half-life, decrease in necessary dose, and decrease in drug interaction) through improvement in metabolic stability.

Substitution by a positron-emitting isotope ($^{11}C$, $^{18}F$, $^{15}O$, and $^{13}N$, etc.) may be used in a positron emission tomography (PET) test in order to test a substrate receptor occupancy.

The isotope-labeled compound of the present invention can be produced by a conventional approach generally known to those skilled in the art or by, for example, the same or similar production method as in Examples or Production Examples using an appropriate isotope-labeled reagent instead of an unlabeled reagent.

(Production method)

The antibody-drug conjugate of formula (I) or the salt thereof can be produced by the application of various synthesis methods known in the art, through the use of a feature based on its basic structure or the types of substituents. Depending on the type of a functional group, the replacement of the functional group with an appropriate protective group (group easily convertible to the functional group) at a stage from a starting material to an intermediate may be effective for a production technique. Examples of such a protective group can include protective groups described in P. G. M. Wuts and T. W. Greene, "Greene's Protective Groups in Organic Synthesis", 5th edition, John Wiley & Sons Inc., 2014, which can be appropriately selected and used depending on their reaction conditions. In such a method, the protective group can be introduced and, after reaction, removed, if necessary, to obtain the desired compound.

Hereinafter, representative methods for producing the antibody-drug conjugate of formula (I) or the salt thereof as well as drug (D), drug-linker conjugate ($L^{A}$-D), and antibody or antigen binding fragment (Ab) constituting the antibody-drug conjugate or the salt thereof will be described. Each production method may be performed with reference to a reference document given in the description. The production method of the present invention is not limited to the following examples.

In the present specification, the following abbreviations may be used.

DMF: N,N-dimethylformamide, DMAc: N,N-dimethylacetamide, THF: tetrahydrofuran, MeCN: acetonitrile, MeOH: methanol, EtOH: ethanol, iPrOH: isopropyl alcohol, tBuOH: tert-butyl alcohol, DOX: 1,4-dioxane, DMSO: dimethyl sulfoxide, TEA: triethylamine, DIPEA: N,N-diisopropylethylamine, tBuOK: potassium tert-butoxide, $PdCl_2(dppf)\cdot CH_2Cl_2$: [1,1'-bis(diphenylphosphino)ferrocene]palladium(II) dichloride-dichloromethane adduct, Pd/C: palladium carbon, PyBOP: (benzotriazol-1-yloxy)tripyrrolidinophosphonium hexafluorophosphate, $iPr_2O$: diisopropyl ether, HATU: 1-[bis(dimethylamino)methylene]-1H-1,2,3-triazolo[4,5-b]pyridinium 3-oxide hexafluorophosphate, DABCO: 1,4-diazabicyclo[2.2.2]octane, TFA: trifluoroacetic acid, TfOH: trifluoromethanesulfonic acid, COMU: N-[({[(1Z)-1-cyano-2-ethoxy-2-oxoethylidene]amino}oxy)(morpholin-4-yl)methylene]-N-methylmethanminium hexafluorophosphate, NMM: N-methylmorpholine, CDI: 1,1'-carbodiimidazole, NMO: N-methylmorpholine N-oxide, NMP: N-methyl-2-pyrrolidone, LHMDS: lithium bis(trimethylsilyl)amide, NHMDS: sodium bis(trimethylsilyl)amide, DMEDA: N,N'-dimethylethylenediamine, Triton B: benzyl trimethylammonium hydroxide, Xantphos: 4,5-bis(diphenylphosphino)-9,9-dimethylxanthene, Ruphos: 2-dicyclohexylphosphino-2',6'-diisopropoxide phenyl, Xphos: 2-dicyclohexylphosphino-2',4',6'-triisopropylbiphenyl, BINAP: 2,2'-bis(diphenylphosphino)-1,1'-binaphthyl.

I. Production method for drug (D)

Drug (D) can be obtained by a method described in, for example, International Publication No. WO 2022/173032, WO 2023/171781, or WO 2024/019103. Hereinafter, general synthesis methods for drug (D) will be shown.

(Drug production method 1)

This production method is the first method for producing drug (D) represented by formula (II) among drugs (D).

[Chemical Formula 162]

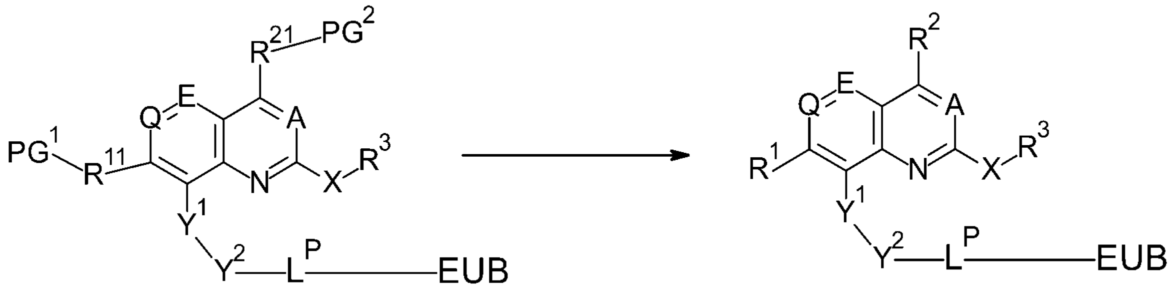

( 1 )

( II )

wherein $PG^1$-$R^{11}$ is a group in which protective group $PG^1$ is bonded to NH or OH contained in $R^1$, and $PG^2$-$R^{21}$ is a group in which protective group $PG^2$ is bonded to NH or OH contained in $R^2$ (the same holds true for the description below).

The drug represented by formula (II) can be obtained by applying compound (1) to deprotection reaction conditions under acidic conditions. In this context, examples of the protective group that may be deprotected under acidic conditions include a tert-butoxycarbonyl group, a triphenylmethyl group, a tetrahydro-2H-pyran-2-yl group, a methoxymethyl group, a dimethylmethanediyl group, and a tert-butylsulfinyl group.

This reaction is performed by using compound (1) and the same equivalent or an excessive equivalent of a deprotection reagent, and stirring them under cooling to under heating to reflux usually for 0.1 hours to 5 days in a solvent inert to the reaction. In this context, examples of the deprotection reagent used include, but are not particularly limited to, acids such as hydrogen chloride (DOX solution), trifluoroacetic acid, methanesulfonic acid, phosphoric acid, p-toluenesulfonic acid, and trifluoromethanesulfonic acid, and mixtures thereof. In this context, examples of the solvent used include, but are not particularly limited to, alcohols such as MeOH and EtOH, halogenated hydrocarbons such as dichloromethane, 1,2-dichloromethane, and chloroform, ethers such as diethyl ether, THF, DOX, and dimethoxyethane, DMF, DMSO, MeCN, TfOH, and water, and mixtures thereof.

The deprotection may be performed through catalytic hydrogenation reaction or under basic conditions by selecting a protective group. Examples of the protective group that may be deprotected through catalytic hydrogenation reaction include a benzyl group, a p-methoxybenzyl group, and a benzyloxycarbonyl group. The deprotection may be performed with a fluoride ion source such as tetra-n-butylammonium fluoride. Examples of the protective group include a tert-butyl(dimethyl)silyl group and a (trimethylsilyl)ethoxymethyl group. Examples of the protective group that may be deprotected under basic conditions include an acetyl group, a trifluoroacetyl group, and a benzoyl group. The deprotection may be performed in stages by selecting protective groups that can be deprotected under different deprotection conditions as $PG^1$ and $PG^2$, respectively.

For a reference document of this reaction, see the following, for example.

P. G. M. Wuts and T. W. Greene, "Greene's Protective Groups in Organic Synthesis (fifth edition, 2014)"

When starting material compound (1) has axial chirality, this reaction may be performed using a stereoisomer obtained by temporarily separating compound (1).

(Drug production method 2)

This production method is the second method for producing drug (D) represented by formula (II) among drugs (D).

[Chemical Formula 163]

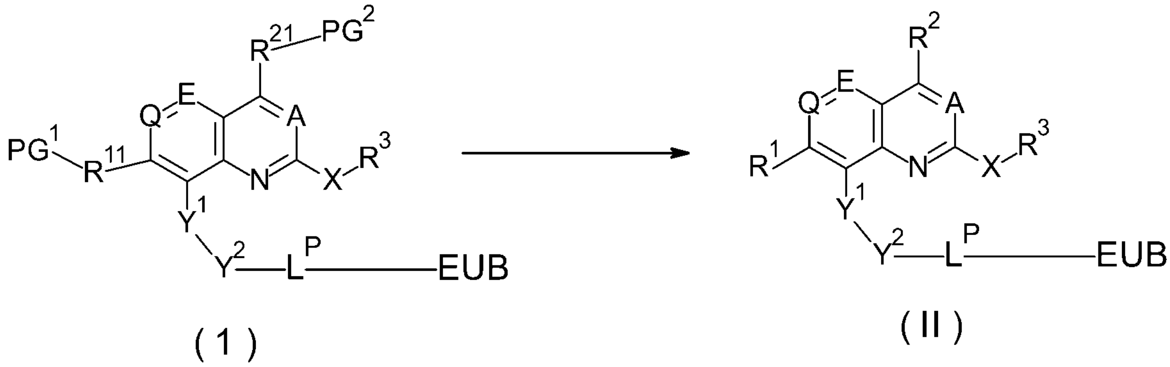

The drug represented by formula (II) can be obtained by applying compound (1) to deprotection reaction conditions under acidic conditions, then isolating a free form by treatment under basic conditions, and then applying it to salification reaction conditions. In this context, examples of the protective group that may be deprotected under acidic conditions include a tert-butoxycarbonyl group, a triphenylmethyl group, a tetrahydro-2H-pyran-2-yl group, a methoxymethyl group, a dimethylmethanediyl group, and a tert-butylsulfinyl group.

In this reaction, compound (1) and the same equivalent or an excessive equivalent of a deprotection reagent are used, stirred under cooling to under heating to reflux usually for 0.1 hours to 5 days in a solvent inert to the reaction, and treated with a basic aqueous solution, followed by the isolation of a free form. Subsequent operation is performed by using the same equivalent or an excessive equivalent of an acidic reagent, and stirring them under cooling to under heating to reflux usually for 0.1 hours to 5 days in a solvent inert to the reaction. In this context, examples of the deprotection reagent used include, but are not particularly limited to, acids such as hydrogen chloride (DOX solution), trifluoroacetic acid, methanesulfonic acid, phosphoric acid, p-toluenesulfonic acid, and trifluoromethanesulfonic acid, and mixtures thereof. In this context, examples of the solvent used include, but are not particularly limited to, alcohols such as MeOH and EtOH, halogenated hydrocarbons such as dichloromethane, 1,2-dichloromethane, and chloroform, ethers such as diethyl ether, THF, DOX, and dimethoxyethane, DMF, DMSO, MeCN, TfOH, and water, and mixtures thereof. In this context, examples of the acidic reagent used include acids such as hydrogen chloride (DOX solution), phosphoric acid, and p-toluenesulfonic acid. In this context, examples of the basic aqueous solution used include, but are not particularly limited to, an aqueous sodium bicarbonate solution.

The deprotection may be performed through catalytic hydrogenation reaction or under basic conditions by selecting a protective group. Examples of the protective group that may be deprotected through catalytic hydrogenation reaction include a benzyl group, a p-methoxybenzyl group, and a benzyloxycarbonyl group. The deprotection may be performed with a fluoride ion source such as tetra-n-butylammonium fluoride. Examples of the protective group include a tert-butyl(dimethyl)silyl group and a (trimethylsilyl)ethoxymethyl group. Examples of the protective group that may be deprotected under basic conditions include an acetyl group, a trifluoroacetyl group, and a benzoyl group. The deprotection may be performed in stages by selecting protective groups that can be deprotected under different deprotection conditions as $PG^1$ and $PG^2$, respectively.

For a reference document of this reaction, see the following, for example.

P. G. M. Wuts and T. W. Greene, "Greene's Protective Groups in Organic Synthesis (fifth edition, 2014)"

When starting material compound (1) has axial chirality, this reaction may be performed using a stereoisomer obtained by temporarily separating compound (1).

(Drug starting material production method 1)

This production method is a method for producing compound (1)-1 included in compound (1) serving as a starting material of drug production method 1 and drug production method 2.

[Chemical Formula 164]

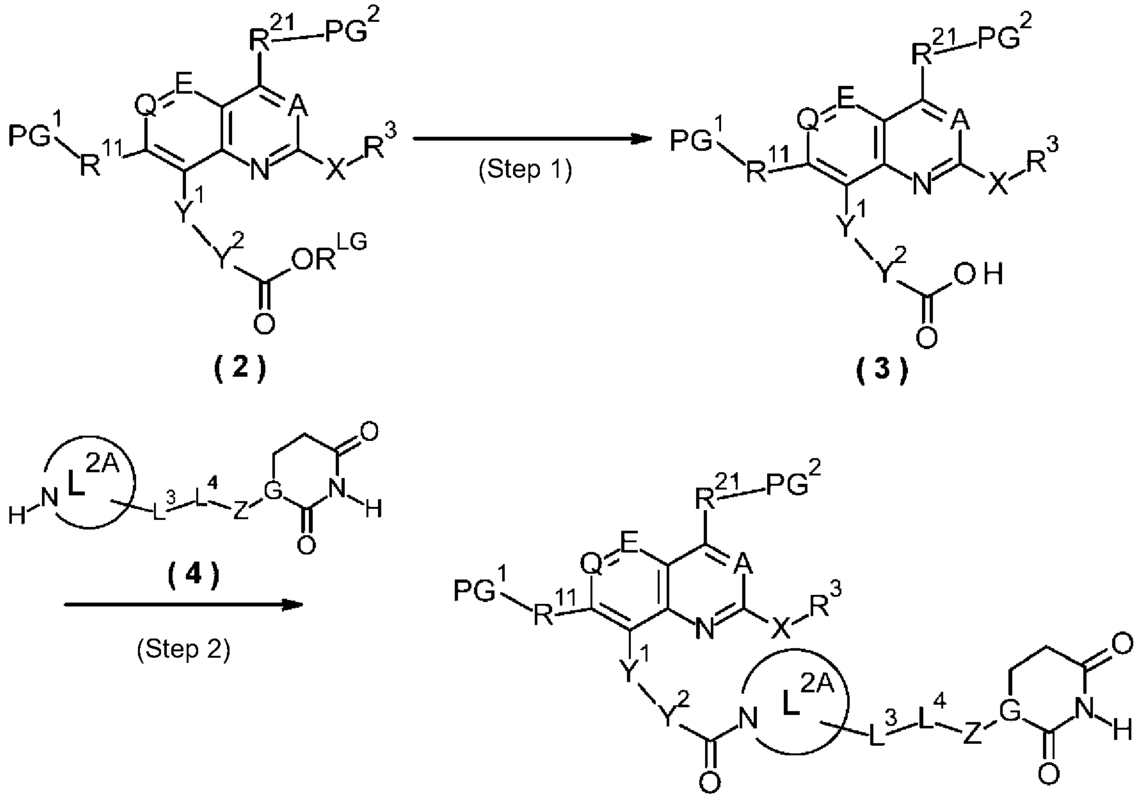


(2) (3) (4) (1)-1

wherein $L^{2A}$ represents piperidinediyl optionally substituted by $C_{1-3}$ alkyl, piperazinediyl optionally substituted by $C_{1-3}$ alkyl, pyrrolidinediyl optionally substituted by $C_{1-3}$ alkyl, bridged piperazinediyl, or 2,6-diazaspiro[3.4]octanediyl, and $R^{LG}$ represents a $C_{1-12}$ alkyl group (the same holds true for the description below).

(Step 1)

This step is the step of obtaining compound (3) by applying compound (2) to hydrolysis conditions.

This reaction is performed by using compound (2) and the same equivalent or an excessive equivalent of a hydrolysis reagent, and stirring them under cooling to under heating to reflux usually for 1 hour to 5 days in a solvent inert to the reaction. In this context, examples of the hydrolysis reagent used include, but are not particularly limited to, an aqueous sodium hydroxide solution, an aqueous potassium hydroxide solution, an aqueous lithium hydroxide solution, and trimethyl tin hydroxide. Examples of the solvent include, but are not particularly limited to, alcohols such as methanol, ethanol, and n-propanol, ether solvents such as tetrahydrofuran, diethyl ether, and 1,4-dioxane, halogenated hydrocarbons such as dichloromethane, 1,2-dichloroethane, and chloroform, acetonitrile, water, and mixtures thereof.

(Step 2)

This step is the step of obtaining compound (1)-1 by applying compound (3) and compound (4) to condensation reaction conditions.

This reaction is performed by using compound (3) and compound (4) in the same equivalents or one of them in an excessive equivalent, adding a condensing agent and a base to a mixture thereof, and stirring them at room temperature usually for 1 hour to 1 days in a solvent inert to the reaction. In this context, examples of the condensing agent used include, but are not particularly limited to, HATU, 1-(3-dimethylaminopropyl)-3-ethylcarbodiimide and hydrochloride thereof, dicyclohexylcarbodiimide, 1,1'-carbonyldiimidazole, COMU, and PyBOP. Examples of the base include, but are not particularly limited to, organic bases such as triethylamine, N,N-diisopropylethylamine, and pyridine, and inorganic bases such as potassium carbonate, sodium carbonate, and cesium carbonate. Examples of the solvent include, but are not particularly limited to, halogenated hydrocarbons such as dichloromethane, 1,2-dichloroethane, and chloroform, ether solvents such as tetrahydrofuran, diethyl ether, and 1,4-dioxane, alcohols such as methanol, ethanol, and n-propanol,

N,N-dimethylformamide, and mixtures thereof.

(Drug starting material production method 2)

This production method is the first method for producing compound (2)-1 included in compound (2) serving as a starting material of drug starting material production method 1.

[Chemical Formula 165-1]

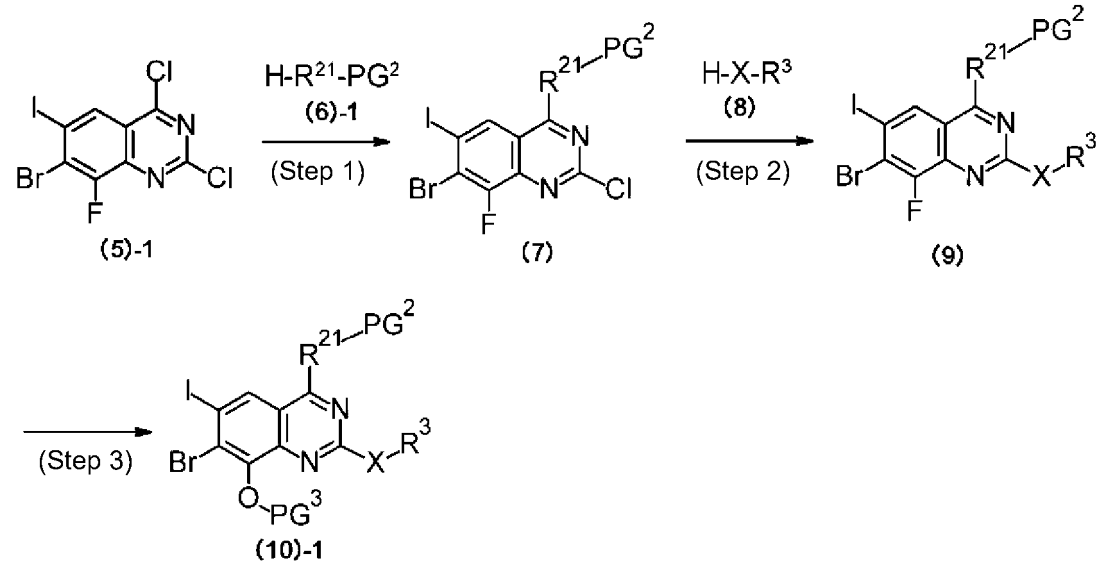


[Chemical Formula 165-2]

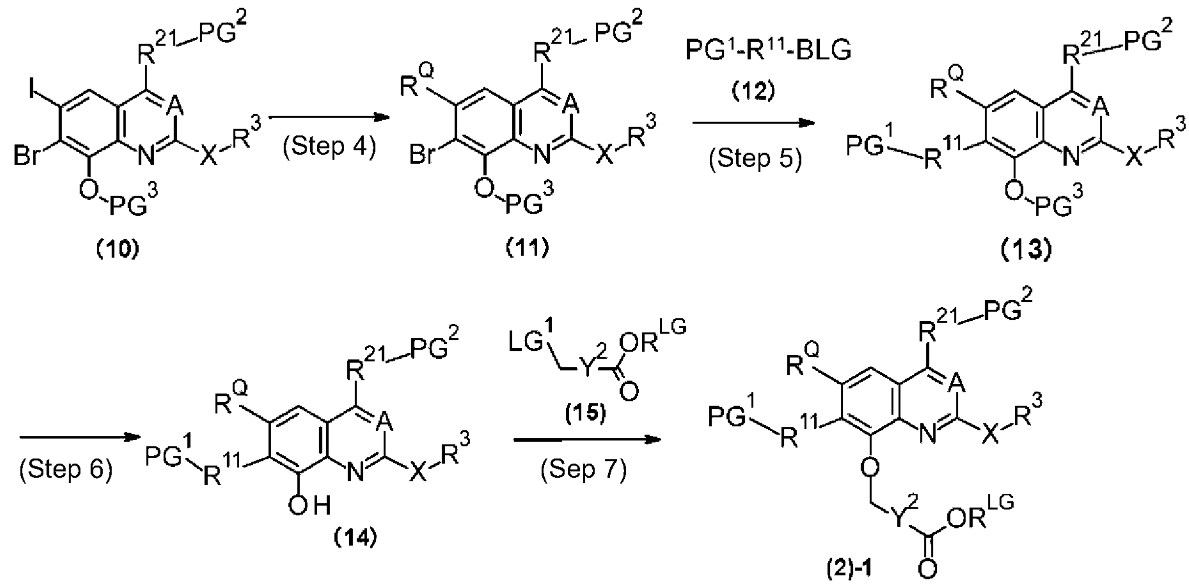


wherein $PG^3$ represents a protective group for OH, $LG^1$ represents a leaving group, BLG represents a boronic acid group, a boronic acid group protected with a boronic acid protective group, such as a boronic acid pinacol ester group, or a trifluoroboric acid base (hereinafter, also referred to as a boronic acid group, etc.). In this context, examples of the leaving group include Cl, Br, a methanesulfonyloxy group, and a p-toluenesulfonyloxy group (the same holds true for the description below).

(Step 1)

This step is a method for producing compound (7) through ipso-substitution reaction of compound (5)-1 and

compound (6)-1.

This reaction involves using compound (5)-1 and compound (6)-1 in the same equivalents or one of them in an excessive equivalent, and stirring a mixture thereof under cooling to under heating to reflux, preferably at 0°C to 120°C, usually for 0.1 hours to 5 days in a solvent inert to the reaction or without a solvent. In this context, examples of the solvent used include, but are not particularly limited to, halogenated hydrocarbons such as dichloromethane, 1,2-dichloroethane, and chloroform, aromatic hydrocarbons such as benzene, toluene, and xylene, ethers such as diethyl ether, THF, dehydrated THF, DOX, and 1,2-dimethoxyethane, DMF, DMAc, DMSO, ethyl acetate, MeCN, NMP, and mixtures thereof. The reaction in the presence of an organic base such as TEA, DIPEA, NMM, DABCO, or tBuOK, or an inorganic base such as sodium hydroxide, potassium carbonate, sodium carbonate, or cesium carbonate may be advantageous for allowing the reaction to proceed smoothly.

Alternatively, compound (7) may be produced by catalytic hydrogenation reaction of a compound obtained through Mizoroki-Heck reaction of compound (5)-1 and compound (6)-1.

[Reference]

Chem. Rev., 2003, 103, p. 2945-2964

(Step 2)

This step is a method for producing compound (9) through ipso-substitution reaction of compound (7) and compound (8).

The reaction conditions are the same as those for step 1 of drug starting material production method 2.

Compound (9) may be produced by Negishi coupling of a compound with a hydrogen atom of compound (8) converted to halogen and compound (7).

[Reference]

ACC. Chem. Res., 1982, 15, p. 340-348

(Step 3)

This step is a method for producing compound (10)-1 through ipso-substitution reaction of compound (9) and $PG^3$-OH.

In this context, examples of $PG^3$-OH include benzyl alcohol, p-methoxybenzyl alcohol, and 1-phenylethanol.

The reaction conditions are the same as those for step 1 of drug starting material production method 2.

(Step 4)

This step is a method for producing compound (11) through Suzuki-Miyaura coupling reaction of compound (10) including both of compound (10)-1 obtained in step 3 of this synthesis method, and compound (10)-2 obtained in (Step 3 of drug starting material production method 9) mentioned later, and a boronic acid derivative consisting of $R^Q$-boronic acid group, etc. In this context, examples of the boronic acid group, etc. used include, but are not particularly limited to, a boronic acid group, a boronic acid ester group, a boronic acid pinacol ester group, a triol borate base, and a trifluoroboric acid base.

This reaction involves using compound (10) and a boronic acid derivative consisting of $R^Q$-boronic acid group, etc. in the same equivalents or one of them in an excessive equivalent, and stirring a mixture thereof at room temperature to under heating to reflux, preferably at 20°C to 140°C, usually for 0.1 hours to 5 days in a solvent inert to the reaction in the presence of a base and a palladium catalyst. In this context, examples of the solvent used include, but are not particularly limited to, halogenated hydrocarbons such as dichloromethane, 1,2-dichloroethane, and chloroform, aromatic hydrocarbons such as benzene, toluene, and xylene, ethers such as diethyl ether, THF, DOX, and 1,2-dimethoxyethane, alcohols such as MeOH, EtOH, isopropyl alcohol, butanol, and amyl alcohol, DMF, DMSO, MeCN, 1,3-dimethylimidazolidin-2-one, water, and mixtures thereof. Examples of the base include inorganic bases such as tripotassium phosphate, sodium carbonate, potassium carbonate, sodium hydroxide, barium hydroxide, and cesium carbonate. Examples of the palladium catalyst include tetrakis(triphenylphosphine)palladium, bis(triphenylphosphine)palladium(II) dichloride, [1,1'-bis(diphenylphosphino)ferrocene]palladium(II) dichloride-dichloromethane adduct, (1E,4E)-1,5-diphenylpenta-1,4-dien-3-one/palladium (3:2), (2-dicyclohexylphosphino-2',6'-diisopropoxy-1,1'-biphenyl)[2-(2'-amino-1,1'-biphenyl)]palladium(II) methanesulfonate, palladium(II) acetate, and mesyl[(tri-tert-butylphosphine)-2-(2-aminobiphenyl)]palladium(II). The reaction in the presence of a ligand such as dicyclohexyl(2',6'-dimethoxybiphenyl-2-yl)phosphine, dicyclohexyl(2',6'-diisopropoxy-[1,1'-biphenyl]-2-yl)phosphine, 1,1'-bis(diphenylphosphino)ferrocene, butyl di-1-adamantylphosphine, or di(adamantan-1-yl)(butyl)phosphine may be advantageous for allowing the reaction to proceed smoothly. A precatalyst of (2-dicyclohexylphosphino-2',6'-diisopropoxy-1,1'-biphenyl)[2-(2'-amino-1,1'-biphenyl)]palladium(II) can also be used as the palladium catalyst in this reaction. The heating of the mixture by microwave irradiation may be advantageous for allowing the reaction to proceed smoothly.

[Reference]

J. Am. Chem. Soc., 2005, 127, p. 4685-4696
Org. Lett. 2011, 13, p. 3948-3951
Org. Lett. 2012, 14, p. 1278-1281

Compound (11) (wherein $R^Q$ is hydrogen) may be produced through deiodination reaction of compound (10) using a Pd catalyst and a reducing agent.

[Reference]

J. Org. Chem., 1977, 42, p. 3491-3494
Tetrahedron Letters 2013, 54, 5207-5210

(Step 5)

This step is a method for producing compound (13) through Suzuki-Miyaura coupling reaction of compound (11) and compound (12).
The reaction conditions are the same as those for step 4 of drug starting material production method 2.
Compound (13) may have axial chirality and may be obtained as a mixture of stereoisomers. Each stereoisomer can be isolated by usual resolution operation, for example, resolution using ODS column chromatography or silica gel column chromatography, of compound (13) wherein $PG^1$ is a protective group or a compound (e.g., compound (14)) obtained by deprotection reaction of compound (13).

(Step 6)

This step is a method for producing compound (14) by deprotecting compound (13) through catalytic hydrogenation reaction.
This reaction can be performed by stirring compound (13) under cooling to under heating, preferably at room temperature, for 1 hour to 5 days under ordinary pressure to increased pressure in a hydrogen atmosphere in a solvent inert to the reaction, such as MeOH, EtOH, or ethyl acetate, in the presence of a metal catalyst. Examples of the metal catalyst used include palladium catalysts such as Pd/C and palladium black, platinum catalysts such as platinum plates and platinum oxide, and nickel catalysts such as reduced nickel and Raney nickel. When a tetrahydro-2H-pyran-2-yl group or the like is used as protective group $PG^1$, a base may be used for suppressing deprotection thereof. In this context, examples of the base used include, but are not particularly limited to, sodium bicarbonate, sodium carbonate, potassium carbonate, and cesium carbonate.
Compound (14) may have axial chirality and can be obtained as a mixture of stereoisomers. Each stereoisomer can be isolated by usual resolution operation, for example, resolution using ODS column chromatography or silica gel column chromatography, of compound (14) wherein $PG^1$ is a protective group or a compound obtained by deprotection reaction of compound (14).
For a reference document of this reaction, see the following, for example. The Chemical Society of Japan ed., "Jikken Kagaku Koza (Courses in Experimental Chemistry in English)", fifth edition, vol. 13, Maruzen Co., Ltd., 2004

(Step 7)

This step is a method for producing compound (2)-1 through reaction of compound (14) and compound (15).
This reaction is performed by using compound (14) and compound (15) in the same equivalents or one of them in an excessive equivalent, and reacting a mixture thereof under cooling to under heating to reflux, preferably at 0°C to 80°C, usually for 0.1 hours to 5 days in a solvent inert to the reaction in the presence of a base. In this context, examples of the

solvent used include, but are not particularly limited to, aromatic hydrocarbons such as benzene, toluene, and xylene, alcohols such as MeOH and EtOH, ethers such as diethyl ether, THF, DOX, and 1,2-dimethoxyethane, halogenated hydrocarbons such as dichloromethane, 1,2-dichloroethane, and chloroform, DMF, DMSO, ethyl acetate, MeCN, and mixtures thereof. Examples of the base include, but are not particularly limited to, organic bases such as TEA, DIPEA, 1,8-diazabicyclo[5.4.0]-7-undecene, n-butyllithium, and tBuOK, and inorganic bases such as sodium hydroxide, sodium carbonate, potassium carbonate, cesium carbonate, and sodium hydride. The reaction in the presence of a phase transfer catalyst such as tetra-n-butylammonium chloride may be advantageous.

For a reference document of this reaction, see the following, for example.

The Chemical Society of Japan ed., "Jikken Kagaku Koza (Courses in Experimental Chemistry in English)", fifth edition, vol. 14, Maruzen Co., Ltd., 2005

Compound (2)-1 may have axial chirality and can be obtained as a mixture of stereoisomers. Each stereoisomer can be isolated by usual resolution operation, for example, resolution using ODS column chromatography or silica gel column chromatography, of compound (2)-1 wherein $PG^1$ is a protective group or a compound obtained by deprotection reaction of compound (2)-1.

Compound (15) can be produced as a compound having a sulfonyloxy group as $LG^1$ by sulfonylating a compound having a hydroxy group as a moiety corresponding to $LG^1$ in the presence of a base. In this context, examples of the sulfonylation reagent used include, but are not particularly limited to, methanesulfonyl chloride, p-toluenesulfonyl chloride, and methanesulfonic anhydride. Examples of the base include, but are not particularly limited to, TEA, DIPEA, pyridine, and tetramethylethylenediamine.

For a reference document of this reaction, see the following, for example. Synthesis 1999, 9, p. 1633-1636

(Drug starting material production method 3)

This production method is the second method for producing compound (2)-1 included in compound (2) serving as a starting material of drug starting material production method 1.

[Chemical Formula 166-1]

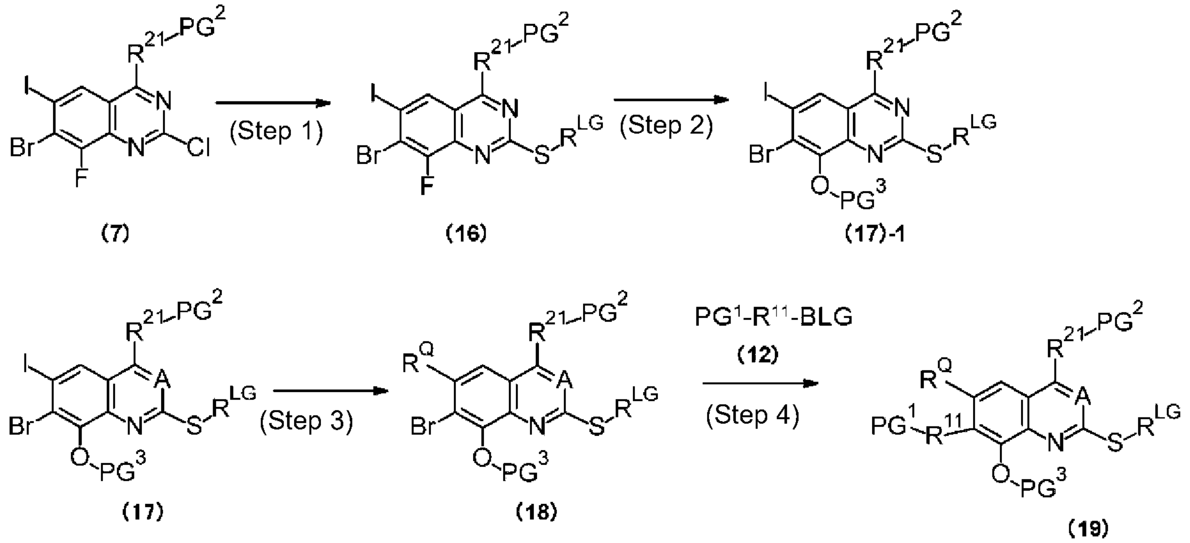


(7) (16) (17)-1

(17) (18) (19)

[Chemical Formula 166-2]

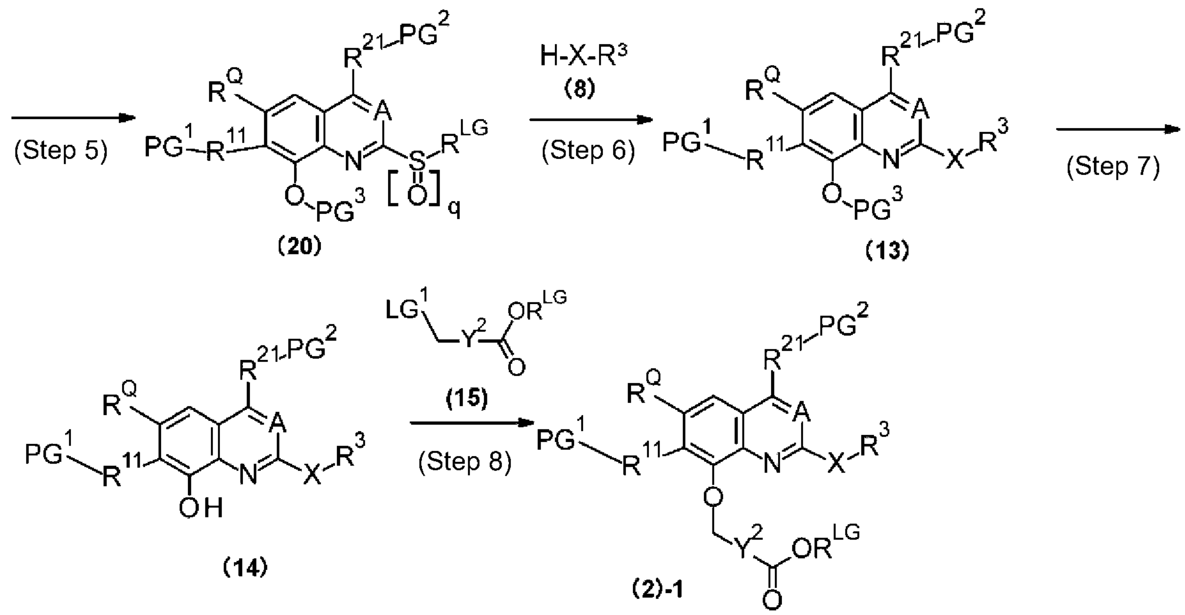


wherein q represents 1 or 2 (the same holds true for the description below).

(Step 1)

This step is a method for producing compound (16) through ipso-substitution reaction of compound (7) and $R^{LG}$-SH. In this context, examples of $R^{LG}$-SH used include $C_{1-12}$ alkylthiol, for example, ethanethiol and dodecanethiol.
The reaction conditions are the same as those for step 1 of drug starting material production method 2.

(Step 2)

This step is a method for producing compound (17)-1 through ipso-substitution reaction of compound (16) and $PG^3$-OH. In this context, examples of $PG^3$-OH used include benzyl alcohol, p-methoxybenzyl alcohol, and 1-phenylethanol.
The reaction conditions are the same as those for step 1 of drug starting material production method 2.

(Step 3)

This step is a method for producing compound (18) through Suzuki-Miyaura coupling reaction of compound (17) including both of compound (17)-1 obtained in step 2 of this synthesis method, and compound (17)-2 obtained in (Step 4 of drug starting material production method 8) mentioned later, and a boronic acid derivative consisting of $R^Q$-boronic acid group, etc.
The reaction conditions are the same as those for step 4 of drug starting material production method 2.
Compound (18) (wherein $R^Q$ is hydrogen) may be produced through deiodination reaction of compound (17) using a Pd catalyst and a reducing agent.

[Reference]

J. Org. Chem., 1977, 42, p. 3491-3494
Tetrahedron Letters 2013, 54, 5207-5210

(Step 4)

This step is a method for producing compound (19) through Suzuki-Miyaura coupling reaction of compound (18) and compound (12).

The reaction conditions are the same as those for step 4 of drug starting material production method 2.

Compound (19) may have axial chirality and may be obtained as a mixture of stereoisomers. Each stereoisomer can be isolated by usual resolution operation, for example, resolution using ODS column chromatography or silica gel column chromatography, of compound (19). The stereoisomer of compound (19) can also be isolated by deprotecting compound (19), isolating a stereoisomer, and re-protecting it with a protective group.

Examples of the protective group for re-protection include a tetrahydro-2H-pyran-2-yl group.

(Step 5)

This step is a method for producing compound (20) through oxidation reaction of compound (19).

This reaction involves treating compound (19) with the same equivalent or an excessive equivalent of an oxidizing agent under cooling to under heating, preferably at - 20°C to 80°C, usually for 0.1 hours to 3 days in a solvent inert to the reaction. In this reaction, oxidation using m-chloroperbenzoic acid, perbenzoic acid, peracetic acid, sodium hypochlorite, or hydrogen peroxide is preferably used. Examples of the solvent include aromatic hydrocarbons such as benzene and toluene, ethers such as THF, halogenated hydrocarbons such as chloroform and dichloromethane, DMF, DMSO, ethyl acetate, MeCN, and mixtures thereof. Other examples of the oxidizing agent include cumene hydroperoxide, oxone, active manganese dioxide, chromic acid, potassium permanganate, and sodium periodate.

[Reference]

The Chemical Society of Japan ed., "Jikken Kagaku Koza (Courses in Experimental Chemistry in English)", fifth edition, vol. 17, Maruzen Co., Ltd., 2004

(Step 6)

This step is a method for producing compound (13) through ipso-substitution reaction of compound (20) and compound (8).

The reaction conditions are the same as those for step 1 of drug starting material production method 2.

Compound (13), when having axial chirality, can be obtained as a mixture of stereoisomers. Each stereoisomer can be isolated by usual resolution operation, for example, resolution using ODS column chromatography or silica gel column chromatography.

(Step 7)

This step is a method for producing compound (14) by deprotecting compound (13) through catalytic hydrogenation reaction.

The reaction conditions are the same as those for step 6 of drug starting material production method 2.

Compound (14) may have axial chirality and may be obtained as a mixture of stereoisomers. Each stereoisomer can be isolated by usual resolution operation, for example, resolution using ODS column chromatography or silica gel column chromatography, of compound (14).

(Step 8)

This step is a method for producing compound (2)-1 through reaction of compound (14) and compound (15).

The reaction conditions are the same as those for step 7 of drug starting material production method 2.

Compound (2)-1 may have axial chirality and may be obtained as a mixture of stereoisomers. Each stereoisomer can be isolated by usual resolution operation, for example, resolution using ODS column chromatography or silica gel column chromatography, of compound (2)-1. The stereoisomer of compound (2)-1 can also be isolated by deprotecting compound (2)-1, isolating a stereoisomer, and re-protecting it with a protective group.

Examples of the protective group for re-protection include a tetrahydro-2H-pyran-2-yl group.

(Drug starting material production method 4)

This production method is the third method for producing compound (2)-1 included in compound (2) serving as a starting material of drug starting material production method 1.

[Chemical Formula 167]

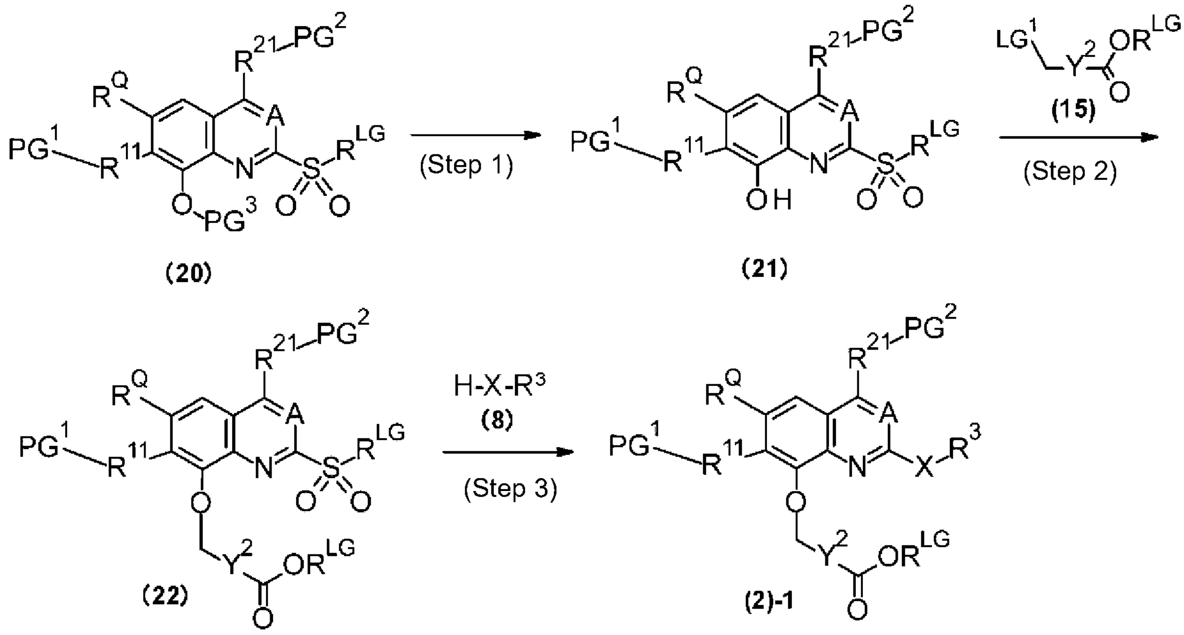


(Step 1)

This step is a method for producing compound (21) by deprotecting compound (20) through catalytic hydrogenation reaction.

The reaction conditions are the same as those for step 6 of drug starting material production method 2.

Compound (21) may have axial chirality and may be obtained as a mixture of stereoisomers. Each stereoisomer can be isolated by usual resolution operation, for example, resolution using ODS column chromatography or silica gel column chromatography, of compound (21).

(Step 2)

This step is a method for producing compound (22) through alkylation reaction of compound (21) and compound (15).

The reaction conditions are the same as those for step 7 of drug starting material production method 2.

Compound (22) may have axial chirality and may be obtained as a mixture of stereoisomers. Each stereoisomer can be isolated by usual resolution operation, for example, resolution using ODS column chromatography or silica gel column chromatography, of compound (22). The stereoisomer of compound (22) can also be isolated by deprotecting compound (22), isolating a stereoisomer, and re-protecting it with a protective group.

Examples of the protective group for re-protection include a tetrahydro-2H-pyran-2-yl group.

(Step 3)

This step is a method for producing compound (2)-1 through ipso-substitution reaction of compound (22) and compound (8).

The reaction conditions are the same as those for step 1 of drug starting material production method 2.

Compound (2)-1 may have axial chirality and can be obtained as a mixture of stereoisomers. Each stereoisomer can be isolated by usual resolution operation, for example, resolution using ODS column chromatography or silica gel column chromatography, of a compound obtained by deprotection reaction of compound (2)-1 or compound (2)-1.

In this context, the reaction conditions of the deprotection reaction used are the same as those for the step described in production method 1.

(Drug starting material production method 5)

This production method is the first method for producing compound (2)-2 included in compound (2) serving as a starting material of drug starting material production method 1.

[Chemical Formula 168-1]

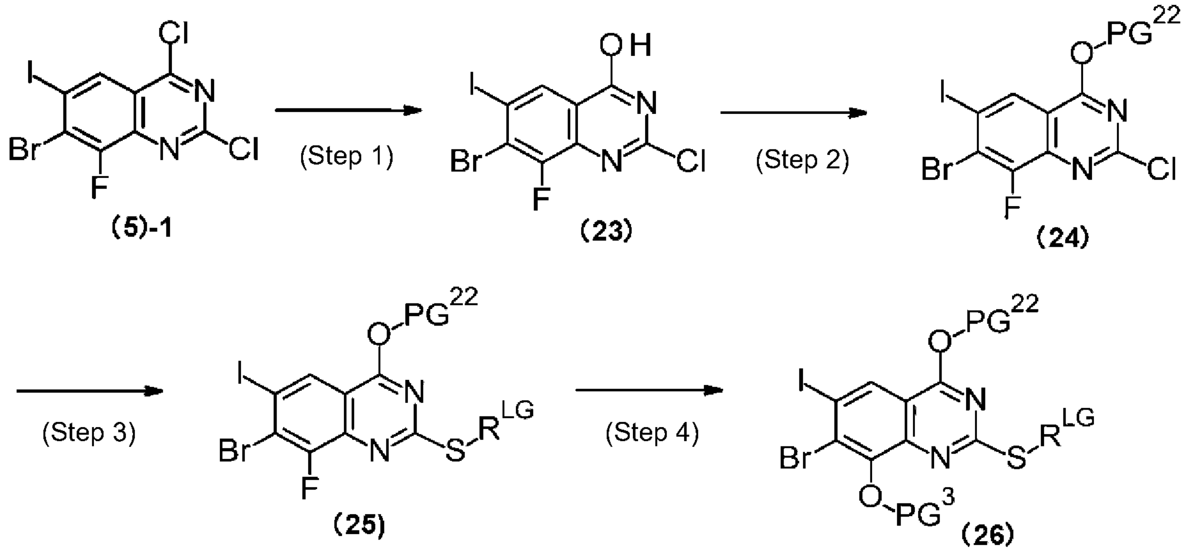


(5)-1 (23) (24) (25) (26)

[Chemical Formula 168-2]

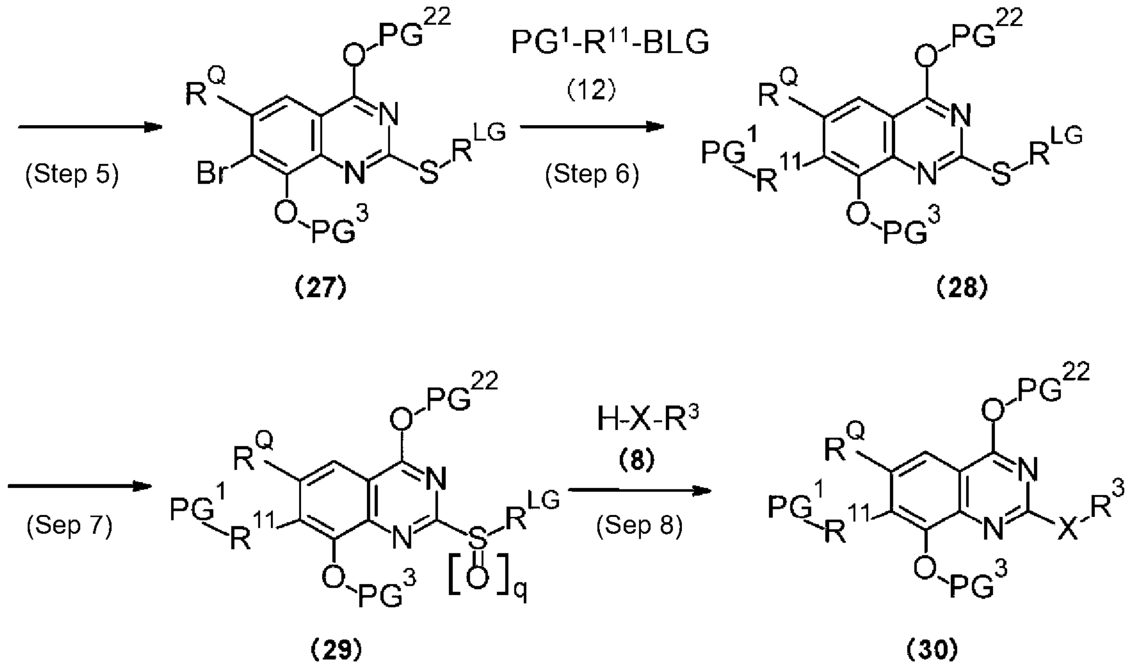


(27) (28) (29) (30)

[Chemical Formula 168-3]

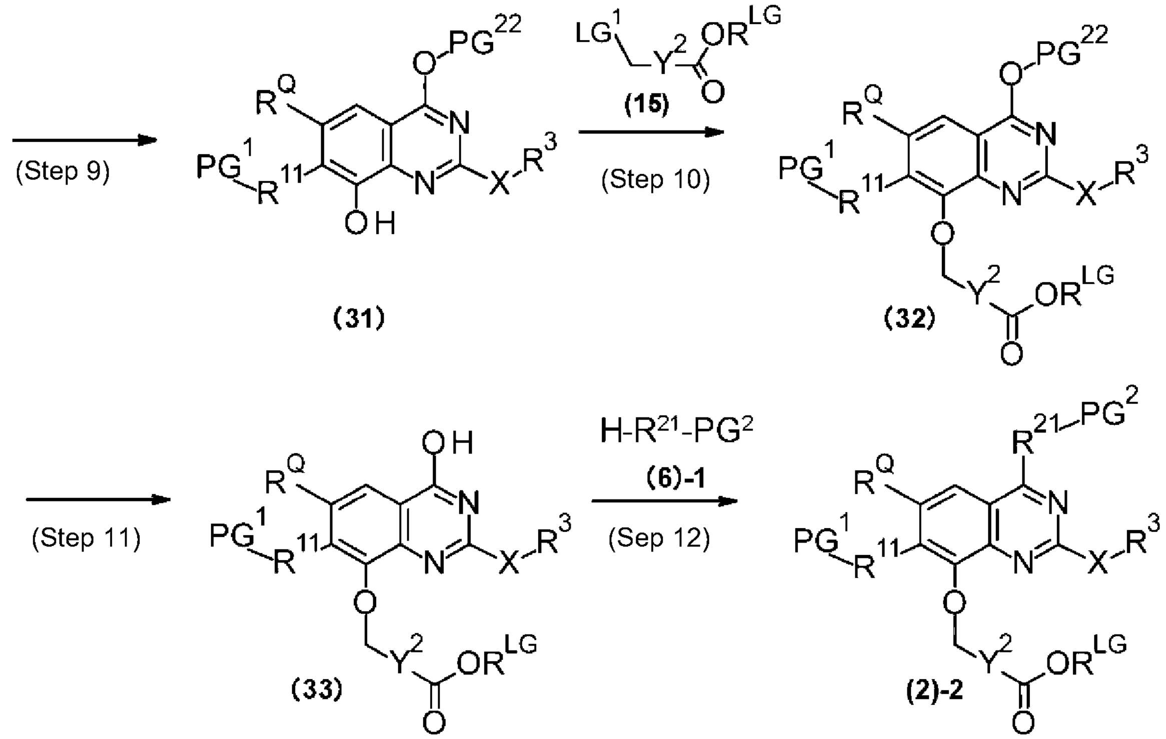


wherein $PG^{22}$ represents a tert-butyl group (the same holds true for the description below).

(Step 1)

This step is a method for producing compound (23) by hydrolyzing compound (5)-1.

This reaction is performed by using compound (5)-1 and a hydrolysis reagent in the same equivalents or one of them in an excessive equivalent, and stirring them under cooling to under heating to reflux usually for 0.1 hours to 5 days in a solvent inert to the reaction. In this context, examples of the solvent used include, but are not particularly limited to, alcohols such as methanol and ethanol, acetone, DMF, and THF. A mixed solvent of the solvent described above with water may be suitable for the reaction. Examples of the hydrolysis reagent include, but are not particularly limited to, an aqueous sodium hydroxide solution, an aqueous potassium hydroxide solution, sodium hydroxide, potassium hydroxide, and lithium hydroxide.

For a reference document of this reaction, see the following, for example. The Chemical Society of Japan ed., "Jikken Kagaku Koza (Courses in Experimental Chemistry in English) (fifth edition)", vol. 16 (2005) (Maruzen Co., Ltd.) Angew. Chem. Int. Ed. 2005, 44, p. 1378-1382

(Step 2)

This step is a method for producing compound (24) by protecting a hydroxy group of compound (23) with a tert-butyl group.

This reaction is performed by using compound (23) and a tert-butyl protection reagent in the same equivalents or one of them in an excessive equivalent, and stirring them under cooling to under heating to reflux usually for 0.1 hours to 5 days in a solvent inert to the reaction. In this context, examples of the solvent used include, but are not particularly limited to, ethers such as THF, DOX, halogenated hydrocarbons such as dichloromethane, tBuOH, and DMF. Examples of the tert-butyl protection reagent include, but are not particularly limited to, isobutene and 2-tert-butyl-1,3-diisopropylisourea.

Compound (24) may be produced through dehydration condensation reaction of compound (23) and tBuOH.

For a reference document of this reaction, see the following, for example. P. G. M. Wuts and T. W. Greene, "Greene's Protective Groups in Organic Synthesis", fifth edition, John Wiley & Sons Inc., 2014
Org. Lett., 2012, 14, 17, p. 4678-4681

(Step 3)

This step is a method for producing compound (25) through ipso-substitution reaction of compound (24) and $R^{LG}$-SH.
The reaction conditions are the same as those for step 1 of drug starting material production method 2.

(Step 4)

This step is a method for producing compound (26) through ipso-substitution reaction of compound (25) and $PG^3$-OH.
The reaction conditions are the same as those for step 1 of drug starting material production method 2.

(Step 5)

This step is a method for producing compound (27) through Suzuki-Miyaura coupling reaction of compound (26) and a boronic acid derivative consisting of $R^Q$-boronic acid group, etc.
The reaction conditions are the same as those for step 4 of drug starting material production method 2.
Compound (27) (wherein $R^Q$ is hydrogen) may be produced through deiodination reaction of compound (26) using a Pd catalyst and a reducing agent.

[Reference]

J. Org. Chem., 1977, 42, p. 3491-3494
Tetrahedron Letters 2013, 54, 5207-5210

(Step 6)

This step is a method for producing compound (28) through Suzuki-Miyaura coupling reaction of compound (27) and compound (12).
The reaction conditions are the same as those for step 4 of drug starting material production method 2.
Compound (28) may have axial chirality and may be obtained as a mixture of stereoisomers. Each stereoisomer can be isolated by usual resolution operation, for example, resolution using ODS column chromatography or silica gel column chromatography, of compound (28). The stereoisomer of compound (28) can also be isolated by deprotecting compound (28), isolating a stereoisomer, and re-protecting it with a protective group.
Examples of the protective group for re-protection include a tetrahydro-2H-pyran-2-yl group.

(Step 7)

This step is a method for producing compound (29) through oxidation reaction of compound (28).
The reaction conditions are the same as those for step 5 of drug starting material production method 3.
For a reference document of this reaction, see the following, for example. P. G. M. Wuts and T. W. Greene, "Greene's Protective Groups in Organic Synthesis", fifth edition, John Wiley & Sons Inc., 2014

(Step 8)

This step is a method for producing compound (30) through ipso-substitution reaction of compound (29) and compound (8).
The reaction conditions are the same as those for step 1 of drug starting material production method 2.

(Step 9)

This step is a method for producing compound (31) by deprotecting compound (30) through catalytic hydrogenation reaction.
The reaction conditions are the same as those for step 6 of drug starting material production method 2.
Compound (31) may have axial chirality and may be obtained as a mixture of stereoisomers. Each stereoisomer can be isolated by usual resolution operation, for example, resolution using ODS column chromatography or silica gel

column chromatography, of compound (31).

(Step 10)

This step is a method for producing compound (32) through alkylation reaction of compound (31) and compound (15).

The reaction conditions are the same as those for step 7 of drug starting material production method 2.

Compound (32) may have axial chirality and may be obtained as a mixture of stereoisomers. Each stereoisomer can be isolated by usual resolution operation, for example, resolution using ODS column chromatography or silica gel column chromatography, of compound (32). The stereoisomer of compound (32) can also be isolated by deprotecting compound (32), isolating a stereoisomer, and re-protecting it with a protective group.

(Step 11)

This step is a method for producing compound (33) by subjecting compound (32) to deprotection reaction.

The reaction conditions are the same as those for the step described in drug production method 1.

Compound (33) can be deprotected and then re-protected with a protective group. Examples of the protective group for re-protection include a tetrahydro-2H-pyran-2-yl group.

A series of operations of deprotection and re-protection with a protective group as described above may be performed as one-step reaction.

(Step 12)

This step is a method for producing compound (2)-2 through reaction of compound (33) and compound (6)-1.

This reaction involves using compound (33) and compound (6)-1 in the same equivalents or one of them in an excessive equivalent, and stirring a mixture thereof under cooling to under heating, preferably at -20°C to 60°C, usually for 0.1 hours to 5 days in a solvent inert to the reaction in the presence of a condensing agent. Examples of the solvent include, but are not particularly limited to, aromatic hydrocarbons such as benzene and toluene, ethers such as THF and DOX, halogenated hydrocarbons such as chloroform and dichloromethane, alcohols such as methanol and ethanol, DMF, DMSO, ethyl acetate, MeCN, and mixtures thereof. Examples of the condensing agent include PyBOP, HATU, and CDI. The reaction in the presence of an organic base such as TEA, DIPEA, or NMM, or an inorganic base such as potassium carbonate, sodium carbonate, or cesium carbonate may be advantageous for allowing the reaction to proceed smoothly.

(Drug starting material production method 6)

This production method is a method for producing compound (13)-1 included in compound (13) serving as an intermediate of drug starting material production method 2.

[Chemical Formula 169]

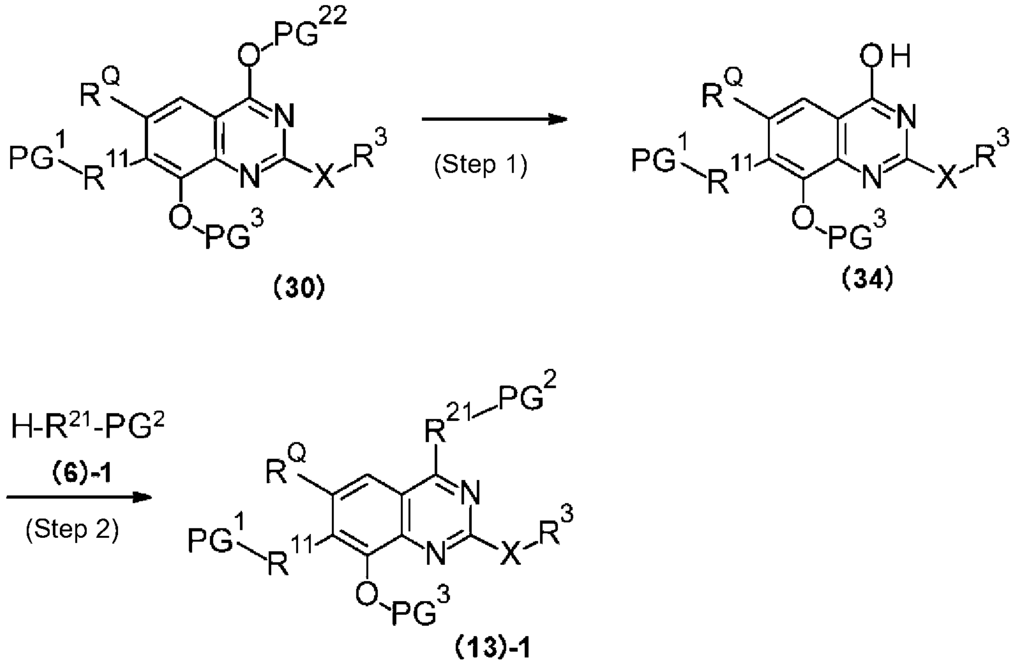


(30)

(34)

(13)-1

(Step 1)

This step is a method for producing compound (34) by subjecting compound (30) to deprotection reaction.

The reaction conditions are the same as those for the step described in drug production method 1.

Compound (34) can be deprotected and then re-protected with a protective group. Examples of the protective group for re-protection include a tetrahydro-2H-pyran-2-yl group.

A series of operations of deprotection and re-protection with a protective group as described above may be performed as one-step reaction.

(Step 2)

This step is a method for producing compound (13)-1 through reaction of compound (34) and compound (6)-1.

The reaction conditions are the same as those for step 12 of drug starting material production method 5.

(Drug starting material production method 7)

This production method is a method for producing compound (30) serving as a starting material of drug starting material production method 6.

[Chemical Formula 170]

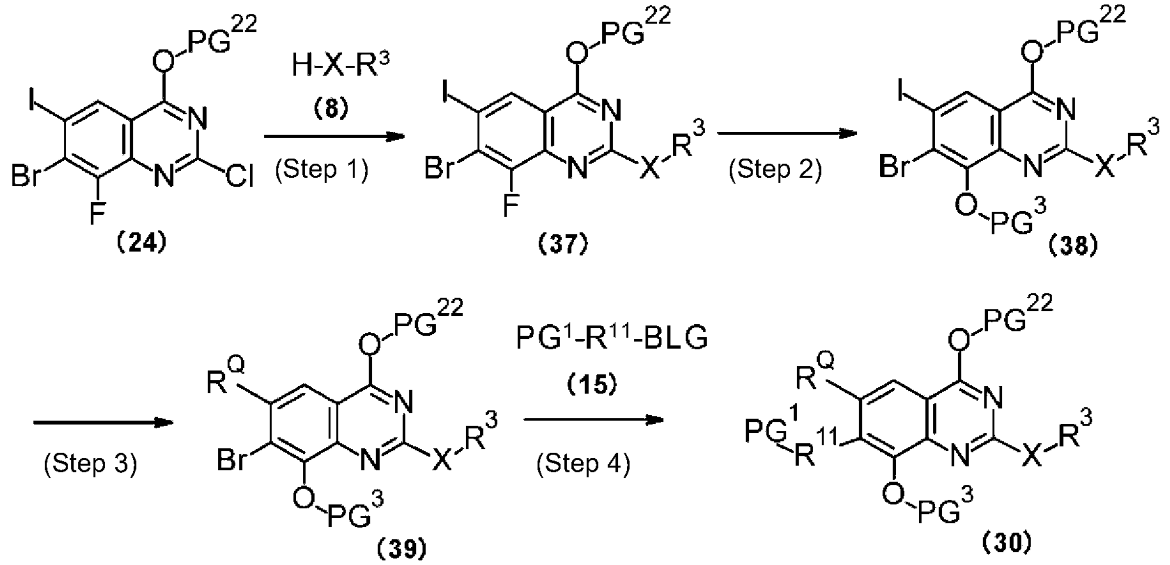


(Step 1)

This step is a method for producing compound (37) through ipso-substitution reaction of compound (24) and compound (8).
The reaction conditions are the same as those for step 1 of drug starting material production method 2.

(Step 2)

This step is a method for producing compound (38) through ipso-substitution reaction of compound (37) and $PG^3$-OH.
The reaction conditions are the same as those for step 1 of drug starting material production method 2.

(Step 3)

This step is a method for producing compound (39) through Suzuki-Miyaura coupling reaction of compound (38) and a boronic acid derivative consisting of $R^Q$-boronic acid group, etc.
The reaction conditions are the same as those for step 4 of drug starting material production method 2.
Compound (39) (wherein $R^Q$ is hydrogen) may be produced through dehalogenation reaction of compound (38) using a Pd catalyst and a reducing agent.

[Reference]

J. Org. Chem., 1977, 42, p. 3491-3494
Tetrahedron Letters 2013, 54, 5207-5210

(Step 4)

This step is a method for producing compound (30) through Suzuki-Miyaura coupling reaction of compound (39) and compound (15).
The reaction conditions are the same as those for step 4 of drug starting material production method 2.
Compound (30) may have axial chirality and may be obtained as a mixture of stereoisomers. Each stereoisomer can be isolated by usual resolution operation, for example, resolution using ODS column chromatography or silica gel column chromatography, of compound (30). The stereoisomer of compound (30) can also be isolated by deprotecting compound (30), isolating a stereoisomer, and re-protecting it with a protective group.

(Drug starting material production method 8)

This production method is a method for producing compound (17)-2 included in compound (17) serving as an intermediate of drug starting material production method 3.

[Chemical Formula 171]

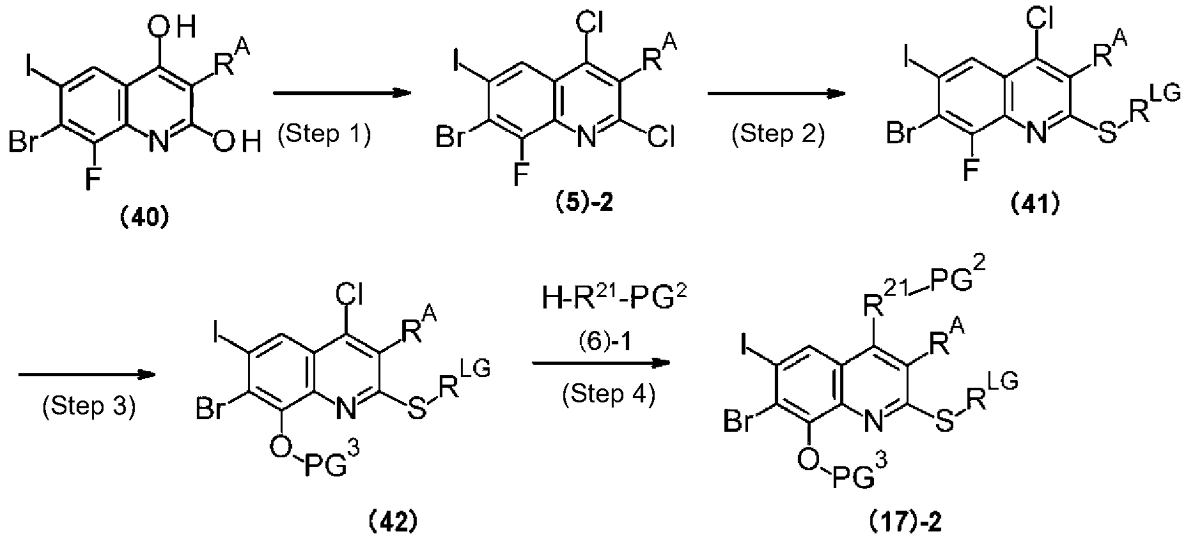


(Step 1)

This step is a method for producing compound (5)-2 through chlorination reaction of compound (40).

This reaction is performed by using compound (40) and a chlorinating agent in the same equivalents or one of them in an excessive equivalent, and stirring a mixture thereof under cooling to under heating to reflux, preferably at 60°C to under heating to reflux, usually for 0.1 hours to 5 days in a solvent inert to the reaction or without a solvent. In this context, examples of the solvent used include, but are not particularly limited to, aromatic hydrocarbons such as toluene, ethers such as THF and DOX, halogenated hydrocarbons such as dichloromethane, DMF, and DMAc. Examples of the chlorinating agent include phosphorus oxychloride and thionyl chloride. The reaction in the presence of an organic base such as TEA, DIPEA, or NMM may be advantageous for allowing the reaction to proceed smoothly.

(Step 2)

This step is a method for producing compound (41) through ipso-substitution reaction of compound (5)-2 and $R^{LG}$-SH.

The reaction conditions are the same as those for step 1 of drug starting material production method 2.

(Step 3)

This step is a method for producing compound (42) through ipso-substitution reaction of compound (41) and $PG^3$-OH.

The reaction conditions are the same as those for step 1 of drug starting material production method 2.

(Step 4)

This step is a method for producing compound (17)-2 through ipso-substitution reaction of compound (42) and compound (6)-1.

The reaction conditions are the same as those for step 1 of drug starting material production method 2.

(Drug starting material production method 9)

This production method is a method for producing compound (10)-2.

[Chemical Formula 172]

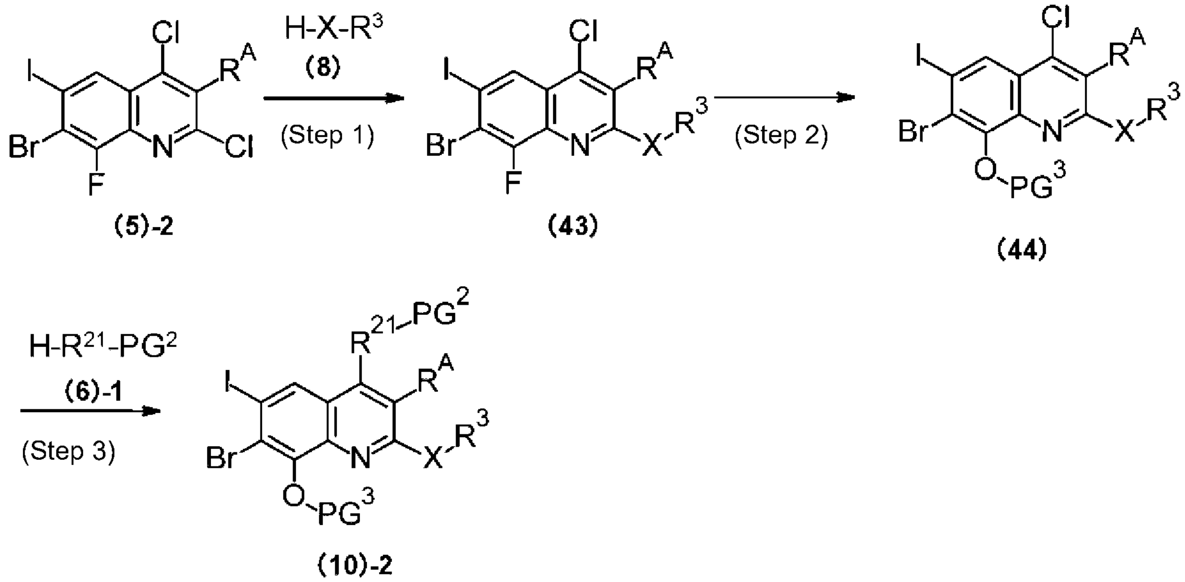


(Step 1)

This step is a method for producing compound (43) through ipso-substitution reaction of compound (5)-2 and compound (8).

The reaction conditions are the same as those for step 1 of drug starting material production method 2.

Compound (43) may be produced by Negishi coupling of a compound with a hydrogen atom of compound (8) converted to halogen and compound (5)-2.

(Step 2)

This step is a method for producing compound (44) through ipso-substitution reaction of compound (43) and $PG^3$-OH.

The reaction conditions are the same as those for step 1 of drug starting material production method 2.

(Step 3)

This step is a method for producing compound (10)-2 through ipso-substitution reaction of compound (44) and compound (6)-1.

The reaction conditions are the same as those for step 1 of drug starting material production method 2.

(Drug starting material production method 10)

This production method is a method for producing compound (4)-1 included in compound (4) serving as an intermediate of drug starting material production method 1.

[Chemical Formula 173]

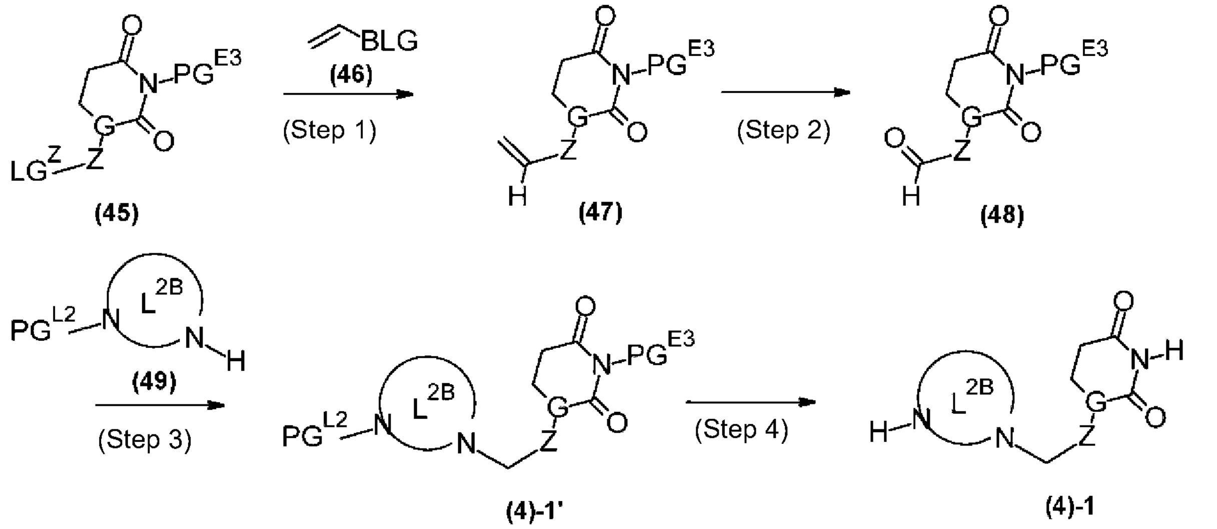


wherein $L^{2B}$ represents piperazinediyl optionally substituted by $C_{1-3}$ alkyl, bridged piperazinediyl, or 2,6-diazaspiro[3.4] octanediyl, $PG^{E3}$ and $PG^{L2}$ each represent an NH protective group, $LG^Z$ represents a leaving group, and BLG represents a boronic acid group, a boronic acid group protected with a boronic acid protective group, such as a boronic acid pinacol ester group, or a trifluoroboric acid base (hereinafter, also referred to as a boronic acid group, etc.). For a compound wherein $PG^{E3}$ moiety of compound (45) is H, compound (4)-1 can be produced through similar reaction. In this context, examples of the leaving group include Cl, Br, a methanesulfonyloxy group, and a p-toluenesulfonyloxy group (the same holds true for the description below).

(Step 1)

This step is the step of obtaining compound (47) by applying compound (45) and compound (46) to Suzuki-Miyaura coupling reaction conditions.

The reaction conditions are the same as those for step 4 of drug starting material production method 2.

(Step 2-1A)

This step is the step of obtaining a 1,2-diol compound by applying compound (47) to oxidation reaction conditions.

This reaction is performed by using compound (47), an oxidizing agent, and an excessive equivalent of a re-oxidizing agent, and stirring them under ice cooling to at room temperature usually for 1 hour to 2 days in a solvent inert to the reaction. In this context, examples of the oxidizing agent used include, but are not particularly limited to, osmium(VIII) tetroxide, PI osmium(VIII) oxide, and PEM polymer-microcapsulated osmium(VIII) oxide. In this context, examples of the re-oxidizing agent used include, but are not particularly limited to, NMO, trimethylamine oxide, tert-butyl hydroperoxide (tBuOOH), and $K_3Fe(CN)_6$. In this context, examples of the solvent used include, but are not particularly limited to, ether solvents such as tetrahydrofuran, diethyl ether, and 1,4-dioxane, halogenated hydrocarbons such as dichloromethane, dichloroethane, and chloroform, tBuOH, acetone, acetonitrile, toluene, water, and mixtures thereof.

(Step 2-1B)

This step is the step of obtaining compound (48) by applying the compound obtained in (Step 2-1A) above to oxidative cleavage reaction conditions.

This reaction is performed by using the compound obtained in (Step 2-1A) and the same equivalent or an excessive equivalent of an oxidizing agent, and stirring them under ice cooling to at room temperature usually for 1 hour to 2 days in a solvent inert to the reaction. In this context, examples of the oxidizing agent used include, but are not particularly limited to, sodium periodate and periodic acid. In this context, examples of the solvent used include, but are not particularly limited to, ether solvents such as tetrahydrofuran, diethyl ether, and 1,4-dioxane, acetonitrile, water, halogenated hydrocarbons such as dichloromethane, dichloroethane, and chloroform, and mixtures thereof.

(Step 2-1A) and (Step 2-1B) described above may be performed as one-step reaction.

[Reference]

J. Org. Chem. 1956, 21, 4, 478-479

(Step 2-2)

This step is the step of obtaining compound (48) by applying compound (47) to oxidation reaction conditions. This step is a method for obtaining compound (48) by a method different from that in step 2-1A and step 2-1B.

This reaction is performed by stirring compound (47) under ice cooling to at room temperature usually for 1 hour to 1 day in an ozone atmosphere in a solvent inert to the reaction, followed by treatment with a reducing agent. In this context, examples of the reducing agent include, but are not particularly limited to, dimethyl sulfide, triphenylphosphine, and metal zinc. In this context, examples of the solvent used include, but are not particularly limited to, alcohol solvents such as methanol and ethanol, halogenated hydrocarbons such as dichloromethane, dichloroethane, and chloroform, ethyl acetate, and mixtures thereof.

(Step 3)

This step is the step of obtaining compound (4)-1' by applying compound (48) and compound (49) to reductive amination reaction conditions.

This reaction is performed by using compound (48) and compound (49) in the same equivalents or one of them in an excessive equivalent, and stirring them under ice cooling to at room temperature usually for 1 hour to 5 days in a solvent inert to the reaction in the presence of a reducing agent and acetic acid. In this context, examples of the reducing agent used include, but are not particularly limited to, $NaBH(OAc)_3$, 2-picoline borane, and $NaBH_3CN$. In this context, examples of the solvent used include, but are not particularly limited to, halogenated hydrocarbons such as dichloromethane, dichloroethane, and chloroform, ether solvents such as tetrahydrofuran, diethyl ether, and 1,4-dioxane, alcohol solvents such as methanol and ethanol, and acetonitrile.

(Step 4)

This step is the step of obtaining compound (4)-1 by applying (4)-1' to deprotection conditions.

The reaction conditions are the same as those for drug production method 1.

(Drug starting material production method 11)

This production method is a method for producing compound (45)-1' included in compound (45) serving as a starting material of drug starting material production method 10, and compound (45)-1, wherein $PG^{E3}$ moiety of compound (45)-1' is H, included in compound (45).

[Chemical Formula 174]

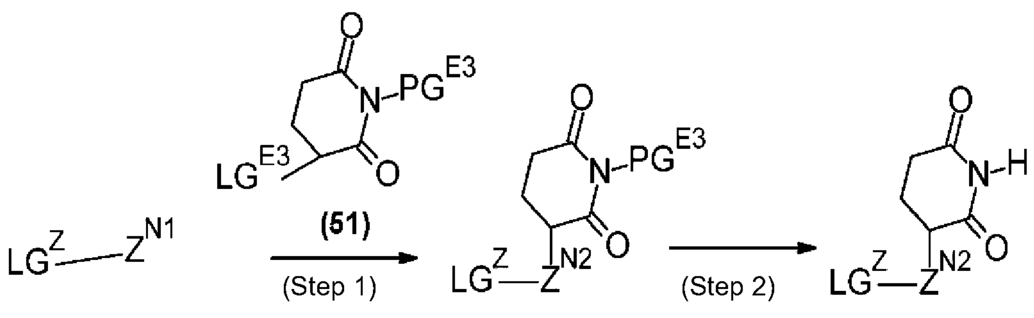


(50) (51) (45)-1' (45)-1

wherein $LG^{E3}$ represents a leaving group. In this context, examples of the leaving group include Cl, Br, a methanesulfonyloxy group, and a p-toluenesulfonyloxy group (the same holds true for the description below).

(Step 1)

This step is the step of obtaining compound (45)-1' by applying compound (50) and compound (51) to alkylation

reaction conditions.

This reaction is performed by using compound (50) and compound (51) in the same equivalents or one of them in an excessive equivalent, and stirring them under ice cooling to under heating to reflux usually for 1 hour to 1 day in a solvent inert to the reaction in the presence of a base. In this context, examples of the base used include, but are not particularly limited to, sodium hydroxide, tBuOK, LHMDS, NHMDS, potassium carbonate, and cesium carbonate. In this context, examples of the solvent used include, but are not particularly limited to, ether solvents such as tetrahydrofuran, diethyl ether, and 1,4-dioxane, DMF, and MeCN.

In this context, when the compound obtained through the reaction has a protective group, compound (45)-1' can be obtained by applying the obtained compound to deprotection reaction conditions after the reaction.

The reaction conditions are the same as those for drug production method 1.

(Step 2)

This step is the step of obtaining compound (45)-1 by applying (45)-1' to deprotection conditions.

The reaction conditions are the same as those for drug production method 1.

(Drug starting material production method 12)

This production method is a method for producing compound (45)-2' included in compound (45) serving as a starting material of drug starting material production method 10, and compound (45)-2, wherein $PG^{E3}$ moiety of compound (45)-2' is H, included in compound (45).

[Chemical Formula 175]

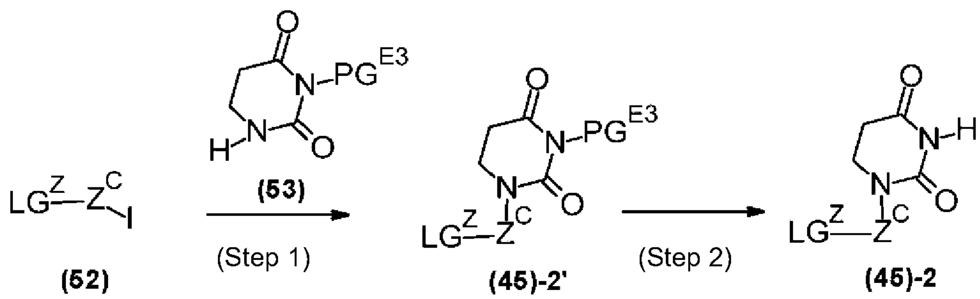


(52) (45)-2' (45)-2

(Step 1)

This step is the step of obtaining compound (45)-2' by applying compound (52) and compound (53) to coupling reaction conditions using a copper catalyst.

This reaction is performed by using compound (52) and compound (53) in the same equivalents or one of them in an excessive equivalent, and stirring them at room temperature to under heating to reflux usually for 1 hour to 5 days in a solvent inert to the reaction using a copper catalyst, a ligand, and a base. In this context, examples of the copper catalyst used include, but are not particularly limited to, copper(I) iodide, copper(I) chloride, and copper(I) oxide. In this context, examples of the ligand used include, but are not particularly limited to, DMEDA and trans-N,N'-dimethylcyclohexane-1,2'-diamine. In this context, examples of the base used include, but are not particularly limited to, organic bases such as diisopropylethylamine and triethylamine, and inorganic bases such as potassium carbonate, cesium carbonate, sodium carbonate, and tripotassium phosphate. In this context, examples of the solvent used include, but are not particularly limited to, ether solvents such as tetrahydrofuran and 1,4-dioxane, alcohol solvents such as methanol and ethanol, dimethyl sulfoxide, N,N-dimethylformamide, and water. The heating of the mixture by microwave irradiation may be advantageous for allowing the reaction to proceed smoothly.

(Step 2)

This step is the step of obtaining compound (45)-2 by applying (45)-2' to deprotection conditions.

The reaction conditions are the same as those for drug production method 1.

(Drug starting material production method 13)

This production method is a method for producing compound (4)-2 included in compound (4) serving as an

intermediate of drug starting material production method 1.

[Chemical Formula 176]

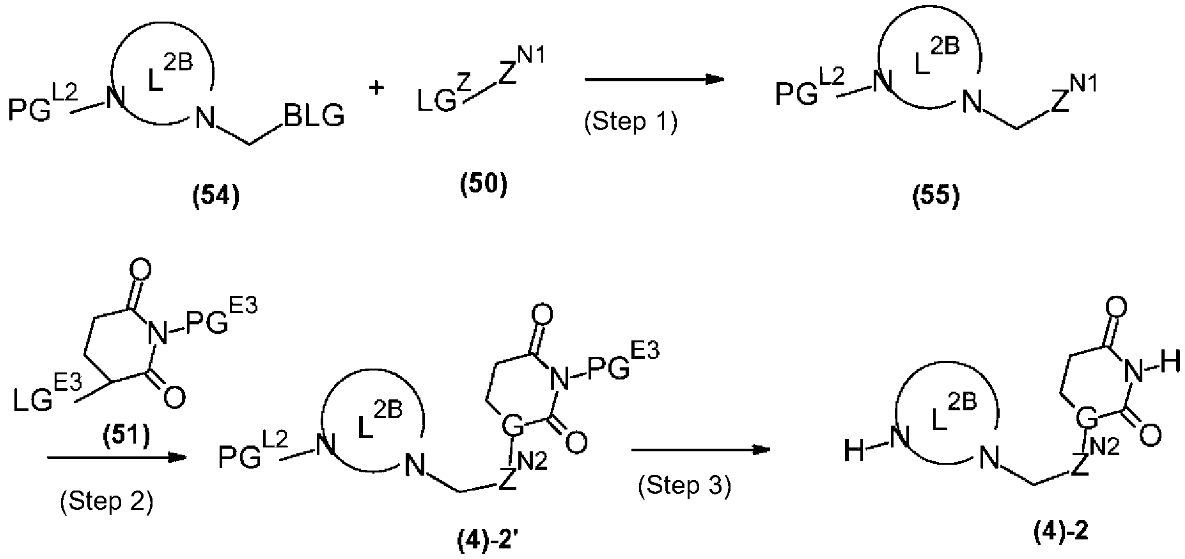


(54) (50) (55)

(51) (4)-2' (4)-2

wherein for a compound wherein $PG^{E3}$ moiety of compound (51) is H, a compound wherein $PG^{E3}$ moiety of compound (4)-2' is H can be produced through similar reaction (the same holds true for the description below).

(Step 1)

This step is the step of obtaining compound (55) by applying compound (54) and compound (50) to Suzuki-Miyaura coupling reaction conditions.

The reaction conditions are the same as those for step 4 of drug starting material production method 2.

(Step 2)

This step is the step of obtaining compound (4)-2' by applying compound (55) and compound (51) to alkylation reaction conditions.

The reaction conditions are the same as those for step 1 of drug starting material production method 11.

(Step 3)

This step is the step of obtaining compound (4)-2 by applying (4)-2' to deprotection conditions.

The reaction conditions are the same as those for drug production method 1.

(Drug starting material production method 14)

This production method is a method for producing compound (4)-3 included in compound (4) serving as an intermediate of drug starting material production method 1.

[Chemical Formula 177]

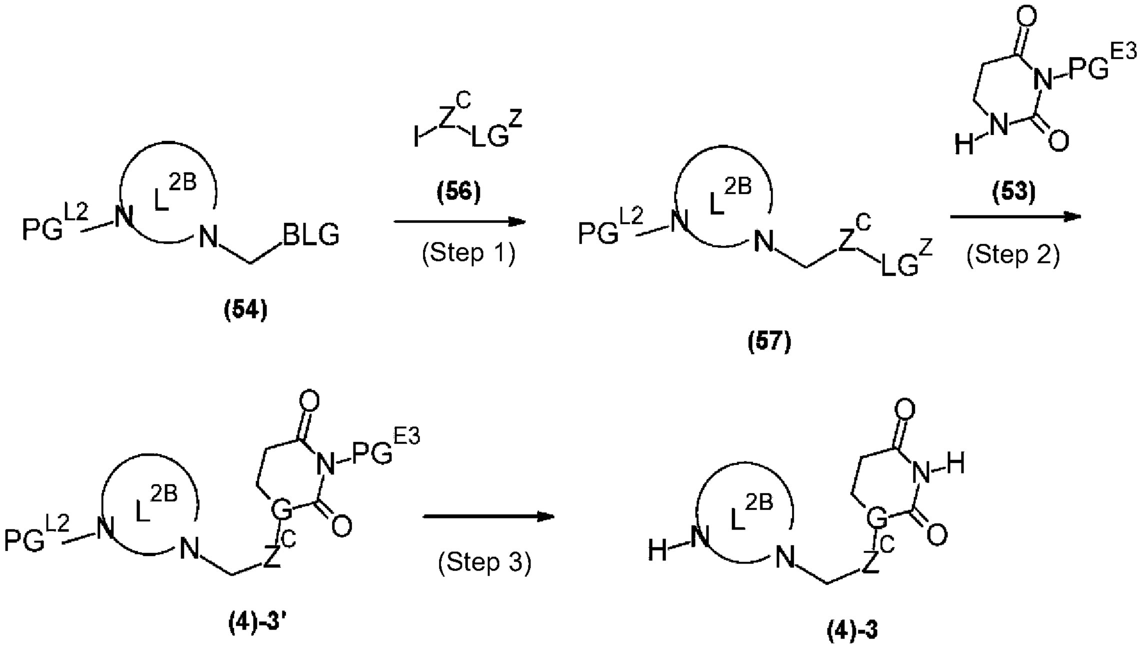


(54) (57) (4)-3' (4)-3

wherein $LG^Z$ represents a leaving group. In this context, examples of the leaving group include Cl, Br, a methanesulfonyloxy group, and a p-toluenesulfonyloxy group (the same holds true for the description below).

(Step 1)

This step is the step of obtaining compound (57) by applying compound (54) and compound (56) to Suzuki-Miyaura coupling reaction conditions.

The reaction conditions are the same as those for step 4 of drug starting material production method 2.

(Step 2)

This step is the step of obtaining compound (4)-3' by applying compound (57) and compound (53) to coupling reaction conditions using a copper catalyst.

The reaction conditions are the same as those for step 1 of drug starting material production method 12.

(Step 3)

This step is the step of obtaining compound (4)-3 by applying (4)-3' to deprotection conditions.

The reaction conditions are the same as those for drug production method 1.

(Drug starting material production method 15)

This production method is a method for producing compound (4)-1 included in compound (4) serving as an intermediate of drug starting material production method 1.

[Chemical Formula 178]

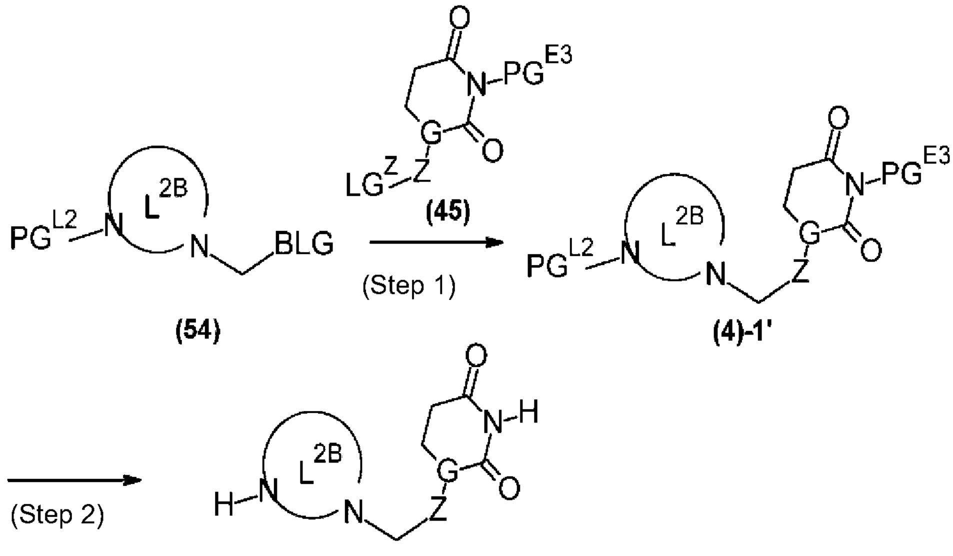


(54) (45) (4)-1' (4)-1

wherein for a compound wherein $PG^{E3}$ moiety of compound (45) is H, compound (4)-1 can be produced through similar reaction (the same holds true for the description below).

(Step 1)

This step is the step of obtaining compound (4)-1' by applying compound (54) and compound (45) to Suzuki-Miyaura coupling reaction conditions.

The reaction conditions are the same as those for step 4 of drug starting material production method 2.

(Step 2)

This step is the step of obtaining compound (4)-1 by applying (4)-1' to deprotection conditions.

The reaction conditions are the same as those for drug production method 1.

(Drug starting material production method 16)

This production method is a method for producing compound (4)-5 included in compound (4) serving as an intermediate of drug starting material production method 1.

[Chemical Formula 179]

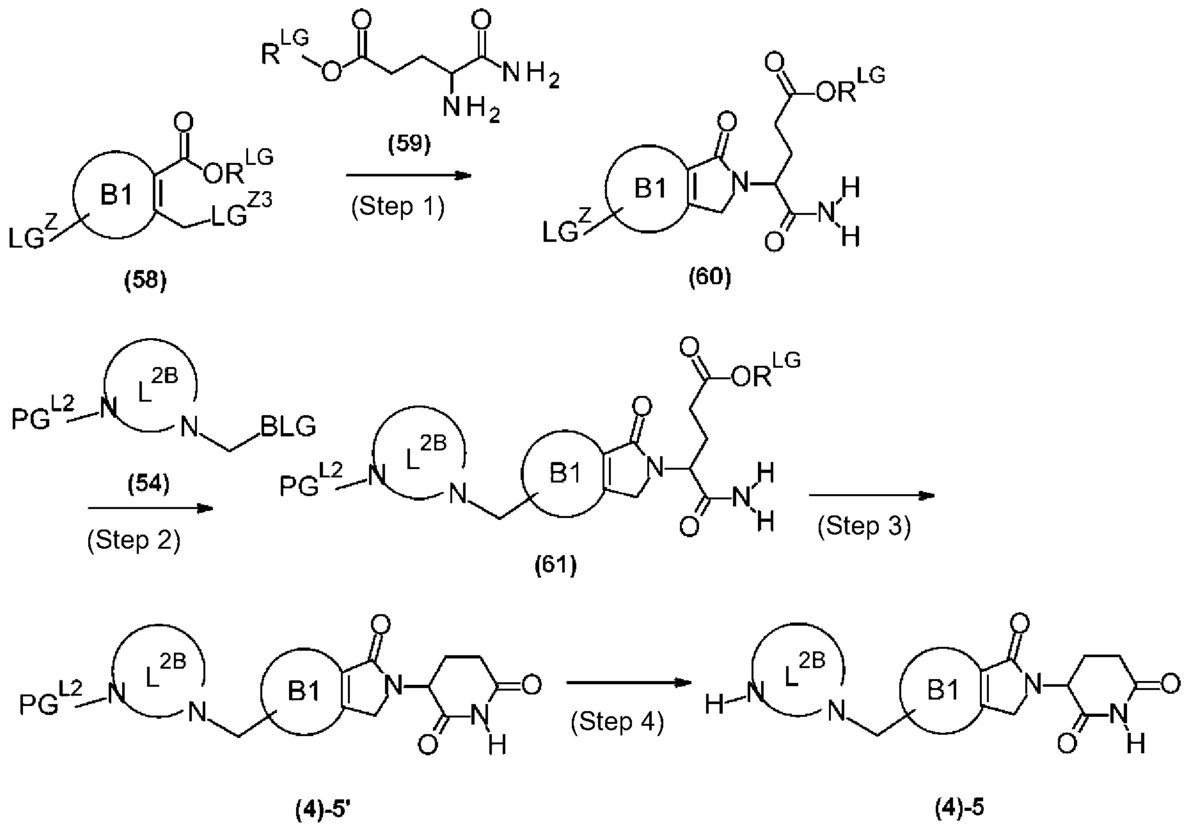


(58) (60) (61) (4)-5' (4)-5

wherein $LG^{Z3}$ represents a leaving group. In this context, examples of the leaving group include Cl, Br, a methanesulfonyloxy group, and a p-toluenesulfonyloxy group (the same holds true for the description below).

(Step 1)

This step is the step of obtaining compound (60) by applying compound (58) and compound (59) to cyclization reaction conditions.

This reaction is performed by using compound (58) and compound (59) in the same equivalents or one of them in an excessive equivalent, and stirring them under ice cooling to under heating to reflux usually for 1 hour to 5 days in a solvent inert to the reaction in the presence of a base. In this context, examples of the base used include, but are not particularly limited to, organic bases such as triethylamine and N,N'-diisopropylethylamine, and inorganic bases such as potassium carbonate and cesium carbonate. In this context, examples of the solvent used include, but are not particularly limited to, ether solvents such as tetrahydrofuran and 1,4-dioxane, and N,N-dimethylformamide.

(Step 2)

This step is the step of obtaining compound (61) by applying compound (60) and compound (54) to Suzuki-Miyaura coupling reaction conditions.

The reaction conditions are the same as those for step 4 of drug starting material production method 2.

(Step 3)

This step is the step of obtaining compound (4)-5' by applying compound (61) to cyclization reaction conditions.

The reaction conditions are the same as those for step 3 of drug starting material production method 17.

(Step 4)

This step is the step of obtaining compound (4)-5 by applying compound (4)-5' to deprotection conditions.

The reaction conditions are the same as those for drug production method 1.

(Drug starting material production method 17)

This production method is a method for producing compound (4)-6. Compound (4)-6 can be used instead of compound (4) described in drug starting material production method 1 to synthesize some compounds included in the compound of formula (1).

[Chemical Formula 180]

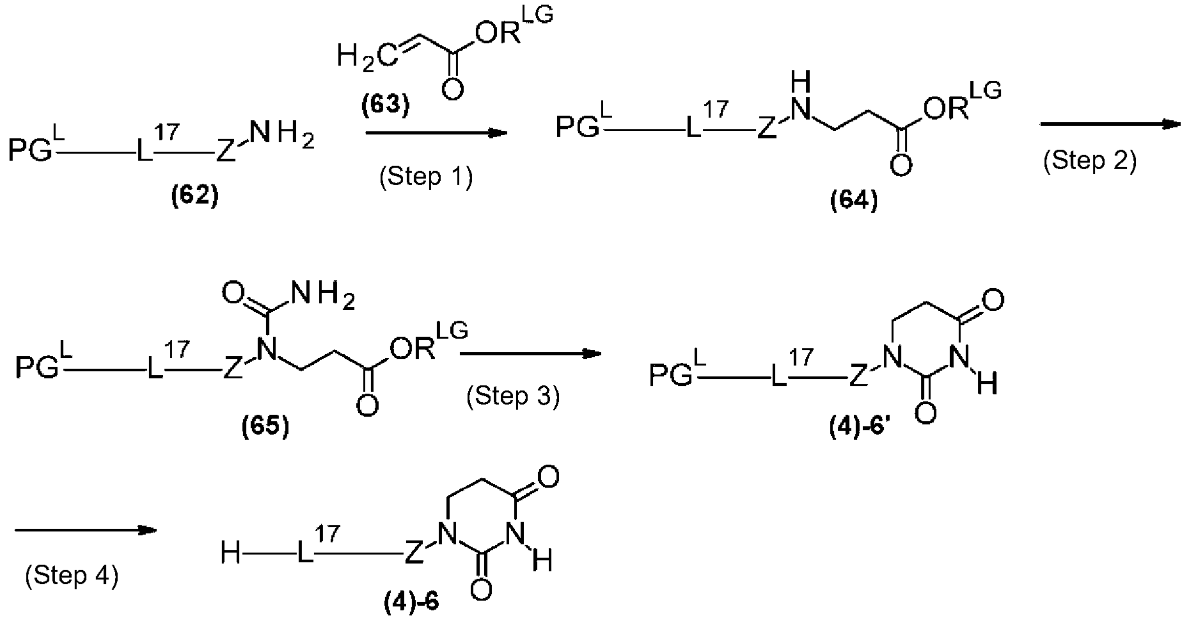


wherein $L^{17}$ represents $-(-L^2-L^3-L^4)-$, $L^2$ represents piperidinediyl optionally substituted by $C_{1-3}$ alkyl, piperazinediyl optionally substituted by $C_{1-3}$ alkyl, pyrrolidinediyl optionally substituted by $C_{1-3}$ alkyl, 3,8-diazabicyclo[3.2.1]octanediyl or 2,6-diazaspiro[3.4]octanediyl, $L^3$ represents a bond, $-N(R^{L3})-$, $C_{1-3}$ alkylene, or piperazinediyl, $L^4$ represents a bond, $-N(R^{L4})-$, -O-, piperazinediyl, or $C_{1-3}$ alkylene, $R^{L3}$ represents H or $C_{1-3}$ alkyl, $R^{L4}$ represents H or $C_{1-3}$ alkyl, $PG^L$ represents protective group for NH or OH, and $R^{LG}$ represents a $C_{1-12}$ alkyl group (the same holds true for the description below).

(Step 1)

This step is the step of obtaining compound (64) by applying compound (62) and compound (63) to Michael addition reaction conditions.

This reaction is performed by using compound (62) and compound (63) in equivalents or one of them in an excessive equivalent, and stirring them at room temperature to under heating to reflux usually for 1 day to 5 days in the presence of a base and an excessive amount of an acid reagent. In this context, examples of the base used include, but are not particularly limited to, organic bases such as 1,8-diazabicyclo[5.4.0]undec-7-ene and N,N'-diisopropylethylamine. In this context, examples of the acidic reagent used include, but are not particularly limited to, lactic acid, trifluoroacetic acid, and acetic acid.

(Step 2)

This step is the step of obtaining compound (65) by applying compound (64) to ureation reaction conditions.

This reaction is performed by stirring compound (64) at room temperature to under heating to reflux usually for 1 day to 5 days in a solvent inert to the reaction or without a solvent in the presence of a ureation reagent and an acid. In this context, examples of the ureation reagent used include, but are not particularly limited to, sodium cyanate and potassium cyanate. In this context, examples of the acid used include, but are not particularly limited to, acetic acid, hydrochloric acid, and trifluoroacetic acid. In this context, examples of the solvent used include, but are not particularly limited to, halogenated hydrocarbons such as dichloromethane, dichloroethane, and chloroform, ether solvents such as tetrahydrofuran and 1,4-dioxane, acetic acid, toluene, water, and mixtures thereof.

(Step 3)

This step is the step of obtaining compound (4)-6' by applying compound (65) to cyclization reaction conditions.

This reaction is performed by stirring compound (65) under ice cooling to under heating to reflux usually for 1 hour to 5 days in a solvent inert to the reaction in the presence of a base. In this context, examples of the base used include, but are not particularly limited to, Triton B, potassium trimethylsilanolate, and sodium ethoxide. In this context, examples of the solvent used include, but are not particularly limited to, ether solvents such as tetrahydrofuran and 1,4-dioxane, N,N-dimethylformamide, and acetonitrile.

(Step 4)

This step is the step of obtaining compound (4)-6 by applying compound (4)-6' to deprotection conditions.

The reaction conditions are the same as those for drug production method 1.

(Drug starting material production method 18)

This production method is a method for producing compound (67). Compound (67) can be used instead of compound (4) described in drug starting material production method 1 to synthesize some compounds included in the compound of formula (1).

[Chemical Formula 181]

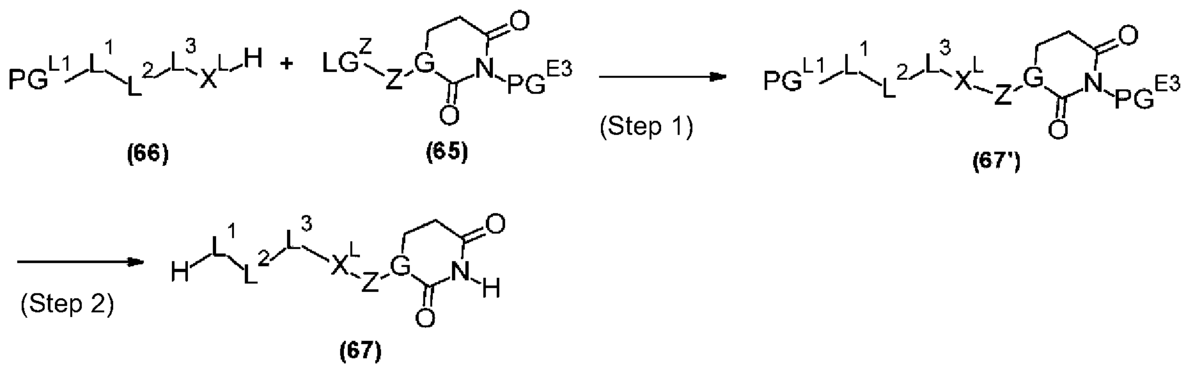

(66) (65) (67') (67)

wherein $L^1$ represents piperidinediyl optionally substituted by $C_{1-3}$ alkyl, piperazinediyl optionally substituted by $C_{1-3}$ alkyl, pyrrolidinediyl optionally substituted by $C_{1-3}$ alkyl, 3,8-diazabicyclo[3.2.1]octanediyl, or 2,6-diazaspiro[3.4]octanediyl, $L^2$ represents a bond, -$N(R^{L3})$-, $C_{1-3}$ alkylene, or piperazinediyl, $L^3$ represents a bond, -$N(R^{L4})$-, -O-, piperazinediyl, or $C_{1-3}$ alkylene, $R^{L3}$ represents H or $C_{1-3}$ alkyl, $R^{L4}$ represents H or $C_{1-3}$ alkyl, $X^L$ represents an oxygen atom or a nitrogen atom, $PG^{L1}$ represents a protective group for NH or OH, and $PG^{E3}$ represents a protective group for NH or H (the same holds true for the description below).

(Step 1)

This step is the step of obtaining compound (67') by applying compound (66) and compound (65) to ipso reaction conditions.

The reaction conditions are the same as those for step 1 of drug starting material production method 2.

Compound (67') may be produced under coupling reaction conditions using a copper catalyst.

The reaction conditions are the same as those for step 1 of drug starting material production method 12.

Compound (67') may be produced under carbon-nitrogen bond formation reaction conditions.

This reaction is performed by using compound (66) and compound (65) in the same equivalents or one of them in an excessive equivalent, adding a metal catalyst, a ligand, and a base to a mixture thereof, and stirring the resultant at 80°C to under heating to reflux usually for 1 hour to 5 days in a solvent inert to the reaction.

In this context, examples of the metal reagent catalyst used include, but are not particularly limited to, palladium(II) acetate, tris(dibenzylideneacetone)dipalladium, [1,1'-bis(diphenylphosphino)ferrocene]palladium(II) dichloride, and [1,1'-bis(diphenylphosphino)ferrocene]palladium(II) dichloride dichloromethane adducts. Examples of the ligand include, but are not particularly limited to, Xantphos, Ruphos, Xphos, and BINAP. Examples of the base include, but are not particularly limited to, inorganic bases such as potassium carbonate, sodium carbonate, cesium carbonate, and sodium tert-butoxide, and organic bases such as triethylamine and N,N-diisopropylethylamine. Examples of the

solvent include, but are not particularly limited to, 1,4-dioxane, toluene, N,N-dimethylformamide, and mixtures thereof. This reaction may be performed under microwave irradiation.

Compound (67') may be produced under alkylation reaction conditions.

The reaction conditions are the same as those for step 1 of starting material production method 2.

(Step 2)

This step is the step of obtaining compound (67) by applying compound (67') to deprotection conditions.

The reaction conditions are the same as those for drug production method 1.

(Drug starting material production method 19)

This production method is a method for producing compound (1)-2 included in compound (1) serving as a starting material of drug production method 1.

[Chemical Formula 182]

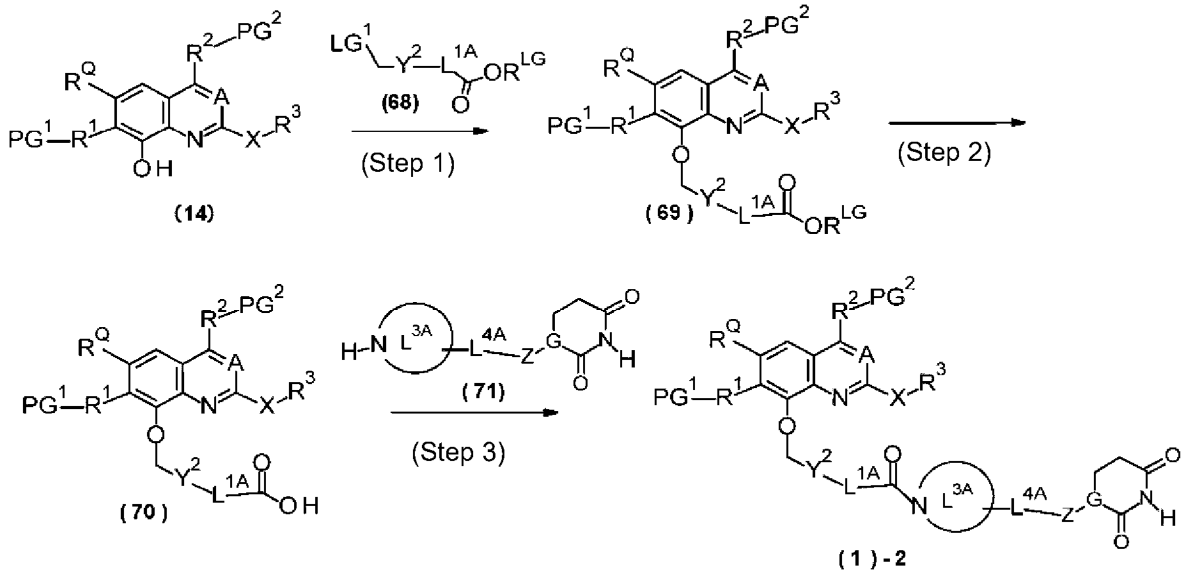


wherein $L^{1A}$ and $L^{4A}$ each represent one group selected from the group consisting of optionally substituted hetero cycloalkylene, optionally substituted heteroarylene, saturated 7-to 9-membered spiro hetero cycloalkylene containing 1 or 2 nitrogen atoms, saturated 7- to 9-membered bridged hetero cycloalkylene containing 2 nitrogen atoms, and optionally substituted $C_{1-6}$ alkylene, and $L^{3A}$ represents piperidinediyl optionally substituted by $C_{1-3}$ alkyl, piperazinediyl optionally substituted by $C_{1-3}$ alkyl, pyrrolidinediyl optionally substituted by $C_{1-3}$ alkyl, bridged piperazinediyl, or 2,6-diazaspiro[3.4] octanediyl (the same holds true for the description below).

(Step 1)

This step is a method for producing compound (69) by applying compound (14) and compound (68) to alkylation reaction.

The reaction conditions are the same as those for step 7 of drug starting material production method 2.

(Step 2)

This step is the step of obtaining compound (70) by applying compound (69) to hydrolysis conditions.

The reaction conditions are the same as those for step 1 of drug starting material production method 1.

(Step 3)

This step is the step of obtaining compound (1)-2 by applying compound (70) and compound (71) to condensation reaction conditions.

The reaction conditions are the same as those for step 2 of drug starting material production method 1.

(Drug starting material production method 20)

This production method is a method for producing compound (74). Compound (74) can be used instead of compound (4) described in drug starting material production method 1 to synthesize some compounds included in the compound of formula (1).

[Chemical Formula 183]

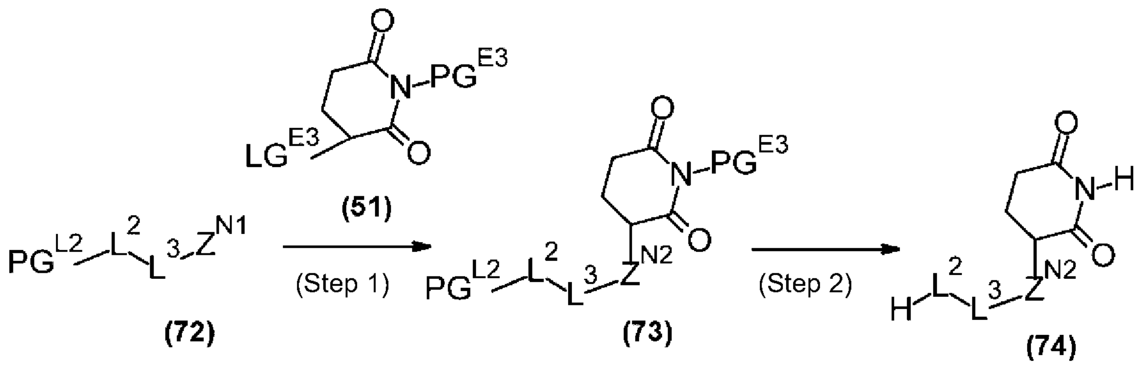


wherein for a compound wherein $PG^{E3}$ moiety of compound (51) is H, compound (74) can be produced through similar reaction.

(Step 1)

This step is the step of obtaining compound (73) by applying compound (72) and compound (51) to nucleophilic substitution reaction conditions.

The reaction conditions are the same as those for step 1 of drug starting material production method 11.

(Step 2)

This step is the step of obtaining compound (74) by applying compound (73) to deprotection conditions.

The reaction conditions are the same as those for drug production method 1.

(Drug starting material production method 21)

[Chemical Formula 184]

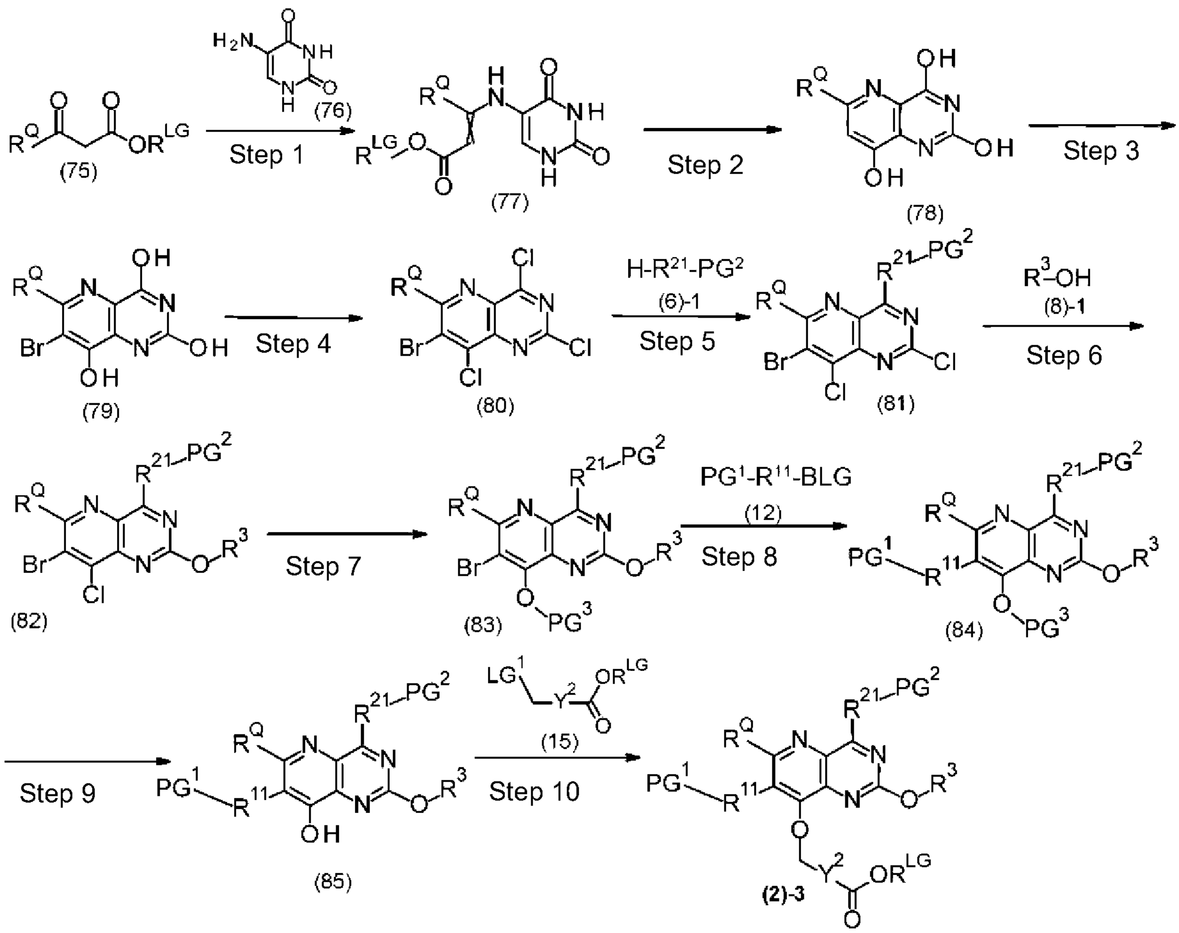


This production method is a method for producing compound (2)-3 included in (2) serving as starting material of drug starting material production method 1.

(Step 1)

This step is a method for producing compound (77) through reaction of compound (75) and compound (76).

This reaction is performed by using compound (75) and ortho ester such as trimethyl orthoformate for conversion to corresponding enolate under acidic conditions, then adding compound (76) in the same amounts or one of them in an excessive amount, and stirring a mixture thereof under heating to reflux, preferably at 60°C to under heating to reflux, usually for 0.1 hours to 5 days in a solvent inert to the reaction. In this context, examples of the solvent used include, but are not particularly limited to, aromatic hydrocarbons such as toluene, ethers such as THF and DOX, DMF, and DMAc.

(Step 2)

This step is a method for producing compound (78) from compound (77).

This reaction is performed by stirring compound (77) under heating to reflux, preferably at 150°C to under heating to reflux, usually for 0.1 hours to 5 days in a solvent inert to the reaction. In this context, examples of the solvent used include, but are not particularly limited to, NMP.

(Step 3)

This step is a method for producing compound (79) from compound (78).

This reaction is performed by using compound (78) and a brominating agent in equal amounts or one of them in an excessive amount, and stirring a mixture thereof under cooling to under heating to reflux, preferably at room temperature, usually for 0.1 hours to 5 days in a solvent inert to the reaction or without a solvent. In this context, examples of the solvent used include, but are not particularly limited to, aromatic hydrocarbons such as toluene, ethers such as THF and DOX,

halogenated hydrocarbons such as dichloromethane, and DMF. Examples of the brominating agent include N-bromosuccinimide, N-bromosaccharin, 1,3-dibromo-5,5-dimethylhydantoin, and dibromoisocyanuric acid.

(Step 4)

This step is a method for producing compound (80) from compound (79).
The reaction conditions are the same as those for step 1 of drug starting material production method 8.

(Step 5)

This step is a method for producing compound (81) through ipso-substitution reaction of compound (80) and compound (6)-1.
The reaction conditions are the same as those for step 1 of drug starting material production method 2.

(Step 6)

This step is a method for producing compound (82) through ipso-substitution reaction of compound (81) and compound (8)-1.
The reaction conditions are the same as those for step 1 of drug starting material production method 2.

(Step 7)

This step is a method for producing compound (82) through ipso-substitution reaction of compound (82) and $PG^3$-OH.
In this context, examples of $PG^3$-OH used include benzyl alcohol, p-methoxybenzyl alcohol, and 1-phenylethanol.
The reaction conditions are the same as those for step 1 of drug starting material production method 2.

(Step 8)

This step is a method for producing compound (84) through Suzuki-Miyaura coupling reaction of compound (83) and boronic acid derivative compound (12).
The reaction conditions are the same as those for step 4 of drug starting material production method 2.
Compound (84), when having axial chirality, can be obtained as a mixture of stereoisomers. Each stereoisomer can be isolated by usual resolution operation, for example, resolution using ODS column chromatography or silica gel column chromatography.
Compound (84) may be subjected to deprotection reaction and then the conversion of $PG^1$ to another protective group such that it can be deprotected under conditions different from those for protective group $PG^2$ to be introduced later.
In this context, the reaction conditions of the deprotection reaction used are the same as those for the step described in drug starting material production method 1.
Examples of protective group $PG^1$ to be subsequently converted include a tetrahydro-2H-pyran-2-yl group.
For a reference document of this reaction, see the following, for example. P. G. M. Wuts and T. W. Greene, "Greene's Protective Groups in Organic Synthesis", fifth edition, John Wiley & Sons Inc., 2014

(Step 9)

This step is a method for producing compound (85) by deprotecting compound (84) through catalytic hydrogenation reaction.
The reaction conditions are the same as those for step 6 of drug starting material production method 2.

(Step 10)

This step is a method for producing compound (2)-3 through reaction of compound (85) and compound (15).
The reaction conditions are the same as those for step 7 of drug starting material production method 2.

(Drug starting material production method 22)

[Chemical Formula 185]

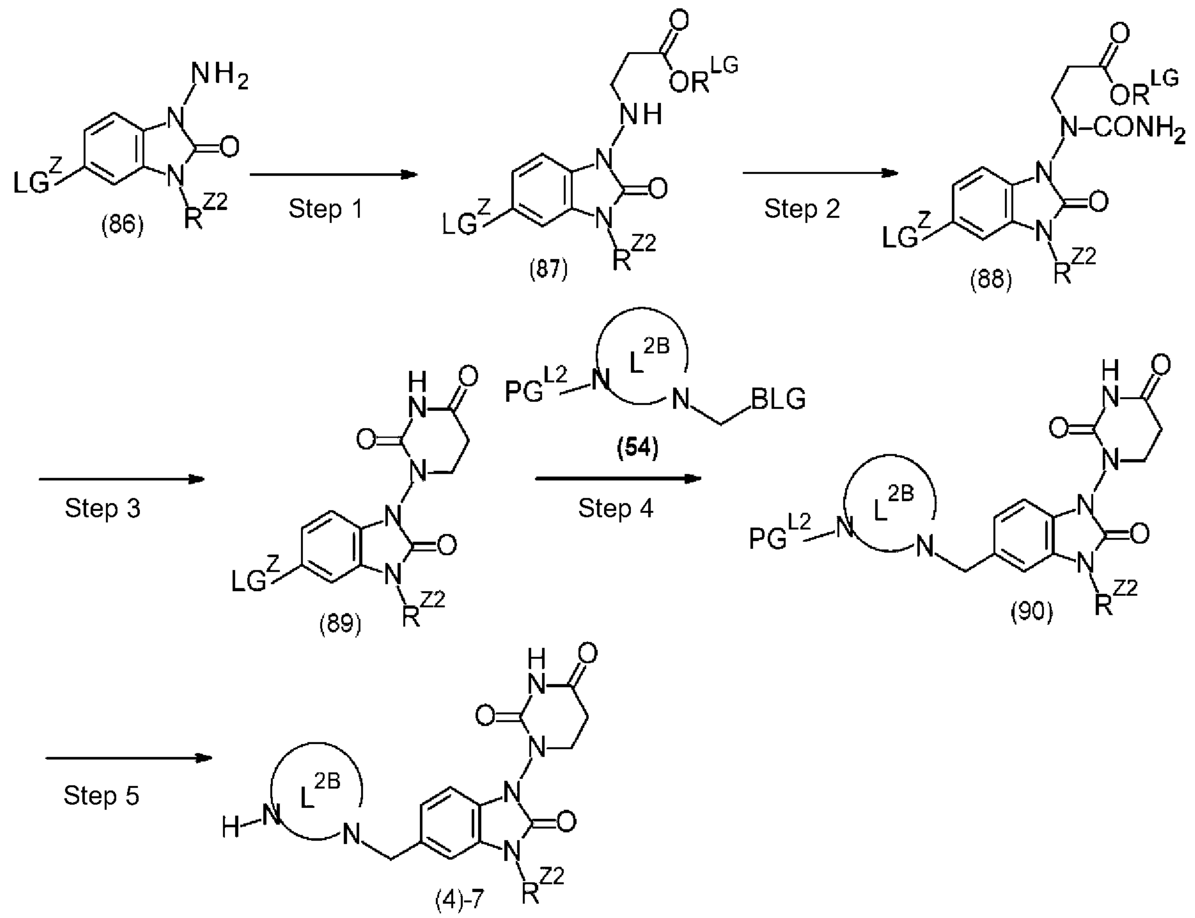


This production method is a method for producing compound (4)-7 included in compound (4) serving as an intermediate of drug starting material production method 1.

(Step 1)

This step is a method for producing compound (87) from compound (86).

The reaction conditions are the same as those for step 1 of drug starting material production method 17.

(Step 2)

This step is a method for producing compound (88) from compound (87).

This reaction is performed by adding an ammonia reagent at room temperature in a solvent inert to the reaction in the presence of compound (87), triphosgene, and a base, and stirring the resultant usually for 1 day to 5 days. In this context, examples of the ammonia reagent used include, but are not particularly limited to, an ammonia methanol solution. In this context, examples of the base used include, but are not particularly limited to, organic bases such as pyridine, triethylamine, and N,N-diisopropylethylamine. In this context, examples of the solvent used include, but are not particularly limited to, halogenated hydrocarbons such as dichloromethane, dichloroethane, and chloroform.

(Step 3)

This step is a method for producing compound (89) from compound (88).

The reaction conditions are the same as those for step 3 of drug starting material production method 17.

(Step 4)

This step is the step of obtaining compound (90) by applying compound (89) and compound (54) to Suzuki-Miyaura coupling reaction conditions.

The reaction conditions are the same as those for step 4 of drug starting material production method 2.

(Step 5)

This step is the step of obtaining compound (4)-7 by applying compound (90) to deprotection conditions.

The reaction conditions are the same as those for drug production method 1.

(Drug starting material production method 23)

This production method is a method for producing compound (45)-3 included in compound (45) serving as a starting material of drug starting material production method 10.

[Chemical Formula 186]

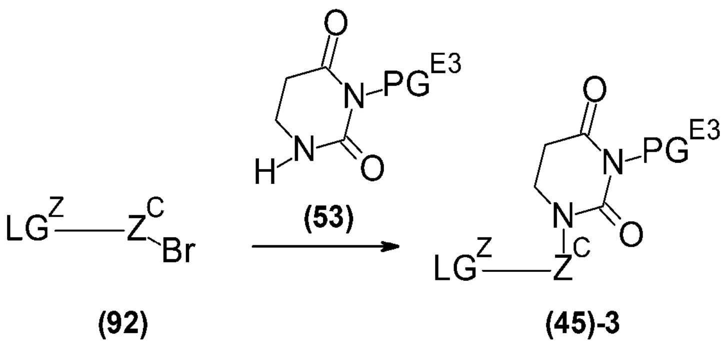

This step is the step of obtaining compound (45)-3 by applying compound (92) and compound (53) to coupling reaction conditions using copper catalyst.

The reaction conditions are the same as those for step 1 of drug starting material production method 12.

(Drug starting material production method 24)

[Chemical Formula 187]

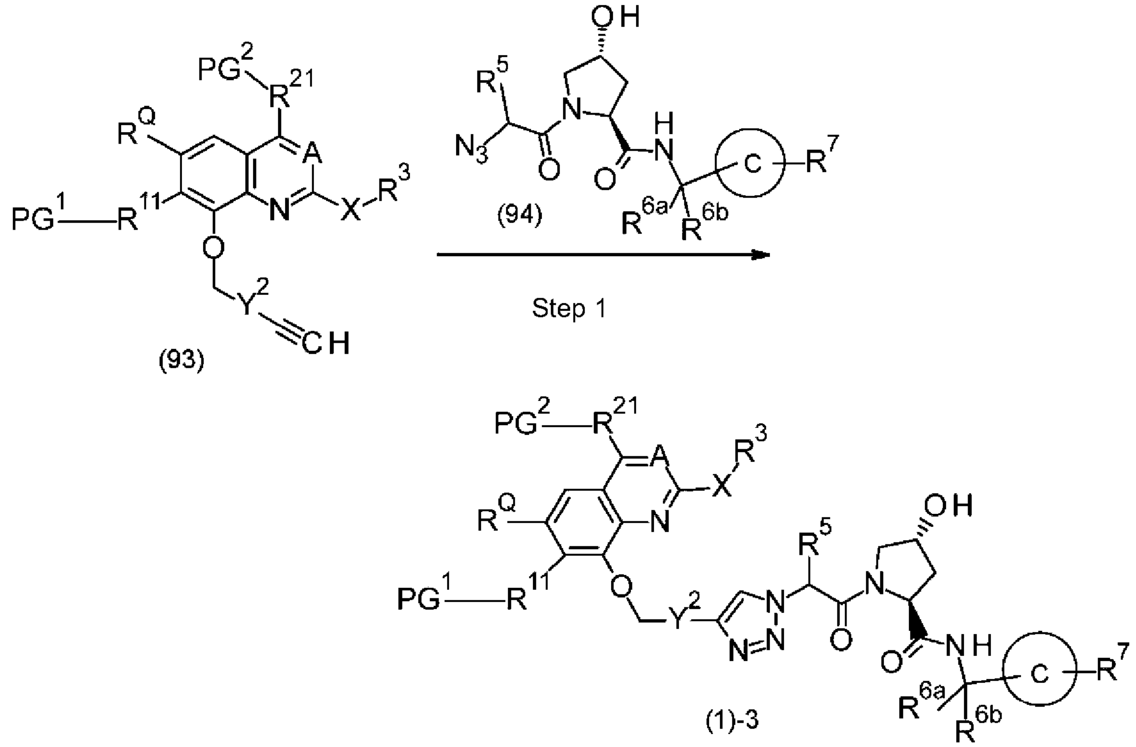


This production method is the first method for producing compound (1)-3 included in starting material compound (1).

(Step 1)

This step is a method for producing compound (1)-3 through cycloaddition reaction of compound (93) and compound (94).

This reaction involves using compound (93) and compound (94) in equal amounts or one of them in an excessive amount, and stirring a mixture thereof under cooling to under heating to reflux, preferably at 0°C to 100°C, usually for 0.1 hours to 5 days in a solvent inert to the reaction or without a solvent, preferably in the presence of a copper salt, further preferably in the presence of a copper salt and a reducing agent. In this context, examples of the solvent used include, but are not particularly limited to, halogenated hydrocarbons such as dichloromethane, 1,2-dichloroethane, and chloroform, aromatic hydrocarbons such as benzene, toluene, and xylene, ethers such as diethyl ether, THF, DOX, and 1,2-dimethoxyethane, DMF, DMSO, ethyl acetate, MeCN, tBuOH, water, and mixtures thereof. Examples of the copper salt include CuI, $CuSO_4$, and copper(I) trifluoromethanesulfonate (CuOTf). Examples of the reducing agent include sodium ascorbate. Reaction in the presence of TEA, DIPEA, N-methylmorpholine (NMM), 2,6-lutidine, tris[(1-benzyl-1H-1,2,3-triazol-4-yl)methyl]amine (TBTA), or the like may be advantageous for allowing the reaction to proceed smoothly.

[Reference]

Angew. Chem. Int. Ed. 2002, 41, p. 2596-2599.

This reaction may be performed using a compound obtained by initially subjecting $PG^1$ of compound (93) to deprotection reaction.

(Drug starting material production method 25)

[Chemical Formula 188]

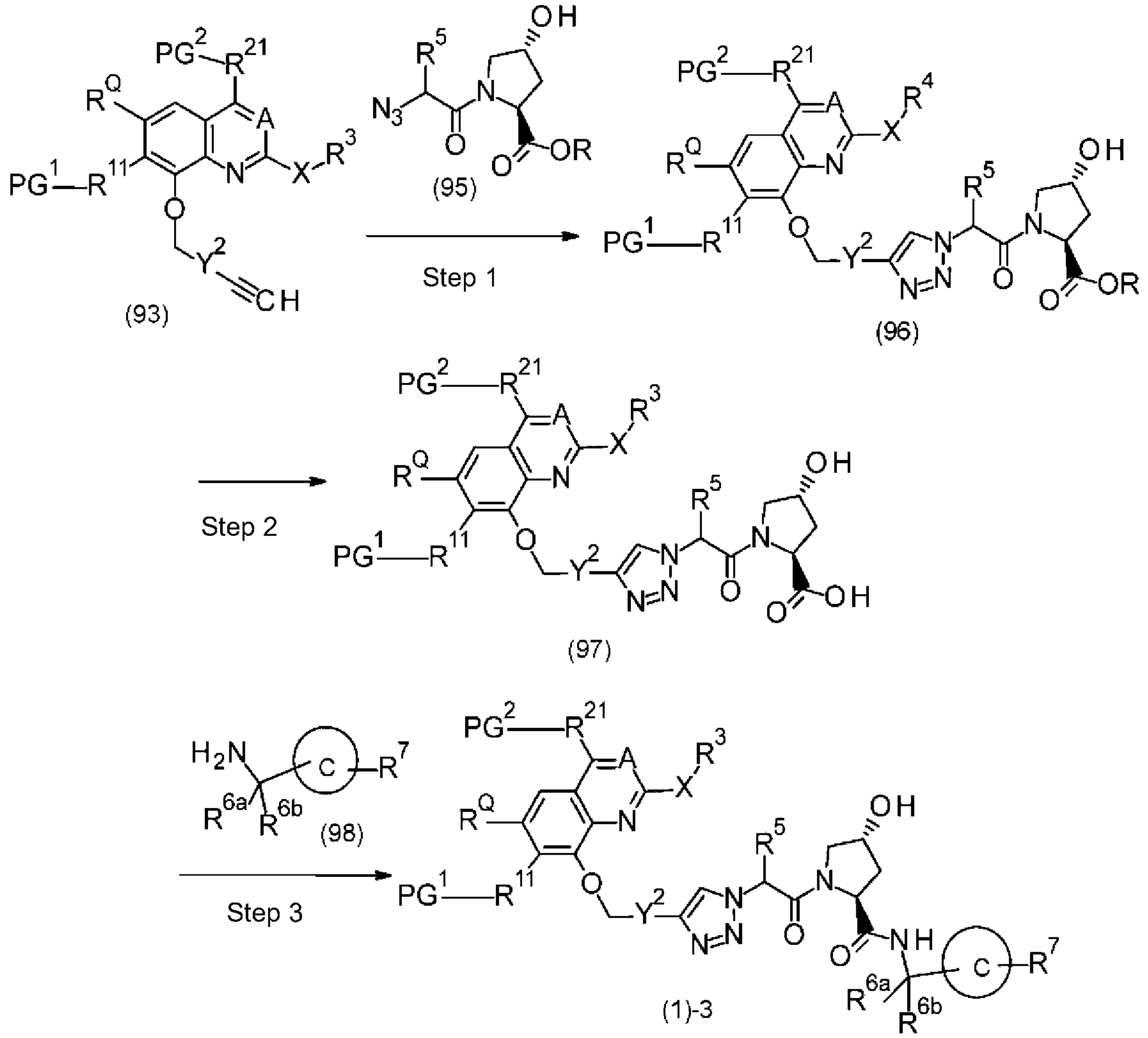


wherein R represents a $C_{1-3}$ alkyl group (the same holds true for the description below).

This production method is the second method for producing compound (1)-3 included in starting material compound (1).

(Step 1)

This step is a method for producing compound (96) through cycloaddition reaction of compound (93) and compound (95).

The reaction conditions are the same as those for step 1 of drug starting material production method 24.

(Step 2)

This step is a method for producing compound (97) by hydrolyzing compound (96).

This reaction is performed by stirring compound (96) under cooling to under heating to reflux usually for 0.1 hours to 5 days. In this context, examples of the solvent used include, but are not particularly limited to, alcohols, acetone, DMF, and THF. A mixed solvent of the solvent described above with water may be suitable for the reaction. Examples of the hydrolysis reagent include, but are not particularly limited to, an aqueous sodium hydroxide solution, an aqueous potassium hydroxide solution, and trimethyl tin hydroxide.

For a reference document of this reaction, see the following, for example. The Chemical Society of Japan ed., "Jikken Kagaku Koza (Courses in Experimental Chemistry in English) (fifth edition)", vol. 16 (2005) (Maruzen Co., Ltd.) Angew. Chem. Int. Ed. 2005, 44, p. 1378-1382

(Step 3)

This step is a method for producing compound (1)-3 through amidation reaction of compound (97) and compound (98).

This reaction involves using compound (97) and compound (98) in equal amounts or one of them in an excessive amount, and stirring a mixture thereof under cooling to under heating, preferably at -20°C to 60°C, usually for 0.1 hours to 5 days in a solvent inert to the reaction in the presence of a condensing agent. Examples of the solvent include, but are not particularly limited to, aromatic hydrocarbons such as toluene, ethers such as THF and DOX, halogenated hydrocarbons such as dichloromethane, alcohols, DMF, DMSO, ethyl acetate, MeCN, and mixtures thereof. Examples of the condensing agent include (benzotriazol-1-yloxy)tripyrrolidinophosphoniuim hexafluorophosphate (PyBOP), O-(7-azabenzotriazol-1-yl)-N,N,N',N'-tetramethyluronium hexafluorophosphate (HATU), 1-(3-dimethylaminopropyl)-3-ethylcarbodiimide and hydrochloride thereof, N,N'-dicyclohexylcarbodiimide (DCC), 1,1'-carbonyldiimidazole (CDI), and diphenylphosphorylazide (DPPA). It may be preferred for the reaction to use an additive (e.g., 1-hydroxybenzotriazole). The reaction in the presence of an organic base such as TEA, DIPEA, or NMM, or an inorganic base such as potassium carbonate, sodium carbonate, or potassium hydroxide may be advantageous for allowing the reaction to proceed smoothly.

A method of converting compound (97) to a reactive derivative, followed by acylation reaction may be used. Examples of the reactive derivative of carboxylic acid include acid halide obtained through reaction with a halogenating agent such as phosphorus oxychloride or thionyl chloride, mixed acid anhydride obtained through reaction with isobutyl chloroformate or the like, and active ester obtained by condensation with 1-hydroxybenzotriazole or the like. The reaction of compound (98) with such a reactive derivative can be performed under cooling to under heating, preferably at -20°C to 120°C, in a solvent inert to the reaction, such as a halogenated hydrocarbon, an aromatic hydrocarbon, or an ether.

[Reference]

S. R. Sandler and W. Karo, "Organic Functional Group Preparations", second edition, vol. 1, Academic Press Inc., 1991

The Chemical Society of Japan ed., "Jikken Kagaku Koza (Courses in Experimental Chemistry in English) (fifth edition)", vol. 16 (2005) (Maruzen Co., Ltd.)

(Drug starting material production method 26)

[Chemical Formula 189]

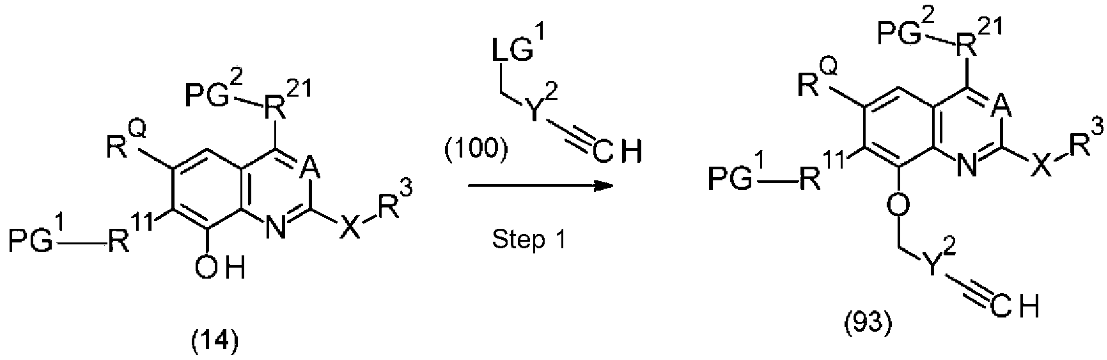


(14)

(93)

This production method is the first method for producing starting material compound (93).

This production method is a method for producing compound (93) through reaction of compound (14) and compound (100).

This reaction is performed by using compound (14) and compound (100) in equal amounts or one of them in an excessive amount, and reacting a mixture thereof under cooling to under heating to reflux, preferably at 0°C to 80°C, usually for 0.1 hours to 5 days in a solvent inert to the reaction in the presence of a base. In this context, examples of the solvent used include, but are not particularly limited to, aromatic hydrocarbons such as benzene, toluene, and xylene, alcohols such as MeOH and EtOH, ethers such as diethyl ether, THF, DOX, and 1,2-dimethoxyethane, halogenated hydrocarbons such as dichloromethane, 1,2-dichloroethane, and chloroform, DMF, DMSO, ethyl acetate, MeCN, and

mixtures thereof. Examples of the base include, but are not particularly limited to, organic bases such as TEA, DIPEA, 1,8-diazabicyclo[5.4.0]-7-undecene, n-butyllithium, and tBuOK, and inorganic bases such as sodium hydroxide, sodium carbonate, potassium carbonate, cesium carbonate, and sodium hydride. The reaction in the presence of a phase transfer catalyst such as tetra-n-butylammonium chloride may be advantageous.

For a reference document of this reaction, see the following, for example. The Chemical Society of Japan ed., "Jikken Kagaku Koza (Courses in Experimental Chemistry in English)", fifth edition, vol. 14, Maruzen Co., Ltd., 2005

Compound (93) may have axial chirality and can be obtained as a mixture of stereoisomers. Each stereoisomer can be isolated by usual resolution operation, for example, resolution using ODS column chromatography or silica gel column chromatography, of compound (93) wherein $PG^2$ is a protective group or a compound obtained by deprotection reaction of compound (93).

In this context, the reaction conditions of the deprotection reaction used are the same as those for the step described in drug production method 1.

Compound (100) can be produced as a compound having halogen as $LG^1$ by halogenating a compound having a hydroxy group as a moiety corresponding to $LG^1$. In this context, examples of the halogenating agent used include, but are not particularly limited to, thionyl chloride, phosphorus oxychloride, hydrobromic acid, and phosphorus tribromide.

For a reference document of this reaction, see the following, for example. The Chemical Society of Japan ed., "Jikken Kagaku Koza (Courses in Experimental Chemistry in English)", fifth edition, vol. 13, Maruzen Co., Ltd., 2004

Compound (100) can be produced as a compound having a sulfonyloxy group as $LG^1$ by sulfonylating a compound having a hydroxy group as a moiety corresponding to $LG^1$ in the presence of a base. In this context, examples of the sulfonylation reagent used include, but are not particularly limited to, methanesulfonyl chloride, p-toluenesulfonyl chloride, and methanesulfonic anhydride. Examples of the base include, but are not particularly limited to, TEA, DIPEA, pyridine, and tetramethylethylenediamine.

For a reference document of this reaction, see the following, for example. Synthesis 1999, 9, p. 1633-1636

(Drug starting material production method 27)

[Chemical Formula 190]

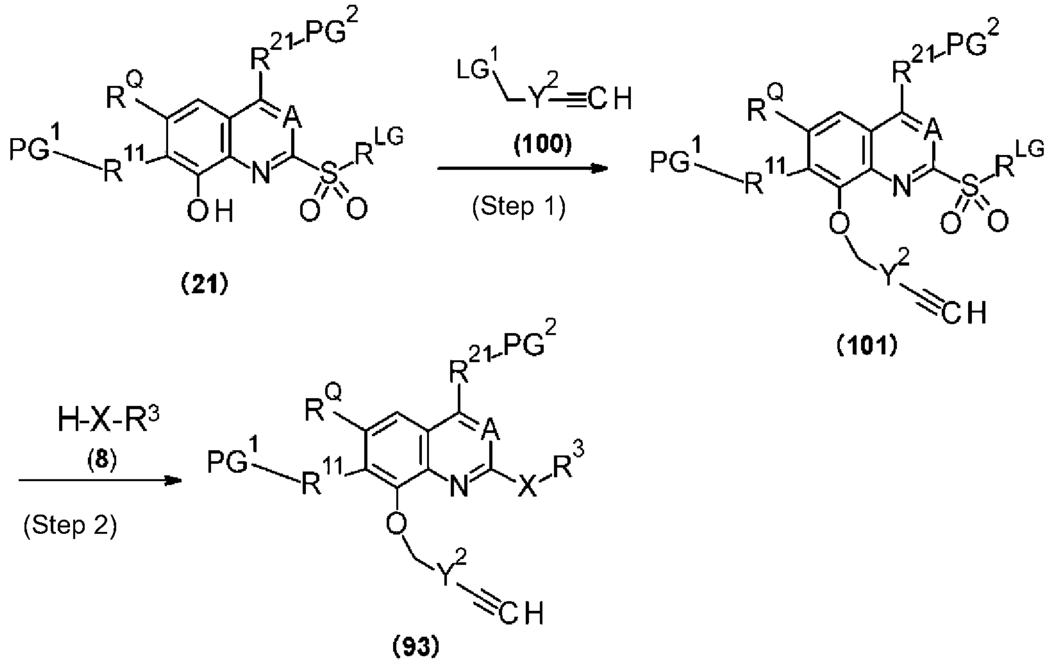


This production method is the second method for producing starting material compound (93).

(Step 1)

This step is a method for producing compound (101) through reaction of compound (21) and compound (100).

The reaction conditions are the same as those for drug starting material production method 26.

(Step 2)

This step is a method for producing compound (93) through ipso-substitution reaction of compound (101) and compound (8).
The reaction conditions are the same as those for step 1 of drug starting material production method 2.
Compound (93) may have axial chirality and can be obtained as a mixture of stereoisomers. Each stereoisomer can be isolated by usual resolution operation, for example, resolution using ODS column chromatography or silica gel column chromatography, of compound (93) wherein $PG^2$ is a protective group or a compound obtained by deprotection reaction of compound (93).
In this context, the reaction conditions of the deprotection reaction used are the same as those for the step described in drug production method 1.

(Drug starting material production method 28)

[Chemical Formula 191]

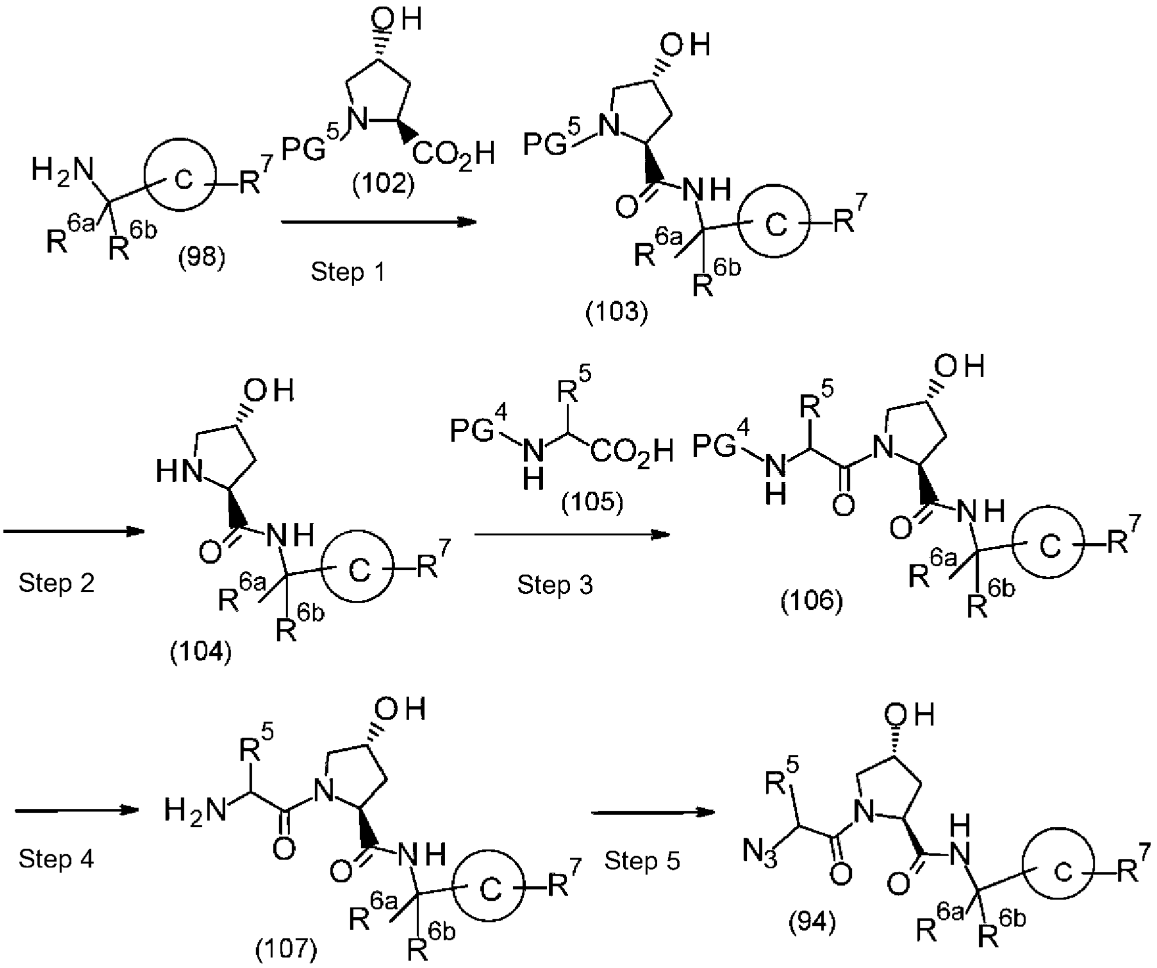

(98) (102) (103) (104) (105) (106) (107) (94)

wherein $PG^4$ and $PG^5$ each represent a protective group.
This production method is a method for producing starting material compound (94).

(Step 1)

This step is a method for producing compound (103) through amidation reaction of compound (98) and compound (102).
The reaction conditions are the same as those for step 3 of drug starting material production method 25.

(Step 2)

This step is a method for producing compound (104) by subjecting compound (103) to deprotection reaction.
The reaction conditions are the same as those for the step described in drug production method 1.

(Step 3)

This step is a method for producing compound (31) through amidation reaction of compound (104) and compound (105).
The reaction conditions are the same as those for step 3 of drug starting material production method 25.

(Step 4)

This step is a method for producing compound (107) by subjecting compound (106) to deprotection reaction.
The reaction conditions are the same as those for the step described in drug production method 1.

(Step 5)

This step is a method for producing compound (94) through reaction of compound (107) and a diazo transfer reagent.
This reaction involves treating compound (107) with an equal amount or an excessive amount of a diazo transfer reagent under cooling to under heating, preferably at 0°C to 50°C, usually for 0.1 hours to 3 days in a solvent inert to the reaction. Examples of the diazo transfer reagent include, but are not particularly limited to, trifluoromethanesulfonyl azide, imidazole-1-sulfonyl azide and salts thereof, and 2-azido-1,3-dimethylimidazolinium hexafluorophosphate (ADMP). The reaction in the presence of an organic base such as TEA, 4-dimethylaminopyridine (DMAP), or 2,6-lutidine or a catalytic amount of a copper salt such as $CuSO_4$ may be advantageous. Examples of the solvent include THF, halogenated hydrocarbons such as dichloromethane, MeCN, alcohols, water, and mixtures thereof.

[Reference]

J. Org. Chem. 2012, 77, p. 1760-1764
Nature 2019, 574, p. 86-89
Org. Biomol. Chem. 2014, 12, p. 4397-4406

(Drug starting material production method 29)

[Chemical Formula 192-1]

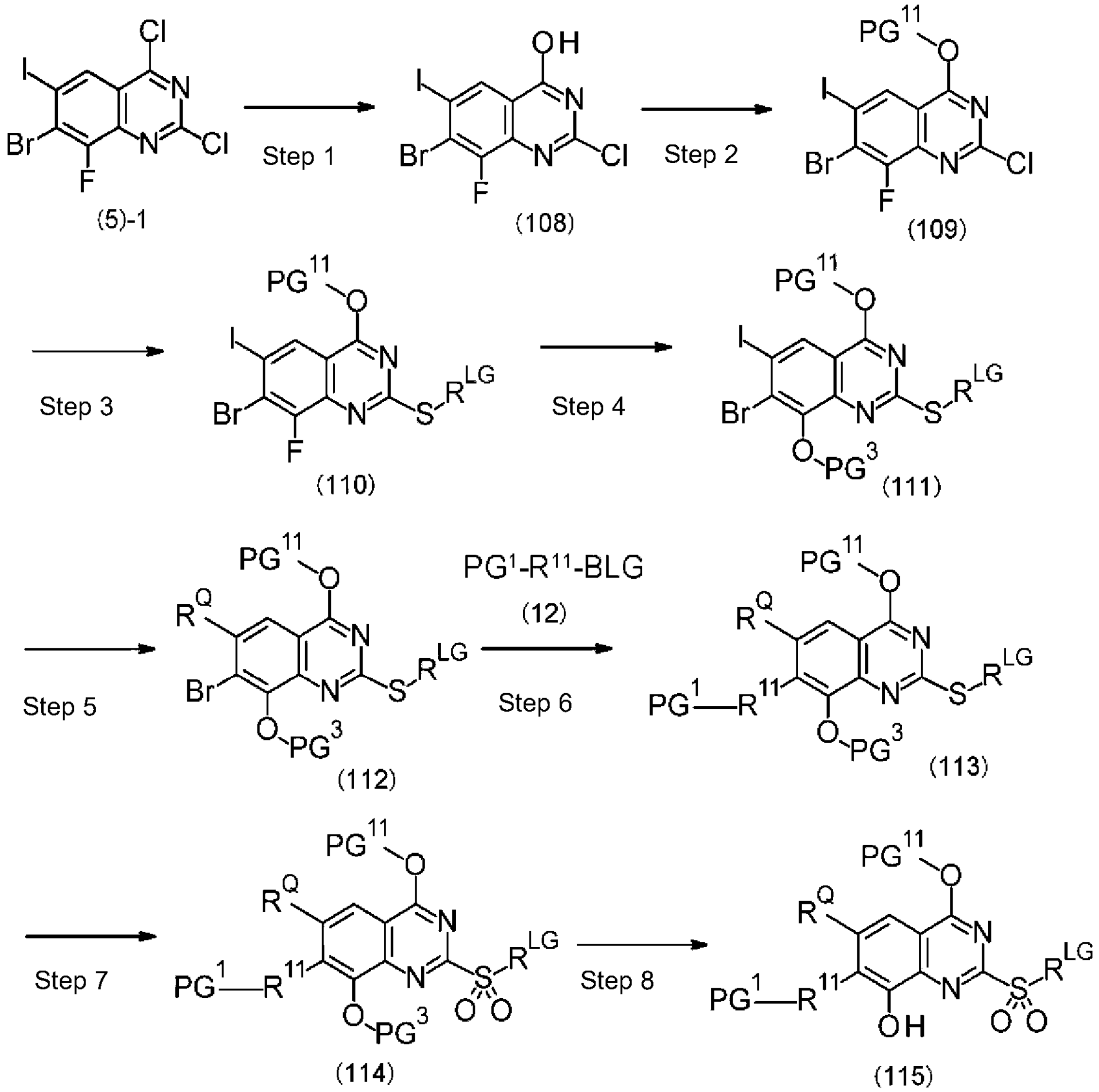


(5)-1 (108) (109) (110) (111) (112) (113) (114) (115)

[Chemical Formula 192-2]

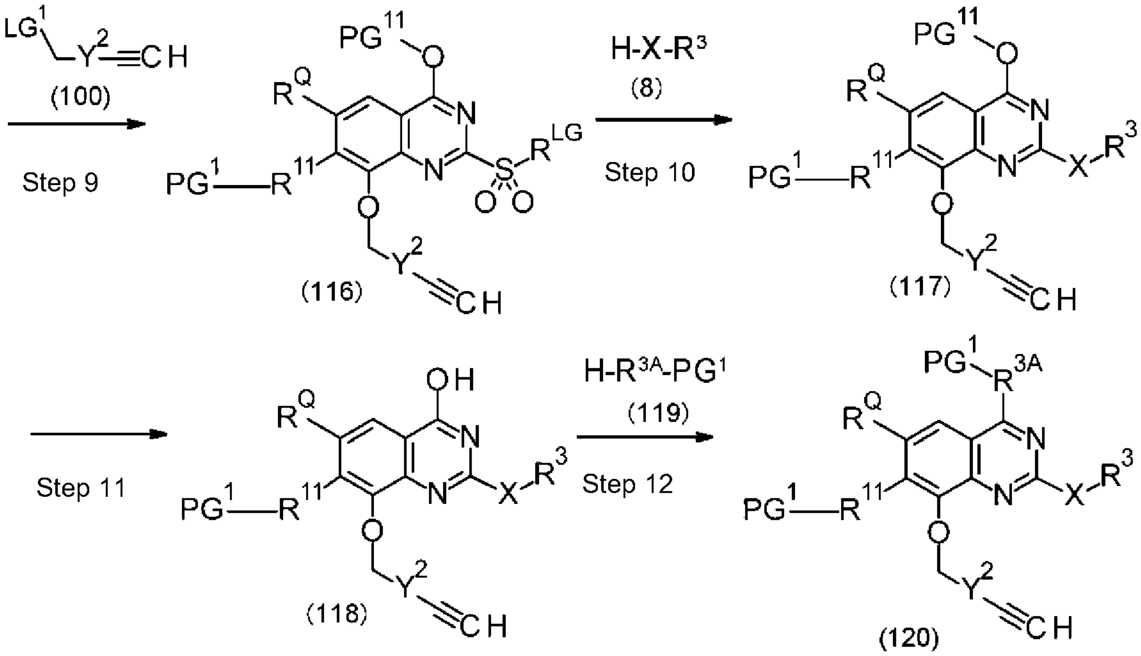


wherein $PG^{11}$ represents a tert-butyl group, and $R^{3A}$ represents -O- or -N($R^P$)-.

This production method is a method for producing starting material compound (120).

(Step 1)

This step is a method for producing compound (108) by hydrolyzing compound (5)-1.

This reaction is performed by stirring compound (5)-1 under cooling to under heating to reflux usually for 0.1 hours to 5 days. In this context, examples of the solvent used include, but are not particularly limited to, alcohols, acetone, DMF, and THF. A mixed solvent of the solvent described above with water may be suitable for the reaction. Examples of the hydrolysis reagent include, but are not particularly limited to, an aqueous sodium hydroxide solution and an aqueous potassium hydroxide solution.

For a reference document of this reaction, see the following, for example. The Chemical Society of Japan ed., "Jikken Kagaku Koza (Courses in Experimental Chemistry in English) (fifth edition)", vol. 16 (2005) (Maruzen Co., Ltd.) Angew. Chem. Int. Ed. 2005, 44, p. 1378-1382.

(Step 2)

This step is a method for producing compound (109) by protecting a hydroxy group of compound (108) with a tert-butyl group ($PG^{11}$).

This reaction is performed by stirring compound (108) under cooling to under heating to reflux usually for 0.1 hours to 5 days. In this context, examples of the solvent used include, but are not particularly limited to, ethers such as THF and DOX, halogenated hydrocarbons such as dichloromethane, tBuOH, and DMF. Examples of the tert-butyl protection reagent include, but are not particularly limited to, isobutene and 2-tert-butyl-1,3-diisopropylisourea.

Compound (109) may be produced through dehydration condensation reaction of compound (108) and tBuOH.

For a reference document of this reaction, see the following, for example. P. G. M. Wuts and T. W. Greene, "Greene's Protective Groups in Organic Synthesis", fifth edition, John Wiley & Sons Inc., 2014
Org. Lett., 2012, 14, 17, p. 4678-4681

(Step 3)

This step is a method for producing compound (110) through ipso-substitution reaction of compound (109) and $R^{LG}$-SH.

The reaction conditions are the same as those for step 1 of drug starting material production method 2.

(Step 4)

This step is a method for producing compound (111) through ipso-substitution reaction of compound (110) and $PG^3$-OH.
The reaction conditions are the same as those for step 1 of drug starting material production method 2.

(Step 5)

This step is a method for producing compound (112) through Suzuki-Miyaura coupling reaction of compound (111) and a boronic acid derivative consisting of $R^Q$-boronic acid group, etc.
The reaction conditions are the same as those for step 4 of drug starting material production method 2.
Compound (112) (wherein $R^Q$ is hydrogen) may be produced through dehalogenation reaction of compound (111) using a Pd catalyst and a reducing agent.

[Reference]

J. Org. Chem., 1977, 42, p. 3491-3494
Tetrahedron Letters 2013, 54, 5207-5210

(Step 6)

This step is a method for producing compound (113) through Suzuki-Miyaura coupling reaction of compound (112) and compound (12).
The reaction conditions are the same as those for step 4 of drug starting material production method 2.

(Step 7)

This step is a method for producing compound (114) through oxidation reaction of compound (113).
The reaction conditions are the same as those for step 5 of drug starting material production method 3.
Compound (114), when having axial chirality, may be obtained as a mixture of stereoisomers. Each stereoisomer can be isolated by usual resolution operation, for example, resolution using ODS column chromatography or silica gel column chromatography.
Compound (114) may be subjected to deprotection reaction and then the conversion of $PG^2$ to another protective group such that it can be deprotected under conditions different from those for protective group $PG^1$ to be introduced later.
In this context, the reaction conditions of the deprotection reaction used are the same as those for the step described in drug production method 1.
Examples of protective group $PG^2$ to be subsequently converted include a tetrahydro-2H-pyran-2-yl group.
For a reference document of this reaction, see the following, for example. P. G. M. Wuts and T. W. Greene, "Greene's Protective Groups in Organic Synthesis", fifth edition, John Wiley & Sons Inc., 2014

(Step 8)

This step is a method for producing compound (115) by deprotecting compound (114) through catalytic hydrogenation reaction.
The reaction conditions are the same as those for step 6 of drug starting material production method 2.

(Step 9)

This step is a method for producing compound (116) through reaction of compound (115) and compound (100).
The reaction conditions are the same as those for step 1 of drug starting material production method 26.

(Step 10)

This step is a method for producing compound (117) through ipso-substitution reaction of compound (116) and compound (8).
The reaction conditions are the same as those for step 1 of drug starting material production method 2.

(Step 11)

This step is a method for producing compound (118) by subjecting compound (117) to deprotection reaction.

The reaction conditions are the same as those for the step described in drug production method 1.

(Step 12)

This step is a method for producing compound (120) through reaction of compound (118) and compound (119).

This reaction involves using compound (118) and compound (119) in equal amounts or one of them in an excessive amount, and stirring a mixture thereof under cooling to under heating, preferably at -20°C to 60°C, usually for 0.1 hours to 5 days in a solvent inert to the reaction in the presence of a condensing agent. Examples of the solvent include, but are not particularly limited to, aromatic hydrocarbons such as toluene, ethers such as THF and DOX, halogenated hydrocarbons such as dichloromethane, alcohols, DMF, DMSO, ethyl acetate, MeCN, and mixtures thereof. Examples of the condensing agent include PyBOP, HATU, and CDI. The reaction in the presence of an organic base such as TEA, DIPEA, or NMM, or an inorganic base such as potassium carbonate, sodium carbonate, or cesium carbonate may be advantageous for allowing the reaction to proceed smoothly.

II. Production method for drug-linker conjugate ($L^A$-D)

(Drug-linker conjugate production method 1)

A drug-linker conjugate represented by formula (LD-27) can be obtained as a drug-linker conjugate for use in the synthesis of the antibody-drug conjugate of the present invention, through reaction of compound (121) with compound (122) in the presence of a base, followed by reaction with a drug.

[Chemical Formula 193]

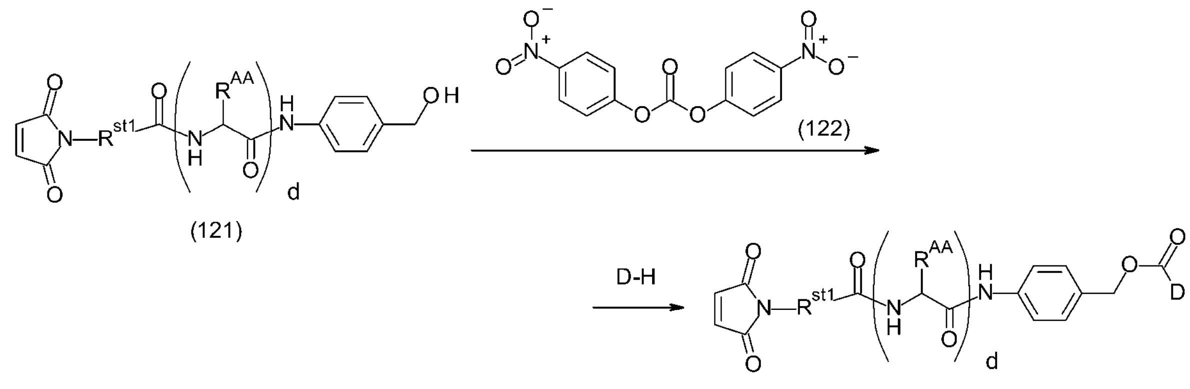


(122)

(121)

(LD-27)

wherein $R^{st1}$ is $C_{1-12}$ alkylene, $R^{AA}$ are each independently H, methyl, isopropyl, benzyl, - $(CH_2)_4$-$NH_2$, -$(CH_2)_3$-NH-C(=NH)-$NH_2$, or -$(CH_2)_3$-NH-C(=O)-$NH_2$, d is an integer of 2 to 4, D-H represents a drug, and H of D-H represents a hydrogen atom of -NH- or -OH contained in the drug (the same holds true for the description below).

For a reference document of this reaction, see the following, for example. International Publication No. WO 2004/010957

(Drug-linker conjugate production method 2)

A drug-linker conjugate represented by formula (LD-28) can be obtained as a drug-linker conjugate for use in the synthesis of the antibody-drug conjugate of the present invention, through reaction of a drug with compound (123) in the presence of a condensing agent.

[Chemical Formula 194]

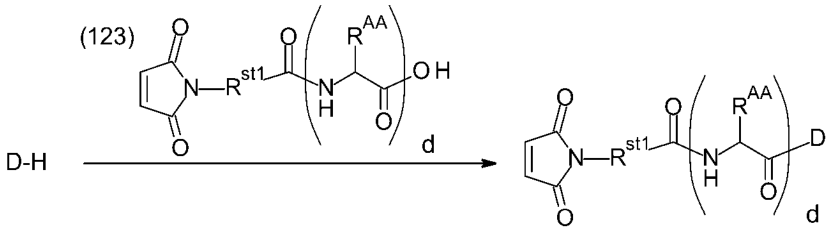

(LD-28)

wherein $R^{st1}$ is $C_{1-12}$ alkylene, $R^{AA}$ are each independently H, methyl, isopropyl, benzyl, - $(CH_2)_4$-$NH_2$, -$(CH_2)_3$-NH-C(=NH)-$NH_2$, or -$(CH_2)_3$-NH-C(=O)-$NH_2$, and d is an integer of 2 to 4.

For a reference document of this reaction, see the following, for example. International Publication No. WO 2023/037268

III. Synthesis of antibody-drug conjugate

An antibody-drug conjugate represented by formula (AD-26) can be obtained as the antibody-drug conjugate of the present invention through reaction of drug-linker conjugate (LD-29) with antibody or antigen binding fragment (Ab).

[Chemical Formula 195]

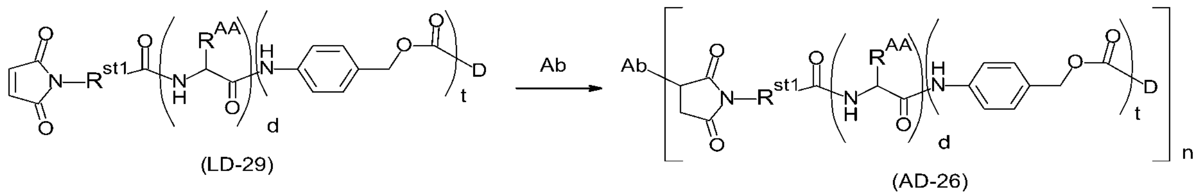

(LD-29) (AD-26)

wherein $R^{st1}$ is $C_{1-12}$ alkylene, $R^{AA}$ are each independently H, methyl, isopropyl, benzyl, - $(CH_2)_4$-$NH_2$, -$(CH_2)_3$-NH-C(=NH)-$NH_2$, or -$(CH_2)_3$-NH-C(=O)-$NH_2$, d is an integer of 2 to 4, t is 0 or 1, and n is a number of 2 to 8.

For a reference document of this reaction, see the following, for example. International Publication No. WO 2004/010957

The antibody-drug conjugate of formula (I) or the salt thereof is isolated and purified as a free compound, a salt thereof, a hydrate, a solvate, or a substance in a crystalline polymorphic or amorphous solid form. The salt of the antibody-drug conjugate of formula (I) may be produced through salification reaction by a routine method.

The isolation and purification are performed by the application of usual chemical operation such as extraction, fractional crystallization, or various fractional chromatographies.

Various isomers can be produced by selecting appropriate starting material compounds, or can be resolved through the use of the difference in physicochemical properties between the isomers. For example, optical isomers are obtained by use of a general optical resolution method of racemic bodies (e.g., fractional crystallization leading to a diastereomeric salt with an optically active base or acid, or chromatography using a chiral column or the like) and may be produced from an appropriate optically active starting material compound.

The antibody-drug conjugate of formula (I) or the salt thereof, or an intermediate thereof, when having axial chirality, is obtained as a mixture of stereoisomers. Each stereoisomer can be isolated by usual resolution operation, for example, resolution using octadecylsilyl (ODS) column chromatography or silica gel column chromatography.

The pharmacological activity of the antibody-drug conjugate of formula (I) or the salt thereof can be confirmed by a method described in Test Examples.

6. Pharmaceutical composition

The present invention also provides a pharmaceutical composition containing the antibody-drug conjugate of the present invention or the salt thereof (also referred to as "pharmaceutical composition of the present invention").

The pharmaceutical composition of the present invention containing one or two or more antibody-drug conjugates of formula (I) or salts thereof as an active ingredient can be prepared by a method usually used using an excipient usually used in the art, i.e., a pharmaceutical excipient, a pharmaceutical carrier, or the like.

The dosage form of the pharmaceutical composition of the present invention can be, for example, an injection, a

formulation for intravenous drip, a suppository, eye drops, an eye ointment, a transdermal solution, an ointment, a transdermal patch, a transmucosal solution, a transmucosal patch, or an inhalant, and can be administered by parenteral administration such as intra-articular, intravenous, intramuscular, subcutaneous, intraperitoneal, or intratumoral administration. The injection or the formulation for intravenous drip can be administered by a proper method, for example, intravenous administration, subcutaneous administration, intraperitoneal administration, or intratumoral administration.

The injection for parenteral administration contains a sterile aqueous or non-aqueous solution, suspension, or emulsion. The aqueous vehicle includes, for example, injectable distilled water or physiological saline. The non-aqueous vehicle includes, for example, alcohols such as EtOH. Such a composition may further contain an isotonic agent, an antiseptic, a wetting agent, an emulsifier, a dispersant, a stabilizer, or a solubilizer. These are sterilized by filtration through a bacteria retaining filter, blending with a germicide, or irradiation, for example. These may be produced as a sterile solid composition and used after being dissolved or suspended in sterile water or a sterile injectable solvent before use.

The pharmaceutical composition of the present invention contains 0.01 to 100 % by weight or 0.01 to 50% by weight in certain embodiments of one or more compounds of formula (I) or salts thereof serving as an active ingredient, though varying depending on an administration route, a dosage form, an administration site, or the type of an excipient or an additive.

The antibody-drug conjugate of formula (I) or the salt thereof can be used in combination with various therapeutic or prophylactic agents for diseases on which the antibody-drug conjugate of formula (I) or the salt thereof mentioned above is considered to exhibit efficacy. The combinations used may be administered concurrently or separately either continuously or at the desired time intervals. The preparations for concurrent administration may be a combination drug or may be separately formulated.

7. Pharmaceutical application of pharmaceutical composition, etc. of present invention

The antibody-drug conjugate of the present invention or the salt thereof, and the pharmaceutical composition of the present invention (hereinafter, also collectively referred to as "pharmaceutical composition, etc. of the present invention") can be used for the treatment of a cancer. The present invention also includes a method for treating a cancer, including the step of administering a therapeutically effective amount of the pharmaceutical composition, etc. of the present invention to a subject. The present invention also includes use of the antibody-drug conjugate of the present invention or the salt thereof in the production of a pharmaceutical composition for the treatment of a cancer.

The cancer to be treated with the pharmaceutical composition, etc. of the present invention can be any of blood cancers and solid cancers. Examples of the therapeutic target include, but are not particularly limited to, stomach cancer, lung cancer, blood cancers such as acute lymphocytic leukemia (ALL), acute myelogenous leukemia (AML), Hodgkin's lymphoma, non-Hodgkin's lymphoma, B cell lymphoma, multiple myeloma, and T cell lymphoma, solid cancers such as myelodysplastic syndrome, adenocarcinoma, squamous cell carcinoma, adenosquamous carcinoma, undifferentiated carcinoma, large-cell cancer, non-small cell lung cancer, small-cell lung cancer, mesothelioma, skin cancer, skin T cell lymphoma, breast cancer, prostate cancer, bladder cancer, vaginal cancer, cervix cancer, head and neck cancer, uterine cancer, uterine cervical cancer, liver cancer, gallbladder cancer, bile duct cancer, kidney cancer, pancreatic cancer, colon cancer, colorectal cancer, rectal cancer, small intestine cancer, gastric cancer, esophageal cancer, testicular cancer, ovarian cancer, and brain tumor, cancers of bone tissues, cartilage tissues, adipose tissues, muscle tissues, vascular tissues, and hematopoietic tissues, sarcomas such as chondrosarcoma, Ewing's sarcoma, malignant hemangioendothelioma, malignant schwannoma, osteosarcoma, and soft tissue sarcoma, and blastomas such as glioblastoma, glioblastoma multiforme, hepatoblastoma, medulloblastoma, nephroblastoma, neuroblastoma, pancreatoblastoma, pleuropulmonary blastoma, and retinoblastoma.

The cancer to be treated with the pharmaceutical composition, etc. of the present invention intracellularly expresses G12D mutant KRAS, in certain embodiments.

Specific Examples to be referred to for gaining further understanding of the present invention will be provided here. However, these are merely illustrative and do not limit the present invention.

EXAMPLES

Hereinafter, methods for producing the antibody-drug conjugate of formula (I) or the salt thereof as well as drug (D), drug-linker conjugate ($L^A$-D), and antibody or antigen binding fragment (Ab) constituting the antibody-drug conjugate or the salt thereof will be described in more detail with reference to Examples. Antibody-drug conjugates or salts thereof described in Examples given below are specific antibody-drug conjugates included in formula (I) or salts thereof. However, the methods for producing the antibody-drug conjugate of formula (I) or the salt thereof are not limited to the production methods of specific Examples given below, and the antibody-drug conjugate of formula (I) or the salt thereof may be produced by combinations of these production methods or methods obvious to those skilled in the art.

In the present specification, a compound may be named using naming software such as ACD/Name(Registered

Trademark) (Advanced Chemistry Development, Inc.).

For the sake of convenience, the concentration mol/L is indicated in M. For example, a 1 M aqueous sodium hydroxide solution means a 1 mol/L aqueous sodium hydroxide solution.

Example 1 Synthesis of drug (D)

Drugs D1 to D6 were synthesized by the methods described in Examples 8, 20, 23, 48, 70, and 72 of International Publication No. WO 2022/173032.

Methods for synthesizing drugs D7 to D12 will be shown below. A number added to each intermediate represents what number of the step in which the intermediate was synthesized in the synthesis route of each drug. For example, an intermediate synthesized in step 1 in the synthesis of drug D7 is given D7-1.

Synthesis of drug D7

(Step 1)

7-Bromo-2,4-dichloro-8-fluoro-6-iodoquinazoline (40 g) was suspended in THF (400 mL), and an aqueous sodium hydroxide solution (1 M, 190 mL) was added dropwise thereto under ice cooling so as to attain an internal temperature of 10°C or lower, and stirred directly for 2 hours. The reaction solution was poured at once into an Erlenmeyer flask containing hydrochloric acid (1 M, 190 mL) and ice water (approximately 900 g), and stirred at room temperature for approximately 30 minutes (until ice was melted). Insoluble matter was collected by filtration while washed with water, and dried under reduced pressure to obtain 7-bromo-2-chloro-8-fluoro-6-iodoquinazolin-4-ol (D7-1, 33.56 g) as a solid.

(Step 2)

To a mixture of 7-bromo-2-chloro-8-fluoro-6-iodoquinazolin-4-ol (D7-1, 24.6 g) and THF (260 mL) heated to 60°C, 2-tert-butyl-1,3-diisopropylisourea (73.4 g) was added dropwise over 15 minutes under nitrogen stream. Stirring was performed at this temperature for 2.5 hours. The resultant was allowed to cool to room temperature, and a colorless solid was filtered off while washed with THF (approximately 500 mL). The filtrate was concentrated, and MeOH (210 mL) was added to the obtained solid, which was suspended and washed by stirring at room temperature for 1 hour. The solid was collected by filtration using MeOH (100 mL) to obtain 7-bromo-4-tert-butoxy-2-chloro-8-fluoro-6-iodoquinazoline (D7-2, 23.2 g) as a solid.

(Step 3)

To a $CH_2Cl_2$ (200 mL) suspension of 7-bromo-4-tert-butoxy-2-chloro-8-fluoro-6-iodoquinazoline (D7-2, 21 g), ethanethiol (3.6 mL) and DABCO (7.7 g) were added at room temperature, and stirred overnight at room temperature in an argon atmosphere. The reaction was terminated by the addition of water under ice cooling. $CHCl_3$ was added thereto for separation between an organic layer and an aqueous layer, and the aqueous layer is subjected to extraction with $CHCl_3$ three times. A combined organic layer was dried over anhydrous sodium sulfate, filtered, and concentrated under reduced pressure to obtain 7-bromo-4-tert-butoxy-2-(ethylsulfanyl)-8-fluoro-6-iodoquinazoline (D7-3, 23.4 g) as a solid.

(Step 4)

To a THF (400 mL) solution of 7-bromo-4-tert-butoxy-2-(ethylsulfanyl)-8-fluoro-6-iodoquinazoline (D7-3, 32 g) and (1S)-1-phenylethan-1-ol (11 mL), tBuOK (10 g) was added under ice cooling, and stirred under ice cooling for 1 hour in an argon atmosphere. The reaction was terminated by the addition of a saturated aqueous solution of ammonium chloride under ice cooling. Water and ethyl acetate were added thereto for separation between an organic layer and an aqueous layer, and the organic layer was washed with a saturated aqueous solution of sodium chloride and dried over anhydrous sodium sulfate. After filtration and concentration under reduced pressure, the residue was purified by silica gel column chromatography (hexane/ethyl acetate) to obtain 7-bromo-4-tert-butoxy-2-(ethylsulfanyl)-6-iodo-8-[(1S)-1-phenylethoxy]quinazoline (D7-4, 36.6 g) as an oil.

(Step 5)

7-Bromo-4-tert-butoxy-2-(ethylsulfanyl)-6-iodo-8-[(1S)-1-phenylethoxy]quinazoline (D7-4, 36.6 g), cyclopropylboronic acid (7.5 g), $PdCl_2(dppf) \cdot CH_2Cl_2$ (7.6 g), tripotassium phosphate (53 g), MeCN (440 mL), and water (80 mL) were mixed at room temperature and stirred at 90°C for 4 hours in an argon atmosphere. The reaction solution was brought back

to room temperature and then diluted with ethyl acetate and water. After separation between an organic layer and an aqueous layer, the organic layer was washed with a saturated aqueous solution of sodium chloride and dried over anhydrous sodium sulfate. After filtration and concentration under reduced pressure, the residue was purified by silica gel column chromatography (hexane/ethyl acetate) to obtain 7-bromo-4-tert-butoxy-6-cyclopropyl-2-(ethylsulfanyl)-8-[(1S)-1-phenylethoxy]quinazoline (D7-5, 22.9 g) as an oil.

(Step 6)

To 7-bromo-4-tert-butoxy-6-cyclopropyl-2-(ethylsulfanyl)-8-[(1S)-1-phenylethoxy]quinazoline (D7-5, 14.21 g), 6-fluoro-5-methyl-4-(4,4,5,5-tetramethyl-1,3,2-dioxaborolan-2-yl)-2-(triphenylmethyl)-2H-indazole (19.3 g), palladium(II) acetate (0.67 g), dicyclohexyl(2',6'-diisopropoxy-[1,1'-biphenyl]-2-yl)phosphine (2.67 g), anhydrous barium hydroxide (14.6 g), and DOX (500 mL)/water (100 mL) were added, and operations of deaeration and purging with argon gas were carried out several times, followed by heating and stirring overnight at 50°C in an argon atmosphere. A reaction suspension allowed to cool was filtered through Celite(Registered Trademark) while washed with ethyl acetate to filter off gray insoluble matter. The filtrate was concentrated into approximately 1/4 under reduced pressure, and water was then added thereto, followed by extraction with ethyl acetate twice. A combined organic layer was washed with a saturated aqueous solution of sodium chloride and dried over anhydrous magnesium sulfate. After concentration under reduced pressure, the resulting residue was purified by silica gel column chromatography (hexane/ethyl acetate) to obtain 4-tert-butoxy-6-cyclopropyl-2-(ethylsulfanyl)-7-[6-fluoro-5-methyl-2-(triphenylmethyl)-2H-indazol-4-yl]-8-[(1S)-1-phenylethoxy]quinazoline (D7-6, diastereomer mixture of approximately 3.3:1 derived from axial chirality, 16.44 g) as a solid.

(Step 7)

4-tert-Butoxy-6-cyclopropyl-2-(ethylsulfanyl)-7-[6-fluoro-5-methyl-2-(triphenylmethyl)-2H-indazol-4-yl]-8-[(1S)-1-phenylethoxy]quinazoline (D7-6, a diastereomer mixture of approximately 3.3:1 derived from axial chirality, 33.71 g) was dissolved in $CH_2Cl_2$ (500 mL) under nitrogen stream, and m-chloroperbenzoic acid (containing approximately 30% water, 22.4 g) was added thereto under ice cooling (internal temperature: 5 to 10°C) and stirred at room temperature for 2 hours. An aqueous solution (300 mL) of sodium thiosulfate pentahydrate (11 g) and a saturated aqueous solution of sodium bicarbonate (300 mL) were poured to the reaction solution under ice cooling and stirred at room temperature for 30 minutes, followed by extraction with ethyl acetate twice. A combined organic layer was washed with a saturated aqueous solution of sodium bicarbonate and a saturated aqueous solution of sodium chloride, dried over anhydrous magnesium sulfate, filtered, and concentrated under reduced pressure to obtain 4-tert-butoxy-6-cyclopropyl-2-(ethanesulfonyl)-7-[6-fluoro-5-methyl-2-(triphenylmethyl)-2H-indazol-4-yl]-8-[(1S)-1-phenylethoxy]quinazoline (diastereomer mixture derived from axial chirality). iPrOH (1000 mL) was added to the obtained residue and stirred overnight at room temperature. The resulting solid was collected by filtration, washed with iPrOH, and dried under reduced pressure to obtain 4-tert-butoxy-6-cyclopropyl-2-(ethanesulfonyl)-7-[6-fluoro-5-methyl-2-(triphenylmethyl)-2H-indazol-4-yl]-8-[(1S)-1-phenylethoxy]quinazoline (diastereomer mixture of approximately 1:1 derived from axial chirality, 11.52 g) as a solid. The filtrate was concentrated under reduced pressure to obtain (7M)-4-tert-butoxy-6-cyclopropyl-2-(ethanesulfonyl)-7-[6-fluoro-5-methyl-2-(triphenylmethyl)-2H-indazol-4-yl]-8-[(1S)-1-phenylethoxy]quinazoline (D7-7, single diastereomer, 23.29 g) of interest as a solid.

(Step 8)

To a THF (50 mL) solution of (7M)-4-tert-butoxy-6-cyclopropyl-2-(ethanesulfonyl)-7-[6-fluoro-5-methyl-2-(triphenylmethyl)-2H-indazol-4-yl]-8-[(1S)-1-phenylethoxy]quinazoline (D7-7, 2.00 g), 4-methylbenzene-1-sulfonic acid monohydrate (230 mg) was added at room temperature in a nitrogen atmosphere, and stirred at 50°C for 1 hour. The reaction solution was allowed to cool to room temperature, and TEA (350 μL) was then added thereto, followed by concentration under reduced pressure. The obtained residue was purified by silica gel column chromatography (basic silica gel, hexane/ethyl acetate) to obtain (7M)-4-tert-butoxy-6-cyclopropyl-2-(ethanesulfonyl)-7-(6-fluoro-5-methyl-1H-indazol-4-yl)-8-[(1S)-1-phenylethoxy]quinazoline(D7-8, 740 mg) as a solid in a foam.

(Step 9)

To a THF (10 mL) solution of (7M)-4-tert-butoxy-6-cyclopropyl-2-(ethanesulfonyl)-7-(6-fluoro-5-methyl-1H-indazol-4-yl)-8-[(1S)-1-phenylethoxy]quinazoline (D7-8, 735 mg), 3,4-dihydro-2H-pyran (900 μL) and 4-methylbenzene-1-sulfonic acid monohydrate (35 mg) were added at room temperature in a nitrogen atmosphere. The reaction solution was stirred overnight at room temperature and concentrated under reduced pressure. The obtained residue was purified by silica gel column chromatography (basic silica gel, hexane/ethyl acetate) to obtain (7M)-4-tert-butoxy-6-cyclopropyl-2-(ethanesulfonyl)-7-[6-fluoro-5-methyl-1-(oxan-2-yl)-1H-indazol-4-yl]-8-[(1S)-1-phenylethoxy]quinazoline (D7-9, 735 mg) as a solid in a foam.

(Step 10)

To a MeOH (15 mL)/THF (15 mL) solution of (7M)-4-tert-butoxy-6-cyclopropyl-2-(ethanesulfonyl)-7-[6-fluoro-5-methyl-1-(oxan-2-yl)-1H-indazol-4-yl]-8-[(1S)-1-phenylethoxy]quinazoline (D7-9, 730 mg), sodium bicarbonate (450 mg) and 10% Pd/C (containing approximately 50% water, 250 mg) were added at room temperature in an argon atmosphere. Stirring was performed overnight at normal pressure and temperature in a hydrogen atmosphere. After purging with argon, the reaction solution was filtered through Celite(Registered Trademark) and washed with ethyl acetate. The filtrate was concentrated under reduced pressure to obtain (7M)-4-tert-butoxy-6-cyclopropyl-2-(ethanesulfonyl)-7-[6-fluoro-5-methyl-1-(oxan-2-yl)-1H-indazol-4-yl]quinazolin-8-ol (D7-10, 630 mg) as a solid in a foam.

(Step 11)

(7M)-4-tert-Butoxy-6-cyclopropyl-2-(ethanesulfonyl)-7-[6-fluoro-5-methyl-1-(oxan-2-yl)-1H-indazol-4-yl]quinazolin-8-ol (D7-10, 1.4 g) and cesium carbonate (2.4 g) were suspended in DMF (17 mL) under nitrogen stream, and 1-(chloromethyl)-4-ethynylbenzene (500 mg) was added thereto at room temperature and stirred at 60°C for 1.5 hours. Water (100 ml) was added to the reaction mixture, followed by extraction with ethyl acetate twice. An organic layer was dried over anhydrous magnesium sulfate, filtered, and concentrated under reduced pressure, and the residue was purified by silica gel column chromatography (hexane/ethyl acetate) to obtain (7M)-4-tert-butoxy-6-cyclopropyl-2-(ethanesulfonyl)-8-[(4-ethynylphenyl)methoxy]-7-[6-fluoro-5-methyl-1-(oxan-2-yl)-1H-indazol-4-yl]quinazoline (D7-11, 1.47 g) as a solid in a foam.

(Step 12)

To a THF (11 mL) solution of (7M)-4-tert-butoxy-6-cyclopropyl-2-(ethanesulfonyl)-8-[(4-ethynylphenyl)methoxy]-7-[6-fluoro-5-methyl-1-(oxan-2-yl)-1H-indazol-4-yl]quinazoline (D7-11, 1.47 g) and (2S)-2-methoxypropan-1-ol (0.25 mL), tBuOK (275 mg) was added under cooling in an ice/MeOH bath (-20°C to -15°C) in a nitrogen atmosphere, and stirred at the same temperature for 30 minutes. A saturated aqueous solution of ammonium chloride was added to the reaction mixture at the same temperature, followed by extraction with ethyl acetate twice. A combined organic layer was washed with water and saturated saline, dried over anhydrous magnesium sulfate, and filtered. The filtrate was concentrated under reduced pressure to obtain (7M)-4-tert-butoxy-6-cyclopropyl-8-[(4-ethynylphenyl)methoxy]-7-[6-fluoro-5-methyl-1-(oxan-2-yl)-1H-indazol-4-y1]-2-[(2S)-2-methoxypropoxy]quinazoline (D7-12, 1.43 g) as a solid in a foam.

(Step 13)

To a THF (15 mL) solution of (7M)-4-tert-butoxy-6-cyclopropyl-8-[(4-ethynylphenyl)methoxy]-7-[6-fluoro-5-methyl-1-(oxan-2-yl)-1H-indazol-4-yl]-2-[(2S)-2-methoxypropoxy]quinazoline (D7-12, 1.43 g), 4-methylbenzene-1-sulfonic acid monohydrate (120 mg) was added at room temperature in a nitrogen atmosphere, and stirred at 50°C for 3 hours. The reaction solution was concentrated under reduced pressure, and the obtained residue was purified by silica gel column chromatography (hexane/ethyl acetate) to obtain (7M)-6-cyclopropyl-8-[(4-ethynylphenyl)methoxy]-7-[6-fluoro-5-methyl-1-(oxan-2-yl)-1H-indazol-4-yl]-2-[(2S)-2-methoxypropoxy]quinazolin-4-ol (D7-13, 1.05 g) as a solid in a foam.

(Step 14)

To a THF (15 mL) solution of (7M)-6-cyclopropyl-8-[(4-ethynylphenyl)methoxy]-7-[6-fluoro-5-methyl-1-(oxan-2-yl)-1H-indazol-4-yl]-2-[(2S)-2-methoxypropoxy]quinazolin-4-ol (D7-13, 1.05 g), cesium carbonate(2.68 g) and PyBOP (1.20 g) were added in order at room temperature in a nitrogen atmosphere, and stirred at room temperature for 1 hour. tert-Butyl (3S)-3-(methylamino)pyrrolidine-1-carboxylate (0.94 mL) was added thereto at room temperature and stirred overnight at 60°C. Ethyl acetate was added to the reaction solution, and insoluble matter was filtered through Celite(Registered Trademark). The filtrate was concentrated under reduced pressure, and the obtained residue was purified by silica gel column chromatography (basic silica gel, hexane/ethyl acetate) to obtain tert-butyl (3S)-3-[{(7M)-6-cyclopropyl-8-[(4-ethynylphenyl)methoxy]-7-[6-fluoro-5-methyl-1-(oxan-2-yl)-1H-indazol-4-yl]-2-[(2S)-2-methoxypropoxy]quinazolin-4-yl}(methyl)amino]pyrrolidine-1-carboxylate (D7-14, 1.62 g) as an oil.

(Step 15)

To a tBuOH/THF/water (1:1:1 mixed solution, 2.0 mL) solution of (4R)-1-[(2S)-2-azido-3-methylbutanoyl]-4-hydroxy-N-{(1R)-2-hydroxy-1-[4-(4-methyl-1,3-thiazol-5-yl)phenyl]ethyl}-L-prolinamide (75 mg) and tert-butyl (3S)-3-[{(7M)-6-cyclopropyl-8-[(4-ethynylphenyl)methoxy]-7-[6-fluoro-5-methyl-1-(oxan-2-yl)-1H-indazol-4-yl]-2-[(2S)-2-methoxypropoxy]quinazolin-4-yl}(methyl)amino]pyrrolidine-1-carboxylate (D7-14, 230 mg), anhydrous copper(II) sulfate(10 mg), sodium ascorbate(30 mg) were added at room temperature in an argon atmosphere, and stirred at room temperature for 1 hour. Ethylenediaminetetraacetic acid disodium salt (175 mg) was added to the reaction mixture at room temperature and stirred at the same temperature for 1 hour. Water and $CHCl_3$/MeOH (9/1) were added to the reaction mixture. After separation between an aqueous layer and an organic layer, the aqueous layer was subjected to extraction with $CHCl_3$/MeOH (9/1). A combined organic layer was dried over anhydrous sodium sulfate and filtered, and the filtrate was concentrated under reduced pressure. The obtained residue was purified by silica gel column chromatography (basic silica gel, $CHCl_3$/MeOH) to obtain tert-butyl (3S)-3-[{(7M)-6-cyclopropyl-7-[6-fluoro-5-methyl-1-(oxan-2-yl)-1H-indazol-4-yl]-8-{[4-(1-{(2S)-1-[(2S,4R)-4-hydroxy-2-({(1R)-2-hydroxy-1-[4-(4-methyl-1,3-thiazol-5-yl)phenyl]ethyl}carbamoyl)pyrrolidin-1-yl]-3-methyl-1-oxobutan-2-yl}-1H-1,2,3-triazol-4-yl)phenyl]methoxy}-2-[(2S)-2-methoxypropoxy]quinazolin-4-yl}(methyl)amino]pyrrolidine-1-carboxylate (D7-15, 197 mg) as a solid in a foam.

(Step 16)

To a $CH_2Cl_2$ (2 mL) solution of tert-butyl (3S)-3-[{(7M)-6-cyclopropyl-7-[6-fluoro-5-methyl-1-(oxan-2-yl)-1H-indazol-4-yl]-8-{[4-(1-{(2S)-1-[(2S,4R)-4-hydroxy-2-({(1R)-2-hydroxy-1-[4-(4-methyl-1,3-thiazol-5-yl)phenyl]ethyl}carbamoyl)pyrrolidin-1-yl]-3-methyl-1-oxobutan-2-yl}-1H-1,2,3-triazol-4-yl)phenyl]methoxy}-2-[(2S)-2-methoxypropoxy] quinazolin-4-yl}(methyl)amino]pyrrolidine-1-carboxylate (D7-15, 197 mg), trifluoroacetic acid (0.5 mL) was added under ice cooling in a nitrogen atmosphere, and stirred at room temperature for 2 hours. The reaction mixture was concentrated under reduced pressure, and MeCN, a saturated aqueous solution of sodium bicarbonate, and water were added to the residue and stirred at room temperature for 20 minutes. The reaction solution was purified by ODS column chromatography (MeCN/0.1% aqueous formic acid solution), and a fraction containing the compound of interest was concentrated. The residue was dissolved in $CHCl_3$/iPrOH (9/1), a saturated aqueous solution of sodium bicarbonate was added thereto, followed by extraction with $CHCl_3$/MeOH (5/1) twice. A combined organic layer was dried over anhydrous sodium sulfate and concentrated under reduced pressure to obtain (4R)-1-[(2S)-2-{4-[4-({[(7M)-6-cyclopropyl-7-(6-fluoro-5-methyl-1H-indazol-4-yl)-2-[(2S)-2-methoxypropoxy]-4-{methyl[(3S)-pyrrolidin-3-yl]amino}quinazolin-8-yl]oxy}methyl)phenyl]-1H-1,2,3-triazol-1-yl}-3-methylbutanoyl]-4-hydroxy-N-{(1R)-2-hydroxy-1-[4-(4-methyl-1,3-thiazol-5-yl)phenyl] ethyl}-L-prolinamide (D7, 65 mg) as a solid in a foam.

Synthesis of drug D8

(Step 1)

To a tBuOH/THF/ water (1:1:1 mixed solution, 2.0 mL) solution of (4R)-1-[(2S)-2-azido-3-methylbutanoyl]-N-{(1R)-1-[4-(1-ethyl-1H-pyrazol-5-yl)phenyl]-2-hydroxyethyl}-4-hydroxy-L-prolinamide (80 mg) and tert-butyl (3S)-3-[{(7M)-6-cyclopropyl-8-[(4-ethynylphenyl)methoxy]-7-[6-fluoro-5-methyl-1-(oxan-2-yl)-1H-indazol-4-yl]-2-[(2S)-2-methoxypropoxy]quinazolin-4-yl}(methyl)amino]pyrrolidine-1-carboxylate (D7-14, 125 mg), anhydrous copper(II) sulfate (10 mg) and sodium ascorbate (30 mg) were added at room temperature, and stirred at room temperature for 1 hour. Ethylenediaminetetraacetic acid disodium salt (175 mg) was added to the reaction mixture at room temperature and stirred at the same temperature for 30 minutes. Water was added to the reaction mixture and stirred, and the resulting insoluble matter was collected by filtration to obtain tert-butyl (3S)-3-[{(7M)-6-cyclopropyl-8-{[4-(1-{(2S)-1-[(2S,4R)-2-({(1R)-1-[4-(1-ethyl-1H-pyrazol-5-yl)phenyl]-2-hydroxyethyl}carbamoyl)-4-hydroxypyrrolidin-1-yl]-3-methyl-1-oxobutan-2-yl}-1H-1,2,3-triazol-4-yl)phenyl]methoxy}-7-[6-fluoro-5-methyl-1-(oxan-2-yl)-1H-indazol-4-yl]-2-[(2S)-2-methoxypropoxy]quinazolin-4-yl}(methyl)amino]pyrrolidine-1-carboxylate (D8-1, 225 mg) as a solid.

(Step 2)

To a $CH_2Cl_2$ (1 mL) solution of tert-butyl (3S)-3-[{(7M)-6-cyclopropyl-8-{[4-(1-{(2S)-1-[(2S,4R)-2-({(1R)-1-[4-(1-ethyl-1H-pyrazol-5-yl)phenyl]-2-hydroxyethyl}carbamoyl)-4-hydroxypyrrolidin-1-yl]-3-methyl-1-oxobutan-2-yl}-1H-1,2,3-triazol-4-yl)phenyl]methoxy}-7-[6-fluoro-5-methyl-1-(oxan-2-yl)-1H-indazol-4-yl]-2-[(2S)-2-methoxypropoxy]quinazolin-4-yl}(methyl)amino]pyrrolidine-1-carboxylate (D8-1, 224 mg), TFA (1 mL) was added at room temperature. Stirring was performed at room temperature for 2 hours. The reaction solvent was concentrated under reduced

pressure, and MeCN, a saturated aqueous solution of sodium bicarbonate, and water were added to the residue and stirred at room temperature for 20 minutes. The obtained suspension was purified by ODS column chromatography (MeCN/0.1% aqueous formic acid solution), and the fraction of interest was concentrated under reduced pressure. The residue was dissolved in $CHCl_3$/iPrOH (9/1), and a saturated aqueous solution of sodium bicarbonate was added thereto, followed by extraction with $CHCl_3$/MeOH (5/1) twice. A combined organic layer was dried over anhydrous sodium sulfate and concentrated under reduced pressure to obtain (4R)-1-[(2S)-2-{4-[4-({[(7M)-6-cyclopropyl-7-(6-fluoro-5-methyl-1H-indazol-4-yl)-2-[(2S)-2-methoxypropoxy]-4-{methyl[(3S)-pyrrolidin-3-yl]amino}quinazolin-8-yl]oxy}methyl)phenyl]-1H-1,2,3-triazol-1-yl}-3-methylbutanoyl]-N-{(1R)-1-[4-(1-ethyl-1H-pyrazol-5-yl)phenyl]-2-hydroxyethyl}-4-hydroxy-L-prolinamide (D8, 109 mg) as a solid in a foam.

Synthesis of drug D9

(Step 1)

To a mixture of tert-butyl (1S,4S)-5-[7-bromo-2-(ethylsulfanyl)-8-fluoro-6-iodoquinazolin-4-yl]-2,5-diazabicyclo[2.2.1]heptane-2-carboxylate (40.2 g), dehydrated THF (400 mL), and (1S)-1-phenylethan-1-ol (10 mL) thoroughly cooled and stirred in an ice/MeOH bath, tBuOK (15.3 g) was added, and stirred under cooling for 30 minutes and at room temperature for 30 minutes in an argon atmosphere. Ice and a saturated aqueous solution of ammonium chloride were poured to the reaction mixture, followed by extraction with ethyl acetate twice. A combined organic layer was washed with a saturated aqueous solution of sodium chloride, dried over anhydrous magnesium sulfate, and concentrated under reduced pressure to obtain tert-butyl (1S,4S)-5-{7-bromo-2-(ethylsulfanyl)-6-iodo-8-[(1S)-1-phenylethoxy]quinazolin-4-yl}-2,5-diazabicyclo[2.2.1]heptane-2-carboxylate (D9-1, 45.94 g) as a solid in a foam.

(Step 2)

A mixture of tert-butyl (1S,4S)-5-{7-bromo-2-(ethylsulfanyl)-6-iodo-8-[(1S)-1-phenylethoxy]quinazolin-4-yl}-2,5-diazabicyclo[2.2.1]heptane-2-carboxylate (D9-1, 45.93 g), cyclopropylboronic acid (9.8 g), tripotassium phosphate (55 g), $PdCl_2$(dppf)·$CH_2Cl_2$(5.4 g), MeCN (360 mL), DOX (240 mL), and water (120 mL) was deaerated, then purged with argon gas, and stirred at 90°C for 5 hours in an argon atmosphere. A reaction mixture allowed to cool was concentrated into about half the amount under reduced pressure, and a saturated aqueous solution of sodium bicarbonate (100 mL) and water (300 mL) were poured to the residue, followed by extraction with ethyl acetate twice. A combined organic layer was washed with a saturated aqueous solution of sodium chloride, and thiol-modified silica gel (approximately 10 g) and basic silica gel (approximately 10 g) were then added thereto and stirred at room temperature for 30 minutes. Insoluble matter was filtered off while washed with ethyl acetate, and the filtrate was concentrated under reduced pressure. The residue was purified by silica gel column chromatography (hexane/ethyl acetate) to obtain tert-butyl (1S,4S)-5-{7-bromo-6-cyclopropyl-2-(ethylsulfanyl)-8-[(1S)-1-phenylethoxy]quinazolin-4-yl}-2,5-diazabicyclo[2.2.1]heptane-2-carboxylate (D9-2, 27.74 g) as a solid in a foam.

(Step 3)

A mixture of tert-butyl (1S,4S)-5-{7-bromo-6-cyclopropyl-2-(ethylsulfanyl)-8-[(1S)-1-phenylethoxy]quinazolin-4-yl}-2,5-diazabicyclo[2.2.1]heptane-2-carboxylate (D9-2, 17.7 g), 6-fluoro-5-methyl-4-(4,4,5,5-tetramethyl-1,3,2-dioxaborolan-2-yl)-2-(triphenylmethyl)-2H-indazole (19.6 g), palladium(II) acetate (0.7 g), dicyclohexyl(2',6'-diisopropoxy-[1,1'-biphenyl]-2-yl)phosphine (2.7 g), barium hydroxide (14.6 g), DOX (500 mL), and water (50 mL) was deaerated, then purged with argon gas, and stirred at 50°C for 6 hours in an argon atmosphere. A reaction suspension allowed to cool was filtered through Celite(Registered Trademark) while washed with ethyl acetate. The filtrate was concentrated into approximately 1/4 under reduced pressure, and water was then poured thereto, followed by extraction with ethyl acetate twice. A combined organic layer was washed with a saturated aqueous solution of sodium chloride and then dried over anhydrous magnesium sulfate. After concentration under reduced pressure, the residue was purified by silica gel column chromatography (basic silica gel, hexane/ethyl acetate) twice to obtain tert-butyl (1S,4S)-5-{6-cyclopropyl-2-(ethylsulfanyl)-7-[6-fluoro-5-methyl-2-(triphenylmethyl)-2H-indazol-4-yl]-8-[(1S)-1-phenylethoxy]quinazolin-4-yl}-2,5-diazabicyclo[2.2.1]heptane-2-carboxylate (D9-3, isomer mixture of approximately 4.5:1 related to axial chirality, 20.64 g) as a solid in a foam.

(Step 4)

To a mixture of tert-butyl (1S,4S)-5-{6-cyclopropyl-2-(ethylsulfanyl)-7-[6-fluoro-5-methyl-2-(triphenylmethyl)-2H-indazol-4-yl]-8-[(1S)-1-phenylethoxy]quinazolin-4-yl}-2,5-diazabicyclo[2.2.1]heptane-2-carboxylate (D9-3,

isomer mixture of approximately 4.5:1 related to axial chirality, 20.63 g) and EtOH(250 mL), 4-methylbenzene-1-sulfonic acid monohydrate (4.8 g) was added with stirring at room temperature, and stirred at room temperature for 1 hour in an argon atmosphere. Ice and a saturated aqueous solution of sodium bicarbonate were poured to the reaction mixture, and the resulting white insoluble matter was dissolved in ethyl acetate, followed by extraction with ethyl acetate twice. A combined organic layer was washed with a saturated aqueous solution of sodium chloride, dried over anhydrous magnesium sulfate, and then concentrated under reduced pressure. The residue was purified by silica gel column chromatography (hexane/ethyl acetate) to obtain tert-butyl (1S,4S)-5-{(7M)-6-cyclopropyl-2-(ethylsulfanyl)-7-(6-fluoro-5-methyl-1H-indazol-4-yl)-8-[(1S)-1-phenylethoxy]quinazolin-4-yl}-2,5-diazabicyclo[2.2.1]heptane-2-carboxylate (D9-4, single isomer related to axial chirality, 11.17 g) as a solid in a foam.

(Step 5)

To a mixture of tert-butyl (1S,4S)-5-{(7M)-6-cyclopropyl-2-(ethylsulfanyl)-7-(6-fluoro-5-methyl-1H-indazol-4-yl)-8-[(1S)-1-phenylethoxy]quinazolin-4-yl}-2,5-diazabicyclo[2.2.1]heptane-2-carboxylate (D9-4, 11.1 g) and THF (150 mL), 3,4-dihydro-2H-pyran (10 mL) and 4-methylbenzene-1-sulfonic acid monohydrate (0.7 g) were added with stirring at room temperature, and stirred at room temperature for 14 hours in an argon atmosphere. 3,4-Dihydro-2H-pyran (5 mL) and 4-methylbenzene-1-sulfonic acid monohydrate (0.3 g) were further added thereto, and further stirred at room temperature for 5 hours. The reaction was terminated by the addition of TEA (2 mL), followed by concentration under reduced pressure. The residue was purified by silica gel column chromatography (hexane/ethyl acetate) to obtain tert-butyl (1S,4S)-5-{(7M)-6-cyclopropyl-2-(ethylsulfanyl)-7-[6-fluoro-5-methyl-1-(oxan-2-yl)-1H-indazol-4-yl]-8-[(1S)-1-phenylethoxy]quinazolin-4-yl}-2,5-diazabicyclo[2.2.1]heptane-2-carboxylate (D9-5, 11.15 g) as a solid in a foam.

(Step 6)

To a mixture of tert-butyl (1S,4S)-5-{(7M)-6-cyclopropyl-2-(ethylsulfanyl)-7-[6-fluoro-5-methyl-1-(oxan-2-yl)-1H-indazol-4-yl]-8-[(1S)-1-phenylethoxy]quinazolin-4-yl}-2,5-diazabicyclo[2.2.1]heptane-2-carboxylate (D9-5, 11.14 g) and $CH_2Cl_2$ (170 mL), m-chloroperbenzoic acid (containing approximately 30% water, 7.9 g) was added under ice cooling, and stirred at room temperature for 1.5 hours in an argon atmosphere. Ice, a saturated aqueous solution of sodium thiosulfate, and a saturated aqueous solution of sodium bicarbonate were added thereto, and stirred at room temperature for approximately 20 minutes, and the reaction solution was then diluted with $CH_2Cl_2$ and separated. An organic layer was dried over anhydrous magnesium sulfate and concentrated under reduced pressure. The residue was purified by silica gel column chromatography (hexane/ethyl acetate) to obtain tert-butyl (1S,4S)-5-{(7M)-6-cyclopropyl-2-(ethanesulfonyl)-7-[6-fluoro-5-methyl-1-(oxan-2-yl)-1H-indazol-4-yl]-8-[(1S)-1-phenylethoxy]quinazolin-4-yl}-2,5-diazabicyclo[2.2.1]heptane-2-carboxylate (D9-6, 9.02 g) as a solid in a foam.

(Step 7)

To a mixture of tert-butyl (1S,4S)-5-{(7M)-6-cyclopropyl-2-(ethanesulfonyl)-7-[6-fluoro-5-methyl-1-(oxan-2-yl)-1H-indazol-4-yl]-8-[(1S)-1-phenylethoxy]quinazolin-4-yl}-2,5-diazabicyclo[2.2.1]heptane-2-carboxylate (D9-6, 4.5 g) and MeOH (100 mL), 10% Pd/C (containing 52% water, 1 g) was added, and stirred overnight at room temperature in a hydrogen atmosphere of normal pressure. Celite(Registered Trademark) was added thereto, and the resultant was filtered while washed with ethyl acetate and $CHCl_3$. Toluene (approximately 20 mL) was added to the filtrate and concentrated under reduced pressure, and MeOH (80 mL) and 10% Pd/C (containing 52% water, 1.4 g) were added to the obtained solid and stirred at room temperature for 3 hours in a hydrogen atmosphere of normal pressure. Celite(Registered trademark) was added thereto, and the resultant was filtered while washed with ethyl acetate and $CHCl_3$. The filtrate was concentrated under reduced pressure, and the residue was purified by silica gel column chromatography (hexane/ethyl acetate) to obtain tert-butyl (1S,4S)-5-{(7M)-6-cyclopropyl-2-(ethanesulfonyl)-7-[6-fluoro-5-methyl-1-(oxan-2-yl)-1H-indazol-4-yl]-8-hydroxyquinazolin-4-yl}-2,5-diazabicyclo[2.2.1]heptane-2-carboxylate (D9-7, 3.42 g) as a solid in a foam.

(Step 8)

To a mixture of tert-butyl (1S,4S)-5-{(7M)-6-cyclopropyl-2-(ethanesulfonyl)-7-[6-fluoro-5-methyl-1-(oxan-2-yl)-1H-indazol-4-yl]-8-hydroxyquinazolin-4-yl}-2,5-diazabicyclo[2.2.1]heptane-2-carboxylate (D9-7, 3.01 g), DMF (30 mL), and methyl 4-(chloromethyl)benzoate (1 g), cesium carbonate (4 g) was added with stirring at room temperature, and stirred overnight at room temperature in an argon atmosphere. Ice water and a saturated aqueous solution of ammonium chloride were poured to the reaction mixture, followed by extraction with ethyl acetate twice. A combined organic layer was washed with a saturated aqueous solution of sodium chloride and dried over anhydrous magnesium

sulfate. After concentration under reduced pressure, the obtained residue was purified by silica gel column chromatography (basic silica gel, hexane/ethyl acetate) to obtain tert-butyl (1S,4S)-5-[(7M)-6-cyclopropyl-2-(ethanesulfonyl)-7-[6-fluoro-5-methyl-1-(oxan-2-yl)-1H-indazol-4-yl]-8-{[4-(methoxycarbonyl)phenyl]methoxy}quinazolin-4-yl]-2,5-diazabicyclo[2.2.1]heptane-2-carboxylate (D9-8, 3.32 g) as a solid in a foam.

(Step 9)

To a mixture of tert-butyl (1S,4S)-5-[(7M)-6-cyclopropyl-2-(ethanesulfonyl)-7-[6-fluoro-5-methyl-1-(oxan-2-yl)-1H-indazol-4-yl]-8-{[4-(methoxycarbonyl)phenyl]methoxy}quinazolin-4-yl]-2,5-diazabicyclo[2.2.1]heptane-2-carboxylate (D9-8, 200 mg), tetrahydro-2H-pyran-4-ol (32 mg), and THF (4 mL), tBuOK (56 mg) was added under ice cooling in an ice/MeOH bath, and stirred at the same temperature for 30 minutes in an argon atmosphere. Ice water and a saturated aqueous solution of ammonium chloride were poured to the reaction mixture, followed by extraction with ethyl acetate twice. A combined organic layer was washed with a saturated aqueous solution of sodium chloride and dried over anhydrous magnesium sulfate. After concentration under reduced pressure, the residue was purified by silica gel column chromatography (basic silica gel, hexane/ethyl acetate) to obtain tert-butyl (1S,4S)-5-{(7M)-6-cyclopropyl-7-[6-fluoro-5-methyl-1-(oxan-2-yl)-1H-indazol-4-yl]-8-{[4-(methoxycarbonyl)phenyl]methoxy}-2-[(oxan-4-yl)oxy]quinazolin-4-yl}-2,5-diazabicyclo[2.2.1]heptane-2-carboxylate (D9-9, 144 mg) as a solid in a foam.

(Step 10)

To a mixture of tert-butyl (1S,4S)-5-{(7M)-6-cyclopropyl-7-[6-fluoro-5-methyl-1-(oxan-2-yl)-1H-indazol-4-yl]-8-{[4-(methoxycarbonyl)phenyl]methoxy}-2-[(oxan-4-yl)oxy]quinazolin-4-yl}-2,5-diazabicyclo[2.2.1]heptane-2-carboxylate (D9-9, 142 mg), MeOH (1 mL), and THF (1 mL), an aqueous sodium hydroxide solution (1 M, 1 mL) was added under ice cooling, and stirred at room temperature for 6 hours. Hydrochloric acid (1 M, 1 mL) was added thereto under ice cooling, followed by extraction with $CHCl_3$/MeOH (9/1) twice. A combined organic layer was dried over anhydrous sodium sulfate. The solvent was distilled off under reduced pressure to obtain 4-[({(7M)-4-[(1S,4S)-5-(tert-butoxycarbonyl)-2,5-diazabicyclo[2.2.1]heptan-2-yl]-6-cyclopropyl-7-[6-fluoro-5-methyl-1-(oxan-2-yl)-1H-indazol-4-yl]-2-[(oxan-4-yl)oxy]quinazolin-8-yl}oxy)methyl]benzoic acid (D9-10, 132 mg) as a solid.

(Step 11)

To a $CH_2Cl_2$ (10 mL) solution of 3-hydroxy-1-[(4-methoxyphenyl)methyl]piperidine-2,6-dione (800 mg), trifluoromethanesulfonic anhydride (809 μL) and pyridine (520 μL) were added under ice cooling in an argon atmosphere, and stirred at the same temperature for 1 hour. After concentration under reduced pressure, the obtained residue was purified by silica gel column chromatography (hexane/ethyl acetate) to obtain 1-[(4-methoxyphenyl)methyl]-2,6-dioxopiperidin-3-yl trifluoromethanesulfonate (D9-11, 1.1 g) as an oil.

(Step 12)

To a THF (20 mL) solution of 6-bromo-1-methyl-1,3-dihydro-2H-benzimidazol-2-one (350 mg), tBuOK (260 mg) was added under cooling in an ice/sodium chloride bath in an argon atmosphere, and stirred at the same temperature for 30 minutes. A THF (7 mL) solution of 1-[(4-methoxyphenyl)methyl]-2,6-dioxopiperidin-3-yl trifluoromethanesulfonate (D9-11, 700 mg) was added dropwise thereto over 10 minutes and stirred at the same temperature for 30 minutes. Water was added to the reaction mixture, followed by extraction with ethyl acetate three times. A combined organic layer was washed with a saturated aqueous solution of sodium chloride, dried over anhydrous magnesium sulfate, and concentrated under reduced pressure. The obtained solid was collected by filtration with ethyl acetate, washed, and dried under reduced pressure to obtain 3-(5-bromo-3-methyl-2-oxo-2,3-dihydro-1H-benzimidazol-1-yl)-1-[(4-methoxyphenyl)methyl]piperidine-2,6-dione (D9-12, 524 mg) as a solid.

(Step 13)

To 3-(5-bromo-3-methyl-2-oxo-2,3-dihydro-1H-benzimidazol-1-yl)-1-[(4-methoxyphenyl)methyl]piperidine-2,6-dione (D9-12, 500 mg), trifluoroacetic acid (5 mL) and trifluoromethanesulfonic acid (5 mL) were added, and stirred at 60°C for 1 hour. The solution was concentrated into about half the amount under reduced pressure, ethyl acetate was added thereto, and the resultant was poured to a saturated aqueous solution of sodium bicarbonate under ice cooling and stirred for a while. An organic layer was separated, and an aqueous layer was subjected to extraction with ethyl acetate. A combined organic layer was washed with a saturated aqueous solution of sodium chloride, dried over anhydrous magnesium sulfate, and concentrated under reduced pressure. The obtained residue was purified by silica gel column

chromatography (hexane/ethyl acetate) to obtain 3-(5-bromo-3-methyl-2-oxo-2,3-dihydro-1H-benzimidazol-1-yl)piperidine-2,6-dione (D9-13, 300 mg) as a solid.

(Step 14)

A mixture of 3-(5-bromo-3-methyl-2-oxo-2,3-dihydro-1H-benzimidazol-1-yl)piperidine-2,6-dione (D9-13, 4.32 g), potassium vinyltrifluoroborate (7.5 g), $PdCl_2(dppf)\cdot CH_2Cl_2$ (1.16 g), cesium carbonate (9.2 g), DOX (50 mL), and water (5 mL) was stirred at 80°C for 3 hours in an argon atmosphere. Ethyl acetate and water were added thereto, followed by filtration through Celite(Registered Trademark). Two layers were separated, and an aqueous layer was subjected to extraction with ethyl acetate. A combined organic layer was washed with a saturated aqueous solution of sodium chloride, dried over anhydrous magnesium sulfate, and concentrated under reduced pressure. Purification was performed by silica gel column chromatography (hexane/ethyl acetate). The obtained solid was collected by filtration while washed with $iPr_2O$, and dried under reduced pressure to obtain 3-(5-ethenyl-3-methyl-2-oxo-2,3-dihydro-1H-benzimidazol-1-yl)piperidine-2,6-dione (D9-14, 559 mg) as a solid.

(Step 15)

To a mixture of 3-(5-ethenyl-3-methyl-2-oxo-2,3-dihydro-1H-benzimidazol-1-yl)piperidine-2,6-dione (D9-14, 373 mg), DOX (10 mL), and water (1 mL), sodium periodate (570 mg), osmium tetroxide (2.5% by weight tBuOH solution, 13.3 g), and 4-methylmorpholine N-oxide (88 mg) were added at room temperature, and stirred at room temperature for 30 minutes. Water was added to the reaction mixture, followed by extraction with ethyl acetate. An organic layer was washed with a saturated aqueous solution of sodium chloride, dried over anhydrous magnesium sulfate, and concentrated under reduced pressure. The obtained residue was purified by silica gel column chromatography (hexane/ethyl acetate) to obtain 1-(2,6-dioxopiperidin-3-yl)-3-methyl-2-oxo-2,3-dihydro-1H-benzimidazole-5-carbaldehyde (D9-15, 280 mg) as a solid.

(Step 16)

To a mixture of 1-(2,6-dioxopiperidin-3-yl)-3-methyl-2-oxo-2,3-dihydro-1H-benzimidazole-5-carbaldehyde (D9-15, 148 mg), tert-butyl (2S)-2-methylpiperazine-1-carboxylate (210 mg), $CH_2Cl_2$ (2 mL), and N-methyl-2-pyrrolidone (2 mL), acetic acid (31 μL) was added at room temperature, and stirred at room temperature for 30 minutes. Sodium triacetoxyborohydride (220 mg) was added thereto at room temperature and stirred overnight at room temperature. Water and a saturated aqueous solution of sodium bicarbonate were added thereto and stirred for a while, followed by extraction with ethyl acetate twice. A combined organic layer was washed with water and a saturated aqueous solution of sodium chloride and dried over anhydrous magnesium sulfate, and insoluble matter was filtered off, followed by concentration under reduced pressure. The obtained residue was purified by silica gel column chromatography ($CHCl_3$/iPrOH) to obtain tert-butyl (2S)-4-{[1-(2,6-dioxopiperidin-3-yl)-3-methyl-2-oxo-2,3-dihydro-1H-benzimidazol-5-yl]methyl}-2-methylpiperazine-1-carboxylate (D9-16, 224 mg) as a solid in a foam.

(Step 17)

To a mixture of tert-butyl (2S)-4-{[1-(2,6-dioxopiperidin-3-yl)-3-methyl-2-oxo-2,3-dihydro-1H-benzimidazol-5-yl]methyl}-2-methylpiperazine-1-carboxylate (D9-16, 200 mg) and $CH_2Cl_2$ (2 mL), hydrogen chloride (4 M DOX solution, 3 mL) was added, and stirred overnight at room temperature. $iPr_2O$ was added to the reaction mixture, and the resulting solid was collected by filtration, washed with $iPr_2O$, and dried under reduced pressure to obtain 3-(3-methyl-5-{[(3S)-3-methylpiperazin-1-yl]methyl}-2-oxo-2,3-dihydro-1H-benzimidazol-1-yl)piperidine-2,6-dione dihydrochloride (D9-17, 178 mg) as a solid.

(Step 18)

To a mixture of 4-[({(7M)-4-[(1S,4S)-5-(tert-butoxycarbonyl)-2,5-diazabicyclo[2.2.1]heptan-2-yl]-6-cyclopropyl-7-[6-fluoro-5-methyl-1-(oxan-2-yl)-1H-indazol-4-yl]-2-[(oxan-4-yl)oxy]quinazolin-8-yl}oxy)methyl]benzoic acid (D9-10, 65 mg), 3-(3-methyl-5-{[(3S)-3-methylpiperazin-1-yl]methyl}-2-oxo-2,3-dihydro-1H-benzimidazol-1-yl)piperidine-2,6-dione dihydrochloride (D9-17, 41 mg), DIPEA (50 μL), and DMF (1 mL), HATU (35 mg) was added under ice cooling, and stirred at room temperature for 1 hour. Ethyl acetate, water, and a saturated aqueous solution of sodium bicarbonate were added thereto to separate two layers. An aqueous layer was subjected to extraction with ethyl acetate, and a combined organic layer was washed twice with a saturated aqueous solution of sodium chloride and then dried over anhydrous sodium sulfate. Insoluble matter was filtered off, followed by concentration under reduced pressure. The

residue was purified by silica gel column chromatography ($CHCl_3$/iPrOH) to obtain tert-butyl (1S,4S)-5-{(7M)-6-cyclopropyl-8-({4-[(2S)-4-{[1-(2,6-dioxopiperidin-3-yl)-3-methyl-2-oxo-2,3-dihydro-1H-benzimidazol-5-yl]methyl}-2-methylpiperazine-1-carbonyl]phenyl}methoxy)-7-[6-fluoro-5-methyl-1-(oxan-2-yl)-1H-indazol-4-yl]-2-[(oxan-4-yl)oxy]quinazolin-4-yl}-2,5-diazabicyclo[2.2.1]heptane-2-carboxylate (D9-18, 80 mg) as a solid.

(Step 19)

To a mixture of tert-butyl (1S,4S)-5-{(7M)-6-cyclopropyl-8-({4-[(2S)-4-{[1-(2,6-dioxopiperidin-3-yl)-3-methyl-2-oxo-2,3-dihydro-1H-benzimidazol-5-yl]methyl}-2-methylpiperazine-1-carbonyl]phenyl}methoxy)-7-[6-fluoro-5-methyl-1-(oxan-2-yl)-1H-indazol-4-yl]-2-[(oxan-4-yl)oxy]quinazolin-4-yl}-2,5-diazabicyclo[2.2.1]heptane-2-carboxylate (D9-18, 300 mg) and $CH_2Cl_2$ (2 mL), trifluoroacetic acid (1.2 mL) was added under ice cooling, and stirred at room temperature for 2 hours. After concentration under reduced pressure, the residue was purified by ODS column chromatography (0.1% formic acid MeCN solution/0.1% aqueous formic acid solution), and water and a saturated aqueous solution of sodium bicarbonate were added to a fraction containing the compound of interest, followed by extraction with $CHCl_3$/iPrOH (5/1) twice. A combined organic layer was dried over anhydrous sodium sulfate. Insoluble matter was filtered off, followed by concentration under reduced pressure. The residue was purified again by ODS column chromatography (0.1% formic acid MeCN solution/0.1% aqueous formic acid solution), and water and a saturated aqueous solution of sodium bicarbonate were added to a fraction containing the compound of interest, followed by extraction with $CHCl_3$/iPrOH (5/1) twice. A combined organic layer was dried over anhydrous sodium sulfate. Insoluble matter was filtered off, followed by concentration under reduced pressure to obtain 3-(5-{[(3S)-4-{4-[({(7M)-6-cyclopropyl-4-[(1S,4S)-2,5-diazabicyclo[2.2.1]heptan-2-yl]-7-(6-fluoro-5-methyl-1H-indazol-4-yl)-2-[(oxan-4-yl)oxy]quinazolin-8-yl} oxy)methyl]benzoyl} -3-methylpiperazin-1-yl]methyl}-3-methyl-2-oxo-2,3-dihydro-1H-benzimidazol-1-yl)piperidine-2,6-dione (D9, 133 mg) as a solid.

Synthesis of drug D10

(Step 1)

A mixture of 1-(6-bromo-1-methyl-1H-indazol-3-yl)-1,3-diazinane-2,4-dione (600 mg), potassium vinyltrifluoroborate (995 mg), $PdCl_2$(dppf)·$CH_2Cl_2$ (152 mg), cesium carbonate (1.21 g), DOX (12.5 mL), and water (3 mL) was stirred at 80°C for 3 hours in an argon atmosphere. After cooling to room temperature, water was added thereto, followed by extraction with ethyl acetate. An organic layer was washed with a saturated aqueous solution of sodium chloride, dried over anhydrous magnesium sulfate, and concentrated under reduced pressure. The obtained residue was purified by silica gel column chromatography (hexane/ethyl acetate, $CHCl_3$/MeOH) to obtain 1-(6-ethenyl-1-methyl-1H-indazol-3-yl)-1,3-diazinane-2,4-dione (D10-1, 546 mg) as a solid.

(Step 2)

1-(6-Ethenyl-1-methyl-1H-indazol-3-yl)-1,3-diazinane-2,4-dione (D10-1, 1.44 g) was dissolved in a mixed solvent of MeOH (30 mL) and $CH_2Cl_2$ (30 mL) at room temperature and stirred for 1.5 hours while bubbled with ozone gas under cooling with dry ice-acetone refrigerant. Dimethyl sulfide (1.2 mL) was added thereto at the same temperature, then heated to room temperature, and stirred overnight. The reaction solution was concentrated under reduced pressure, and the residue was washed with a mixed solvent of ethyl acetate and MeOH to obtain 3-(2,4-dioxo-1,3-diazinan-1-yl)-1-methyl-1H-indazole-6-carbaldehyde (D10-2, 527 mg) as a solid.

(Step 3)

To a mixture of 3-(2,4-dioxo-1,3-diazinan-1-yl)-1-methyl-1H-indazole-6-carbaldehyde (D10-2, 50 mg), acetic acid (11 μL), $CH_2Cl_2$ (1 mL), and N-methyl-2-pyrrolidone (1 mL), tert-butyl (2S)-2-methylpiperazine-1-carboxylate (115 mg) was added, and stirred at room temperature for 30 minutes. Sodium triacetoxyborohydride (120 mg) was added to the reaction mixture and stirred at room temperature for 16 hours. After dilution with ethyl acetate, a saturated aqueous solution of sodium bicarbonate was added thereto and stirred for a while to separate an aqueous layer. The aqueous layer was subjected to extraction with ethyl acetate, and a combined organic layer was washed twice with a saturated aqueous solution of sodium chloride and dried over anhydrous sodium sulfate. Insoluble matter was filtered off, and the filtrate was concentrated under reduced pressure. The residue was purified by silica gel column chromatography ($CHCl_3$/MeOH) to obtain tert-butyl (2S)-4-{[3-(2,4-dioxo-1,3-diazinan-1-yl)-1-methyl-1H-indazol-6-yl]methyl}-2-methylpiperazine-1-carboxylate (D10-3, 74 mg) as a solid in a foam.

(Step 4)

To a mixture of tert-butyl (2S)-4-{[3-(2,4-dioxo-1,3-diazinan-1-yl)-1-methyl-1H-indazol-6-yl]methyl}-2-methylpiperazine-1-carboxylate (D10-3, 74 mg) and $CH_2Cl_2$ (2 mL), hydrogen chloride (4 M DOX solution, 2 mL) was added, and stirred at room temperature for 6 hours. Concentration was performed under reduced pressure to obtain 1-(1-methyl-6-{[(3S)-3-methylpiperazin-1-yl]methyl}-1H-indazol-3-yl)-1,3-diazinane-2,4-dione n-hydrochloride (D10-4, 75 mg) as a solid.

(Step 5)

To a mixture of 4-[({(7M)-4-[(1S,4S)-5-(tert-butoxycarbonyl)-2,5-diazabicyclo[2.2.1]heptan-2-yl]-6-cyclopropyl-7-[6-fluoro-5-methyl-1-(oxan-2-yl)-1H-indazol-4-yl]-2-[(oxan-4-yl)oxy]quinazolin-8-yl}oxy)methyl]benzoic acid (D9-10, 130 mg), 1-(1-methyl-6-{[(3S)-3-methylpiperazin-1-yl]methyl}-1H-indazol-3-yl)-1,3-diazinane-2,4-dione n-hydrochloride (D10-4, 76 mg), DIPEA (130 μL), and DMF (1.3 mL), HATU (78 mg) was added, and stirred overnight at room temperature. Water and a saturated aqueous solution of sodium bicarbonate were added to the reaction mixture and stirred for a while, and the resulting solid was collected by filtration to obtain tert-butyl (1S,4S)-5-1(7M)-6-cyclopropyl-8-({4-[(2S)-4-{[3-(2,4-dioxo-1,3-diazinan-1-yl)-1-methyl-1H-indazol-6-yl]methyl}-2-methylpiperazine-1-carbonyl] phenyl}methoxy)-7-[6-fluoro-5-methyl-1-(oxan-2-yl)-1H-indazol-4-yl]-2-[(oxan-4-yl)oxy]quinazolin-4-yl}-2,5-diazabicyclo[2.2.1]heptane-2-carboxylate (D10-5, 164 mg) as a solid.

(Step 6)

To a $CH_2Cl_2$ (3 mL) solution of tert-butyl (1S,4S)-5-{(7M)-6-cyclopropyl-8-({4-[(2S)-4-{[3-(2,4-dioxo-1,3-diazinan-1-yl)-1-methyl-1H-indazol-6-yl]methyl}-2-methylpiperazine-1-carbonyl]phenyl}methoxy)-7-[6-fluoro-5-methyl-1-(oxan-2-yl)-1H-indazol-4-yl]-2-[(oxan-4-yl)oxy]quinazolin-4-yl}-2,5-diazabicyclo[2.2.1]heptane-2-carboxylate (D10-5, 164 mg), trifluoroacetic acid (1 mL) was added under ice cooling, and stirred at room temperature for 4 hours. After concentration under reduced pressure, the residue was purified by ODS column chromatography (0.1% formic acid MeCN solution/0.1% aqueous formic acid solution) to collect a fraction containing the compound of interest, which was concentrated under reduced pressure. The obtained residue was dissolved in $CH_2Cl_2$, and hydrogen chloride (4 M ethyl acetate solution, 300 μL) was added thereto under ice cooling and stirred for a while. Concentration was performed under reduced pressure to obtain 1-(6-{[(3S)-4-{4-[({(7M)-6-cyclopropyl-4-[(1S,4S)-2,5-diazabicyclo[2.2.1]heptan-2-yl]-7-(6-fluoro-5-methyl-1H-indazol-4-yl)-2-[(oxan-4-yl)oxy]quinazolin-8-yl}oxy)methyl]benzoyl}-3-methylpiperazin-1-yl] methyl}-1-methyl-1H-indazol-3-yl)-1,3-diazinane-2,4-dione n-hydrochloride (D10, 71 mg) as a solid in a foam.

Synthesis of drug D11

(Step 1)

To a mixture of tert-butyl (1S,4S)-5-[(7M)-6-cyclopropyl-2-(ethanesulfonyl)-7-[6-fluoro-5-methyl-1-(oxan-2-yl)-1H-indazol-4-yl]-8-{[4-(methoxycarbonyl)phenyl]methoxy}quinazolin-4-yl]-2,5-diazabicyclo[2.2.1]heptane-2-carboxylate (D9-8, 300 mg), (2S)-2-methoxypropan-1-ol (40 mg), and THF (4 mL), tBuOK (85 mg) was added under cooling in an ice/MeOH bath, and stirred at the same temperature for 30 minutes in an argon atmosphere. The reaction was terminated by the addition of ice water and an aqueous ammonium chloride solution to the reaction mixture. After extraction with ethyl acetate twice, a combined organic layer was washed with a saturated aqueous solution of sodium chloride and dried over anhydrous magnesium sulfate. After concentration under reduced pressure, the residue was purified by silica gel column chromatography (basic silica gel, hexane/ethyl acetate) to obtain tert-butyl (1S,4S)-5-{(7M)-6-cyclopropyl-7-[6-fluoro-5-methyl-1-(oxan-2-yl)-1H-indazol-4-yl]-8-{[4-(methoxycarbonyl)phenyl]methoxy}-2-[(2S)-2-methoxypropoxy]quinazolin-4-yl}-2,5-diazabicyclo[2.2.1]heptane-2-carboxylate (D11-1, 255 mg) as a solid in a foam.

(Step 2)

To a mixture of tert-butyl (1S,4S)-5-{(7M)-6-cyclopropyl-7-[6-fluoro-5-methyl-1-(oxan-2-yl)-1H-indazol-4-yl]-8-{[4-(methoxycarbonyl)phenyl]methoxy}-2-[(2S)-2-methoxypropoxy]quinazolin-4-yl}-2,5-diazabicyclo[2.2.1]heptane-2-carboxylate (D11-1, 252 mg), MeOH (3.4 mL), and THF (1.7 mL), an aqueous sodium hydroxide solution (1 M, 1.7 mL) was added under ice cooling, and stirred at room temperature for 6 hours. Hydrochloric acid (1 M, 1.77 mL) and ice water were added thereto, followed by extraction with $CHCl_3$/MeOH (10/1) twice. A combined organic layer was dried over anhydrous magnesium sulfate. The solvent was distilled off under reduced pressure to obtain 4-[({(7M)-4-[(1S,4S)-5-(tert-butoxycarbonyl)-2,5-diazabicyclo[2.2.1]heptan-2-yl]-6-cyclopropyl-7-[6-fluoro-5-methyl-1-(oxan-2-yl)-1H-indazol-4-yl]-2-[(2S)-2-methoxypropoxy]quinazolin-8-yl}oxy)methyl]benzoic acid (D11-2, 250 mg) as a solid in a foam.

(Step 3)

To a mixture of 4-[({(7M)-4-[(1S,4S)-5-(tert-butoxycarbonyl)-2,5-diazabicyclo[2.2.1]heptan-2-yl]-6-cyclopropyl-7-[6-fluoro-5-methyl-1-(oxan-2-yl)-1H-indazol-4-yl]-2-[(2S)-2-methoxypropoxy]quinazolin-8-yl}oxy)methyl]benzoic acid (D11-2, 247 mg), 3-(3-methyl-5-{[(3S)-3-methylpiperazin-1-yl]methyl}-2-oxo-2,3-dihydro-1H-benzimidazol-1-yl)piperidine-2,6-dione dihydrochloride (D9-17, 160 mg), DIPEA (0.3 mL), and DMF (4 mL), HATU (230 mg) was added under cooling in an ice/MeOH bath, and stirred at room temperature for 2 hours in an argon atmosphere. Ice and a saturated aqueous solution of ammonium chloride were poured to the reaction mixture, followed by extraction with ethyl acetate twice. A combined organic layer was washed with a saturated aqueous solution of sodium chloride and then dried over anhydrous magnesium sulfate. After concentration under reduced pressure, the obtained residue was purified by silica gel column chromatography ($CHCl_3$/iPrOH) to obtain tert-butyl (1S,4S)-5-{(7M)-6-cyclopropyl-8-({4-[(2S)-4-{[1-(2,6-dioxopiperidin-3-yl)-3-methyl-2-oxo-2,3-dihydro-1H-benzimidazol-5-yl]methyl}-2-methylpiperazine-1-carbonyl]phenyl}methoxy)-7-[6-fluoro-5-methyl-1-(oxan-2-yl)-1H-indazol-4-yl]-2-[(2S)-2-methoxypropoxy]quinazolin-4-yl}-2,5-diazabicyclo [2.2.1]heptane-2-carboxylate (D11-3, 273 mg) as a solid.

(Step 4)

To tert-butyl (1S,4S)-5-{(7M)-6-cyclopropyl-8-({4-[(2S)-4-{[1-(2,6-dioxopiperidin-3-yl)-3-methyl-2-oxo-2,3-dihydro-1H-benzimidazol-5-yl]methyl}-2-methylpiperazine-1-carbonyl]phenyl}methoxy)-7-[6-fluoro-5-methyl-1-(oxan-2-yl)-1H-indazol-4-yl]-2-[(2S)-2-methoxypropoxy]quinazolin-4-yl}-2,5-diazabicyclo[2.2.1]heptane-2-carboxylate (D11-3, 268 mg), $CH_2Cl_2$ (6 mL) was added, and trifluoroacetic acid (2 mL) was added thereto while cooled and stirred in an ice/MeOH bath, and stirred at room temperature for 2 hours in an argon atmosphere. After concentration under reduced pressure, ice water and a saturated aqueous solution of sodium bicarbonate were added to the residue. After extraction with $CHCl_3$/iPrOH (10/1) twice, a combined organic layer was washed with a saturated aqueous solution of sodium chloride and dried over anhydrous magnesium sulfate. After concentration under reduced pressure, hexane/ethyl acetate (3/1, approximately 5 mL) was added to the obtained solid, and the resulting precipitate was collected by filtration while washed with the same solvent, and dried under reduced pressure to obtain 3-(5-{[(3S)-4-{4-[({(7M)-6-cyclopropyl-4-[(1S,4S)-2,5-diazabicyclo[2.2.1]heptan-2-yl]-7-(6-fluoro-5-methyl-1H-indazol-4-yl)-2-[(2S)-2-methoxypropoxy] quinazolin-8-yl}oxy)methyl]benzoyl}-3-methylpiperazin-1-yl]methyl}-3-methyl-2-oxo-2,3-dihydro-1H-benzimidazol-1-yl) piperidine-2,6-dione (D11, 198 mg) as a solid.

Synthesis of drug D12

(Step 1)

To a THF (32 mL)/water (32 mL) solution of (2R)-2-amino-2-[4-(1H-1,2,4-triazol-1-yl)phenyl]ethan-1-ol monohydrochloride (3.27 g), sodium carbonate (3.39 g) and benzyl chloroformate (3.00 g) were added at 0°C, and stirred at 25°C for 5 hours. Water (50 mL) was added to the reaction mixture, followed by extraction with ethyl acetate three times. A combined organic layer was washed with a saturated aqueous solution of sodium chloride, dried over anhydrous sodium sulfate, filtered, and concentrated under reduced pressure. Ethyl acetate (15 mL) was added to the obtained residue and stirred at 25°C for 30 minutes. The resulting solid was collected by filtration and dried under reduced pressure to obtain benzyl {(1R)-2-hydroxy-1-[4-(1H-1,2,4-triazol-1-yl)phenyl]ethyl}carbamate (D12-1, 3.08 g) as a solid.

(Step 2)

To a DMF (30 mL) solution of benzyl {(1R)-2-hydroxy-1-[4-(1H-1,2,4-triazol-1-yl)phenyl]ethyl}carbamate (D12-1, 3.08 g), 1H-tetrazole (3.19 g) and di-tert-butyl N,N-diisopropylphosphoramidite (12.6 g) were added, and stirred at 25°C for 16 hours. $CH_2Cl_2$ (10 mL) was added to the reaction mixture, and m-chloroperbenzoic acid (3.69 g, purity: 85%) was then added thereto at 0°C and stirred at 25°C for 16 hours. A 10% aqueous sodium sulfite solution (150 mL) was added to the reaction mixture at 0°C over 30 minutes, followed by extraction with $CH_2Cl_2$ three times. A combined organic layer was washed twice with a saturated aqueous solution of sodium chloride, dried over anhydrous sodium sulfate, filtered, and concentrated under reduced pressure. The obtained residue was purified by silica gel column chromatography (petroleum ether/ethyl acetate) to obtain benzyl {(1R)-2-[(di-tert-butoxyphosphoryl)oxy]-1-[4-(1H-1,2,4-triazol-1-yl)phenyl]ethyl}carbamate (D12-2, 2.92 g) as a solid.

(Step 3)

To a MeOH (15 mL) solution of benzyl {(1R)-2-[(di-tert-butoxyphosphoryl)oxy]-1-[4-(1H-1,2,4-triazol-1-yl)phenyl]ethyl} carbamate (D12-2, 1.5 g), sodium bicarbonate (713 mg) and 10% Pd/C (958 mg) were added. Stirring was performed at 25°C for 4 hours in a hydrogen atmosphere. The reaction mixture was filtered through Celite(Registerd Trademark), and the filtrate was concentrated under reduced pressure to obtain (2R)-2-amino-2-[4-(1H-1,2,4-triazol-1-yl) phenyl]ethyl di-tert-butyl phosphate (D12-3, 1.1 g) as an oil.

(Step 4)

To a DMF (4.5 mL) solution of (2R)-2-amino-2-[4-(1H-1,2,4-triazol-1-yl)phenyl]ethyl di-tert-butyl phosphate (D12-3, 1.6 g), N-(tert-butoxycarbonyl)-L-valyl-(4R)-4-hydroxy-L-proline (1.33 g), DIPEA (1.41 mL), and HATU (2.30 g) were added, and stirred at 25°C for 4 hours. Water (50 mL) was added to the reaction mixture, followed by extraction with ethyl acetate three times. A combined organic layer was washed with a saturated aqueous solution of sodium chloride, dried over anhydrous sodium sulfate, filtered, and concentrated under reduced pressure. The obtained residue was purified by ODS column chromatography (MeCN/0.05% aqueous ammonium bicarbonate solution) to obtain N-(tert-butoxycarbonyl)-L-valyl-(4R)-N-{(1R)-2-[(di-tert-butoxyphosphoryl)oxy]-1-[4-(1H-1,2,4-triazol-1-yl)phenyl]ethyl}-4-hydroxy-L-prolinamide (D12-4, 1.04 g) as a solid.

(Step 5)

To a 1,1,1,3,3,3-hexafluoropropan-2-ol (10 mL) solution of N-(tert-butoxycarbonyl)-L-valyl-(4R)-N-{(1R)-2-[(di-tert-butoxyphosphoryl)oxy]-1-[4-(1H-1,2,4-triazol-1-yl)phenyl]ethyl}-4-hydroxy-L-prolinamide (D12-4, 1.04 g), trifluoroacetic acid (1.09 mL) was added, and stirred at 25°C for 1 hour. Concentration was performed under reduced pressure to obtain L-valyl-(4R)-4-hydroxy-N-{(1R)-2-(phosphonooxy)-1-[4-(1H-1,2,4-triazol-1-yl)phenyl]ethyl}-L-prolinamide n-trifluoroacetate(D12-5, 1 g) as a solid.

(Step 6)

To a mixture of methyl tert-butyl ether (30 mL) and water (30 mL), sodium azide (880 mg) was added at 25°C, and cooled in ice. A MeCN (6 mL) solution of 1-(fluorosulfonyl)-2,3-dimethyl-1H-imidazol-3-ium trifluoromethanesulfonate (3 g) was added dropwise thereto at 0°C over 2 minutes, and the mixture was stirred at 0°C for 30 minutes. A saturated aqueous solution of sodium chloride (20 mL) was slowly added to the reaction mixture and then left standing at 25°C for 10 minutes. After separation between an organic layer and an aqueous layer, the organic layer was used as fluorosulfonylazide (methyl tert-butyl ether solution containing approximately 1.14 g, 30 mL) in subsequent reaction.

To a DMSO (5 mL) solution of L-valyl-(4R)-4-hydroxy-N- {(1R)-2-(phosphonooxy)-1-[4-(1H-1,2,4-triazol-1-yl) phenyl]ethyl} -L-prolinamide n-trifluoroacetate (D12-5, 729 mg), potassium bicarbonate (441 mg) and the prepared fluorosulfonylazide solution (21.6 mL) were added, and stirred at 25°C for 16 hours. Water was added to the reaction mixture, followed by extraction with ethyl acetate three times. A combined organic layer was washed with a saturated aqueous solution of sodium chloride, dried over anhydrous sodium sulfate, filtered, and concentrated under reduced pressure. The obtained residue was purified by ODS column chromatography (MeCN/0.05% aqueous TFA solution) to obtain (4R)-1-[(2S)-2-azido-3-methylbutanoyl]-4-hydroxy-N-{(1R)-2-(phosphonooxy)-1-[4-(1H-1,2,4-triazol-1-yl)phenyl] ethyl}-L-prolinamide (D12-6, 685 mg) as a solid.

(Step 7)

To a THF (1 mL)/MeOH (1 mL) solution of tert-butyl (3S)-3-[{(7M)-6-cyclopropyl-8-[(4-ethynylphenyl)methoxy]-7-[6-fluoro-5-methyl-1-(oxan-2-yl)-1H-indazol-4-yl]-2-[(2S)-2-methoxypropoxy]quinazolin-4-yl}(methyl)amino]pyrrolidine-1-carboxylate (80 mg), 4-methylbenzene-1-sulfonic acid monohydrate (40 mg) was added at room temperature, and stirred at 60°C for 7 hours. The reaction mixture was concentrated under reduced pressure, and a saturated aqueous solution of sodium bicarbonate (1 mL), $CH_2Cl_2$ (2 mL), and di-t-butyl dicarbonate (30 mg) were added to the obtained residue at room temperature and stirred at room temperature for 1 hour. After separation between an organic layer and an aqueous layer, the organic layer was dried over anhydrous magnesium sulfate, filtered, and concentrated under reduced pressure. The obtained residue was purified by silica gel column chromatography (basic silica gel, hexane/ethyl acetate) to obtain tert-butyl (3S)-3-[{(7M)-6-cyclopropyl-8-[(4-ethynylphenyl)methoxy]-7-(6-fluoro-5-methyl-1H-indazol-4-yl)-2-[(2S)-2-methoxypropoxy]quinazolin-4-yl}(methyl)amino]pyrrolidine-1-carboxylate (D12-7, 63 mg) as an oil.

(Step 8)

A mixture of tert-butyl (3S)-3-[{(7M)-6-cyclopropyl-8-[(4-ethynylphenyl)methoxy]-7-(6-fluoro-5-methyl-1H-indazol-4-yl)-2-[(2S)-2-methoxypropoxy]quinazolin-4-yl}(methyl)amino]pyrrolidine-1-carboxylate (D12-7, 200 mg), (4R)-1-[(2S)-2-azido-3-methylbutanoyl]-4-hydroxy-N-{(1R)-2-(phosphonooxy)-1-[4-(1H-1,2,4-triazol-1-yl)phenyl]ethyl}-L-prolinamide (D12-6, 199 mg), copper(I) iodide (156 mg), tris[(1-benzyl-1H-1,2,3-triazol-4-yl)methyl]amine (144 mg), sodium ascorbate (162 mg), THF (1.5 mL), water (1 mL), and tBuOH (1 mL) was stirred at 25°C for 16 hours in a nitrogen atmosphere. The reaction mixture was filtered, and the filtrate was purified by ODS column chromatography (MeCN/0.05% aqueous TFA solution) to obtain tert-butyl (3S)-3-[{(7M)-6-cyclopropyl-7-(6-fluoro-5-methyl-1H-indazol-4-yl)-8-{[4-(1-{(2S)-1-[(2S,4R)-4-hydroxy-2-({(1R)-2-(phosphonooxy)-1-[4-(1H-1,2,4-triazol-1-yl)phenyl]ethyl}carbamoyl)pyrrolidin-1-yl]-3-methyl-1-oxobutan-2-yl}-1H-1,2,3-triazol-4-yl)phenyl]methoxy}-2-[(2S)-2-methoxypropoxy]quinazolin-4-yl}(methyl)amino]pyrrolidine-1-carboxylate (D12-8, 290 mg) as a solid.

(Step 9)

To a MeOH (3 mL) solution of tert-butyl (3S)-3-[{(7M)-6-cyclopropyl-7-(6-fluoro-5-methyl-1H-indazol-4-yl)-8-{[4-(1-{(2S)-1-[(2S,4R)-4-hydroxy-2-({(1R)-2-(phosphonooxy)-1-[4-(1H-1,2,4-triazol-1-yl)phenyl]ethyl}carbamoyl)pyrrolidin-1-yl]-3-methyl-1-oxobutan-2-yl}-1H-1,2,3-triazol-4-yl)phenyl]methoxy}-2-[(2S)-2-methoxypropoxy]quinazolin-4-yl}(methyl)amino]pyrrolidine-1-carboxylate (D12-8, 280 mg), methanesulfonic acid (111 μL) was added, and stirred at 25°C for 16 hours. The reaction mixture was concentrated under reduced pressure, and the residue was purified by ODS column chromatography (MeCN/0.05% aqueous TFA solution) to obtain (2R)-2-({(4R)-1-[(2S)-2-{4-[4-({[(7M)-6-cyclopropyl-7-(6-fluoro-5-methyl-1H-indazol-4-yl)-2-[(2S)-2-methoxypropoxy]-4-{methyl[(3S)-pyrrolidin-3-yl]amino}quinazolin-8-yl]oxy}methyl)phenyl]-1H-1,2,3-triazol-1-yl}-3-methylbutanoyl]-4-hydroxy-L-prolyl}amino)-2-[4-(1H-1,2,4-triazol-1-yl)phenyl]ethyl dihydrogen phosphate n-trifluoroacetate (D12, 290 mg) as a solid.

The chemical structural formula and physicochemical data of the compound obtained in each step are shown in tables below.

In the tables below, the following abbreviations may be used.

Ex: Example No., CHEM: chemical structural formula (a compound with "*" in the chemical structural formula represents that the compound has single axial chirality or chiral center), n HCl: n-hydrochloride (a compound with Example No. represents being monohydrochloride to tetrahydrochloride), n HCOOH: n-formate (a compound with Example No. represents being a free form, or monoformate to triformate), n $CF_3COOH$: n-trifluoroacetate (a compound with Example No. represents being a free form, or monotrifluoroacetate to tritrifluoroacetate), DAT: physicochemical data, ESI+: m/z value in mass spectrometry (ionization method: ESI, $[M+H]^+$ unless otherwise specified), ESI-: m/z value in mass spectrometry (ionization method: ESI, $[M-H]^-$ unless otherwise specified), NMR: peak δ value (ppm) in $^1$H-NMR (500 MHz) at 27°C in DMSO-$d_6$, NMR (24°C): peak δ value (ppm) in $^1$H-NMR (400 MHz) at 24°C in DMSO-$d_6$, NMR (90°C): peak δ value (ppm) in $^1$H-NMR (500 MHz) at 90°C in DMSO-$d_6$, NMR (100°C): peak δ value (ppm) in $^1$H-NMR (500 MHz) at 100°C in DMSO-$d_6$, J: coupling constant, s: singlet (spectrum), d: doublet (spectrum), dd: double doublet (spectrum), t: triplet (spectrum), tt: triple triplet (spectrum), dq: double quadruplet (spectrum), q: quadruplet (spectrum), m: multiplet (spectrum), br: broad (spectrum) (e.g.: br s).

[Table 1-1]

| Ex | CHEM |
|---|---|
| D1 | 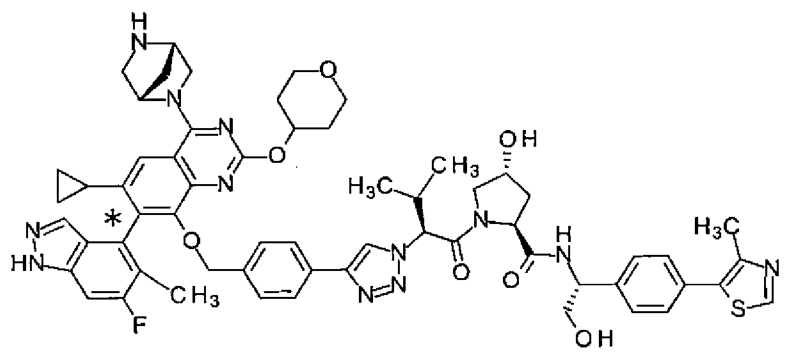 |

(continued)

| Ex | CHEM |
|---|---|
| D2 | 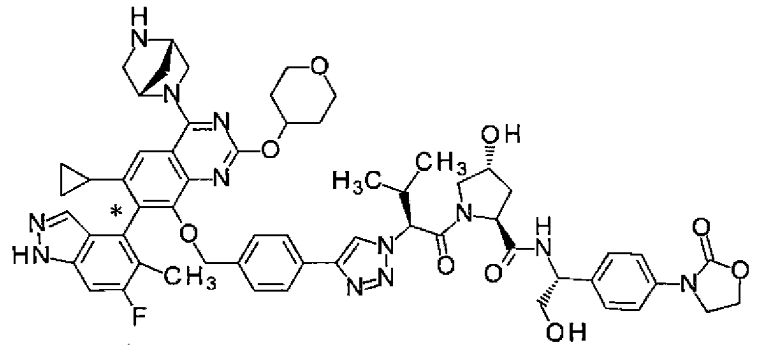 |
| D3 | 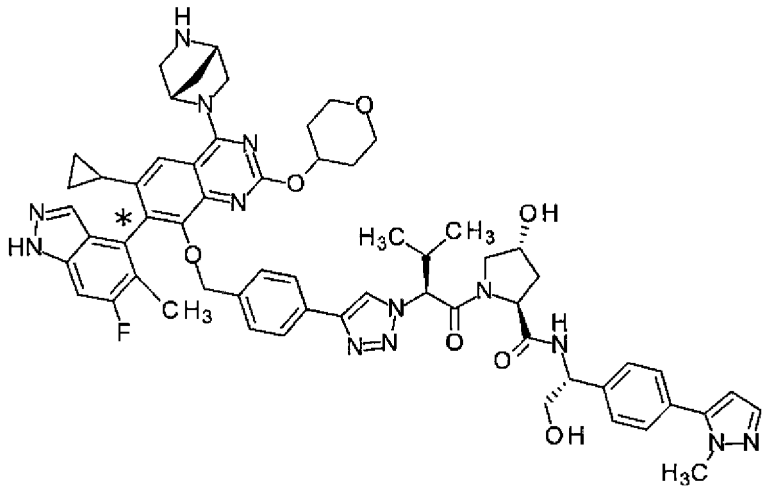 |
| D4 | 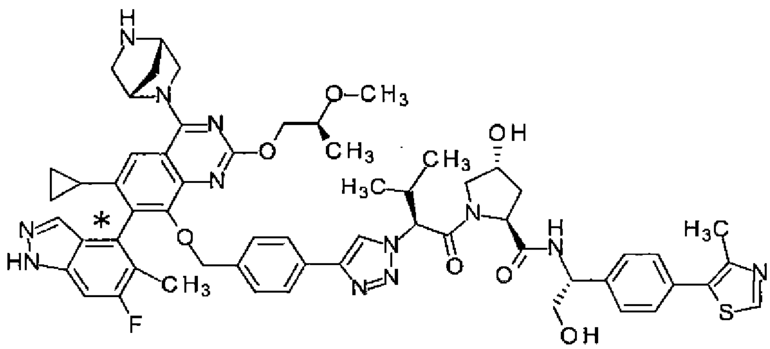 |

[Table 1-2]

| Ex | CHEM |
|---|---|
| D5 | 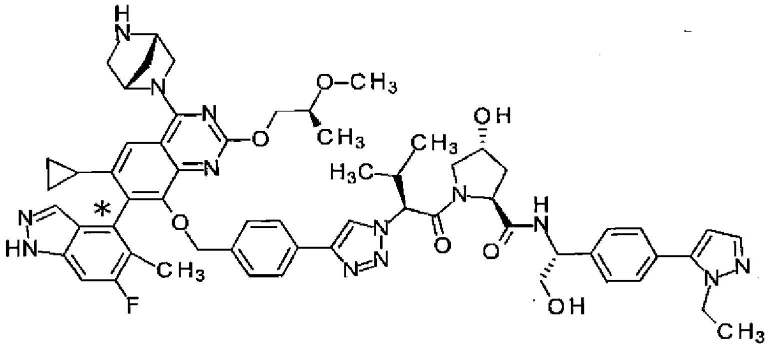 |
| D6 | 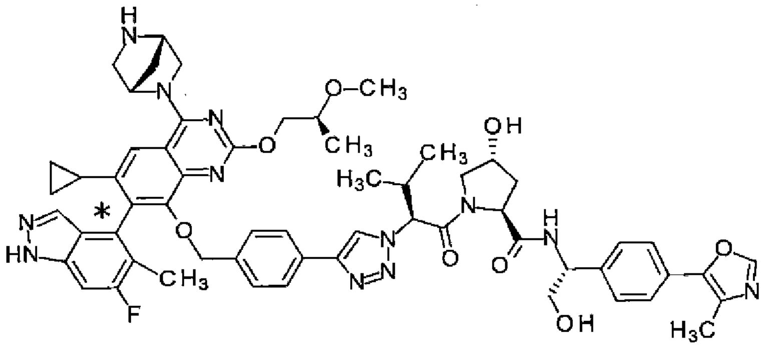 |
| D7-1 | 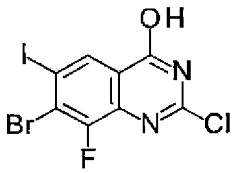 |
| D7-2 | 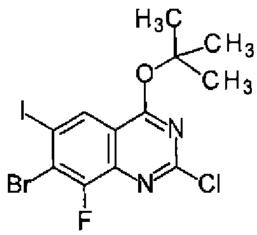 |
| D7-3 | 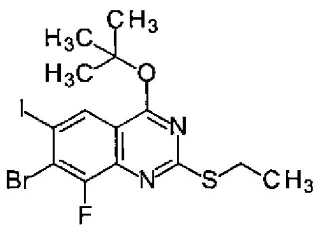 |

[Table 1-3]

| Ex | CHEM |
| --- | --- |
| D7-4 | 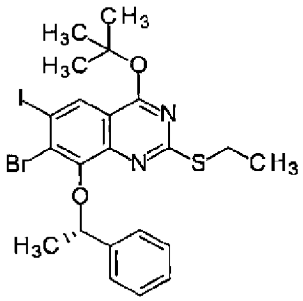 |
| D7-5 | 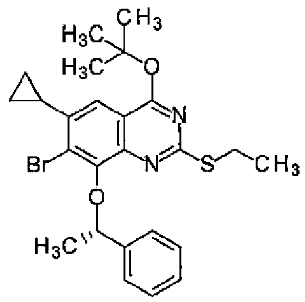 |
| D7-6 | 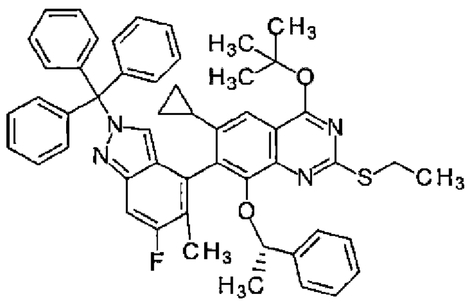 |
| D7-7 | 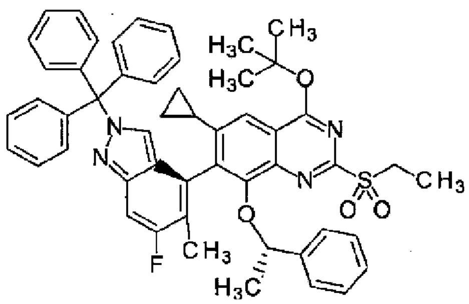 |
| D7-8 | 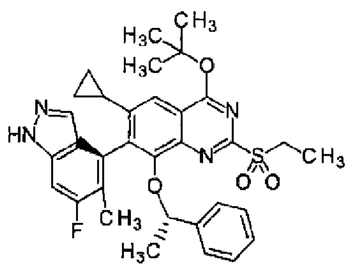 |

[Table 1-4]

| Ex | CHEM |
|---|---|
| D7-9 | 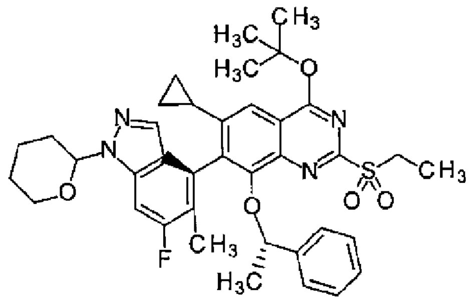 |
| D7-10 | 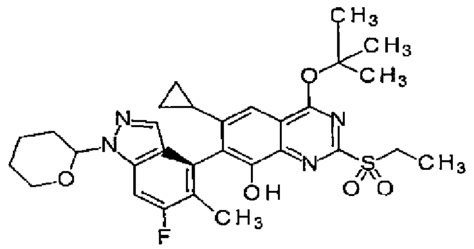 |
| D7-11 | 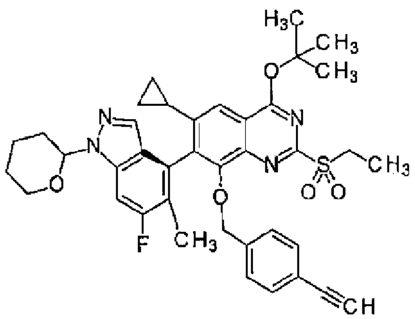 |
| D7-12 | 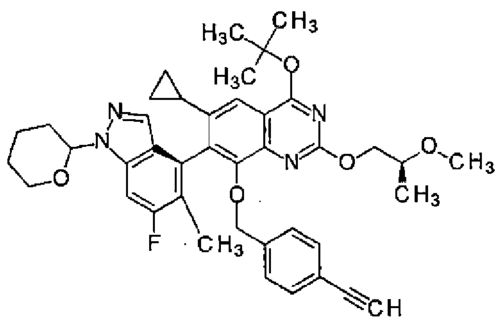 |
| D7-13 | 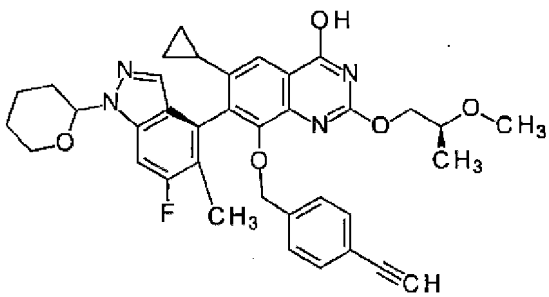 |

[Table 1-5]

| Ex | CHEM |
|---|---|
| D7-14 | 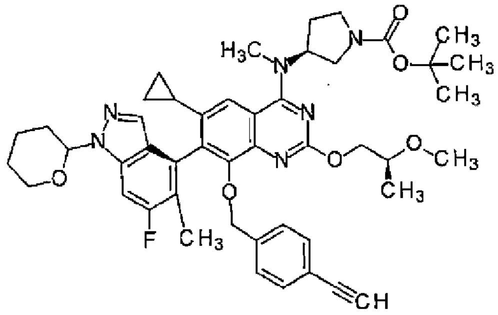 |
| D7-15 | 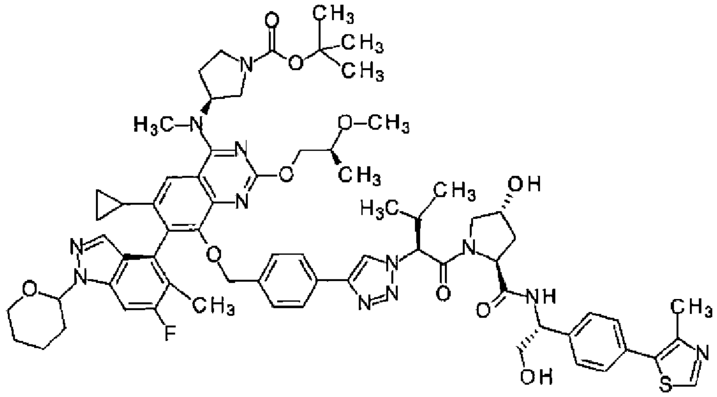 |
| D7 | 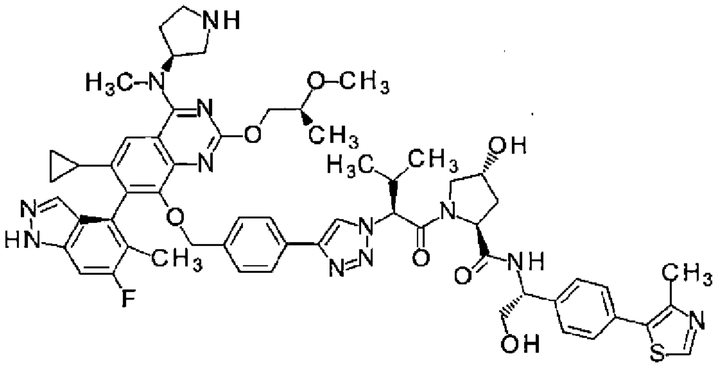 |
| D8-1 | 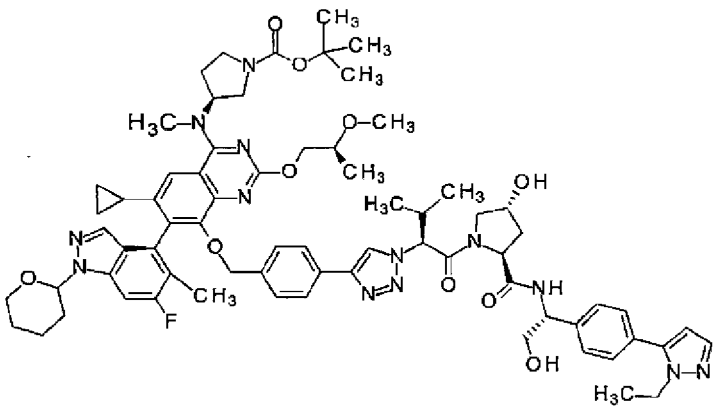 |

[Table 1-6]

| Ex | CHEM |
|---|---|
| D8 | 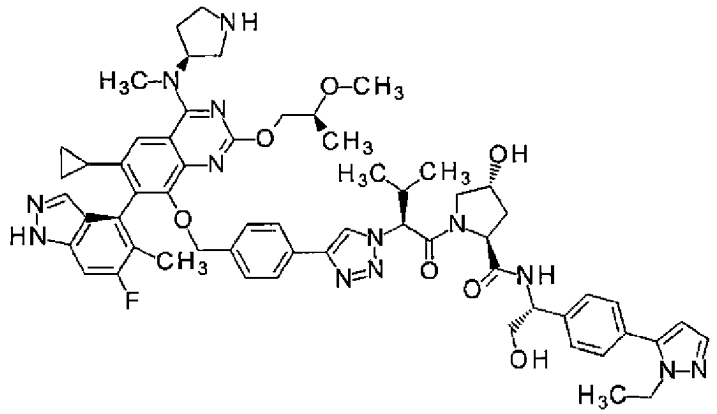 |
| D 9-1 | 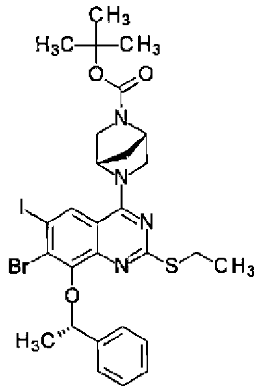 |
| D9-2 | 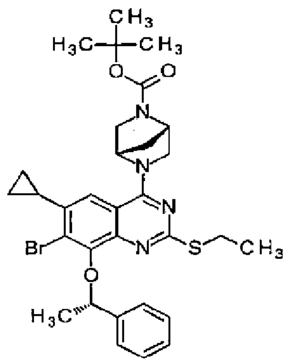 |
| D9-3 | 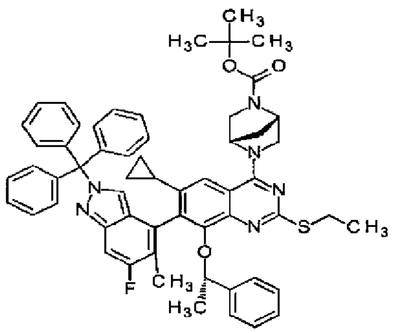 |

[Table 1-7]

| Ex | CHEM |
|---|---|
| D9-4 | 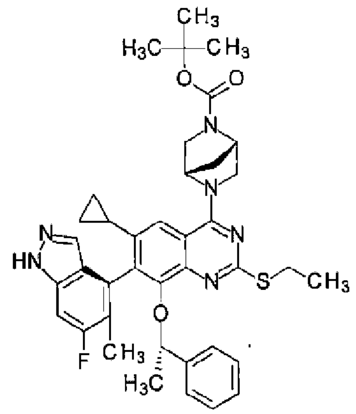 |
| D9-5 | 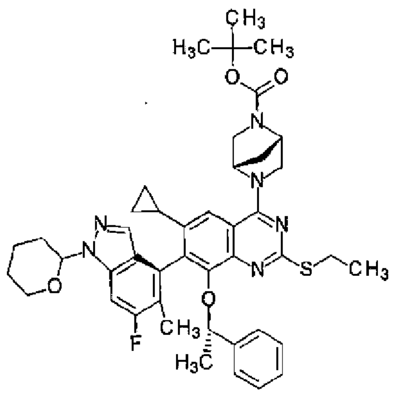 |
| D9-6 | 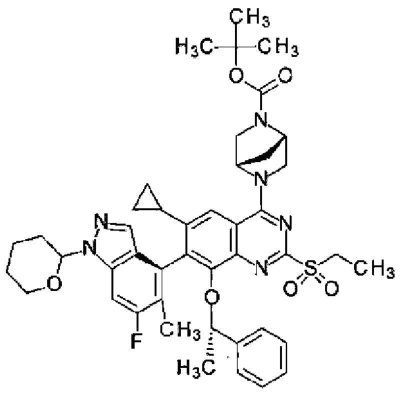 |
| D9-7 | 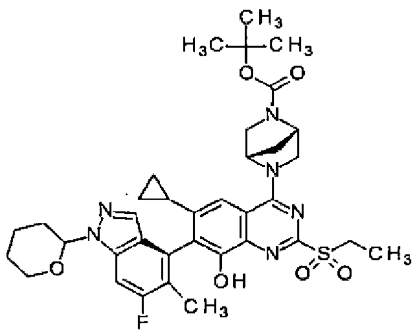 |

[Table 1-8]

| Ex | CHEM |
|---|---|
| D9-8 | 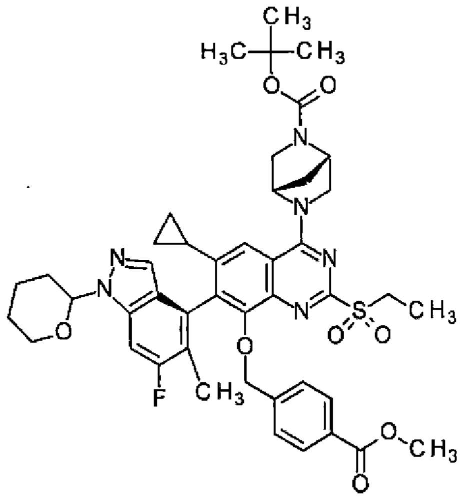 |
| D9-9 | 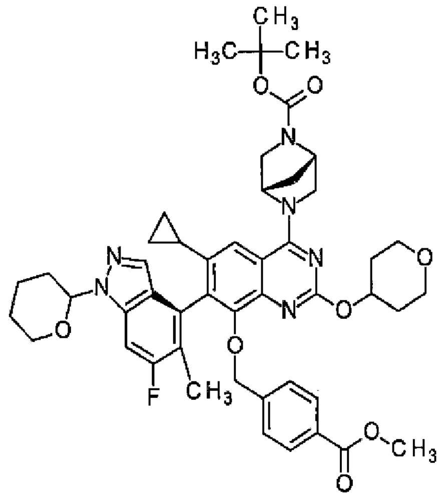 |
| D9-10 | 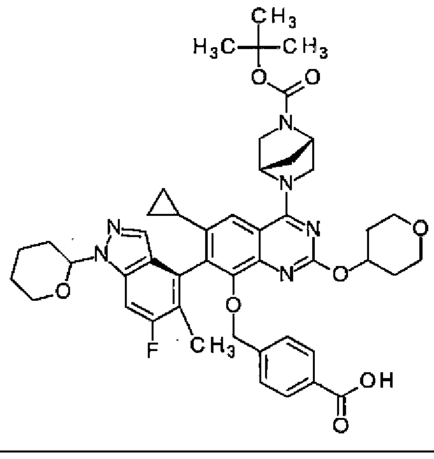 |

[Table 1-9]

| Ex | CHEM |
|---|---|
| D9-11 | 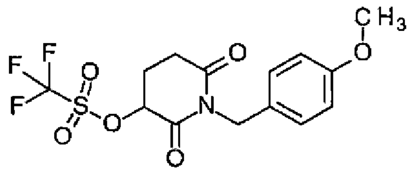 |
| D9-12 | 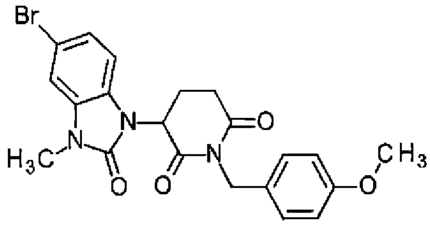 |
| D9-13 | 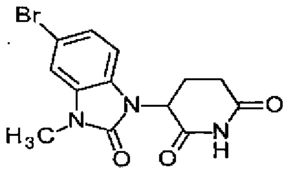 |
| D9-14 | 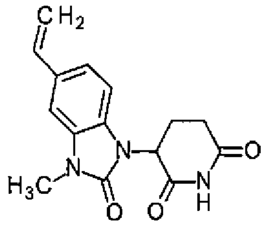 |
| D9-15 | 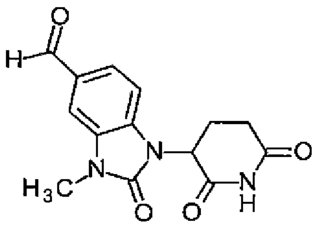 |
| D9-16 | 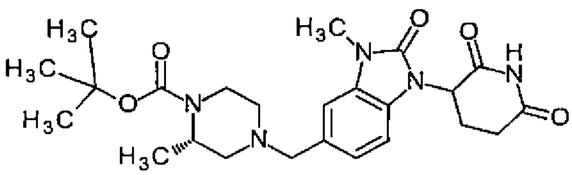 |
| D9-17 | 2 HCl 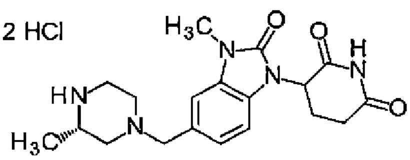 |

[Table 1-10]

| Ex | CHEM |
|---|---|
| D9-18 | 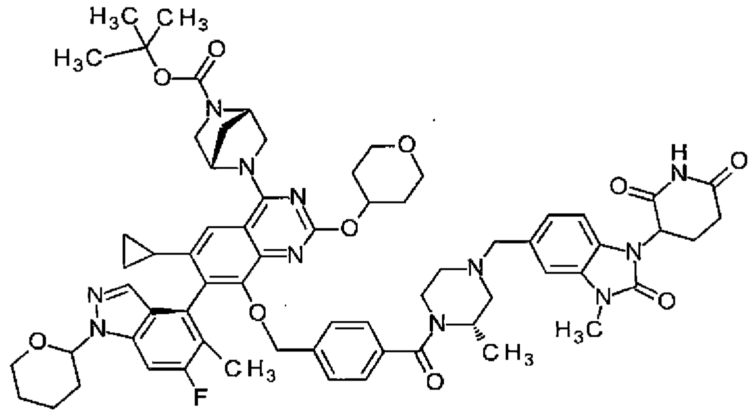 |
| D9 | 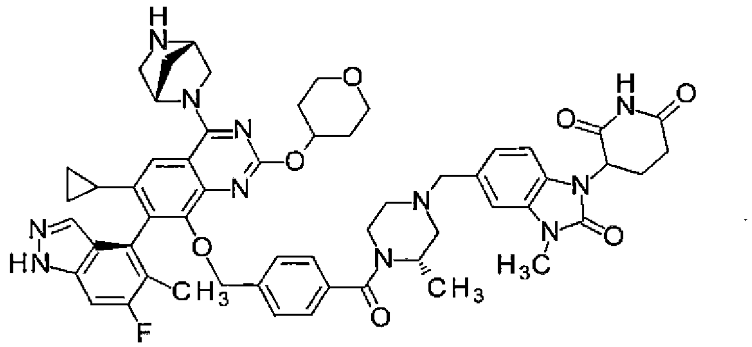 |
| D10-1 | 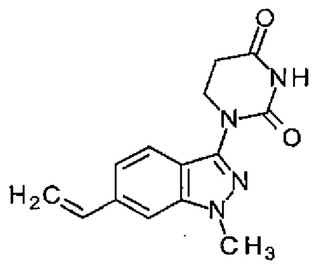 |
| D10-2 | 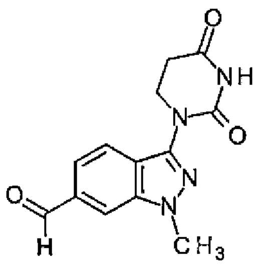 |
| D10-3 | 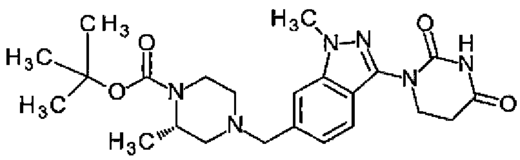 |

[Table 1-11]

| Ex | CHEM |
|---|---|
| D10-4 | 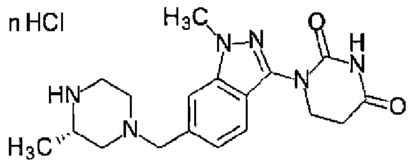 |
| D10-5 | 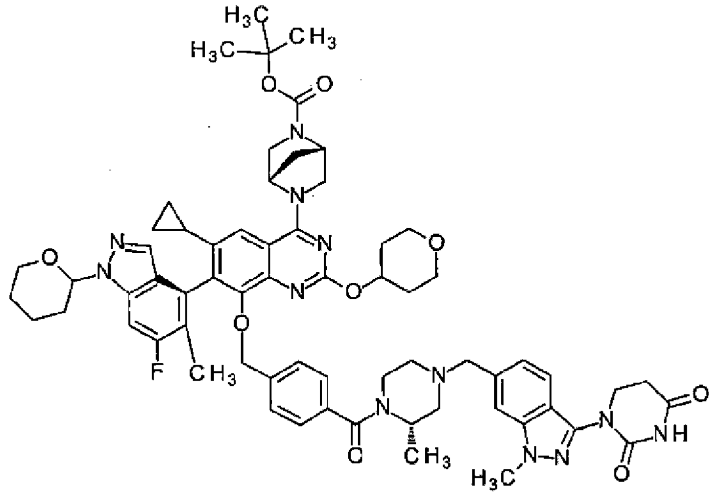 |
| D10 | 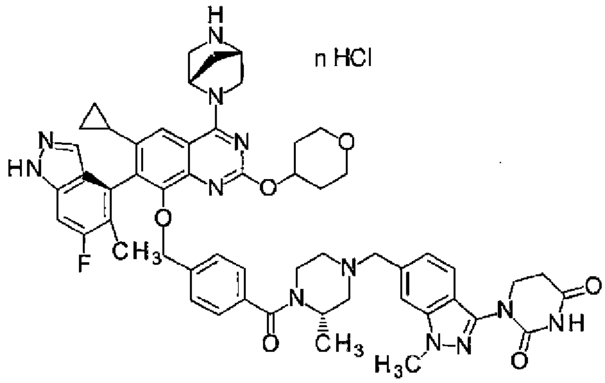 |
| D11-1 | 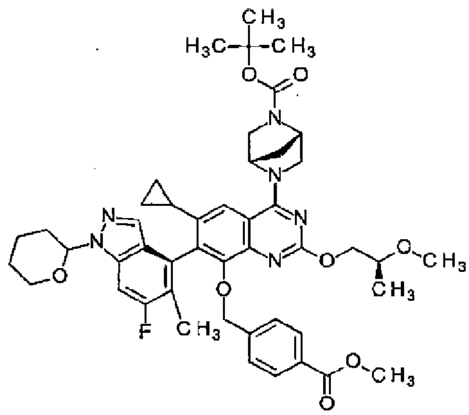 |

[Table 1-12]

| Ex | CHEM |
|---|---|
| D11-2 | 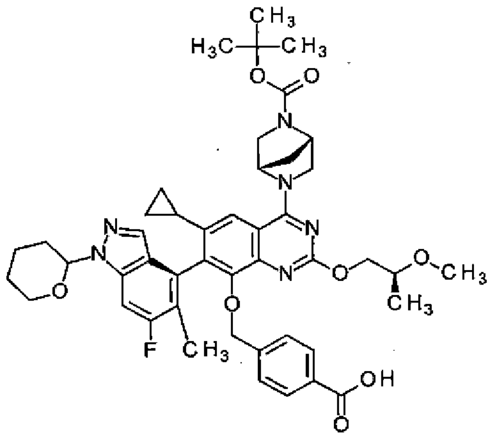 |
| D11-3 | 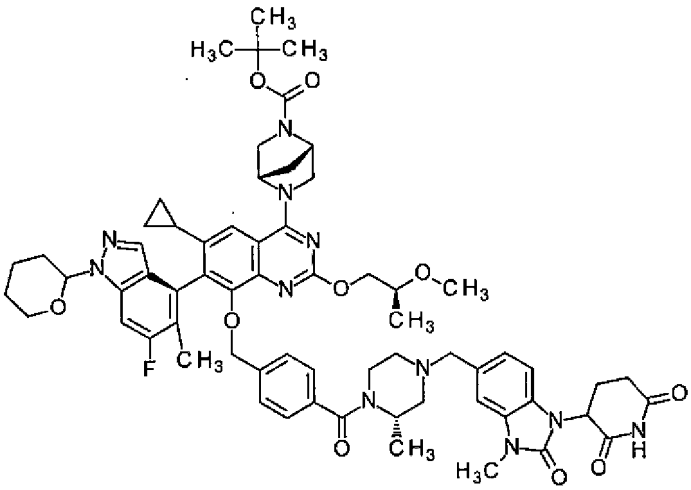 |
| D11 | 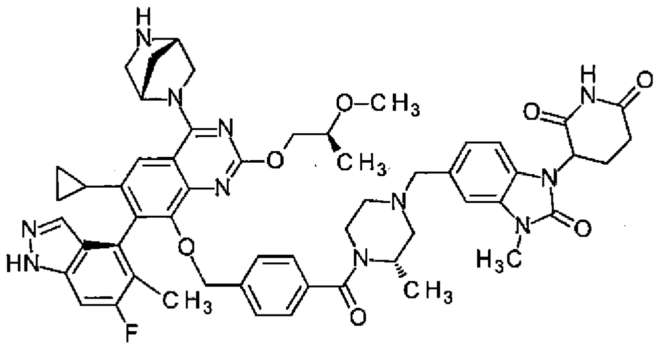 |

[Table 1-13]

| Ex | CHEM |
|---|---|
| D12-1 | 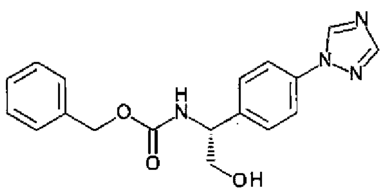 |

(continued)

| Ex | CHEM |
| --- | --- |
| D12-2 | 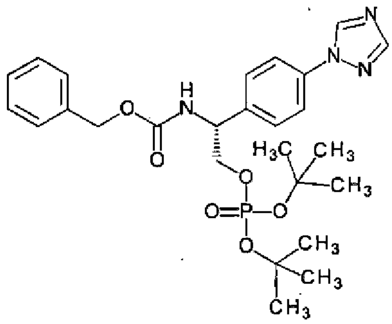 |
| D12-3 | 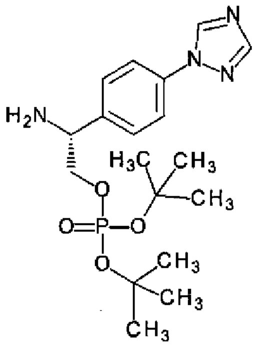 |
| D12-4 | 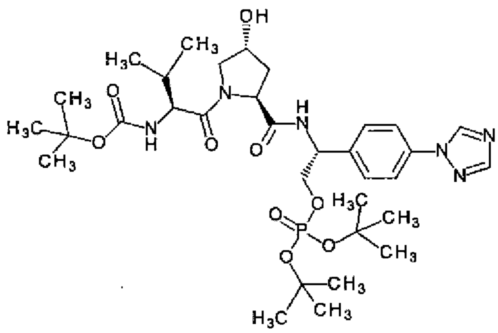 |
| D12-5 | 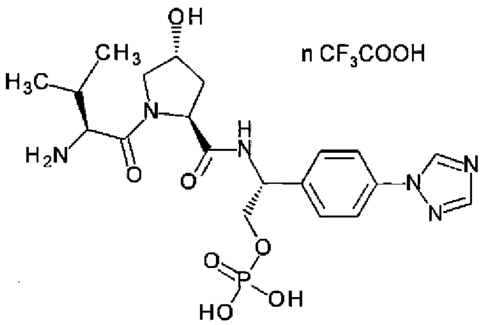 n $CF_3COOH$ |

[Table 1-14]

| Ex | CHEM |
|---|---|
| D12-6 | 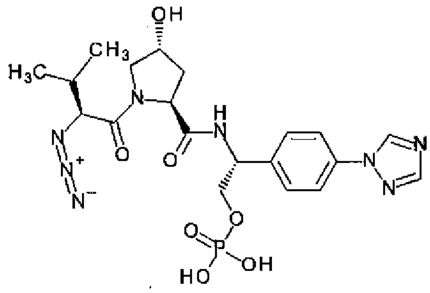 |
| D12-7 | 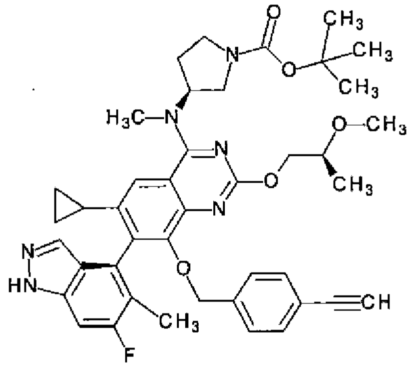 |
| D12-8 | 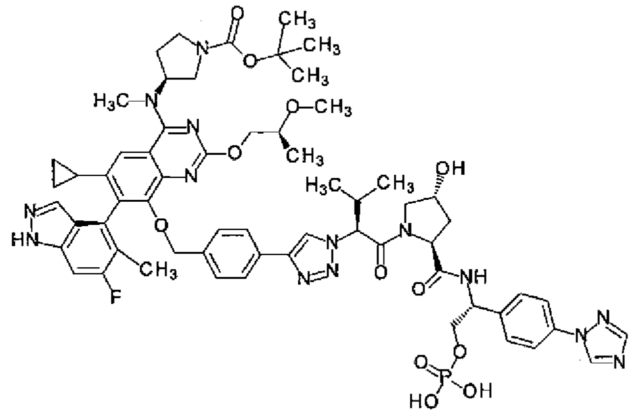 |
| D12 | 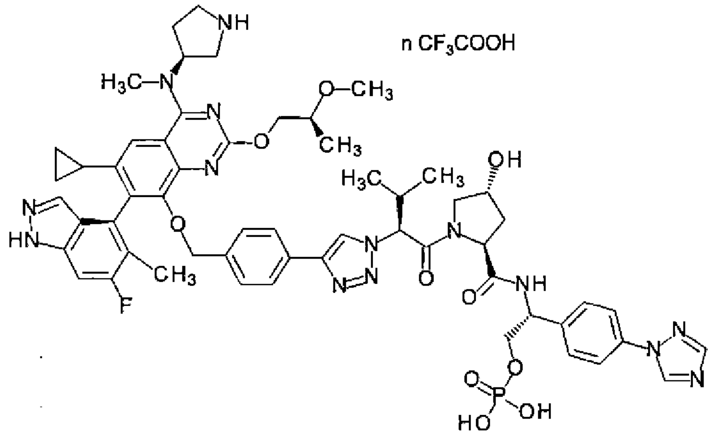 n $CF_3COOH$ |

[Table 2-1]

| Ex | DAT |
|---|---|
| D7-1 | ESI+: 402.9, 404.9, 406.9 |
| D7-2 | ESI+: 480.9, 482.8 [M+Na]+ |
| D7-3 | ESI+: 508.9 [M+Na]+ |
| D7-4 | ESI+: 587.2 |
| D7-5 | ESI+: 503.4 |
| D7-6 | ESI+: 813.4 |
| D7-7 | ESI+: 867.7 [M+Na]+<br>NMR: 0.43-0.50 (1H, m), 0.66-0.75 (1H, m), 0.77-0.89 (2H, m), 0.94 (3H, d), 1.25 (3H, t), 1.46-1.54 (1H, m), 1.75 (9H, s), 1.80 (3H, d), 3.47-3.54 (2H, m), 5.94 (1H, q), 6.90-6.94 (2H, m), 7.01-7.09 (6H, m), 7.16-7.24 (3H, m), 7.28-7.35 (9H, m), 7.35 (1H, s), 7.44 (1H, d), 7.52 (1H, d) |
| D7-8 | ESI+: 603.6 |
| D7-9 | ESI+: 687.7 |
| D7-10 | ESI+: 605.4 [M+Na]+ |
| D7-11 | ESI+: 697.3 |
| D7-12 | ESI+: 693.6 |
| D7-13 | ESI+: 637.4 |
| D7-14 | ESI+: 819.7 |
| D7-15 | ESI+: 1291.9 |
| D7 | ESI+: 1107.6<br>NMR (100°C): 0.50-0.57 (1H, m), 0.57-0.70 (3H, m), 0.77 (3H, d), 1.06 (3H, d), 1.15 (3H, d), 1.37-1.45 (1H, m), 1.89-2.00 (2H, m), 2.01 (3H, d), 2.01-2.09 (1H, m), 2.09-2.17 (1H, m), 2.20-2.34 (1H, m), 2.45 (3H, s), 2.50-2.60 (1H, m), 2.83-2.90 (1H, m), 2.97 (1H, dd), 3.03 (1H, ddd), 3.18 (1H, dd), 3.25 (3H, s), 3.30 (3H, s), 3.56-3.64 (1H, m), 3.64-3.75 (3H, m), 3.83 (1H, dd), 4.29 (1H, dd), 4.33-4.39 (2H, m), 4.41-4.47 (1H, m), 4.52 (1H, br t), 4.77-4.83 (1H, m), 4.86-4.97 (3H, m), 5.25 (1H, d), 5.28 (1H, d), 6.84 (2H, d), 7.30 (1H, d), 7.38-7.44 (5H, m), 7.46 (1H, d), 7.60 (2H, d), 8.00 (1H, br d), 8.42 (1H, s), 8.88 (1H, s), 12.75 (1H, br s) |
| D8-1 | ESI+: 1312.0 [M+Na]+ |
| D8 | ESI+: 1105.0<br>NMR(100°C): 0.51-0.71 (4H, m), 0.77 (3H, d), 1.07 (3H, d), 1.16 (3H, d), 1.30 (3H, t), 1.38-1.45 (1H, m), 1.87-1.97 (1H, m), 1.98-2.15 (2H, m), 2.01 (3H, d), 2.22-2.31 (1H, m), 2.50-2.60 (1H, m), 3.04-3.11 (1H, m), 3.15 (1H, dd), 3.22-3.32 (2H, m), 3.28 (3H, s), 3.30 (3H, s), 3.42 (1H, dd), 3.60 (1H, br d), 3.66-3.76 (3H, m), 3.84(1H, br dd), 4.11(2H, q), 4.29-4.39 (3H, m), 4.46 (1H, br s), 4.52 (1H,br t), 4.81 (1H, br s), 4.88-4.94 (2H, m), 4.94-5.01 (1H, m), 5.25 (1H, d), 5.29 (1H, br d), 6.26 (1H, d), 6.85 (2H, d), 7.31 (1H, d), 7.35-7.39 (2H, m), 7.40-7.46 (5H, m), 7.60 (2H, br d), 8.01 (1H, br d), 8.42 (1H, s), 12.76 (1H, br s) |

[Table 2-2]

| Ex | DAT |
|---|---|
| D9-1 | ESI+: 713.1 |
| D9-2 | ESI+: 627.5 |
| D9-3 | ESI+: 937.6 |
| D9-4 | ESI+:695.4<br>NMR: 0.52-0.66 (3H, m), 0.66-0.75 (1H, m), 0.86 (3H, br d), 1,27 (3H, t), 1.30-1.46 (10H, m), 1.83 (3H, br s), 1.90-2.01 (1H, m), 2.04 (1H, br d), 2.98-3.10 (2H, m), 3.45-3.55 (2H, m), 3.67-3.82 (1H, m), 4.33 (1H, br d), 4.52-4.64 (1H, m), 5.15 (1H, br d), 6.02-6.10 (1H, m), 6.82 (2H, br d), 7.12-7.20 (3H, m), 7.31 (1H, br s), 7.37 (1H, d), 7.51-7.57 (1H, m), 13.04 (1H, br s) |

(continued)

| Ex | DAT |
|---|---|
| D9-5 | ESI+: 779.5 |
| D9-6 | ESI+: 811.5 |
| D9-7 | ESI+: 707.4 |
| D9-8 | ESI+: 855.5 |
| D9-9 | ESI+: 863.6 |
| D9-10 | ESI+: 849.7 |
| D9-11 | ESI+: 382.3 |
| D9-12 | ESI+: 460.4 |
| D9-13 | ESI+: 338.2 |
| D9-14 | ESI+: 286.2 |
| D9-15 | ESI+: 288.3 |
| D9-16 | ESI+: 472.4 |
| D9-17 | ESI+: 372.5 |
| D9-18 | ESI+: 1202.8 |
| D9 | ESI+: 1018.6<br>NMR (90°C): 0.48-0.68 (4H, m), 1.26 (3H, d), 1.37 (1H, tt), 1.62-1.71 (2H, m), 1.73 (1H, br d), 1.86 (1H, br d), 1.99 (3H, d), 1.99-2.09 (4H, m), 2.13 (1H, dd), 2.61-2.74 (3H, m), 2.80 (1H, br d), 2.83-2.93 (1H, m), 3.04 (1H, dd), 3.09-3.22 (2H, m), 3.28-3.37 (2H, m), 3.32 (3H, s), 3.46 (1H, d), 3.56 (1H, d), 3.69-3.79 (3H, m), 3.82 (2H, dq), 4.12-4.24 (1H, m), 4.16 (1H, dd), 4.81 (1H, d), 5.11 (1H, br s), 5.15 (1H, tt), 5.22-5.30 (2H, m), 6.82-6.87 (2H, m), 6.96-7.04 (2H, m), 7.07-7.11 (3H, m), 7.27 (1H, d), 7.44 (1H, d), 7.46 (1H, s), 10.71 (1H, br s), 12.78 (1H, br s) |
| D10-1 | ESI+: 271.2 |
| D10-2 | ESI+: 273.1 |
| D10-3 | ESI+: 457.6 |
| D10-4 | ESI+: 357.5 |
| D10-5 | ESI+: 1188.0 |
| D10 | ESI+: 1003.8 |

[Table 2-3]

| Ex | DAT |
|---|---|
| D11-1 | ESI+: 851.8 |
| D11-2 | ESI+: 837.7 |
| D11-3 | ESI+: 1190.8 |
| D11 | ESI+: 1006.7<br>NMR (90°C): 0.48-0.68 (4H, m), 1.12 (3H, d), 1.26 (3H, d), 1.37 (1H, tt), 1.74 (1H, br d), 1.87 (1H, br d), 1.98 (3H, d), 1.98-2.09 (2H, m), 2.12 (1H, dd), 2.60-2.74 (3H, m), 2.80 (1H, br d), 2.83-2.93 (1H, m), 3.05 (1H, dd), 3.10-3.21 (2H, m), 3.28 (3H, s), 3.32 (3H, s), 3.46 (1H, d), 3.56 (1H, d), 3.65-3.78 (4H, m), 4.13-4.22 (2H, m), 4.23-4.28 (1H, m), 4.28-4.34 (1H, m), 4.83 (1H, d), 5.12 (1H, s), 5.23-5.30 (2H, m), 6.84 (2H, d), 6.96-7.04 (2H, m), 7.04-7.10 (3H, m), 7.26 (1H, d), 7.44 (1H, d), 7.46 (1H, s), 10.73 (1H, br s), 12.77 (1H, br s) |

[Table 2-4]

| Ex | DAT |
|---|---|
| D12-1 | ESI+: 339.1 |
| D12-2 | ESI+: 531.3 |
| D12-3 | ESI+: 419.1 [M+Na]+ |
| D12-4 | ESI+: 709.2 |
| D12-5 | ESI+: 497.1 |
| D12-6 | ESI+: 523.0 |
| D12-7 | ESI+: 735.6 |
| D12-8 | ESI+: 1258.6 |
| D12 | ESI+: 1157.5 |

Example 2 Synthesis of drug-linker conjugate

Synthesis of drug-linker conjugate L1-D1

To a DMF (1 mL) solution of a single-axial chirality compound (4R)-1-[(2S)-2-(4-{4-[({6-cyclopropyl-4-[(1S,4S)-2,5-diazabicyclo[2.2.1]heptan-2-yl]-7-(6-fluoro-5-methyl-1H-indazol-4-yl)-2-[(oxan-4-yl)oxy]quinazolin-8-yl}oxy)methyl]phenyl}-1H-1,2,3-triazol-1-yl)-3-methylbutanoyl]-4-hydroxy-N-{(1R)-2-hydroxy-1-[4-(4-methyl-1,3-thiazol-5-yl)phenyl]ethyl}-L-prolinamide (D1, 154.3 mg) obtained by the method described in International Publication No. WO 2022/173032, and N-[6-(2,5-dioxo-2,5-dihydro-1H-pyrrol-1-yl)hexanoyl]-L-valyl-N-[4-({[(4-nitrophenoxy)carbonyl]oxy}methyl)phenyl]-L-alaninamide (100 mg), DIPEA (53.5 uL) was added, and the reaction mixture was stirred at 25°C for 2 hours. The reaction mixture was diluted by the addition of acetonitrile (1 mL) and water (1 mL) and then purified by ODS chromatography (water (0.05% HCOOH)/MeCN = 100/0-85/15) to obtain N-[6-(2,5-dioxo-2,5-dihydro-1H-pyrrol-1-yl)hexanoyl]-L-valyl-N-[4-({[(1S,4S)-5-{6-cyclopropyl-7-(6-fluoro-5-methyl-1H-indazol-4-yl)-8-{[4-(1-{(2S)-1-[(2S,4R)-4-hydroxy-2-({(1R)-2-hydroxy-1-[4-(4-methyl-1,3-thiazol-5-yl)phenyl]ethyl}carbamoyl)pyrrolidin-1-yl]-3-methyl-1-oxobutan-2-yl}-1H-1,2,3-triazol-4-yl)phenyl]methoxy}-2-[(oxan-4-yl)oxy]quinazolin-4-yl}-2,5-diazabicyclo[2.2.1]heptane-2-carbonyl]oxy}methyl)phenyl]-L-alaninamide n-formate (L1-D1, 86.9 mg) as a solid.

Drug-linker conjugates L1-D6, L1-D7, L1-D10, L1-D11, L1-D12, and L2-D12 were synthesized with a replaced drug (D) moiety in the same manner as in the synthesis of drug-linker conjugate L1-D1. For drugs D7, D11, and D12, n-trifluoroacetate was used as a starting material of drug-linker conjugate synthesis. The n-trifluoroacetate of drug D7 was synthesized in the same manner as in steps 8 and 9 of the synthesis of drug D12 using D12-7 and the compound described in Production Example 141 of International Publication No. WO 2023/171781 as a starting material.

Synthesis of drug-linker conjugate L3-D12

To a mixture of (2R)-2-({(4R)-1-[(2S)-2-{4-[4-({[(7M)-6-cyclopropyl-7-(6-fluoro-5-methyl-1H-indazol-4-yl)-2-[(2S)-2-methoxypropoxy]-4-{methyl[(3S)-pyrrolidin-3-yl]amino}quinazolin-8-yl]oxy}methyl)phenyl]-1H-1,2,3-triazol-1-yl}-3-methylbutanoyl]-4-hydroxy-L-prolyl}amino)-2-[4-(1H-1,2,4-triazol-1-yl)phenyl]ethyl dihydrogen phosphate n-trifluoroacetate (D12, 50 mg), N-[6-(2,5-dioxo-2,5-dihydro-1H-pyrrol-1-yl)hexanoyl]glycylglycyl-L-phenylalanylglycine (25.2 mg), and DMF (2 mL), DIPEA (23 μL) and HATU (13.1 mg) were added at 20°C in a nitrogen atmosphere, and stirred at 20°C for 1 hour. The reaction mixture was adjusted to pH 4 by the addition of a 25% aqueous TFA solution and then filtered, and the filtrate was purified by ODS column chromatography (MeCN/0.05% aqueous TFA solution) to obtain (2R)-2-({(4R)-1-[(2S)-2-(4-{4-[({(7M)-6-cyclopropyl-4-{[(3S)-1-{N-[6-(2,5-dioxo-2,5-dihydro-1H-pyrrol-1-yl)hexanoyl]glycylglycyl-L-phenylalanylglycyl}pyrrolidin-3-yl](methyl)amino}-7-(6-fluoro-5-methyl-1H-indazol-4-yl)-2-[(2S)-2-methoxypropoxy]quinazolin-8-yl}oxy)methyl]phenyl}-1H-1,2,3-triazol-1-yl)-3-methylbutanoyl]-4-hydroxy-L-prolyl}amino)-2-[4-(1H-1,2,4-triazol-1-yl)phenyl]ethyl dihydrogen phosphate n-trifluoroacetate (L3-D12, 40.2 mg) as a solid.

The chemical structural formula and physicochemical data of the compound obtained in each step are shown in tables below.

Drug-linker conjugates L1-D2 to L1-D5, L1-D8, and L1-D9 can be synthesized with a replaced drug (D) moiety in the same manner as in the synthesis of drug-linker conjugate L1-D1.

[Table 3-1]

| Ex | CHEM |
|---|---|
| L1-D1 | 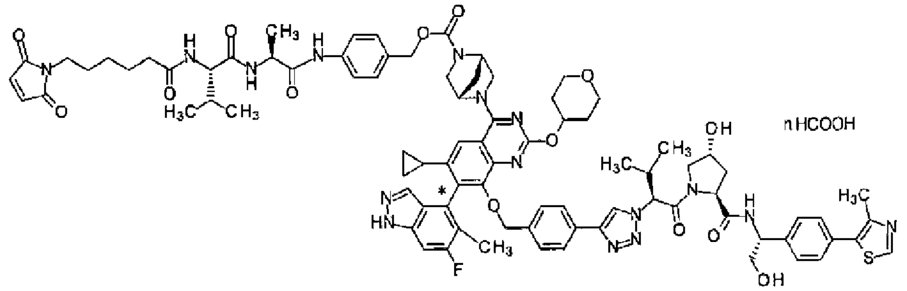 n HCOOH |
| L1-D6 | 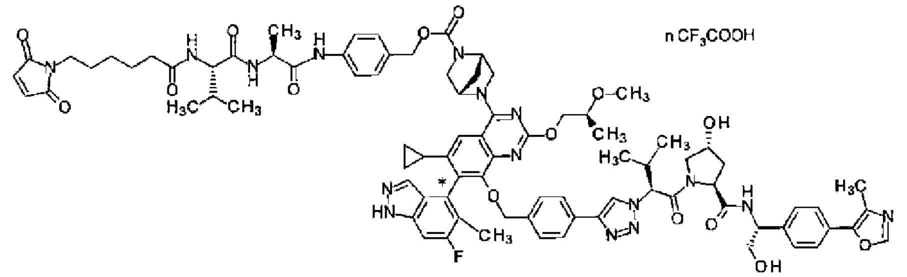 n $CF_3COOH$ |
| L1-D7 | 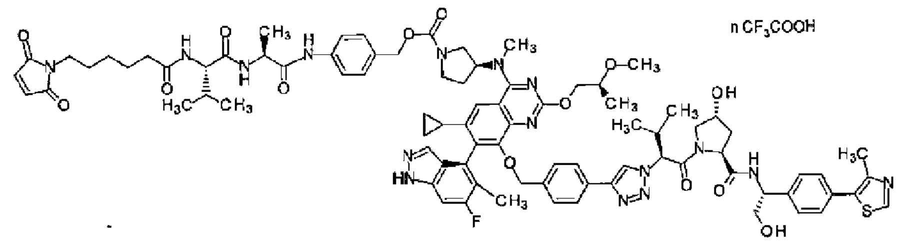 n $CF_3COOH$ |
| L1-D10 | 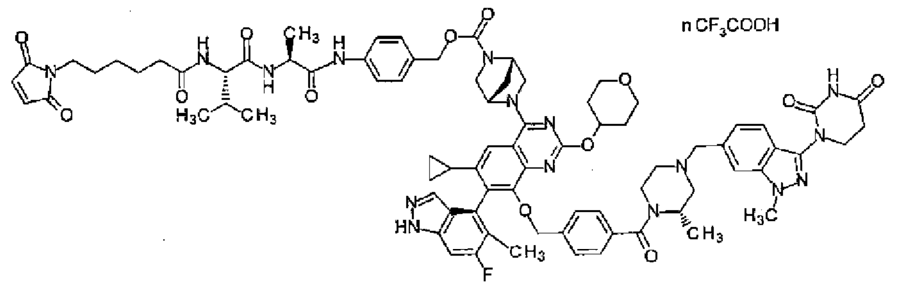 n $CF_3COOH$ |
| L1-D11 | 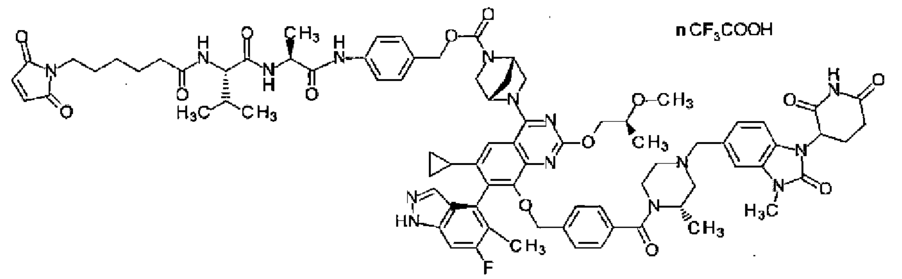 n $CF_3COOH$ |

[Table 3-2]

| Ex | CHEM |
|---|---|
| L1-D12 | 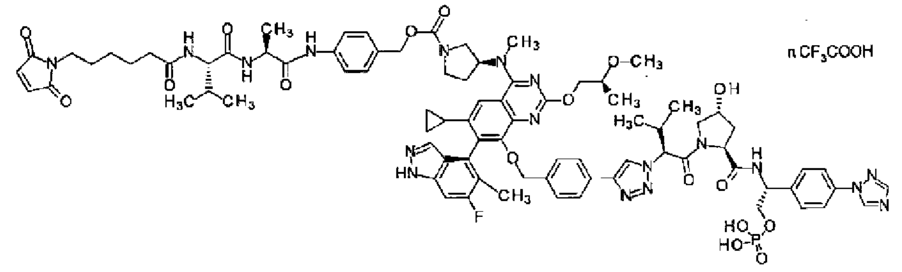 |
| L2-D12 | 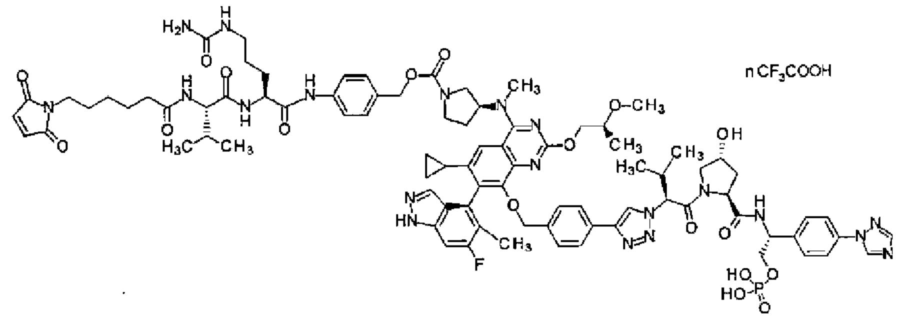 |
| L3-D12 | 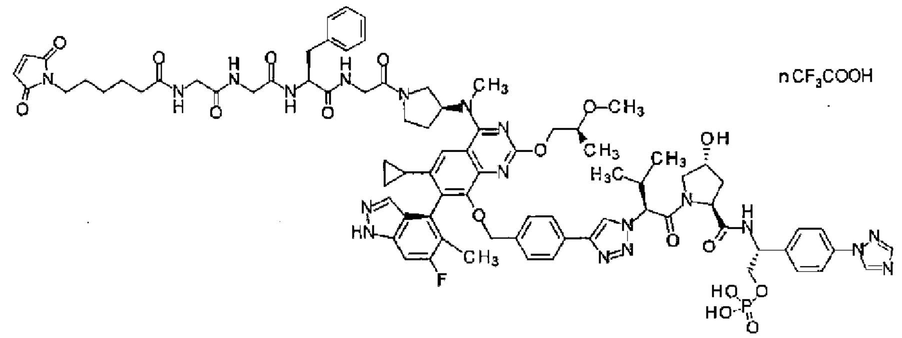 |

[Table 4-1]

| Ex | DAT |
|---|---|
| L1-D1 | ESI+:1629.7<br>NMR (24°C): 13.11 (br s, 1H), 9.94 (br s, 1H), 8.98 (s, 1H), 8.65 (s, 1 H), 8.49 (br d, *J* = 7.6 Hz, 1H), 8.15 (br d, *J* = 6.8 Hz, 1H), 7.81 (br d, *J* = 8.0 Hz, 1H), 7.67 (br d, *J* = 7.2 Hz, 2H), 7.61-7.52 (m, 3H), 7.46-7.25 (m, 8H), 6.98 (s, 2H), 6.78 (br d, *J* = 7.2 Hz, 2H), 5.36-4.82 (m, 10H), 4.74-4.62 (m, 2H), 4.46 (br t, *J* = 8.0 Hz, 1H), 4.41-4.29 (m, 3H), 4.20-4.11 (m, 1H), 3.91-3.75 (m, 5H), 3.70 (br d, *J* = 9.6 Hz, 1H), 3.59 (br d, *J* = 10.8 Hz, 5H), 2.46 (br s, 3H), 2.20-1.91 (m, 12H), 1.83-1.74 (m, 1H), 1.67 (br d, *J* = 3.6 Hz, 2H), 1.47 (br d, *J* = 6.4 Hz, 4H), 1.38-1.32 (m, 1H), 1.28 (br d, *J* = 5.6 Hz, 3H), 1.24-1.13 (m, 3H), 1.07 (br d, *J* = 4.8 Hz, 3H), 0.87-0.77 (m, 6H), 0.72 (br d, *J* = 5.6 Hz, 3H), 0.64 (br s, 3H), 0.58-0.52 (m, 1H). |

[Table 4-2]

| Ex | DAT |
|---|---|
| L1-D6 | ESI+: 1601.7 |
| L1-D7 | ESI+: 1619.5 |
| L1-D10 | ESI+: 1515.7 |
| L1-D11 | ESI+: 1518.7 |
| L1-D12 | ESI+: 1669.7 |

(continued)

| Ex | DAT |
|---|---|
| L2-D12 | ESI+: 1755.8 |
| L3-D12 | ESI+: 1669.3 |

[Table 5-1]

| Ex | CHEM |
|---|---|
| L1-D2 | 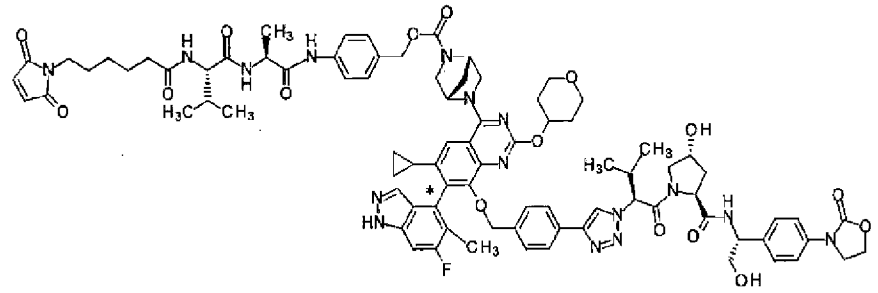 |
| L1-D3 | 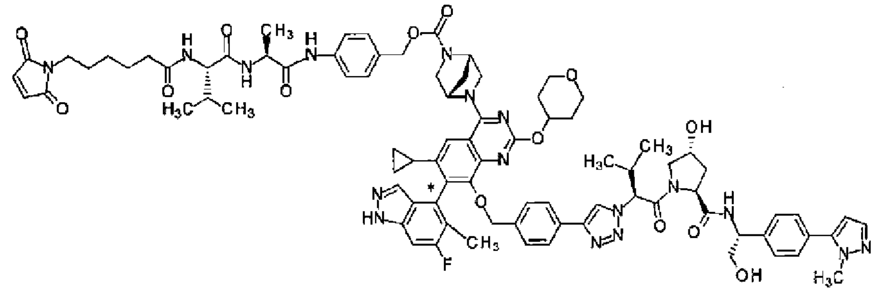 |
| L1-D4 | 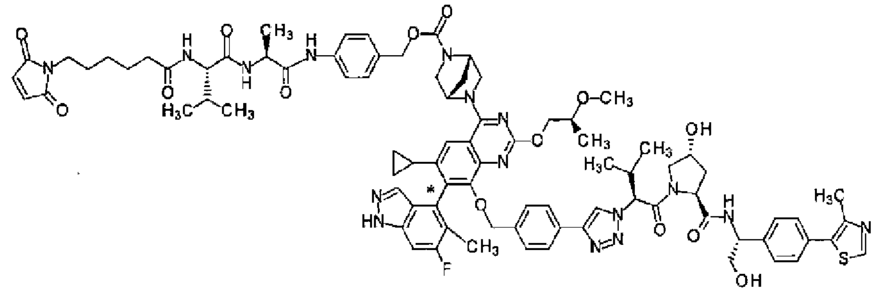 |
| L1-D5 | 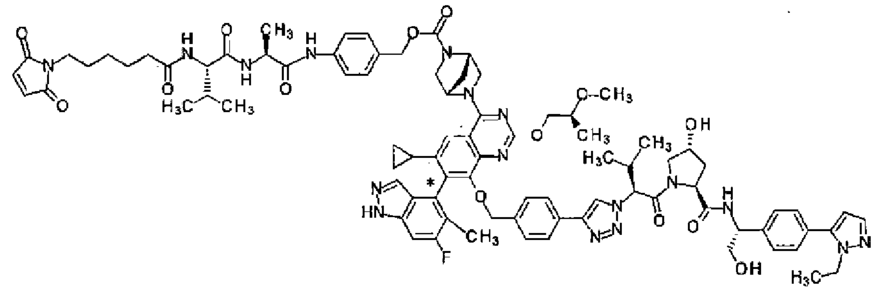 |

[Table 5-2]

| Ex | CHEM |
|---|---|
| L1-D8 | 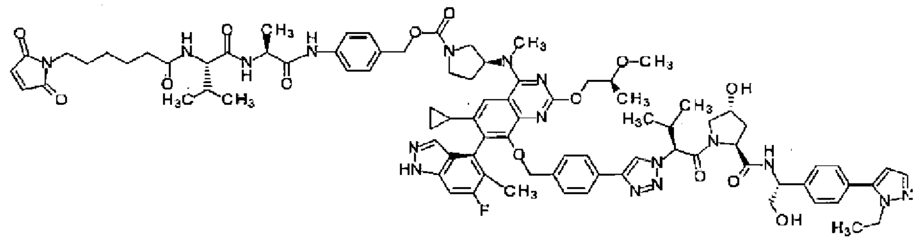 |
| L1-D9 | 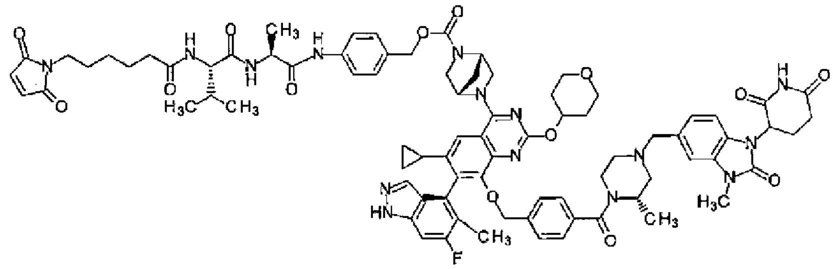 |

Example 3 Synthesis of antibody-drug conjugate

Methods for synthesizing antibody-drug conjugates will be shown below. Ab1 to Ab3 each represent an antibody, L1 to L7 each represent a linker, and D1 to D12 each represent a drug. For example, Ab1-L1-D1 represents an antibody-drug conjugate of antibody Ab1 and drug D1 connected via linker L1. Ab1 represents an anti-EGFR antibody cetuximab and was prepared by a routine method with reference to amino acid sequence information on cetuximab (entry: D03455) registered in KEGG DRUG Database (https://www.genome.jp/kegg/drug/drug_ja.html), and used in subsequent studies. Ab2 represents an anti-HER2 antibody trastuzumab, and commercially available Herceptin(Registered Trademark) for injection, 440 mg/20 mL (Roche) was used in subsequent studies. Ab3 represents an anti-TROP2 antibody sacituzumab mutant, and a sacituzumab mutant was designed with reference to amino acid sequence information on sacituzumab (entry: D10984) registered in KEGG DRUG Database by introducing a mutation to substitute tyrosine at position 92 of the light chain of the antibody by threonine, prepared according to a routine method on the basis of the designed sequence, and used in subsequent studies.

Common procedure A: Measurement of drug concentration of antibody-drug conjugate

A drug concentration in an antibody-drug conjugate was calculated by the BCA method using Thermo Scientific Pierce(Registered Trademark) BCA Protein Assay Kits (Cat No. 23225, Thermo Fisher Scientific Inc.) or the like. A calibration curve of absorbance at 562 nm was prepared using an antibody not conjugated with a compound, and the drug concentration was calculated from the absorbance at 562 nm of the antibody-drug conjugate.

Common procedure B: Measurement of drug-to-antibody ratio of antibody-drug conjugate

A drug-to-antibody ratio of an antibody-drug conjugate was calculated from a chromatogram obtained by hydrophobic interaction chromatography (HIC) measurement using Agilent 1260 series HPLC system (Agilent Technologies, 1260 Infinity). An antibody-drug conjugate sample was injected into a column (TSKgel(Registered Trademark) Butyl-NPR, Cat No. 0014947, TOSOH BIOSCIENCE) equilibrated with mobile phase A (100%), followed by elution on a gradient of 13 minutes (0 to 100% B) at a flow rate of 0.6 mL/min. Mobile phase A was 1.5 M ammonium sulfate and 50 mM dipotassium hydrogen phosphate, pH 7.0. Mobile phase B was 21.3 mM potassium dihydrogen phosphate, 28.6 mM dipotassium hydrogen phosphate, and 25% iPrOH, pH 7.0. Detection was performed at 280 nm, and an average drug-to-antibody ratio (DAR) was calculated on the basis of the area of each peak.

Common procedure C: Measurement of drug-to-antibody ratio of antibody-drug conjugate

An antibody-drug conjugate subjected to reduction treatment with DTT was injected into LC-MS using a time-to-flight mass spectrometry apparatus (Agilent Technologies, 6224 TOF LC/MS) connected to Agilent 1260 series HPLC system (Agilent Technologies, 1260 Infinity). Measurement data was analyzed with MassHunter Qualitative Analysis Software B.07.00 (Agilent Technologies). The spectral intensity of each MS peak of the antibody-drug conjugate was

obtained from a deconvolution mass spectrum obtained by analysis, and an average drug-to-antibody ratio (DAR) was calculated.

Common procedure D: Measurement of monomer purity of antibody-drug conjugate

A monomer purity of an antibody-drug conjugate was calculated from a chromatogram obtained by size exclusion chromatography (SEC) measurement using Agilent 1260 series HPLC system (Agilent Technologies, 1260 Infinity). A mobile phase (pH 7.0 ± 0.1) composed of 78 mM potassium dihydrogen phosphate, 122 mM dipotassium hydrogen phosphate, 250 mM KCl, and 15% iPrOH was injected at a flow rate of 0.75 mL/min using a size exclusion chromatography column (TSKgel(Registered Trademark) G3000SWXL, Cat No. 0008541, TOSOH BIOSCIENCE) to elute the antibody-drug conjugate, followed by the detection of absorption at UV 280 nm. An integral value of each peak was obtained, and the monomer purity was calculated.

Synthesis of antibody-drug conjugate Ab1-L1-D1

A 5.10 mg/mL cetuximab solution (980.4 μL, 20 mM aqueous sodium citrate solution) was collected into a 50 mL polypropylene tube, to which a 2 mM aqueous TCEP solution (49.3 μL) and a 200 mM aqueous EDTA solution (25 μL) were then added. A 20 mM sodium citrate buffer solution (162.5 μL) of pH 6.0 was added thereto, followed by incubation at 22°C for 3 hours with mild mixing in an incubator shaker. Subsequently, a 10 mM DMA solution of N-[6-(2,5-dioxo-2,5-dihydro-1H-pyrrol-1-yl)hexanoyl]-L-valyl-N-[4-({[(1S,4S)-5-{6-cyclopropyl-7-(6-fluoro-5-methyl-1H-indazol-4-yl)-8-{[4-(1-{(2S)-1-[(2S,4R)-4-hydroxy-2-({(1R)-2-hydroxy-1-[4-(4-methyl-1,3-thiazol-5-yl)phenyl]ethyl}carbamoyl)pyrrolidin-1-yl]-3-methyl-1-oxobutan-2-yl}-1H-1,2,3-triazol-4-yl)phenyl]methoxy}-2-[(oxan-4-yl)oxy]quinazolin-4-yl}-2,5-diazabicyclo[2.2.1]heptane-2-carbonyl]oxy}methyl)phenyl]-L-alaninamide n-formate (32.9 μL; the concentration was calculated from a molecular weight in terms of a free form) and propylene glycol (1250 μL) were added thereto, followed by incubation at 22°C for 1 hour with mild mixing in an incubator shaker. Zeba(TM) spin desalting column (Cat No. 87772, Thermo Fisher Scientific Inc.) was equilibrated with a histidine buffer solution (20 mM, pH 5.5) containing 7% sucrose in accordance with the method prescribed by the manufacturer. On this Zeba(TM) spin desalting column, an aqueous antibody-drug conjugate reaction solution was placed, and an antibody-drug conjugate was obtained by centrifugal operation (1000 g, 4 minutes) using a centrifuge. Subsequently, the antibody-drug conjugate solution was placed in a container of Amicon(Registered Trademark) Ultra (Merck, UFC903096) equilibrated with a histidine buffer solution (20 mM, pH 5.5) containing 7% sucrose. The antibody-drug conjugate solution was washed by centrifugal operation (centrifuged at <4500 g for 2 to 5 minutes) using a centrifuge, and concentrated to obtain 1.65 mL of an antibody-drug conjugate. A 0.49 mg/mL solution containing the antibody-drug conjugate (Ab1-L1-D1, DAR 3.47, monomer purity: 89.85%) was confirmed by common procedures A, C, and D.

Drug-linker conjugates Ab1-L1-D6, Ab1-L1-D7, Ab1-L1-D10, Ab1-L1-D11, Ab1-L1-D12, Ab1-L2-D12, Ab1-L3-D12, Ab2-L3-D12, and Ab3-L3-D12 were synthesized with a replaced drug (D), linker (L), and antibody (Ab) moiety as shown in the tables below in the same manner as in the synthesis of drug-linker conjugate Ab1-L1-D1.

The chemical structural formulas, DAR and measurement methods thereof, and monomer purities of the obtained antibody-drug conjugates are shown in tables below. In the section about the DAR measurement method in the tables, "HIC" and "LC/MS" represent that the value of DAR was measured by common procedure B and common procedure C, respectively.

Antibody-drug conjugates Ab1-L1-D2, Ab1-L1-D3, Ab1-L1-D4, Ab1-L1-D5, Ab1-L1-D8, and Ab1-L1-D9 can be synthesized with a replaced drug (D) moiety as shown in the tables below in the same manner as in the synthesis of antibody-drug conjugate Ab1-L1-D1.

[Table 6-1]

| Ex | CHEM |
|---|---|
| Ab1-L1-D1 | 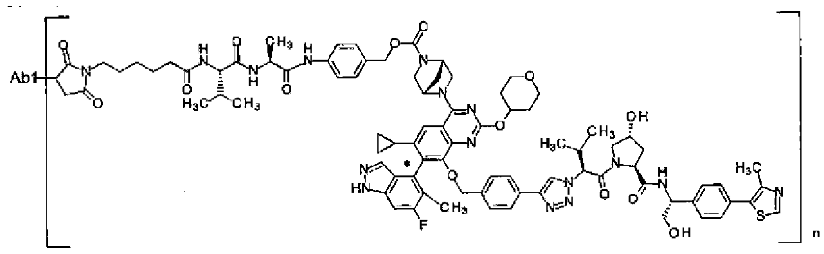 |

(continued)

| Ex | CHEM |
|---|---|
| Ab1-L1-D6 | 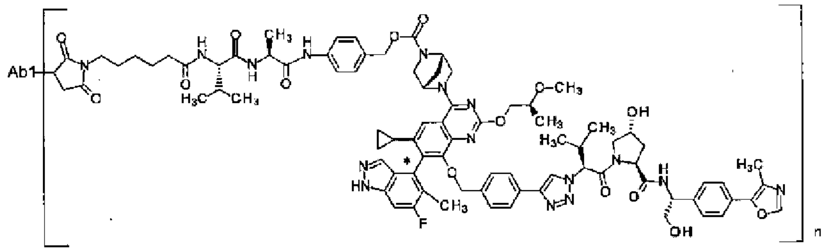 |
| Ab1-L1-D7 | 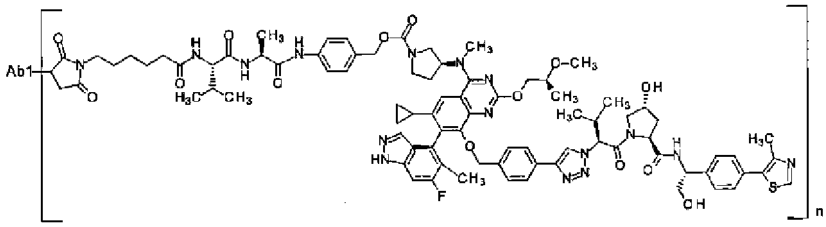 |
| Ab1-L1-D10 | 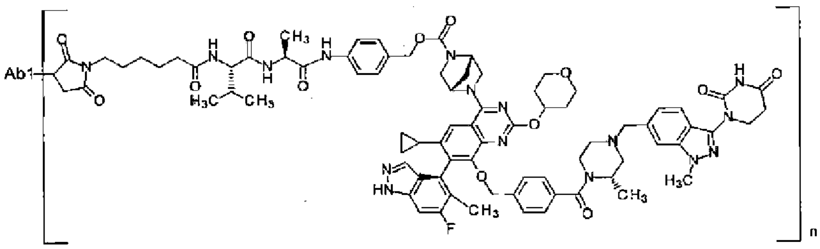 |
| Ab1-L1-D11 | 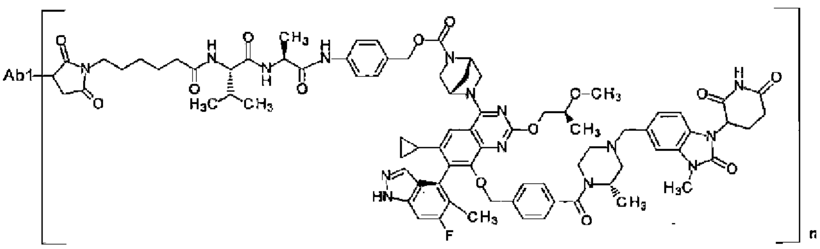 |

[Table 6-2]

| Ex | CHEM |
|---|---|
| Ab1-L1-D12 | 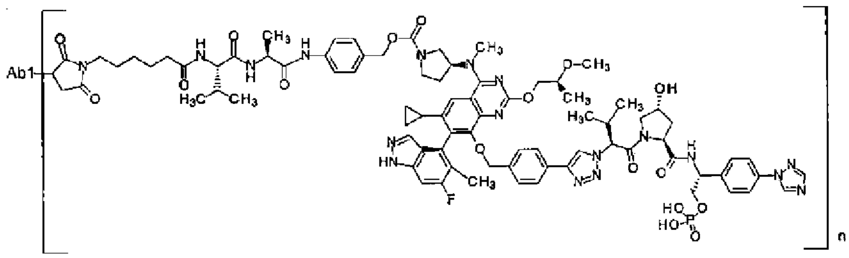 |

(continued)

| Ex | CHEM |
|---|---|
| Ab1-L2-D12 | 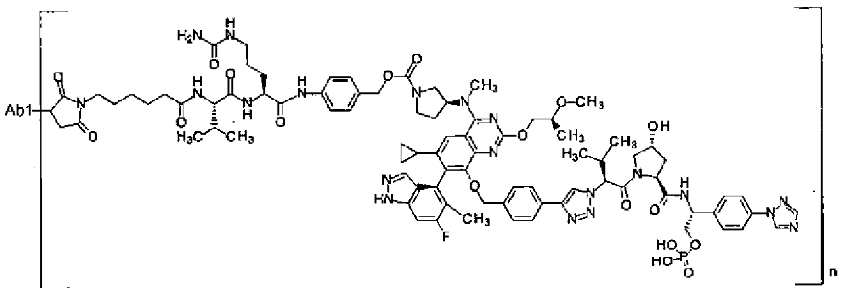 |
| Ab1-L3-D12 | 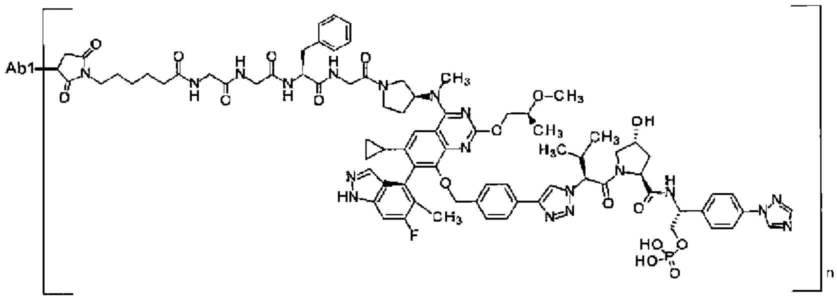 |
| Ab2-L3-D12 | 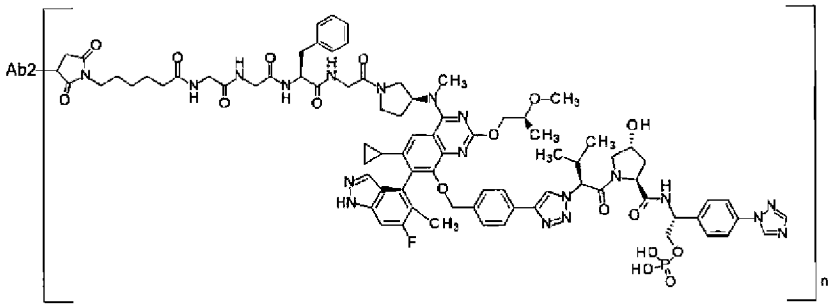 |
| Ab3-L3-D12 | 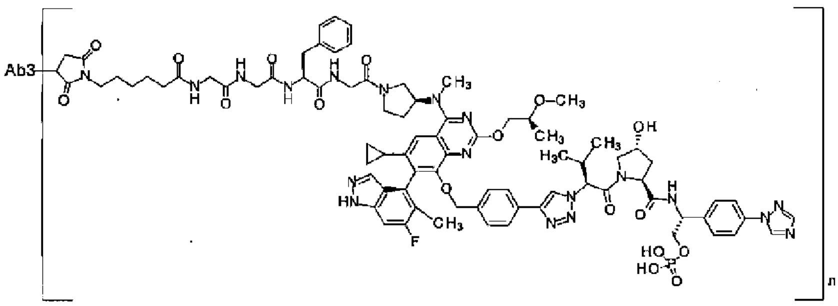 |

[Table 7]

| Ex | DAR | DAR measurement method | Monomer purity (%) |
|---|---|---|---|
| Ab1-L1-D1 | 3.47 | LC/MS | 89.85 |
| Ab1-L1-D6 | 2.30 | HIC | 92.68 |
| Ab1-L1-D7 | 2.08 | HIC | 99.81 |
| Ab1-L1-D10 | 2.17 | HIC | 96.18 |
| Ab1-L1-D11 | 2.27 | HIC | 95.82 |
| Ab1-L1-D12 | 4.40 | HIC | 96.72 |
| Ab1-L2-D12 | 2.51 | HIC | 97.84 |

(continued)

| Ex | DAR | DAR measurement method | Monomer purity (%) |
|---|---|---|---|
| Ab1-L3-D12 | 3.78 | HIC | 98.72 |
| Ab2-L3-D12 | 4.36 | HIC | 98.03 |
| Ab3-L3-D12 | 4.12 | HIC | 98.34 |

[Table 8]

| Ex | CHEM |
|---|---|
| Ab1-L1-D2 | 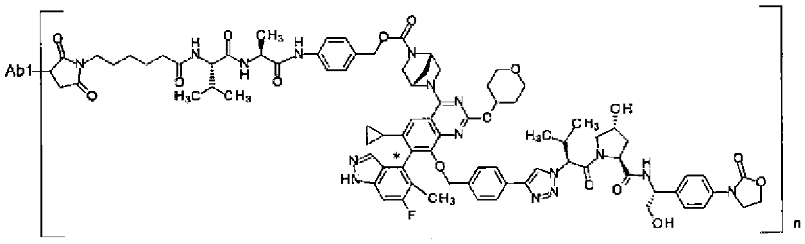 |
| Ab1-L1-D3 | 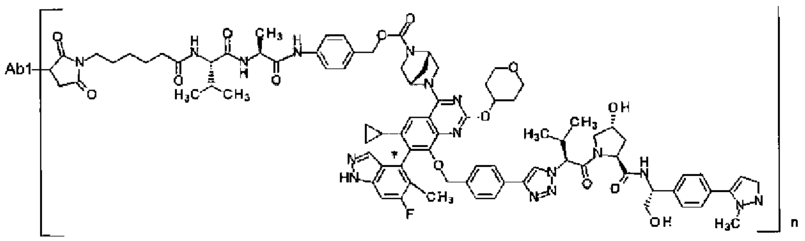 |
| Ab1-L1-D4 | 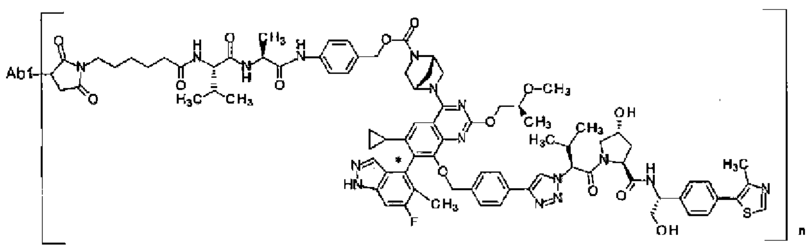 |
| Ab1-L1-D5 | 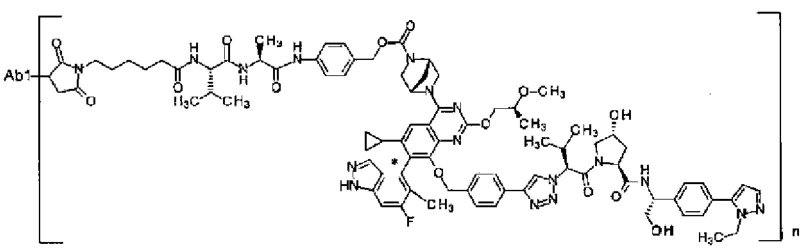 |
| Ab1-L1-D8 | 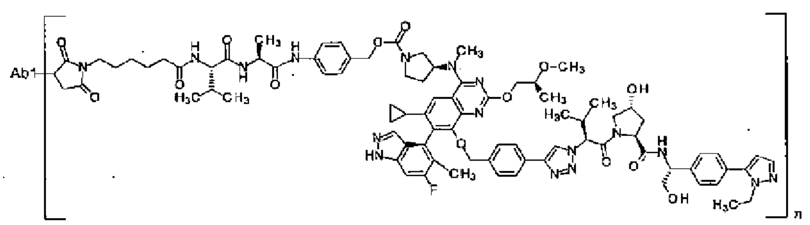 |

(continued)

| Ex | CHEM |
|---|---|
| Ab1-L1-D9 | 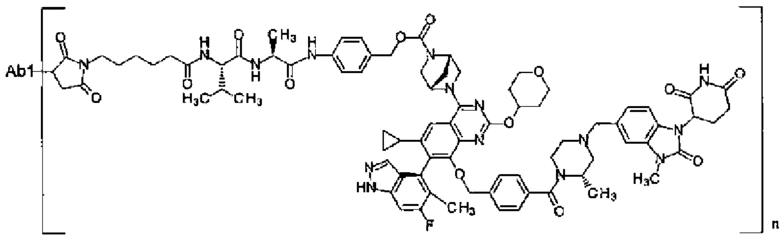 |

[Test Example 1: Evaluation of KRAS-degrading effects of drugs D1 to D11 in human G12D mutant KRAS-positive pancreatic cancer line AsPC-1]

Drugs D1 to D6 were evaluated for their KRAS-degrading effects in accordance with the method of Test Example 1-1 or Test Example 1-2 in International Publication No. WO 2022/173032. The evaluation results are described as results of evaluating Example compounds 8, 20, 23, 48, 70, and 72 in Table 1 or 2 of the patent bulletin, which are incorporated by reference.

Drugs D7 and D8 were evaluated for their KRAS-degrading effects in accordance with the method of Test Example 1 in International Publication No. WO 2023/171781. The evaluation results are described as results of evaluating Example compounds 11 and 13 in Table 1-1 of the patent bulletin, which are incorporated by reference.

Drugs D9 to D11 were evaluated for their KRAS-degrading effects in accordance with the method of Test Example 1 in International Publication No. WO 2024/019103. The evaluation results are described as results of evaluating Example compounds 8, 16, and 10 in Table 1 of the patent bulletin, which are incorporated by reference.

Drug D12 was evaluated for its KRAS-degrading effect in a dephosphorylated form in which an intramolecular phosphoric acid ester site was degraded into -OH, in accordance with the method of Test Example 1 in International Publication No. WO 2023/171781. The evaluation results are described as results of evaluating Example compound 40 in Table 1-1 of the patent bulletin, which are incorporated by reference.

In a table below, D represents No. of an evaluated drug. $DC_{50}$ of D10 was calculated from a molecular weight in terms of 4 hydrochloride. This result indicated that the test substances degrade mutant RAS G12D protein in mutant KRAS G12D-positive cells.

[Table 9]

| D | $DC_{50}$ (nM) | Dmax (%) |
|---|---|---|
| D7 | 10 | - |
| D8 | 10 | - |
| D9 | 13 | 86 |
| D10 | 71 | 86 |
| D11 | 8 | 86 |

[Test Example 2-1: Evaluation of binding activity of cetuximab and cetuximab-drug conjugate against EGFR by ELISA]

The binding activity of cetuximab and cetuximab-drug conjugates against EGFR was detected by use of ELISA.

The cetuximab-drug conjugates used were Ab1-L1-D1, Ab1-L1-D12, Ab1-L1-D6, Abl-L1-D10, Ab1-L1-D11, Ab1-L3-D12, Ab1-L2-D12, and Ab1-L1-D7. Human EGFR protein (Abcam plc, ab155639) was diluted with phosphate buffered saline (PBS; FUJIFILM Wako Pure Chemical Corp., 045-29795), and the solution was added at 20 ng/20 μL/well to Clear Flat-Bottom Immuno Nonsterile 384-Well Plates (Thermo Fisher Scientific Inc., 464718) and left standing overnight at 4°C so that the human EGFR protein was immobilized on the plate. On the next day, supernatants were removed, and each well was washed by the addition of 100 μL of TBS-T (Thermo Fisher Scientific Inc., 28360). Supernatants were removed, and TBS-T containing 20% Blocking One (Nacalai Tesque Inc., 03953-95) was added at 50 μL per well, and left standing at room temperature for 1 hour. The test substance used was cetuximab or a cetuximab-drug conjugate. The test substance was serially diluted (10 points in the range of final concentrations from 10 nM to 0.3 pM) with TBS-T containing 5% Blocking One. Supernatants were removed from the plate, and each well was washed by the

addition of 100 μL of TBS-T. Supernatants were removed, and the diluted test substance was added at 20 μL/well, and left standing at room temperature for 30 minutes. Then, supernatants were removed, and each well was washed by the addition of 100 μL of TBS-T. Washing was carried out a total of three times. Supernatants were removed, and a solution of Goat Anti-Human IgG Fc-HRP (SouthernBiotech, 2014-05) diluted 4000-fold with TBS-T containing 5% Blocking One was added at 20 μL per well, and left standing at room temperature for 30 minutes. Then, supernatants were removed, and each well was washed by the addition of 100 μL of TBS-T. Washing was carried out a total of three times. Supernatants were removed, and TMB Blue Substrate-Chromogen (Agilent Technologies, S1601) was added at 20 μL per well, and left standing at room temperature for 5 minutes. Then, 20 μL of 1 M $H_2SO_4$ was added to each well, and the optical density of each well was measured using Infinite M200PRO (Tecan Trading AG) at a measurement wavelength set to 450 nm and a correction wavelength set to 570 nm. A 50% effective concentration ($EC_{50}$) was calculated by nonlinear regression analysis using a Sigmoid-Emax model.

The binding activity of the test substances against EGFR is shown in a table below. From this result, the cetuximab-drug conjugates were found to have binding activity equivalent to that of cetuximab against EGFR.

[Table 10]

| Ex | $EC_{50}$ (nM) |
|---|---|
| Ab1 | 0.032 |
| Ab1-L1-D1 | 0.064 |
| Ab1-L1-D12 | 0.071 |
| Ab1-L1-D6 | 0.044 |
| Ab1-L1-D10 | 0.045 |
| Ab1-L1-D11 | 0.042 |
| Ab1-L3-D12 | 0.042 |
| Ab1-L2-D12 | 0.041 |
| Ab1-L1-D7 | 0.068 |

[Test Example 2-2: Evaluation of binding activity of trastuzumab and trastuzumab-drug conjugate against HER2 by ELISA]

The binding activity of trastuzumab and a trastuzumab-drug conjugate against HER2 was detected by use of ELISA.

The trastuzumab-drug conjugate used was Ab2-L3-D12. Human Her2/ERBB2 protein (Sino Biological, 10004-H08H) was diluted with PBS, and the solution was added at 10 ng/20 μL/well to Clear Flat-Bottom Immuno Nonsterile 384-Well Plates and left standing overnight at 4°C so that the human Her2/ERBB2 protein was immobilized on the plate. On the next day, supernatants were removed, and each well was washed by the addition of 100 μL of TBS-T. Supernatants were removed, and TBS-T containing 20% Blocking One was added at 50 μL per well, and left standing at room temperature for 1 hour. The test substance used was trastuzumab or a trastuzumab-drug conjugate. The test substance was serially diluted (10 points in the range of final concentrations from 10 nM to 0.3 pM) with TBS-T containing 5% Blocking One. Supernatants were removed from the plate, and each well was washed by the addition of 100 μL of TBS-T. Supernatants were removed, and the diluted test substance was added at 20 μL/well, and left standing at room temperature for 1 hour. Subsequent procedures were performed in the same manner as in Test Example 2-1.

The binding activity of the test substance against HER2 are shown in a table below. From this result, the trastuzumab-drug conjugate was found to have binding activity equivalent to that of trastuzumab against HER2.

[Table 11]

| Ex | $EC_{50}$ (nM) |
|---|---|
| Ab2 | 0.046 |
| Ab2-L3-D12 | 0.095 |

[Test Example 2-3: Evaluation of binding activity of sacituzumab mutant and sacituzumab mutant-drug conjugate against TROP2 by ELISA]

The binding activity of a sacituzumab mutant and a sacituzumab mutant-drug conjugate against TROP2 was detected by use of ELISA.

The sacituzumab mutant-drug conjugate used was Ab3-L3-D12. Human TROP2 protein (Sino Biological, 10428-H08H) was diluted with PBS, and the solution was added at 10 ng/20 μL/well to a 384-well plate, and left standing overnight at 4°C so that the human TROP2 protein was immobilized on the plate. On the next day, supernatants were removed, and each well was washed by the addition of 100 μL of TBS-T. Supernatants were removed, and TBS-T containing 20% Blocking One was added at 50 μL per well, and left standing at room temperature for 1 hour. The test substance used was a sacituzumab mutant or a sacituzumab mutant-drug conjugate. The test substance was serially diluted (10 points in the range of final concentrations from 10 μM to 0.3 pM) with TBS-T containing 5% Blocking One. Subsequent procedures were performed in the same manner as in Test Example 2-2.

As a result of this test, the sacituzumab mutant-drug conjugate was found to have binding activity equivalent to that of sacituzumab mutant against TROP2.

[Test Example 3: Evaluation of KRAS-degrading effect in human G12D mutant KRAS-positive colorectal cancer line GP2d]

The intracellular expression level of KRAS G12D was measured by cell ELISA, and the KRAS-degrading effects of test substances were evaluated.

The test substances used were Abl-L1-D1, Abl-L1-D12, Abl-L1-D6, Abl-L1-D10, Abl-L1-D11, Ab1-L3-D12, Ab1-L2-D12, and Abl-L1-D7. GP2d cells (ECACC, 95090714) were suspended in DMEM (Sigma-Aldrich Co., LLC, D6429) medium (hereinafter, "DMEM medium") containing 10% fetal bovine serum (Cytiva, SH30071.03 or SH30070.03") and inoculated at 36 μL ($2.0 \times 10^4$ cells) per well to a 384-well plate (Greiner Bio-One International GmbH, 781182). Cell culture was performed at 37°C in the presence of 5% $CO_2$. On the next day, a test substance (8 to 10 points in the range of final concentrations from 1000 nM to 0.01 nM) and PBS, a vehicle for the test substance, as a negative control were diluted 2-fold with a medium, each added at 4 μL per well, and then cultured overnight.

On the next day, culture supernatants were removed, and a 4% paraformaldehyde phosphate buffer solution (FUJIFILM Wako Pure Chemical Corp., 163-20145) was added at 20 μL per well and left standing at room temperature for 30 minutes to fix the cells. Then, supernatants were removed, and PBS containing 0.1% Triton X-100 (FUJIFILM Wako Pure Chemical Corp., 169-21105 or Amersham Biosciences, 17-1315-01) was added at 20 μL per well. After being left standing at room temperature for 10 minutes, each well was washed with PBS. Next, supernatants were removed, and PBS containing 0.5% sodium dodecyl sulfate (SDS; Invitrogen Corp., 15553-035) was added at 20 μL per well. After being left standing at room temperature for 10 minutes, each well was washed with PBS. Supernatants were removed, and a blocking solution was added at 20 μL per well. After being left standing at room temperature for 30 minutes, supernatants were removed. A solution of an anti-β-actin antibody (Anti-beta Actin antibody [mAbcam 8226]) (Abcam plc, ab8226) diluted 1000-fold with a blocking solution was added as a primary antibody to positive control wells, and a solution of an anti-Ras (G12D Mutant Specific) (Cell Signaling Technology, Inc., #14429) antibody and an anti-β-actin antibody diluted 1000-fold with a blocking solution was added to the other wells so as to attain 15 to 20 μL per well, which was then left standing overnight at 4°C. On the next day, each well was washed with PBS. Supernatants were removed, and a solution of Donkey anti-Mouse IgG H&L (IRDye 680RD) (Li-COR Biosciences, 926-68072) and Goat anti-Rabbit IgG H&L (IRDye 800CW) (Li-COR Biosciences, 926-32211) diluted 1000-fold with a blocking solution was added as a secondary antibody at 15 μL per well. After being left standing at room temperature for 1 hour, each well was washed with PBS. Supernatants were removed, and then, the plate was dried in air at room temperature for 2 hours or longer. Fluorescent signals were measured at 721 to 740 nm (channel 700) and 816 to 840 nm (channel 800) using Odyssey M (LI-COR Biosciences). The signal value of RAS G12D measured at the fluorescent wavelength of 816 to 840 nm was corrected with the signal value of β-actin measured at the fluorescent wavelength of 721 to 740 nm. The signal value obtained by the addition of PBS was defined as 0%, and the signal value obtained by staining with only the anti-β-actin antibody was defined as 100%. The rate of degradation at a compound concentration that exhibited the highest degrading effect was defined as Dmax. The 50% degradation value ($DC_{50}$) of the amount of RAS G12D was calculated by nonlinear regression analysis using a Sigmoid-Emax model. Results of evaluating the test substances are shown in a table below. 45% degradation@1000nM represents that the test substance degraded 45% mutant RAS G12D protein at a concentration of 1000 nM. From this result, the cetuximab-drug conjugates were found to degrade mutant RAS G12D protein in mutant KRAS G12D-positive cells.

[Table 12]

| Ex | $DC_{50}$ (nM) | Dmax (%) |
|---|---|---|
| Ab1-L1-D1 | 7.9 | 56 |
| Ab1-L1-D12 | 0.28 | 83 |
| Ab1-L1-D6 | 1.5 | 79 |
| Ab1-L1-D10 | 45% degradation@1000nM | |
| Ab1-L1-D11 | 29 | 66 |
| Ab1-L3-D12 | 26 | 56 |
| Ab1-L2-D12 | 0.35 | 88 |
| Ab1-L1-D7 | 0.82 | 84 |

[Test Example 4: Evaluation of anchorage-independent cell growth-inhibiting effect in human G12D mutant KRAS-positive colorectal cancer line GP2d]

Test substances were evaluated for their anchorage-independent cell growth-inhibiting effects by spheroid three-dimensional culture. The test substances used were Abl-L1-D1, Ab1-L1-D12, Ab1-L1-D6, Abl-L1-D10, Ab1-L1-D11, Abl-L3-D12, Ab1-L2-D12, and Abl-L1-D7.

GP2d cells were inoculated at 36 μL/well (5 x $10^2$ cells per well) to a low-cell adsorption U-bottom 384-well plate (PrimeSurface: Sumitomo Bakelite Co., Ltd., MS-9384w). Cell culture conditions were performed at 37°C in the presence of 5% $CO_2$ using DMEM medium containing 10% fetal bovine serum. On the next day, a test substance (8 to 10 points in the range of final concentrations from 1000 nM to 0.01 nM) and PBS, a vehicle for the test substance, as a negative control were diluted 2-fold with a fresh medium, each added at 4 μL per well. After culture at 37°C for 6 days in the presence of 5% $CO_2$, CellTiter-Glo 2.0 (Promega Corp., G9243) was added at 20 μL per well. After stirring at ordinary temperature for 1 hour using a plate mixer (FINEPCR), luminescent signals were measured with ARVO X3 (PerkinElmer, Inc.). The signal value obtained by PBS treatment was defined as 100%, and the signal value obtained using only the medium in the absence of cells was defined as 0%. A 50% inhibition value ($IC_{50}$) was calculated by nonlinear regression analysis using a Sigmoid-Emax model. Results of evaluating the test substances are shown in a table below. 51% inh@300nM represents that the test substance had 51% inhibitory activity at a concentration of 300 nM. From this result, the cetuximab-drug conjugates exhibited a cell growth-inhibiting effect on mutant KRAS G12D-positive cells.

[Table 13]

| Ex | $IC_{50}$ (nM) |
|---|---|
| Ab1-L1-D1 | 2.8 |
| Ab1-L1-D12 | 9.2 |
| Ab1-L1-D6 | 17 |
| Ab1-L1-D10 | 221 |
| Ab1-L1-D11 | 64 |
| Ab1-L3-D12 | 51% inh@300nM |
| Ab1-L2-D12 | 12 |

[Test Example 5: Evaluation of antitumor effect in mouse bearing human G12D mutant KRAS-positive colorectal cancer line GP2d]

GP2d cells are cultured at 37°C in the presence of 5% $CO_2$ using DMEM medium containing 10% fetal bovine serum. The GP2d cells are recovered, suspended in PBS, and prepared into 4.0 × $10^7$ cells/mL by the addition of a 2-fold amount of VitroGel Hydrogel Matrix (TheWell Bioscience Inc., VHM01), and the cell suspension is subcutaneously inoculated in a volume of 100 μL to a 4 to 5-week-old male nude mouse (BALB/c-nu(nu/nu), The Jackson Laboratory Japan, Inc.). Approximately 2 weeks after inoculation, grouping is performed such that tumor volumes and body weights are equivalent among groups. The administration of a test substance is started from the next day. The test is conducted using 5 animals each of the vehicle group and the test substance administration group. The test substance is diluted with

physiological saline (Otsuka Pharmaceutical Factory). The test substance or a vehicle control is administered into the tail vein. The administration is performed twice at an interval of once a week. Tumor sizes and body weights are measured twice a week. The tumor volumes are calculated according to the following expression.

$$[\text{Tumor volume (mm}^3)] = [\text{Major axis (mm) of tumor}] \times [\text{Minor axis (mm) of tumor}]^2 \times 0.5$$

The rate (%) of inhibition of tumor growth by the test substance is calculated by defining the tumor volume of the test substance administration group on the day before the start of administration as 100% inhibition, and the tumor volume of the vehicle group after 2 weeks from the initial administration as 0% inhibition. When the tumor volume of the test substance administration group falls below the tumor volume on the day before the start of administration, the rate (%) of tumor regression of the test substance is calculated by defining the tumor volume on the day before the start of administration as 0% regression, and a tumor volume of 0 as 100% regression.

INDUSTRIAL APPLICABILITY

The antibody-drug conjugate of the present invention or the salt thereof is excellent in G12D mutant KRAS protein degradation-inducing effect and can be used as an active ingredient in a pharmaceutical composition, for example, a pharmaceutical composition for the treatment of pancreatic cancer. Moreover, the drug-linker conjugate of the present invention or the salt thereof contains a compound having a G12D mutant KRAS protein degradation-inducing effect and can be used in the synthesis of the antibody-drug conjugate of the present invention or the salt thereof.

SEQUENCE LISTING FREE TEXT

SEQ ID NO: 1 is the amino acid sequence of the heavy chain of cetuximab, and SEQ ID NO: 2 is the amino acid sequence of the light chain of cetuximab. SEQ ID NO: 3 is the amino acid sequence of the heavy chain variable region of BA03, and SEQ ID NO: 4 is the amino acid sequence of the light chain variable region of BA03.

## Claims

1. An antibody-drug conjugate of formula (I) or a salt thereof:

[Chemical Formula 1]

$$\mathrm{Ab}-\!\left(\mathrm{L}^{\mathrm{A}}-\mathrm{D}\right)_n$$

(I)

wherein

Ab is an antibody or an antigen binding fragment thereof,
D is a heterocyclic compound having a G12D mutant KRAS protein degradation-inducing effect,
$L^A$ is a linker for connecting Ab and D, and
n is a number of 1 to 20.

2. The compound or the salt thereof according to claim 1, wherein

D is a heterocyclic compound represented by formula (II),

[Chemical Formula 2]

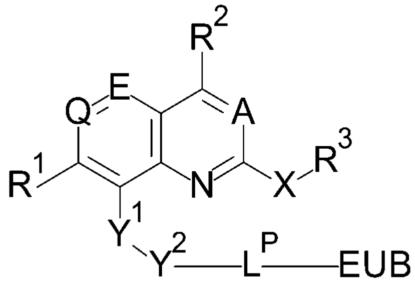

(II)

wherein
A is $CR^A$ or N,
$R^A$ is H, cyano, or optionally substituted $C_{1-3}$ alkyl,
Q is $CR^Q$ or N,
$R^Q$ is H, halogen, $C_{3-6}$ cycloalkyl, vinyl, or optionally substituted $C_{1-3}$ alkyl,
E is CH or N,
$R^1$ is naphthyl optionally substituted by one or two groups selected from the group consisting of cyano, OH, halogen, and optionally substituted $C_{1-3}$ alkyl, or is one group selected from the group consisting of the following formula (III), formula (IV), and formula (V):

[Chemical Formula 3]

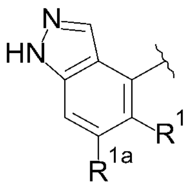

(III)

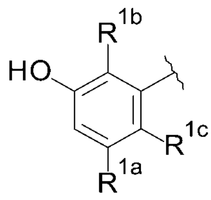

(IV)

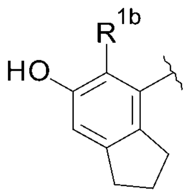

(V)

$R^{1a}$, $R^{1b}$, and $R^{1c}$ are each independently H, vinyl, halogen, or optionally substituted $C_{1-3}$ alkyl,
$R^2$ is $-V^1-V^2$ or W,
$V^1$ is a bond, $-CH_2-$, -O-, -S-, or $-N(R^{V1})-$,
$R^{V1}$ is H or optionally substituted $C_{1-3}$ alkyl,
$V^2$ is one group selected from the group consisting of the following formula (VI) and formula (VII):

[Chemical Formula 4]

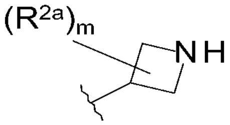

(VI)

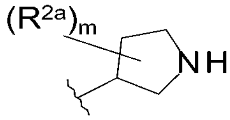

(VII)

W is one group selected from the group consisting of the following formula (VIII), formula (IX), formula (X), formula (XI), formula (XII), formula (XIII), formula (XIV), formula (XV), and formula (XVI):

[Chemical Formula 5]

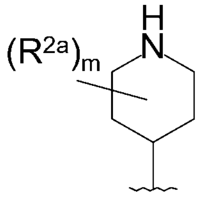 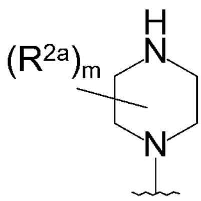 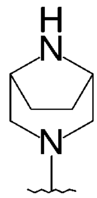 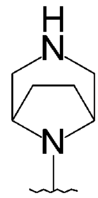

(VIII) (IX) (X) (XI)

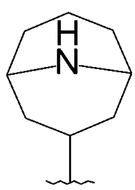 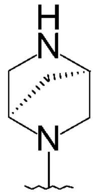 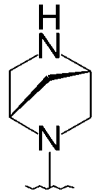 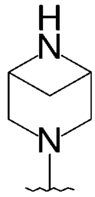 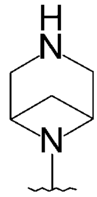

(XII) (XIII) (XIV) (XV) (XVI)

$R^{2a}$ are each independently OH, $OCH_3$, F, or optionally substituted $C_{1-3}$ alkyl, wherein $R^{2a}$ is bonded only to a carbon atom serving as a constituting atom of a ring selected from the group consisting of an azetidine ring represented by formula (VI), a pyrrolidine ring represented by formula (VII), a piperidine ring represented by formula (VIII), and a piperazine ring represented by formula (IX),
m is an integer of 0 to 2,
$R^3$ is optionally substituted $C_{1-6}$ alkyl, optionally substituted hetero cycloalkyl, or optionally substituted heteroaryl,
X is a bond, $-CH_2-$, -O-, -S-, or $-NR^{4X}-$,
$R^{4X}$ is H or optionally substituted $C_{1-3}$ alkyl,
$Y^1$ is -O-(optionally substituted $C_{1-3}$ alkylene)-$^{*Y2}$, -S-(optionally substituted $C_{1-3}$ alkylene)-$^{*Y2}$, $-SO_2$-(optionally substituted $C_{1-3}$ alkylene)-$^{*Y2}$, $-NR^Y$-(optionally substituted $C_{1-3}$ alkylene)-$^{*Y2}$, -(optionally substituted $C_{1-3}$ alkylene)-O-$^{*Y2}$, -(optionally substituted $C_{1-3}$ alkylene)-S-$^{*Y2}$, -(optionally substituted $C_{1-3}$ alkylene)-$SO_2$-$^{*Y2}$, or -(optionally substituted $C_{1-3}$ alkylene)-$NR^Y$-$^{*Y2}$ (wherein $^{*Y2}$ represents a site of connection to $Y^2$),
$R^Y$ is H or optionally substituted $C_{1-3}$ alkyl,
$Y^2$ is a bond, optionally substituted phenylene, or optionally substituted heteroarylene,
$L^P$ is a group that chemically bonds $Y^2$ to EUB,
EUB is a group having the ability to bind to one E3 ubiquitin ligase selected from the group consisting of VHL, celebron, IAP, MDM2, DCAF11, DCAF15, DCAF16, BIRC2, KEAP1, RNF4, RNF114, FEM1B, and AhR, and
$L^A$ is a linker for connecting Ab and D, wherein $L^A$ is bonded to an arbitrary nitrogen atom of -NH- or oxygen atom of -OH contained in D.

**3.** The antibody-drug conjugate or the salt thereof according to claim 2, wherein

$V^2$ is one group selected from the group consisting of the following formula (VIa) and formula (VIIa) (wherein $^{*LA}$ represents a site of connection to $L^A$):

[Chemical Formula 6]

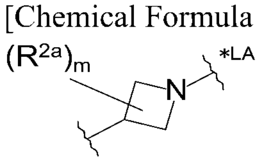 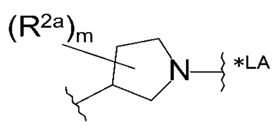

(VIa) (VIIa)

W is one group selected from the group consisting of the following formula (VIIIa), formula (IXa), formula (Xa), formula (XIa), formula (XIIa), formula (XIIIa), formula (XIVa), formula (XVa), and formula (XVIa) (wherein $^{*LA}$ represents a site of connection to $L^A$):

[Chemical Formula 7]

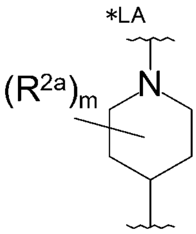
(VIIIa)
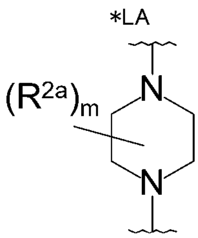
(IXa)
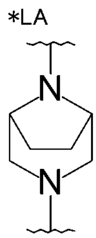
(Xa)
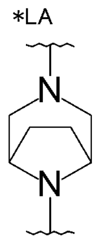
(XIa)

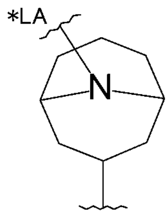
(XIIa)
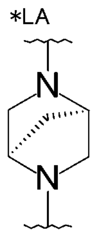
(XIIIa)
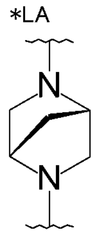
(XIVa)
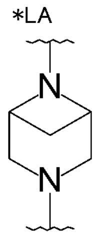
(XVa)
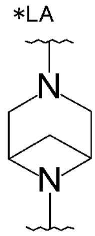
(XVIa)

and

$L^A$ is a linker for connecting Ab and D, wherein $L^A$ is bonded to D through a nitrogen atom contained in $V^2$ or W at the site of connection represented by $^{*LA}$.

**4.** The antibody-drug conjugate or the salt thereof according to claim 2, wherein

A is N,
Q is $CR^Q$, $R^Q$ is cyclopropyl,
E is CH,
$R^1$ is the following formula (III-2):

[Chemical Formula 8]

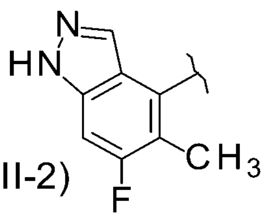
(III-2)

$R^2$ is $-V^1-V^2$ or W,
$V^1$ is $-N(CH_3)-$,
$V^2$ is the following formula (VII-2):

[Chemical Formula 9]

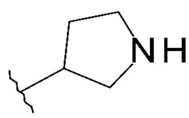
(VII-2)

W is the following formula (XIV):

[Chemical Formula 10]

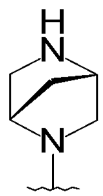

(XIV)

$R^3$ is n-propyl optionally substituted by $-OCH_3$, or tetrahydropyranyl,
X is -O-,
$Y^1$ is -O-(methylene)-$^{*Y2}$ (wherein $^{*Y2}$ represents a site of connection to $Y^2$),
$Y^2$ is phenylene,
$L^P$ is a group that chemically bonds $Y^2$ to EUB, and
EUB is a group having the ability to bind to one E3 ubiquitin ligase selected from the group consisting of VHL and celebron.

**5.** The antibody-drug conjugate or the salt thereof according to claim 4, wherein

$V^2$ is the following formula (VII-2a) (wherein $^{*LA}$ represents a site of connection to $L^A$):

[Chemical Formula 11]

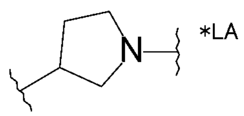

(VII-2a)

W is the following formula (XIVa) (wherein $^{*LA}$ represents a site of connection to $L^A$):

[Chemical Formula 12]

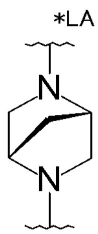

(XIVa)

and
$L^A$ is a linker for connecting Ab and D, wherein $L^A$ is bonded to D through a nitrogen atom contained in $V^2$ or W at the site of connection represented by $^{*LA}$.

**6.** The antibody-drug conjugate or the salt thereof according to claim 1 or 2, wherein D is (4R)-1-[(2S)-2-(4-{4-[({6-cyclopropyl-4-[(1S,4S)-2,5-diazabicyclo[2.2.1]heptan-2-yl]-7-(6-fluoro-5-methyl-1H-indazol-4-yl)-2-[(oxan-4-yl)oxy]quinazolin-8-yl}oxy)methyl]phenyl}-1H-1,2,3-triazol-1-yl)-3-methylbutanoyl]-4-hydroxy-N-{(1R)-2-hydroxy-1-[4-(4-methyl-1,3-thiazol-5-yl)phenyl]ethyl}-L-prolinamide, (4R)-1-[(2S)-2-(4-{4-[({6-cyclopropyl-4-[(1S,4S)-2,5-diazabicyclo[2.2.1]heptan-2-yl]-7-(6-fluoro-5-methyl-1H-indazol-4-yl)-2-[(oxan-4-yl)oxy]quinazolin-8-yl}oxy)methyl]phenyl}-1H-1,2,3-triazol-1-yl)-3-methylbutanoyl]-4-hydroxy-N-{(1R)-2-hydroxy-1-[4-(2-oxo-1,3-oxazolidin-3-yl)phenyl]ethyl}-L-prolinamide, (4R)-1-[(2S)-2-(4-{4-[({6-cyclopropyl-4-[(1S,4S)-2,5-diazabicyclo[2.2.1]heptan-2-yl]-7-(6-fluoro-5-methyl-1H-indazol-4-yl)-2-[(oxan-4-yl)oxy]quinazolin-8-yl}oxy)methyl]phenyl}-1H-1,2,3-triazol-1-yl)-3-methylbutanoyl]-4-hydroxy-N-{(1R)-2-hydroxy-1-[4-(1-methyl-1H-pyrazol-5-yl)phenyl]ethyl}-L-prolinamide, (4R)-1-[(2S)-2-(4-{4-[({6-cyclopropyl-4-[(1S,4S)-2,5-diazabicyclo[2.2.1]heptan-2-yl]-7-(6-fluoro-5-methyl-1H-indazol-4-yl)-2-[(2S)-2-methoxypropoxy]quinazolin-8-yl}oxy)methyl]phenyl}-1H-1,2,3-triazol-1-yl)-3-methylbutanoyl]-4-hydroxy-N-{(1R)-2-hydroxy-1-[4-(4-methyl-1,3-thiazol-5-yl)phenyl]ethyl}-L-prolinamide, (4R)-1-[(2S)-2-(4-{4-[({6-cyclopropyl-4-[(1S,4S)-2,5-diazabicyclo[2.2.1]heptan-2-yl]-7-(6-fluoro-5-methyl-1H-indazol-4-yl)-2-[(2S)-2-methoxypropoxy]quinazolin-8-yl}oxy)methyl]phenyl}-1H-1,2,3-triazol-1-yl)-3-methylbutanoyl]-N-{(1R)-1-[4-(1-ethyl-1H-pyrazol-5-yl)phenyl]-2-hydroxyethyl}-4-hydroxy-L-prolinamide, (4R)-1-[(2S)-2-(4-{4-[({6-cyclopropyl-4-[(1S,4S)-2,5-diazabicyclo[2.2.1]heptan-2-yl]-7-(6-fluoro-5-methyl-1H-indazol-4-yl)-2-[(2S)-2-methoxypropoxy]quinazolin-8-yl}oxy)methyl]phenyl}-1H-1,2,3-triazol-1-yl)-3-methylbutanoyl]-4-hydroxy-N-{(1R)-2-hydroxy-1-[4-(4-methyl-1,3-oxazol-5-yl)phenyl]ethyl}-L-prolinamide, (4R)-1-[(2S)-2-{4-[4-({[(7M)-6-cyclopropyl-7-(6-fluoro-5-methyl-1H-indazol-4-yl)-2-[(2S)-2-methoxypropoxy]-4-{methyl[(3S)-pyrrolidin-3-yl]amino}quinazolin-8-yl]oxy}methyl)phenyl]-1H-1,2,3-triazol-1-yl}-3-methylbutanoyl]-4-hydroxy-N-{(1R)-2-hydroxy-1-[4-(4-methyl-1,3-thiazol-5-yl)phenyl]ethyl}-L-prolinamide, (4R)-1-[(2S)-2-{4-[4-({[(7M)-6-cyclopropyl-7-(6-fluoro-5-methyl-1H-indazol-4-yl)-2-[(2S)-2-methoxypropoxy]-4-{methyl[(3S)-pyrrolidin-3-yl]amino}quinazolin-8-yl]oxy}methyl)phenyl]-1H-1,2,3-triazol-1-yl}-3-methylbutanoyl]-N-{(1R)-1-[4-(1-ethyl-1H-pyrazol-5-yl)phenyl]-2-hydroxyethyl}-4-hydroxy-L-prolinamide, 3-(5-{[(3S)-4-{4-[({(7M)-6-cyclopropyl-4-[(1S,4S)-2,5-diazabicyclo[2.2.1]heptan-2-yl]-7-(6-fluoro-5-methyl-1H-indazol-4-yl)-2-[(oxan-4-yl)oxy]quinazolin-8-yl}oxy)methyl]benzoyl}-3-methylpiperazin-1-yl]methyl}-3-methyl-2-oxo-2,3-dihydro-1H-benzimidazol-1-yl)piperidine-2,6-dione, 1-(6-{[(3S)-4-{4-[({(7M)-6-cyclopropyl-4-[(1S,4S)-2,5-diazabicyclo[2.2.1]heptan-2-yl]-7-(6-fluoro-5-methyl-1H-indazol-4-yl)-2-[(oxan-4-yl)oxy]quinazolin-8-yl}oxy)methyl]benzoyl}-3-methylpiperazin-1-yl]methyl}-1-methyl-1H-indazol-3-yl)-1,3-diazinane-2,4-dione, or 3-(5-{[(3S)-4-{4-[({(7M)-6-cyclopropyl-4-[(1S,4S)-2,5-diazabicyclo[2.2.1]heptan-2-yl]-7-(6-fluoro-5-methyl-1H-indazol-4-yl)-2-[(2S)-2-methoxypropoxy]quinazolin-8-yl}oxy)methyl]benzoyl}-3-methylpiperazin-1-yl]methyl}-3-methyl-2-oxo-2,3-dihydro-1H-benzimidazol-1-yl)piperidine-2,6-dione, or (2R)-2-({(4R)-1-[(2S)-2-{4-[4-({[(7M)-6-cyclopropyl-7-(6-fluoro-5-methyl-1H-indazol-4-yl)-2-[(2S)-2-methoxypropoxy]-4-{methyl[(3S)-pyrrolidin-3-yl]amino}quinazolin-8-yl]oxy}methyl)phenyl]-1H-1,2,3-triazol-1-yl}-3-methylbutanoyl]-4-hydroxy-L-prolyl}amino)-2-[4-(1H-1,2,4-triazol-1-yl)phenyl]ethyl dihydrogen phosphate.

**7.** The antibody-drug conjugate or the salt thereof according to any one of claims 1 to 6, wherein

$L^A$ is a linker represented by formula (XXVIII):

[Chemical Formula 13]

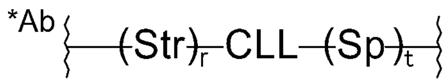

(XXVIII)

wherein
Str is a stretcher unit and is bonded to Ab and CLL, r is 0 or 1,
CLL is a partial structure cleavable *in vivo*,
Sp is a spacer unit and is bonded to CLL and D, t is 0 or 1, and
*Ab represents a site of connection to Ab.

**8.** The antibody-drug conjugate or the salt thereof according to claim 7, wherein Str is a stretcher unit represented by formula (ST-1), and r is 1:

[Chemical Formula 14]

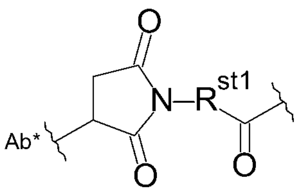

(ST-1)

wherein $R^{st1}$ is optionally substituted $C_{1-12}$ alkylene, $-(CH_2CH_2O)_{a1}-C_{1-6}$ alkylene-, $-C_{1-6}$ alkylene-$(OCH_2CH_2)_{a2}$-, -optionally substituted $C_{1-6}$ alkylene-NH-C(=O)-optionally substituted $C_{1-6}$ alkylene-, -optionally substituted $C_{1-6}$ alkylene-C(=O)-NH-optionally substituted $C_{1-6}$ alkylene-, optionally substituted $C_{1-6}$ alkylene-C(=O)-NH-$(CH_2CH_2O)_{a3}-C_{1-6}$ alkylene-, or optionally substituted $C_{1-6}$ alkylene-NH-C(=O)-$(CH_2CH_2O)_{a4}-C_{1-6}$ alkylene-, each of a1, a2, a3, and a4 is an integer of 1 to 10, and $^{*Ab}$ represents a site of connection to Ab.

**9.** The antibody-drug conjugate or the salt thereof according to claim 8, wherein $R^{st1}$ is $C_5$ alkylene.

**10.** The antibody-drug conjugate or the salt thereof according to claim 7, wherein CLL is a partial structure cleavable *in vivo* represented by formula (CL-1):

[Chemical Formula 15]

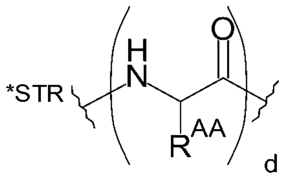

(CL-1)

wherein $R^{AA}$ are each independently H, methyl, isopropyl, isobutyl, sec-butyl, benzyl, p-hydroxybenzyl, hydroxymethyl, 1-hydroxyethyl, $-CH_2-C(=O)-OH$, $-(CH_2)_2-C(=O)-OH$, - $CH_2-C(=O)-NH_2$, $-(CH_2)_2-C(=O)-NH_2$, $-(CH_2)_4-NH_2$, $-(CH_2)_3-NH-C(=NH)-NH_2$, $-(CH_2)_3-NH-C(=O)-NH_2$, $-CH_2-SH$, or $-CH_2-S-CH_3$, or one group selected from the group consisting of the following formula (RAA-1) and (RAA-2):

[Chemical Formula 16]

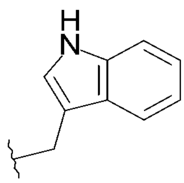

(RAA-1)

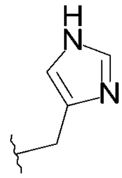

(RAA-2)

d is an integer of 1 to 6, and $^{*STR}$ represents a site of connection to Str.

**11.** The antibody-drug conjugate or the salt thereof according to claim 10, wherein $R^{AA}$ are each independently methyl or isopropyl, and d is 2.

**12.** The antibody-drug conjugate or the salt thereof according to claim 10, wherein $R^{AA}$ are each independently isopropyl or $-(CH_2)_3-NH-C(=O)-NH_2$, and d is 2.

**13.** The antibody-drug conjugate or the salt thereof according to claim 10, wherein $R^{AA}$ are each independently H or benzyl, and d is 4.

**14.** The antibody-drug conjugate or the salt thereof according to claim 7, wherein Sp is a spacer unit represented by formula (SP-1), and t is 1:

[Chemical Formula 17]

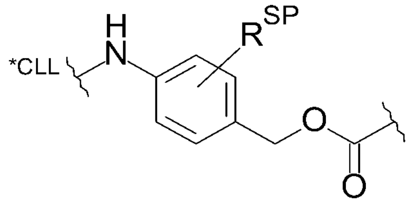

(SP-1)

wherein $R^{SP}$ is H, $C_{1-6}$ alkyl, -O-$C_{1-6}$ alkyl, halogen, or halogeno $C_{1-6}$ alkyl, and $^{*CLL}$ represents a site of connection to CLL.

**15.** The antibody-drug conjugate or the salt thereof according to claim 14, wherein $R^{SP}$ is H.

**16.** The antibody-drug conjugate or the salt thereof according to claim 7, wherein t is 0.

**17.** The antibody-drug conjugate or the salt thereof according to claim 1, wherein formula (I) is represented by formula (AD-1a) or (AD-2a):

[Chemical Formula 18]

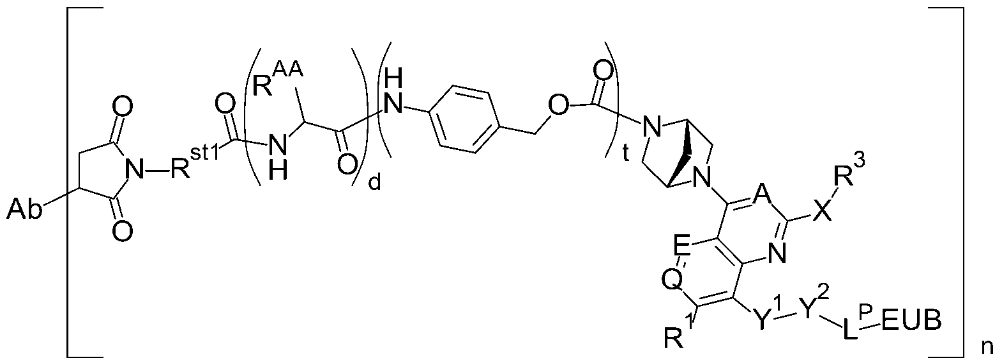

(AD-1a)

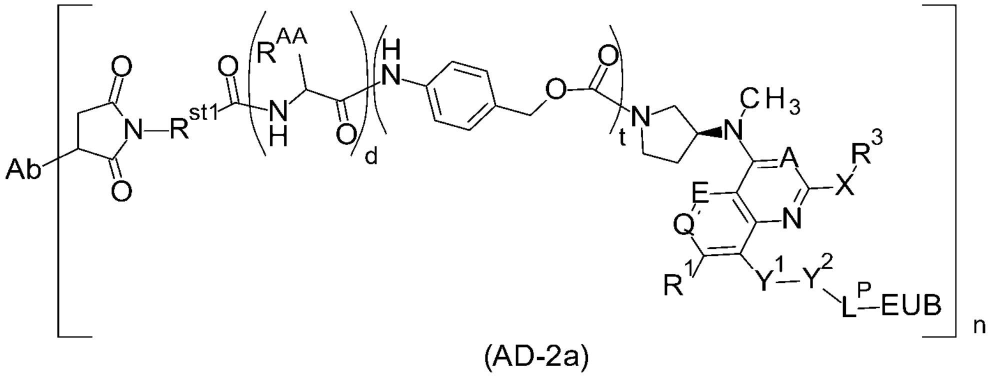

(AD-2a)

wherein

Ab is an antibody or an antigen binding fragment thereof,
A is $CR^A$ or N,
$R^A$ is H, cyano, or optionally substituted $C_{1-3}$ alkyl,
Q is $CR^Q$ or N,
$R^Q$ is H, halogen, $C_{3-6}$ cycloalkyl, vinyl, or optionally substituted $C_{1-3}$ alkyl,
E is CH or N,
$R^1$ is naphthyl optionally substituted by one or two groups selected from the group consisting of cyano, OH,

halogen, and optionally substituted $C_{1-3}$ alkyl, or is one group selected from the group consisting of the following formula (III), formula (IV), and formula (V):

[Chemical Formula 19]

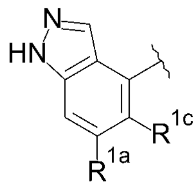

(III)

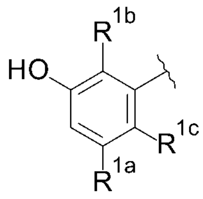

(IV)

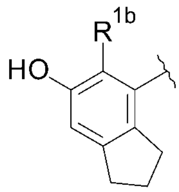

(V)

$R^{1a}$, $R^{1b}$, and $R^{1c}$ are each independently H, vinyl, halogen, or optionally substituted $C_{1-3}$ alkyl,
$R^3$ is optionally substituted $C_{1-6}$ alkyl, optionally substituted hetero cycloalkyl, or optionally substituted heteroaryl,
X is a bond, $-CH_2-$, -O-, -S-, or $-NR^{4X}-$,
$R^{4X}$ is H or optionally substituted $C_{1-3}$ alkyl,
$Y^1$ is -O-(optionally substituted $C_{1-3}$ alkylene)-$^{*Y2}$, -S-(optionally substituted $C_{1-3}$ alkylene)-$^{*Y2}$, $-SO_2$-(optionally substituted $C_{1-3}$ alkylene)-$^{*Y2}$, $-NR^Y$-(optionally substituted $C_{1-3}$ alkylene)-$^{*Y2}$, -(optionally substituted $C_{1-3}$ alkylene)-O-$^{*Y2}$, -(optionally substituted $C_{1-3}$ alkylene)-S-$^{*Y2}$, -(optionally substituted $C_{1-3}$ alkylene)-$SO_2$-$^{*Y2}$, or -(optionally substituted $C_{1-3}$ alkylene)-$NR^Y$-$^{*Y2}$ (wherein $^{*Y2}$ represents a site of connection to $Y^2$),
$R^Y$ is H or optionally substituted $C_{1-3}$ alkyl,
$Y^2$ is a bond, optionally substituted phenylene, or optionally substituted heteroarylene,
$L^P$ is a group that chemically bonds $Y^2$ to EUB,
EUB is a group having the ability to bind to one E3 ubiquitin ligase selected from the group consisting of VHL, celebron, IAP, MDM2, DCAF11, DCAF15, DCAF16, BIRC2, KEAP1, RNF4, RNF114, FEM1B, and AhR,
$R^{st1}$ is $C_{1-12}$ alkylene,
$R^{AA}$ are each independently H, methyl, isopropyl, benzyl, or $-(CH_2)_3-NH-C(=O)-NH_2$, d is an integer of 2 to 4,
t is 0 or 1, and
n is a number of 1 to 20.

**18.** The antibody-drug conjugate or the salt thereof according to claim 1, wherein formula (I) is represented by formula (AD-3a), (AD-4a), (AD-5a), (AD-6a), (AD-7a), (AD-8a), (AD-9a), (AD-10a), (AD-11a), (AD-12a), (AD-13a), or (AD-25a):

[Chemical Formula 20-1]

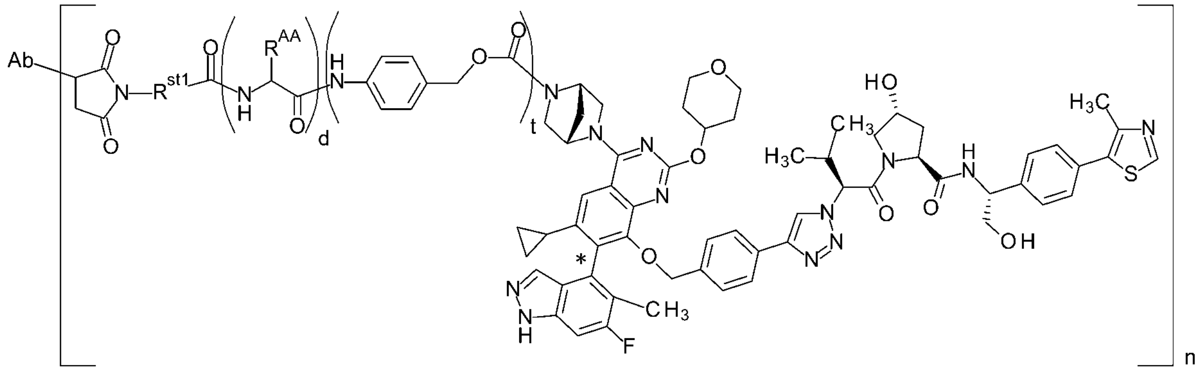

(AD-3a)

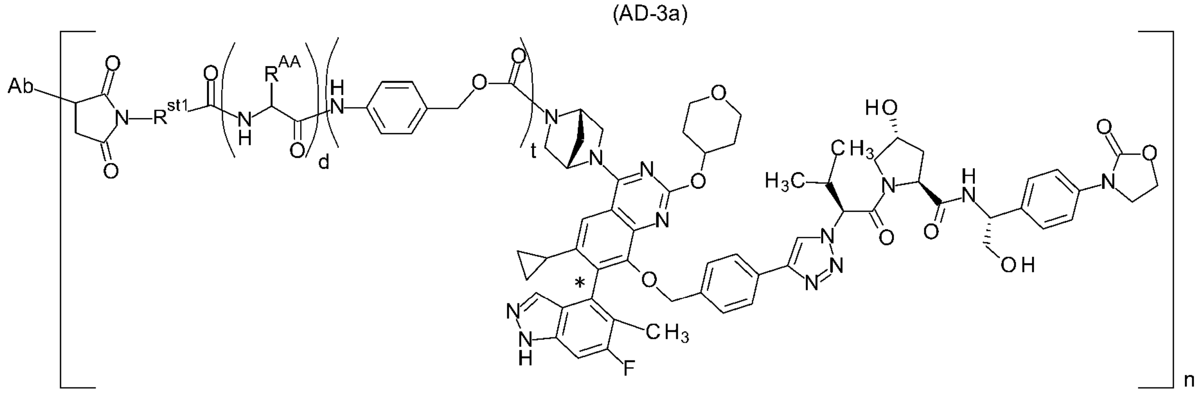

(AD-4a)

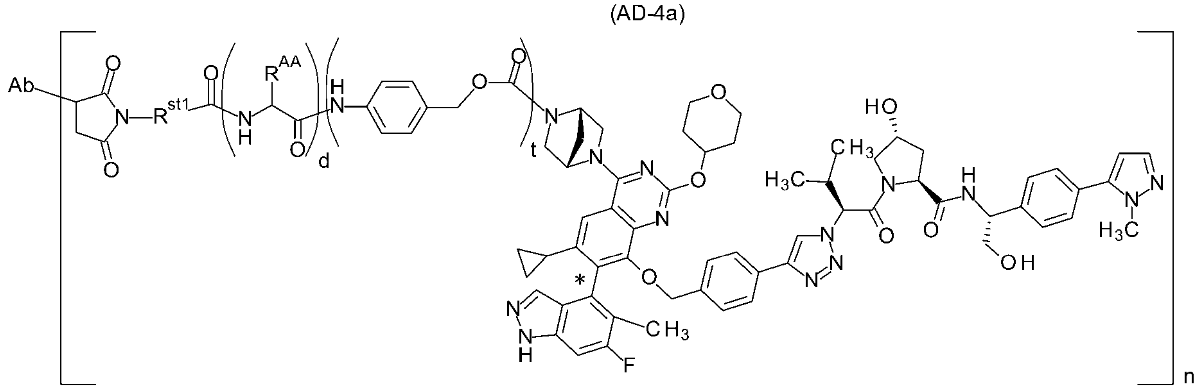

(AD-5a)

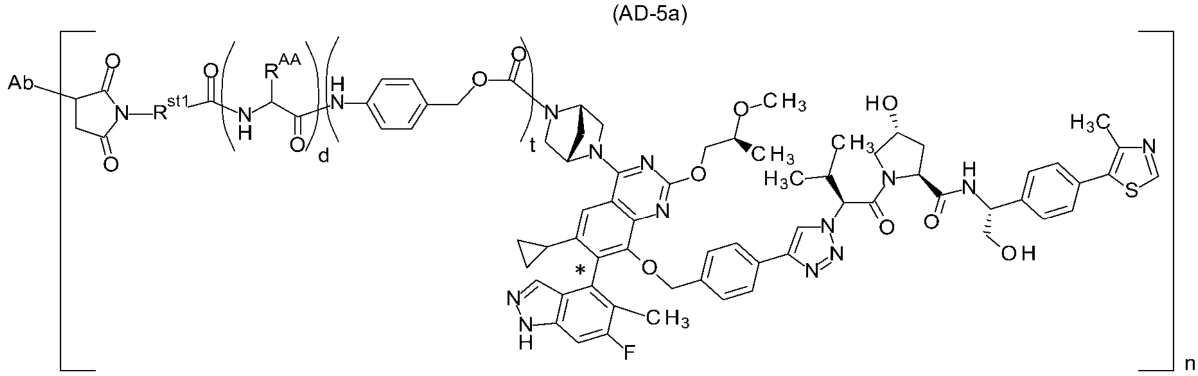

(AD-6a)

[Chemical Formula 20-2]

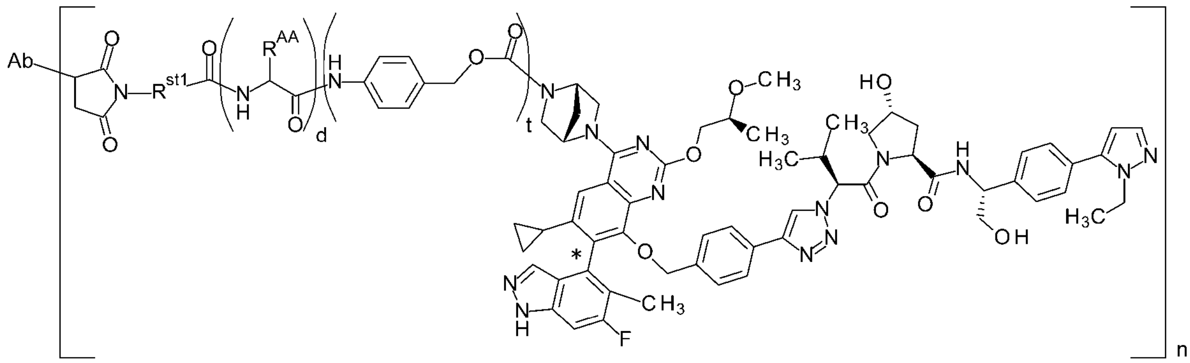

(AD-7a)

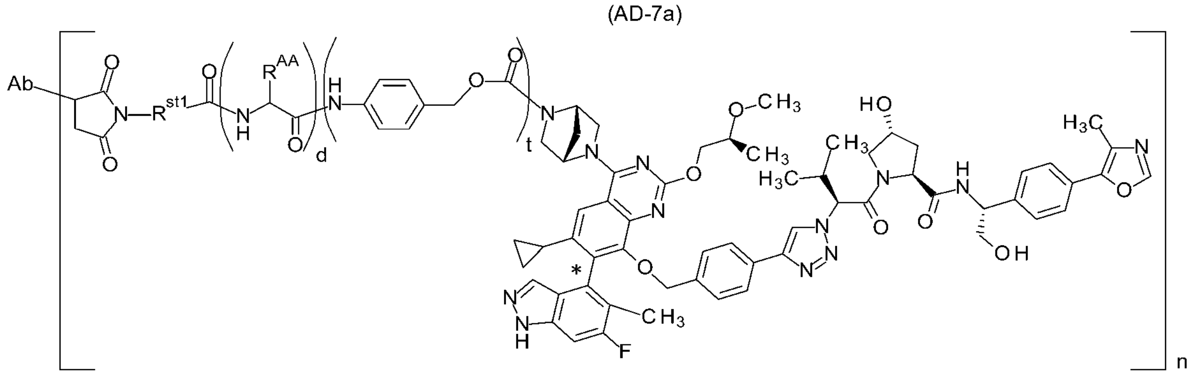

(AD-8a)

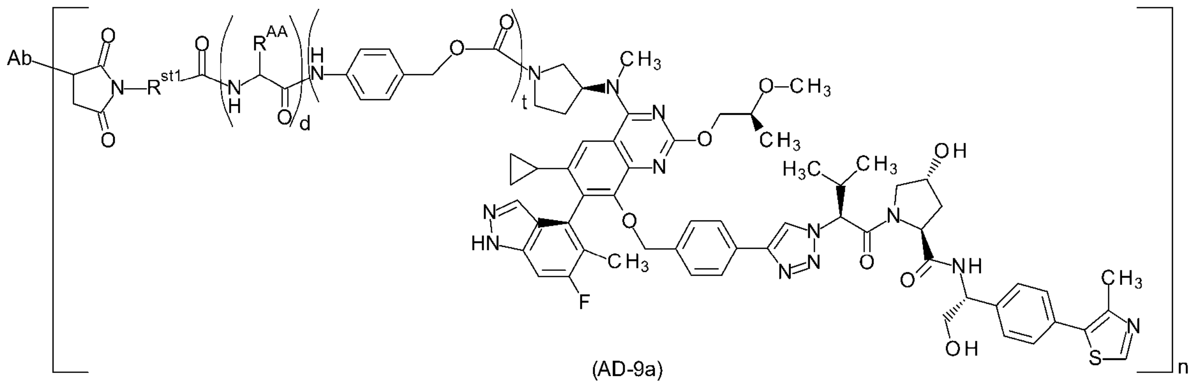

(AD-9a)

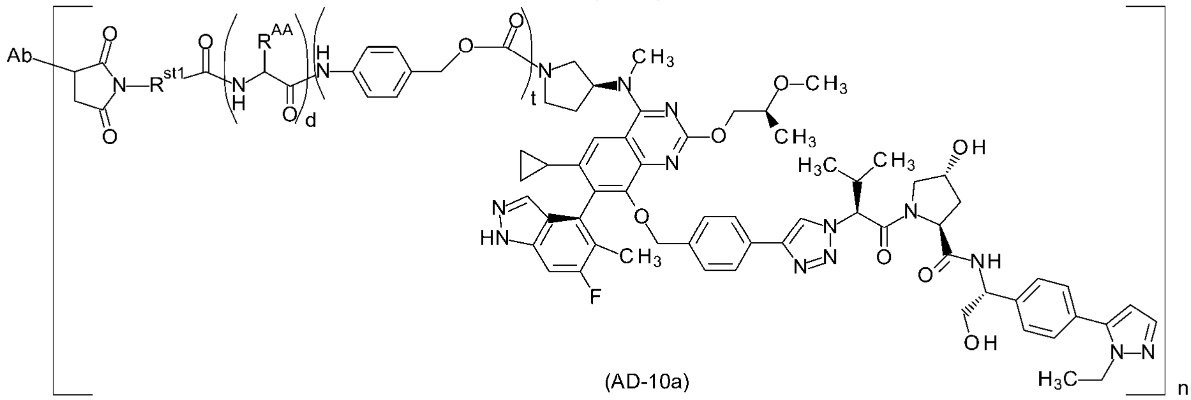

(AD-10a)

[Chemical Formula 20-3]

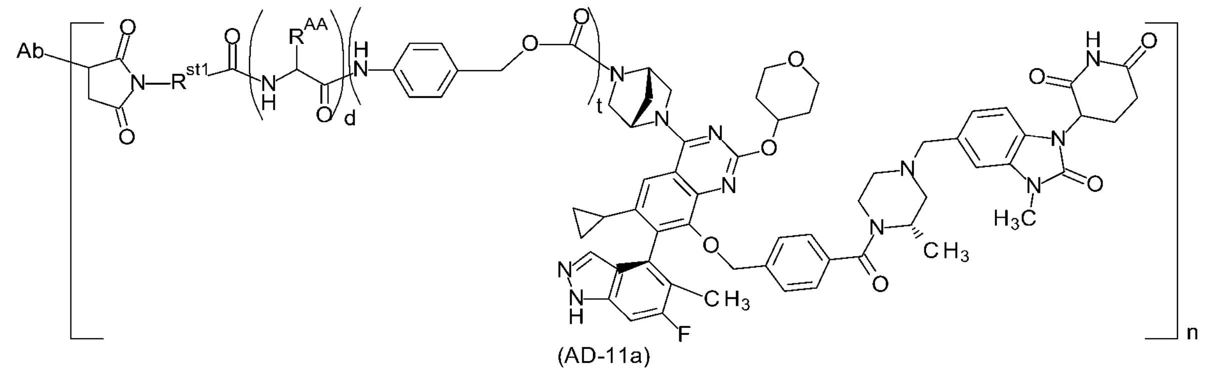

(AD-11a)

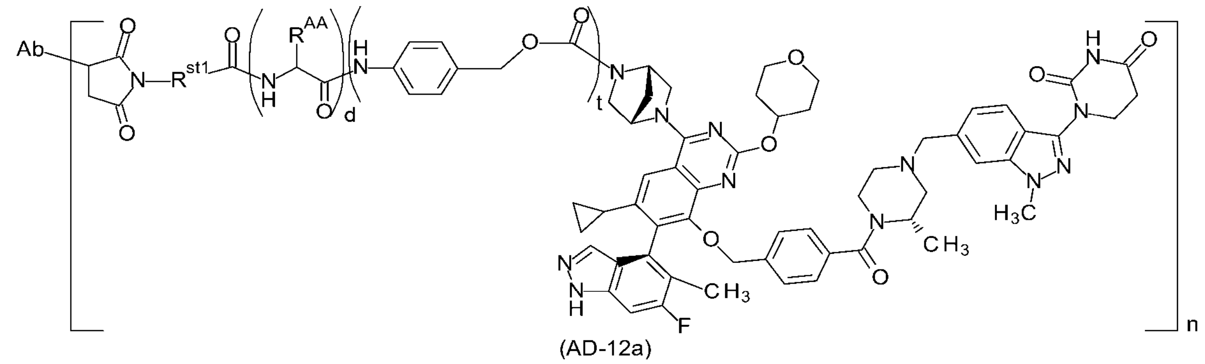

(AD-12a)

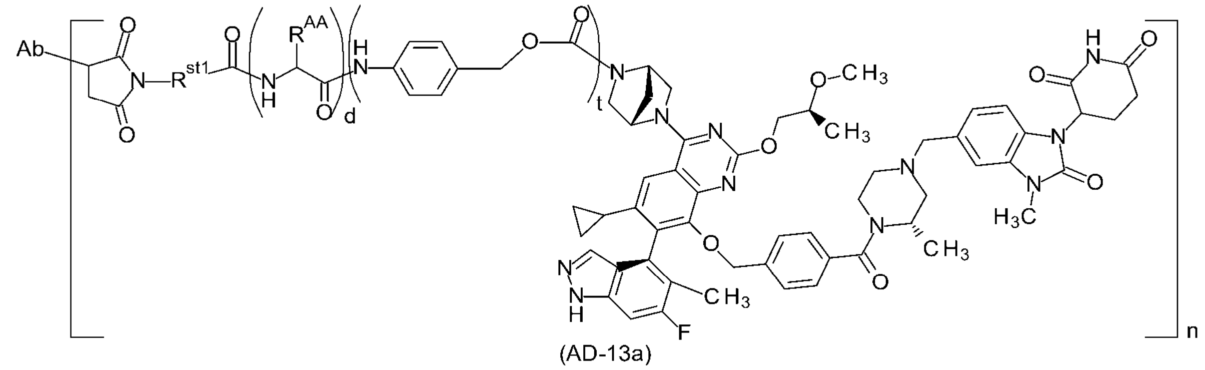

(AD-13a)

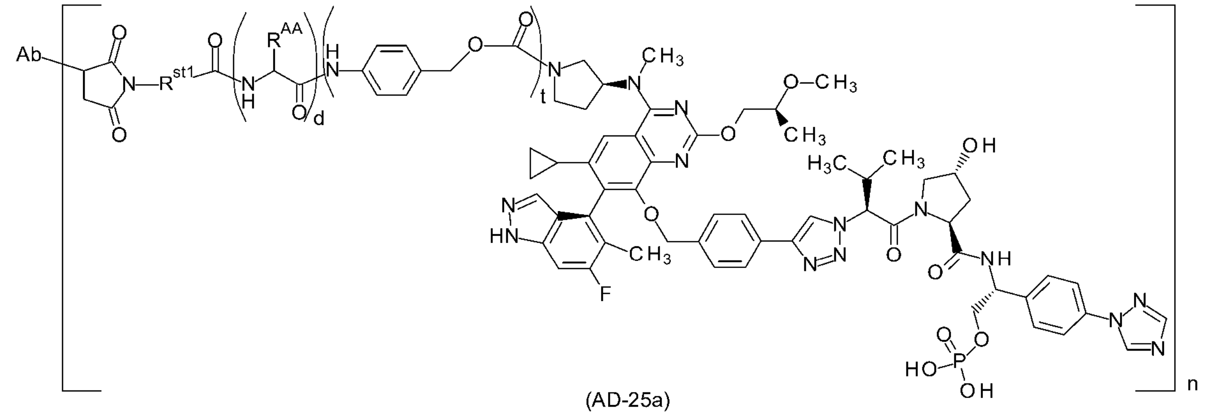

(AD-25a)

wherein $R^{st1}$ is $C_{1-12}$ alkylene, $R^{AA}$ are each independently H, methyl, isopropyl, benzyl, or - $(CH_2)_3$-NH-C(=O)-$NH_2$, d is an integer of 2 to 4, t is 0 or 1, and n is a number of 1 to 20 (a compound with "*" in the chemical structural formulas represents that the compound has single axial chirality or chiral center; the same holds true for the description below).

**19.** The antibody-drug conjugate or the salt thereof according to claim 1, wherein formula (I) is represented by formula (AD-14), (AD-15), (AD-16), (AD-17), (AD-18), (AD-19), (AD-20), (AD-21), (AD-22), (AD-23), (AD-24), or (AD-25):

[Chemical Formula 21-1]

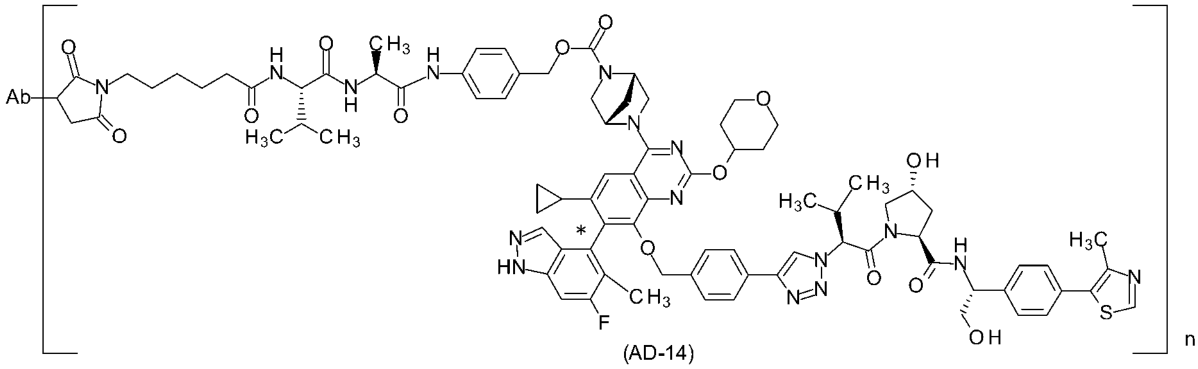

(AD-14)

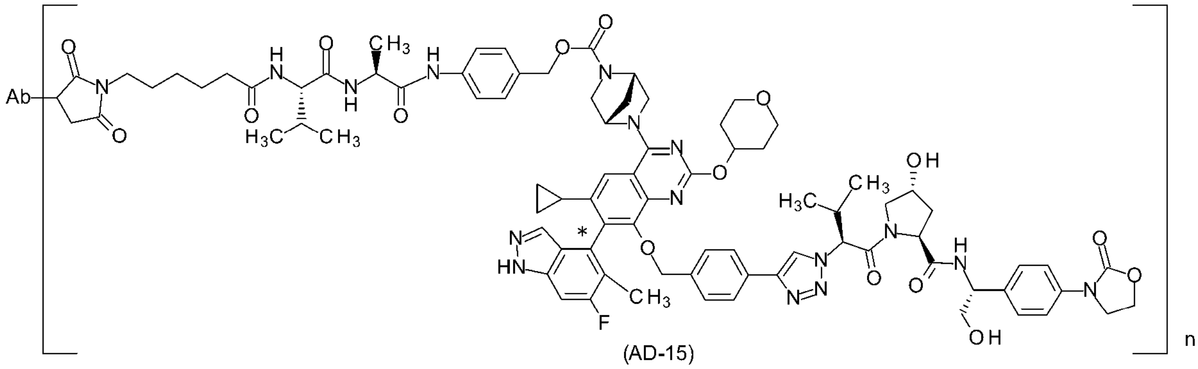

(AD-15)

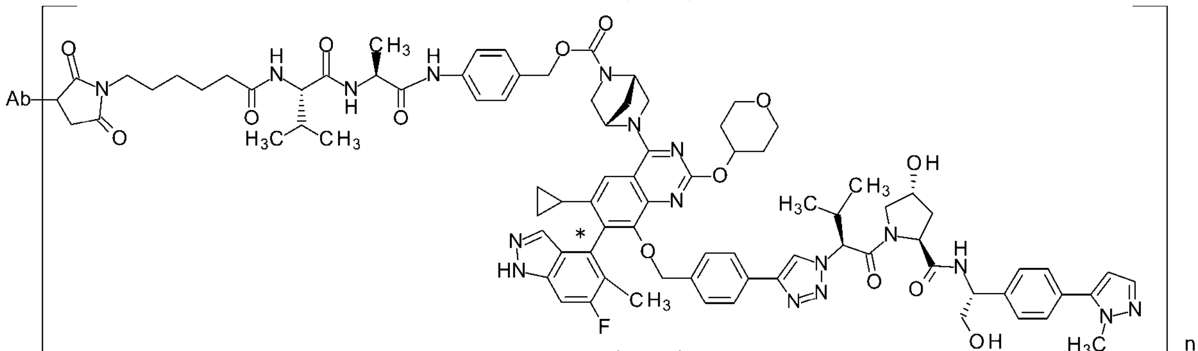

(AD-16)

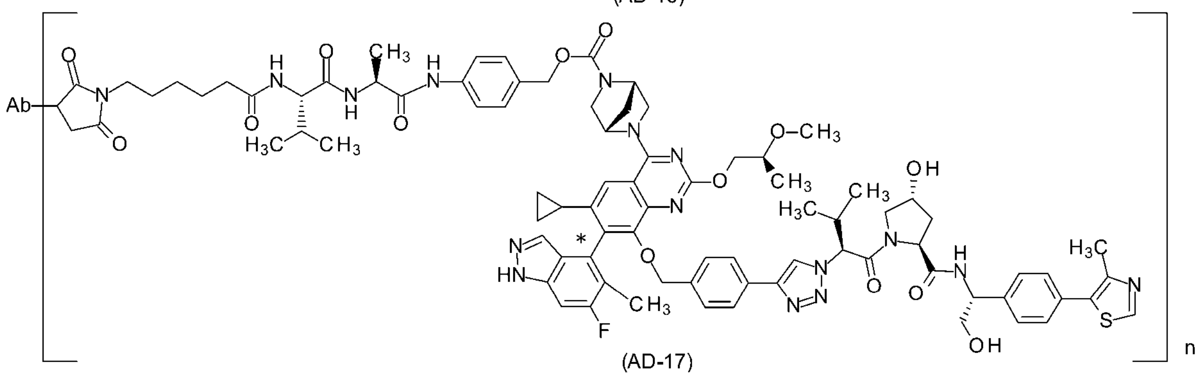

(AD-17)

[Chemical Formula 21-2]

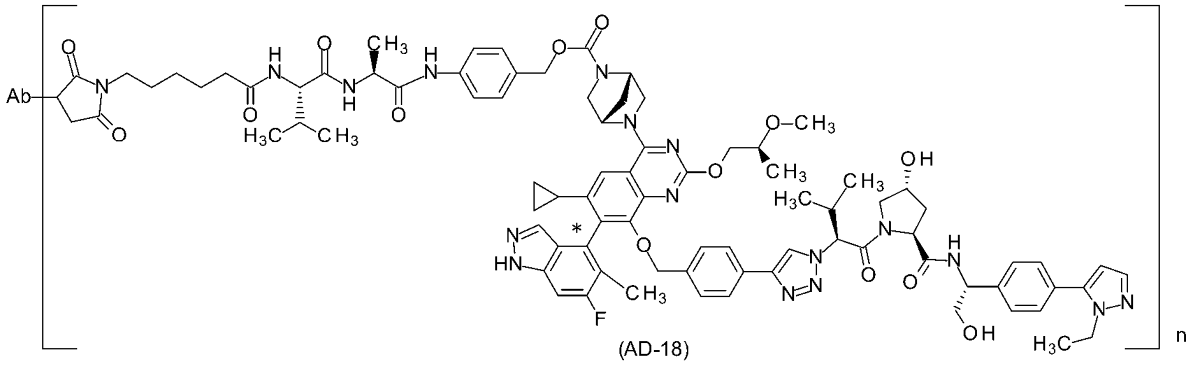

(AD-18)

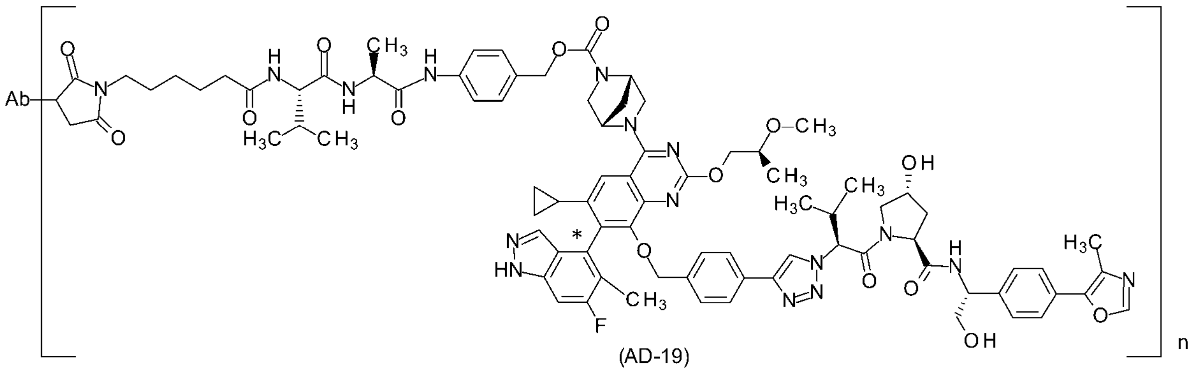

(AD-19)

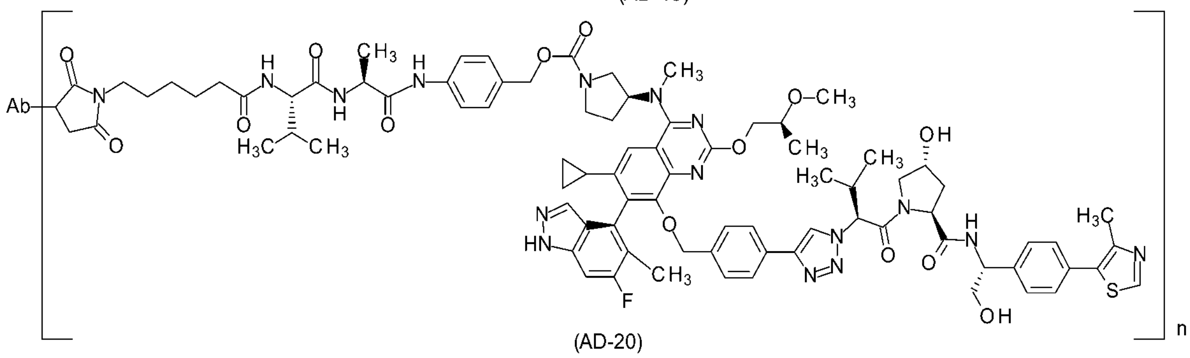

(AD-20)

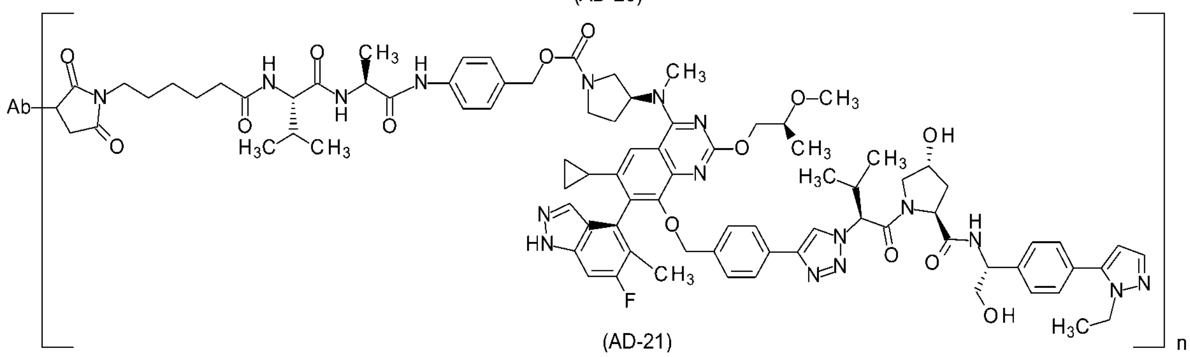

(AD-21)

[Chemical Formula 21-3]

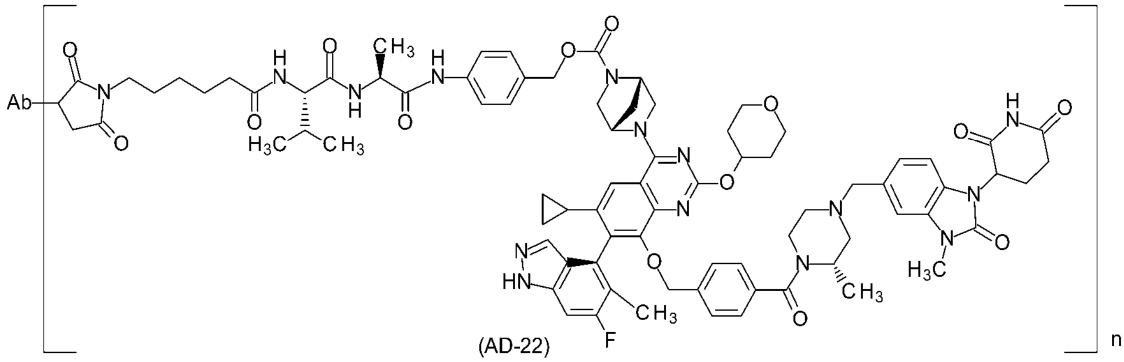

(AD-22)

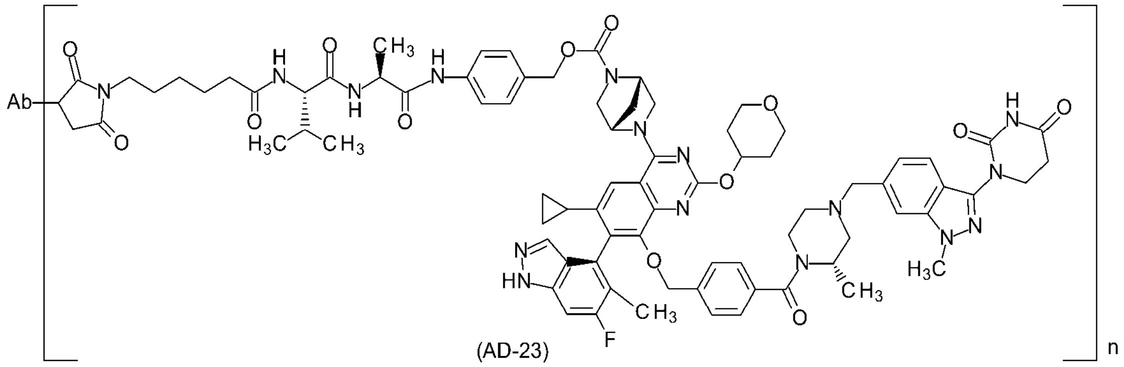

(AD-23)

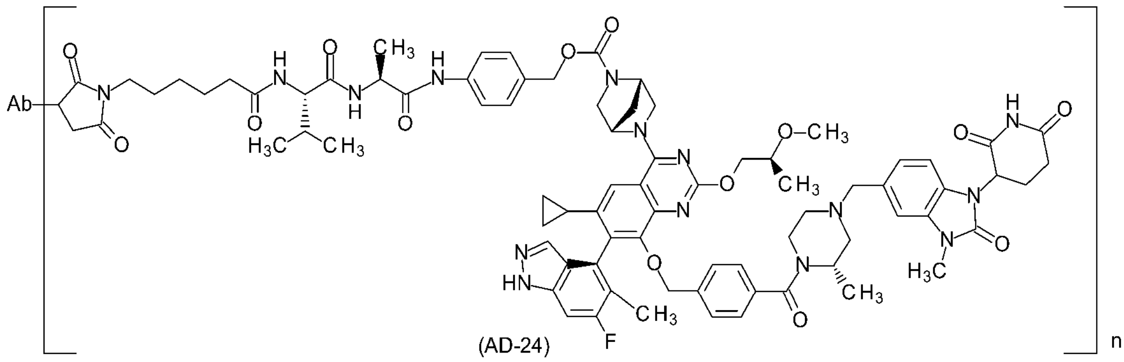

(AD-24)

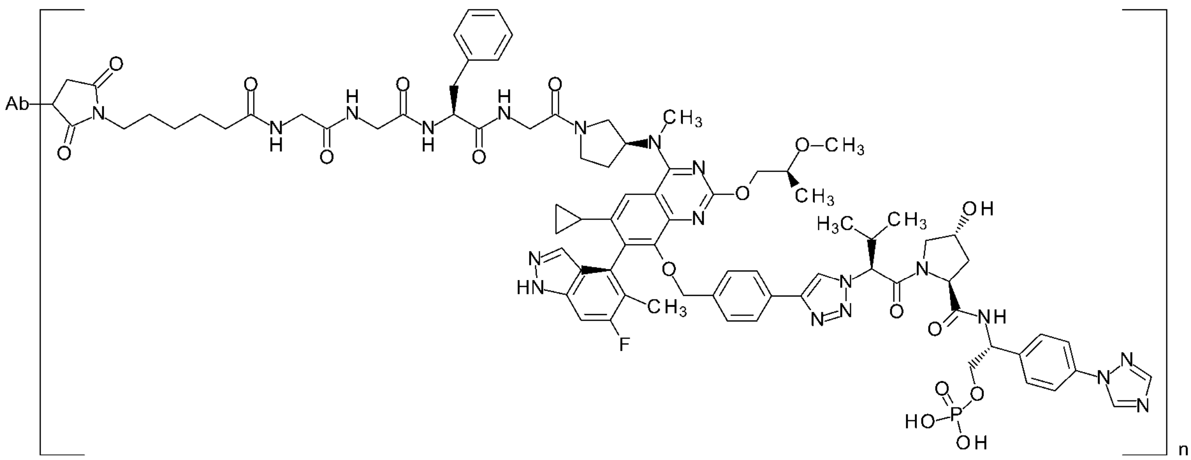

(AD-25)

wherein n is a number of 1 to 20.

**20.** The antibody-drug conjugate or the salt thereof according to any one of claims 1 to 19, wherein Ab is cetuximab, and n is a number of 2 to 5.

**21.** The antibody-drug conjugate or the salt thereof according to any one of claims 1 to 19, wherein Ab is an antibody or an antigen binding fragment that binds to one or two or more antigens selected from the group consisting of 5T4, ADAM9, ALPP, ALPPL2, AXL, B7H3, B7H4, BCMA, CA9, CCR2, CCR7, CD123, CD166, CD19, CD20, CD22, CD25, CD30, CD33, CD37, CD38, CD45, CD46, CD70, CD74, CD79b, CDH3, CDH6, CLDN1, CLDN4, CLDN6, CLDN18.2, cMET, EGFR, EphA3, FAP, FGFR3, Fibronectin, FOLRa, Globo H, GPRC5D, HER2, HER3, IGF1R, Integrin $\alpha$V, KAAG1, LIV1, MSLN, MT1-MMP, MUC1, MUC4, NaPi2b, Nectin4, PD-L1, PSMA, PTK7, ROR1, ROR2, SEZ6, SialylTn, TF, TROP2, TSPAN8, and VEGF.

**22.** The antibody-drug conjugate or the salt thereof according to any one of claims 1 to 19, wherein Ab is an antibody or an antigen binding fragment that binds to one or two or more antigens selected from the group consisting of EGFR, HER2, cMET, and TROP2.

**23.** The antibody-drug conjugate or the salt thereof according to any one of claims 1 to 19, wherein Ab is an anti-EGFR antibody or an antigen binding fragment thereof.

**24.** The antibody-drug conjugate or the salt thereof according to claim 23, wherein Ab is an anti-EGFR antibody comprising a heavy chain variable region and a light chain variable region described in the following (1) or (2) or an antigen binding fragment thereof:

(1) a heavy chain variable region comprising CDR1 consisting of an amino acid sequence from amino acid positions 31 to 35 of SEQ ID NO: 1, CDR2 consisting of an amino acid sequence from amino acid positions 50 to 65 of SEQ ID NO: 1, and CDR3 consisting of an amino acid sequence from amino acid positions 98 to 108 of SEQ ID NO: 1, and
a light chain variable region comprising CDR1 consisting of an amino acid sequence from amino acid positions 24 to 34 of SEQ ID NO: 2, CDR2 consisting of an amino acid sequence from amino acid positions 50 to 56 of SEQ ID NO: 2, and CDR3 consisting of an amino acid sequence from amino acid positions 89 to 97 of SEQ ID NO: 2; or
(2) a heavy chain variable region comprising CDR1 consisting of an amino acid sequence from amino acid positions 31 to 35 of SEQ ID NO: 3, CDR2 consisting of an amino acid sequence from amino acid positions 50 to 65 of SEQ ID NO: 3, and CDR3 consisting of an amino acid sequence from amino acid positions 98 to 108 of SEQ ID NO: 3, and
a light chain variable region comprising CDR1 consisting of an amino acid sequence from amino acid positions 24 to 34 of SEQ ID NO: 4, CDR2 consisting of an amino acid sequence from amino acid positions 50 to 56 of SEQ ID NO: 4, and CDR3 consisting of an amino acid sequence from amino acid positions 89 to 97 of SEQ ID NO: 4.

**25.** The antibody-drug conjugate or the salt thereof according to claim 23, wherein Ab is an anti-EGFR antibody comprising a heavy chain variable region and a light chain variable region selected from the group consisting of the following (1) to (3) or an antigen binding fragment thereof:

(1) a heavy chain variable region consisting of an amino acid sequence from amino acid positions 1 to 119 of SEQ ID NO: 1 and a light chain variable region consisting of an amino acid sequence from amino acid positions 1 to 107 of SEQ ID NO: 2;
(2) a heavy chain variable region consisting of an amino acid sequence from amino acid positions 1 to 119 of SEQ ID NO: 3 and a light chain variable region consisting of an amino acid sequence from amino acid positions 1 to 107 of SEQ ID NO: 4; and
(3) a heavy chain variable region and a light chain variable region having at least 90% or higher identity to the heavy chain variable region and the light chain variable region described in (1) or (2) above.

**26.** The antibody-drug conjugate or the salt thereof according to claim 23, wherein Ab is an IgG1 or IgG4 type anti-EGFR antibody.

**27.** The antibody-drug conjugate or the salt thereof according to claim 23, wherein Ab is an anti-EGFR antibody consisting of a heavy chain of SEQ ID NO: 1 and a light chain of SEQ ID NO: 2.

**28.** The antibody-drug conjugate or the salt thereof according to any one of claims 21 to 23, wherein Ab is a post-translationally modified antibody, or an antibody that an arbitrary amino acid residue is substituted with cysteine or a non-natural amino acid.

**29.** A pharmaceutical composition comprising the antibody-drug conjugate or the salt thereof according to any one of

claims 1 to 28 and a pharmaceutically acceptable excipient.

**30.** The pharmaceutical composition according to claim 29 for use in the treatment of a cancer.

**31.** The pharmaceutical composition according to claim 30, wherein the cancer is blood cancer or solid cancer.

**32.** The pharmaceutical composition according to claim 30, wherein the cancer is a cancer expressing G12D mutant KRAS.

**33.** The antibody-drug conjugate or the salt thereof according to any one of claims 1 to 28 for use in the treatment of a cancer.

**34.** A method for treating a cancer, comprising the step of administering a therapeutically effective amount of the antibody-drug conjugate or the salt thereof according to any one of claims 1 to 28 to a subject.

**35.** Use of the antibody-drug conjugate or the salt thereof according to any one of claims 1 to 28 in the production of a pharmaceutical composition for the treatment of a cancer.

**36.** A drug-linker conjugate represented by formula (LD-1) or a salt thereof:

[Chemical Formula 22]

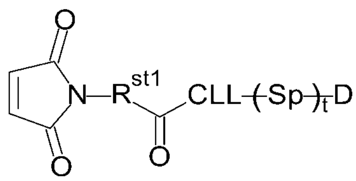

(LD-1)

wherein

D is a heterocyclic compound having a G12D mutant KRAS protein degradation-inducing effect,
$R^{st1}$ is $C_{1-12}$ alkylene,
CLL is a partial structure cleavable *in vivo*,
Sp is a spacer unit and is bonded to CLL and D, and t is 0 or 1.

**37.** The drug-linker conjugate or the salt thereof according to claim 36, wherein

D is a heterocyclic compound represented by formula (II):

[Chemical Formula 23]

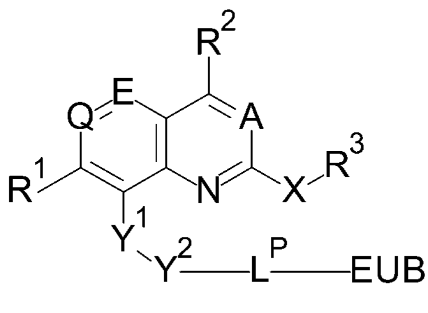

(II)

wherein
A is $CR^A$ or N,

$R^A$ is H, cyano, or optionally substituted $C_{1-3}$ alkyl,
Q is $CR^Q$ or N,
$R^Q$ is H, halogen, $C_{3-6}$ cycloalkyl, vinyl, or optionally substituted $C_{1-3}$ alkyl,
E is CH or N,
$R^1$ is naphthyl optionally substituted by one or two groups selected from the group consisting of cyano, OH, halogen, and optionally substituted $C_{1-3}$ alkyl, or is one group selected from the group consisting of the following formula (III), formula (IV), and formula (V):

[Chemical Formula 24]

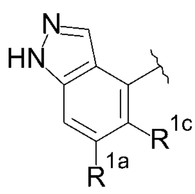
(III)

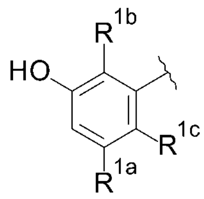
(IV)

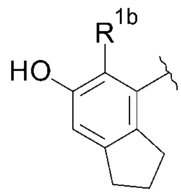
(V)

$R^{1a}$, $R^{1b}$, and $R^{1c}$ are each independently H, vinyl, halogen, or optionally substituted $C_{1-3}$ alkyl,
$R^2$ is $-V^1-V^2$ or W,
$V^1$ is a bond, $-CH_2-$, -O-, -S-, or $-N(R^{V1})-$,
$R^{V1}$ is H or optionally substituted $C_{1-3}$ alkyl,
$V^2$ is one group selected from the group consisting of the following formula (VI) and formula (VII):

[Chemical Formula 25]

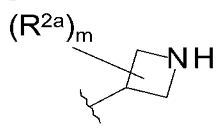
(VI)

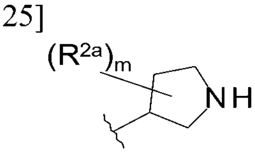
(VII)

W is one group selected from the group consisting of the following formula (VIII), formula (IX), formula (X), formula (XI), formula (XII), formula (XIII), formula (XIV), formula (XV) and formula (XVI):

[Chemical Formula 26]

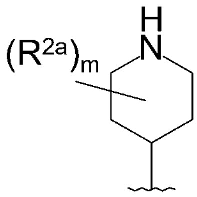
(VIII)

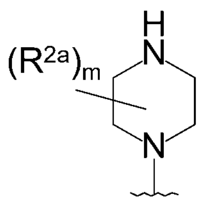
(IX)

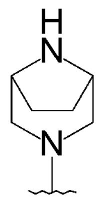
(X)

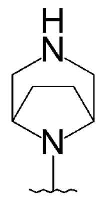
(XI)

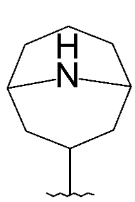
(XII)

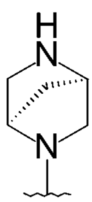
(XIII)

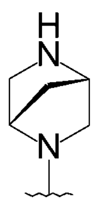
(XIV)

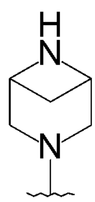
(XV)

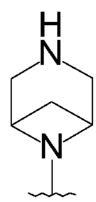
(XVI)

$R^{2a}$ are each independently OH, $OCH_3$, F, or optionally substituted $C_{1-3}$ alkyl, wherein $R^{2a}$ is bonded only to a carbon atom serving as a constituting atom of a ring selected from the group consisting of an azetidine ring represented by formula (VI), a pyrrolidine ring represented by formula (VII), a piperidine ring represented by formula (VIII) and a piperazine ring represented by formula (IX),
m is an integer of 0 to 2,
$R^3$ is optionally substituted $C_{1-6}$ alkyl, optionally substituted hetero cycloalkyl, or optionally substituted heteroaryl,
X is a bond, $-CH_2-$, -O-, -S-, or $-NR^{4X}-$,
$R^{4X}$ is H or optionally substituted $C_{1-3}$ alkyl,
$Y^1$ is -O-(optionally substituted $C_{1-3}$ alkylene)-$^{*Y2}$, -S-(optionally substituted $C_{1-3}$ alkylene)-$^{*Y2}$, $-SO_2$-(optionally substituted $C_{1-3}$ alkylene)-$^{*Y2}$, $-NR^Y$-(optionally substituted $C_{1-3}$ alkylene)-$^{*Y2}$, -(optionally substituted $C_{1-3}$ alkylene)-O-$^{*Y2}$, -(optionally substituted $C_{1-3}$ alkylene)-S-$^{*Y2}$, -(optionally substituted $C_{1-3}$ alkylene)-$SO_2$-$^{*Y2}$, or -(optionally substituted $C_{1-3}$ alkylene)-$NR^Y$-$^{*Y2}$ (wherein $^{*Y2}$ represents a site of connection to $Y^2$),
$R^Y$ is H or optionally substituted $C_{1-3}$ alkyl,
$Y^2$ is a bond, optionally substituted phenylene, or optionally substituted heteroarylene,
$L^P$ is a group that chemically bonds $Y^2$ to EUB,
EUB is a group having the ability to bind to one E3 ubiquitin ligase selected from the group consisting of VHL, celebron, IAP, MDM2, DCAF11, DCAF15, DCAF16, BIRC2, KEAP1, RNF4, RNF114, FEM1B, and AhR, and
Sp is a spacer unit and is bonded to CLL and D, wherein Sp is bonded to an arbitrary nitrogen atom of -NH- or oxygen atom of -OH contained in D.

**38.** The drug-linker conjugate or the salt thereof according to claim 37, wherein

$V^2$ is one group selected from the group consisting of the following formula (VIb) and formula (VIIb) (wherein $^{*SP}$ represents a site of connection to Sp):

[Chemical Formula 27]

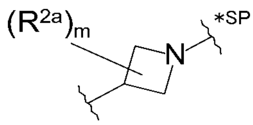

(VIb)

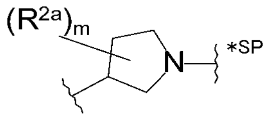

(VIIb)

W is one group selected from the group consisting of the following formula (VIIIb), formula (IXb), formula (Xb), formula (XIb), formula (XIIb), formula (XIIIb), formula (XIVb), formula (XVb), and formula (XVIb) (wherein $^{*SP}$ represents a site of connection to Sp):

[Chemical Formula 28]

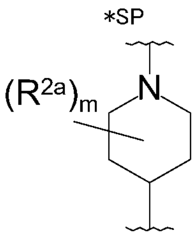
(VIIIb)

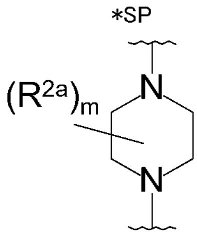
(IXb)

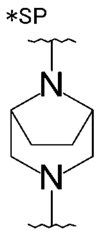
(Xb)

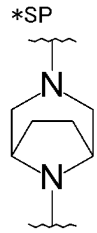
(XIb)

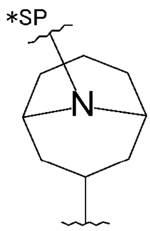
(XIIb)

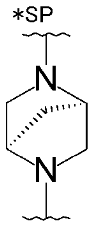
(XIIIb)

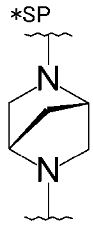
(XIVb)

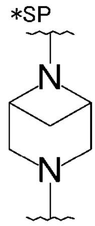
(XVb)

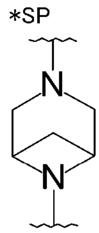
(XVIb)

and

Sp is a spacer unit and is bonded to CLL and D, wherein Sp is bonded to D through a nitrogen atom contained in $V^2$ or W at the site of connection represented by $^{*SP}$.

**39.** The drug-linker conjugate or the salt thereof according to claim 37, wherein

A is N,
Q is $CR^Q$, $R^Q$ is cyclopropyl,
E is CH,
$R^1$ is the following formula (III-2):

[Chemical Formula 29]

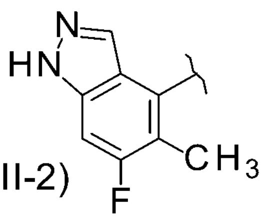
(III-2)

$R^2$ is $-V^1-V^2$ or W,
$V^1$ is $-N(CH_3)-$,
$V^2$ is the following formula (VII-2):

[Chemical Formula 30]

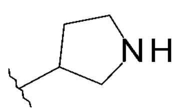
(VII-2)

W is the following formula (XIV):

[Chemical Formula 31]

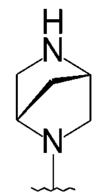

(XIV)

$R^3$ is n-propyl optionally substituted by $-OCH_3$, or tetrahydropyranyl,
X is -O-,
$Y^1$ is -O-(methylene)-$^{*Y2}$ (wherein $^{*Y2}$ represents a site of connection to $Y^2$),
$Y^2$ is phenylene,
$L^P$ is a group that chemically bonds $Y^2$ to EUB, and
EUB is a group having the ability to bind to one E3 ubiquitin ligase selected from the group consisting of VHL and celebron.

**40.** The drug-linker conjugate or the salt thereof according to claim 39, wherein

$V^2$ is the following formula (VII-2b) (wherein $^{*SP}$ represents a site of connection to Sp):

[Chemical Formula 32]

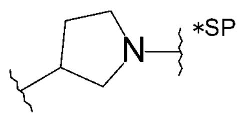

(VII-2b)

W is the following formula (XIVb) (wherein $^{*SP}$ represents a site of connection to Sp):

[Chemical Formula 33]

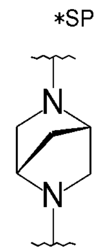

(XIVb)

and
Sp is a spacer unit and is bonded to CLL and D, wherein Sp is bonded to D through a nitrogen atom contained in $V^2$ or W at the site of connection represented by $^{*SP}$.

**41.** The drug-linker conjugate or the salt thereof according to claim 36 or 37, wherein D is (4R)-1-[(2S)-2-(4-{4-[({6-cyclopropyl-4-[(1S,4S)-2,5-diazabicyclo[2.2.1]heptan-2-yl]-7-(6-fluoro-5-methyl-1H-indazol-4-yl)-2-[(oxan-4-yl)oxy]quinazolin-8-yl}oxy)methyl]phenyl}-1H-1,2,3-triazol-1-yl)-3-methylbutanoyl]-4-hydroxy-N-{(1R)-2-hydroxy-1-[4-(4-methyl-1,3-thiazol-5-yl)phenyl]ethyl}-L-prolinamide, (4R)-1-[(2S)-2-(4-{4-[({6-cyclopropyl-4-[(1S,4S)-2,5-diazabicyclo[2.2.1]heptan-2-yl]-7-(6-fluoro-5-methyl-1H-indazol-4-yl)-2-[(oxan-4-yl)oxy]quinazolin-8-yl}oxy)methyl]phenyl}-1H-1,2,3-triazol-1-yl)-3-methylbutanoyl]-4-hydroxy-N-{(1R)-2-hydroxy-1-[4-(2-oxo-1,3-oxazolidin-3-yl)phenyl]ethyl}-L-prolinamide, (4R)-1-[(2S)-2-(4-{4-[({6-cyclopropyl-4-[(1S,4S)-2,5-diazabicyclo[2.2.1]heptan-2-yl]-7-(6-fluoro-5-methyl-1H-indazol-4-yl)-2-[(oxan-4-yl)oxy]quinazolin-8-yl}oxy)methyl]phenyl}-1H-1,2,3-triazol-1-yl)-3-methylbutanoyl]-4-hydroxy-N-{(1R)-2-hydroxy-1-[4-(1-methyl-1H-pyrazol-5-yl)phenyl]ethyl}-L-prolinamide,

(4R)-1-[(2S)-2-(4-{4-[({6-cyclopropyl-4-[(1S,4S)-2,5-diazabicyclo[2.2.1]heptan-2-yl]-7-(6-fluoro-5-methyl-1H-indazol-4-yl)-2-[(2S)-2-methoxypropoxy]quinazolin-8-yl}oxy)methyl]phenyl}-1H-1,2,3-triazol-1-yl)-3-methylbutanoyl]-4-hydroxy-N-{(1R)-2-hydroxy-1-[4-(4-methyl-1,3-thiazol-5-yl)phenyl]ethyl}-L-prolinamide, (4R)-1-[(2S)-2-(4-{4-[({6-cyclopropyl-4-[(1S,4S)-2,5-diazabicyclo[2.2.1]heptan-2-yl]-7-(6-fluoro-5-methyl-1H-indazol-4-yl)-2-[(2S)-2-methoxypropoxy]quinazolin-8-yl}oxy)methyl]phenyl}-1H-1,2,3-triazol-1-yl)-3-methylbutanoyl]-N-{(1R)-1-[4-(1-ethyl-1H-pyrazol-5-yl)phenyl]-2-hydroxyethyl}-4-hydroxy-L-prolinamide, (4R)-1-[(2S)-2-(4-{4-[({6-cyclopropyl-4-[(1S,4S)-2,5-diazabicyclo[2.2.1]heptan-2-yl]-7-(6-fluoro-5-methyl-1H-indazol-4-yl)-2-[(2S)-2-methoxypropoxy]quinazolin-8-yl}oxy)methyl]phenyl}-1H-1,2,3-triazol-1-yl)-3-methylbutanoyl]-4-hydroxy-N-{(1R)-2-hydroxy-1-[4-(4-methyl-1,3-oxazol-5-yl)phenyl]ethyl}-L-prolinamide, (4R)-1-[(2S)-2-{4-[4-({[(7M)-6-cyclopropyl-7-(6-fluoro-5-methyl-1H-indazol-4-yl)-2-[(2S)-2-methoxypropoxy]-4-{methyl[(3S)-pyrrolidin-3-yl]amino}quinazolin-8-yl]oxy}methyl)phenyl]-1H-1,2,3-triazol-1-yl}-3-methylbutanoyl]-4-hydroxy-N-{(1R)-2-hydroxy-1-[4-(4-methyl-1,3-thiazol-5-yl)phenyl]ethyl}-L-prolinamide, (4R)-1-[(2S)-2-{4-[4-({[(7M)-6-cyclopropyl-7-(6-fluoro-5-methyl-1H-indazol-4-yl)-2-[(2S)-2-methoxypropoxy]-4-{methyl[(3S)-pyrrolidin-3-yl]amino}quinazolin-8-yl]oxy}methyl)phenyl]-1H-1,2,3-triazol-1-yl}-3-methylbutanoyl]-N-{(1R)-1-[4-(1-ethyl-1H-pyrazol-5-yl)phenyl]-2-hydroxyethyl}-4-hydroxy-L-prolinamide, 3-(5-{[(3S)-4-{4-[({(7M)-6-cyclopropyl-4-[(1S,4S)-2,5-diazabicyclo[2.2.1]heptan-2-yl]-7-(6-fluoro-5-methyl-1H-indazol-4-yl)-2-[(oxan-4-yl)oxy]quinazolin-8-yl}oxy)methyl]benzoyl}-3-methylpiperazin-1-yl]methyl}-3-methyl-2-oxo-2,3-dihydro-1H-benzimidazol-1-yl)piperidine-2,6-dione, 1-(6-{[(3S)-4-{4-[({(7M)-6-cyclopropyl-4-[(1S,4S)-2,5-diazabicyclo[2.2.1]heptan-2-yl]-7-(6-fluoro-5-methyl-1H-indazol-4-yl)-2-[(oxan-4-yl)oxy]quinazolin-8-yl}oxy)methyl]benzoyl}-3-methylpiperazin-1-yl]methyl}-1-methyl-1H-indazol-3-yl)-1,3-diazinane-2,4-dione, 3-(5-{[(3S)-4-{4-[({(7M)-6-cyclopropyl-4-[(1S,4S)-2,5-diazabicyclo[2.2.1]heptan-2-yl]-7-(6-fluoro-5-methyl-1H-indazol-4-yl)-2-[(2S)-2-methoxypropoxy]quinazolin-8-yl}oxy)methyl]benzoyl}-3-methylpiperazin-1-yl]methyl}-3-methyl-2-oxo-2,3-dihydro-1H-benzimidazol-1-yl)piperidine-2,6-dione, or (2R)-2-({(4R)-1-[(2S)-2-{4-[4-({[(7M)-6-cyclopropyl-7-(6-fluoro-5-methyl-1H-indazol-4-yl)-2-[(2S)-2-methoxypropoxy]-4-{methyl[(3S)-pyrrolidin-3-yl]amino}quinazolin-8-yl]oxy}methyl)phenyl]-1H-1,2,3-triazol-1-yl}-3-methylbutanoyl]-4-hydroxy-L-prolyl}amino)-2-[4-(1H-1,2,4-triazol-1-yl)phenyl]ethyl dihydrogen phosphate.

**42.** The drug-linker conjugate or the salt thereof according to claim 36, wherein formula (LD-1) is represented by formula (LD-2a) or (LD-3a):

[Chemical Formula 34]

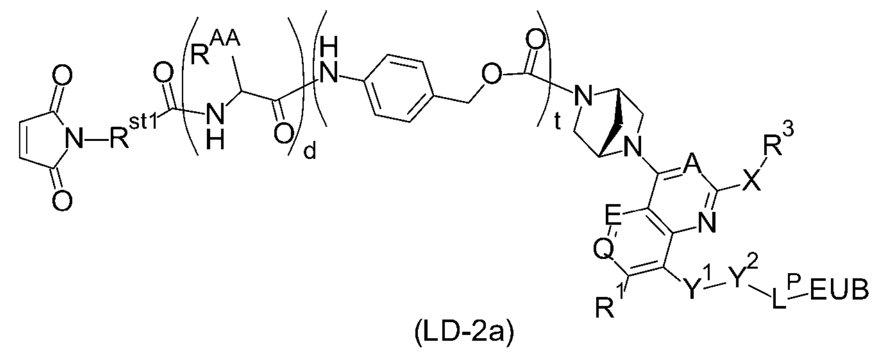

(LD-2a)

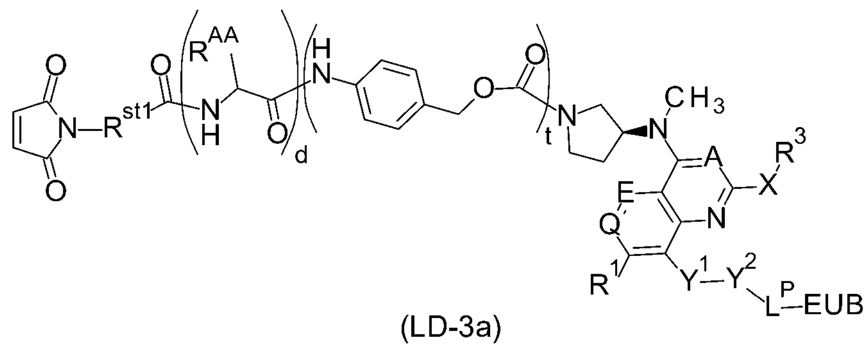

(LD-3a)

wherein

A is $CR^A$ or N,

$R^A$ is H, cyano, or optionally substituted $C_{1-3}$ alkyl,
Q is $CR^Q$ or N,
$R^Q$ is H, halogen, $C_{3-6}$ cycloalkyl, vinyl, or optionally substituted $C_{1-3}$ alkyl,
E is CH or N,
$R^1$ is naphthyl optionally substituted by one or two groups selected from the group consisting of cyano, OH, halogen, and optionally substituted $C_{1-3}$ alkyl, or is one group selected from the group consisting of the following formula (III), formula (IV), and formula (V):

[Chemical Formula 35]

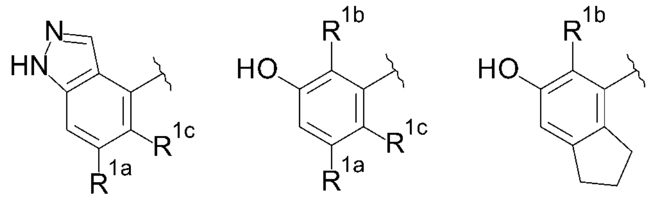

(III) (IV) (V)

$R^{1a}$, $R^{1b}$, and $R^{1c}$ are each independently H, vinyl, halogen, or optionally substituted $C_{1-3}$ alkyl,
$R^3$ is optionally substituted $C_{1-6}$ alkyl, optionally substituted hetero cycloalkyl, or optionally substituted heteroaryl,
X is a bond, $-CH_2-$, -O-, -S-, or $-NR^{4X}-$,
$R^{4X}$ is H or optionally substituted $C_{1-3}$ alkyl,
$Y^1$ is -O-(optionally substituted $C_{1-3}$ alkylene)-$^{*Y2}$, -S-(optionally substituted $C_{1-3}$ alkylene)-$^{*Y2}$, $-SO_2$-(optionally substituted $C_{1-3}$ alkylene)-$^{*Y2}$, $-NR^Y$-(optionally substituted $C_{1-3}$ alkylene)-$^{*Y2}$, -(optionally substituted $C_{1-3}$ alkylene)-O-$^{*Y2}$, -(optionally substituted $C_{1-3}$ alkylene)-S-$^{*Y2}$, -(optionally substituted $C_{1-3}$ alkylene)-$SO_2$-$^{*Y2}$, or -(optionally substituted $C_{1-3}$ alkylene)-$NR^Y$-$^{*Y2}$ (wherein $^{*Y2}$ represents a site of connection to $Y^2$),
$R^Y$ is H or optionally substituted $C_{1-3}$ alkyl,
$Y^2$ is a bond, optionally substituted phenylene, or optionally substituted heteroarylene,
$L^P$ is a group that chemically bonds $Y^2$ to EUB,
EUB is a group having the ability to bind to one E3 ubiquitin ligase selected from the group consisting of VHL and celebron,
$R^{st1}$ is $C_{1-12}$ alkylene,
$R^{AA}$ are each independently H, methyl, isopropyl, benzyl, or $-(CH_2)_3-NH-C(=O)-NH_2$, d is an integer of 2 to 4, and t is 0 or 1.

**43.** The drug-linker conjugate or the salt thereof according to claim 36, wherein formula (LD-1) is represented by formula (LD-4a), (LD-5a), (LD-6a), (LD-7a), (LD-8a), (LD-9a), (LD-10a), (LD-11a), (LD-12a), (LD-13a), (LD-14a), or (LD-26a):

[Chemical Formula 36-1]

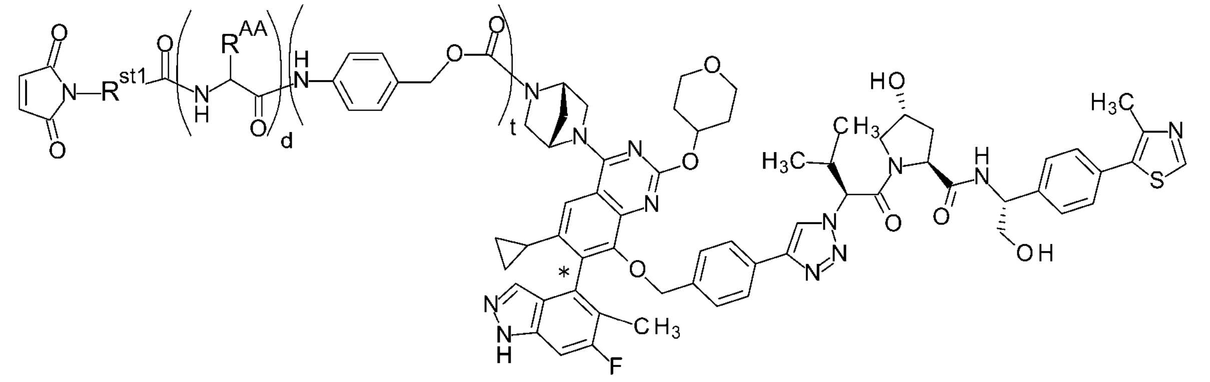

(LD-4a)

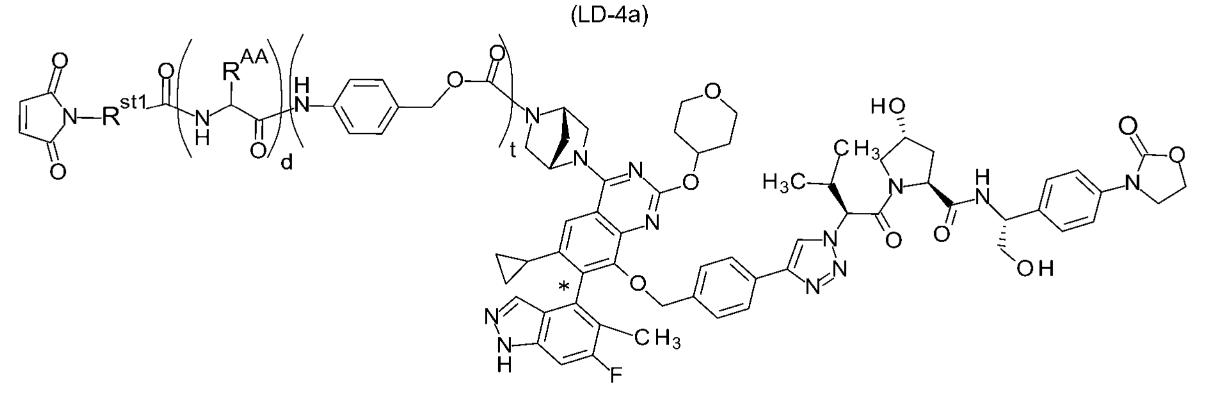

(LD-5a)

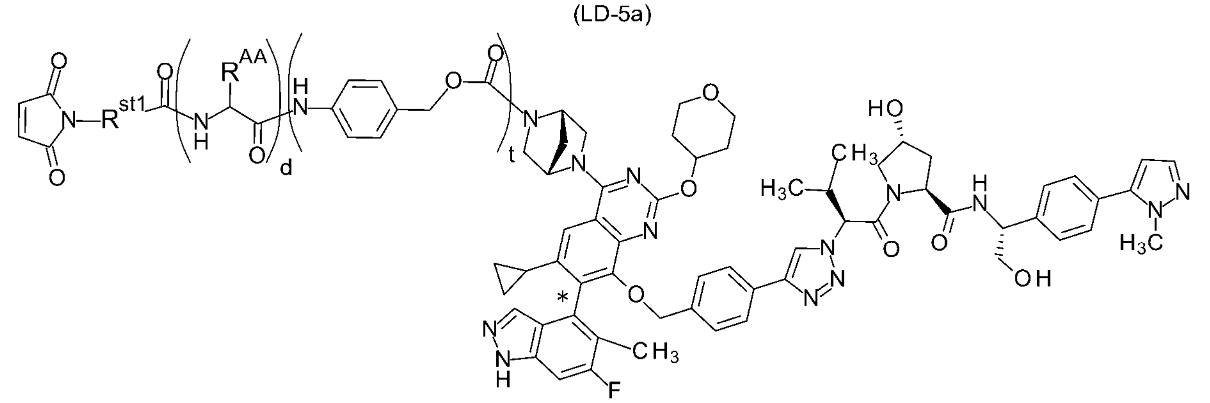

(LD-6a)

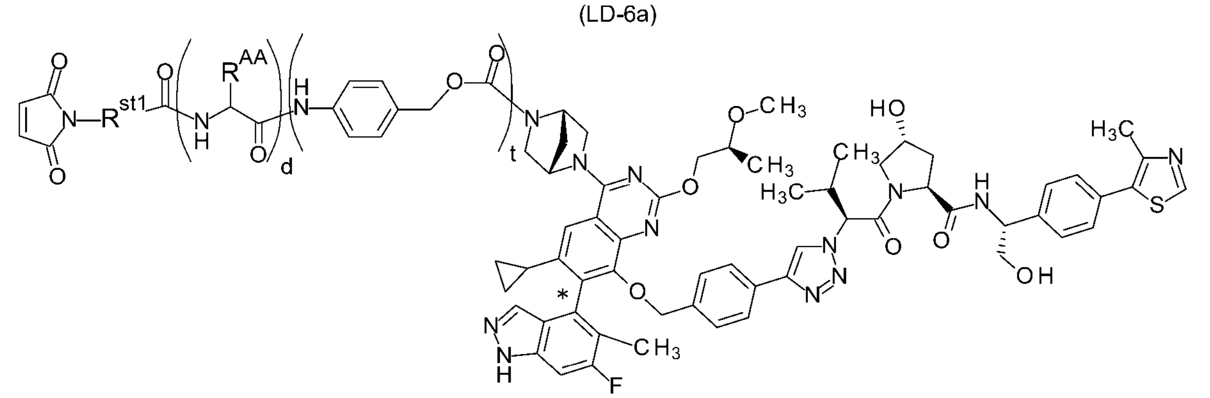

(LD-7a)

[Chemical Formula 36-2]

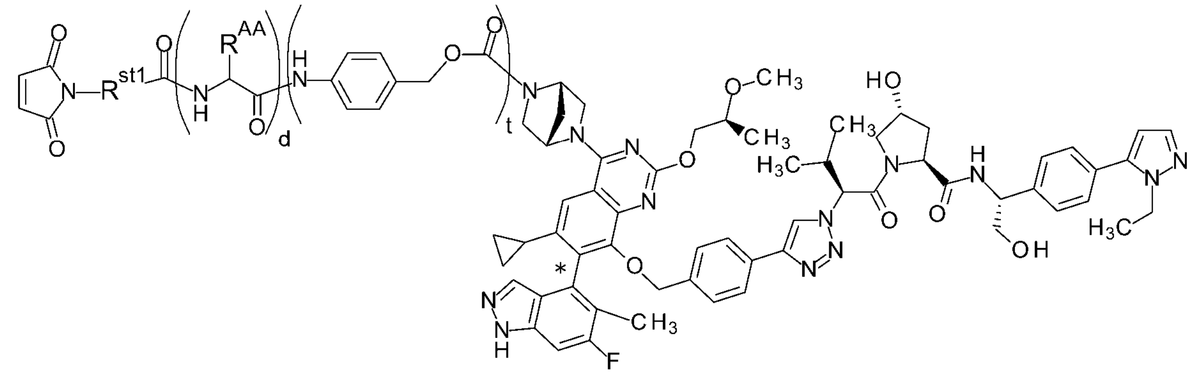

(LD-8a)

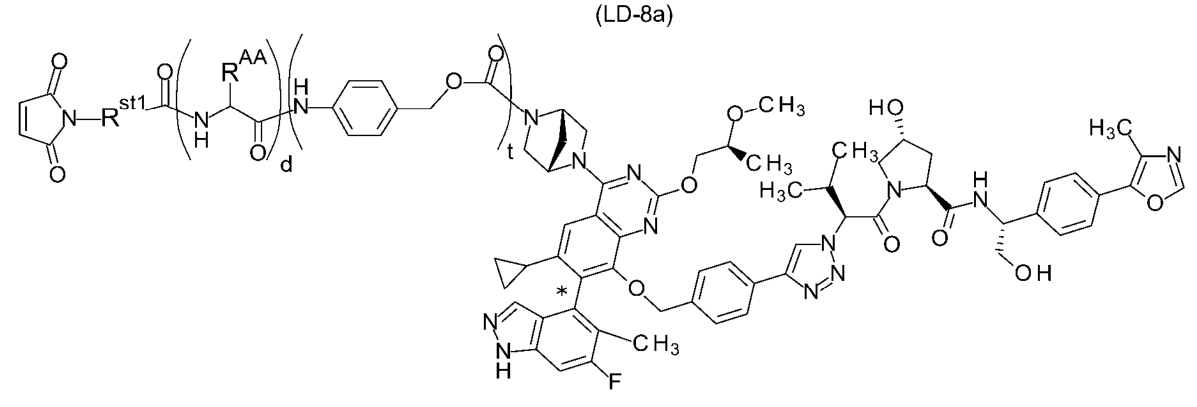

(LD-9a)

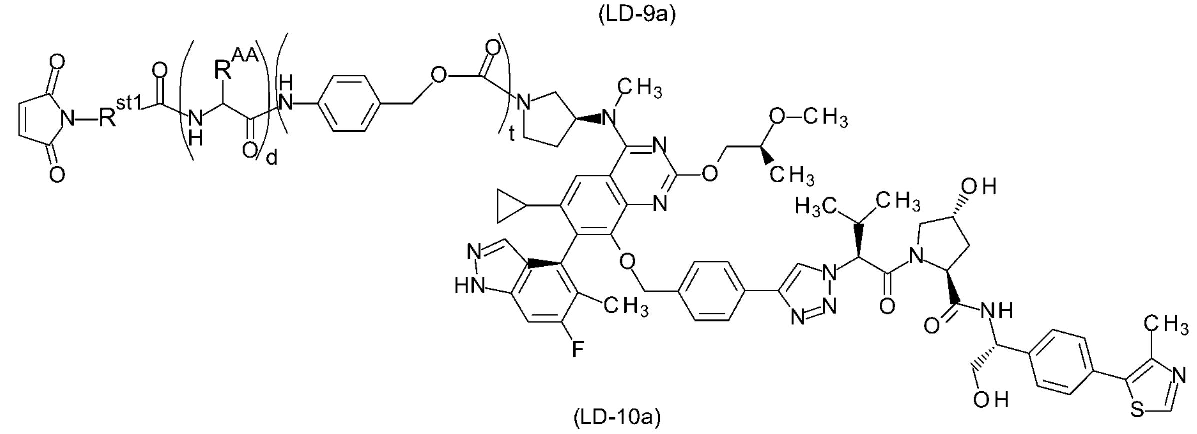

(LD-10a)

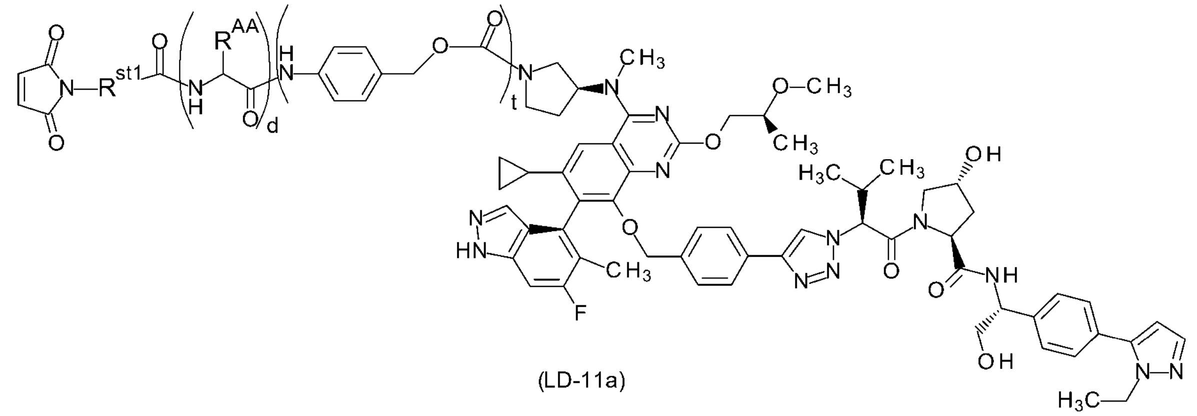

(LD-11a)

[Chemical Formula 36-3]

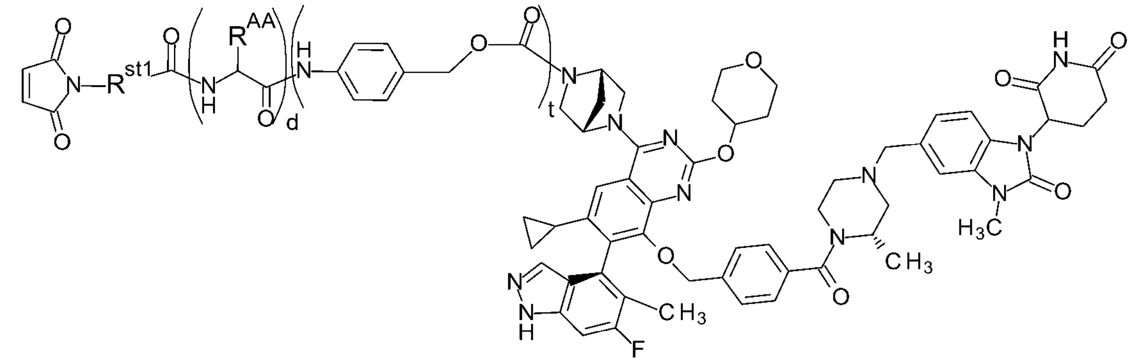

(LD-12a)

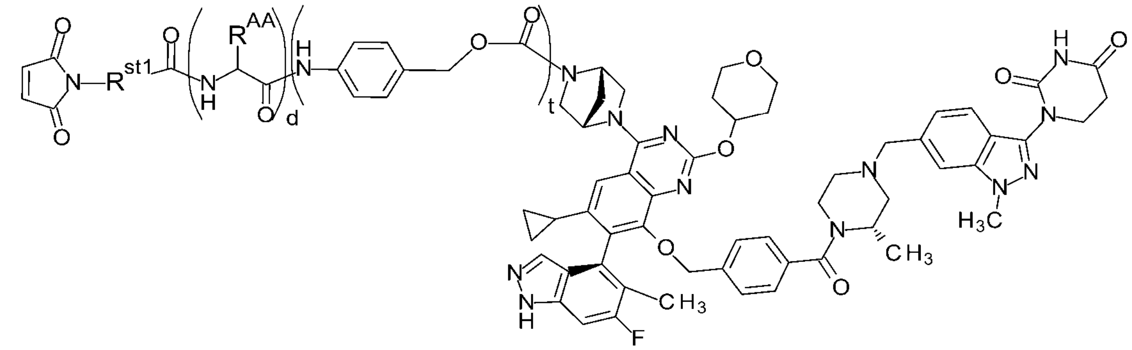

(LD-13a)

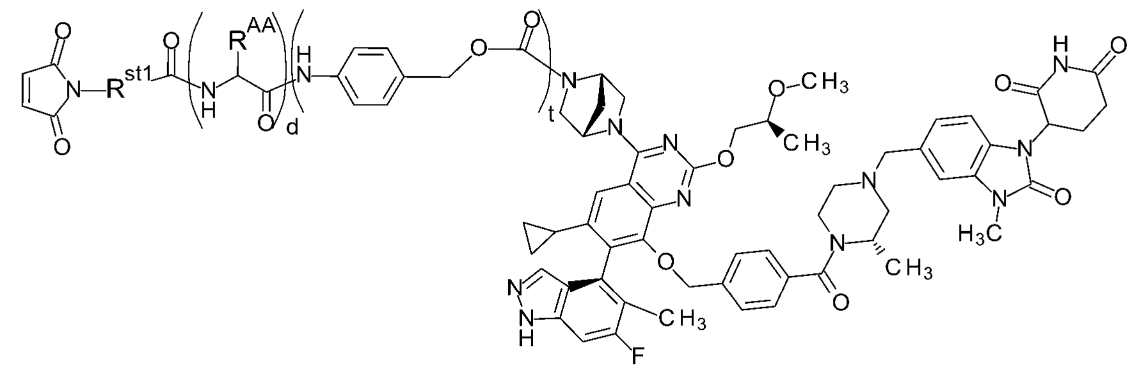

(LD-14a)

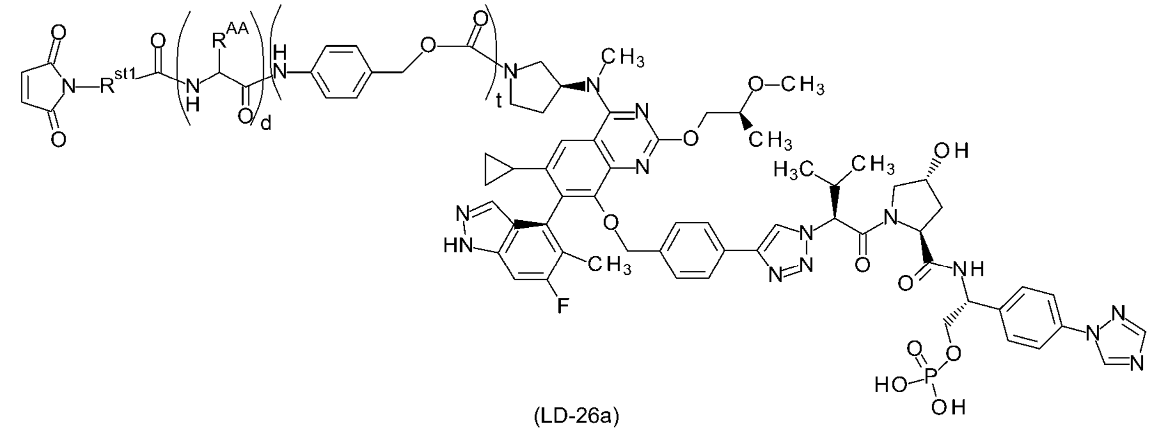

(LD-26a)

wherein $R^{st1}$ is $C_{1-12}$ alkylene, $R^{AA}$ are each independently H, methyl, isopropyl, benzyl, or - $(CH_2)_3$-NH-C(=O)-$NH_2$, d is an integer of 2 to 4, and t is 0 or 1.

**44.** The drug-linker conjugate or the salt thereof according to claim 36, wherein formula (LD-1) is represented by formula

(LD-15), (LD-16), (LD-17), (LD-18), (LD-19), (LD-20), (LD-21), (LD-22), (LD-23), (LD-24), (LD-25), or (LD-26):

[Chemical Formula 37-1]

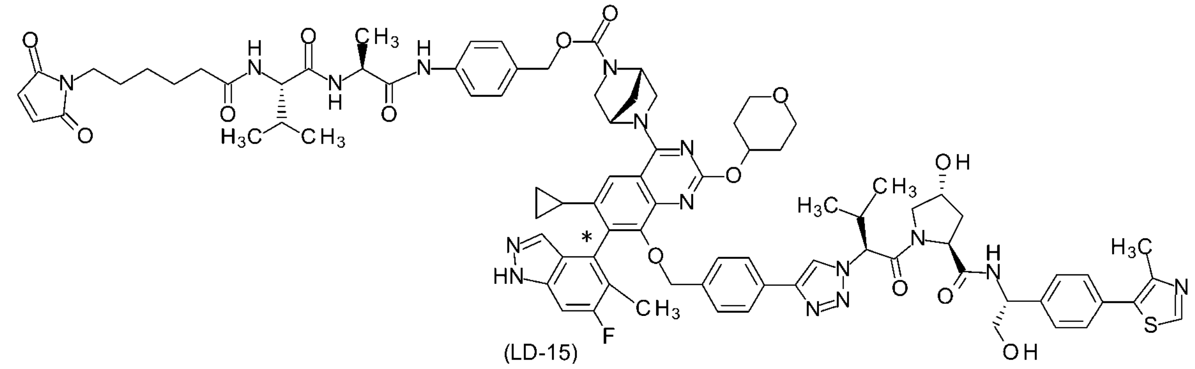

(LD-15)

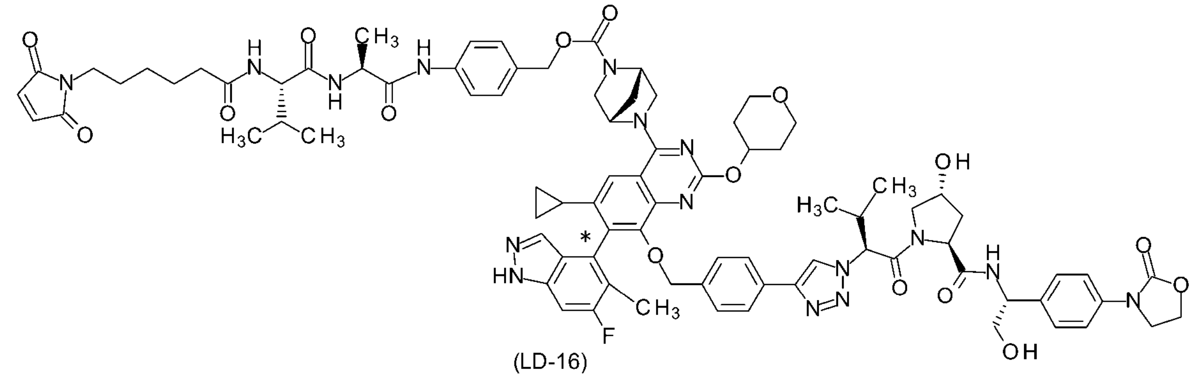

(LD-16)

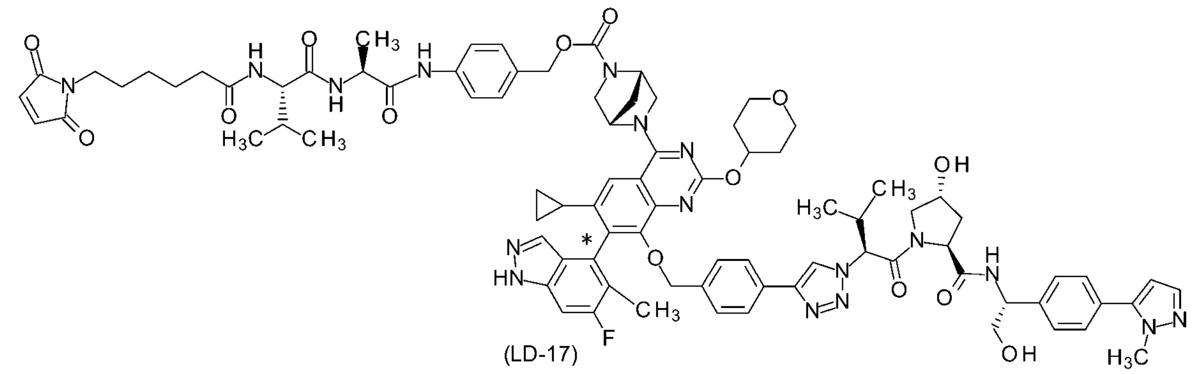

(LD-17)

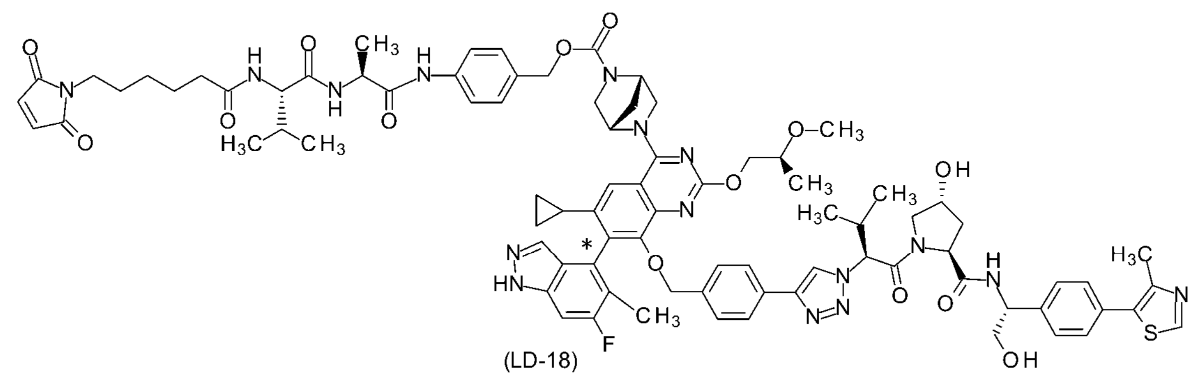

(LD-18)

[Chemical Formula 37-2]

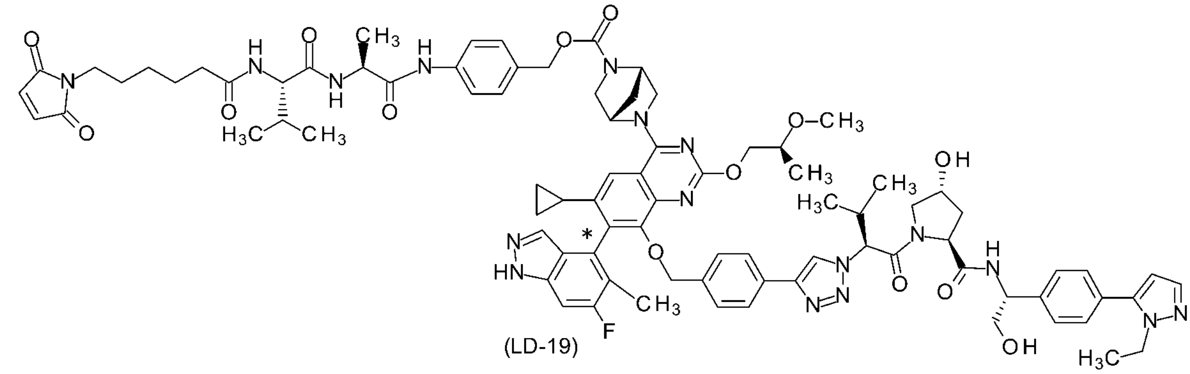

(LD-19)

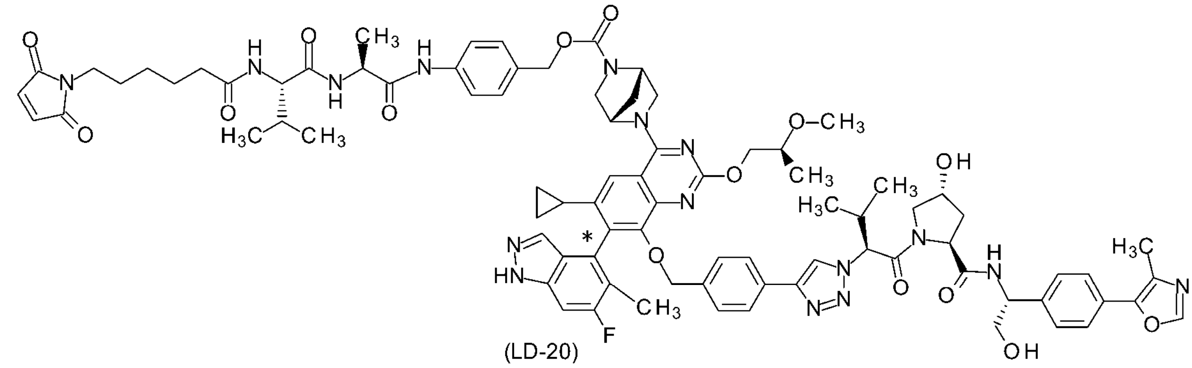

(LD-20)

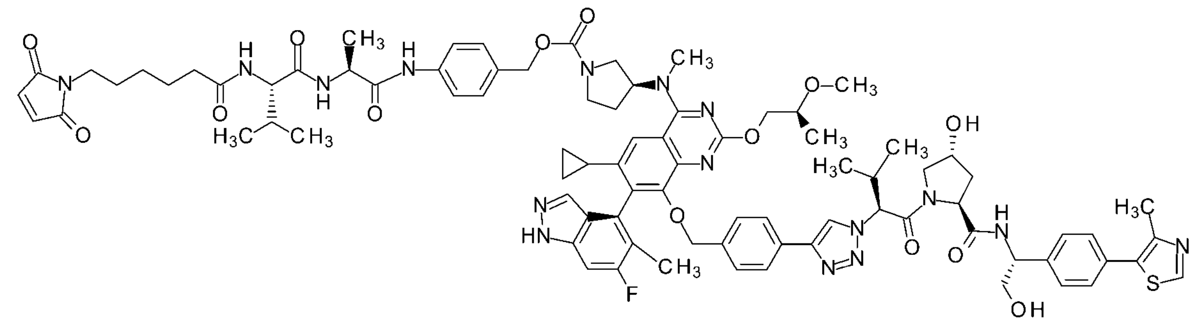

(LD-21)

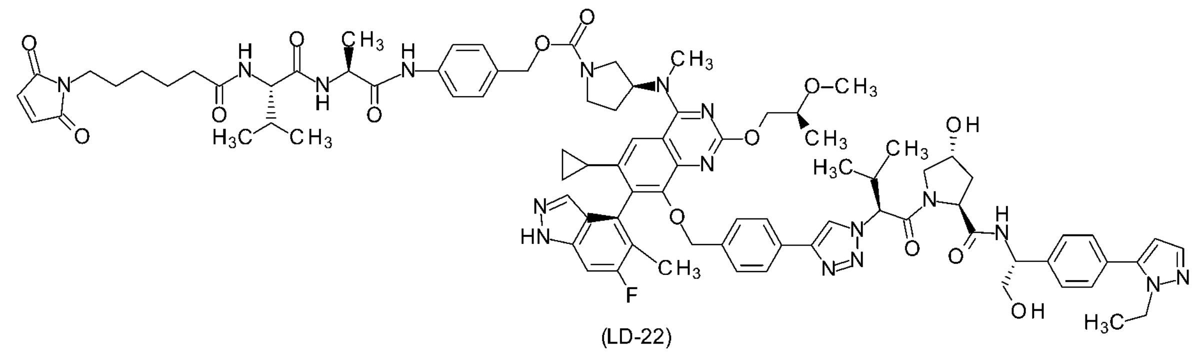

(LD-22)

[Chemical Formula 37-3]

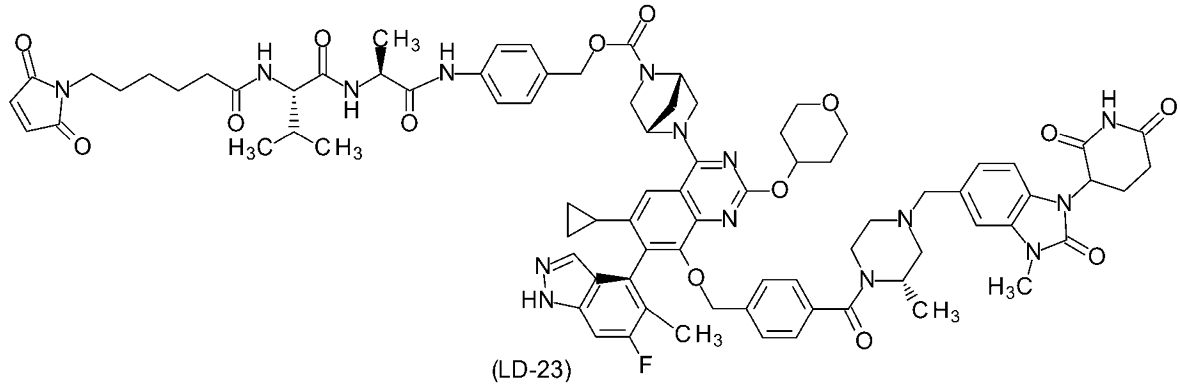

(LD-23)

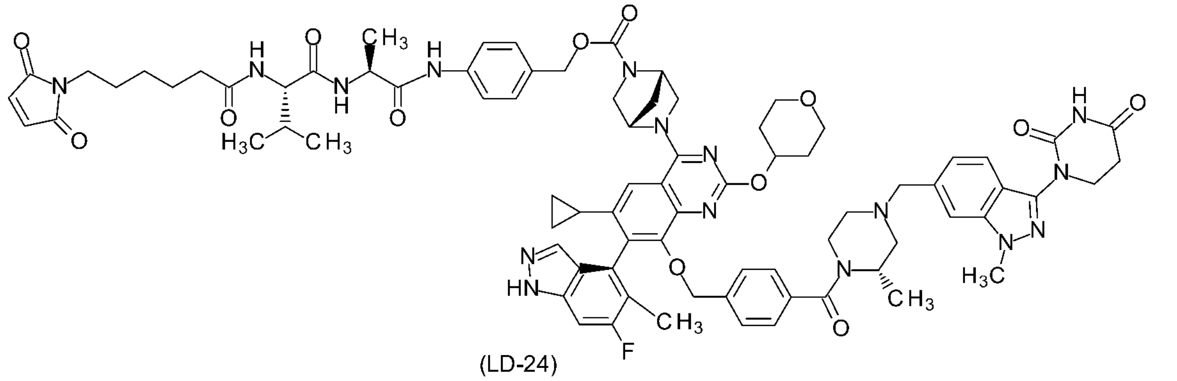

(LD-24)

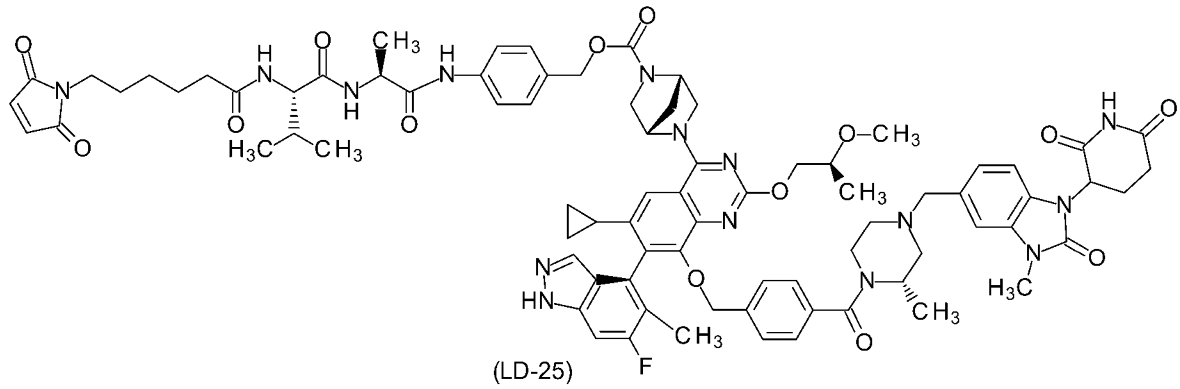

(LD-25)

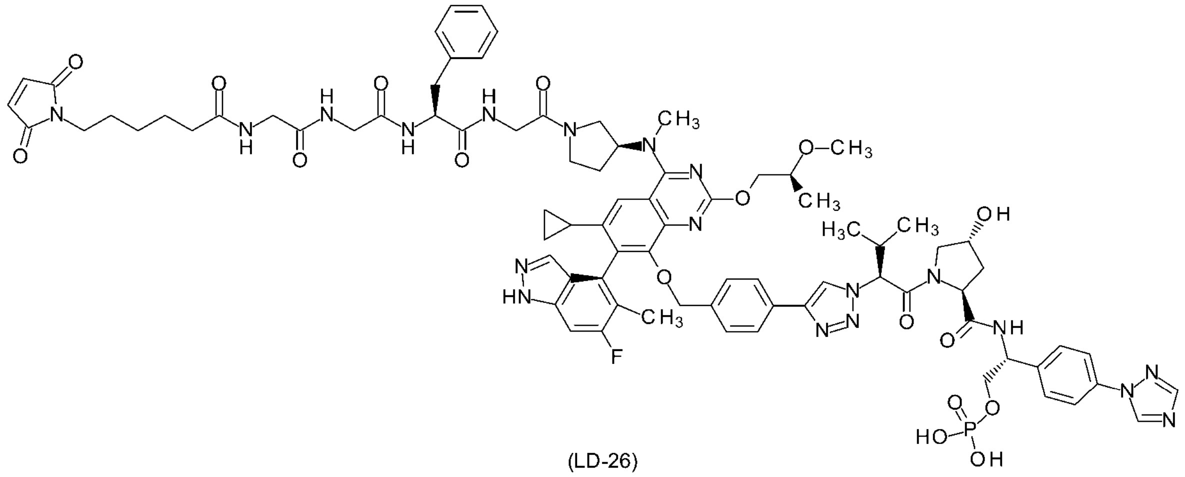

(LD-26)

**INTERNATIONAL SEARCH REPORT**

International application No. **PCT/JP2024/032664**

**A. CLASSIFICATION OF SUBJECT MATTER**

***A61K 47/68***(2017.01)i; ***A61K 31/517***(2006.01)i; ***A61K 39/395***(2006.01)i; ***A61P 35/00***(2006.01)i; ***A61P 35/02***(2006.01)i; ***C07D 403/14***(2006.01)i; ***C07D 487/08***(2006.01)i; ***C07K 16/28***(2006.01)i

FI: A61K47/68 ZNA; A61P35/02; A61K39/395 D; A61K39/395 N; A61K39/395 E; A61K39/395 T; A61K31/517; A61K39/395 L; A61K39/395 C; C07K16/28; A61P35/00 ZNA; C07D487/08; C07D403/14

According to International Patent Classification (IPC) or to both national classification and IPC

**B. FIELDS SEARCHED**

Minimum documentation searched (classification system followed by classification symbols)

A61K47/68; A61K31/517; A61K39/395; A61P35/00; A61P35/02; C07D403/14; C07D487/08; C07K16/28

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

CAplus/REGISTRY/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| Y | WO 2022/173032 A1 (ASTELLAS PHARMA INC.) 18 August 2022 (2022-08-18) claims, examples, test examples | 1-44 |
| Y | 清水広介ら, がん治療に向けたリポソームＤＤＳにおける抗体利用への期待, Drug Delivery System, 2019, vol. 34, no. 1, pp. 22-28, (SHIMIZU, Kosuke et al., Advantages of antibody usage in liposomal DDS for cancer chemotherapy) in particular, p. 22, right column, p. 25, left column | 1-44 |
| Y | JP 2019-522633 A (GENENTECH, INC.) 15 August 2019 (2019-08-15) claims, paragraphs [0007]-[0015], [0021]-[0022], [0447]-[0453], examples | 1-44 |
| Y | JP 2021-510375 A (DEVELOPMENT CENTER FOR BIOTECHNOLOGY) 22 April 2021 (2021-04-22) claims, paragraphs [0014]-[0022], [0057]-[0060], examples | 1-44 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **12 November 2024** | **26 November 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (July 2022)

**INTERNATIONAL SEARCH REPORT**

International application No.
**PCT/JP2024/032664**

**C. DOCUMENTS CONSIDERED TO BE RELEVANT**

| Category* | Citation of document, with indication, where appropriate, of the relevant passages | Relevant to claim No. |
|---|---|---|
| P, A | WO 2023/171781 A1 (ASTELLAS PHARMA INC.) 14 September 2023 (2023-09-14) claims, examples | 1-44 |
| P, A | WO 2024/019103 A1 (ASTELLAS PHARMA INC.) 25 January 2024 (2024-01-25) claims, examples | 1-44 |

Form PCT/ISA/210 (second sheet) (July 2022)

**INTERNATIONAL SEARCH REPORT**

International application No.
**PCT/JP2024/032664**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**
**Information on patent family members**

International application No.
**PCT/JP2024/032664**

| Patent document cited in search report | Publication date (day/month/year) | Patent family member(s) | Publication date (day/month/year) |
|---|---|---|---|
| WO 2022/173032 A1 | 18 August 2022 | US 2024/0182483 A1<br>claims, examples, test examples<br>EP 4293024 A1<br>CN 116848120 A<br>CA 3211051 A1<br>AU 2022221145 A1<br>KR 10-2023-0145361 A<br>TW 202245751 A | |
| JP 2019-522633 A | 15 August 2019 | US 2019/0175612 A1<br>claims, paragraphs [0007]-[0016], [0022]-[0023], [0553]-[0559], examples<br>WO 2017/201449 A1<br>EP 3458101 A1<br>CN 109152843 A | |
| JP 2021-510375 A | 22 April 2021 | US 2021/0015942 A1<br>claims, paragraphs [0014]-[0030], [0071]-[0074], examples<br>WO 2019/140003 A1<br>EP 3737422 A1<br>TW 201938201 A<br>KR 10-2020-0108289 A<br>CN 112135637 A<br>CA 3088059 A1<br>AU 2019206507 A1 | |
| WO 2023/171781 A1 | 14 September 2023 | AU 2023231912 A1<br>claims, examples<br>TW 202346270 A | |
| WO 2024/019103 A1 | 25 January 2024 | JP 2024-56893 A<br>TW 202409002 A | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2022148421 A
- WO 2022148422 A
- CN 115785199
- WO 2023077441 A
- WO 2022173032 A
- WO 2022228576 A
- WO 2023280026 A
- WO 2023171781 A
- WO 2024019103 A
- WO 2017201449 A
- WO 2019140003 A
- WO 2021195598 A
- WO 2023056069 A
- US 5225539 A
- US 6180370 B
- WO 2015095124 A
- WO 2015095223 A
- WO 2015095227 A
- WO 2014057687 A
- WO 2013173337 A
- WO 2008141044 A
- WO 2016040856 A
- WO 2010081111 A
- WO 2013185115 A
- WO 2004010957 A
- WO 2023037268 A


### Non-patent literature cited in the description


- *Nature Rev. Cancer*, 2011, vol. 11, 761-774
- *Nature Rev. Drug Discov.*, 2014, vol. 13, 828-851
- *Nature Rev. Drug Discov.*, 2016, vol. 15, 771-785
- *Nat Rev Cancer*, 2018, vol. 18, 767-777
- *Drug. Discov. Today Technol.*, 2019, vol. 31, 15-27
- *Cancer Science*, 2016, vol. 107 (7), 1039-1046
- *Clinical Cancer Research*, 2005, vol. 11, 843-852
- *Cancer Research*, 2016, vol. 76 (10), 3003-3013
- *Current Research in Chemical Biology*, 2022, vol. 2, 100020
- *Front. Chem.*, 2021, vol. 9, 707317
- *J. Am. Chem. Soc.*, 2021, vol. 143, 5141
- *Signal Transduct. Target. Ther*, 2020, vol. 5, 129
- *Nat. Chem. Biol.*, 2019, vol. 15 (7), 737
- *Communications Biology.*, 2020, vol. 3, 140
- *Sci. Rep.*, 2020, vol. 10 (1), 15543
- *ACS Chem. Biol.*, 2019, vol. 14, 2430
- *Cell Chem. Biol.*, 2021, vol. 28 (4), 559
- *J. Am. Chem. Soc.*, 2022, vol. 144, 701
- *ACS Chem. Biol.*, 2019, vol. 14, 2822
- *J. Pharma. Sci.*, 2008, vol. 97, 2426-2447
- **KABAT et al.** *Sequences of Proteins of Immunological Interest*, 1991 (91-3242)
- *Nucleic Acids Res.*, 2015, vol. 43, W580-W584
- *Bioconjugate Chem.*, 2016, vol. 27, 2081-2088
- **KOHLER** ; **MILSTEIN**. *Nature*, 1975, vol. 256, 495-497
- Monoclonal Antibodies. Plenum Press, 1980, 365-367
- *Mol. Immunol.*, 1992, vol. 29, 633-639
- *J. Immunol.*, 2000, vol. 164 (8), 4178-4184
- **P. HEINRICH STAHL**. Handbook of Pharmaceutical Salts Properties, Selection, and Use. Wiley-VCH, 2008
- **P. G. M. WUTS** ; **T. W. GREENE**. Greene's Protective Groups in Organic Synthesis. John Wiley & Sons Inc, 2014
- **P. G. M. WUTS** ; **T. W. GREENE**. Greene's Protective Groups in Organic Synthesis. 2014
- *Chem. Rev.*, 2003, vol. 103, 2945-2964
- *ACC. Chem. Res.*, 1982, vol. 15, 340-348
- *J. Am. Chem. Soc.*, 2005, vol. 127, 4685-4696
- *Org. Lett.*, 2011, vol. 13, 3948-3951
- *Org. Lett.*, 2012, vol. 14, 1278-1281
- *J. Org. Chem.*, 1977, vol. 42, 3491-3494
- *Tetrahedron Letters*, 2013, vol. 54, 5207-5210
- **JIKKEN KAGAKU KOZA**. Courses in Experimental Chemistry in English. Maruzen Co., Ltd., 2004, vol. 13

- **JIKKEN KAGAKU KOZA**. Courses in Experimental Chemistry in English. Maruzen Co., Ltd., 2005, vol. 14
- *Synthesis*, 1999, vol. 9, 1633-1636
- **JIKKEN KAGAKU KOZA**. Courses in Experimental Chemistry in English. Maruzen Co., Ltd., 2004, vol. 17
- Jikken Kagaku Koza (Courses in Experimental Chemistry in English. Maruzen Co., Ltd, 2005, vol. 16
- Angew. Chem. Int. Ed. 2005, vol. 44, 1378-1382
- *Org. Lett.*, 2012, vol. 14 (17), 4678-4681
- *J. Org. Chem.*, 1956, vol. 21 (4), 478-479
- **P. G. M. WUTS** ; **T. W. GREENE**. Greene's Protective Groups in Organic Synthesis. John Wiley & Sons Inc., 2014
- Angew. Chem. Int. Ed. 2002, vol. 41, 2596-2599
- **S. R. SANDLER** ; **W. KARO**. Organic Functional Group Preparations. Academic Press Inc, 1991, vol. 1
- Jikken Kagaku Koza (Courses in Experimental Chemistry in English. Maruzen Co., Ltd., 2005, vol. 16
- Jikken Kagaku Koza (Courses in Experimental Chemistry in English. Maruzen Co., Ltd., 2005, vol. 14
- Jikken Kagaku Koza (Courses in Experimental Chemistry in English. Maruzen Co., Ltd, 2004, vol. 13
- *J. Org. Chem.*, 2012, vol. 77, 1760-1764
- *Nature*, 2019, vol. 574, 86-89
- *Org. Biomol. Chem.*, 2014, vol. 12, 4397-4406
- Angew. Chem. Int. Ed.. 2005, vol. 44, 1378-1382